(19) Europäisches Patentamt
European Patent Office
Office européen des brevets

(11) EP 2 443 248 B1

(12) **EUROPEAN PATENT SPECIFICATION**

(45) Date of publication and mention of the grant of the patent:
**27.12.2017 Bulletin 2017/52**

(21) Application number: **10790004.5**

(22) Date of filing: **14.06.2010**

(51) Int Cl.:
***C12N 9/10*** (2006.01) ***C12N 15/81*** (2006.01)
***C12P 7/64*** (2006.01)

(86) International application number:
**PCT/US2010/038527**

(87) International publication number:
**WO 2010/147900 (23.12.2010 Gazette 2010/51)**

# (54) IMPROVEMENT OF LONG CHAIN OMEGA-3 AND OMEGA-6 POLYUNSATURATED FATTY ACID BIOSYNTHESIS BY EXPRESSION OF ACYL-CoA LYSOPHOSPHOLIPID ACYLTRANSFERASES

VERBESSERTE BIOSYNTHESE LANGKETTIGER MEHRFACH UNGESÄTTIGTER OMEGA-3- UND OMEGA-6-FETTSÄUREN DURCH EXPRESSION VON ACYL-COA-LYSOPHOSPHOLIPID-ACYLTRANSFERASEN

AMÉLIORATION DE LA BIOSYNTHÈSE D'ACIDE GRAS OMÉGA-3 ET OMÉGA-6 POLYINSATURÉ À CHAÎNE LONGUE PAR L'EXPRESSION D'ACYL-COA LYSOPHOSPHOLIPIDE ACYLTRANSFÉRASE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **16.06.2009 US 187359 P**

(43) Date of publication of application:
**25.04.2012 Bulletin 2012/17**

(73) Proprietor: **E. I. du Pont de Nemours and Company**
**Wilmington, DE 19805 (US)**

(72) Inventors:
- **YADAV, Narendra, S.**
  **Wilmington, Delaware 19803 (US)**
- **ZHANG, Hongxiang**
  **Chadds Ford, Pennsylvania 19317 (US)**
- **ZHU, Qun**
  **West Chester, Pennsylvania 19382 (US)**

(74) Representative: **Dzieglewska, Hanna Eva**
**Dehns**
**St Bride's House**
**10 Salisbury Square**
**London EC4Y 8JD (GB)**

(56) References cited:
**WO-A1-2008/034648**
**WO-A2-2006/052807**
**WO-A2-2006/052870**
**WO-A2-2008/076377**
**WO-A2-2009/001315**
**US-A1- 2006 115 881**
**US-A1- 2006 168 687**

- **CHEN ET AL: "The yeast acylglycerol acyltransferase LCA1 is a key component of Lands cycle for phosphatidylcholine turnover", FEBS LETTERS, ELSEVIER, AMSTERDAM, NL, vol. 581, no. 28, 8 November 2007 (2007-11-08), pages 5511-5516, XP022374820, ISSN: 0014-5793, DOI: 10.1016/J.FEBSLET.2007.10.061**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 443 248 B1

**Description**

FIELD OF THE INVENTION

This invention is in the field of biotechnology. More specifically, this invention pertains to eicosapentaenoic acid ["EPA"]-producing recombinant oleaginous *Yarrowia lipolytica* host cells whichover-express genes encoding acyl-CoA:lysophospholipid acyltransferases ["LPLATs"].

BACKGROUND OF THE INVENTION

Glycerophospholipids, the main component of biological membranes, contain a glycerol core with fatty acids attached as R groups at the *sn*-1 position and *sn*-2 position, and a polar head group joined at the *sn*-3 position via a phosphodiester bond. The specific polar head group (e.g., phosphatidic acid, chloline, ethanolamine, glycerol, inositol, serine, cardiolipin) determines the name given to a particular glycerophospholipid, thus resulting in phosphatidylcholines ["PC"], phosphatidylethanolamines ["PE"], phosphatidylglycerols ["PG"], phosphatidylinositols ["PI"], phosphatidylserines ["PS"] and cardiolipins ["CL"]. Glycerophospholipids possess tremendous diversity, not only resulting from variable phosphoryl head groups, but also as a result of differing chain lengths and degrees of saturation of their fatty acids. Generally, saturated and monounsaturated fatty acids are esterified at the *sn*-1 position, while polyunsaturated fatty acids are esterified at the *sn*-2 position.

Glycerophospholipid biosynthesis is complex. Table 1 below summarizes the steps in the *de novo* pathway, originally described by Kennedy and Weiss (J. Biol. Chem., 222:193-214 (1956)):

Table 1: General Reactions Of *de Novo* Glycerophospholipid Biosynthesis

| | |
|---|---|
| *sn*-Glycerol-3-Phosphate → Lysophosphatidic Acid (1-acyl-*sn*-glycerol 3-phosphate or "LPA") | Glycerol-3-phosphate acyltransferase (GPAT) [E.C. 2.3.1.15] esterifies 1st acyl-CoA to *sn*-1 position of *sn*-glycerol 3-phosphate |
| LPA → Phosphatidic Acid (1,2-diacylglycerol phosphate or "PA") | Lysophosphatidic acid acyltransferase (LPAAT) [E.C. 2.3.1.51] esterifies 2nd acyl-CoA to *sn*-2 position of LPA |
| PA → 1,2-Diacylglycerol ("DAG") **Or** | Phosphatidic acid phosphatase [E.C. 3.1.3.4] removes a phosphate from PA; DAG can subsequently be converted to PC, PE or TAG (TAG synthesis requires either a diacylglycerol acyltransferase (DGAT) [E.C. 2.3.1.20] or a phospholipid:diacylglycerol acyltransferase (PDAT) [E.C.2.3.1.158]) |
| PA → Cytidine Diphosphate Diacylglycerol ("CDP-DG") | CDP-diacylglycerol synthase [EC 2.7.7.41] causes condensation of PA and cytidine triphosphate, with elimination of pyrophosphate; CDP-DG can subsequently be converted to PI, PS, PG or CL |

Following their *de novo* synthesis, glycerophospholipids can undergo rapid turnover of the fatty acyl composition at the *sn*-2 position. This "remodeling", or "acyl editing", is important for membrane structure and function, biological response to stress conditions, and manipulation of fatty acid composition and quantity in biotechnological applications. Specifically, the remodeling has been attributed to deacylation of the glycerophospholipid and subsequent reacylation of the resulting lysophospholipid.

In the Lands' cycle (Lands, W.E., J. Biol. Chem., 231:883-888 (1958)), remodeling occurs through the concerted action of: 1) a phospholipase, such as phospholipase $A_2$, that releases fatty acids from the *sn*-2 position of phosphatidylcholine; and, 2) acyl-CoA:lysophospholipid acyltransferases ["LPLATs"], such as lysophosphatidylcholine acyltransferase ["LPCAT"] that reacylates the lysophosphatidylcholine ["LPC"] at the *sn*-2 position. Other glycerophospholipids can also be involved in the remodeling with their respective lysophospholipid acyltransferase activity, including LPLAT enzymes having lysophosphatidylethanolamine acyltransferase ["LPEAT"] activity, lysophosphatidylserine acyltransferase ["LPSAT"] activity, lysophosphatidylglycerol acyltransferase ["LPGAT"] activity and lysophosphatidylinositol acyltransferase ["LPIAT"] activity. In all cases, LPLATs are responsible for removing acyl-CoA fatty acids from the cellular acyl-CoA pool and acylating various lysophospholipid substrates at the *sn*-2 position in the phospholipid pool. Finally, LPLATs also include LPAAT enzymes that are involved in the *de novo* biosynthesis of PA from LPA. LPCAT activity is associated with two structurally distinct protein families, wherein one belongs to the LPAAT family of proteins and the other belongs to the membrane bound O-acyltransferase ["MBOAT"] family of proteins.

In other cases, this *sn*-2 position remodeling has been attributed to the forward and reverse reactions of enzymes having LPCAT activity (Stymne S. and A.K. Stobart, Biochem J., 223(2):305-314(1984)).

Several recent reviews by Shindou et al. provide an overview of glycerophospholipid biosynthesis and the role of LPLATs (J. Biol. Chem., 284(1):1-5 (2009); J. Lipid Res., 50:S46-S51 (2009)). Numerous LPLATs have been reported in public and patent literature, based on a variety of conserved motifs.

The effect of LPLATs on polyunsaturated fatty acid ["PUFA"] production has also been contemplated, since fatty acid biosynthesis requires rapid exchange of acyl groups between the acyl-CoA pool and the phospholipid pool. Specifically, desaturations occur mainly at the *sn*-2 position of phospholipids, while elongation occurs in the acyl-CoA pool. For example, Intl. App. Pub. No. WO 2004/076617 describes the isolation of an LPCAT from *Caenorhabditis elegans* (clone T06E8.1) and reports increase in the efficiency of Δ6 desaturation and Δ6 elongation, as well as an increase in biosynthesis of the long-chain PUFAs eicosadienoic acid ["EDA"; 20:2] and eicosatetraenoic acid ["ETA"; 20:4], respectively, when the LPCAT was expressed in an engineered strain of *Saccharomyces cerevisiae* that was fed exogenous 18:2 or α-linolenic ["ALA"; 18:3] fatty acids, respectively.

Furthermore, Example 16 of Intl. App. Pub. No. WO 2004/087902 describes the isolation of *Mortierella alpina* LPAAT-like proteins (encoded by the proteins of SEQ ID NO:93 and SEQ ID NO:95, having 417 amino acids in length or 389 amino acids in length, respectively) that are identical except for an N-terminal extension of 28 amino acid residues in SEQ ID NO:93. Intl. App. Pub. No. WO 2004/087902 also reports expression of one of these proteins using similar methods to those of Intl. App. Pub. No. WO 2004/076617, which results in similar improvements in EDA and ETA biosynthesis.

Both Intl. App. Publications No. WO 2004/076617 and No. WO 2004/087902 teach that the improvements in EDA and ETA biosynthesis are due to reversible LPCAT activity in some LPAAT-like proteins, although not all LPAAT-like proteins have LPCAT activity. They do not teach that LPCAT expression would result in the improvements in strains that do not require exogenous feeding of fatty acid substrates or in microbial species other than *Saccharomyces cerevisiae.* They also do not teach that LPCAT expression in engineered microbes results in increased production of high LC-PUFAs other than EDA and ETA, such as ARA, EPA and DHA, or that LPCAT expression can result in improvement in alternate desaturation reactions, other than Δ6 desaturation. Neither reference teaches the effect of the LPCAT or LPAAT-like proteins on either Δ6 elongation without exogenous feeding of fatty acids or on Δ4 desaturation.

Numerous other references generally describe benefits of co-expressing LPLATs with PUFA biosynthetic genes, to increase the amount of a desired fatty acid in the oil of a transgenic organism, increase total oil content or selectively increase the content of desired fatty acids (e.g., Intl. App. Pubublications No. WO 2004/087902, No. WO 2006/069936, No. WO 2006/052870, No. WO 2009/001315, No. WO 2009/014140).

Despite the work described above, to date no one has studied the effect of LPAATs and LPCATs in an oleaginous organism engineered for high-level production of LC-PUFAs other than EDA and ETA, such as eicosapentaenoic acid ["EPA"; *cis*-5, 8, 11, 14, 17-eicosapentaenoic acid] and/or docosahexaenoic acid ["DHA"; *cis*-4, 7, 10, 13, 16, 19-docosahexaenoic acid] and for improved $C_{18}$ to $C_{20}$ elongation conversion efficiency, and/or improved Δ4 desaturation conversion efficiency without exogenously feeding fatty acids.

## SUMMARY OF THE INVENTION

In one embodiment, the invention concerns a recombinant oleaginous eicosapentaenoic acid (EPA)-producing *Yarrowia lipolytica* host cell which comprises at least one transgene comprising a polynucleotide encoding a polypeptide having at least acyl-CoA:lysophospholipid acyltransferase activity, wherein the polypeptide has at least 95% amino acid identity, based on the Clustal W method of alignment, when compared to the amino acid sequence of SEQ ID NO:11; wherein the transgene comprises the polynucleotide operably linked to at least one regulatory sequence, wherein said at least one regulatory sequence includes a promoter which is a constitutive promoter or which is a regulatable promoter having an inducible activity which allows induced expression; and,
wherein the polypeptide is over-expressed when compared to a control host cell.

In another embodiment, the invention concerns a method for making an oil comprising eicosapentaenoic acid comprising:

a) culturing an oleaginous *Yarrowia lipolytica* host cell of the invention, in which host cell the polynucleotide encoding a polypeptide having at least acyl-CoA:lysophospholipid acyltransferase activity is stably integrated, wherein the host cell has an increase in C18 to C20 elongation conversion efficiency of at least 13 % compared to a control host cell and wherein an oil comprising eicosapentaenoic acid is produced; and,
b) optionally recovering the microbial oil of step (a).

## BIOLOGICAL DEPOSITS

The following biological materials have been deposited with the American Type Culture Collection (ATCC), 10801 University Boulevard, Manassas, VA 20110-2209, and bear the following designations, accession numbers and dates of deposit.

| Biological Material | Accession No. | Date of Deposit |
|---|---|---|
| *Yarrowia lipolytica* Y4128 | ATCC PTA-8614 | August 23, 2007 |
| *Yarrowia lipolytica* Y8406 | ATCC PTA-10025 | May 14, 2009 |
| *Yarrowia lipolytica* Y8412 | ATCC PTA-10026 | May 14, 2009 |

The biological materials listed above were deposited under the terms of the Budapest Treaty on the International Recognition of the Deposit of Microorganisms for the Purposes of Patent Procedure.

BRIEF DESCRIPTION OF THE DRAWINGS AND

SEQUENCE DESCRIPTIONS

FIG. 1A and FIG. 1B illustrate the ω-3/ω-6 fatty acid biosynthetic pathway, and should be viewed together when considering the description of this pathway.
FIG. 2 diagrams the development of *Yarrowia lipolytica* strain Y8406, producing greater than 51.2 EPA % TFAs.
FIG. 3 provides a plasmid map for pY116.
FIG. 4 provides plasmid maps for the following: (A) pZKSL-5S5A5; and, (B) pZP3-Pa777U.
FIG. 5 provides plasmid maps for the following: (A) pZKUM; and, (B) pZKL2-5mB89C.
FIG. 6 provides plasmid maps for the following: (A) pZKL1-2SR9G85; and, (B) pZSCP-Ma83.
FIG. 7 diagrams the development of *Yarrowia lipolytica* strain Y5037, producing 18.6 EPA % TFAs, 22.8 DPA % TFAs and 9.7 DHA % TFAs.
FIG. 8 provides plasmid maps for the following: (A) pZKL4-220EA41B; and, (B) pZKL3-4GER44.
FIG. 9 provides a plasmid map for pZKLY-G20444.
FIG. 10 provides plasmid maps for the following: (A) pY201, comprising a chimeric YAT1::ScAle1S::Lip1 gene; and, (B) pY168, comprising a chimeric YAT1::YlAle1::Lip1 gene.
FIG. 11 provides plasmid maps for the following: (A) pY208, comprising a chimeric YAT1::MaLPAAT1S::Lip1 gene; and, (B) pY207, comprising a chimeric YAT1::YILPAAT1::Lip1 gene.
FIG. 12 provides plasmid maps for the following: (A) pY175, comprising a chimeric YAT1::CeLPCATS::Lip1 gene; and, (B) pY153, comprising a chimeric FBAIN::CeLPCATS::YILPAAT1 gene.
FIG. 13 provides plasmid maps for the following: (A) pY222, comprising a chimeric YAT1::ScLPAATS::Lip1 gene; and (B) pY177, comprising a chimeric YAT1::YILPAAT1::Lip1 gene.

The invention can be more fully understood from the following detailed description and the accompanying sequence descriptions.

The following sequences comply with 37 C.F.R. §1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.

SEQ ID NOs:1-101 are promoters, genes or proteins (or fragments thereof) or plasmids, as identified in Table 2.

Table 2: Summary of Gene and Protein SEQ ID Numbers

| Description | Nucleic acid SEQ ID NO. | Protein SEQ ID NO. |
|---|---|---|
| *Caenorhabditis elegans* LPCAT ("CeLPCAT") | 1 (849 bp) | 2 (282 AA) |
| membrane bound *O*-acyltransferase motif M(V/I)LxxKL | -- | 3 |
| membrane bound *O*-acyltransferase motif RxKYYxxW | -- | 4 |
| membrane bound *O*-acyltransferase motif SAxWHG | -- | 5 |

(continued)

| Description | Nucleic acid SEQ ID NO. | Protein SEQ ID NO. |
|---|---|---|
| Synthetic LPCAT derived from *Caenorhabditis elegans,* codon-optimized for expression in *Yarrowia lipolytica* ("CeLPCATS") | 6 (859 bp) | 7 (282 AA) |
| *Saccharomyces cerevisiae* Ale1 ("ScAle1"; also ORF "YOR175C") | 8 (1860 bp) | 9 (619 AA) |
| *Yarrowia lipolytica* Ale1 ("YlAle1") | 10 (1539 bp) | 11 (512 AA) |
| Synthetic Ale1 derived from *Saccharomyces cerevisiae,* codon-optimized for expression in *Yarrowia lipolytica* ("ScAle1S") | 12 (1870 bp) | 13 (619 AA) |
| *Mortierella alpina* LPAAT1 ("MaLPAAT1") | 14 (945 bp) | 15 (314 AA) |
| *Yarrowia lipolytica* LPAAT1 ("YlLPAAT1") | 16 (1549 bp) | 17 (282 AA) |
| *Saccharomyces cerevisiae* LPAAT ("ScLPAAT"; also ORF "YDL052C") | -- | 18 (303 AA) |
| 1-acyl-sn-glycerol-3-phosphate acyltransferase motif NHxxxxD | -- | 19 |
| 1-acyl-*sn*-glycerol-3-phosphate acyltransferase motif EGTR | -- | 20 |
| Synthetic LPAAT1 derived from *Mortierella alpina,* codon-optimized for expression in *Yarrowia lipolytica* ("MaLPAAT1S") | 21 (955 bp) | 22 (314 AA) |
| Shindou et al. membrane bound *O*-acyltransferase motif WHGxxxGYxxxF | -- | 23 |
| Shindou et al. membrane bound *O*-acyltransferase motif YxxxxF | -- | 24 |
| Shindou et al. membrane bound *O*-acyltransferase motif YxxxYFxxH | -- | 25 |
| U.S. Pat. Pub. No. 2008-0145867-A1 motif | -- | 26 |
| M-[V/I]-[L/I]-xxK-[L/V/I]-xxxxxxDG | | |
| U.S. Pat. Pub. No. 2008-0145867-A1 motif RxKYYxxWxxx-[E/D]-[A/G] xxxxGxG-[F/Y]-xG | -- | 27 |
| U.S. Pat. Pub. No. 2008-0145867-A1 motif $EX_{11}WNX_2$-T/V]-$X_2W$ | -- | 28 |
| U.S. Pat. Pub. No. 2008-0145867-A1 motif SAxWHGxxPGYxx-[T/F]-F | -- | 29 |
| Lewin, T.W. et al. & Yamashita et al. 1-acyl-*sn*-glycerol-3-phosphate acyltransferase motif GxxFI-[D/R]-R | -- | 30 |
| Lewin, T.W. et al. 1-acyl-sn-glycerol-3-phosphate acyltransferase motif [V/I]-[P/X]-[I/V/L]-[I/V]-P-[V/I] | -- | 31 |
| Yamashita et al. 1-acyl-*sn*-glycerol-3-phosphate acyltransferase motif IVPIVM | -- | 32 |
| Plasmid pY116 | 33 (8739 bp) | -- |
| Plasmid pZKSL-5S5A5 | 34 (13,975 bp) | -- |
| Synthetic mutant Δ5 desaturase ("EgD5SM"), derived from *Euglena gracilis* ("EgD5S") (U.S. Pat. Pub. No. 2010-0075386-A1) | 35 (1350 bp) | 36 (449 AA) |
| Synthetic mutant Δ5 desaturase ("EaD5SM"), derived from *Euglena anabaena* ("EaD5S") (U.S. Pat. Pub. No. 2010-0075386-A1) | 37 (1365 bp) | 38 (454 AA) |
| Plasmid pZP3-Pa777U | 39 (13,066 bp) | -- |
| Plasmjd pZKUM | 40 (4313 bp) | -- |

(continued)

| Description | Nucleic acid SEQ ID NO. | Protein SEQ ID NO. |
|---|---|---|
| Plasmid pZKL2-5mB89C | 41 (15,991 bp) | -- |
| *Yarrowia lipolytica* diacylglycerol cholinephosphotransferase gene ("YlCPT1") | 42 (1185 bp) | 43 (394 AA) |
| Synthetic mutant Δ8 desaturase ("EgD8M") (U.S. Patent 7,709,239), derived from *Euglena gracilis* ("EgD8S") (U.S. Patent 7,256,033) | 44 (1272 bp) | 45 (422 AA) |
| Synthetic Δ9 elongase derived from *Euglena gracilis,* codon-optimized for expression in *Yarrowia lipolytica* ("EgD9eS") | 46 (777 bp) | 47 (258 AA) |
| Plasmid pZKL1-2SR9G85 | 48 (14,554 bp) | -- |
| DGLA synthase, comprising E389D9eS/EgD8M gene fusion | 49 (2127 bp) | 50 (708 AA) |
| Synthetic Δ12 desaturase derived from *Fusarium moniliforme,* codon-optimized for expression in *Yarrowia lipolytica* ("FmD12S") | 51 (1434 bp) | 52 (477 AA) |
| Plasmid pZSCP-Ma83 | 53 (15,119 bp) | -- |
| Synthetic $C_{16/18}$ elongase derived from *Mortierella alpina* ELO3, codon-optimized for expression in | 54 (828 bp) | 55 (275 AA) |
| *Yarrowia lipolytica* ("ME3S") | | |
| Synthetic malonyl-CoA synthetase derived from *Rhizobium leguminosarum* bv. viciae 3841 (GenBank Accession No. YP_766603), codon-optimized for expression in *Yarrowia lipolytica* ("MCS") | 56 (1518 bp) | 57 (505 AA) |
| Synthetic Δ8 desaturase derived from *Euglena anabaena* UTEX 373, codon-optimized for expression in *Yarrowia lipolytica* ("EaD8S") | 58 (1260 bp) | 59 (420 AA) |
| Plasmid pZKL4-220EA41B | 60 (16,424 bp) | -- |
| Synthetic C20 elongase derived from *Euglena anabaena,* codon-optimized for expression in *Yarrowia lipolvtica* ("EaC20ES") | 61 (900 bp) | 62 (299 AA) |
| Synthetic C20 elongase derived from *Euglena gracilis,* codon-optimized for expression in *Yarrowia lipolytica* ("EgC20ES") | 63 (912 bp) | 64 (303 AA) |
| Truncated synthetic Δ4 desaturase derived from *Euglena anabaena,* codon-optimized for expression in *Yarrowia lipolytica* ("EaD4S-1") | 65 (1644 bp) | 66 (547 AA) |
| Truncated synthetic Δ4 desaturase version B derived from *Euglena anabaena,* codon-optimized for expression in *Yarrowia lipolytica* ("EaD4SB") | 67 (1644 bp) | 68 (547 AA) |
| Plasmid pZKL3-4GER44 | 69 (17,088 bp) | -- |
| Synthetic Δ4 desaturase derived from *Eutreptiella cf_gymnastica* CCMP1594, codon-optimized for expression in *Yarrowia lipolytica* ("E1594D4S") | 70 (1548 bp) | 71 (515 AA) |
| Truncated synthetic Δ4 desaturase derived from *Euglena gracilis,* codon-optimized for expression in *Yarrowia lipolvtica* ("EgD4S-1") | 72 (1542 bp) | 73 (513 AA) |
| Plasmid pZKLY-G20444 | 74 (15,617 bp) | -- |
| Synthetic DHA synthase derived from *Euglena gracilis,* codon-optimized for expression in *Yarrowia lipolytica* ("EgDHAsyn1S") | 75 (2382 bp) | 76 (793 AA) |
| Plasmid pY201 | 77 (9641 bp) | -- |
| *Escherichia coli* LoxP recombination site, recognized by a Cre recombinase enzyme | 78 (34 bp) | -- |
| Primer 798 | 79 | -- |

(continued)

| Description | Nucleic acid SEQ ID NO. | Protein SEQ ID NO. |
|---|---|---|
| Primer 799 | 80 | -- |
| Primer 800 | 81 | -- |
| Primer 801 | 82 | -- |
| Plasmid pY168 | 83 (9320 bp) | -- |
| Plasmid pY208 | 84 (8726 bp) | -- |
| Primer 856 | 85 | -- |
| Primer 857 | 86 | -- |
| Plasmid pY207 | 87 (8630 bp) | -- |
| Plasmid pY175 | 88 (8630 bp) | -- |
| Plasmid pY153 | 89 (8237 bp) | -- |
| Mutant Δ5 desaturase ("EgD5M"), derived from *Euglena gracilis* ("EgD5") (U.S. Pat. Pub. No. 2010-0075386-A1) | 90 (1350 bp) | 91 (449 AA) |
| *Mortierella alpina* LPAAT (corresponding to SEQ ID NOs:16 and 17 within Intl. App. Pub. No. WO 2004/087902) | 92 (1254 bp) | 93 (417 AA) |
| *Mortierella alpina* LPAAT (corresponding to SEQ ID NOs:18 and 19 within Intl. App. Pub. No. WO 2004/087902) | 94 (1170 bp) | 95 (389 AA) |
| Synthetic LPAAT derived from *Saccharomyces cerevisiae,* codon-optimized for expression in *Yarrowia lipolytica* ("ScLPAATS") | 96 (926 bp) | 97 (303 AA) |
| Primer 869 | 98 | -- |
| Primer 870 | 99 | -- |
| Plasmid pY222 | 100 (7891 bp) | -- |
| Plasmid pY177 | 101 (9598 bp) | -- |

DETAILED DESCRIPTION

Described herein are methods for increasing $C_{18}$ to $C_{20}$ elongation conversion efficiency and/or Δ4 desaturation conversion efficiency in long-chain polyunsaturated fatty acid ["LC-PUFA"]-producing recombinant oleaginous microbial host cells, based on expression of polypeptides (e.g., Ale1, LPAAT, and LPCAT) having LPLAT activity. By increasing the conversion efficiency of $C_{18}$ to $C_{20}$ elongation and/or Δ4 desaturation, the concentration of the LC-PUFAs eicosapentaenoic acid ["EPA"; *cis-5,* 8, 11, 14, 17-eicosapentaenoic acid] and/or docosahexaenoic acid ["DHA"; *cis-4,* 7, 10, 13, 16, 19-docosahexaenoic acid] increased as a weight percent of the total fatty acids.

PUFAs, such as EPA and DHA (or derivatives thereof), are used as dietary substitutes, or supplements, particularly infant formulas, for patients undergoing intravenous feeding or for preventing or treating malnutrition. Alternatively, the purified PUFAs (or derivatives thereof) may be incorporated into cooking oils, fats or margarines formulated so that in normal use the recipient would receive the desired amount for dietary supplementation. The PUFAs may also be incorporated into infant formulas, nutritional supplements or other food and drink products and may find use as cardiovascular-protective, anti-depression, anti-inflammatory or cholesterol lowering agents. Optionally, the compositions may be used for pharmaceutical use, either human or veterinary.

In this disclosure, a number of terms and abbreviations are used. The following definitions are provided.

"Open reading frame" is abbreviated as "ORF".

"Polymerase chain reaction" is abbreviated as "PCR".

"American Type Culture Collection" is abbreviated as "ATCC".

"Polyunsaturated fatty acid(s)" is abbreviated as "PUFA(s)".

"Diacylglycerol acyltransferase" is abbreviated as "DAG AT" or "DGAT".

"Triacylglycerols" are abbreviated as "TAGs".

"Co-enzyme A" is abbreviated as "CoA".

"Total fatty acids" are abbreviated as "TFAs".

"Fatty acid methyl esters" are abbreviated as "FAMEs".

"Dry cell weight" is abbreviated as "DCW".

"Long-chain polyunsaturated fatty acid(s)" is abbreviated as "LC-PUFA(s)".

"Acyl-CoA:lysophospholipid acyltransferase(s)" or "lysophospholipid acyltransferase(s)" is abbreviated as "LPLAT(s)".

The term "glycerophospholipids" refers to a broad class of molecules, having a glycerol core with fatty acids at the *sn*-1 position and *sn*-2 position, and a polar head group (e.g., phosphate, choline, ethanolamine, glycerol, inositol, serine, cardiolipin) joined at the *sn*-3 position via a phosphodiester bond. Glycerophospholipids thus include phosphatidylcholines ["PC"], phosphatidylethanolamines ["PE"], phosphatidylglycerols ["PG"], phosphatidylinositols ["PI"], phosphatidylserines ["PS"] and cardiolipins ["CL"].

"Lysophospholipids" are derived from glycerophospholipids, by deacylation of the sn-2 position fatty acid. Lysophospholipids include, e.g., lysophosphatidic acid ["LPA"], lysophosphatidylcholine ["LPC"], lysophosphatidyletanolamine ["LPE"], lysophosphatidylserine ["LPS"], lysophosphatidylglycerol ["LPG"] and lysophosphatidylinositol ["LPI"].

The term "acyltransferase" refers to an enzyme responsible for transferring an acyl group from a donor lipid to an acceptor lipid molecule.

The term "acyl-CoA:lysophospholipid acyltransferase" or "lysophospholipid acyltransferase" ["LPLAT"] refers to a broad class of acyltransferases, having the ability to acylate a variety of lysophospholipid substrates at the *sn*-2 position. More specifically, LPLATs include LPA acyltransferases ["LPAATs"] having the ability to catalyze conversion of LPA to PA, LPC acyltransferases ["LPCATs"] having the ability to catalyze conversion of LPC to PC, LPE acyltransferases ["LPEATs"] having the ability to catalyze conversion of LPE to PE, LPS acyltransferases ["LPSATs"] having the ability to catalyze conversion of LPS to PS, LPG acyltransferases ["LPGATs"] having the ability to catalyze conversion of LPG to PG, and LPI acyltransferases ["LPIATs"] having the ability to catalyze conversion of LPI to PI. Standardization of LPLAT nomenclature has not been formalized, so various other designations are used in the art (for example, LPAATs have also been referred to as acyl-CoA:1-acyl-*sn*-glycerol-3-phosphate 2-O-acyltransferases, 1-acyl-*sn*-glycerol-3-phosphate acyltransferases and/or 1-acylglycerolphosphate acyltransferases ["AGPATs"] and LPCATs are often referred to as acyl-CoA:1-acyl lysophosphatidyl-choline acyltransferases). Additionally, it is important to note that some LPLATs, such as the *Saccharomyces cerevisiae* Ale1 (ORF YOR175C; SEQ ID NO:9), have broad specificity and thus a single enzyme may be capable of catalyzing several LPLAT reactions, including LPAAT, LPCAT and LPEAT reactions (Tamaki, H. et al., J. Biol. Chem., 282:34288-34298 (2007); Stahl, U. et al., FEBS Letters, 582:305-309 (2008); Chen, Q. et al., FEBS Letters, 581:5511-5516 (2007); Benghezal, M. et al., J. Biol. Chem., 282:30845-30855 (2007); Riekhof, et al., J. Biol. Chem., 282:28344-28352 (2007)).

More specifically, the term "polypeptide having at least lysophosphtidylcholine acyltransferase ["LPCAT"] activity" will refer to those enzymes capable of catalyzing the reaction: acyl-CoA + 1-acyl-*sn*-glycero-3-phosphocholine = CoA + 1,2-diacyl-sn-glycero-3-phosphocholine (EC 2.3.1.23). LPCAT activity has been described in two structurally distinct protein families, i.e., the LPAAT protein family (Hishikawa, et al., Proc. Natl. Acad. Sci. U.S.A., 105:2830-2835 (2008); Intl. App. Pub. No. WO 2004/076617) and the ALE1 protein family (Tamaki, H. et al., *supra;* Stahl, U. et al., *supra;* Chen, Q. et al., *supra;* Benghezal, M. et al., *supra;* Riekhof, et al., *supra*).

The term "LPCAT" refers to a protein of the ALE1 protein family that: 1) has LPCAT activity (EC 2.3.1.23) and shares at least about 45% amino acid identity, based on the Clustal W method of alignment, when compared to an amino acid sequence selected from the group consisting of SEQ ID NO:9 (ScAle1) and SEQ ID NO:11 (YlAle1); and/or, 2) has LPCAT activity (EC 2.3.1.23) and has at least one membrane bound O-acyltransferase ["MBOAT"] protein family motif selected from the group consisting of: M(V/I)LxxKL (SEQ ID NO:3), RxKYYxxW (SEQ ID NO:4), SAxWHG (SEQ ID NO:5) and $EX_{11}WNX_2$-[T/V]-$X_2W$ (SEQ ID NO:28). Examples of ALE1 polypeptides include ScAle1 and YlAle1.

The term "ScAle1" refers to a LPCAT (SEQ ID NO:9) isolated from *Saccharomyces cerevisiae* (ORF "YOR175C"), encoded by the nucleotide sequence set forth as SEQ ID NO:8. In contrast, the term "ScAle1S" refers to a synthetic LPCAT derived from *S. cerevisiae* that is codon-optimized for expression in *Yarrowia lipolytica* (i.e., SEQ ID NOs:12 and 13).

The term "YlAle1" refers to a LPCAT (SEQ ID NO:11) isolated from *Yarrowia lipolytica,* encoded by the nucleotide sequence set forth as SEQ ID NO:10.

The term "LPCAT" also refers to a protein that has LPCAT activity (EC 2.3.1.23) and shares at least about 90% amino acid identity, based on the Clustal W method of alignment, when compared to an amino acid sequence as set forth in SEQ ID NO:2 (CeLPCAT).

The term "CeLPCAT" refers to a LPCAT enzyme (SEQ ID NO:2) isolated from *Caenorhabditis elegans,* encoded by the nucleotide sequence set forth as SEQ ID NO:1. In contrast, the term "CeLPCATS" refers to a synthetic LPCAT derived from *C. elegans* that is codon-optimized for expression in *Yarrowia lipolytica* (i.e., SEQ ID NOs:6 and 7).

The term "polypeptide having at least lysophosphatidic acid acyltransferase ["LPAAT"] activity" will refer to those enzymes capable of catalyzing the reaction: acyl-CoA + 1-acyl-*sn*-glycerol 3-phosphate = CoA + 1,2-diacyl-*sn*-glycerol 3-phosphate (EC 2.3.1.51).

The term "LPAAT" refers to a protein that: 1) has LPAAT activity and shares at least about 43.9% amino acid identity, based on the Clustal W method of alignment, when compared to an amino acid sequence selected from the group consisting of SEQ ID NO:15 (MaLPAAT1), SEQ ID NO:17 (YlLPAAT1) and SEQ ID NO:18 (ScLPAAT1); and/or, 2) has LPAAT activity and has at least one 1-acyl-*sn*-glycerol-3-phosphate acyltransferase family motif selected from the group consisting of: NHxxxxD (SEQ ID NO:19) and EGTR (SEQ ID NO:20). Examples of LPAAT polypeptides include ScLPAAT, MaLPAAT1 and YlLPAAT1.

The term "ScLPAAT" refers to a LPAAT (SEQ ID NO:18) isolated from *Saccharomyces cerevisiae* (ORF "YDL052C").

The term "MaLPAAT1" refers to a LPAAT (SEQ ID NO:15) isolated from *Mortierella alpina,* encoded by the nucleotide sequence set forth as SEQ ID NO:14. In contrast, the term "MaLPAAT1S" refers to a synthetic LPAAT derived from *M. alpina* that is codon-optimized for expression in *Yarrowia lipolytica* (i.e., SEQ ID NOs:21 and 22).

The term "YlLPAAT1" refers to a LPAAT (SEQ ID NO:17) isolated from *Yarrowia lipolytica,* encoded by the nucleotide sequence set forth as SEQ ID NO:16.

The term "ortholog" refers to a homologous protein from a different species that evolved from a common ancestor protein as evidenced by being in one clade of phylogenetic tree analysis and that catalyzes the same enzymatic reaction.

The term "conserved domain" or "motif" means a set of amino acids conserved at specific positions along an aligned sequence of evolutionarily related proteins. While amino acids at other positions can vary between homologous proteins, amino acids that are highly conserved at specific positions likely indicate amino acids that are essential in the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

The term "oil" refers to a lipid substance that is liquid at 25 °C and usually polyunsaturated. In oleaginous organisms, oil constitutes a major part of the total lipid. "Oil" is composed primarily of triacylglycerols ["TAGs"] but may also contain other neutral lipids, phospholipids and free fatty acids. The fatty acid composition in the oil and the fatty acid composition of the total lipid are generally similar; thus, an increase or decrease in the concentration of PUFAs in the total lipid will correspond with an increase or decrease in the concentration of PUFAs in the oil, and vice versa.

"Neutral lipids" refer to those lipids commonly found in cells in lipid bodies as storage fats and are so called because at cellular pH, the lipids bear no charged groups. Generally, they are completely non-polar with no affinity for water. Neutral lipids generally refer to mono-, di-, and/or triesters of glycerol with fatty acids, also called monoacylglycerol, diacylglycerol or triacylglycerol, respectively, or collectively, acylglycerols. A hydrolysis reaction must occur to release free fatty acids from acylglycerols.

The term "triacylglycerols" ["TAGs"] refers to neutral lipids composed of three fatty acyl residues esterified to a glycerol molecule. TAGs can contain LC-PUFAs and saturated fatty acids, as well as shorter chain saturated and unsaturated fatty acids.

The term "total fatty acids" ["TFAs"] herein refer to the sum of all cellular fatty acids that can be derivitized to fatty acid methyl esters ["FAMEs"] by the base transesterification method (as known in the art) in a given sample, which may be the biomass or oil, for example. Thus, total fatty acids include fatty acids from neutral lipid fractions (including diacylglycerols, monoacylglycerols and TAGs) and from polar lipid fractions (including the PC and the PE fractions), but not free fatty acids.

The term "total lipid content" of cells is a measure of TFAs as a percent of the dry cell weight ["DCW"], athough total lipid content can be approximated as a measure of FAMEs as a percent of the DCW ["FAMEs % DCW"]. Thus, total lipid content ["TFAs % DCW"] is equivalent to, e.g., milligrams of total fatty acids per 100 milligrams of DCW.

The concentration of a fatty acid in the total lipid is expressed herein as a weight percent of TFAs ["% TFAs"], e.g., milligrams of the given fatty acid per 100 milligrams of TFAs. Unless otherwise specifically stated in the disclosure herein, reference to the percent of a given fatty acid with respect to total lipids is equivalent to concentration of the fatty acid as % TFAs (e.g., % EPA of total lipids is equivalent to EPA % TFAs).

In some cases, it is useful to express the content of a given fatty acid(s) in a cell as its weight percent of the dry cell weight ["% DCW"]. Thus, for example, EPA % DCW would be determined according to the following formula: (EPA % TFAs) * (TFAs % DCW)]/100. The content of a given fatty acid(s) in a cell as its weight percent of the dry cell weight ["% DCW"] can be approximated, however, as: (EPA % TFAs) * (FAMEs % DCW)]/100.

The terms "lipid profile" and "lipid composition" are interchangeable and refer to the amount of individual fatty acids contained in a particular lipid fraction, such as in the total lipid or the oil, wherein the amount is expressed as a weight percent of TFAs. The sum of each individual fatty acid present in the mixture should be 100.

The term "fatty acids" refers to long chain aliphatic acids (alkanoic acids) of varying chain lengths, from about $C_{12}$ to $C_{22}$, although both longer and shorter chain-length acids are known. The predominant chain lengths are between $C_{16}$ and $C_{22}$. The structure of a fatty acid is represented by a simple notation system of "X:Y", where X is the total number of carbon ["C"] atoms in the particular fatty acid and Y is the number of double bonds. Additional details concerning the differentiation between "saturated fatty acids" versus "unsaturated fatty acids", "monounsaturated fatty acids" versus "polyunsaturated fatty acids" ["PUFAs"], and "omega-6 fatty acids" ["ω-6" or "*n*-6"] versus "omega-3 fatty acids" ["ω-3" or "*n*-3"] are provided in U.S. Patent 7,238,482.

Nomenclature used to describe PUFAs herein is given in Table 3. In the column titled "Shorthand Notation", the omega-reference system is used to indicate the number of carbons, the number of double bonds and the position of the double bond closest to the omega carbon, counting from the omega carbon, which is numbered 1 for this purpose. The remainder of the Table summarizes the common names of ω-3 and ω-6 fatty acids and their precursors, the abbreviations that will be used throughout the specification and the chemical name of each compound.

Table 3: Nomenclature of Polyunsaturated Fatty Acids And Precursors

| Common Name | Abbreviation | Chemical Name | Shorthand Notation |
|---|---|---|---|
| Myristic | -- | tetradecanoic | 14:0 |
| Palmitic | Palmitate | hexadecanoic | 16:0 |
| Palmitoleic | -- | 9-hexadecenoic | 16:1 |
| Stearic | -- | octadecanoic | 18:0 |
| Oleic | -- | *cis*-9-octadecenoic | 18:1 |
| Linoleic | LA | *cis*-9, 12-octadecadienoic | 18:2 ω-6 |
| γ-Linolenic | GLA | *cis*-6, 9, 12-octadecatrienoic | 18:3 ω-6 |
| Eicosadienoic | EDA | *cis*-11, 14-eicosadienoic | 20:2 ω-6 |
| Dihomo-γ-Linolenic | DGLA | *cis*-8, 11, 14-eicosatrienoic | 20:3 ω-6 |
| Arachidonic | ARA | *cis*-5, 8, 11, 14-eicosatetraenoic | 20:4 ω-6 |
| α-Linolenic | ALA | *cis*-9, 12, 15-octadecatrienoic | 18:3 ω-3 |
| Stearidonic | STA | *cis*-6, 9, 12, 15-octadecatetraenoic | 18:4 ω-3 |
| Eicosatrienoic | ETrA | *cis*-11, 14, 17-eicosatrienoic | 20:3 ω-3 |
| Sciadonic | SCI | *cis*-5, 11, 14-eicosatrienoic | 20:3b ω-6 |
| Juniperonic | JUP | *cis*-5, 11, 14, 17-eicosatetraenoic | 20:4b ω-3 |
| Eicosatetraenoic | ETA | *cis*-8, 11, 14, 17-eicosatetraenoic | 20:4 ω-3 |
| Eicosapentaenoic | EPA | *cis*-5, 8, 11, 14, 17-eicosapentaenoic | 20:5 ω-3 |
| Docosatetraenoic | DTA | *cis*-7, 10, 13, 16-docosatetraenoic | 22:4 ω-6 |
| Docosapentaenoic | DPAn-6 | *cis*-4, 7, 10, 13, 16-docosapentaenoic | 22:5 ω-6 |
| Docosapentaenoic | DPA | *cis*-7, 10, 13, 16, 19-docosapentaenoic | 22:5 ω-3 |
| Docosahexaenoic | DHA | *cis*-4, 7, 10, 13, 16, 19-docosahexaenoic | 22:6 ω-3 |

The term "long-chain polyunsaturated fatty acid" ["LC-PUFA"] refers to those PUFAs that have chain lengths of $C_{20}$ or greater. Thus, the term LC-PUFA includes at least EDA, DGLA, ARA, ETrA, ETA, EPA, DTA, DPAn-6, DPA and DHA.

A metabolic pathway, or biosynthetic pathway, in a biochemical sense, can be regarded as a series of chemical reactions occurring in order within a cell, catalyzed by enzymes, to achieve either the formation of a metabolic product to be used or stored by the cell, or the initiation of another metabolic pathway (then called a flux generating step). Many of these pathways are elaborate, and involve a step by step modification of the initial substance to shape it into a product having the exact chemical structure desired.

The term "PUFA biosynthetic pathway" refers to a metabolic process that converts oleic acid to ω-6 fatty acids such as LA, EDA, GLA, DGLA, ARA, DRA, DTA and DPAn-6 and ω-3 fatty acids such as ALA, STA, ETrA, ETA, EPA, DPA and DHA. This process is well described in the literature (e.g., see Intl. App. Pub. No. WO 2006/052870). Briefly, this process involves elongation of the carbon chain through the addition of carbon atoms and desaturation of the molecule through the addition of double bonds, via a series of special elongation and desaturation enzymes termed "PUFA biosynthetic pathway enzymes" that are present in the endoplasmic reticulum membrane. More specifically, "PUFA biosynthetic pathway enzymes" refer to any of the following enzymes (and genes which encode said enzymes) associated with the biosynthesis of a PUFA, including: Δ4 desaturase, Δ5 desaturase, Δ6 desaturase, Δ12 desaturase, Δ15 desaturase, Δ17 desaturase, Δ9 desaturase, Δ8 desaturase, Δ9 elongase, $C_{14/16}$ elongase, $C_{16/18}$ elongase, $C_{18/20}$ elongase and/or $C_{20/22}$ elongase.

The term "desaturase" refers to a polypeptide that can desaturate, i.e., introduce a double bond, in one or more fatty acids to produce a fatty acid or precursor of interest. Despite use of the omega-reference system throughout the specification to refer to specific fatty acids, it is more convenient to indicate the activity of a desaturase by counting from the carboxyl end of the substrate using the delta-system. Of particular interest herein are: Δ8 desaturases; Δ5 desaturases; Δ17 desaturases; Δ12 desaturases; Δ15 desaturases; Δ9 desaturases; Δ6 desaturases; and Δ4 desaturases. Δ17 desaturases, and also Δ15 desaturases, are also occasionally referred to as "omega-3 desaturases", "w-3 desaturases", and/or "ω-3 desaturases", based on their ability to convert ω-6 fatty acids into their ω-3 counterparts.

The term "elongase" refers to a polypeptide that can elongate a fatty acid carbon chain to produce an acid 2 carbons longer than the fatty acid substrate that the elongase acts upon. This process of elongation occurs in a multistep mechanism in association with fatty acid synthase, as described in Intl. App. Pub. No. WO 2005/047480. Examples of reactions catalyzed by elongase systems are the conversion of GLA to DGLA, STA to ETA, ARA to DTA and EPA to DPA. In general, the substrate selectivity of elongases is somewhat broad but segregated by both chain length and the degree and type of unsaturation. For example, a $C_{14/16}$ elongase will utilize a $C_{14}$ substrate (e.g., myristic acid), a $C_{16/18}$ elongase will utilize a $C_{16}$ substrate (e.g., palmitate), a $C_{18/20}$ elongase will utilize a $C_{18}$ substrate (e.g., LA, ALA, GLA, STA) and a $C_{20/22}$ elongase (also known as a C20 elongase or Δ5 elongase as the terms can be used interchangeably) will utilize a $C_{20}$ substrate (e.g., ARA, EPA). For the purposes herein, two distinct types of $C_{18/20}$ elongases can be defined: a Δ6 elongase will catalyze conversion of GLA and STA to DGLA and ETA, respectively, while a Δ9 elongase is able to catalyze the conversion of LA and ALA to EDA and ETrA, respectively.

The terms "conversion efficiency" and "percent substrate conversion" refer to the efficiency by which a particular enzyme, such as a desaturase or elongase, can convert substrate to product. The conversion efficiency is measured according to the following formula:

$$([product]/[substrate+product])*100,$$

where 'product' includes the immediate product and all products in the pathway derived from it.

The term "$C_{18}$ to $C_{20}$ elongation conversion efficiency" refers to the efficiency by which $C_{18//20}$ elongases can convert $C_{18}$ substrates (i.e., LA, ALA, GLA, STA) to $C_{20}$ products (i.e., EDA, ETrA, DGLA, ETA). These $C_{18//20}$ elongases can be either Δ9 elongases or Δ6 elongases.

The terms "Δ9 elongation conversion efficiency" and "Δ9 elongase conversion efficiency" refer to the efficiency by which Δ9 elongase can convert $C_{18}$ substrates (i.e., LA, ALA) to $C_{20}$ products (i.e., EDA, ETrA).

The terms "Δ4 desaturation conversion efficiency" and "Δ4 desaturase conversion efficiency" refer to the efficiency by which Δ4 desaturase can convert substrates (i.e., DTA, DPAn-3) to products (i.e., DPAn-6, DHA).

The term "oleaginous" refers to those organisms that tend to store their energy source in the form of oil (Weete, In: Fungal Lipid Biochemistry, 2nd Ed., Plenum, 1980). Generally, the cellular oil content of oleaginous microorganisms follows a sigmoid curve, wherein the concentration of lipid increases until it reaches a maximum at the late logarithmic or early stationary growth phase and then gradually decreases during the late stationary and death phases (Yongmanitchai and Ward, Appl. Environ. Microbiol., 57:419-25 (1991)). It is not uncommon for oleaginous microorganisms to accumulate in excess of about 25% of their dry cell weight as oil. Oleaginous microorganisms include various bacteria, algae, euglenoids, moss, fungi (e.g., *Mortierella*), yeast and stramenopiles (e.g., *Schizochytrium).*

The term "oleaginous yeast" refers to those microorganisms classified as yeasts that can make oil. Examples of oleaginous yeast include, but are no means limited to, the following genera: *Yarrowia, Candida, Rhodotorula, Rhodosporidium, Cryptococcus, Trichosporon* and *Lipomyces.*

The term "fermentable carbon source" means a carbon source that a microorganism will metabolize to derive energy. Typical carbon sources include, but are not limited to: monosaccharides, disaccharides, oligosaccharides, polysaccharides, alkanes, fatty acids, esters of fatty acids, glycerol, monoglycerides, diglycerides, triglycerides, carbon dioxide, methanol, formaldehyde, formate and carbon-containing amines.

As used herein the term "biomass" refers specifically to spent or used cellular material from the fermentation of a recombinant production host producing PUFAs in commercially significant amounts, wherein the preferred production host is a recombinant strain of an oleaginous yeast of the genus *Yarrowia.* The biomass may be in the form of whole cells, whole cell lysates, homogenized cells, partially hydrolyzed cellular material, and/or partially purified cellular material (e.g., microbially produced oil).

The terms "polynucleotide", "polynucleotide sequence", "nucleic acid sequence", "nucleic acid fragment" and "isolated nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. Nucleotides (usually found in their 5'-monophosphate form) are referred to by a single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

As used herein, a nucleic acid fragment is "hybridizable" to another nucleic acid fragment, such as a cDNA, genomic DNA, or RNA molecule, when a single-stranded form of the nucleic acid fragment can anneal to the other nucleic acid fragment under the appropriate conditions of temperature and solution ionic strength. Hybridization and washing conditions are well known and exemplified in Sambrook, J., Fritsch, E. F. and Maniatis, T. Molecular Cloning: A Laboratory Manual, 2nd ed., Cold Spring Harbor Laboratory: Cold Spring Harbor, NY (1989), particularly Chapter 11 and Table 11.1.

A "substantial portion" of an amino acid or nucleotide sequence is that portion comprising enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Basic Local Alignment Search Tool; Altschul, S. F., et al., J. Mol. Biol., 215:403-410 (1993)). In general, a sequence of ten or more contiguous amino acids or thirty or more nucleotides is necessary in order to identify putatively a polypeptide or nucleic acid sequence as homologous to a known protein or gene. Moreover, with respect to nucleotide sequences, gene-specific oligonucleotide probes comprising 20-30 contiguous nucleotides may be used in sequence-dependent methods of gene identification (e.g., Southern hybridization) and isolation, such as *in situ* hybridization of bacterial colonies or bacteriophage plaques. In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence.

The term "complementary" is used to describe the relationship between nucleotide bases that are capable of hybridizing to one another. For example, with respect to DNA, adenosine is complementary to thymine and cytosine is complementary to guanine.

As used herein, the terms "homology" and "homologous" are used interchangeably. They refer to nucleic acid fragments wherein changes in one or more nucleotide bases do not affect the ability of the nucleic acid fragment to mediate gene expression or produce a certain phenotype. These terms also refer to modifications of the nucleic acid fragments such as deletion or insertion of one or more nucleotides that do not substantially alter the functional properties of the resulting nucleic acid fragment relative to the initial, unmodified fragment.

Moreover, the skilled artisan recognizes that homologous nucleic acid sequences are also defined by their ability to hybridize, under moderately stringent conditions, e.g., 0.5X SSC, 0.1% SDS, 60 °C, with the sequences exemplified herein, or to any portion of the nucleotide sequences disclosed herein and which are functionally equivalent thereto. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions. An extensive guide to the hybridization of nucleic acids is found in Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel et al., Eds., Greene Publishing and Wiley-Interscience, New York (1995).

As used herein, the term "percent identity" refers to a relationship between two or more polypeptide sequences or two or more polynucleotide sequences, as determined by comparing the sequences. "Identity" also means the degree of sequence relatedness between polypeptide or polynucleotide sequences, as the case may be, as determined by the percentage of match between compared sequences. "Percent identity" and "percent similarity" can be readily calculated by known methods, including but not limited to those described in: 1) Computational Molecular Biology (Lesk, A. M., Ed.) Oxford University: NY (1988); 2) Biocomputing: Informatics and Genome Projects (Smith, D. W., Ed.) Academic: NY (1993); 3) Computer Analysis of Sequence Data, Part I (Griffin, A. M., and Griffin, H. G., Eds.) Humania: NJ (1994); 4) Sequence Analysis in Molecular Biology (von Heinje, G., Ed.) Academic (1987); and, 5) Sequence Analysis Primer (Gribskov, M. and Devereux, J., Eds.) Stockton: NY (1991).

Preferred methods to determine percent identity are designed to give the best match between the sequences tested. Methods to determine percent identity and percent similarity are codified in publicly available computer programs. Sequence alignments and percent identity calculations may be performed using the MegAlign™ program of the LASERGENE bioinformatics computing suite (DNASTAR Inc., Madison, WI). Multiple alignment of the sequences is performed using the "Clustal method of alignment" which encompasses several varieties of the algorithm including the "Clustal V method of alignment" and the "Clustal W method of alignment" (described by Higgins and Sharp, CABIOS, 5:151-153 (1989); Higgins, D.G. et al., Comput. Appl. Biosci., 8:189-191(1992)) and found in the MegAlign™ (version 8.0.2) program of the LASERGENE bioinformatics computing suite (DNASTAR Inc.). Default parameters for multiple protein alignment using the Clustal W method of alignment correspond to GAP PENALTY=10, GAP LENGTH PENALTY=0.2, Delay Divergent Seqs(%)=30, DNA Transition Weight=0.5, Protein Weight Matrix=Gonnet Series, DNA Weight Matrix=IUB with the 'slow-accurate' option. After alignment of the sequences using either Clustal program, it is possible to obtain a "percent identity" by viewing the "sequence distances" table in the program.

It is well understood by one skilled in the art that many levels of sequence identity are useful in identifying polypeptides, from other species, wherein such polypeptides have the same or similar function or activity. Useful examples of percent identities include any integer percentage from 34% to 100%, such as 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. Also, of interest is any full-length or partial complement of this isolated nucleotide fragment. Suitable nucleic acid fragments not only have the above homologies but typically encode a polypeptide having at least 50 amino acids, preferably at least 100 amino acids, more preferably at least 150 amino acids, still more preferably at least 200 amino acids, and most preferably at least 250 amino acids.

"Codon degeneracy" refers to the nature in the genetic code permitting variation of the nucleotide sequence without affecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

"Synthetic genes" can be assembled from oligonucleotide building blocks that are chemically synthesized using procedures known to those skilled in the art. These oligonucleotide building blocks are annealed and then ligated to form gene segments that are then enzymatically assembled to construct the entire gene. Accordingly, the genes can be tailored for optimal gene expression based on optimization of nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell, where sequence information is available. For example, the codon usage profile for *Yarrowia lipolytica* is provided in U.S. Patent 7,125,672.

"Gene" refers to a nucleic acid fragment that expresses a specific protein, and which may refer to the coding region alone or may include regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene that is introduced into the host organism by gene transfer. Foreign genes can comprise native genes inserted into a non-native organism, native genes introduced into a new location within the native host, or chimeric genes. A "transgene" is a gene that has been introduced into the genome by a transformation procedure. A "codon-optimized gene" is a gene having its frequency of codon usage designed to mimic the frequency of preferred codon usage of the host cell.

"Coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, enhancers, silencers, 5' untranslated leader sequence (e.g., between the transcription start site and the translation initiation codon), introns, polyadenylation recognition sequences, RNA processing sites, effector binding sites and stem-loop structures.

"Promoter" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental or physiological conditions. Promoters that cause a gene to be expressed in most cell types at most times are commonly referred to as "constitutive promoters". It is further recognized that since in most cases the exact boundaries of regulatory sequences have not been completely defined, DNA fragments of different lengths may have identical promoter activity.

The terms "3' non-coding sequence" and "transcription terminator" refer to DNA sequences located downstream of a coding sequence. This includes polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The 3' region can influence the transcription, RNA processing or stability, or translation of the associated coding sequence.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from post-transcriptional processing of the primary transcript and is referred to as the mature RNA. "Messenger RNA" or "mRNA" refers to the RNA that is without introns and which can be translated into protein by the cell. "cDNA" refers to a double-stranded DNA that is complementary to, and derived from, mRNA.

The term "operably linked" refers to the association of nucleic acid sequences on a single nucleic acid fragment so that the function of one is affected by the other. For example, a promoter is operably linked with a coding sequence when it is capable of affecting the expression of that coding sequence. That is, the coding sequence is under the transcriptional control of the promoter. Coding sequences can be operably linked to regulatory sequences in sense or antisense orientation.

The term "expression", as used herein, refers to the transcription and stable accumulation of sense (mRNA) or antisense RNA. Expression may also refer to translation of mRNA into a polypeptide.

"Transformation" refers to the transfer of a nucleic acid molecule into a host organism. The nucleic acid molecule may be a plasmid that replicates autonomously, for example, or, it may integrate into the genome of the host organism. Host organisms containing the transformed nucleic acid fragments are referred to as "transgenic" or "recombinant" or "transformed" or "transformant" organisms.

"Stable transformation" refers to the transfer of a nucleic acid fragment into the genome of a host organism, including both nuclear and organellar genomes, resulting in genetically stable inheritance (i.e., the nucleic acid fragment is "stably integrated"). In contrast, "transient transformation" refers to the transfer of a nucleic acid fragment into the nucleus, or DNA-containing organelle, of a host organism resulting in gene expression without integration or stable inheritance.

The terms "plasmid" and "vector" refer to an extra chromosomal element often carrying genes that are not part of the central metabolism of the cell, and usually in the form of circular double-stranded DNA fragments. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction that is capable of introducing an expression cassette(s) into a cell.

The term "expression cassette" refers to a fragment of DNA comprising the coding sequence of a selected gene and regulatory sequences preceding (5' non-coding sequences) and following (3' non-coding sequences) the coding sequence that are required for expression of the selected gene product. Thus, an expression cassette is typically composed of: 1) a promoter sequence; 2) a coding sequence ["ORF"]; and, 3) a 3' untranslated region (i.e., a terminator) that, in eukaryotes, usually contains a polyadenylation site. The expression cassette(s) is usually included within a vector, to facilitate cloning and transformation. Different expression cassettes can be transformed into different organisms including bacteria, yeast, plants and mammalian cells, as long as the correct regulatory sequences are used for each host.

The term "sequence analysis software" refers to any computer algorithm or software program that is useful for the analysis of nucleotide or amino acid sequences. "Sequence analysis software" may be commercially available or independently developed. Typical sequence analysis software will include, but is not limited to: 1) the GCG suite of programs (Wisconsin Package Version 9.0, Genetics Computer Group (GCG), Madison, WI); 2) BLASTP, BLASTN, BLASTX (Altschul et al., J. Mol. Biol., 215:403-410 (1990)); 3) DNASTAR (DNASTAR, Inc. Madison, WI); 4) Sequencher (Gene Codes Corporation, Ann Arbor, MI); and, 5) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

As previously described, genes encoding LPLATs are found in all eukaryotic cells, based on their intimate role in *de novo* synthesis and remodeling of glycerophospholipids, wherein LPLATs remove acyl-CoA fatty acids from the cellular acyl-CoA pool and acylate various lysophospholipid substrates at the *sn*-2 position in the phospholipid pool. Publicly available sequences encoding LPLATs include ScAle1 (SEQ ID NO:9), ScLPAAT (SEQ ID NO:18), MaLPAAT1 (SEQ ID NO:15) and CeLPCAT (SEQ ID NO:2). The ScAle1 (SEQ ID NO:9) and ScLPAAT (SEQ ID NO:18) protein sequences were used as a query to identify orthologs from the public Y. *lipolytica* protein database (the "Yeast project *Genolevures"* (Center for Bioinformatics, LaBRI, Talence Cedex, France) (see also Dujon, B. et al., Nature, 430(6995):35-44 (2004)). Based on analysis of the best hits, the Ale1 and LPAAT orthologs from *Yarrowia lipolytica* are identified herein as YlAle1 (SEQ ID NO:11) and YILPAAT1 (SEQ ID NO:17), respectively (see Example *5, infra*).

When the sequence of a particular LPLAT gene or protein within a preferred host organism is not known, the LPLAT sequences set forth herein as SEQ ID NOs:2, 9, 11, 15, 17 and 18, or portions of them, may be used to search for LPLAT homologs in the same or other algal, fungal, oomycete, euglenoid, stramenopiles, yeast or plant species using sequence analysis software. In general, such computer software matches similar sequences by assigning degrees of homology to various substitutions, deletions, and other modifications. Use of software algorithms, such as the BLASTP method of alignment with a low complexity filter and the following parameters: Expect value = 10, matrix = Blosum 62 (Altschul, et al., Nucleic Acids Res., 25:3389-3402 (1997)), is well-known for comparing any LPLAT protein against a database of nucleic or protein sequences and thereby identifying similar known sequences within a preferred host organism.

Use of a software algorithm to comb through databases of known sequences is particularly suitable for the isolation of homologs having a relatively low percent identity to publicly available LPLAT sequences, such as those described in SEQ ID NOs:2, 9, 11, 15, 17 and 18. It is predictable that isolation would be relatively easier for LPLAT homologs of at least about 70%-85% identity to publicly available LPLAT sequences. Further, those sequences that are at least about 85%-90% identical would be particularly suitable for isolation and those sequences that are at least about 90%-95% identical would be the most facilely isolated.

LPLAT homologs can also be identified by the use of motifs unique to the LPLAT enzymes. These motifs likely represent regions of the LPLAT protein that are important to the structure, stability or activity of the protein and these motifs are useful as diagnostic tools for the rapid identification of novel LPLAT genes.

A variety of LPLAT motifs have been proposed, with slight variation based on the specific species that are included in analyzed alignments. For example, Shindou et al. (Biochem. Biophys. Res. Comm., 383:320-325 (2009)) proposed the following membrane bound *O*-acyltransferase ["MBOAT"] family motifs to be important for LPLAT activity, based on alignment of sequences from *Homo sapiens, Gallus gallus, Danio rerio* and *Caenorhabditis elegans:* WD, WHGxxxGYxxxF (SEQ ID NO:23), YxxxxF (SEQ ID NO:24) and YxxxYFxxH (SEQ ID NO:25). Of these, the WD, WHGxxxGYxxxF and YxxxxF motifs are present in ScAle and YlAle1, but the YxxxYFxxH motif is not. Alternate non-plant motifs for Ale1 homologs are also described in U.S. Pat. Pub. No. 2008-0145867-A1; specifically, these include: M-[V/I]-[L/I]-xxK-[L/V/I]-xxxxxxDG (SEQ ID NO:26), RxKYYxxWxxx-[E/D]-[A/G]xxxxGxG-[F/Y]-xG (SEQ ID NO:27), $EX_{11}WNX_2$-[T/V]-$X_2W$ (SEQ ID NO:28) and SAxWHGxxPGYxx-[T/F]-F (SEQ ID NO:29).

Similarly, Lewin, T.W. et al. (Biochemistry, 38:5764-5771 (1999)) and Yamashita et al. (Biochim, Biophys. Acta, 1771:1202-1215 (2007)) proposed the following 1-acyl-sn-glycerol-3-phosphate acyltransferase ["LPAAT"] family motifs to be important for LPLAT activity, based on alignment of sequences from bacteria, yeast, nematodes and mammals: NHxxxxD (SEQ ID NO:19), GxxFI-[D/R]-R (SEQ ID NO:30), EGTR (SEQ ID NO:20) and either [V/I]-[P/X]-[I/V/L]-[I/V]-P-[V/I] (SEQ ID NO:31) or IVPIVM (SEQ ID NO:32). The NHxxxxD and EGTR motifs are present in MaLPAAT1, YILPAAT1 and CeLPCAT, but the other motifs are not.

Based on publicly available Ale1, LPCAT and LPAAT protein sequences, including those described herein, the instant disclosure concerns the following MBOAT family motifs: M(V/I)LxxKL (SEQ ID NO:3), RxKYYxxW (SEQ ID NO:4), SAxWHG (SEQ ID NO:5) and $EX_nWNX_2$-[T/V]-$X_2W$ (SEQ ID NO:28). Similarly, 1-acyl-*sn*-glycerol-3-phosphate acyltransferase family motifs are those set forth as: NHxxxxD (SEQ ID NO:19) and EGTR (SEQ ID NO:20).

Alternatively, publicly available LPLAT sequences or their motifs may be hybridization reagents for the identification of homologs. Hybridization methods are well known to those of ordinary skill in the art as noted above.

Any of the LPLAT nucleic acid fragments or any identified homologs may be used to isolate genes encoding homologous proteins from the same or other algal, fungal, oomycete, euglenoid, stramenopiles, yeast or plant species. Isolation of homologous genes using sequence-dependent protocols is well known in the art. Examples of sequence-dependent protocols include, but are not limited to: 1) methods of nucleic acid hybridization; 2) methods of DNA and RNA amplification, as exemplified by various uses of nucleic acid amplification technologies, such as polymerase chain reaction ["PCR"] (U.S. Patent 4,683,202); ligase chain reaction ["LCR"] (Tabor, S. et al., Proc. Natl. Acad. Sci. U.S.A., 82:1074 (1985)); or strand displacement amplification ["SDA"] (Walker, et al., Proc. Natl. Acad. Sci. U.S.A., 89:392 (1992)); and, 3) methods of library construction and screening by complementation.

For example, genes encoding proteins or polypeptides similar to publicly available LPLAT genes or their motifs could be isolated directly by using all or a portion of those publicly available nucleic acid fragments as DNA hybridization probes to screen libraries from any desired organism using well known methods. Specific oligonucleotide probes based upon the publicly available nucleic acid sequences can be designed and synthesized by methods known in the art (Maniatis, *supra*). Moreover, the entire sequences can be used directly to synthesize DNA probes by methods known to the skilled artisan, such as random primers DNA labeling, nick translation or end-labeling techniques, or RNA probes using available *in vitro* transcription systems. In addition, specific primers can be designed and used to amplify a part or the full length of the publicly available sequences or their motifs. The resulting amplification products can be labeled directly during amplification reactions or labeled after amplification reactions, and used as probes to isolate full-length DNA fragments under conditions of appropriate stringency.

Typically, in PCR-type amplification techniques, the primers have different sequences and are not complementary to each other. Depending on the desired test conditions, the sequences of the primers should be designed to provide for both efficient and faithful replication of the target nucleic acid. Methods of PCR primer design are common and well known (Thein and Wallace, "The use of oligonucleotides as specific hybridization probes in the Diagnosis of Genetic Disorders", in Human Genetic Diseases: A Practical Approach, K. E. Davis Ed., (1986) pp 33-50, IRL: Herndon, VA; Rychlik, W., In Methods in Molecular Biology, White, B. A. Ed., (1993) Vol. 15, pp 31-39, PCR Protocols: Current Methods and Applications. Humania: Totowa, NJ).

Generally two short segments of available LPLAT sequences may be used in PCR protocols to amplify longer nucleic acid fragments encoding homologous genes from DNA or RNA. PCR may also be performed on a library of cloned nucleic acid fragments wherein the sequence of one primer is derived from the available nucleic acid fragments or their motifs. The sequence of the other primer takes advantage of the presence of the polyadenylic acid tracts to the 3' end of the mRNA precursor encoding genes.

Alternatively, the second primer sequence may be based upon sequences derived from the cloning vector. For example, the skilled artisan can follow the RACE protocol (Frohman et al., Proc. Natl. Acad. Sci. U.S.A., 85:8998 (1988)) to generate cDNAs by using PCR to amplify copies of the region between a single point in the transcript and the 3' or 5' end. Primers oriented in the 3' and 5' directions can be designed from the available sequences. Using commercially available 3' RACE or 5' RACE systems (e.g., BRL, Gaithersburg, MD), specific 3' or 5' cDNA fragments can be isolated (Ohara et al., Proc. Natl. Acad. Sci. U.S.A., 86:5673 (1989); Loh et al., Science, 243:217 (1989)).

Based on any of these well-known methods just discussed, it would be possible to identify and/or isolate LPLAT gene homologs in any preferred eukaryotic organism of choice. The activity of any putative LPLAT gene can readily be confirmed by expression of the gene within a LC-PUFA-producing host organism, since the $C_{18}$ to $C_{20}$ elongation and/or Δ4 desaturation are increased relative to those within an organism lacking the LPLAT transgene (*supra*).

It has been previously hypothesized that LPCATs could be important in the accumulation of EPA in the TAG fraction of *Yarrowia lipolytica* (U.S. Pat. Pub. No. 2006-0115881-A1). As described therein, this hypothesis was based on the following studies: 1) Stymne S. and A.K. Stobart (Biochem J., 223(2):305-314(1984)), who hypothesized that the exchange between the acyl-CoA pool and PC pool may be attributed to the forward and backward reaction of LPCAT; 2) Domergue, F. et al. (J. Bio. Chem., 278:35115 (2003)), who suggested that accumulation of GLA at the sn-2 position of PC and the inability to efficiently synthesize ARA in yeast was a result of the elongation step involved in PUFA biosynthesis occurring within the acyl-CoA pool, while Δ5 and Δ6 desaturation steps occurred predominantly at the *sn*-2 position of PC; 3) Abbadi, A. et al. (The Plant Cell, 16:2734-2748 (2004)), who suggested that LPCAT plays a criticial role in the successful reconstitution of a Δ6 desaturase/Δ6 elongase pathway, based on analysis on the constraints of PUFA accumulation in transgenic oilseed plants; and, 4) Intl. App. Pub. No. WO 2004/076617 A2 (Renz, A. et al.), who provided a gene encoding LPCAT from *Caenorhabditis elegans* (T06E8.1) ["CeLPCAT"] that substantially improved the efficiency of elongation in a genetically introduced Δ6 desaturase/Δ6 elongase pathway in *S. cerevisiae* fed with exogenous fatty acid substrates suitable for Δ6 elongation. Renz et al. concluded that LPCAT allowed efficient and continuous exchange of the newly synthesized fatty acids between phospholipids and the acyl-CoA pool, since desaturases catalyze the introduction of double bonds in PC-coupled fatty acids while elongases exclusively catalyze the elongation of CoA esterified fatty acids (acyl-CoAs). However, Intl. App. Pub. No. WO 2004/076617 did not teach the effect of CeLPCAT on Δ6 elongation conversion efficiency in host cells that were not exogenously fed fatty acids, Δ5 elongation conversion efficiency, or Δ4 desaturation conversion efficiency.

Herein, it is demonstrated that LPAAT and LPCAT are indeed important in the accumulation of EPA and DHA in the TAG fraction of *Yarrowia lipolytica.* However, unexpectedly, it was found that over-expression of LPLATs can result in an improvement in the Δ9 elongase conversion efficiency and/or Δ4 desaturase conversion efficiency. As previously defined, conversion efficiency is a term that refers to the efficiency by which a particular enzyme, such as a Δ4 desaturase or Δ9 elongase, can convert substrate to product. Thus, in a strain engineered to produce EPA, improvement in Δ9 elongase conversion efficiency was demonstrated to result in increased EPA % TFAs or EPA % DCW. Similarly, improvement in Δ9 elongase and/or Δ4 desaturase conversion efficiency in a strain engineered to produce DHA was demonstrated to result in increased DHA % TFAs or DHA % DCW.

PUFA desaturations occur on phospholipids, while fatty acid elongations occur on acyl-CoAs. Based on previous studies, it was therefore expected that LPLAT over-expression would result in improved desaturations due to improved substrate availability in phospholipids, while expression of LPLATs was not expected to result in improved elongations that require improved substrate availability in the CoA pool.

Despite these assumptions, Example 5 demonstrates that LPLAT expression did not improve the conversion efficiency of all desaturations in strains of *Yarrowia* producing DHA, in a comparable manner. Specifically, the conversion efficiency of Δ4 desaturase was selectively improved, while similar improvements were not found in Δ12, Δ8, Δ5 or Δ17 desaturations. It is hypothesized that Δ4 desaturase was therefore limiting as a result of limited availability of the DPA substrate in phospholipids.

Additionally, Examples 4 and 5 demonstrate that LPLAT expression, based on at least one stably integrated polynucleotide encoding the LPLAT polypeptide, significantly improved the Δ9 elongase conversion efficiency in strains of *Yarrowia* producing EPA and DHA, respectively. Surprisingly, however, the LPLATs did not also result in a comparable improvement in the efficiency of the $C_{20/22}$ elongation of EPA to DPA in DHA strains. Generally, there was no significant change in the total lipid content in strains over-expressing LPLATs versus those that were not.

Clearly, broad generalizations are difficult concerning the effect of LPLAT over-expression in host cells producing PUFAs. Instead, the effect of LPLAT activity must be considered based on subsets of desaturases and elongases having specific activity (i.e., Δ12 desaturase, Δ8 desaturase, Δ5 desaturase, Δ17 desaturase, Δ4 desaturase, Δ9 elongase, $C_{14/16}$ elongase, $C_{16/18}$ elongase, $C_{18/20}$ elongase ["also Δ6 elongase"], $C_{20/22}$ elongase ["also Δ5 elongase"]).

On the basis of the above discussion, disclosed herein are methods for improving $C_{18}$ to $C_{20}$ elongation conversion efficiency in a LC-PUFA-producing recombinant oleaginous microbial host cell, wherein said methods comprise:

a) introducing into said LC-PUFA-producing recombinant host cell at least one isolated polynucleotide encoding a polypeptide having at least acyl-CoA:lysophospholipid acyltransferase activity wherein the polypeptide is selected from the group consisting of:

(i) a polypeptide having at least 45% amino acid identity, based on the Clustal W method of alignment, when compared to an amino acid sequence selected from the group consisting of SEQ ID NO:9 (ScAle1) and SEQ ID NO:11 (YlAle1);
(ii) a polypeptide having at least one membrane bound *O*-acyltransferase protein family motif selected from the group consisting of: M(V/I)LxxKL (SEQ ID NO:3), RxKYYxxW (SEQ ID NO:4), SAxWHG (SEQ ID NO:5) and $EX_nWNX_2$-[T/V]-$X_2W$ (SEQ ID NO:28);
(iii) a polypeptide having at least 90% amino acid identity, based on the Clustal W method of alignment, when compared to an amino acid sequence as set forth in SEQ ID NO:2 (CeLPCAT);
(iv) a polypeptide having at least 43.9% amino acid identity, based on the Clustal W method of alignment, when compared to an amino acid sequence selected from the group consisting of SEQ ID NO:15 (MaLPAAT1), SEQ ID NO:17 (YlLPAAT1) and SEQ ID NO:18 (ScLPAAT1); and,
(v) a polypeptide having at least one 1-acyl-sn-glycerol-3-phosphate acyltransferase protein family motif selected from the group consisting of: NHxxxxD (SEQ ID NO:19) and EGTR (SEQ ID NO:20);

wherein the at least one isolated polynucleotide encoding a polypeptide having at least acyl-CoA:lysophospholipid acyltransferase activity is operably linked to at least one regulatory sequence, said regulatory sequence being the same or different; and,
b) growing the oleaginous microbial host cell;

wherein the $C_{18}$ to $C_{20}$ elongation conversion efficiency of the oleaginous microbial host cell is increased relative to the control host cell.

The increase in $C_{18}$ to $C_{20}$ elongation conversion efficiency may be at least 4% in at least one LC-PUFA-producing oleaginous microbial host cell, based on at least one stably integrated polynucleotide encoding the LPLAT polypeptide, when compared to the control host cell, for instance the increase in $C_{18}$ to $C_{20}$ elongation conversion efficiency may be greater than 4%, e.g. an increase of at least about 4-10%, 10-20%, 20-40%, or 40-60% or greater.

For example, in one method demonstrated herein, the increase in $C_{18}$ to $C_{20}$ elongation conversion efficiency is at least 13% in an EPA-producing host cell when compared to the control host cell and the increase in $C_{18}$ to $C_{20}$ elongation conversion efficiency is at least 4% in a DHA-producing host cell when compared to the control host cell.

Similarly, methods are also described herein for increasing Δ4 desaturation conversion efficiency in a LC-PUFA-producing oleaginous microbial recombinant host cell, wherein said methods comprise:

a) introducing into said LC-PUFA-producing recombinant host cell at least one isolated polynucleotide encoding a polypeptide having at least acyl-CoA:lysophospholipid acyltransferase activity wherein the polypeptide is selected from the group consisting of:

(i) a polypeptide having at least 45% amino acid identity, based on the Clustal W method of alignment, when compared to an amino acid sequence selected from the group consisting of SEQ ID NO:9 (ScAle1) and SEQ ID NO:11 (YlAle1);
(ii) a polypeptide having at least one membrane bound *O*-acyltransferase protein family motif selected from the group consisting of: M(V/I)LxxKL (SEQ ID NO:3), RxKYYxxW (SEQ ID NO:4), SAxWHG (SEQ ID NO:5) and $EX_nWNX_2$-[T/V]-$X_2W$ (SEQ ID NO:28);
(iii) a polypeptide having at least 90% amino acid identity, based on the Clustal W method of alignment, when compared to an amino acid sequence as set forth in SEQ ID NO:2 (CeLPCAT);
(iv) a polypeptide having at least 43.9% amino acid identity, based on the Clustal W method of alignment, when compared to an amino acid sequence selected from the group consisting of SEQ ID NO:15 (MaLPAAT1), SEQ ID NO:17 (YILPAAT1) and SEQ ID NO:18 (ScLPAAT1); and,
(v) a polypeptide having at least one 1-acyl-sn-glycerol-3-phosphate acyltransferase protein family motif selected from the group consisting of: NHxxxxD (SEQ ID NO:19) and EGTR (SEQ ID NO:20);

wherein the at least one isolated polynucleotide encoding a polypeptide having at least acyl-CoA:lysophospholipid acyltransferase activity is operably linked to at least one regulatory sequence, said regulatory sequence being the same or different; and,
b) growing the oleaginous microbial host cell;

wherein the Δ4 desaturation conversion efficiency of the oleaginous microbial host cell is increased relative to the control host cell.

The increase in Δ4 desaturation conversion efficiency may be at least 5% in at least one LC-PUFA-producing oleaginous microbial host cell, based on at least one stably integrated polynucleotide encoding the LPLAT polypeptide, when compared to the control host cell, for instance the increase in Δ4 desaturation conversion efficiency may be greater than 5%, e.g. an increase of at least about 5-10%, 10-20%, 20-40%, or 40-60% or greater.

For example, in one method demonstrated herein, the increase in Δ4 desaturation conversion efficiency in a DHA-producing host was at least 18% when compared to the control host cell.

Recombinant host cells are also described herein, and form a first embodiment of the invention. Specifically, these recombinant host cells are recombinant oleaginous eicosapentaenoic acid (EPA)-producing *Yarrowia lipolytica* host cells which comprise at least one transgene comprising a polynucleotide encoding a polypeptide having at least acyl-CoA:lysophospholipid acyltransferase activity, wherein the polypeptide has at least 95% amino acid identity, based on the Clustal W method of alignment, when compared to the amino acid sequence of SEQ ID NO:11 (YlAle1);
wherein the transgene comprises the polynucleotide operably linked to at least one regulatory sequence, wherein said at least one regulatory sequence includes a promoter which is a constitutive promoter or which is a regulatable promoter having an inducible activity which allows induced expression; and,
wherein the polypeptide is over-expressed when compared to a control host cell.

In preferred recombinant host cells, the polynucleotide encoding the polypeptide having at least acyl-CoA:lysophospholipid acyltransferase activity is stably integrated. Further, the recombinant host cell may have an increase in $C_{18}$ to $C_{20}$ elongation conversion efficiency of at least 4% when compared to a control host cell.

More preferred recombinant host cells, having at least one stably integrated polynucleotide encoding the LPLAT polypeptide, have at least one improvement selected from the group consisting of:

a) an increase in $C_{18}$ to $C_{20}$ elongation conversion efficiency of at least 13% when compared to a control host cell; or
b) an increase of at least 9% EPA of TFAs when compared to a control host cell.

Based on the above improvements, one of skill in the art will appreciate the value of expressing a LPLAT in a recombinant host cell that is producing long-chain PUFAs, such EDA, DGLA, ARA, DTA, DPAn-6, ETrA, ETA, EPA, DPA and DHA, if it is desirable to optimize the production of these fatty acids.

Standard resource materials that are useful to make recombinant constructs describe, *inter alia*: 1) specific conditions and procedures for construction, manipulation and isolation of macromolecules, such as DNA molecules, plasmids, etc.; 2) generation of recombinant DNA fragments and recombinant expression constructs; and, 3) screening and isolation of clones. See, Sambrook, J., Fritsch, E. F. and Maniatis, T., Molecular Cloning: A Laboratory Manual, 2nd ed., Cold Spring Harbor Laboratory: Cold Spring Harbor, NY (1989) (hereinafter "Maniatis"); by Silhavy, T. J., Bennan, M. L. and Enquist, L. W., Experiments with Gene Fusions, Cold Spring Harbor Laboratory: Cold Spring Harbor, NY (1984); and by Ausubel, F. M. et al., Current Protocols in Molecular Biology, published by Greene Publishing Assoc. and Wiley-Interscience, Hoboken, NJ (1987).

In general, the choice of sequences included in the construct depends on the desired expression products, the nature of the host cell and the proposed means of separating transformed cells versus non-transformed cells. The skilled artisan is aware of the genetic elements that must be present on the plasmid vector to successfully transform, select and propagate host cells containing the chimeric gene. Typically, however, the vector or cassette contains sequences directing transcription and translation of the relevant gene(s), a selectable marker and sequences allowing autonomous replication or chromosomal integration. Suitable vectors comprise a region 5' of the gene that controls transcriptional initiation, i.e., a promoter, the gene coding sequence, and a region 3' of the DNA fragment that controls transcriptional termination, i.e., a terminator. It is most preferred when both control regions are derived from genes from the transformed host cell, although they need not be derived from genes native to the production host.

Transcription initiation regions or promoters useful for driving expression of heterologous genes or portions of them in the desired host cell are numerous and well known. These control regions may comprise a promoter, enhancer, silencer, intron sequences, 3' UTR and/or 5' UTR regions, and protein and/or RNA stabilizing elements. Such elements may vary in their strength and specificity. Virtually any promoter, i.e., native, synthetic, or chimeric, capable of directing expression of these genes in the selected host cell is suitable, although transcriptional and translational regions from the host species are particularly useful. Expression in a host cell can occur in an induced or constitutive fashion. Induced expression occurs by inducing the activity of a regulatable promoter operably linked to the LPLAT gene of interest, while constitutive expression occurs by the use of a constituitive promoter.

3' non-coding sequences encoding transcription termination regions may be provided in a recombinant construct and may be from the 3' region of the gene from which the initiation region was obtained or from a different gene. A large number of termination regions are known and function satisfactorily in a variety of hosts when utilized in both the same and different genera and species from which they were derived. Termination regions may also be derived from various genes native to the preferred hosts. The termination region is usually selected more for convenience rather than for any particular property.

Particularly useful termination regions for use in yeast are derived from a yeast gene, particularly *Saccharomyces, Schizosaccharomyces, Candida, Yarrowia* or *Kluyveromyces*. The 3'-regions of mammalian genes encoding $\gamma$-interferon and $\alpha$-2 interferon are also known to function in yeast. The 3'-region can also be synthetic, as one of skill in the art can utilize available information to design and synthesize a 3'-region sequence that functions as a transcription terminator. A termination region may be unnecessary, but is highly preferred.

The vector may also comprise a selectable and/or scorable marker, in addition to the regulatory elements described above. Preferably, the marker gene is an antibiotic resistance gene such that treating cells with the antibiotic results in growth inhibition, or death, of untransformed cells and uninhibited growth of transformed cells. For selection of yeast transformants, any marker that functions in yeast is useful with resistance to kanamycin, hygromycin and the amino glycoside G418 and the ability to grow on media lacking uracil, lysine, histine or leucine being particularly useful.

Merely inserting a gene (e.g., encoding a LPLAT) into a cloning vector does not ensure its expression at the desired rate, concentration, amount, etc. In response to the need for a high expression rate, many specialized expression vectors have been created by manipulating a number of different genetic elements that control transcription, RNA stability, translation, protein stability and location, oxygen limitation, and secretion from the host cell. Some of the manipulated features include: the nature of the relevant transcriptional promoter and terminator sequences, the number of copies of the cloned gene and whether the gene is plasmid-borne or integrated into the genome of the host cell, the final cellular location of the synthesized protein, the efficiency of translation and correct folding of the protein in the host organism, the intrinsic stability of the mRNA and protein of the cloned gene within the host cell and the codon usage within the cloned gene, such that its frequency approaches the frequency of preferred codon usage of the host cell. Each of these may be used in the methods and host cells described herein to further optimize expression of LPLAT genes.

For example, LPLAT expression can be increased at the transcriptional level through the use of a stronger promoter (either regulated or constitutive) to cause increased expression, by removing/deleting destabilizing sequences from either the mRNA or the encoded protein, or by adding stabilizing sequences to the mRNA (U.S. Patent 4,910,141). Alternatively, additional copies of the LPLAT genes may be introduced into the recombinant host cells to thereby increase EPA and/or DHA production and accumulation, either by cloning additional copies of genes within a single expression construct or by introducing additional copies into the host cell by increasing the plasmid copy number or by multiple integration of the cloned gene into the genome.

After a recombinant construct is created comprising at least one chimeric gene comprising a promoter, a LPLAT open reading frame ["ORF"] and a terminator, it is placed in a plasmid vector capable of autonomous replication in the host cell or is directly integrated into the genome of the host cell. Integration of expression cassettes can occur randomly within the host genome or can be targeted through the use of constructs containing regions of homology with the host genome sufficient to target recombination with the host locus. Where constructs are targeted to an endogenous locus, all or some of the transcriptional and translational regulatory regions can be provided by the endogenous locus.

When two or more genes are expressed from separate replicating vectors, each vector may have a different means of selection and should lack homology to the other construct(s) to maintain stable expression and prevent reassortment of elements among constructs. Judicious choice of regulatory regions, selection means and method of propagation of the introduced construct(s) can be experimentally determined so that all introduced genes are expressed at the necessary levels to provide for synthesis of the desired products.

Constructs comprising the gene(s) of interest may be introduced into a host cell by any standard technique. These techniques include transformation, e.g., lithium acetate transformation (Methods in Enzymology, 194:186-187 (1991)), biolistic impact, electroporation, microinjection, vacuum filtration or any other method that introduces the gene of interest into the host cell.

For convenience, a host cell that has been manipulated by any method to take up a DNA sequence, for example, in an expression cassette, is referred to herein as "transformed" or "recombinant" or "transformant". The transformed host will have at least one copy of the expression construct and may have two or more, depending upon whether the gene is integrated into the genome, amplified, or is present on an extrachromosomal element having multiple copy numbers.

The transformed host cell can be identified by selection for a marker contained on the introduced construct. Alternatively, a separate marker construct may be co-transformed with the desired construct, as many transformation techniques introduce many DNA molecules into host cells.

Typically, transformed hosts are selected for their ability to grow on selective media, which may incorporate an antibiotic or lack a factor necessary for growth of the untransformed host, such as a nutrient or growth factor. An introduced marker gene may confer antibiotic resistance, or encode an essential growth factor or enzyme, thereby permitting growth on selective media when expressed in the transformed host. Selection of a transformed host can also occur when the expressed marker protein can be detected, either directly or indirectly. Additional selection techniques are described in U.S. Patent 7,238,482 and U.S. Patent 7,259,255.

Regardless of the selected host or expression construct, multiple transformants must be screened to obtain a strain displaying the desired expression level and pattern. For example, Juretzek et al. (Yeast, 18:97-113 (2001)) note that the stability of an integrated DNA fragment in *Yarrowia lipolytica* is dependent on the individual transformants, the recipient strain and the targeting platform used. Such screening may be accomplished by Southern analysis of DNA blots (Southern, J. Mol. Biol., 98:503 (1975)), Northern analysis of mRNA expression (Kroczek, J. Chromatogr. Biomed. Appl., 618(1-2):133-145 (1993)), Western analysis of protein expression, phenotypic analysis or GC analysis of the PUFA products.

The metabolic process wherein oleic acid is converted to LC-PUFAs involves elongation of the carbon chain through the addition of carbon atoms and desaturation of the molecule through the addition of double bonds. This requires a series of special desaturation and elongation enzymes present in the endoplasmic reticulum membrane. However, as seen in FIG. 1 and as described below, multiple alternative pathways exist for LC-PUFA production.

Specifically, FIG. 1 depicts the pathways described below. All pathways require the initial conversion of oleic acid to linoleic acid ["LA"], the first of the ω-6 fatty acids, by a Δ12 desaturase. Then, using the "Δ9 elongase/ Δ8 desaturase pathway" and LA as substrate, long-chain ω-6 fatty acids are formed as follows: 1) LA is converted to eicosadienoic acid ["EDA"] by a Δ9 elongase; 2) EDA is converted to dihomo-γ-linolenic acid ["DGLA"] by a Δ8 desaturase; 3) DGLA is converted to arachidonic acid ["ARA"] by a Δ5 desaturase; 4) ARA is converted to docosatetraenoic acid ["DTA"] by a $C_{20/22}$ elongase; and, 5) DTA is converted to docosapentaenoic acid ["DPAn-6"] by a Δ4 desaturase.

The "Δ9 elongase/ Δ8 desaturase pathway" can also use α-linolenic acid ["ALA"] as substrate to produce long-chain ω-3 fatty acids as follows: 1) LA is converted to ALA, the first of the ω-3 fatty acids, by a Δ15 desaturase; 2) ALA is converted to eicosatrienoic acid ["ETrA"] by a Δ9 elongase; 3) ETrA is converted to eicosatetraenoic acid ["ETA"] by a Δ8 desaturase; 4) ETA is converted to eicosapentaenoic acid ["EPA"] by a Δ5 desaturase; 5) EPA is converted to docosapentaenoic acid ["DPA"] by a $C_{20/22}$ elongase; and, 6) DPA is converted to docosahexaenoic acid ["DHA"] by a Δ4 desaturase. Optionally, ω-6 fatty acids may be converted to ω-3 fatty acids. For example, ETA and EPA are produced from DGLA and ARA, respectively, by Δ17 desaturase activity. Advantageously for the purposes herein, the Δ9 elongase/ Δ8 desaturase pathway enables production of an EPA oil that lacks significant amounts of γ-linolenic acid ["GLA"].

Alternative pathways for the biosynthesis of ω-3/ω-6 fatty acids utilize a Δ6 desaturase and $C_{18/20}$ elongase, that is, the "Δ6 desaturase/ Δ6 elongase pathway". More specifically, LA and ALA may be converted to to GLA and stearidonic acid ["STA"], respectively, by a Δ6 desaturase; then, a $C_{18/20}$ elongase converts GLA to DGLA and/or STA to ETA.

A LC-PUFA-producing recombinant host cell will possess at least one of the biosynthetic pathways described above, whether this pathway is native to the host cell or is genetically engineered. The host cell may be capable of producing at least about 2-5% LC-PUFAs in the total lipids of the recombinant host cell, for instance at least about 5-15%, 15-35%, 35-50%, 50-65%or 65-75% LC-PUFAs in the total lipids. The structural form of the LC-PUFAs is not limiting; thus, for example, the EPA or DHA may exist in the total lipids as free fatty acids or in esterified forms such as acylglycerols, phospholipids, sulfolipids or glycolipids.

A variety of eukaryotic microbial organisms, including bacteria, yeast, algae, stramenopile, oomycete, euglenoid and/or fungus, can produce (or can be engineered to produce) LC-PUFAs. These may include hosts that grow on a variety of feedstocks, including simple or complex carbohydrates, fatty acids, organic acids, oils, glycerols and alcohols, and/or hydrocarbons over a wide range of temperature and pH values. The host cell of the invention is a *Yarrowia lipolytica* host cell.

The microbial hosts may be oleaginous organisms. In particular, the host cell of the invention is an oleaginous organism. Oleaginous organisms are naturally capable of oil synthesis and accumulation, wherein the total oil content can comprise greater than about 25% of the dry cell weight, for instance greater than about 30% or 40% of the dry cell weight. Various bacteria, algae, euglenoids, moss, fungi, yeast and stramenopiles are naturally classified as oleaginous. Within this broad group of hosts, of particular interest are those organisms that naturally produce ω-3/ω-6 fatty acids. For example, ARA, EPA and/or DHA is produced via *Cyclotella* sp., *Crypthecodinium* sp., *Mortierella* sp., *Nitzschia* sp., *Pythium, Thraustochytrium* sp. and *Schizochytrium* sp. Thus, for example, transformation of *Mortierella alpina,* which is commercially used for production of ARA, with any of the present LPLAT genes under the control of inducible or regulated promoters could yield a transformant organism capable of synthesizing increased quantities of ARA. The method of transformation of *M. alpina* is described by Mackenzie et al. (Appl. Environ. Microbiol., 66:4655 (2000)). Similarly, methods for transformation of Thraustochytriales microorganisms (e.g., *Thraustochytrium, Schizochytrium*) are disclosed in U.S. Patent 7,001,772. A non-oleaginous organism can be genetically modified to become oleaginous, e.g., yeast such as *Saccharomyces cerevisiae* (U.S. Pat. Pub. No. 2007/0015237-A1).

The microbial host cells may be oleaginous yeast. Genera typically identified as oleaginous yeast include, but are not limited to: *Yarrowia, Candida, Rhodotorula, Rhodosporidium, Cryptococcus, Trichosporon* and *Lipomyces.* More specifically, illustrative oil-synthesizing yeast include: *Rhodosporidium toruloides, Lipomyces starkeyii, L. lipoferus, Candida revkaufi, C. pulcherrima, C. tropicalis, C. utilis, Trichosporon pullans, T. cutaneum, Rhodotorula glutinus, R. graminis* and *Yarrowia lipolytica* (formerly classified as *Candida lipolytica*). The host cell of the invention is the oleaginous yeast *Yarrowia lipolytica*: most preferred are the *Y. lipolytica* strains designated as ATCC #76982, ATCC #20362, ATCC #8862, ATCC #18944 and/or LGAM S(7)1 (Papanikolaou S., and Aggelis G., Bioresour. Technol., 82(1):43-9 (2002)).

Specific teachings applicable for engineering ARA, EPA and DHA production in *Y. lipolytica* are provided in U.S. Pat. Pub. No. 2006-0094092-A1, U.S. Pat. Pub. No. 2006-0115881-A1, U.S. Pat. Pub. No. 2009-0093543-A1 and U.S. Pat. Pub. No. 2006-0110806-A1, respectively. These references also describe the preferred method of expressing genes in *Yarrowia lipolytica* by integration of a linear DNA fragment into the genome of the host, preferred promoters, termination regions, integration loci and disruptions, and preferred selection methods when using this particular host species.

One of skill in the art would be able to use the cited teachings in U.S. Pat. Pub. No. 2006-0094092-A1, U.S. Pat. Pub. No. 2006-0115881-A1, U.S. Pat. Pub. No. 2009-0093543-A1 and U.S. Pat. Pub. No. 2006-0110806-A1 to recombinantly engineer other host cells for PUFA production.

The transformed recombinant host cell is grown under conditions that optimize expression of chimeric genes (e.g., encoding desaturases, elongases, LPLATs, etc.) and produce the greatest and the most economical yield of LC-PUFA(s). In general, media conditions may be optimized by modifying the type and amount of carbon source, the type and amount of nitrogen source, the carbon-to-nitrogen ratio, the amount of different mineral ions, the oxygen level, growth temperature, pH, length of the biomass production phase, length of the oil accumulation phase and the time and method of cell harvest.

*Yarrowia lipolytica* are generally grown in a complex media such as yeast extract-peptone-dextrose broth ["YPD"] or a defined minimal media that lacks a component necessary for growth and thereby forces selection of the desired expression cassettes (e.g., Yeast Nitrogen Base (DIFCO Laboratories, Detroit, MI)).

Fermentation media for the methods and host cells described herein must contain a suitable carbon source, such as are taught in U.S. Patent 7,238,482 and U.S. Pat. Appl. No. 12/641,929 (filed December 19, 2009). Although it is contemplated that the source of carbon utilized may encompass a wide variety of carbon-containing sources, preferred carbon sources are sugars, glycerol and/or fatty acids. Most preferred is glucose, sucrose, invert sucrose, fructose and/or fatty acids containing between 10-22 carbons. For example, the fermentable carbon source can be selected from the group consisting of invert sucrose, glucose, fructose and combinations of these, provided that glucose is used in combination with invert sucrose and/or fructose.

The term "invert sucrose", also referred to herein as "invert sugar", refers to a mixture comprising equal parts of fructose and glucose resulting from the hydrolysis of sucrose. Invert sucrose may be a mixture comprising 25 to 50% glucose and 25 to 50% fructose. Invert sucrose may also comprise sucrose, the amount of which depends on the degree of hydrolysis.

Nitrogen may be supplied from an inorganic (e.g., $(NH_4)_2SO_4$) or organic (e.g., urea or glutamate) source. In addition to appropriate carbon and nitrogen sources, the fermentation media must also contain suitable minerals, salts, cofactors, buffers, vitamins and other components known to those skilled in the art suitable for the growth of the high EPA- and/or DHA-producing host cells and the promotion of the enzymatic pathways for EPA and/or DHA production. Particular attention is given to several metal ions, such as $Fe^{+2}$, $Cu^{+2}$, $Mn^{+2}$, $Co^{+2}$, $Zn^{+2}$ and $Mg^{+2}$, that promote synthesis of lipids and PUFAs (Nakahara, T. et al., Ind. Appl. Single Cell Oils, D. J. Kyle and R. Colin, eds. pp 61-97 (1992)).

Preferred growth media for the methods and host cells described herein are common commercially prepared media, such as Yeast Nitrogen Base (DIFCO Laboratories, Detroit, MI). Other defined or synthetic growth media may also be used and the appropriate medium for growth of *Yarrowia lipolytica* will be known by one skilled in the art of microbiology or fermentation science. A suitable pH range for the fermentation is typically between about pH 4.0 to pH 8.0, wherein pH 5.5 to pH 7.5 is preferred as the range for the initial growth conditions. The fermentation may be conducted under aerobic or anaerobic conditions, wherein microaerobic conditions are preferred.

Typically, accumulation of high levels of PUFAs in oleaginous yeast cells requires a two-stage process, since the metabolic state must be "balanced" between growth and synthesis/storage of fats. Thus, most preferably, a two-stage fermentation process is necessary for the production of EPA and/or DHA in *Yarrowia lipolytica.* This approach is described in U.S. Patent 7,238,482, as are various suitable fermentation process designs (i.e., batch, fed-batch and continuous) and considerations during growth.

In some aspects, the primary product is oleaginous microbial biomass. As such, isolation and purification of the LC-PUFA-containing oils from the biomass may not be necessary (i.e., wherein the whole cell biomass is the product).

However, certain end uses and/or product forms may require partial and/or complete isolation/purification of the LC-PUFA-containing oil from the biomass, to result in partially purified biomass, purified oil, and/or purified LC-PUFAs. Fatty acids, including PUFAs, may be found in the host microorganisms as free fatty acids or in esterified forms such as acylglycerols, phospholipids, sulfolipids or glycolipids. These fatty acids may be extracted from the host cells through a variety of means well-known in the art. One review of extraction techniques, quality analysis and acceptability standards for yeast lipids is that of Z. Jacobs (Critical Reviews in Biotechnology, 12(5/6):463-491 (1992)). A brief review of downstream processing is also available by A. Singh and O. Ward (Adv. Appl. Microbiol., 45:271-312 (1997)).

In general, means for the purification of fatty acids (including LC-PUFAs) may include extraction (e.g., U.S. Patent 6,797,303 and U.S. Patent 5,648,564) with organic solvents, sonication, supercritical fluid extraction (e.g., using carbon dioxide), saponification and physical means such as presses, or combinations thereof. See U.S. Patent 7,238,482.

Many food and feed products incorporate ω-3 and/or ω-6 fatty acids, particularly ALA, GLA, ARA, EPA, DPA and DHA. It is contemplated that oleaginous yeast biomass comprising LC-PUFAs, partially purified biomass comprising LC-PUFAs, purified oil comprising LC-PUFAs, and/or purified LC-PUFAs made by the methods and host cells described herein impart the health benefits, upon ingestion of foods or feed improved by their addition. These oils can be added to food analogs, drinks, meat products, cereal products, baked foods, snack foods and dairy products, to name a few. See U.S. Pat. Appl. Pub. No. 2006-0094092.

These compositions may impart health benefits by being added to medical foods including medical nutritionals, dietary supplements, infant formula and pharmaceuticals. The skilled artisan will appreciate the amount of the oils to be added to food, feed, dietary supplements, nutriceuticals, pharmaceuticals, and other ingestible products as to impart health benefits. Health benefits from ingestion of these oils are described in the art, known to the skilled artisan and continuously investigated. Such an amount is referred to herein as an "effective" amount and depends on, among other things, the nature of the ingested products containing these oils and the physical conditions they are intended to address.

## EXAMPLES

The present invention is further described in the following non-limiting Examples.

## GENERAL METHODS

Standard recombinant DNA and molecular cloning techniques used in the Examples are well known in the art and are described by: 1) Sambrook, J., Fritsch, E. F. and Maniatis, T. Molecular Cloning: A Laboratory Manual; Cold Spring Harbor Laboratory: Cold Spring Harbor, NY (1989) (Maniatis); 2) T. J. Silhavy, M. L. Bennan, and L. W. Enquist, Experiments with Gene Fusions; Cold Spring Harbor Laboratory: Cold Spring Harbor, NY (1984); and, 3) Ausubel, F. M. et al., Current Protocols in Molecular Biology, published by Greene Publishing Assoc. and Wiley-Interscience, Hoboken, NJ (1987).

Materials and methods suitable for the maintenance and growth of microbial cultures are well known in the art. Techniques suitable for use in the following examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, Eds), American Society for Microbiology: Washington, D.C. (1994)); or by Thomas D. Brock in Biotechnology: A Textbook of Industrial Microbiology, 2nd ed., Sinauer Associates: Sunderland, MA (1989). All reagents, restriction enzymes and materials used for the growth and maintenance of microbial cells were obtained from Aldrich Chemicals (Milwaukee, WI), DIFCO Laboratories (Detroit, MI), New England Biolabs, Inc. (Beverly, MA), GIBCO/BRL (Gaithersburg, MD), or Sigma Chemical Company (St. Louis, MO), unless otherwise specified. *E. coli* strains were typically grown at 37 °C on Luria Bertani ["LB"] plates.

General molecular cloning was performed according to standard methods (Sambrook et al., *supra*). DNA sequence was generated on an ABI Automatic sequencer using dye terminator technology (U.S. Patent 5,366,860; EP 272,007) using a combination of vector and insert-specific primers. Sequence editing was performed in Sequencher (Gene Codes Corporation, Ann Arbor, MI). All sequences represent coverage at least two times in both directions.

The meaning of abbreviations is as follows: "sec" means second(s), "min" means minute(s), "h" means hour(s), "d" means day(s), "μL" means microliter(s), "mL" means milliliter(s), "L" means liter(s), "μM" means micromolar, "mM" means millimolar, "M" means molar, "mmol" means millimole(s), "μmole" mean micromole(s), "g" means gram(s), "μg"

means microgram(s), "ng" means nanogram(s), "U" means unit(s), "bp" means base pair(s), "kB" means kilobase(s), "DCW" means dry cell weight, and "TFAs" means total fatty acids.

**Nomenclature For Expression Cassettes**

The structure of an expression cassette will be represented by a simple notation system of "X::Y::Z", wherein X describes the promoter fragment, Y describes the gene fragment, and Z describes the terminator fragment, which are all operably linked to one another.

**Transformation And Cultivation Of *Yarrowia lipolytica***

*Yarrowia lipolytica* strain ATCC #20362 was purchased from the American Type Culture Collection (Rockville, MD). *Yarrowia lipolytica* strains were routinely grown at 28-30 °C in several media (e.g., YPD agar medium, Basic Minimal Media ["MM"], Minimal Media + Uracil ["MMU"], Minimal Media + Leucine + Lysine ["MMLeuLys"], Minimal Media + 5-Fluoroorotic Acid ["MM + 5-FOA"], High Glucose Media ["HGM"] and Fermentation medium ["FM"]), as described in U.S. Pat. Appl. Pub. No. 2009-0093543-A1.
Transformation of *Y. lipolytica* was performed as described in U.S. Pat. Appl. Pub. No. 2009-0093543-A1.

**Fatty Acid Analysis Of *Yarrowia lipolytica***

For fatty acid ["FA"] analysis, cells were collected by centrifugation and lipids were extracted as described in Bligh, E. G. & Dyer, W. J. (Can. J. Biochem. Physiol., 37:911-917 (1959)). Fatty acid methyl esters ["FAMEs"] were prepared by transesterification of the lipid extract with sodium methoxide (Roughan, G., and Nishida I., Arch Biochem Biophys., 276(1):38-46 (1990)) and subsequently analyzed with a Hewlett-Packard 6890 GC fitted with a 30-m X 0.25 mm (i.d.) HP-INNOWAX (Hewlett-Packard) column. The ven temperature was from 170 °C (25 min hold) to 185 °C at 3.5 °C/min.
For direct base transesterification, *Yarrowia* cells (0.5 mL culture) were harvested, washed once in distilled water, and dried under vacuum in a Speed-Vac for 5-10 min. Sodium methoxide (100 μl of 1%) and a known amount of C15:0 triacylglycerol (C15:0 TAG; Cat. No. T-145, Nu-Check Prep, Elysian, MN) was added to the sample, and then the sample was vortexed and rocked for 30 min at 50 °C. After adding 3 drops of 1 M NaCl and 400 μl hexane, the sample was vortexed and spun. The upper layer was removed and analyzed by GC.
FAME peaks recorded via GC analysis were identified by their retention times, when compared to that of known fatty acids, and quantitated by comparing the FAME peak areas with that of the internal standard (C15:0 TAG) of known amount. Thus, the approximate amount (μg) of any fatty acid FAME ["μg FAME"] is calculated according to the formula: (area of the FAME peak for the specified fatty acid/ area of the standard FAME peak) * (μg of the standard C15:0 TAG), while the amount (μg) of any fatty acid ["μg FA"] is calculated according to the formula: (area of the FAME peak for the specified fatty acid/area of the standard FAME peak) * (μg of the standard C15:0 TAG) * 0.9503, since 1 μg of C15:0 TAG is equal to 0.9503 μg fatty acids. Note that the 0.9503 conversion factor is an approximation of the value determined for most fatty acids, which range between 0.95 and 0.96.
The lipid profile, summarizing the amount of each individual fatty acid as a weight percent of TFAs, was determined by dividing the individual FAME peak area by the sum of all FAME peak areas and multiplying by 100.
For quantitating the amount of an individual fatty acid or the total fatty acids as a weight percent of the dry cell weight ["% DCW"], cells from 10 mL of the culture were collected by centrifugation, washed once with 10 mL water and collected by centrifugation again. Cells were resuspended in 1-2 mL water, poured into a pre-weighed aluminium weighing pan, and rinsed with 1-2 mL water that was also added to the same weighing pan. The pan was placed under vacuum at 80 °C overnight. The pan was weighed and the DCW calculated by subtracting the weight of the empty pan. Determination of the fatty acid as a % DCW can then be calculated based on either μg FAME or μg FA as a fraction of the μg DCW (for example, FAME % DCW was calculated as μg FAME/μg DCW*100).

EXAMPLE 1

Generation Of *Yarrowia lipolytica* Strain Y8406 To Produce About 51% EPA Of Total Fatty Acids

The present Example describes the construction of strain Y8406, derived from *Yarrowia lipolytica* ATCC #20362, capable of producing about 51% EPA relative to the total lipids via expression of a Δ9 elongase/Δ8 desaturase pathway. This strain was used as the EPA-producing host cell in Example 4.
The development of strain Y8406 (FIG. 2) required the construction of strains Y2224, Y4001, Y4001U, Y4036, Y4036U, L135, L135U9, Y8002, Y8006U6, Y8069, Y8069U, Y8154, Y8154U, Y8269 and Y8269U.

Generation Of Y4036U Strain

Briefly, strain Y8406 was derived from *Yarrowia lipolytica* ATCC #20362 via construction of strain Y2224 (a FOA resistant mutant from an autonomous mutation of the *Ura3* gene of wildtype *Yarrowia* strain ATCC #20362), strain Y4001 (producing 17% EDA with a *Leu-* phenotype), strain Y4001U1 (*Leu-* and *Ura-*), strain Y4036 (producing 18% DGLA with a *Leu*-phenotype) and strain Y4036U (*Leu-* and *Ura-*). Further details regarding the construction of strains Y2224, Y4001, Y4001U, Y4036 and Y4036U are described in the General Methods of U.S. Pat. App. Pub. No. 2008-0254191.

The final genotype of strain Y4036U with respect to wild type *Yarrowia lipolytica* ATCC #20362 was *Ura3-,* YAT1::ME3S::Pex16, EXP1::EgD9eS::Lip1, FBAINm::EgD9eS::Lip2, GPAT::EgD9e::Lip2, FBAINm::EgD8M::Pex20, EXP1::EgD8M::Pex16, GPD::FmD12::Pex20, YAT1::FmD12::OCT (wherein FmD12 is a *Fusarium moniliforme* Δ12 desaturase gene [U.S. Patent 7,504,259]; ME3S is a codon-optimized $C_{16/18}$ elongase gene, derived from *Mortierella alpina* [U.S. Patent 7,470,532]; EgD9e is a *Euglena gracilis* Δ9 elongase gene [U.S. Patent 7,645,604]; EgD9eS is a codon-optimized Δ9 elongase gene, derived from *Euglena gracilis* [U.S. Patent 7,645,604]; EgD8M is a synthetic mutant Δ8 desaturase [U.S. Patent 7,709,239], derived from *Euglena gracilis* [U.S. Patent 7,256,033]).

Generation Of L135 Strain (*Ura3+, Leu-,* Δpex3) With Chromosomal Deletion Of Pex3

Construction of strain L135 is described in Example 12 of Intl. App. Pub. No. WO 2009/046248. Briefly, construct pY157 was used to knock out the chromosomal gene encoding the peroxisome biogenesis factor 3 protein [peroxisomal assembly protein Peroxin 3 or "Pex3p"] in strain Y4036U, thereby producing strain L135 (also referred to as strain Y4036 (Δpex3)). Knockout of the chromosomal Pex3 gene in strain L135, as compared to in strain Y4036 (whose native Pex3p had not been knocked out) resulted in the following: higher lipid content (TFAs % DCW) (ca. 6.0% versus 4.7%), higher DGLA % TFAs (46% versus 19%), higher DGLA % DCW (ca. 2.8% versus 0.9%) and reduced LA % TFAs (12% versus 30%). Additionally, the Δ9 elongase percent conversion efficiency was increased from ca. 48% in strain Y4036 to 83% in strain L135.

The final genotype of strain L135 with respect to wildtype *Yarrowia lipolytica* ATCC #20362 was *Ura3+*, *Leu-, Pex3-, unknown1-,* YAT1::ME3S::Pex16, EXP1::EgD9eS::Lip1, FBAINm::EgD9eS::Lip2, GPAT::EgD9e::Lip2, FBAINm::EgD8M::Pex20, EXP1::EgD8M::Pex16, GPD::FmD12::Pex20, YAT1::FmD12::OCT.

Generation of L135U9 (*Leu-, Ura3-*) Strain

Strain L135U was created *via* temporary expression of the *Cre* recombinase enzyme in plasmid pY116 (FIG. 3; SEQ ID NO:33; described in Example 7 of Intl. App. Pub. No. WO 2008/073367) within strain L135 to produce a *Leu-* and *Ura-* phenotype. Plasmid pY116 was used for transformation of freshly grown L135 cells according to the General Methods. The transformant cells were plated onto MMLeuUra plates and maintained at 30 °C for 3 to 4 days. Three colonies were picked, inoculated into 3 mL liquid YPD media at 30 °C and shaken at 250 rpm/min for 1 day. The cultures were diluted to 1:50,000 with liquid MMLeuUra media, and 100 μL was plated onto new YPD plates and maintained at 30 °C for 2 days. Eight colonies were picked from each of three plates (24 colonies total) and streaked onto MMLeu and MMLeuUra selection plates. The colonies that could grow on MMLeuUra plates but not on MMLeu plates were selected and analyzed by GC to confirm the presence of C20:2 (EDA). One strain, having a *Leu-* and *Ura-* phenotype, was designated as L135U9.

Generation Of Y8002 Strain To Produce About 32% ARA Of TFAs

Construct pZKSL-5S5A5 (FIG. 4A; SEQ ID NO:34) was generated to integrate three Δ5 desaturase genes into the *Lys* loci of strain L135U9, to thereby enable production of ARA. The pZKSL-5S5A5 plasmid contained the following components:

Table 4: Description of Plasmid pZKSL-5S5A5 (SEQ ID NO:34)

| RE Sites And Nucleotides Within SEQ ID NO:34 | Description Of Fragment And Chimeric Gene Components |
|---|---|
| *Asc*I/*Bsi*WI (5925-6645) | 720 bp 5' portion of *Yarrowia Lys5* gene (GenBank Accession No. M34929; labeled as "lys5 5' region" in Figure) |
| *Pac*I/*Sph*I (2536-3225) | 689 bp 3' portion of *Yarrowia Lys5* gene (GenBank Accession No. M34929; labeled as "Lys5-3"' in Figure) |

(continued)

| | |
|---|---|
| *EcoR*I/*BsiW*I (9338-6645) | FBAIN::EgD5SM::Pex20, comprising:<br>• FBAIN: *Yarrowia lipolytica* FBAIN promoter (U.S. Patent 7,202,356);<br>• EgD5SM: Synthetic mutant Δ5 desaturase (SEQ ID NO:35; U.S. Pat. Pub. No. 2010-0075386-A1), derived from *Euglena gracilis* (U.S. Patent 7,678,560) (labeled as "ED5S" in Figure);<br>• Pex20: Pex20 terminator sequence from *Yarrowia Pex20* gene (GenBank Accession No. AF054613) |
| *Pme*I /*Cla*I (11503-1) | YAT1::EaD5SM::OCT, comprising:<br>• YAT1: *Yarrowia lipolytica* YAT1 promoter (labeled as "YAT" in Figure; U.S. Pat. Appl. Pub. No. 2006-0094102-A1);<br>• EaD5SM: Synthetic, mutant Δ5 desaturase (SEQ ID NO:37; U.S. Pat. Pub. No. 2010-0075386-A1), derived from *Euglena anabaena* (U.S. Pat. Appl. Pub. No. 2008-0274521-A1) (labeled as "EaD5S" in Figure);<br>• OCT: OCT terminator sequence of *Yarrowia OCT* gene (GenBank Accession No. X69988) |
| *Cla*I/*Pac*I (1-2536) | EXP1::EgD5M::Pex16, comprising:<br>• EXP1: *Yarrowia lipolytica* export protein (EXP1) promoter (labeled as "EXP" in Figure; Intl. App. Pub. No. WO 2006/052870);<br>• EgD5M: Mutant Δ5 desaturase (SEQ ID NO:90; U.S. Pat. Pub. No. 2010-0075386-A1) with elimination of internal *EcoR*I, *Bgl*II, *Hind*III and *Nco*I restriction enzyme sites, derived from *Euglena gracilis* (U.S. Patent 7,678,560) (labeled as "Euglena D5WT" in Figure);<br>• Pex16: Pex16 terminator sequence from *Yarrowia Pex16* gene (GenBank Accession No. U75433) |
| *Eco*RI/*Pme*I (9360-11503) | *Yarrowia Leu2* gene (GenBank Accession No. M37309) |

The pZKSL-5S5A5 plasmid was digested with *Asc*I/*Sph*I, and then used for transformation of strain L135U9 according to the General Methods. The transformant cells were plated onto MMUraLys plates and maintained at 30 °C for 2 to 3 days. Single colonies were then re-streaked onto MMUraLys plates, and then inoculated into liquid MMUraLys at 30 °C and shaken at 250 rpm/min for 2 days. The cells were subjected to fatty acid analysis, according to the General Methods.

GC analyses showed the presence of ARA in the transformants containing the 3 chimeric genes of pZKSL-5S5A5, but not in the parent L135U9 strain. Five strains (i.e., #28, #62, #73, #84 and #95) that produced about 32.2%, 32.9%, 34.4%, 32.1 % and 38.6% ARA of TFAs were designated as strains Y8000, Y8001, Y8002, Y8003 and Y8004, respectively. Further analyses showed that the three chimeric genes of pZKSL-5S5A5 were not integrated into the *Lys5* site in the Y8000, Y8001, Y8002, Y8003 and Y8004 strains. All strains possessed a *Lys*+ phenotype.

The final genotype of strains Y8000, Y8001, Y8002, Y8003 and Y8004 with respect to wildtype *Yarrowia lipolytica* ATCC #20362 was *Ura-, Pex3-unknown 1-, unknown* 2-, *Leu+, Lys+,* YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgD8M::Pex16, FBAIN::EgD5SM::Pex20, EXP1::EgD5M::Pex16, YAT1::EaD5SM::Oct.

Generation Of Y8006 Strains To Produce About 41% ARA Of TFAs

Construct pZP3-Pa777U (FIG. 4B; SEQ ID NO:39; described in Table 9 of U.S. Pat. Appl. Pub. No. 2009-0093543-A1) was generated to integrate three Δ17 desaturase genes into the *Pox3* loci (GenBank Accession No. AJ001301) of strain Y8002.

The pZP3-Pa777U plasmid was digested with *Asc*I/*Sph*I, and then used for transformation of strain Y8002 according to the General Methods. The transformant cells were plated onto MM plates and maintained at 30 °C for 2 to 3 days. Single colonies were then re-streaked onto MM plates, and then inoculated into liquid MM at 30 °C and shaken at 250 rpm/min for 2 days. The cells were subjected to fatty acid analysis, according to the General Methods.

GC analyses showed the presence of 26% to 31% EPA of TFAs in most of the selected 96 transformants containing the 3 chimeric genes of pZP3-Pa777U, but not in the parent Y8002 strain. Strain #69 produced about 38% EPA of TFAs and was designated as Y8007. There was one strain (i.e., strain #9) that did not produce EPA, but produced about 41% ARA of TFAs. This strain was designated as Y8006. Based on the lack of EPA production in strain Y8006, its genotype with respect to wildtype *Yarrowia lipolytica* ATCC #20362 is assumed to be *Pex3-, unknown 1-*, *unknown 2-*, *unknown 3-, Leu+*, *Lys+*, Ura+, YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgD8M::Pex16, FBAIN::EgD5SM::Pex20, EXP1::EgD5M::Pex16, YAT1::EaD5SM::Oct.

In contrast, the final genotype of strain Y8007 with respect to wildtype *Yarrowia lipolytica* ATCC #20362 was *Pex3-, unknown 1-*, *unknown 2-, unknown 3-, Leu+, Lys+, Ura+,* YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgD8M::Pex16, FBAIN::EgD5SM::Pex20, EXP1::EgD5M::Pex16, YAT1::EaD5SM::Oct, YAT1::PaD17S::Lip1, EXP1::PaD17::Pex16, FBAINm::PaD17::Aco (wherein PaD17 is a *Pythium aphanidermatum* Δ17 desaturase [U.S. Patent 7,556,949] and PaD17S is a codon-optimized Δ17 desaturase, derived from *Pythium aphanidermatum* [U.S. Patent 7,556,949].

Integration of the 3 chimeric genes of pZP3-Pa777U into the *Pox3* loci (GenBank Accession No. AJ001301) in strains Y8006 and Y8007 was not confirmed.

Generation Of Strain Y8006U6 *(Ura3-)*

To disrupt the *Ura3* gene, construct pZKUM (FIG. 5A; SEQ ID NO:40; described in Table 15 of U.S. Pat. Appl. Pub. No. 2009-0093543-A1) was used to integrate a *Ura3* mutant gene into the *Ura3* gene of strain Y8006.

Plasmid pZKUM was digested with *Sal*I/*PacI,* and then used to transform strain Y8006 according to the General Methods. Following transformation, cells were plated onto MM + 5-FOA selection plates and maintained at 30 °C for 2 to 3 days.

A total of 8 transformants grown on MM + 5-FOA plates were picked and re-streaked onto MM plates and MM + 5-FOA plates, separately. All 8 strains had a *Ura-* phenotype (i.e., cells could grow on MM + 5-FOA plates, but not on MM plates). The cells were scraped from the MM + 5-FOA plates and subjected to fatty acid analysis, according to the General Methods.

GC analyses showed the presence of 22.9%, 25.5%, 23.6% 21.6%, 21.6% and 25% ARA of TFAs in the pZKUM-transformant strains #1, #2, #4, #5, #6 and #7, respectively, grown on MM + 5-FOA plates. These six strains were designated as strains Y8006U1, Y8006U2, Y8006U3, Y8006U4, Y8006U5 and Y8006U6, respectively (collectively, Y8006U).

Generation Of Y8069 Strain To Produce About 37.5% EPA Of TFAs

Construct pZP3-Pa777U (FIG. 4B; SEQ ID NO:39; described in Table 9 of U.S. Pat. Appl. Pub. No. 2009-0093543-A1) was used to integrate three Δ17 desaturase genes into the *Pox3* loci (GenBank Accession No. AJ001301) of strain Y8006U6.

The pZP3-Pa777U plasmid was digested with *Asc*I/*Sph*I, and then used for transformation of strain Y8006U6 according to the General Methods. The transformant cells were plated onto MM plates and maintained at 30 °C for 2 to 3 days. Single colonies were then re-streaked onto MM plates, and then inoculated into liquid MM at 30 °C and shaken at 250 rpm/min for 2 days. The cells were subjected to fatty acid analysis, according to the General Methods.

GC analyses showed the presence of EPA in the transformants containing the 3 chimeric genes of pZP3-Pa777U, but not in the parent Y8006U6 strain. Most of the selected 24 strains produced 24-37% EPA of TFAs. Four strains (i.e., #1, #6, #11 and #14) that produced 37.5%, 43.7%, 37.9% and 37.5% EPA of TFAs were designated as Y8066, Y8067, Y8068 and Y8069, respectively. Integration of the 3 chimeric genes of pZP3-Pa777U into the *Pox3* loci (GenBank Accession No. AJ001301) of strains Y8066, Y8067, Y8068 and Y8069 was not confirmed.

The final genotype of strains Y8066, Y8067, Y8068 and Y8069 with respect to wildtype *Yarrowia lipolytica* ATCC #20362 was Ura+, *Pex3-, unknown 1-*, *unknown 2-, unknown 3-, unknown 4-, Leu+, Lys+,* YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgD8M::Pex16, FBAIN::EgD5SM::Pex20, EXP1::EgD5M::Pex16, YAT1::EaD5SM::Oct, YAT1::PaD17S::Lip1, EXP1::PaD17::Pex16, FBAINm::PaD17::Aco.

Generation Of Strain Y8069U *(Ura3-)*

To disrupt the *Ura3* gene, construct pZKUM (FIG. 5A; SEQ ID NO:40; described in Table 15 of U.S. Pat. Appl. Pub. No. 2009-0093543-A1) was used to integrate a *Ura3* mutant gene into the *Ura3* gene of strain Y8069, in a manner similar to that described for pZKUM transformation of strain Y8006 *(supra).* A total of 3 transformants were grown and identified to possess a *Ura-* phenotype.

GC analyses showed the presence of 22.4%, 21.9% and 21.5% EPA of TFAs in the pZKUM-transformant strains #1, #2 and #3, respectively, grown on MM + 5-FOA plates. These three strains were designated as strains Y8069U1, Y8069U2, and Y8069U3, respectively (collectively, Y8069U).

Generation Of Strain Y8154 To Produce about 44.8% EPA Of TFAs

Construct pZKL2-5mB89C (FIG. 5B; SEQ ID NO:41) was generated to integrate one Δ5 desaturase gene, one Δ9 elongase gene, one Δ8 desaturase gene, and one *Yarrowia lipolytica* diacylglycerol cholinephosphotransferase gene (CPT1) into the *Lip2* loci (GenBank Accession No. AJ012632) of strain Y8069U3 to thereby enable higher level production of EPA. The pZKL2-5mB89C plasmid contained the following components:

Table 5: Description of Plasmid pZKL2-5mB89C (SEQ ID NO:41)

| RE Sites And Nucleotides Within SEQ ID NO:41 | Description Of Fragment And Chimeric Gene Components |
|---|---|
| *Asc*I/*Bsi*WI (730-1) | 722 bp 5' portion of *Yarrowia Lip2* gene (labeled as "Lip2.5N" in Figure; GenBank Accession No. AJ012632) |
| *Pac*I/*Sph*I (4141-3438) | 697 bp 3' portion of *Yarrowia Lip2* gene (labeled as "Lip2.3N" in Figure; GenBank Accession No. AJ012632) |
| *Swa*I/*Bsi*WI (13561-1) | YAT1::YlCPT1::Aco, comprising:<br>• YAT1: *Yarrowia lipolytica* YAT1 promoter (labeled as "YAT" in Figure; U.S. Pat. Appl. Pub. No. 2006-0094102-A1);<br>• YlCPT1: *Yarrowia lipolytica* diacylglycerol cholinephosphotransferase gene (SEQ ID NO:42) (labeled as "Y. lipolytica CPT1 cDNA" in Figure; Intl. App. Pub. No. WO 2006/052870);<br>• Aco: Aco terminator sequence from *Yarrowia Aco* gene (GenBank Accession No. AJ001300) |
| *Pme*I/*Swa*I (10924-13561) | FBAIN::EgD8M::Lip1 comprising:<br>• FBAIN: *Yarrowia lipolytica* FBAIN promoter (U.S. Patent 7,202,356);<br>• EgD8M: Synthetic mutant Δ8 desaturase (SEQ ID NO:44; U.S. Patent 7,709,239), derived from *Euglena gracilis* ("EgD8S"; U.S. Patent 7,256,033) (labeled as "D8S-23" in |
| | Figure);<br>• Lip1: Lip1 terminator sequence from *Yarrowia Lip1* gene (GenBank Accession No. Z50020) |
| *Pme*I/*Cla*I (10924-9068) | YAT1::EgD9eS::Lip2, comprising:<br>• YAT1: *Yarrowia lipolytica* YAT1 promoter (labeled as "YAT" in Figure; U.S. Pat. Appl. Pub. No. 2006-0094102-A1);<br>• EgD9eS: codon-optimized Δ9 elongase (SEQ ID NO:46), derived from *Euglena gracilis* (U.S. Patent 7,645,604);<br>• Lip2: Lip2 terminator sequence from *Yarrowia Lip2* gene (GenBank Accession No. AJ012632) |
| *Cla*I/*Eco*RI (9068-6999) | *Yarrowia* Ura3 gene (GenBank Accession No. AJ306421) |
| *Eco*RI/*Pac*I (6999-4141) | GPDIN::EgD5SM::ACO, comprising:<br>• GPDIN: *Yarrowia lipolytica* GPDIN promoter (U.S. Patent 7,459,546);<br>• EgD5SM: Synthetic mutant Δ5 desaturase (SEQ ID NO:35; U.S. Pat. Pub. No. 2010-0075386-A1), derived from *Euglena gracilis* (U.S. Patent 7,678,560) (labeled as "EgD5S-HPGS" in Figure);<br>• Aco: Aco terminator sequence from *Yarrowia Aco* gene (GenBank Accession No. AJ001300) |

The pZKL2-5mB89C plasmid was digested with *Asc*I/*Sph*I, and then used for transformation of strain Y8069U3 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced approximately 38-44% EPA of TFAs. Seven strains (i.e., #1, #39, #49, #62, #70, #85 and #92) that produced about 44.7%, 45.2%, 45.4%, 44.8%, 46.1%, 48.6% and 45.9% EPA of TFAs were designated as strains Y8151, Y8152, Y8153, Y8154, Y8155, Y8156 and Y8157, respectively. Knockout of the Lip2 gene was not confirmed in these EPA strains.

The final genotype of strains Y8151, Y8152, Y8153, Y8154, Y8155, Y8156 and Y8157 with respect to wildtype *Yarrowia lipolytica* ATCC #20362 was *Ura+, Pex3-, unknown 1-, unknown 2-, unknown 3-, unknown 4-, unknown 5-, Leu+, Lys+,* YAT1::ME3S::Pex16, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::FmD12::Pex20, YAT1::FmD12::Oct, EXP1::EgD5M::Pex16, YAT1::EaD5SM::Oct, FBAIN::EgD5SM::Pex20, GPDIN::EgD5SM::Aco, FBAINm::PaD17::Aco, EXP1::PaD17::Pex16, YAT1::PaD17S::Lip1, YAT1::YlCPT::Aco.

Generation Of Strain Y8154U1 (*Ura3-*)

To disrupt the *Ura3* gene, construct pZKUM (FIG. 5A; SEQ ID NO:40; described in Table 15 of U.S. Pat. Appl. Pub. No. 2009-0093543-A1) was used to integrate a *Ura3* mutant gene into the *Ura3* gene of strain Y8154, in a manner similar to that described for pZKUM transformation of strain Y8006 (*supra*). A total of 8 transformants were grown and identified to possess a *Ura-* phenotype.

GC analyses showed that there was 23.1% EPA of TFAs in the pZKUM-transformant strain #7. This strain was designated as strain Y8154U1.

Generation Of Strain Y8269 To Produce About 45.3% EPA Of TFAs

Construct pZKL1-2SR9G85 (FIG. 6A; SEQ ID NO:48) was generated to integrate one DGLA synthase, one Δ12 desaturase gene and one Δ5 desaturase gene into the *Lip1* loci (GenBank Accession No. Z50020) of strain Y8154U1 to thereby enable higher level production of EPA. A DGLA synthase is a multizyme comprising a Δ9 elongase linked to a Δ8 desaturase (U.S. Pat. Appl. Pub. No. 2008-0254191-A1).

The pZKL1-2SR9G85 plasmid contained the following components:

Table 6: Description of Plasmid pZKL1-2SR9G85 (SEQ ID NO:48)

| RE Sites And Nucleotides Within SEQ ID NO:48 | Description Of Fragment And Chimeric Gene Components |
|---|---|
| *Asc*I/*Bsi*WI (4189-3373) | 809 bp 5' portion of *Yarrowia Lip1* gene (labeled as "Lip1-5'N" in Figure; GenBank Accession No. Z50020) |
| *Pac*I/*Sph*I (7666-6879) | 763 bp 3' portion of *Yarrowia Lip1* gene (labeled as "Lip1.3N" in Figure; GenBank Accession No. Z50020) |
| *Cla*I/*Swa*I (1-3217) | YAT1::E389D9eS/EgD8M::Lip1, comprising:<br>• YAT1: *Yarrowia lipolytica* YAT1 promoter (labeled as "YAT" in Figure; U.S. Pat. Appl. Pub. No. 2006-0094102-A1);<br>• E389D9eS/EgD8M: gene fusion comprising a codon-optimized Δ9 elongase derived from *Eutreptiella* sp. CCMP389 ("E389D9eS"), a linker, and the synthetic mutant Δ8 desaturase derived from *Euglena gracilis* ("EgD8M") (SEQ ID NO:49) (labeled individually as "E389S", "Linker" and "EgD8M" in Figure; U.S. Pat. Appl. Pub. No. 2008-0254191-A1);<br>• Lip1: Lip1 terminator sequence from *Yarrowia Lip1* gene (GenBank Accession No. Z50020) |

(continued)

| RE Sites And Nucleotides Within SEQ ID NO:48 | Description Of Fragment And Chimeric Gene Components |
|---|---|
| *Sal*I/*Cla*I (11982-1) | GPM::EgD5SM::Oct comprising:<br>• GPM: *Yarrowia lipolytica* GPM promoter (labeled as "GPML" in Figure; U.S. Patent 7,202,356);<br>• EgD5SM: Synthetic mutant Δ5 desaturase (SEQ ID NO:35; U.S. Pat. Pub. No. 2010-0075386-A1), derived from *Euglena gracilis* (U.S. Patent 7,678,560) (labeled as "ED5S" in Figure);<br>• OCT: OCT terminator sequence of *Yarrowia OCT* gene (GenBank Accession No. X69988) |
| *Sal*I/*Eco*RI (11982-10363) | *Yarrowia* Ura3 gene (GenBank Accession No. AJ306421) |
| *Eco*RI/*Pac*I (10363-7666) | EXP1::FmD12S::ACO, comprising:<br>• EXP1: *Yarrowia lipolytica* export protein (EXP1) promoter (labeled as "Exp" in Figure; Intl. App. Pub. No. WO 2006/052870);<br>• FmD12S: codon-optimized Δ12 elongase (SEQ ID NO:51), derived from *Fusarium moniliforme* (labeled as "FD12S" in Figure; U.S. Patent 7,504,259);<br>• Aco: Aco terminator sequence from *Yarrowia Aco* gene (GenBank Accession No. AJ001300) |

The pZKL1-2SR9G85 plasmid was digested with *Asc*I/*Sph*I, and then used for transformation of strain Y8154U1 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced 40-44.5% EPA of total lipids. Five strains (i.e., #44, #46, #47, #66 and #87) that produced about 44.8%, 45.3%, 47%, 44.6% and 44.7% EPA of TFAs were designated as Y8268, Y8269, Y8270, Y8271 and Y8272, respectively. Knockout of the *Lip1* loci (GenBank Accession No. Z50020) was not confirmed in these EPA strains.

The final genotype of strains Y8268, Y8269, Y8270, Y8271 and Y8272 with respect to wildtype *Yarrowia lipolytica* ATCC #20362 was Ura+, *Pex3-, unknown 1-*, *unknown 2-, unknown 3-, unknown 4-, unknown 5-, unknown6-,* YAT1::ME3S::Pex16, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, YAT1::E389D9eS/EgD8M::Lip1, GPD::FmD12::Pex20, YAT1::FmD12::Oct, EXP1::FmD12S::Aco, EXP1::EgD5M::Pex16, YAT1::EaD5SM::Oct, FBAIN::EgD5SM::Pex20, GPDIN::EgD5SM::Aco, GPM::EgD5SM::Oct, FBAINm::PaD17::Aco, EXP1::PaD17::Pex16, YAT1::PaD17S::Lip1, YAT1::YlCPT::Aco.

Generation Of Strain Y8269U (*Ura3-*)

To disrupt the *Ura3* gene, construct pZKUM (FIG. 5A; SEQ ID NO:40; described in Table 15 of U.S. Pat. Appl. Pub. No. 2009-0093543-A1) was used to integrate a *Ura3* mutant gene into the *Ura3* gene of strain Y8269, in a manner similar to that described for pZKUM transformation of strain Y8006 (*supra*). A total of 8 transformants were grown and identified to possess a *Ura-* phenotype.

GC analyses showed that there were 23.0%, 23.1% and 24.2% EPA of TFAs in pZKUM-transformant strains #2, #3 and #5, respectively. These strains were designated as strains Y8269U1, Y8269U2 and Y8269U3, respectively (collectively, Y8269U).

Generation Of Strain Y8406 And Strain Y8412 To Produce About 51.2% EPA And 55.8% EPA Of TFAs

Construct pZSCP-Ma83 (FIG. 6B; SEQ ID NO:53) was generated to integrate one Δ8 desaturase gene, one $C_{16/18}$ elongase gene and one malonyl-CoA synthetase gene into the *SCP2* loci (GenBank Accession No. XM_503410) of strain Y8269U1 to thereby enable higher level production of EPA. The pZSCP-Ma83 plasmid contained the following components:

Table 7: Description of Plasmid pZSCP-Ma83 (SEQ ID NO:53)

| RE Sites And Nucleotides Within SEQ ID NO:53 | Description Of Fragment And Chimeric Gene Components |
|---|---|
| *BsiWI/AscI* (1-1328) | 1327 bp 3' portion of *Yarrowia SCP2* gene (labeled as "SCP2-3'" in Figure; GenBank Accession No. XM_503410) |
| *SphI/PacI* (4036-5816) | 1780 bp 5' portion of *Yarrowia SCP2* gene (labeled as "SCP2-5'" in Figure; GenBank Accession No. XM_503410) |
| *SwaI/BsiWI* (12994-1) | GPD::ME3S::Pex20, comprising:<br>• GPD: *Yarrowia lipolytica* GPD promoter (U.S. Patent 7,259,255);<br>• ME3S: codon-optimized $C_{16/18}$ elongase gene (SEQ ID NO:54), derived from *M. alpina* (U.S. Patent 7,470,532);<br>• Pex20: Pex20 terminator sequence from *Yarrowia Pex20* gene (GenBank Accession No. AF054613) |
| *PmeI/SwaI* (10409-12994) | YAT1::MCS::Lip1 comprising:<br>• YAT1: *Yarrowia lipolytica* YAT1 promoter (labeled as "YAT" in Figure; U.S. Pat. Appl. Pub. No. 2006/0094102-A1);<br>• MCS: codon-optimized malonyl-CoA synthetase gene (SEQ ID NO:56), derived from *Rhizobium leguminosarum* bv. viciae 3841 (U.S. Patent Application No. 12/637877);<br>• Lip1: Lip1 terminator sequence from *Yarrowia Lip1* gene (GenBank Accession No. Z50020) |
| *ClaI/PmeI* (7917-10409) | GPD::EaD8S::Pex16 comprising:<br>• GPD: *Yarrowia lipolytica* GPD promoter (U.S. Patent 7,259,255);<br>• EaD8S: codon-optimized Δ8 desaturase gene (SEQ ID NO:58), derived from *Euglena anabaena* (U.S. Pat. Appl. Pub. No. 2008-0254521-A1);<br>• Pex16: Pex16 terminator sequence from *Yarrowia Pex16* gene (GenBank Accession No. U75433) |
| *SalI/EcoRI* (7467-5848) | *Yarrowia* Ura3 gene (GenBank Accession No. AJ306421) |

The pZSCP-Ma83 plasmid was digested with *AscI/SphI,* and then used for transformation of strains Y8269U1, Y8269U2 and Y8269U3, separately, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

A total of 96 strains resulting from each pZSCP-Ma83 transformation (i.e., into Y8269U1, Y8269U2 and Y8269U3) were analyzed by GC. Most of the selected 288 strains produced 43-47% EPA of TFAs. Seven strains of Y8269U1 transformed with pZSCP-Ma83 (i.e., #59, #61, #65, #67, #70, #81 and #94) that produced about 51.3%, 47.9%, 50.8%, 48%, 47.8%, 47.8% and 47.8% EPA of TFAs were designated as strains Y8404, Y8405, Y8406, Y8407, Y8408, Y8409 and Y8410, respectively. Three strains of Y8269U2 transformed with pZSCP-Ma83 (i.e., #4, #13 and #17) that produced about 48.8%, 50.8%, and 49.3% EPA of TFAs were designated as Y8411, Y8412 and Y8413, respectively. And, two strains of Y8269U3 transformed with pZSCP-Ma83 (i.e., #2, and #16) that produced about 49.3% and 53.5% EPA of TFAs were designated as Y8414 and Y8415, respectively.

Knockout of the *SCP2* loci (GenBank Accession No. XM_503410) was not confirmed in any of these EPA strains, produced by transformation with pZSCP-Ma83.

The final genotype of strains Y8404, Y8405, Y8406, Y8407, Y8408, Y8409, Y8410, Y8411, Y8412, Y8413, Y8414 and Y8415 with respect to wildtype *Yarrowia lipolytica* ATCC #20362 was *Ura+*, *Pex3-, unknown 1-*, *unknown 2-, unknown 3-, unknown 4-, unknown 5-, unknown6-, unknown 7-,* YAT1::ME3S::Pex16, GPD::ME3S::Pex20, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16, YAT1::E389D9eS/EgD8M::Lip1, GPD::FmD12::Pex20, YAT1::FmD12::Oct, EXP1::FmD12S::Aco, EXP1::EgD5M::Pex16, YAT1::EaD5SM::Oct, FBAIN::EgD5SM::Pex20, GPDIN::EgD5SM::Aco, GPM::EgD5SM::Oct, FBAINm::PaD17::Aco, EXP1::PaD17::Pex16, YAT1::PaD17S::Lip1, YAT1::YlCPT::Aco, YAT1::MCS::Lip1.

*Yarrowia lipolytica* strain Y8406 was deposited with the American Type Culture Collection on May 14, 2009 and bears the designation ATCC PTA-10025. *Yarrowia lipolytica* strain Y8412 was deposited with the American Type Culture Collection on May 14, 2009 and bears the designation ATCC PTA-10026.

Analysis Of Total Lipid Content And Composition By Flask Assay

Cells from YPD plates of strains Y8404, Y8405, Y8406, Y8407, Y8408, Y8409, Y8410, Y8411, Y8412, Y8413, Y8414 and Y8415 were grown and analyzed for total lipid content and composition, as follows.

Specifically, one loop of freshly streaked cells was inoculated into 3 mL FM medium and grown overnight at 250 rpm and 30 °C. The $OD_{600nm}$ was measured and an aliquot of the cells were added to a final $OD_{600nm}$ of 0.3 in 25 mL FM medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for fatty acid analysis (*supra*) and 10 mL dried for dry cell weight ["DCW"] determination.

For DCW determination, 10 mL culture was harvested by centrifugation for 5 min at 4000 rpm in a Beckman GH-3.8 rotor in a Beckman GS-6R centrifuge. The pellet was resuspended in 25 mL of water and re-harvested as above. The washed pellet was re-suspended in 20 mL of water and transferred to a pre-weighed aluminum pan. The cell suspension was dried overnight in a vacuum oven at 80 °C. The weight of the cells was determined.

Data from flask assays are presented as Table 8. The Table presents the total dry cell weight of the cells ["DCW'], the total lipid content of cells ["FAME % DCW"], the concentration of each fatty acid as a weight percent of TFAs ["% TFAs"] and the EPA content as a percent of the dry cell weight ["EPA FAME % DCW"]. More specifically, fatty acids will be identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

Table 8: Total Lipid Content And Composition In *Yarrowia* Strains Y8404, Y8405, Y8406, Y8407, Y8408, Y8409, Y8410, Y8411, Y8412, Y8413, Y8414 And Y8415 By Flask Assay

| Strain | DCW (g/L) | Total FAME % DCW | % TFAs | | | | | | | | | | | | | EPA FAME % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8404 | 4.1 | 27.3 | 2.8 | 0.8 | 1.8 | 5.1 | 20.4 | 2.1 | 2.9 | 2.5 | 0.6 | 0.8 | 2.4 | 51.1 | 6.3 | 14.0 |
| Y8405 | 3.9 | 29.6 | 2.7 | 0.5 | 2.9 | 5.7 | 20.5 | 2.8 | 2.7 | 2.1 | 0.5 | 0.7 | 2.0 | 51.4 | 5.1 | 15.2 |
| **Y8406** | **4.0** | **30.7** | **2.6** | **0.5** | **2.9** | **5.7** | **20.3** | **2.8** | **2.8** | **2.1** | **0.5** | **0.8** | **2.1** | **51.2** | **5.4** | **15.7** |
| Y8407 | 4.0 | 29.4 | 2.6 | 0.5 | 3.0 | 5.6 | 20.5 | 2.8 | 2.7 | 2.1 | 0.4 | 0.7 | 2.1 | 51.5 | 5.1 | 15.2 |
| Y8408 | 4.1 | 29.8 | 2.9 | 0.6 | 2.7 | 5.7 | 20.2 | 2.8 | 2.6 | 2.1 | 0.5 | 0.9 | 2.1 | 51.2 | 5.5 | 15.3 |
| Y8409 | 3.9 | 30.8 | 2.8 | 0.5 | 2.9 | 5.7 | 20.6 | 2.7 | 2.7 | 2.1 | 0.5 | 0.8 | 2.1 | 51.0 | 5.2 | 15.7 |
| Y8410 | 4.0 | 31.8 | 2.7 | 0.5 | 3.0 | 5.7 | 20.5 | 2.9 | 2.7 | 2.1 | 0.5 | 0.7 | 2.1 | 50.9 | 5.3 | 16.2 |
| Y8411 | 3.6 | 30.5 | 2.7 | 0.3 | 3.3 | 5.1 | 19.9 | 2.6 | 2.4 | 2.0 | 0.5 | 0.6 | 1.8 | 52.9 | 5.7 | 16.1 |
| **Y8412** | **3.2** | **27.0** | **2.5** | **0.4** | **2.6** | **4.3** | **19.0** | **2.4** | **2.2** | **2.0** | **0.5** | **0.6** | **1.9** | **55.8** | **5.6** | **15.1** |
| Y8413 | 2.9 | 27.2 | 3.1 | 0.4 | 2.6 | 5.4 | 19.9 | 2.2 | 2.8 | 2.0 | 0.5 | 0.7 | 1.8 | 52.4 | 5.9 | 14.2 |
| Y8414 | 3.7 | 27.1 | 2.5 | 0.7 | 2.3 | 6.0 | 19.9 | 1.6 | 3.4 | 3.4 | 0.6 | 0.6 | 3.1 | 49.4 | 6.1 | 13.4 |
| Y8415 | 3.6 | 25.9 | 1.4 | 0.3 | 1.9 | 4.5 | 16.0 | 1.3 | 2.7 | 2.9 | 0.5 | 0.6 | 2.5 | 59.0 | 6.1 | 15.3 |

Generation Of Strain Y8406U (*Ura3*-)

To disrupt the *Ura3* gene, construct pZKUM (FIG. 5A; SEQ ID NO:40; described in Table 15 of U.S. Pat. Appl. Pub. No. 2009-0093543-A1) was used to integrate a *Ura3* mutant gene into the *Ura3* gene of strain Y8406 in a manner similar to that described for pZKUM transformation of strain Y8006 (*supra*). Several transformants were grown and identified to possess a *Ura*- phenotype.

GC analyses showed that there were 26.1% EPA of FAMEs in pZKUM-transformant strains #4 and #5. These two strains were designated as strains Y8406U1 and Y8406U2, respectively (collectively, Y8406U).

EXAMPLE 2

Generation Of *Yarrowia lipolytica* Strain Y5037 To Produce About 18.6% EPA, 22.8% DPA And 9.7% DHA Of Total Fatty Acids

The present Example describes the construction of strain Y5037, derived from *Yarrowia lipolytica* ATCC #20362, capable of producing about 18.6% EPA, 22.8% DPA and 9.7% DHA relative to the total lipids via expression of a Δ9 elongase/Δ8 desaturase pathway. This strain was used as the DHA-producing host cell in Example 5.

Briefly, as diagrammed in FIG. 7, strain Y5037 was derived from *Yarrowia lipolytica* ATCC #20362 via construction of strain Y2224 (a FOA resistant mutant from an autonomous mutation of the *Ura3* gene of wildtype *Yarrowia* strain ATCC #20362), strain Y4001 (producing 17% EDA with a *Leu*- phenotype), strain Y4001U1 (*Leu*- and *Ura*-), strain Y4036 (producing 18% DGLA with a *Leu*- phenotype), strain Y4036U (*Leu*- and *Ura*-), strain Y4070 (producing 12% ARA with a *Ura*-phenotype), strain Y4086 (producing 14% EPA), strain Y4086U1 (*Ura3*-), strain Y4128 (producing 37% EPA; deposited with the American Type Culture Collection on August 23, 2007, bearing the designation ATCC PTA-8614), strain Y4128U3 (*Ura*-), strain Y4217 (producing 42% EPA), strain Y4217U2 (*Ura*-), strain Y4259 (producing 46.5% EPA), strain Y4259U2 (*Ura*-), strain Y4305 (producing 53.2% EPA), strain Y4305U3 (*Ura*-), strain Y5004 (producing 17% EPA, 18.7% DPA and 6.4% DHA), strain Y5004U1 (*Ura*-), strain Y5018 (producing 25.4% EPA, 11.4% DPA and 9.4% DHA), strain Y5018U1 (*Ura*-) and strain Y5037 (producing 18.6% EPA, 22.8% DPA and 9.7% DHA relative to the total TFAs). Further details regarding the construction of strains Y2224, Y4001, Y4001U, Y4036, Y4036U, Y4070, Y4086, Y4086U1, Y4128, Y4128U3, Y4217, Y4217U2, Y4259, Y4259U2, Y4305 and Y4305U3 are described in the General Methods of U.S. Pat. App. Pub. No. 2008-0254191-A1 and in Examples 1-3 of U.S. Pat. App. Pub. No. 2009-0093543-A1.

The complete lipid profile of strain Y4305 was as follows: 16:0 (2.8%), 16:1 (0.7%), 18:0 (1.3%), 18:1 (4.9%), 18:2 (17.6%), ALA (2.3%), EDA (3.4%), DGLA (2.0%), ARA (0.6%), ETA (1.7%), and EPA (53.2%). The total lipid content of cells ["TFAs % DCW"] was 27.5.

The final genotype of strain Y4305 with respect to wild type *Yarrowia lipolytica* ATCC #20362 was *SCP2-(YALI0E01298g), YALI0C18711g-, Pex10-, YALI0F24167g-, unknown 1-*, *unknown 3-, unknown 8-,* GPD::FmD12::Pex20, YAT1::FmD12::OCT, GPM/FBAIN::FmD12S::OCT, EXP1::FmD12S::Aco, YAT1::FmD12S::Lip2, YAT1::ME3S::Pex16, EXP1::ME3S::Pex20 (3 copies), GPAT::EgD9e::Lip2, EXP1::EgD9eS::Lip1, FBAINm::EgD9eS::Lip2, FBA::EgD9eS::Pex20, GPD::EgD9eS::Lip2, YAT1::EgD9eS::Lip2, YAT1::E389D9eS::OCT, FBAINm::EgD8M::Pex20, FBAIN::EgD8M::Lip1 (2 copies), EXP1::EgD8M::Pex16, GPDIN::EgD8M::Lip1, YAT1::EgD8M::Aco, FBAIN::EgD5::Aco, EXP1::EgD5S::Pex20, YAT1::EgD5S::Aco, EXP1::EgD5S::ACO, YAT1::RD5S::OCT, YAT1::PaD17S::Lip1, EXP1::PaD17::Pex16, FBAINm::PaD17::Aco, YAT1::YlCPT1::ACO, GPD::YlCPT1::ACO (wherein FmD12 is a *Fusarium moniliforme* Δ12 desaturase gene [U.S. Patent 7,504,259]; FmD12S is a codon-optimized Δ12 desaturase gene, derived from *Fusarium moniliforme* [U.S. Patent 7,504,259]; ME3S is a codon-optimized $C_{16/18}$ elongase gene, derived from *Mortierella alpina* [U.S. Patent 7,470,532]; EgD9e is a *Euglena gracilis* Δ9 elongase gene [U.S. Patent 7,645,604]; EgD9eS is a codon-optimized Δ9 elongase gene, derived from *Euglena gracilis* [U.S. Patent 7,645,604]; E389D9eS is a codon-optimized Δ9 elongase gene, derived from *Eutreptiella* sp. CCMP389 [U.S. Patent 7,645,604]; EgD8M is a synthetic mutant Δ8 desaturase [U.S. Patent 7,709,239], derived from *Euglena gracilis* [U.S. Patent 7,256,033]; EgD5 is a *Euglena gracilis* Δ5 desaturase [U.S. Patent 7,678,560]; EgD5S is a codon-optimized Δ5 desaturase gene, derived from *Euglena gracilis* [U.S. Patent 7,678,560]; RD5S is a codon-optimized Δ5 desaturase, derived from *Peridinium sp.* CCMP626 [U.S. Patent 7,695,950]; PaD17 is a *Pythium aphanidermatum* Δ17 desaturase [U.S. Patent 7,556,949]; PaD17S is a codon-optimized Δ17 desaturase, derived from *Pythium aphanidermatum* [U.S. Patent 7,556,949]; and, YlCPT1 is a *Yarrowia lipolytica* diacylglycerol cholinephosphotransferase gene [Intl. App. Pub. No. WO 2006/052870]).

Strain Y4305U (*Ura3*-) was generated via integrating a *Ura3* mutant gene into the *Ura3* gene of strain Y4305.

Generation Of Y5004 Strain To Produce about 17.0% EPA, 18.7% DPA And 6.4% DHA Of TFAs

Construct pZKL4-220EA41B (FIG. 8A; SEQ ID NO:60) was constructed to integrate two $C_{20/22}$ elongase genes and two Δ4 desaturase genes into the lipase 4-like locus (GenBank Accession No. XM_503825) of strain Y4305U3. The pZKL4-220EA41B plasmid contained the following components:

Table 9: Components Of Plasmid pZKL4-220EA41B (SEQ ID NO:60)

| RE Sites And Nucleotides Within SEQ ID NO:60 | Description Of Fragment And Chimeric Gene Components |
|---|---|
| *Asc* I/*BsiW* I (9777-9025) | 745 bp 5' portion of the *Yarrowia* Lipase 4-like gene (GenBank Accession No. XM_503825; labeled as "Lip4" in Figure) |
| *PacI*/*SphI* (13273-12485) | 782 bp 3' portion of *Yarrowia* Lipase 4 like gene (GenBank Accession No. XM_503825; labeled as "Lip4-3'" in Figure) |
| *SwaI*/*BsiWI* (6882-9025) | FBAINm::EaC20ES::Pex20, comprising:<br>• FBAINm: *Yarrowia lipolytica* FBAINm promoter (U.S. Patent 7,202,356)<br>• EaC20ES: codon-optimized C20 elongase gene (SEQ ID NO:61), derived from *Euglena anabaena* (U.S. Pat. Appl. Pub. No. 2008/0254191-A1);<br>• Pex20: Pex20 terminator sequence from *Yarrowia Pex20* gene (GenBank Accession No. AF054613) |
| *Pme*I/*Swa*I (4903-6882) | YAT1::EgC20ES::Lip1, comprising:<br>• YAT1: *Yarrowia lipolytica* YAT1 promoter (U.S. Pat. Appl. Pub. No. 2006/0094102-A1);<br>• EgC20ES: codon-optimized C20 elongase gene (SEQ ID NO:63), derived from *Euglena gracilis* (U.S. Pat. Appl. Pub. No. 2008/0254191-A1);<br>• Lip1: Lip1 terminator sequence from *Yarrowia Lip1* gene (GenBank Accession No. Z50020) |
| *Pme*I/*Cla*I (4903-2070) | EXP1::EaD4S-1::Lip2, comprising:<br>• EXP1: *Yarrowia lipolytica* export protein (EXP1) promoter (Intl. App. Pub. No. WO 2006/052870);<br>• EaD4S-1: codon-optimized truncated Δ4 desaturase (SEQ ID NO:65), derived from *Euglena anabaena* (U.S. Pat. Appl. Pub. No. 2008/0254191-A1);<br>• Lip2: Lip2 terminator sequence from *Yarrowia Lip2* gene (GenBank Accession No. AJ012632) |
| *SalIIEco*RI (1620-1) | *Yarrowia* Ura3 gene (GenBank Accession No. AJ306421) |
| *Eco*RI/*Pac*I (1-14039) | GPDIN::EaD4SB::Aco, comprising:<br>• GPDIN: *Yarrowia lipolytica* GPDIN promoter (U.S. Patent 7,459,546);<br>• EaD4SB: codon-optimized truncated Δ4 desaturase version B (SEQ ID NO:67), derived from *Euglena anabaena* (U.S. Pat. Appl. Pub. No. 2008/0254191-A1);<br>• Aco: Aco terminator sequence from *Yarrowia Aco* gene (GenBank Accession No. AJ001300) |

The pZKL4-220EA41B plasmid was digested with *Asc*I/*Sph*I, and then used for transformation of strain Y4305U3 *(supra),* according to the General Methods. The transformants were selected on MM plates. After 5 days growth at 30 °C, 72 transformants grown on the MM plates were picked and re-streaked onto fresh MM plates. Once grown, these strains were individually inoculated into 3 mL liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed the presence of DHA in the transformants with pZKL4-220EA41B, but not in the parent Y4305U strain. Most of the selected 72 strains produced about 22% EPA, 18% DPA and 5% DHA of TFAs. Strain #2 produced 17% EPA, 18.7% DPA and 6.4% DHA, while strain #33 produced 21.5% EPA, 21% DPA and 5.5% DHA. These two strains were designated as Y5004 and Y5005, respectively.

Knockout of the lipase 4-like locus (GenBank Accession No. XM_503825) was not confirmed in either strain Y5004 or Y5005.

Generation Of Strain Y5004U (*Ura3*-)

To disrupt the *Ura3* gene, construct pZKUM (FIG. 5A; SEQ ID NO:40; described in Table 15 of U.S. Pat. App. Pub. No. 2009-0093543-A1) was used to integrate a *Ura3* mutant gene into the *Ura3* gene of strain Y5004, in a manner similar to that described for pZKUM transformation of strain Y8006 (Example 1).

A total of 8 transformants grown on MM + 5-FOA plates were picked and re-streaked onto MM plates and MM + 5-FOA plates, separately. All 8 strains had a *Ura*- phenotype (i.e., cells could grow on MM + 5-FOA plates, but not on MM plates). The cells were scraped from the MM + 5-FOA plates and subjected to fatty acid analysis, according to the General Methods.

GC analyses showed the presence of 14.8% EPA, 17.4% DPA and 0.4% DHA of TFAs in transformant #5 and 15.3% EPA, 17.2% DPA and 0.4% DHA of TFAs in transformant #8. These two strains were designated as strains Y5004U1 and Y5004U2, respectively (collectively, Y5004U).

Generation Of Strain Y5018 To Produce About 25.4% EPA, 11.4% DPA And 9.4% DHA Of TFAs

Construct pZKL3-4GER44 (FIG. 8B; SEQ ID NO:69) was constructed to integrate one $C_{20/22}$ elongase gene and three Δ4 desaturase genes into the lipase 3-like locus (GenBank Accession No. XP_506121) of strain Y5004U1. The pZKL3-4GER44 plasmid contained the following components:

Table 10: Components Of Plasmid pZKL3-4GER44 (SEQ ID NO:69)

| RE Sites And Nucleotides Within SEQ ID NO:69 | Description Of Fragment And Chimeric Gene Components |
|---|---|
| *Asc* I/*BsiW* I (10527-9640) | 887 bp 5' portion of the *Yarrowia* Lipase 3-like gene (GenBank Accession No. XP_506121) |
| *Pac I*/*Sph I* (14039-13235) | 804 bp 3' portion of *Yarrowia* Lipase 3-like gene (GenBank Accession No. XP_506121) |
| *Swa I*/*BsiW* I (7485-9640) | FBAINm::EgC20ES::Pex20, comprising:<br>• FBAINm: *Yarrowia lipolytica* FBAINm promoter (U.S. Patent 7,202,356);<br>• EgC20ES: codon-optimized C20 elongase gene (SEQ ID NO:63), derived from *Euglena gracilis* (U.S. Pat. Appl. Pub. No. 2008/0254191-A1);<br>• Pex20: Pex20 terminator sequence from *Yarrowia Pex20* gene (GenBank Accession No. AF054613) |
| *Pme*I/*Swa*I (4774-7485) | YAT1::EaD4S-1::Lip1, comprising:<br>• YAT1: *Yarrowia lipolytica* YAT1 promoter (U.S. Pat. Appl. Pub. No. 2006/0094102-A1);<br>• EaD4S-1: codon-optimized truncated Δ4 desaturase (SEQ ID NO:65), derived from *Euglena anabaena* (U.S. Pat. Appl. Pub. No. 2008/0254191-A1);<br>• Lip1: Lip1 terminator sequence from *Yarrowia Lip1* gene (GenBank Accession No. Z50020) |
| *Cla*I/*Pme*I (2070-4774) | EXP1::E1594D4S::Oct, comprising:<br>• EXP1: *Yarrowia lipolytica* export protein promoter (Intl. App. Pub. No. WO 2006/052870);<br>• E1594D4S: codon-optimized Δ4 desaturase (SEQ ID NO:70), derived from *Eutreptiella cf_gymnastica* CCMP1594 (U.S. Pat. Appl. Pub. No. 2009/0253188-A1) (labeled as "D4S-1594" in Figure);<br>• OCT: OCT terminator sequence of *Yarrowia OCT* gene (GenBank Accession No. X69988) |
| *Sal*I/*Eco*RI (1620-1) | *Yarrowia* Ura3 gene (GenBank Accession No. AJ306421) |

(continued)

| RE Sites And Nucleotides Within SEQ ID NO:69 | Description Of Fragment And Chimeric Gene Components |
|---|---|
| *Eco*RI/*Pac*I (1-14039) | GPDIN::EgD4S-1::Aco, comprising:<br>• GPDIN: *Yarrowia lipolytica* GPDIN promoter (U.S. Patent 7,459,546);<br>• EgD4S-1: codon-optimized truncated Δ4 desaturase (SEQ ID NO:72), derived from *Euglena gracilis* (U.S. Pat. Appl. Pub. No. 2008/0254191-A1);<br>• Aco: Aco terminator sequence from *Yarrowia Aco* gene (GenBank Accession No. AJ001300) |

The pZKL3-4GER44 plasmid was digested with *Asc*I/*Sph*I, and then used for transformation of strain Y5004U1, according to the General Methods. The transformants were selected on MM plates. After 5 days growth at 30 °C, 96 transformants grown on the MM plates were picked and re-streaked onto fresh MM plates. Once grown, these strains were individually inoculated into 3 mL liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced about 19% EPA, 22% DPA and 7% DHA of TFAs. Strain #1 produced 23.3% EPA, 13.7% DPA and 8.9% DHA, while strain #49 produced 25.2% EPA, 11.4% DPA and 9.4% DHA. These two strains were designated as Y5011 and Y5018, respectively.

Knockout of the lipase 3-like locus (GenBank Accession No. XP_506121) was not confirmed in strains Y5011 and Y5018.

Generation Of Strain Y5018U (*Ura3*-)

To disrupt the *Ura3* gene, construct pZKUM (FIG. 5A; SEQ ID NO:40; described in Table 15 of U.S. Pat. App. Pub. No. 2009-0093543-A1) was used to integrate a *Ura3* mutant gene into the *Ura3* gene of strain Y5018, in a manner similar to that described for pZKUM transformation of strain Y8006 (Example 1). A total of 18 transformants were grown and identified to possess a *Ura*- phenotype.

GC analyses showed the presence of 16.6% EPA, 10.4% DPA and 0.0% DHA of FAMEs in pZKUM-transformant strain #2 and 17.0% EPA, 10.8% DPA and 0.0% DHA of FAMEs in pZKUM-transformant strain #4. These two strains were designated as strains Y5018U1 and Y5018U2, respectively (collectively, Y5018U).

Generation Of Strain Y5037 To Produce About 18.6% EPA, 22.8% DPA And 9.7% DHA Of TFAs

Construct pZKLY-G20444 (FIG. 9; SEQ ID NO:74) was constructed to integrate one DHA synthase and two Δ4 desaturase genes into the lipase 7-like locus (GenBank Accession No. AJ549519) of strain Y5018U1. A DHA synthase is a multizyme comprising a C20 elongase linked to a Δ4 desaturase. The pZKLY-G20444 plasmid contained the following components:

Table 11: Components Of Plasmid pZKLY-G20444 (SEQ ID NO:74)

| RE Sites And Nucleotides Within SEQ ID NO:74 | Description Of Fragment And Chimeric Gene Components |
|---|---|
| *Asc*I/*BsiW*I (9370-8476) | 887 bp 5' portion of the *Yarrowia* Lipase 7-like gene (labeled as "LipY-5'" in Figure; GenBank Accession No. AJ549519) |
| *Pac*I/*Sph*I (12840-12078) | 756 bp 3' portion of *Yarrowia* Lipase 7-like gene (labeled as "LipY-3'" in Figure; GenBank Accession No. AJ549519) |

(continued)

| RE Sites And Nucleotides Within SEQ ID NO:74 | Description Of Fragment And Chimeric Gene Components |
|---|---|
| *Pme*I/*Swa*I (4871-8320) | YAT1::EgDHAsyn1S::Lip1, comprising:<br>• YAT1: *Yarrowia lipolytica* YAT1 promoter (U.S. Pat. Appl. Pub. No. 2006/0094102-A1);<br>• EgDHAsyn1S: codon-optimized DHA synthase (SEQ ID NO:75), derived from *Euglena gracilis* (labeled as "EgDHAase" in Figure; U.S. Pat. Appl. Pub. No. 2008/0254191-A1);<br>• Lip1: Lip1 terminator sequence from *Yarrowia Lip1* gene (GenBank Accession No. Z50020) |
| *Cla*I/*Pme*I (2070-4871) *Sal*I/*EcoR*I (1620-1) | EXP1::EaD4S-1::Pex16, comprising:<br>• EXP1: *Yarrowia lipolytica* export protein (EXP1) promoter (Intl. App. Pub. No. WO 2006/052870);<br>• EaD4S-1: codon-optimized truncated Δ4 desaturase (SEQ ID NO:65), derived from *Euglena anabaena* (U.S. Pat. Appl. Pub. No. 2008/0254191-A1);<br>• Pex16: Pex16 terminator sequence from *Yarrowia Pex16* gene (GenBank Accession No. U75433) |
| | *Yarrowia* Ura3 gene (GenBank Accession No. AJ306421) |
| *EcoR*I/*Pme*I (1-12871) | FBAINm::E1594D4S::Pex16, comprising:<br>• FBAINm: *Yarrowia lipolytica* FBAINm promoter (U.S. Patent 7,202,356);<br>• E1594D4S: codon-optimized Δ4 desaturase (SEQ ID NO:70), derived from *Eutreptiella cf_gymnastica* CCMP1594 (U.S. Pat. Appl. Pub. No. 2009/0253188-A1) (labeled as "D4S-1594" in Figure);<br>• Pex16: Pex16 terminator sequence from *Yarrowia Pex16* gene (GenBank Accession No. U75433) |

The pZKLY-G20444 plasmid was digested with *Asc*I/*Sph*I, and then used for transformation of strain Y5018U1, according to the General Methods. The transformants were selected on MM plates. After 5 days growth at 30 °C, 96 transformants grown on the MM plates were picked and re-streaked onto fresh MM plates. Once grown, these strains were individually inoculated into 3 mL liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced about 19% EPA, 22% DPA and 9% DHA of TFAs. Strain #3 produced 18.6% EPA, 22.8% DPA and 9.7% DHA; strain #9 produced 18.4% EPA, 21% DPA and 9.6% DHA; strain #27 produced 17.8% EPA, 20.6% DPA and 10% DHA; and strain #40 produced 18.8% EPA, 21.2% DPA and 9.6% DHA. These four strains were designated as Y5037, Y5038, Y5039 and Y5040, respectively.

Knockout of the lipase 7-like locus (GenBank Accession No, AJ549519) was not confirmed in these knocked out strains.

The final genotype of strains Y5037, Y5038, Y5039 and Y5040 with respect to wild type *Yarrowia lipolytica* ATCC #20362 was *SCP2-(YALI0E01298g), YALI0C18711g-, Pex10-, YALI0F24167g-, unknown 1-, unknown 3-, unknown 8-, unknown 9-, unknown10-, unknown 11-,* GPD::FmD12::Pex20, YAT1::FmD12::OCT, GPM/FBAIN::FmD12S::OCT, EXP1::FmD12S::Aco, YAT1::FmD12S::Lip2, YAT1::ME3S::Pex16, EXP1::ME3S::Pex20 (3 copies), GPAT::EgD9e::Lip2, EXP1::EgD9eS::Lip1, FBAINm::EgD9eS::Lip2, FBA::EgD9eS::Pex20, GPD::EgD9eS::Lip2, YAT1::EgD9eS::Lip2, YAT1 ::E389D9eS::OCT, FBAINm::EgD8M::Pex20, FBAIN::EgD8M::Lip1 (2 copies), EXP1::EgD8M::Pex16, GPDIN::EgD8M::Lip1, YAT1::EgD8M::Aco, FBAIN::EgD5::Aco, EXP1::EgD5S::Pex20, YAT1::EgD5S::Aco, EXP1 ::EgD5S::ACO, YAT1::RD5S::OCT, YAT1 ::PaD17S::Lip1, EXP1::PaD17::Pex16, FBAINm::PaD17::Aco, YAT1::YlCPT1::ACO, GPD::YlCPT1::ACO, FBAINm::EaC20ES::Pex20, YAT1::EgC20ES::Lip1, FBAINm::EgC20ES::Pex20, EXP1::EaD4S-1::Lip2, EXP1::EaD4S-1 ::Pex16, YAT1::EaD4S-1 ::Lip1, GPDIN::EaD4SB::Aco, EXP1::E1594D4S::Oct, FBAINm::E1594D4S::Pex16, GPDIN::EgD4S-1 ::Aco, YAT1 ::EgDHAsyn1S::Lip1.

Generation Of Strain Y5037U *(Ura3-)*

To disrupt the *Ura3* gene, construct pZKUM (FIG. 5A; SEQ ID NO:40; described in Table 15 of U.S. Pat. App. Pub. No. 2009-0093543-A1) was used to integrate a *Ura3* mutant gene into the *Ura3* gene of strain Y5037, in a manner similar to that described for pZKUM transformation of strain Y5004 (*supra*). A total of 12 transformants were grown and identified to possess a *Ura*- phenotype.

GC analyses showed the presence of 12.1% EPA, 10.2% DPA and 3.3% DHA in pZKUM-transformant strain #4 and 12.4% EPA, 10.3% DPA and 3.5% DHA in pZKUM-transformant strain #11. These two strains were designated as strains Y5037U1 and Y5037U2, respectively (collectively, Y5037U).

EXAMPLE 3

Construction Of Various Expression Vectors Comprising Different LPLAT ORFs

The present example describes the construction of a series of vectors, each comprising a LPLAT ORF, suitable for expression in *Yarrowia lipolytica.* LPLAT ORFs included the *Saccharomyces cerevisiae* Ale1, *Yarrowia lipolytica* Ale1, *Mortierella alpina* LPAAT1, *Yarrowia lipolytica* LPAAT1 and *Caenorhabditis elegans* LPCAT. Examples 4, 5 and 6 describe the results obtained following transformation of these vectors into *Yarrowia lipolytica.*

Origin Of LPLATs

A variety of LPLATs have been identified in the patent and open literature, but the functionality of these genes has not been previously directly compared. Table 12 summarizes publicly available LPLATs (i.e., ScAle1, ScLPAAT, MaLPAAT1 and CeLPCAT) and LPLAT orthologs identified herein (i.e., YlAle1 and YlLPAAT1) that are utilized in the Examples, following codon-optimization of heterologous genes for expression in *Yarrowia lipolytica* (*infra*).

Table 12: LPLATs Functionally Characterized

| LPLAT | Organism | ORF Designation | References | SEQ ID NO |
|---|---|---|---|---|
| Ale1 | *Saccharomyces cerevisiae* * | ORF "YOR175C" or "ScAle1" | GenBank Accession No. NP _014818; U.S. Pat. Appl. Pub. No. 20080145867 (and | 8, 9 |
| | | | corresponding to Intl. App. Pub. No. WO 2008/076377); Intl. App. Pub. No. WO 2009/001315 | |
| | *Yarrowia lipolytica* | "YALI0F19514p" or "YlAle1" | GenBank Accession No. XP_505624; Intl. App. Pub. No. WO 2009/001315 | 10, 11 |
| LPAAT | *Saccharomyces cerevisiae* | ORF "YDL052C" or "ScLPAAT" | GenBank Accession No. NP_010231 | 18 |
| | *Mortierella alpina* | "MaLPAAT1" | U.S. Pat. Appl. Pub. No. 2006-0115881-A1; U.S. Pat. Appl. Pub. No. 2009-0325265-A1 | 14, 15 |
| | *Yarrowia lipolytica* | "YALI0E18964g" or "YlLPAAT1" | GenBank Accession No. XP_504127; U.S. Patent 7,189,559 | 16, 17 |
| LPCAT | *Caenorhabditis elegans** | "clone T06E8.1" or "CeLPCAT" | GenBank Accession No. CAA98276; Intl. App. Pub. No. WO 2004/076617 (corresponding to U.S. Pat. Appl. Pub. No. 2006-0168687-A1) | 1, 2 |
| *The *Saccharomyces cerevisiae Ale1* and *Caenorhabditis elegans* LPCAT were used as comparative Examples. | | | | |

More specifically, the ScLPAAT (SEQ ID NO:18) and ScAle1 (SEQ ID NO:9) protein sequences were used as queries to identify orthologs from the public *Y. lipolytica* protein database of the "Yeast project *Genolevures"* (Center for Bioinformatics, LaBRI, Talence Cedex, France) (see also Dujon, B. et al., Nature, 430(6995):35-44 (2004)) using the Washington University in St. Louis School of Medicine BLAST 2.0 (WU-BLAST; http://blast.wustl.edu). Based on analysis of the best hits, the Ale1 and LPAAT orthologs from *Yarrowia lipolytica* are identified herein as YlAle1 (SEQ ID NO:11) and YlLPAAT (SEQ ID NO:17), respectively. The identiy of YlAle1 and YlLPAAT1 as orthologs of ScAle1 and ScLPAAT, respectively, was further confirmed by doing a reciprocal BLAST, i.e., using SEQ ID NOs:11 and 17 as a query against the *Saccharomyces cerevisiae* public protein database to find ScAle1 and ScLPAAT, repectively, as the best hits.

The LPLAT proteins identified above as ScAle1 (SEQ ID NO:9), YlAle1 (SEQ ID NO:11), ScLPAAT (SEQ ID NO:18), MaLPAAT1 (SEQ ID NO:15), YILPAAT1 (SEQ ID NO:17) and CeLPCAT (SEQ ID NO:2) were aligned using the method of Clustal W (slow, accurate, Gonnet option; Thompson et al., Nucleic Acids Res., 22:4673-4680 (1994)) of the MegAlign™ program (version 8.0.2) of the LASERGENE bioinformatics computing suite (DNASTAR, Inc., Madison, WI). This resulted in creation of Table 13, where percent similarity is shown in the upper triangle of the Table while percent divergence is shown in the lower triangle.

Table 13: Percent Identity And Divergence Among Various LPLATs

| YILPAAT1 | CeLPCAT | MaLPAAT1 | ScAle1 | ScLPAAT | YIAle1 | |
|---|---|---|---|---|---|---|
| -- | 26.6 | 34.0 | 9.6 | 43.9 | 11.7 | YILPAAT1 |
| 184.3 | -- | 36.4 | 11.3 | 32.4 | 14.5 | CeLPCAT |
| 137.5 | 126.4 | -- | 11.1 | 34.6 | 15.0 | MaLPAAT1 |
| 545.0 | 442.0 | 456.0 | -- | 13.5 | 45.0 | ScAle1 |
| 97.9 | 145.7 | 134.5 | 365.0 | -- | 15.6 | ScLPAAT |
| 426.0 | 339.0 | 330.0 | 94.3 | 317.0 | -- | YIAle1 |

The percent identities revealed by this method allowed determination of the minimum percent identity between each of the LPAAT polypeptides and the minimum percent identity between each of the Ale1 polypeptides. The range of identity between LPAAT polypeptides was 34.0% identity (MaLPAAT1 and YILPAAT1) to 43.9% identity (ScLPAAT and YILPAAT1), while identity between the ScAle1 and YIAle1 polypeptides was 45%.

Membrane Bound O-Acyltransferase ["MBOAT"] Family Motifs:

Orthologs of the ScAle1 protein sequence (SEQ ID NO:9) were identified by conducting a National Center for Biotechnology Information ["NCBI"] BLASTP 2.2.20 (protein-protein Basic Local Alignment Search Tool; Altschul et al., Nucleic Acids Res., 25:3389-3402 (1997); and Altschul et al., FEBS J., 272:5101-5109 (2005)) search using ScAle1 (SEQ ID NO:9) as the query sequence against fungal proteins in the "nr" protein database (comprising all non-redundant GenBank CDS translations, sequences derived from the 3-dimensional structure from Brookhaven Protein Data Bank ["PDB"], sequences included in the last major release of the SWISS-PROT protein sequence database, PIR and PRF excluding those environmental samples from WGS projects) using default parameters (expect threshold = 10; word size = 3; scoring parameters matrix = BLOSUM62; gap costs: existence = 11, extension = 1). The following hits were obtained:

Table 14: Fungal Orthologs Of ScAle1 (SEQ ID NO:9) Based On BLAST Analysis

| Gen Bank Acession No. | Species |
|---|---|
| NP_014818.1 | *Saccharomyces cerevisiae* |
| XP_001643411.1 | *Vanderwaltozyma polyspora* DSM 70294 |
| XP_448977.1 | *Candida glabrata* |
| XP_455985.1 | *Kluyveromyces lactis* |
| NP_986937.1 | *Ashbya gossypii* ATCC 10895 |
| XP_001385654.2 | *Pichia stipitis* CBS 6054 |
| XP_001487052.1 | *Pichia guilliermondii* ATCC 6260 |
| EDK36331.2 | *Pichia guilliermondii* ATCC 6260 |
| XP_001525914.1 | *Lodderomyces elongisporus* NRRL YB-4239 |
| XP_461358.1 | *Debaryomyces hansenii* CBS767 |
| XP_713184.1 | *Candida albicans* SC5314 |
| XP_001645053.1 | *Vanderwaltozyma polyspora* DSM 70294 |

(continued)

| Gen Bank Acession No. | Species |
|---|---|
| XP_505624.1 | *Yarrowia lipolytica* |
| XP_001805526.1 | *Phaeosphaeria nodorum* SN15 |
| XP_001598340.1 | *Sclerotinia sclerotiorum* 1980 |
| XP_001907785.1 | *Podospora anserine* |
| XP_001931658.1 | *Pyrenophora tritici-repentis* Pt-1C-BFP |
| XP_001560657.1 | *Botryotinia fuckeliana B05.10* |
| XP_963006.1 | *Neurospora crassa* OR74A |
| XP_364011.2 | *Magnaporthe grisea* 70-15 |
| XP_001209647.1 | *Aspergillus terreus* NIH2624 |
| XP_001822945.1 | *Aspergillus oryzae* RIB40 |
| XP_001257694.1 | *Neosartorya fischeri* NRRL 181 |
| XP_747591.2 | *Aspergillus fumigatus* Af293 |
| XP_001270060.1 | *Aspergillus clavatus* NRRL 1 |
| NP_596779.1 | *Schizosaccharomyces pombe* |
| XP_001396584.1 | *Aspergillus niger* |
| XP_001229385.1 | *Chaetomium globosum* CBS 148.51 |
| XP_001248887.1 | *Coccidioides immitis* RS |
| XP_664134.1 | *Aspergillus nidulans* FGSC A4 |
| XP_566668.1 | *Cryptococcus neoformans var. neoformans* JEC21 |
| XP_001839338.1 | *Coprinopsis cinerea okayama* 7#130 |
| XP_757554.1 | *Ustilago maydis* 521 |

The yeast and fungal protein sequences of Table 14 were aligned using DNASTAR. Multiple sequence alignments and percent identity calculations were performed using the Clustal W method of alignment (*supra*).

More specifically, default parameters for multiple protein alignment using the Clustal W method of alignment correspond to: GAP PENALTY=10, GAP LENGTH PENALTY=0.2, Delay Divergent Seqs(%)=30, DNA Transition Weight=0.5, Protein Weight Matrix=Gonnet Series, DNA Weight Matrix=IUB with the 'slow-accurate' option. The resulting alignment was analyzed to determine the presence or absence of the non-plant motifs for Ale1 homologs, as identified in U.S. Pat. Pub. No. 2008-0145867-A1. Specifically, these include: M-[V/I]-[L/I]-xxK-[L/V/I]-xxxxxxDG (SEQ ID NO:26), RxKYYxxWxxx-[E/D]-[A/G]xxxxGxG-[F/Y]-xG (SEQ ID NO:27), $EX_{11}WNX_2$-[T/V]-$X_2W$ (SEQ ID NO:28) and SAxWHGxxPGYxx-[T/F]-F (SEQ ID NO:29), wherein X encodes any amino acid residue. The His residue in SEQ ID NO:29 has been reported to be a likely active site residue within the protein.

Only one motif, i.e., $EX_{11}WNX_2$-[T/V]-$X_2W$ (SEQ ID NO:28), was completely conserved in all 33 of the organisms aligned. The remaining M-[V/I]-[L/I]-xxK-[L/V/I]-xxxxxxDG (SEQ ID NO:26), RxKYYxx-Wxxx-[E/D]-[A/G]xxxxGxG-[F/Y]-xG (SEQ ID NO:27) and SAxWHGxxPGYxx-[T/F]-F (SEQ ID NO:29) motifs were only partially conserved. Thus, these motifs were appropriately truncated to fit with 0 mismatch (i.e., SAxWHG [SEQ ID NO:5]), 1 mismatch (i.e., RxKYYxxW [SEQ ID NO:4]), or 2 mismatches (i.e., M(V/I)(L/I)xxK(LVI) [SEQ ID NO:3]) for the purposes of the present methodologies.

1-Acyl-*sn*-Glycerol-3-Phosphate Acyltransferase ["LPAAT"] Family Motifs*:* Analysis of the protein alignment comprising ScLPAAT (SEQ ID NO:18), MaLPAAT1 (SEQ ID NO:15) and YlLPAAT1 (SEQ ID NO:17) revealed that the 1-acyl-*sn*-glycerol-3-phosphate acyltransferase family motif EGTR (SEQ ID NO:20) was present in each of the LPAAT orthologs. On this basis, MaLPAAT1 was identified as a likely LPAAT, that was clearly distinguishable from the Ma LPAAT-like proteins disclosed in Intl. App. Pub. No. WO 2004/087902 (i.e., SEQ ID NOs:93 and 95).

It is noteworthy that the EGTR (SEQ ID NO:20) motif, while lacking in the LPCAT sequences in Intl. App. Pub. No. WO 2004/087902, is present in CeLPCAT (SEQ ID NO:2). It appears that other residues distinguish LPAAT and LPCAT sequences in LPAAT-like proteins. One such residue could be the extension of the EGTR (SEQ ID NO:20) motif. Specifically, whereas the EGTR motif in ScLPAAT (SEQ ID NO:18), MaLPAAT1 (SEQ ID NO:15) and YILPAAT1 (SEQ ID NO:17) is immediately followed by a serine residue, the EGTR motif in CeLPCAT is immediately followed by an asparagine residue. In contrast, the two LPCATs in Intl. App. Pub. No. WO 2004/087902 have a valine substituted for the arginine residue in the EGTR motif and the motif is immediately followed by a valine residue.

Construction Of pY201, Comprising A Codon-Optimized *Saccharomyces cerevisiae* Ale1 Gene

The *Saccharomyces cerevisiae* ORF designated as "ScAle1" (SEQ ID NO:8) was optimized for expression in *Yarrowia lipolytica,* by DNA 2.0 (Menlo Park, CA). In addition to codon optimization, *5' Pci*1 and 3' *Not*1 cloning sites were introduced within the synthetic gene (i.e., ScAle1S; SEQ ID NO:12). None of the modifications in the ScAle1S gene changed the amino acid sequence of the encoded protein (i.e., the protein sequence encoded by the codon-optimized gene [i.e., SEQ ID NO:13] is identical to that of the wildtype protein sequence [i.e., SEQ ID NO:9]). ScAle1 S was cloned into pJ201 (DNA 2.0) to result in pJ201:ScAle1S.

A 1863 bp *Pci*1/*Not*1 fragment comprising ScAle1S was excised from pJ201:ScAle1S and used to create pY201 (SEQ ID NO:77; Table 15; FIG. 10A). In addition to comprising a chimeric YAT1::ScAle1S::Lip1 gene, pY201 also contains a *Yarrowia lipolytica* URA3 selection marker flanked by LoxP sites for subsequent removal, if needed, by Cre recombinase-mediated recombination. Both the YAT1::ScAle1S::Lip1 chimeric gene and the URA3 gene were flanked by fragments having homology to 5' and 3' regions of the *Yarrowia lipolytica* Pox3 gene to facilitate integration by double homologous recombination, although integration into *Yarrowia lipolytica* is known to usually occur without homologous recombination. Thus, construct pY201 thereby contained the following components:

Table 15: Description of Plasmid pY201 (SEQ ID NO:77)

| RE Sites And Nucleotides Within SEQ ID NO:77 | Description Of Fragment And Chimeric Gene Components |
|---|---|
| *Bsi*W1/*Sbf*1<br>(1-1706 bp) | LoxP::*Ura3*::LoxP, comprising:<br>• LoxP sequence (SEQ ID NO:78)<br>• *Yarrowia lipolytica* Ura3 gene (GenBank Accession No. AJ306421);<br>• LoxP sequence (SEQ ID NO:78) |
| *Sbf*1/*Sph*1<br>(1706-3043 bp) | 3' portion of *Yarrowia lipolytica* POX3 Acyl-CoA oxidase 3 (GenBank Accession No. YALI0D24750g) (i.e., bp 2215-3038 in pY201) |
| *Sph*1/*Asc*1<br>(3043-5743 bp) | • *ColE1* plasmid origin of replication;<br>• Ampicillin-resistance gene ($Amp^R$) for selection in *E. coli* (i.e., bp 3598-4758 [complementary] in pY201);<br>• *E. coli* f1 origin of replication |
| *Asc*I/*Bsi*WI (5743-6513 bp) | 5' portion of *Yarrowia lipolytica* POX3 Acyl-CoA oxidase 3 (GenBank Accession No. YALI0D24750g) (i.e., bp 5743-6512 in pY201) |
| *Bsi*WI/ *Bsi*WI<br>(6514-1 bp)<br><br>[a *Not*1 site, located between ScAle1S and Lip1 is present at bp 9154 bp] | YAT1::ScAle1S::Lip1, comprising:<br>• YAT1: *Yarrowia lipolytica* YAT1 promoter (U.S. Pat. Appl. Pub. No. 2006/0094102-A1) (i.e., bp 6514-7291 in pY201)<br>• ScAle1S: codon-optimized Ale1 (SEQ ID NO:12) derived from *Saccharomyces cerevisiae* YOR175C (i.e., bp 7292-9151 in pY201; labeled as "Sc LPCATs ORF" in Figure);<br>• Lip1: Lip1 terminator sequence from *Yarrowia Lip1* gene (GenBank Accession No. Z50020) (i.e., bp 9160-9481 pY201; labeled as "Lip1-3"' in Figure) |

Construction Of pY168, Comprising A *Yarrowia lipolytica* Ale1 Gene

The *Yarrowia lipolytica* ORF designated as "YlAle1" (GenBank Accession No. XP_505624; SEQ ID NO:10) was amplified by PCR from *Yarrowia lipolytica* ATCC #20362 cDNA library using PCR primers 798 and 799 (SEQ ID NOs:79 and 80, respectively). Additionally, the YAT promoter was amplified by PCR primers 800 and 801 (SEQ ID NOs:81 and 82, respectively) from pY201 (SEQ ID NO:77). Since the primer pairs were designed to create two PCR products having some overlap with one another, a YAT1 ::YlAle1 fusion fragment was then amplified by overlapping PCR using primers 798 and 801 (SEQ ID NOs:79 and 82, respectively) and the two PCR fragments as template. The PCR was carried out in a RoboCycler Gradient 40 PCR machine (Stratagene) using the manufacturer's recommendations and Pfu Ultra™ High-Fidelity DNA Polymerase (Stratagene, Cat. No. 600380). Amplification was carried out as follows: initial denaturation at 95 °C for 4 min, followed by 30 cycles of denaturation at 95 °C for 30 sec, annealing at 55 °C for 1 min, and elongation at 72 °C for 1 min. A final elongation cycle at 72 °C for 10 min was carried out, followed by reaction termination at 4 °C.

The PCR product comprising the YAT1::Yl Ale1 fusion fragment was gel purified and digested with *Cla*I/*Not*I. This *Cla*1-*Not*1 fragment was ligated into pY201 that had been similarly digested (thereby removing the YAT1::ScAle1S fragment) to create pY168 (SEQ ID NO:83), comprising a chimeric YAT1::YlAle1 ::Lip1 gene. The DNA sequence of the *Yarrowia* Ale1 ORF was confirmed by DNA sequencing. The components present in pY168 (FIG. 10B; SEQ ID NO:83) are identical to those present in pY201, with the exception of the YAT1::YlAle1 ::Lip1 gene in pY168, instead of the YAT1::ScAle1 S::Lip1 gene in pY201 (FIG. 10A). Note that YlAle1 is labeled as "Yl LPCAT" in FIG. 10B.

Construction Of pY208, Comprising A *Mortierella alpina* LPAAT1 Gene

The *Mortierella alpina* ORF designated as "MaLPAAT1" (SEQ ID NO:14) was optimized for expression in *Yarrowia lipolytica,* by DNA 2.0 (Menlo Park, CA). In addition to codon optimization, 5' *Pci*1 and 3' *Not*1 cloning sites were introduced within the synthetic gene (i.e., MaLPAAT1S; SEQ ID NO:21). None of the modifications in the MaLPAAT1S gene changed the amino acid sequence of the encoded protein (i.e., the protein sequence encoded by the codon-optimized gene [i.e., SEQ ID NO:22] is identical to that of the wildtype protein sequence [i.e., SEQ ID NO:15]). MaLPAAT1S was cloned into pJ201 (DNA 2.0) to result in pJ201:MaLPAAT1S.

A 945 bp *Pci*1/*Not*1 fragment comprising MaLPAAT1S was excised from pJ201:MaLPAAT1S and used to create pY208 (SEQ ID NO:84), in a 3-way ligation with two fragments of pY201 (SEQ ID NO:77). Specifically, the MaLPAAT1 fragment was ligated with a 3530 bp *Sph*-*Not*I pY201 fragment and a 4248 bp *Nco*I-*Sph*I pY201 fragment to result in pY208. The components present in pY208 (FIG. 11A; SEQ ID NO:84) are identical to those present in pY201, with the exception of the YAT1::MaLPAAT1S::Lip1 gene in pY208, instead of the YAT1::Sc Ale1S::Lip1 gene in pY201 (FIG. 10A).

Construction Of pY207, Comprising A *Yarrowia lipolytica* LPAAT1 Gene

A putative LPAAT1 from *Yarrowia lipolytica* (designated herein as "YlLPAAT1"; SEQ ID NO:17) was described in U.S. Patent 7,189,559 and GenBank Accession No. XP_504127. The protein is annotated as "similar to uniprot|P33333 *Saccharomyces cerevisiae* YDL052c SLC1 fatty acyltransferase".

The YlLPAAT1 ORF (SEQ ID NO:16) was amplified by PCR using *Yarrowia lipolytica* ATCC #20362 cDNA library as a template and PCR primers 856 and 857 (SEQ ID NOs:85 and 86, respectively). The PCR was conducted using the same components and conditions as described above for amplification of the YAT1::Yl Ale1 fusion fragment, prior to synthesis of pY168.

The PCR product comprising YlLPAAT1 ORF was digested with *Pci*I and *Not*I and then utilized in a 3-way ligation with two fragments from pY168. Specifically, the YlLPAAT1 fragment was ligated with a 3530 bp *Sph*-*Not*I pY168 fragment and a 4248 bp *Nco*I-*Sph*I pY168 fragment, to produce pY207, comprising a chimeric YAT1::YlLPAAT1 ::Lip1 gene. The *Y. lipolytica* LPAAT1 ORF was confirmed by DNA sequencing. The components present in pY207 (FIG. 11B; SEQ ID NO:87) are identical to those present in pY201, with the exception of the chimeric YAT1::Yl LPAAT1::Lip1 gene in pY207, instead of the YAT1::ScAle1 S::Lip1 gene in pY201 (FIG. 10A). Note that YlLPAAT1 is labeled as "Yl LPAT1 ORF" in FIG. 11B.

Construction Of pY175, Comprising A *Caenorhabditis elegans* LPCAT Gene

The *Caenorhabditis elegans* ORF designated as "CeLPCAT" (SEQ ID NO:1) was optimized for expression in *Yarrowia lipolytica,* by GenScript Corporation (Piscataway, NJ). In addition to codon optimization, 5' *Nco*1 and 3' *Not*1 cloning sites were introduced within the synthetic gene (i.e., CeLPCATS; SEQ ID NO:6). None of the modifications in the CeLPCATS gene changed the amino acid sequence of the encoded protein (i.e., the protein sequence encoded by the codon-optimized gene [i.e., SEQ ID NO:7] is identical to that of the wildtype protein sequence [i.e., SEQ ID NO:2]).

A *Nco*1-*Not*1 fragment comprising CeLPCATS was used to create pY175 (SEQ ID NO:88), in a 3-way ligation with two fragments from pY168 (SEQ ID NO:83). Specifically, the *Nco*1-*Not*1 fragment comprising CeLPCATS was ligated with a 3530 bp *Sph*-*Not*I pY168 fragment and a 4248 bp *Nco*I-*Sph*I pY168 fragment to result in pY175. The components present in pY175 (FIG. 12A; SEQ ID NO:88) are identical to those present in pY201, with the exception of the YAT1::CeLPCATS::Lip1 gene in pY175, instead of the YAT1::ScAle1S::Lip1 gene in pY201 (FIG. 10A). Note that CeLPCATS is labeled as "Ce.LPCATsyn" in FIG. 12A.

Construction Of pY153, Comprising A *Caenorhabditis elegans* LPCAT Gene

The *Nco*1-*Not*1 fragment comprising CeLPCATS, *supra, was* used to create pY153 (SEQ ID NO:89; FIG. 12B). In addition to comprising a chimeric FBAIN::CeLPCATS::3' Yl LPAAT1 gene, pY153 also contains a *Yarrowia lipolytica* URA3 selection marker. Both the chimeric FBAIN::CeLPCATS::3' Yl LPAAT1 gene and the URA3 gene were flanked by fragments having homology to 5' and 3' regions of the *Yarrowia lipolytica* LPAAT1 gene to facilitate integration by double homologous recombination, although integration into *Yarrowia lipolytica* is known to usually occur without homologous recombination. Thus, construct pY153 thereby contained the following components:

Table 16: Description of Plasmid pY153 (SEQ ID NO:89)

| RE Sites And Nucleotides Within SEQ ID NO: 89 | Description Of Fragment And Chimeric Gene Components |
|---|---|
| *Cla*1/*Sap*1<br>(1-1398 bp) | 5' portion of *Yarrowia lipolytica* gene encoding LPAAT1 (GenBank Accession No. XP_504127) (i.e., bp 1-1112 [complementary] in pY153); |
| *Sap*1/*Xba*1<br>(1398-3993 bp) | Vector backbone including:<br>• *ColE1* plasmid origin of replication (i.e., bp 1380-2260 in pY153);<br>• Ampicillin-resistance gene ($Amp^R$) for selection in *E. coli* (i.e., bp 2330-3190 [complementary] in |
| | pY153);<br>• *E. coli* f1 origin of replication (i.e., bp 3370-3770 in pY153) |
| *Xba*1/ *Pme*1<br>(3993-6719 bp)<br><br>[a*Nco*1site, located between CeLPCATS and FBAIN is present at bp 5756; a *Not*1 site, located between CeLPCATS and YILPAAT1 is present at bp 4904] | FBAIN::CeLPCATS::3' Yl LPAAT1, comprising:<br>• FBAINm: *Yarrowia lipolytica* FBAIN promoter (U.S. Patent 7,202,356) (i.e., bp 5756-6719 [complementary] in pY153);<br>• CeLPCATS: codon-optimized LPCAT (SEQ ID NO:6) derived from *Caenorhabditis elegans* T06E8.1 (GenBank Accession No. CAA98276) (i.e., bp 4910-5758 [complementary] in pY153; labeled as "Ce.LPCATsyn" in Figure);<br>• 3' Yl LPAAT1: 3' untranslated portion of *Yarrowia lipolytica* gene encoding LPAAT1 (GenBank Accession No. XP_504127) (i.e., bp 3987-4905 [complementary] in pY153) |
| *Pme*1-*Cla*I<br>(6719-1 bp) | *Yarrowia lipolytica* URA3 gene (GenBank Accession No. AJ306421) (i.e., bp 6729-1 [complementary] in pY153) |

EXAMPLE 4

Functional Characterization Of Different LPLATs In EPA-Producing *Yarrowia lipolytica* Strain Y8406

*Yarrowia lipolytica* strain Y8406U, producing EPA, was used to functionally characterize the effects of overexpression of the *Saccharomyces cerevisiae* Ale1, *Yarrowia lipolytica* Ale1, *Mortierella alpina* LPAAT1, *Yarrowia lipolytica* LPAAT1 and *Caenorhabditis elegans* LPCAT, following their stable integration into the *Yarrowia* host chromosome. This was in spite of the host containing its native LPLATs, i.e., Ale1 and LPAAT1.

Transformation And Growth

*Yarrowia lipolytica* strain Y8406U (Example 1) was individually transformed with linear *Sph*I-*Asc*I fragments of the integrating vectors described in Example 3, wherein each LPLAT was under the control of the *Yarrowia* YAT promoter. Specifically, vectors pY201 (YAT1::ScAle1S::Lip1), pY168 (YAT1::YlAle1::Lip1), pY208 (YAT1::MaLPAAT1S::Lip1), pY207 (YAT1::YlLPAAT1::Lip1) and pY175 (YAT1::CeLPCATS::Lip1) were transformed according to the General Methods.

Each transformation mix was plated on MM agar plates. Several resultant URA+ transformants were picked and inoculated into 3 mL FM medium (Biomyx Cat. No. CM-6681, Biomyx Technology, San Diego, CA) containing per L: 6.7 g Difco Yeast Nitrogen Base without amino acids, 5 g Yeast Extract, 6 g $KH_2PO_4$, 2 g $K_2HPO_4$, 1.5 g $MgSO_4 \cdot 7H_20$, 1.5 mg thiamine·HCl, and 20 g glucose. After 2 days growth on a shaker at 200 rpm and 30 °C, the cultures were harvested by centrifugation and resuspended in 3 mL HGM medium (Cat. No. 2G2080, Teknova Inc., Hollister, CA) containing 0.63% monopotassium phosphate, 2.7% dipotassium phosphate, 8.0% glucose, adjusted to pH 7.5. After 5 days growth on a shaker at 200 rpm and at 30 °C, 1 mL aliquots of the cultures were harvested by centrifugation and analyzed by GC. Specifically, the cultured cells were collected by centrifugation for 1 min at 13,000 rpm, total lipids were extracted, and fatty acid methyl esters ["FAMEs"] were prepared by trans-esterification, and subsequently analyzed with a Hewlett-Packard 6890 GC (General Methods).

Based on the fatty acid composition of the 3 mL cultures, selected transformants were further characterized by flask assay. Specifically, clones #5 and #11 of strain Y8406U transformed with expression vector pY201 (comprising ScAle1S) were selected and designated as "Y8406U::ScAle1 S-5" and "Y8406U::ScAle1 S-11", respectively; clone #16 of strain Y8406U transformed with expression vector pY168 (comprising YlAle1) was selected and designated as "Y8406U::YlAle1"; clone #8 of strain Y8406U transformed with expression vector pY208 (comprising MaLPAAT1S) was selected and designated as "Y8406U::MaLPAAT1S"; clone #21 of strain Y8406U transformed with expression vector pY207 (comprising YlLPAAT1) was selected and designated as "Y8406U::YlLPAAT1"; and clone #23 of strain Y8406U transformed with expression vector pY175 (comprising CeLPCATS) was selected and designated as "Y8406U::CeLPCATS". Additionally, strain Y8406 (a Ura+ strain that was parent to strain Y8406U (Ura-)) was used as a control.

Each selected transformant and the control was streaked onto MM agar plates. Then, one loop of freshly streaked cells was inoculated into 3 mL FM medium and grown overnight at 250 rpm and 30 °C. The $OD_{600nm}$ was measured and an aliquot of the cells were added to a final $OD_{600nm}$ of 0.3 in 25 mL FM medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for GC analysis (*supra*) and 10 mL dried for dry cell weight ["DCW"] determination.

For DCW determination, 10 mL culture was harvested by centrifugation for 5 min at 4000 rpm in a Beckman GH-3.8 rotor in a Beckman GS-6R centrifuge. The pellet was resuspended in 25 mL of water and re-harvested as above. The washed pellet was re-suspended in 20 mL of water and transferred to a pre-weighed aluminum pan. The cell suspension was dried overnight in a vacuum oven at 80 °C. The weight of the cells was determined.

<u>Lipid Content. Fatty Acid Composition And Conversion Efficiencies</u>

A total of four separate experiments were conducted under identical conditions. Experiment 1 compared control strain Y8406 versus strain Y8406U::ScAle1S-5. Experiment 2 compared control strain Y8406 versus strain Y8406U::YlAle1. Experiment 3 compared control strain Y8406 versus strain Y8406U::YlAle1, strain Y8406U::ScAle1S-11, and strain Y8406U::MaLPAAT1S. Experiment 4 compared control strain Y8406 versus strain Y8406U::MaLPAAT1S, strain Y8406U::YlLPAAT1 and strain Y8406U::CeLPCATS.

In each experiment, the lipid content, fatty acid composition and EPA as a percent of the DCW are quantified for 1, 2 or 3 replicate cultures ["Replicates"] of the control Y8406 strain and the transformant Y8406U strain(s). Additionally, data for each Y8406U transformant is presented as a % of the Y8406 control. Table 17 below summarizes the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA and EPA.

Table 18 summarizes the conversion efficiency of each desaturase and the Δ9 elongase functioning in the PUFA biosynthetic pathway and which are required for EPA production. Specifically, the Δ12 desaturase conversion efficiency ["Δ12 CE"], Δ8 desaturase conversion efficiency ["Δ8 CE"], Δ5 desaturase conversion efficiency ["Δ5 CE"], Δ17 desaturase conversion efficiency ["Δ17 CE"] and Δ9 elongation conversion efficiency ["Δ9e CE"] are provided for each control Y8406 strain and the transformant Y8406U strain(s); data for each Y8406U transformant is presented as a % of the Y8406 control. Conversion efficiency was calculated according to the formula:

$$product(s)/(product(s)+substrate)*100,$$

where product includes both product and product derivatives.

Table 17: Lipid Content And Composition In LPCAT Transformant Strains Of *Yarrowia lipolytica* Y8406

| Expt. | Strain | Repli cates | TFA % DCW | % TFAs | | | | | | | | | | | | EPA % DCW |
|---|---|---|---|---|---|---|---|---|---|---|---|---|---|---|---|---|
| | | | | 16: 0 | 16: 1 | 18: 0 | 18: 1 | 18:2 | ALA | EDA | DGLA | ARA | ERA | ETA | EPA | |
| 1 | Y8406 | AVG.3 | 17.6 | 3.8 | 0.7 | 3.3 | 6.4 | 22.6 | 2.5 | 2.8 | 2.2 | 0.5 | 1.9 | 2.0 | 48.9 | 8.6 |
| | Y8406U:: ScAle1S-5 | AVG.3 | 18.3 | 4.2 | 0.7 | 3.5 | 5.7 | 15.1 | 0.6 | 3.3 | 3.7 | 0.8 | 1.8 | 2.3 | 56.9 | 10.4 |
| | | % Ctrl | 104 | 111 | 100 | 106 | 89 | 67 | 24 | 118 | 168 | 160 | 95 | 115 | 116 | 121 |
| 2 | Y8406 | AVG.3 | 23.2 | 3.5 | 0.6 | 3.3 | 6.4 | 22.3 | 2.7 | 2.6 | 2.1 | 0.5 | 1.6 | 2.0 | 49.9 | 11.6 |
| | Y8406U:: YlAle1 | AVG.3 | 22.3 | 3.8 | 0.7 | 2.9 | 3.9 | 12.7 | 0.4 | 3.0 | 3.8 | 0.8 | 1.6 | 2.4 | 60.9 | 13.6 |
| | | % Ctrl | 96 | 109 | 117 | 88 | 61 | 57 | 15 | 115 | 181 | 160 | 100 | 120 | 122 | 117 |
| 3 | Y8406 | 1 | 26.1 | 2.7 | 0.7 | 2.8 | 6.5 | 20.5 | 2.5 | 3.2 | 2.3 | 0.7 | 0.8 | 0.0 | 50.8 | 13.3 |
| | Y8406U:: YlAle1 | AVG.2 | 23.3 | 3.3 | 0.7 | 2.4 | 3.6 | 12.1 | 0.5 | 3.2 | 3.5 | 0.9 | 0.0 | 2.3 | 62.2 | 14.5 |
| | | % Ctrl | 89 | 122 | 100 | 86 | 55 | 59 | 20 | 100 | 152 | 129 | 0 | na | 122 | 109 |
| | Y8406U:: ScAle1S-11 | AVG.2 | 28.0 | 3.0 | 0.7 | 3.0 | 5.5 | 13.1 | 0.6 | 3.5 | 3.8 | 0.9 | 0.0 | 2.4 | 58.5 | 16.4 |
| | | % Ctrl | 107 | 111 | 100 | 107 | 85 | 64 | 24 | 109 | 165 | 129 | 0 | na | 115 | 123 |
| | Y8406U:: MaLPAAT1S | AVG.2 | 23.7 | 4.4 | 0.8 | 4.2 | 6.6 | 11.2 | 0.7 | 2.7 | 3.7 | 0.9 | 0.0 | 2.5 | 57.0 | 13.5 |
| | | % Ctrl | 91 | 163 | 114 | 150 | 102 | 55 | 28 | 84 | 161 | 129 | 0 | na | 112 | 102 |
| 4 | Y8406 | AVG.2 | 27.9 | 2.8 | 0.6 | 3.1 | 6.2 | 20.6 | 2.9 | 2.9 | 2.0 | 0.6 | 0.7 | 2.0 | 49.4 | 13.8 |
| | Y8406U:: MaLPAAT1S | AVG.2 | 25.2 | 4.8 | 0.8 | 4.8 | 6.9 | 11.6 | 0.8 | 2.5 | 3.0 | 0.7 | 0.0 | 2.3 | 55.3 | 14.0 |
| | | % Ctrl | 90 | 171 | 133 | 155 | 111 | 56 | 28 | 86 | 150 | 117 | 0 | 115 | 112 | 101 |
| | Y8406U:: YlLPAAT1 | AVG.2 | 25.2 | 3.7 | 0.7 | 4.2 | 6.2 | 13.0 | 1.2 | 2.3 | 2.6 | 0.6 | 0.0 | 2.2 | 56.7 | 14.3 |
| | | % Ctrl | 90 | 132 | 117 | 135 | 100 | 63 | 41 | 79 | 130 | 100 | 0 | 110 | 115 | 104 |
| | Y8406U:: CeLPCATS | AVG.2 | 24.7 | 3.8 | 0.6 | 4.6 | 7.1 | 13.9 | 1.6 | 2.3 | 2.6 | 0.6 | 0.4 | 2.2 | 53.6 | 13.2 |
| | | % Ctrl | 89 | 136 | 100 | 148 | 115 | 67 | 55 | 79 | 130 | 100 | 57 | 110 | 109 | 96 |

Table 18: Desaturase And Elongase Conversion Efficiency In LPCAT Transformant Strains Of *Yarrowia lipolytica* Y8406

| Expt. | Strain | Replicates | Δ12 CE | Δ9e CE | Δ8 CE | Δ5 CE | Δ17 CE |
|---|---|---|---|---|---|---|---|
| 1 | Y8406 | AVG.3 | 93 | 70 | 92 | 92 | 90 |
| | Y8406U::ScAle1S-5 | AVG.3 | 94 | 81 | 93 | 91 | 89 |
| | | % Ctrl | 101 | 116 | 101 | 98 | 98 |
| 2 | Y8406 | AVG.3 | 93 | 70 | 93 | 93 | 91 |
| | Y8406U::YlAle1 | AVG.3 | 96 | 85 | 94 | 91 | 90 |
| | | % Ctrl | 103 | 121 | 101 | 98 | 98 |
| 3 | Y8406 | 1 | 93 | 72 | 93 | 96 | 89 |
| | Y8406U::YlAle1 | AVG.2 | 96 | 85 | 96 | 92 | 89 |
| | | % Ctrl | 104 | 119 | 103 | 96 | 100 |
| | Y8406U::ScAle1S-11 | AVG.2 | 94 | 83 | 95 | 91 | 88 |
| | | % Ctrl | 101 | 117 | 102 | 95 | 99 |
| | Y8406U::MaLPAAT1S | AVG.2 | 92 | 85 | 96 | 90 | 89 |
| | | % Ctrl | 100 | 119 | 103 | 94 | 100 |
| 4 | Y8406 | AVG.2 | 93 | 71 | 94 | 93 | 91 |
| | Y8406U::MaLPAAT1S | AVG.2 | 92 | 84 | 96 | 91 | 90 |
| | | % Ctrl | 99 | 118 | 102 | 99 | 100 |
| | Y8406U::YILPAAT1 | AVG.2 | 93 | 82 | 96 | 92 | 92 |
| | | % Ctrl | 100 | 115 | 103 | 100 | 101 |
| | Y8406U::CeLPCATS | AVG.2 | 92 | 80 | 96 | 92 | 91 |
| | | % Ctrl | 99 | 113 | 102 | 99 | 100 |

Based on the data concerning Experiments 1, 2 and 3 in Table 17 and Table 18, overexpression of LPLAT in EPA strains Y8406U::ScAle1S-5, Y8406U::ScAle1S-11, Y8406U::YlAle1 and Y8406U::MaLPAAT1S results in significant reduction (to 67% or below of the control) of the concentration of LA (18:2) as a weight % of TFAs ["LA % TFAs"], an increase (to at least 12% of the control) in the concentration of EPA as a weight % of TFAs ["EPA % TFAs"], and an increase (to at least 16% of the control) in the conversion efficiency of the Δ9 elongase. Compared to Y8406U::ScAle1 S-5 and Y8406U::ScAle1S-11, Y8406U::YlAle1 has lower LA % TFAs, higher EPA % TFAs, better Δ9 elongation conversion efficiency, and slightly lower TFAs % DCW and EPA % DCW. Y8406U::Yl Ale1 and Y8406U::MaLPAAT1S are similar except overexpression of MaLPAAT1S resulted in lower LA % TFAs, EPA % TFAs, and EPA % DCW.

Experiment 4 shows that overexpression of LPLAT in EPA strains Y8406U::YILPAAT1, Y8406U::MaLPAAT1S and Y8406U::CeLPCATS results in significant reduction (to 67% or below of the control) of LA % TFAs, an increase (to at least 9% of the control) in EPA % TFAs, and an increase (to at least 13% of the control) in the conversion efficiency of the Δ9 elongase. Compared to Y8406U::CeLPCATS, Y8406U::YILPAAT1 and Y8406U::MaLPAAT1S both have lower LA % TFAs, higher EPA % TFAs, higher EPA % DCW, and slightly better TFAs % DCW. Y8406U::YILPAAT1 and Y8406U::MaLPAAT1S are similar except overexpression of MaLPAAT1S results in lower LA % TFAs, slightly lower EPA % TFAs and EPA % DCW, and slightly better Δ9 elongase conversion efficiency.

It is well known in the art that most desaturations occur at the sn-2 position of phospholipids, while fatty acid elongations occur on acyl-CoAs. Furthermore, ScAle1 S, YlAle1, MaLPAAT1S and YILPAAT1 were expected to only incorporate acyl groups from the acyl-CoA pool into the sn-2 position of lysophospholipids, such as lysophosphatidic acid ["LPA"] and lysophosphatidylcholine ["LPC"]. Thus, it was expected that expression of ScAle1S, YlAle1, MaLPAATIS, and YILPAAT1 would result in improved desaturations due to improved substrate availability in phospholipids, and not result in improved elongations that require improved substrate availability in the CoA pool. Our data (*supra*) shows that unexpectedly, expression of ScAle1 S, YIAle1, MaLPAAT1S, and YILPAAT1 significantly improved the Δ9 elongase conversion efficiency in strains of *Yarrowia* producing EPA but did not improve the desaturations (measured as Δ12 desaturase conversion efficiency, Δ8 desaturase conversion efficiency, Δ5 desaturase conversion efficiency or Δ17 desaturase conversion efficiency).

CeLPCAT was previously shown to improve Δ6 elongation conversion efficiency in *Saccharomyces cerevisiae* fed LA or GLA (Intl. App. Pub. No. WO 2004/076617). This was attributed to its reversible LPCAT activity that released fatty acids from phospholipids into the CoA pool. An improvement in Δ9 elongation conversion efficiency in an oleaginous microbe, such as *Yarrowia lipolytica,* engineered for high level LC-PUFA production in the absence of feeding fatty acids was not contemplated in Intl. App. Pub. No. WO 2004/076617.

Futhermore, expression of ScAle1S, YlAle1, MaLPAAT1S, YlLPAAT1 and CeLPCATS did not significantly alter either the level of PUFAs accumulated or the total lipid content in strains of *Yarrowia* producing EPA.

Previous studies have shown that both Δ6 elongation and Δ9 elongation are bottlenecks in long chain PUFA biosynthesis due to poor transfer of acyl groups between phospholipid and acyl-CoA pools. Based on the improved Δ9 elongase conversion efficiency resulting from over-expression of LPLATs, demonstrated above, it is anticipated that the LPLATs described herein and their orthologs, such as Sc LPAAT, will also improve Δ6 elongation conversion efficiency.

## EXAMPLE 5

### Functional Characterization Of Different LPLATs In DHA-Producing Y. *lipolytica* Strain Y5037

*Yarrowia lipolytica* strain Y5037U, producing DHA, was used to functionally characterize the effects of overexpression of the *Saccharomyces cerevisiae* Ale1, *Mortierella alpina* LPAAT1 and *Caenorhabditis elegans* LPCAT, following their stable integration into the *Yarrowia* host chromosome. This was in spite of the host containing its native LPLATs, i.e., Ale1 and LPAAT1.

### Transformation And Growth

*Yarrowia lipolytica* strain Y5037U (Example 2) was individually transformed with linear *Sph*I-*Asc*I fragments of the integrating vectors described in Example 3, wherein ScAle1 S and MaLPAAT1S were under the control of the *Yarrowia* YAT promoter, while CeLPCATS was under the control of the *Yarrowia* FBAIN promoter. Specifically, vectors pY201 (YAT1::ScAle1S::Lip1), pY208 (YAT1::MaLPAAT1S::Lip1) and pY153 (FBAIN::CeLPCATS::YlLPAAT1) were transformed according to the General Methods.

Each transformation mix was plated on MM agar plates. Selected transformants were further characterized, as detailed below. More specifically, clone #7 of strain Y5037U, transformed with expression vector pY153 (comprising CeLPCATS) was selected and designated as "Y5037U::FBAIN-CeLPCATS"; clone #18 of strain Y5037U, transformed with expression vector pY201 (comprising ScAle1S) was selected and designated as "Y5037U::ScAle1S"; and clone #6 of strain Y5037U, transformed with expression vector pY208 (comprising MaLPAAT1S) was selected and designated as "Y5037U::MaLPAAT1S". Additionally, strain Y5037 (a Ura+ strain that was parent to strain Y5037 (Ura-)) was used as a control.

A total of four separate experiments were conducted in 3 mL culture based on variable culturing conditions and strains, to examine the effect of LPLAT overexpression on lipid content, fatty acid composition and conversion efficiencies. Experiment 1 compared control strain Y5037 versus strains Y5037U::FBAIN-CeLPCATS and Y5037U::ScAlelS after 2 days of growth in MM medium on a shaker at 200 rpm and 30 °C, followed by 3 days of incubation in 3 mL HGM medium. MM medium (Cat. No. CML-MM, Biomyx Technology), pH 6.1, contains per L: 1.7 g yeast nitrogen base ["YNB"] without amino acids and $NH_4SO_4$, 1 g proline, 0.1 g adenine, 0.1 g lysine, and 20 g glucose.

Experiment 2 compared control strain Y5037 versus strain Y5037U::ScAlelS after 2 days of growth in CSM-U medium on a shaker at 200 rpm and 30 °C, followed by 3 days of incubation in 3 mL HGM medium. CSM-U medium (Cat. No C8140, Teknova Inc,, Hollister, CA) contains: 0.13% amino acid dropout powder minus uracil, 0.17% yeast nitrogen base, 0.5% $(NH_4)_2SO_4$, and 2.0% glucose.

Experiment 3 compared control strain Y5037 versus strains Y5037U::FBAIN-CeLPCATS and Y5037U::ScAlelS after 2 days of growth in MM medium on a shaker at 200 rpm and 30 °C, followed by 5 days of incubation in 3 mL HGM medium.

Experiment 5 compared control strain Y5037 versus strain Y5037U::MaLPAAT1S after 2 days of growth in FM medium on a shaker at 200 rpm and 30 °C, followed by 3 days of incubation in 3 mL HGM medium. The composition of FM medium is described in Example 4.

Following growth for 3 days (Experiments 1, 2, and 5) or 5 days (Experiment 3) in HGM, 1 mL aliquots of the cultures were harvested by centrifugation and analyzed by GC, as described in Example 4.

Experiment 4 compared control strain Y5037 versus strains Y5037U::FBAIN-CeLPCATS and Y5037U::ScAlelS after 2 days of growth in 25 mL FM medium followed by 5 days of incubation in HGM medium as described above. Specifically, one loop of freshly streaked cells from MM agar plates was inoculated into 3 mL FM medium and grown overnight at 250 rpm and 30 °C. The $OD_{600nm}$ was measured and an aliquot of the cells were added to a final $OD_{600nm}$ of 0.3 in 25 mL FM medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for GC analysis and 10 mL dried for dry cell weight ["DCW"] determination (*supra,* Example 4).

Lipid Content. Fatty Acid Composition And Conversion Efficiencies

In each experiment, the lipid content and fatty acid composition are quantified for 1, 2, 3 or 4 replicate cultures ["Replicates"] of the control Y5037 strain and the transformant Y5037U strain(s). Additionally, data for each Y5037U transformant is presented as a % of the Y5037 control. Table 19 below summarizes the concentration of each fatty acid as a weight percent of TFAs ["% TFAs"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA, DPA, DHA and EDD (corresponding to the sum of EPA plus DPA plus DHA). Additionally, the ratio of DHA % TFAs/ DPA % TFAs is provided.

Table 20 summarizes the total DCW (mg/mL), the total lipid content of cells ["TFAs % DCW"], and the conversion efficiency of each desaturase and elongase functioning in the PUFA biosynthetic pathway and which are required for DHA production. Specifically, the Δ12 desaturase conversion efficiency ["Δ12 CE"], Δ8 desaturase conversion efficiency ["Δ8 CE"], Δ5 desaturase conversion efficiency ["Δ5 CE"], Δ17 desaturase conversion efficiency ["Δ17 CE"], Δ4 desaturase conversion efficiency ["Δ4 CE"], Δ9 elongation conversion efficiency ["Δ9e CE"] and Δ5 elongation conversion efficiency ["Δ5e CE"] are provided for each control Y5037 strain and the transformant Y5037U strain(s); data for each Y5037U transformant is presented as a % of the Y5037 control. Conversion efficiency was calculated according to the formula: product(s)/(product(s)+substrate)*100, where product includes both product and product derivatives.

Table 19: Lipid Content and Composition In LPCAT Transformant Strains Of *Yarrowia lipolytica* Y5037

| Ex pt. | Strain | Replicates | % TFAs | | | | | | | | | | | | | | | |
|---|---|---|---|---|---|---|---|---|---|---|---|---|---|---|---|---|---|---|
| | | | 16: 0 | 16: 1 | 18: 0 | 18: 1 | 18: 2 | ALA | EDA | DGLA | ARA | ETrA | ETA | EPA | DPA | DHA | EDD | DHA / DPA |
| 1 | Y5037 | AVG.4 | 4.1 | 1.1 | 3.2 | 5.4 | 21.8 | 0.5 | 2.9 | 1.7 | 0.7 | 1.3 | 1.8 | 18.1 | 20.6 | 6.5 | 45.2 | 0.3 |
| | Y5037U:: FBAIN-CeLPCATS | 1 | 5.2 | 1.2 | 2.7 | 8.5 | 11.1 | 0.3 | 2.5 | 3.6 | 1.1 | 1.4 | 2.7 | 31.7 | 9.6 | 11.0 | 52.4 | 1.1 |
| | | % Ctrl | 127 | 109 | 84 | 157 | 51 | 60 | 86 | 212 | 157 | 108 | 150 | 175 | 47 | 169 | 116 | 367 |
| | Y5037U:: ScAleIS | 1 | 4.4 | 1.4 | 2.0 | 4.4 | 15.7 | 0.5 | 3.5 | 2.7 | 1.0 | 1.2 | 2.2 | 22.0 | 16.8 | 14.4 | 53.3 | 0.9 |
| | | % Ctrl | 107 | 127 | 63 | 81 | 72 | 100 | 121 | 159 | 143 | 92 | 122 | 122 | 82 | 222 | 118 | 300 |
| 2 | Y5037 | AVG.2 | 4.4 | 1.1 | 3.9 | 5.4 | 21.8 | 0.5 | 3.4 | 1.6 | 0.8 | 1.1 | 1.7 | 17.0 | 21.0 | 6.7 | 44.7 | 0.3 |
| | Y5037U:: ScAleIS | 1 | 4.5 | 1.5 | 2.5 | 4.7 | 16.6 | 0.4 | 4.1 | 2.6 | 1.1 | 1.1 | 2.1 | 21.2 | 17.1 | 13.2 | 51.5 | 0.8 |
| | | % Ctrl | 102 | 136 | 64 | 87 | 76 | 80 | 121 | 163 | 138 | 100 | 124 | 125 | 81 | 197 | 115 | 267 |
| 3 | Y5037 | AVG.3 | 3.9 | 1.1 | 1.6 | 4.7 | 20.7 | 0.5 | 3.3 | 1.8 | 1.3 | 1.5 | 3.9 | 19.3 | 20.8 | 7.9 | 47.9 | 0.4 |
| | Y5037U:: FBAIN-CeLPCATS | 1 | 5.8 | 1.1 | 2.6 | 8.0 | 10.0 | 0.3 | 3.0 | 3.6 | 1.9 | 2.2 | 2.7 | 31.0 | 9.9 | 11.8 | 52.7 | 1.2 |
| | | % Ctrl | 149 | 100 | 163 | 170 | 48 | 60 | 91 | 200 | 146 | 147 | 69 | 161 | 48 | 149 | 110 | 300 |
| | Y5037U:: ScAleIS | 1 | 4.6 | 1.3 | 1.8 | 5.9 | 18.1 | 0.3 | 4.4 | 2.4 | 1.3 | 1.8 | 4.0 | 22.1 | 15.1 | 11.7 | 48.9 | 0.8 |
| | | % Ctrl | 118 | 118 | 113 | 126 | 87 | 60 | 133 | 133 | 100 | 120 | 103 | 115 | 73 | 148 | 102 | 200 |
| 5 | Y5037 | 1 | 5.1 | 1.3 | 1.6 | 4.7 | 22.5 | 2.7 | 3.9 | 1.9 | 1.4 | 1.3 | 1.7 | 20.4 | 20.7 | 8.9 | 50.1 | 0.4 |
| | Y5037U:: MaLPAT1 | 1 | 6.1 | 1.5 | 1.8 | 4.5 | 21.1 | 2.2 | 4.0 | 2.1 | 1.5 | 1.2 | 1.7 | 23.4 | 19.5 | 10.7 | 53.7 | 0.6 |
| | | % Ctrl | 120 | 115 | 113 | 96 | 94 | 81 | 103 | 111 | 107 | 92 | 100 | 115 | 94 | 120 | 107 | 150 |
| 4 | Y5037 | AVG.3 | 3.9 | 1.2 | 1.3 | 5.9 | 22.4 | 3.9 | 1.7 | 1.8 | 0.8 | 1.0 | 1.6 | 20.0 | 26.2 | 6.7 | 52.9 | 0.3 |
| | Y5037U:: | AVG.3 | 6.1 | 1.3 | 3.4 | 8.8 | 10.1 | 0.7 | 1.6 | 3.5 | 0.7 | 1.3 | 2.3 | 33.9 | 12.5 | 10.6 | 57.0 | 0.9 |
| | FBAIN-CeLPCATS | % Ctrl | 156 | 108 | 262 | 149 | 45 | 18 | 94 | 194 | 88 | 130 | 144 | 170 | 48 | 158 | 108 | 300 |
| | Y5037U:: ScAleIS | AVG.3 | 5.4 | 1.4 | 2.7 | 8.7 | 21.1 | 1.7 | 5.4 | 2.5 | 0.6 | 1.2 | 1.4 | 20.4 | 19.6 | 7.3 | 47.3 | 0.4 |
| | | % Ctrl | 138 | 117 | 208 | 147 | 94 | 44 | 318 | 139 | 75 | 120 | 88 | 102 | 75 | 109 | 89 | 133 |

Table 20: Desaturase And Elongase Conversion Efficiency In LPCAT Transformant Strains Of *Yarrowia lipolytica* Y5037

| Expt. | Strain | Replicates | DCW mg/mL | TFA % DCW | Δ12 CE | Δ9e CE | Δ8 CE | Δ5 CE | Δ17 CE | Δ5e CE | Δ4 CE |
|---|---|---|---|---|---|---|---|---|---|---|---|
| 1 | Y5037 | AVG.4 | nd | nd | 93 | 71 | 92 | 93 | 90 | 60 | 24 |
| | Y5037U:: FBAIN-CeLPCATS | 1 | nd | nd | 90 | 85 | 94 | 89 | 89 | 39 | 53 |
| | | % Ctrl | nd | nd | 96 | 120 | 102 | 96 | 98 | 66 | 221 |
| | Y5037U:: ScAleIS | 1 | nd | nd | 95 | 80 | 93 | 92 | 89 | 59 | 46 |
| | | % Ctrl | nd | nd | 102 | 113 | 101 | 99 | 98 | 98 | 191 |
| 2 | Y5037 | AVG.2 | nd | nd | 93 | 71 | 91 | 93 | 89 | 62 | 24 |
| | Y5037U:: ScAleIS | 1 | nd | nd | 94 | 79 | 92 | 92 | 88 | 59 | 44 |
| | | % Ctrl | nd | nd | 101 | 111 | 100 | 99 | 98 | 95 | 180 |
| 3 | Y5037 | AVG.3 | nd | nd | 94 | 74 | 92 | 90 | 89 | 60 | 27 |
| | Y5037U:: FBAIN-CeLPCATS | 1 | nd | nd | 91 | 86 | 92 | 90 | 87 | 41 | 54 |
| | | % Ctrl | nd | nd | 96 | 117 | 100 | 100 | 97 | 69 | 198 |
| | Y5037U:: ScAleIS | 1 | nd | nd | 93 | 77 | 90 | 89 | 87 | 55 | 44 |
| | | % Ctrl | nd | nd | 99 | 105 | 98 | 99 | 97 | 92 | 160 |
| 5 | Y5037 | 1 | nd | nd | 95 | 70 | 91 | 93 | 88 | 59 | 30 |
| | Y5037U:: MaLPAT1 | 1 | nd | nd | 95 | 73 | 92 | 93 | 88 | 56 | 36 |
| | | % Ctrl | nd | nd | 100 | 104 | 101 | 100 | 100 | 95 | 118 |
| 4 | Y5037 | AVG.3 | 3.7 | 19.7 | nd | 69 | 95 | 94 | 93 | 62 | 20 |
| | Y5037U:: FBAIN-CeLPCATS | AVG.3 | 3.0 | 14.0 | nd | 86 | 96 | 91 | 91 | 40 | 46 |
| | | % Ctrl | 82 | 71 | nd | 124 | 100 | 97 | 98 | 65 | 226 |
| | Y5037U:: ScAleIS | AVG.3 | 3.7 | 31.6 | nd | 72 | 89 | 92 | 85 | 57 | 27 |
| | | % Ctrl | 101 | 157 | nd | 104 | 93 | 98 | 92 | 92 | 133 |

Based on the data in Table 19 and Table 20, overexpression of LPLAT in DHA strains Y5037U::CeLPCATS, Y5037U::ScAleIS and Y5037U::MaLPAAT1S results in reduction of the concentration of LA as a weight % of TFAs ["LA % TFAs"], an increase in the concentration of EPA as a weight % of TFAs ["EPA % TFAs"], an increase in the concentration of DHA as a weight % of TFAs ["DHA % TFAs"], an increase in the concentration of EPA + DPA + DHA as a weight % of TFAs ["EDD % TFAs"] (with the exception of strain Y5037U::ScAleIS in Experiment 4), an increase in the ratio of DHA % TFAs to DPA % TFAs ["DHA/DPA"], an increase in the conversion efficiency of the Δ9 elongase and an increase in the conversion efficiency of the Δ4 desaturase.

More specifically, depending on the culture conditions, CeLPCATS overexpression in Y5037U::CeLPCATS can reduce LA % TFAs to 45%, increase EPA % TFAs to 175%, increase DHA % TFAs to 169%, increase Δ9 elongation CE to 124%, and increase Δ4 desaturation CE to 226%, as compared to the control. Similarly, depending on the culture conditions, ScAle1 S overexpression in Y5037U::ScAleIS can reduce LA % TFAs to 72%, increase EPA % TFAs to 125%, increase DHA % TFAs to 222%, increase Δ9 elongation CE to 113%, and increase Δ4 desaturation CE to 191%, as compared to the control. Finally, overexpression of MaLPAAT1 in Y5037U::MaLPAAT1S can reduce LA % TFAs to 94%, increase EPA % TFAs to 115%, increase DHA % TFAs to 120%, increase Δ9 elongation CE to 104%, and increase Δ4 desaturation CE to 118%, as compared to the control.

Although Y5037U::CeLPCATS possessed a significantly lower total lipid content ["TFAs % DCW"] in Experiment 4, the total lipid content was significantly improved in strain Y5037U::ScAleIS. This increase in lipid content is a likely explanation for the lower EDD % TFAs in strain Y5037U::ScAleIS.

DHA biosynthesis via EPA involves two steps: elongation of EPA to DPA by $C_{20/22}$ elongase (also known as either a "C20" elongase or a Δ5 elongase) and desaturation of DPA to DHA by Δ4 desaturase. An important bottleneck in the production of DHA from EPA has been the Δ4 desaturation step, evident by the build up of DPA, although the mechanistic details for this limitation were unknown. The results above show that expression of ScAle1S, YlAle1, YlLPAAT1, MaLPAAT1S, and CeLPCATS proteins significantly improved Δ4 desaturation. Thus, Δ4 desaturation was not limiting because of Δ4 desaturase activity *per se.* Instead, Δ4 desaturation was limiting because of limited availability of the DPA substrate at the sn-2 position of phospholipids. The results showed unexpectedly that (unlike other desaturation substrates), limited DPA incorporation into phospholipid can be overcome by overexpression of Ale1, LPAAT and LPCAT proteins.

Previously, Intl. App. Pub. No. WO 2004/076617 showed that expression of CeLPCAT (SEQ ID NO:2) in *Saccharomyces cerevisiae* improved Δ6 elongation of exogenously provided GLA to DGLA. It hypothesized that CeLPCAT removed an acyl chain from the sn-2 position of phospholipids, thereby making the removed acyl group available for elongation in the CoA pool. It was shown in the present studies that the expression of the codon-optimized CeLPCATS, under control of the YAT1 promoter, in strains of *Yarrowia lipolytica* engineered to produce high levels of EPA (Example 4) and DHA (Example 5), respectively, improves Δ9 elongation of endogenously produced LA to EDA. However, expression of CeLPCATS in DHA-producing strain Y5037U::CeLPCATS unexpectedly did <u>not</u> result in improved Δ5 elongation of EPA to DPA. In contrast, expression of CeLPCATS in DHA-producing strain Y5037U::CeLPCATS very significantly improved Δ4 desaturation of DPA to DHA (*supra*). This is especially unexpected since desaturations occur mainly at the sn-2 position of phospholipids and elongation occurs in the CoA pool.

Based on the improved Δ4 desaturation conversion efficiency resulting from over-expression of LPLATs, demonstrated above, it is anticipated that the LPLATs described herein and their orthologs, such as ScLPAAT, will also improve Δ4 desaturation conversion efficiency.

## EXAMPLE 6

### Functional Characterization Of Different LPLATs In ARA-Producing Y. *lipolytica* Strain Y8006U

*Yarrowia lipolytica* strain Y8006U, producing ARA, is used to functionally characterize the effects of overexpression of the *Saccharomyces cerevisiae* Ale1, *Mortierella alpina* LPAAT1 and *Caenorhabditis elegans* LPCAT, following their integration into the *Yarrowia* host chromosome. This was in spite of the host containing its native LPLATs, i.e., Ale1 and LPAAT1.

### Transformation And Growth

*Yarrowia lipolytica* strain Y8006U (Example 1) will be individually transformed with linear *Sph*I-*Asc*I fragments of the integrating vectors described in Example 3, in a manner comparable to that utilized in Example 4. URA+ transformants will be selected, grown for 2 days in FM medium and 5 days in HGM medium and then 1 mL aliquots of the cultures will be harvested by centrifugation and analyzed by GC (Example 4). Based on the fatty acid composition of the 3 mL cultures, selected transformants will be further characterized using strain Y8006 (a Ura+ strain that was parent to strain Y8006U (Ura-)) as a control.

Each selected transformant and the control will be re-grown in FM and HGM medium, as described in Example 4, and then subjected to GC analysis and DCW determination.

The lipid content, fatty acid composition and ARA as a percent of the DCW will be quantified for the control Y8006 strain and the transformant Y8006U strain(s). Additionally, data for each Y8006U transformant will be determined as a % of the Y8006 control. The conversion efficiency of each desaturase and the Δ9 elongase functioning in the PUFA biosynthetic pathway and which is required for ARA production will also be determined and compared to the control, in a manner similar to that in Examples 4 and 5.

It is hypothesized that overexpression of the ScAle1S, YlAle1, MaLPAAT1S, YlLPAAT1 and CeLPCATS LPLATs in the ARA strains will result in a reduction of the concentration of LA (18:2) as a weight % of TFAs ["LA % TFAs"], an increase in the concentration of ARA as a weight % of TFAs ["ARA % TFAs"], and an increase in the conversion efficiency of the Δ9 elongase.

## EXAMPLE 7

### Construction Of Expression Vectors Comprising LPAAT ORFs And An Autonomously Replicating Sequence

The present example describes the construction of vectors comprising autonomously replicating sequences ["ARS"] and LPAAT ORFs suitable for LPAAT gene expression without integration in *Yarrowia lipolytica.* ORFs included the *Saccharomyces cerevisiae* LPAAT encoding SEQ ID NO:18 and the *Yarrowia lipolytica* LPAAT1 encoding SEQ ID NO:17. Example 8 describes the results obtained following transformation of these vectors into *Y. lipolytica.*

Construction Of pY222, Comprising A Codon-Optimized *Saccharomyces cerevisiae* LPAAT Gene

The *Saccharomyces cerevisiae* ORF designated as "ScLPAAT" (SEQ ID NO:18) was optimized for expression in *Yarrowia lipolytica,* by DNA 2.0 (Menlo Park, CA). In addition to codon optimization, 5' *Pci*1 and 3' *Not*1 cloning sites were introduced within the synthetic gene (i.e., ScLPAATS; SEQ ID NO:96). None of the modifications in the ScLPAATS gene changed the amino acid sequence of the encoded protein (i.e., the protein sequence encoded by the codon-optimized gene [i.e., SEQ ID NO:97] is identical to that of the wildtype protein sequence [i.e., SEQ ID NO:18]). ScLPAATS was cloned into pJ201 (DNA 2.0) to result in pJ201 :ScLPAATS.

A 926 bp *Pci*1/*Not*1 fragment comprising ScLPAATS was excised from pJ201 :ScLPAATS and cloned into *Nco*I-*Not*1 cut pYAT-DG2-1 to create pY222 (SEQ ID NO:100; Table 21; FIG. 13A). Thus, pY222 contained the following components:

Table 21: Description of Plasmid pY222 (SEQ ID NO:100)

| RE Sites And Nucleotides Within SEQ ID NO:100 | Description Of Fragment And Chimeric Gene Components |
|---|---|
| *Sal*1/*Swa*I (1-2032) | YAT1::ScLPAATS::Lip1, comprising:<br>• YAT1: *Yarrowia lipolytica* YAT1 promoter (U.S. Pat. Appl. Pub. No. 2006/0094102-A1);<br>• ScLPAATS: codon-optimized ScLPAATS (SEQ ID NO:96) (labeled as "Sc LPAATs ORF" in Figure);<br>• Lip1: Lip1 terminator sequence from *Yarrowia Lip1* gene (GenBank Accession No. Z50020) (labeled as "Lip1-3'" in Figure) |
| *Swa*I/*Ava*I (2032-4946) | • *ColE1* plasmid origin of replication;<br>• Ampicillin-resistance gene ($Amp^R$) for selection in *E. coli;*<br>• *E. coli* f1 origin of replication |
| *Ava*I-*Sph*I (4946-6330) | *Yarrowia lipolytica* centromere and autonomously replicating sequence ["ARS"] 18 locus |
| *Sph*I-*Sal*I (6330-1) | *Yarrowia lipolytica* URA3 gene (GenBank Accession No. AJ306421) |

Construction Of pY177, Comprising A *Yarrowia lipolytica* LPAAT1 Gene

The *Yarrowia lipolytica* centromere and autonomously replicating sequence ["ARS"] was amplified by standard PCR using primer 869 (SEQ ID NO:98) and primer 870 (SEQ ID NO:99), with plasmid pYAT-DG2-1 as template. The PCR product was digested with *Asc*I/*Avr*II and cloned into *Asc*I-*Avr*II digested pY207 (SEQ ID NO:87; Example 3) to create pY177 (SEQ ID NO:101; Table 22; FIG. 13B). Thus, the components present in pY177 are identical to those in pY207 (FIG. 11B), except for the replacement of the 373 bp pY207 sequence between *Asc*I and *Avr*II with the 1341 bp sequence containg ARS. More specifically, pY177 contained the following components:

Table 22: Description of Plasmid pY177 (SEQ ID NO:101)

| RE Sites And Nucleotides Within SEQ ID NO:101 | Description Of Fragment And Chimeric Gene Components |
|---|---|
| *Bsi*W1/*Sbf*1 (1-1706 bp) | LoxP::*Ura3*::LoxP, comprising:<br>• LoxP sequence (SEQ ID NO:78)<br>• *Yarrowia lipolytica* Ura3 gene (GenBank Accession No. AJ306421);<br>• LoxP sequence (SEQ ID NO:78) |
| *Sbf*1/*Sph*1 (1706-3043 bp) | 3' portion of *Yarrowia lipolytica* POX3 Acyl-CoA oxidase 3 (GenBank Accession No. YALI0D24750g) |

(continued)

| RE Sites And Nucleotides Within SEQ ID NO:101 | Description Of Fragment And Chimeric Gene Components |
|---|---|
| *Sph*I/*Asc*I (3043-5743 bp) | • *ColE1* plasmid origin of replication;<br>• Ampicillin-resistance gene ($Amp^R$) for selection in *E. coli;*<br>• *E. coli* f1 origin of replication |
| *Asc*I/*Bsi*WI (5743-6513 bp) | 5' portion of *Yarrowia lipolytica* POX3 Acyl-CoA oxidase 3 (GenBank Accession No. YALI0D24750g) |
| *Asc*I/*Avr*II (5743-7084 bp) | *Yarrowia lipolytica* centromere and autonomously replicating sequence ["ARS"] 18 locus |
| *Avr*II/*Bsi*WI (7084-7481 bp) | 5' portion of *Yarrowia lipolytica* POX3 Acyl-CoA oxidase 3 (GenBank Accession No. YALI0D24750g) |
| *Bsi*WI/*Bsi*WI (7481-1 bp) | YAT1::YlLPAAT1::Lip1, comprising:<br>• YAT1: *Yarrowia lipolytica* YAT1 promoter (U.S. Pat. Appl. Pub. No. 2006/0094102-A1);<br>• YlLPAAT1: *Yarrowia lipolytica* LPAAT1 ("YALI0E18964g"; GenBank Accession No. XP_504127) (SEQ ID NO:16) (labeled as "Yl LPAT1 ORF" in Figure);<br>• Lip1: Lip1 terminator sequence from *Yarrowia Lip1* gene (GenBank Accession No. Z50020) (labeled as "Lip1-3'" in Figure) |

EXAMPLE 8

Functional Characterization Of Different LPAATs In EPA-Producinq *Yarrowia lipolytica* Strain Y8406

*Yarrowia lipolytica* strain Y8406U, producing EPA, was used to functionally characterize the effects of expression of the *Saccharomyces cerevisiae* LPAATS (SEQ ID NO:96) and *Yarrowia lipolytica* LPAAT1 (SEQ ID NO:16) without integration on self-replicating plasmids. This was in spite of the host containing its native LPAATs.

Transformation And Growth

*Yarrowia lipolytica* strain Y8406U (Example 1) was individually transformed with uncut plasmids from Example 7. Specifically, vectors pY177 (YAT1::YlLPAAT1::Lip1) [SEQ ID NO:101] and pY222 (YAT1 ::ScLPAATS::Lip1) [SEQ ID NO:100] were transformed according to the General Methods.

Each transformation mix was plated on MM agar plates. Several resultant URA+ transformants were picked and inoculated into 3 mL CSM-U medium (Teknova Cat. No. C8140, Teknova Inc., Hollister, CA), wherein CSM-U medium refers to CM Broth with glucose minus uracil containing 0.13% amino acid dropout powder minus uracil, 0.17% yeast nitrogen base, 0.5% $(NH_4)_2SO_4$, and 2.0% glucose. After 2 days growth on a shaker at 200 rpm and 30 °C, the cultures were harvested by centrifugation and resuspended in 3 mL HGM medium (Cat. No. 2G2080, Teknova Inc.). After 5 days growth on a shaker, 1 mL aliquots of the cultures were harvested and analyzed by GC, as described in Example 4.

Based on the fatty acid composition of the 3 mL cultures, selected transformants were further characterized by flask assay. Specifically, clones #5 and #6 of strain Y8406U transformed with expression vector pY222 (comprising ScLPAATS) were selected and designated as "Y8406U::ScLPAATS-5" and "Y8406U::ScLPAATS-6", respectively; clone #1 of strain Y8406U transformed with expression vector pY177 (comprising YlLPAAT1) was selected and designated as "Y8406U::YlLPAAT1". Additionally, strain Y8406 (a Ura+ strain that was parent to strain Y8406U (Ura-)) was used as a control.

Each selected transformant and the control was streaked onto MM agar plates. Then, one loop of freshly streaked cells was inoculated into 3 mL CSM-U medium and grown overnight at 250 rpm and 30 °C. The $OD_{600nm}$ was measured and an aliquot of the cells were added to a final $OD_{600nm}$ of 0.3 in 25 mL CSM-U medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for GC analysis and 10 mL dried for dry cell weight ["DCW"] determination, as described in Example 4.

Lipid Content. Fatty Acid Composition And Conversion Efficiencies

The lipid content, fatty acid composition and EPA as a percent of the DCW are quantified for 2 replicate cultures ["Replicates"] of the control Y8406 strain and the transformant Y8406U strain(s). Additionally, data for each Y8406U transformant is presented as a % of the Y8406 control. Table 23 below summarizes the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA and EPA.

Table 24 summarizes the conversion efficiency of each desaturase and the Δ9 elongase functioning in the PUFA biosynthetic pathway and which are required for EPA production, in a manner identical to that described in Example 4.

Table 23: Lipid Content And Composition In ScLPAATS and YlLPAAT1 Transformant Strains Of *Yarrowia lipolytica* Y8406

| Strain | Repli cates | TFA % DCW | % TFAs | | | | | | | | | | | | EPA % DCW |
|---|---|---|---|---|---|---|---|---|---|---|---|---|---|---|---|
| | | | 16: 0 | 16: 1 | 18: 0 | 18: 1 | 18:2 | ALA | EDA | DGLA | ARA | ERA | ETA | EPA | |
| Y8406 | AVG.2 | 22.0 | 2 | 0 | 2 | 4 | 19 | 2 | 3 | 4 | 1 | 2 | 3 | 55 | 12 |
| Y8406U:: YlLPAAT1 | AVG.2 | 24.6 | 2 | 1 | 2 | 6 | 14 | 1 | 3 | 5 | 1 | 2 | 3 | 55 | 14 |
| | % Ctrl | 112 | 98 | 153 | 102 | 148 | 76 | 50 | 120 | 144 | 101 | 109 | 123 | 101 | 113 |
| Y8406U:: ScLPAATS-5 | AVG.2 | 21.6 | 3 | 1 | 3 | 6 | 14 | 1 | 3 | 4 | 1 | 2 | 3 | 57 | 12 |
| | % Ctrl | 98 | 131 | 137 | 125 | 131 | 74 | 56 | 100 | 117 | 86 | 101 | 108 | 104 | 102 |
| Y8406U:: ScLPAATS-6 | AVG.2 | 21.4 | 3 | 1 | 3 | 5 | 14 | 1 | 3 | 4 | 1 | 2 | 3 | 58 | 12 |
| | % Ctrl | 97 | 125 | 133 | 121 | 124 | 72 | 52 | 97 | 119 | 88 | 102 | 111 | 106 | 103 |

Table 24: Desaturase And Elongase Conversion Efficiency In ScLPAATS and YlLPAAT1 Transformant Strains Of *Yarrowia lipolytica* Y8406

| Strain | Replicates | Δ12 CE | Δ9e CE | Δ8 CE | Δ5 CE | Δ17 CE |
|---|---|---|---|---|---|---|
| Y8406 | AVG.2 | 95 | 77 | 92 | 90 | 92 |
| Y8406U::YlLPAAT1 | AVG.2 | 93 | 82 | 92 | 87 | 90 |
| | % Ctrl | 98 | 107 | 99 | 97 | 98 |
| Y8406U:: ScLPAATS-5 | AVG.2 | 94 | 83 | 93 | 89 | 92 |
| | % Ctrl | 98 | 108 | 100 | 99 | 100 |
| Y8406U:: ScLPAATS-6 | AVG.2 | 94 | 83 | 93 | 89 | 92 |
| | % Ctrl | 99 | 109 | 101 | 99 | 100 |

Based on the data in Table 23 and Table 24 above, overexpression of both ScLPAATS and YlLPAAT1 in EPA strains Y8406U::YlLPAAT1, Y8406U::ScLPAATS-5 and Y8406U::ScLPAATS-6 resulted in reduction (to 76% or below of the control) of the concentration of LA (18:2) as a weight % of TFAs ["LA % TFAs"], and an increase (to at least 7% of the control) in the conversion efficiency of the Δ9 elongase. ScLPAATS and YlLPAAT1 have a similar effect on lipid profile.

The results obtained above were then compared to those obtained in Example 4, although different means were utilized to characterize the LPLATs. Specifically, in Example 4, linearized DNA carrying the LPLATs were transformed by chromosomal integration, since the vectors lacked ARS sequences. This resulted in stable integrations and the strains were grown in the relatively rich, non-selective FM growth medium during both preculture and 2 days growth prior to being transferred to HGM.

In Example 8, the functional characterization of YlLPAAT1 and ScLPAATS was done on a replicating plasmid. Thus, *Yarrowia lipolytica* strain Y8406 was transformed with circular DNA carrying each LPAAT and ARS sequence. To maintain these plasmids and assay gene expression without integration, it was necessary to grow the transformants on selective medium (i.e., CSM-U medium) during both preculture and 2 days growth prior to being transferred to HGM.

These differences described above can contribute to differences in lipid profile and content, as illustrated by the expression of YlLPAAT1 in Examples 4 and 8. The change over control in LA % TFAs, EPA % TFAs, and Δ9 elongase conversion efficiency were 63%, 115%, and 115%, respectively, upon expression of YlLPAAT in Example 4, whereas the change over control in LA % TFAs, EPA % TFAs, and Δ9 elongase conversion efficiency were were 76%, 101%, and 107%, respectively, upon expression of YlLPAAT in Example 8. Thus, the improvements in Δ9 elongation and LC-PUFA biosynthesis in Example 8 are minimized when compared to those observed in Example 4. These differences can be attributed to the "position effects" of chromosomal integration and/or different growth conditions.

Since the improvements in LC-PUFA biosynthesis (measured as reduction in LA % TFAs, increase in EPA % TFAs and increase in Δ9 elongase conversion efficiency) are similar for both ScLPAATS and YlLPAAT when transformed in *Yarrowia lipolytica* strain Y8406 on a replicating plasmid, it is anticipated that both LPLAATs will also function similarly when stably integrated into the host chromosome. Thus, ScLPAATS will likely improve the lipid profile in a manner similar to that observed in Examples 4 and 5, when YlLPAAT1 was stably integrated into the host chromosome.

## Claims

**1.** A recombinant oleaginous eicosapentaenoic acid (EPA)-producing *Yarrowia lipolytica* host cell, which comprises at least one transgene comprising a polynucleotide encoding a polypeptide having at least acyl-CoA:lysophospholipid acyltransferase activity, wherein the polypeptide has at least 95% amino acid identity, based on the Clustal W method of alignment, when compared to SEQ ID NO:11;
wherein:

the transgene comprises the polynucleotide operably linked to at least one regulatory sequence, wherein said at least one regulatory sequence includes a promoter which is a constitutive promoter or which is a regulatable promoter having an inducible activity which allows induced expression; and,
wherein the polypeptide is over-expressed when compared to a control host cell.

**2.** . The recombinant host cell of claim 1 wherein the polynucleotide encoding a polypeptide having at least acyl-CoA:lysophospholipid acyltransferase activity is stably integrated in the host cell.

**3.** . The recombinant host cell of claim 1 or 2, wherein said promoter is a constitutive promoter.

4. . The recombinant host cell of claim 1 or 2, wherein said promoter is a regulatable promoter having inducible activity.

5. . A method for making an oil comprising eicosapentaenoic acid comprising:

   a) culturing the oleaginous *Yarrowia lipolytica* host cell of claim 2, wherein the host cell has an increase in C18 to C20 elongation conversion efficiency of at least 13 % compared to a control host cell and wherein an oil comprising eicosapentaenoic acid is produced; and
   b) optionally recovering the microbial oil of step (a).

6. . The method of claim 5 wherein the recovered oil of step (b) is further processed.

## Patentansprüche

1. Rekombinante, ölige, Eicosapentaenonsäure (EPA) erzeugende *Yarrowia lipolytica*-Wirtszelle, die mindestens ein Transgen umfasst, das ein Polynukleotid umfasst, das für ein Polypeptid codiert, das mindestens eine Acyl-CoA:Lysophospholipid-Acyltransferase-Aktivität aufweist, wobei das Polypeptid mindestens 95 % Aminosäureidentität, auf der Basis des Clustal W-Alignment-Verfahrens, im Vergleich mit SEQ ID NO:11 aufweist,
   wobei
   das Transgen das Polynukleotid umfasst, das funktionsfähig mit mindestens einer regulatorischen Sequenz verknüpft ist, wobei die mindestens eine regulatorische Sequenz einen Promotor umfasst, der ein konstitutiver Promotor ist oder der ein regulierbarer Promotor ist, der eine induzierbare Aktivität aufweist, die die induzierte Expression gestattet, und
   wobei das Polypeptid im Vergleich mit einer Kontrollwirtszelle überexprimiert ist.

2. Rekombinante Wirtszelle nach Anspruch 1, wobei das Polynukleotid, das für ein Polypeptid codiert, das mindestens eine Acyl-CoA:Lysophospholipid-Acyltransferase-Aktivität aufweist, stabil in die Wirtszelle integriert ist.

3. Rekombinante Wirtszelle nach Anspruch 1 oder 2, wobei der Promotor ein konstitutiver Promotor ist.

4. Rekombinante Wirtszelle nach Anspruch 1 oder 2, wobei der Promotor ein regulierbarer Promotor ist, der eine induzierbare Aktivität aufweist.

5. Verfahren zum Herstellen eines Öls umfassend Eicosapentaenonsäure, umfassend.

   a) das Züchten der öligen *Yarrowia lipolytica*-Wirtszelle nach Anspruch 2, wobei die Wirtszelle eine Erhöhung der C18- bis C20-Verlängerungsumwandlungseffizienz von mindestens 13 % im Vergleich mit einer Kontrollwirtszelle aufweist und wobei ein Öl, das Eicosapentaenonsäure umfasst, erzeugt wird, und
   b) wahlweise das Gewinnen des mikrobiellen Öls aus Schritt (a).

6. Verfahren nach Anspruch 5, wobei das gewonnene Öl aus Schritt (b) noch weiter verarbeitet wird.

## Revendications

1. Cellule hôte oléagineuse recombinante de *Yarrowia lipolytica* produisant de l'acide eicosapentaénoïque (EPA), qui comprend au moins un transgène comprenant un polynucléotide codant pour un polypeptide ayant au moins une activité acyl-CoA:lysophospholipide acyltransférase, le polypeptide ayant au moins 95 % d'identité d'acide aminé, sur la base du procédé d'alignement Clustal W, lorsque comparé à SEQ ID n°: 11;
   où:

   le transgène comprend le polynucléotide fonctionnellement lié à au moins une séquence de régulation, ladite au moins une séquence de régulation comprenant un promoteur qui est un promoteur constitutif ou qui est un promoteur pouvant être régulé, ayant une activité inductible qui permet l'expression induite; et,
   le polypeptide étant sur-exprimé lorsque comparé à une cellule hôte témoin.

2. Cellule hôte recombinante selon la revendication 1, le polynucléotide codant pour un polypeptide ayant au moins une activité acyl-CoA:lysophospholipide acyltransférase étant intégré de manière stable dans la cellule hôte.

**3.** Cellule hôte recombinante selon la revendication 1 ou 2, ledit promoteur étant un promoteur constitutif.

**4.** Cellule hôte recombinante selon la revendication 1 ou 2, ledit promoteur étant un promoteur pouvant être régulé présentant une activité inductible.

**5.** Procédé de fabrication d'une huile comprenant de l'acide eicosapentaénoïque comprenant:

a) la culture de la cellule hôte oléagineuse de *Yarrowia lipolytica* selon la revendication 2, la cellule hôte présentant une augmentation de l'efficacité de conversion d'allongement de $C_{18}$ à $C_{20}$ d'au moins 13 % comparée à une cellule hôte témoin et une huile comprenant de l'acide eicosapentaénoïque étant produite; et
b) éventuellement la récupération de l'huile microbienne de l'étape (a).

**6.** Procédé selon la revendication 5, l'huile récupérée de l'étape (b) subissant d'autres transformations.

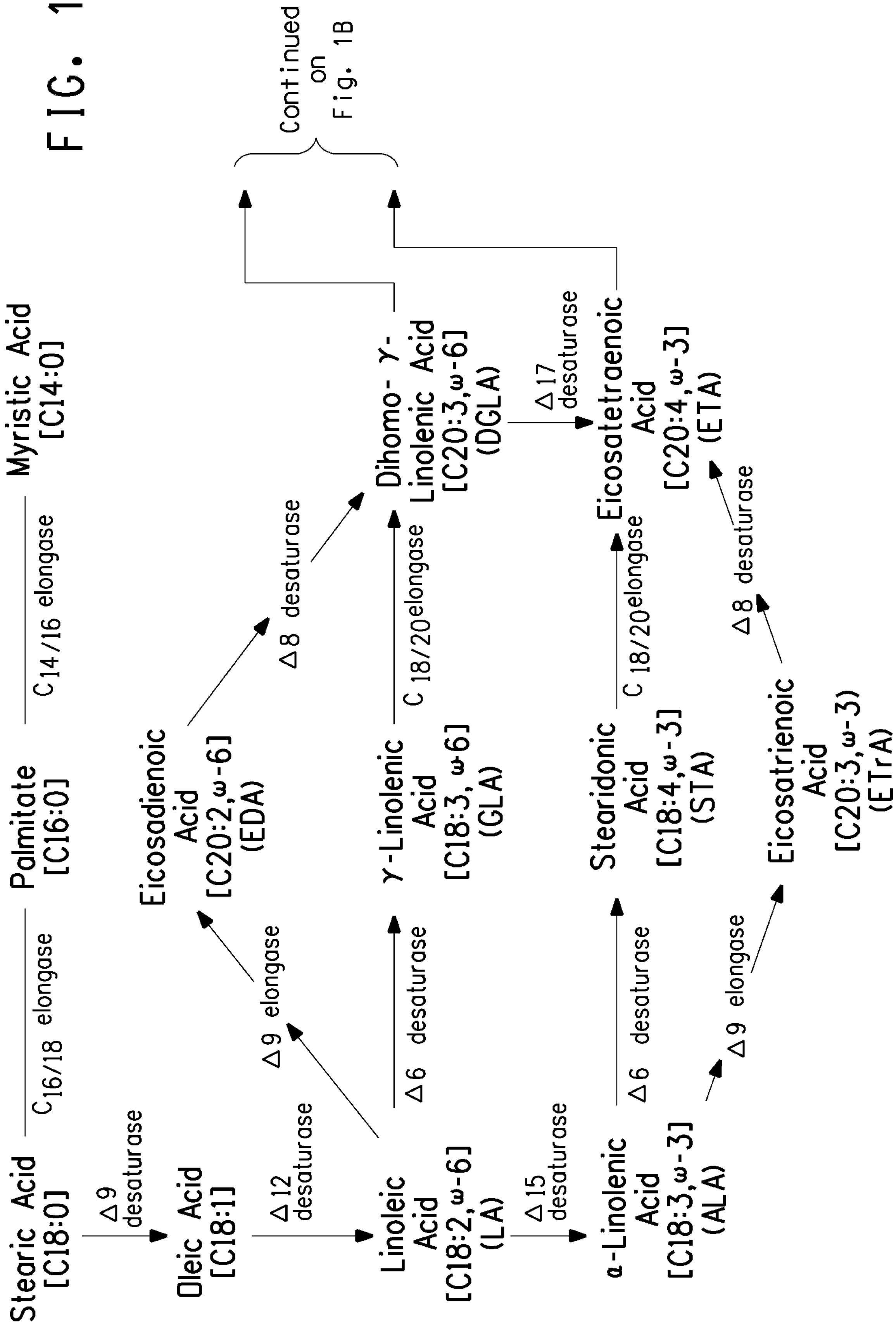

Stearic Acid [C18:0]
$C_{16/18}$ elongase
Palmitate [C16:0]
$C_{14/16}$ elongase
Myristic Acid [C14:0]
Δ9 desaturase
Oleic Acid [C18:1]
Δ12 desaturase
Linoleic Acid [C18:2,ω-6] (LA)
Δ9 elongase
Eicosadienoic Acid [C20:2,ω-6] (EDA)
Δ8 desaturase
Δ6 desaturase
γ-Linolenic Acid [C18:3, ω-6] (GLA)
$C_{18/20}$ elongase
Dihomo- γ-Linolenic Acid [C20:3,ω-6] (DGLA)
Δ15 desaturase
Δ17 desaturase
α-Linolenic Acid [C18:3,ω-3] (ALA)
Δ6 desaturase
Stearidonic Acid [C18:4,ω-3] (STA)
$C_{18/20}$ elongase
Eicosatetraenoic Acid [C20:4,ω-3] (ETA)
Δ9 elongase
Eicosatrienoic Acid [C20:3,ω-3) (ETrA)
Δ8 desaturase
Continued on Fig. 1B


FIG. 1A

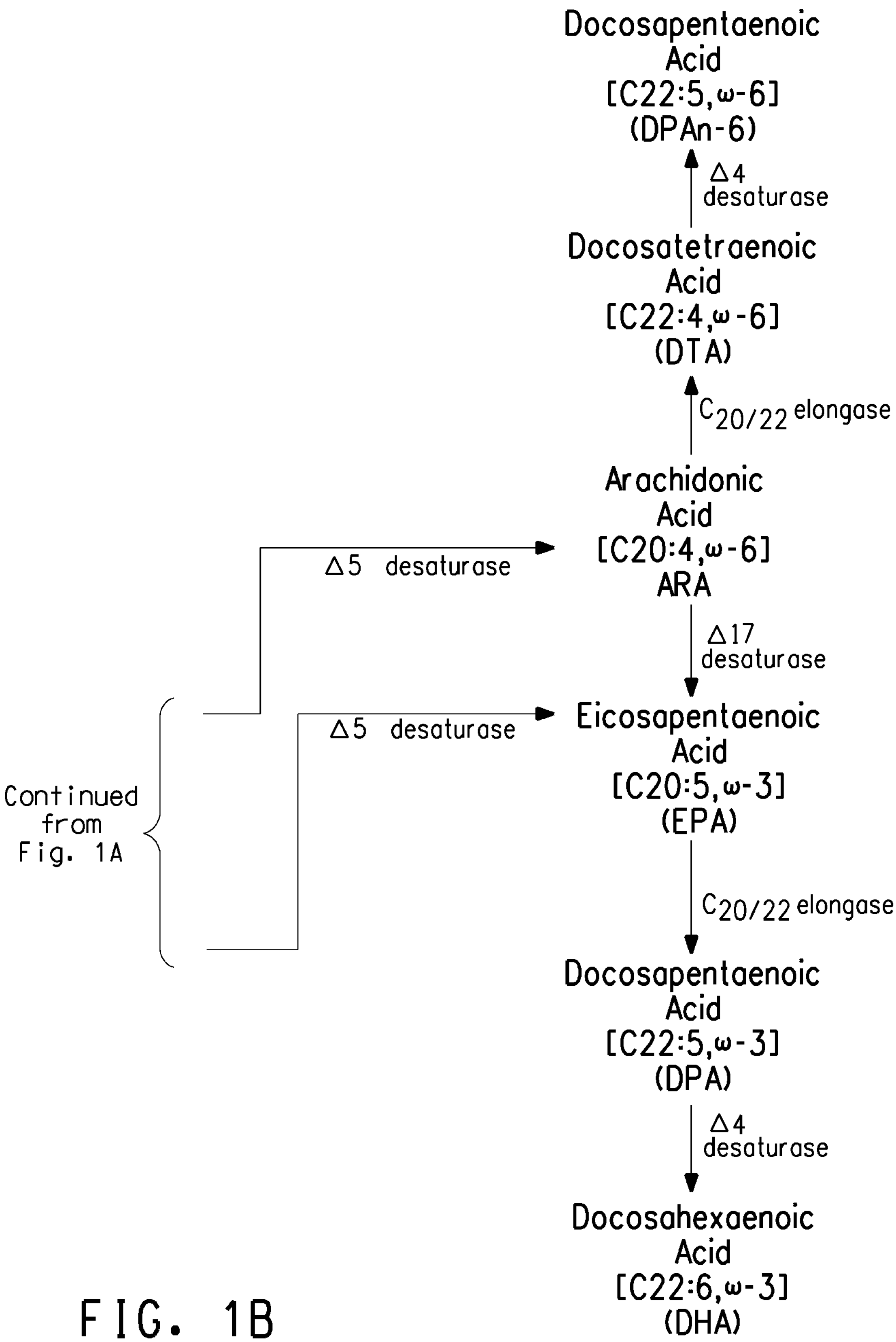
Docosapentaenoic
Acid
[C22:5,ω-6]
(DPAn-6)
Δ4
desaturase
Docosatetraenoic
Acid
[C22:4,ω-6]
(DTA)
C20/22 elongase
Arachidonic
Acid
[C20:4,ω-6]
ARA
Δ5 desaturase
Δ17
desaturase
Δ5 desaturase
Eicosapentaenoic
Acid
[C20:5,ω-3]
(EPA)
Continued
from
Fig. 1A
C20/22 elongase
Docosapentaenoic
Acid
[C22:5,ω-3]
(DPA)
Δ4
desaturase
Docosahexaenoic
Acid
[C22:6,ω-3]
(DHA)

FIG. 1B

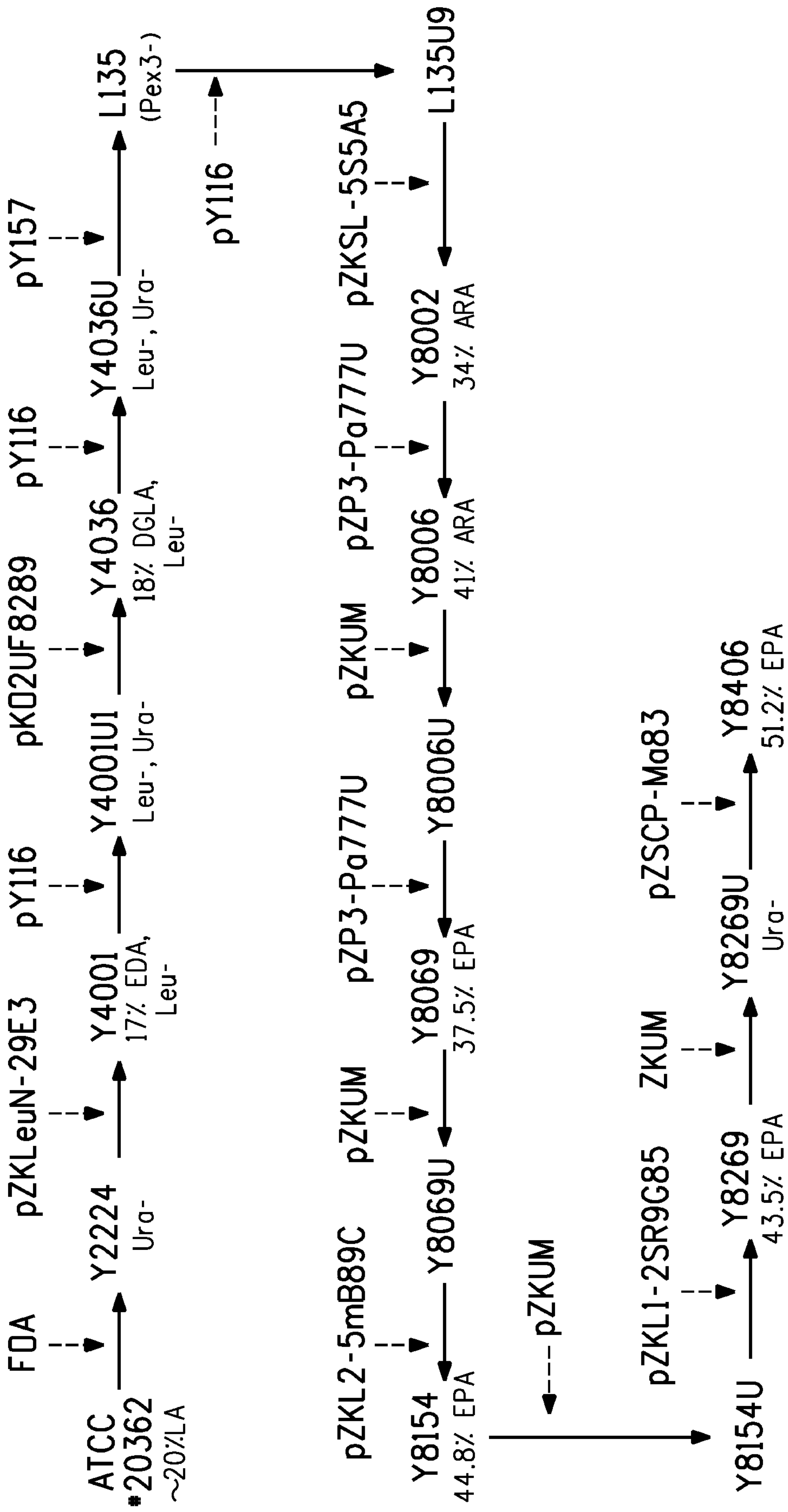
ATCC #20362
~20%LA
FOA
Y2224
Ura-
pZKLeuN-29E3
Y4001
17% EDA,
Leu-
pY116
Y4001U1
Leu-, Ura-
pKO2UF8289
Y4036
18% DGLA,
Leu-
pY116
Y4036U
Leu-, Ura-
pY157
L135
(Pex3-)
pY116
L135U9
pZKSL-5S5A5
Y8002
34% ARA
pZP3-Pa777U
Y8006
41% ARA
pZKUM
Y8006U
pZP3-Pa777U
Y8069
37.5% EPA
pZKUM
Y8069U
pZKL2-5mB89C
Y8154
44.8% EPA
pZKUM
Y8154U
pZKL1-2SR9G85
Y8269
43.5% EPA
ZKUM
Y8269U
Ura-
pZSCP-Ma83
Y8406
51.2% EPA

FIG. 2

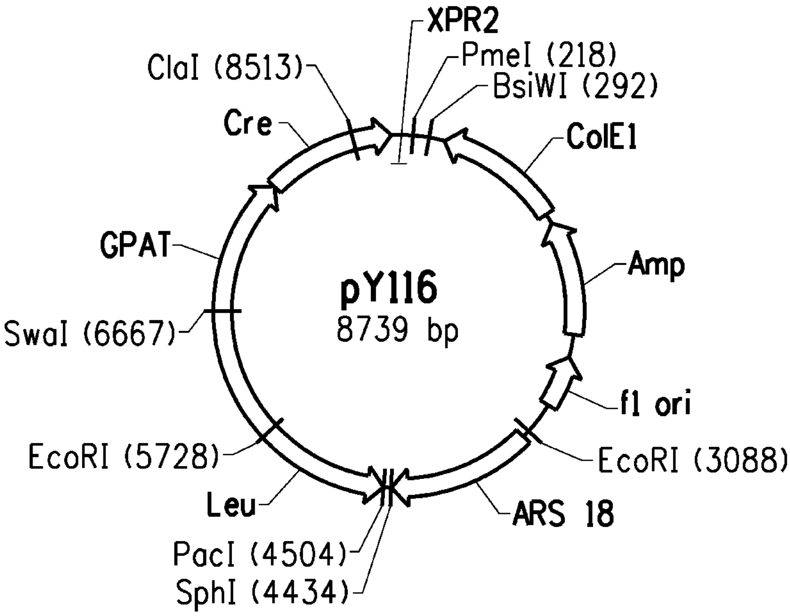
XPR2
PmeI (218)
BsiWI (292)
ClaI (8513)
Cre
ColE1
GPAT
pY116
8739 bp
Amp
SwaI (6667)
f1 ori
EcoRI (5728)
EcoRI (3088)
Leu
ARS 18
PacI (4504)
SphI (4434)

FIG. 3

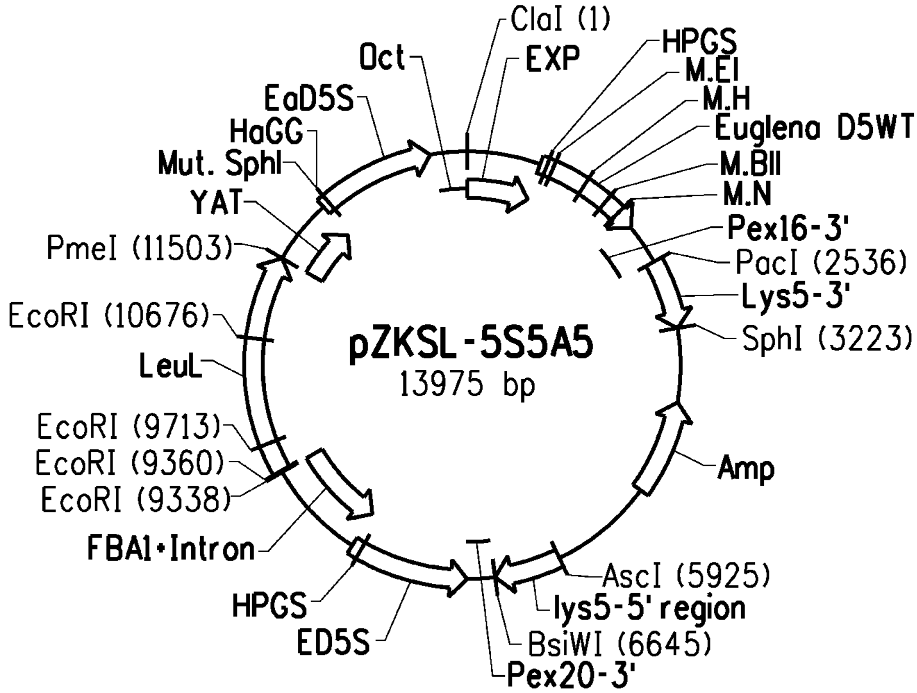

ClaI (1)
Oct
EXP
HPGS
M.EI
M.H
Euglena D5WT
M.BII
M.N
EaD5S
HaGG
Mut. SphI
YAT
Pex16-3'
PacI (2536)
Lys5-3'
SphI (3223)
PmeI (11503)
EcoRI (10676)
LeuL
pZKSL-5S5A5
13975 bp
EcoRI (9713)
EcoRI (9360)
EcoRI (9338)
Amp
FBA1+Intron
AscI (5925)
lys5-5' region
BsiWI (6645)
HPGS
ED5S
Pex20-3'


FIG. 4A

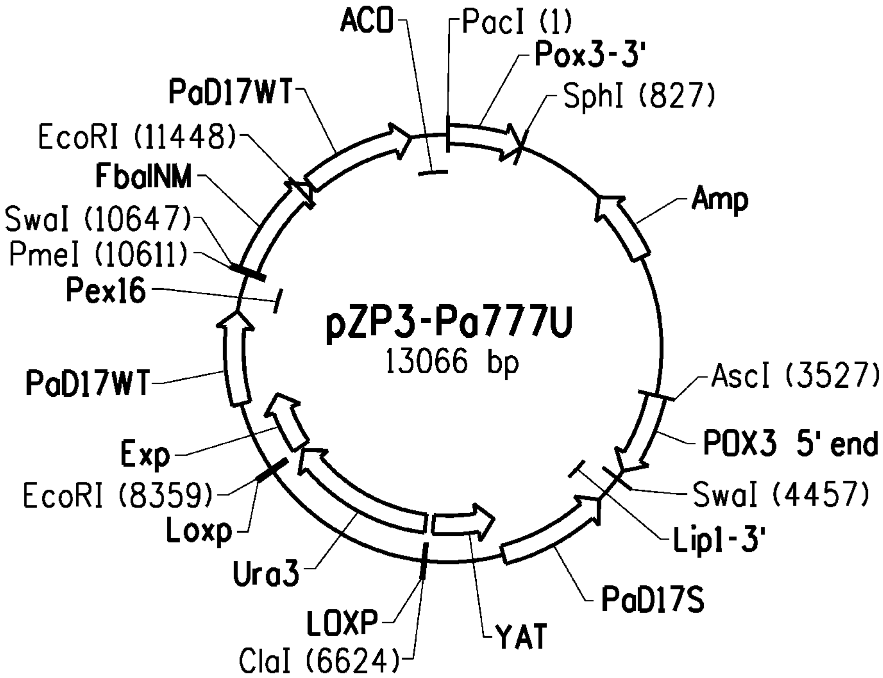

ACO
PacI (1)
Pox3-3'
SphI (827)
PaD17WT
EcoRI (11448)
FbaINM
Amp
SwaI (10647)
PmeI (10611)
Pex16
pZP3-Pa777U
13066 bp
PaD17WT
AscI (3527)
POX3 5' end
Exp
SwaI (4457)
EcoRI (8359)
Lip1-3'
Loxp
Ura3
PaD17S
LOXP
YAT
ClaI (6624)


FIG. 4B

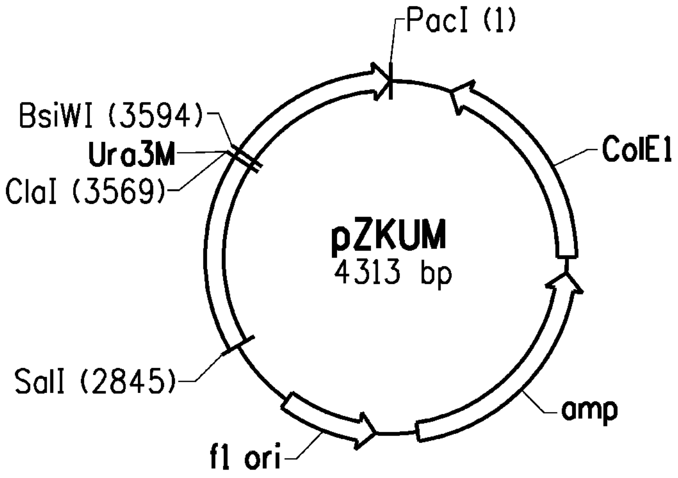
PacI (1)
BsiWI (3594)
Ura3M
ClaI (3569)
ColE1
pZKUM
4313 bp
SalI (2845)
amp
f1 ori

FIG. 5A

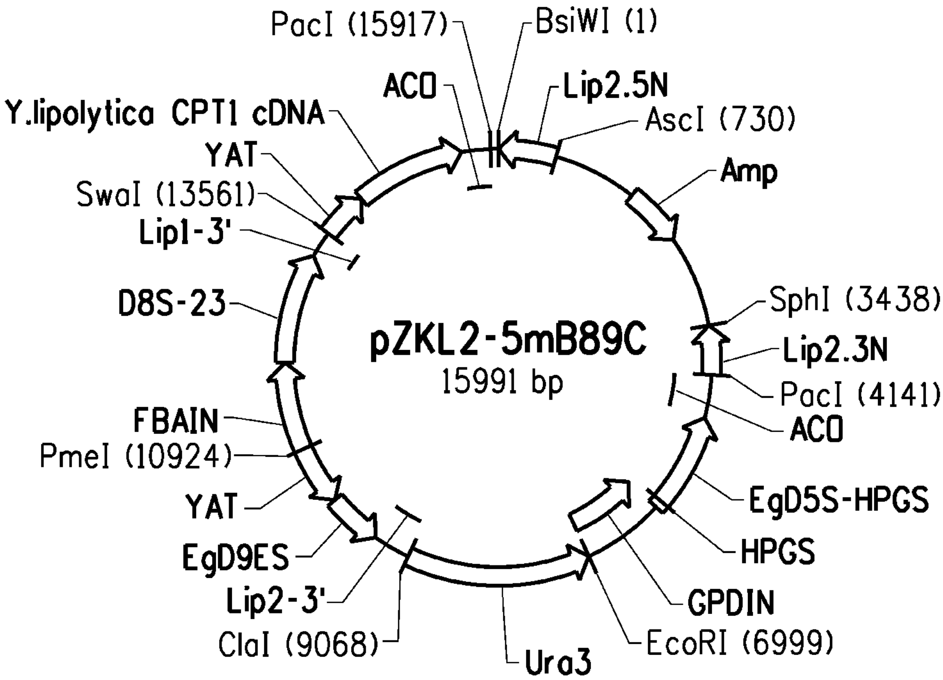
PacI (15917)
BsiWI (1)
ACO
Lip2.5N
AscI (730)
Y.lipolytica CPT1 cDNA
YAT
SwaI (13561)
Lip1-3'
Amp
D8S-23
SphI (3438)
pZKL2-5mB89C
15991 bp
Lip2.3N
PacI (4141)
ACO
FBAIN
PmeI (10924)
YAT
EgD5S-HPGS
HPGS
EgD9ES
Lip2-3'
GPDIN
ClaI (9068)
EcoRI (6999)
Ura3

FIG. 5B

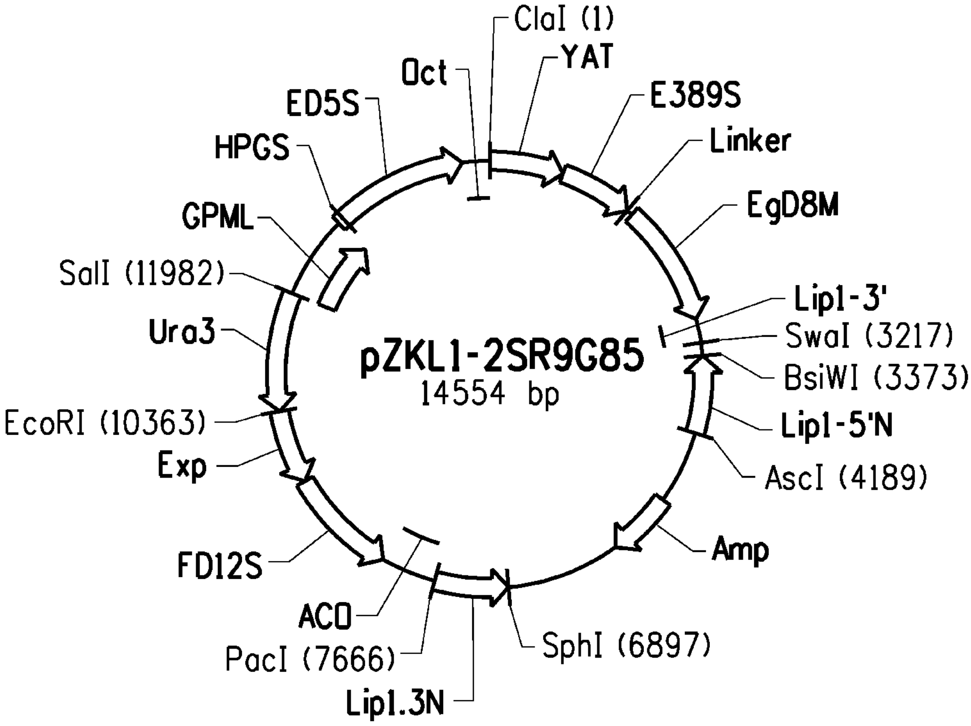
ClaI (1)
YAT
E389S
Linker
EgD8M
Lip1-3'
SwaI (3217)
BsiWI (3373)
Lip1-5'N
AscI (4189)
Amp
SphI (6897)
Lip1.3N
PacI (7666)
ACO
FD12S
Exp
EcoRI (10363)
Ura3
SalI (11982)
GPML
HPGS
ED5S
Oct
pZKL1-2SR9G85
14554 bp

FIG. 6A

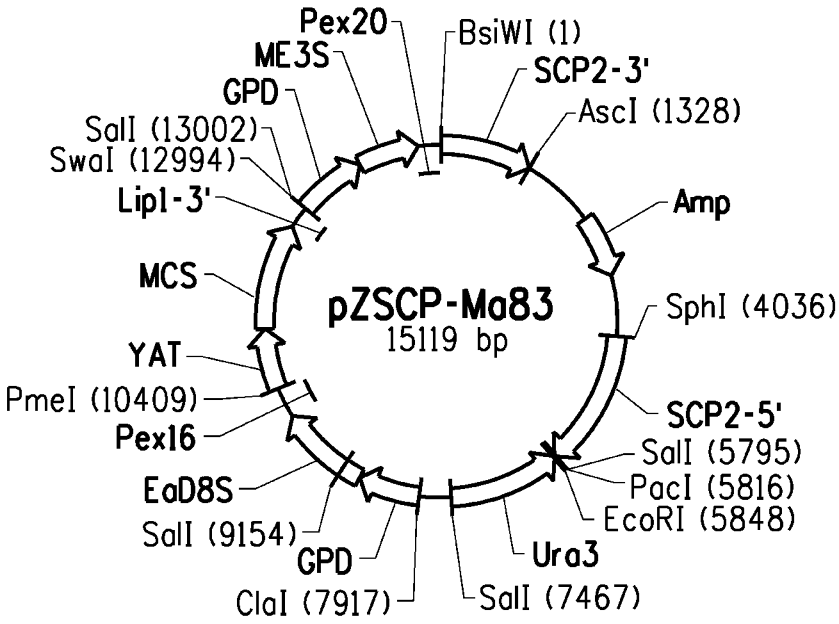
BsiWI (1)
SCP2-3'
AscI (1328)
Amp
SphI (4036)
SCP2-5'
SalI (5795)
PacI (5816)
EcoRI (5848)
Ura3
SalI (7467)
ClaI (7917)
GPD
SalI (9154)
EaD8S
Pex16
PmeI (10409)
YAT
MCS
Lip1-3'
SwaI (12994)
SalI (13002)
GPD
ME3S
Pex20
pZSCP-Ma83
15119 bp

FIG. 6B

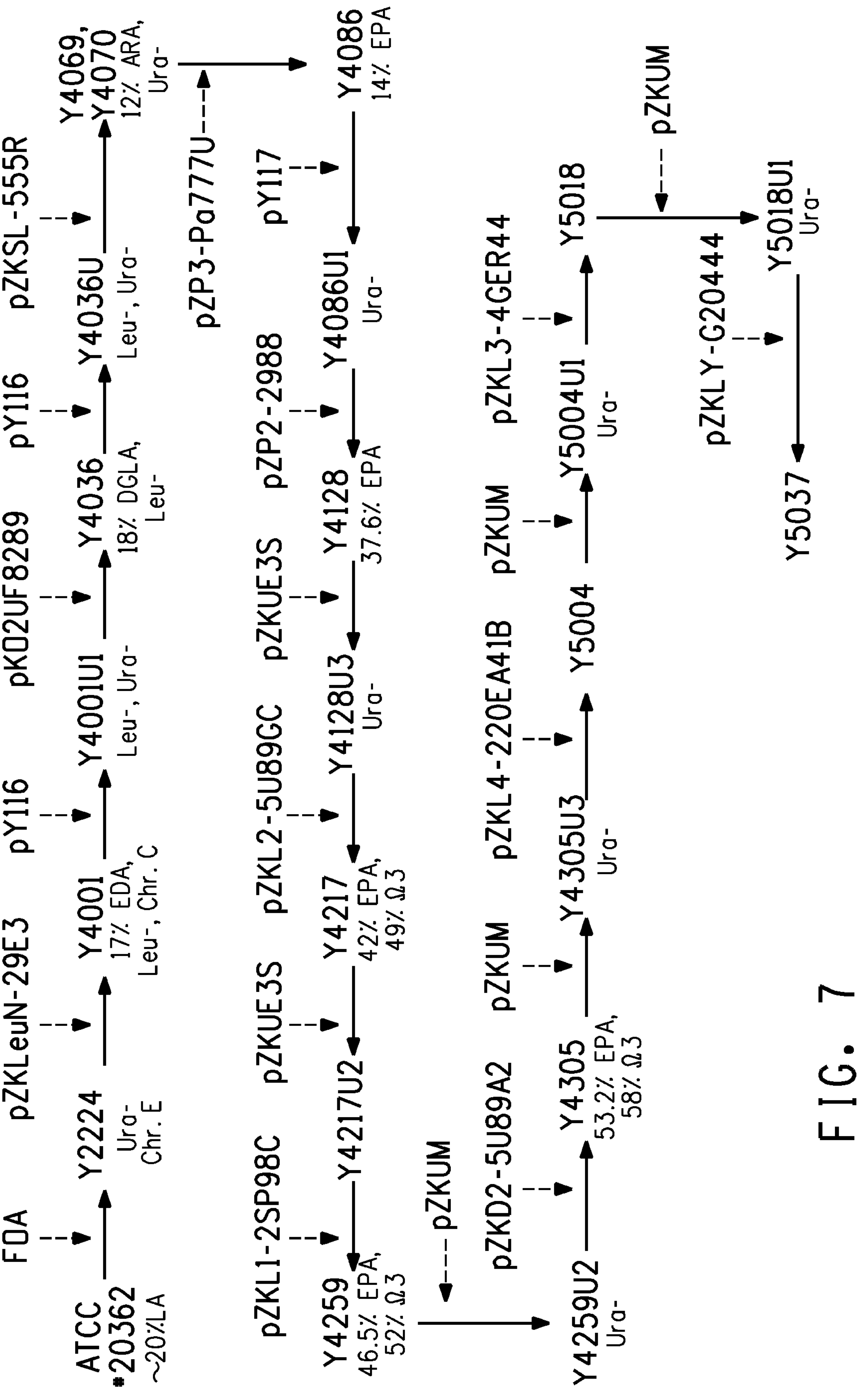
ATCC
#20362
~20%LA
FOA
Y2224
Ura-
Chr. E
pZKLeuN-29E3
Y4001
17% EDA,
Leu-, Chr. C
pY116
Y4001U1
Leu-, Ura-
pKO2UF8289
Y4036
18% DGLA,
Leu-
pY116
Y4036U
Leu-, Ura-
pZKSL-555R
Y4069,
Y4070
12% ARA,
Ura-
pZP3-Pa777U
Y4086
14% EPA
pY117
Y4086U1
Ura-
pZP2-2988
Y4128
37.6% EPA
pZKUE3S
Y4128U3
Ura-
pZKL2-5U89GC
Y4217
42% EPA,
49% Ω3
pZKUE3S
Y4217U2
pZKL1-2SP98C
Y4259
46.5% EPA,
52% Ω3
pZKUM
Y4259U2
Ura-
pZKD2-5U89A2
Y4305
53.2% EPA,
58% Ω3
pZKUM
Y4305U3
Ura-
pZKL4-220EA41B
Y5004
pZKUM
Y5004U1
Ura-
pZKL3-4GER44
Y5018
pZKUM
Y5018U1
Ura-
pZKLY-G20444
Y5037

FIG. 7

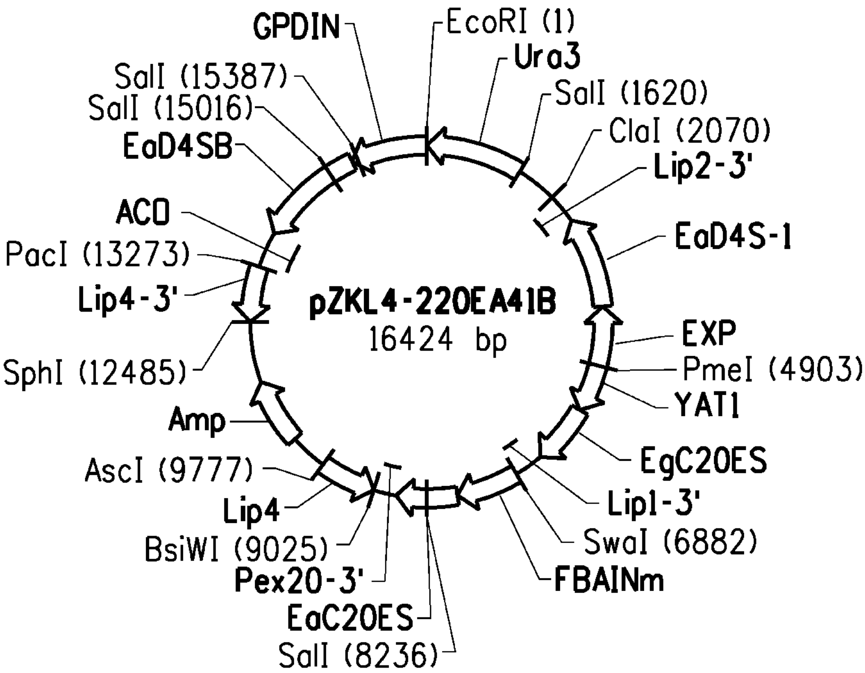
GPDIN
EcoRI (1)
Ura3
SalI (15387)
SalI (1620)
SalI (15016)
ClaI (2070)
EaD4SB
Lip2-3'
ACO
EaD4S-1
PacI (13273)
Lip4-3'
pZKL4-220EA41B
16424 bp
EXP
SphI (12485)
PmeI (4903)
YAT1
Amp
EgC20ES
AscI (9777)
Lip1-3'
Lip4
SwaI (6882)
BsiWI (9025)
FBAINm
Pex20-3'
EaC20ES
SalI (8236)

FIG. 8A

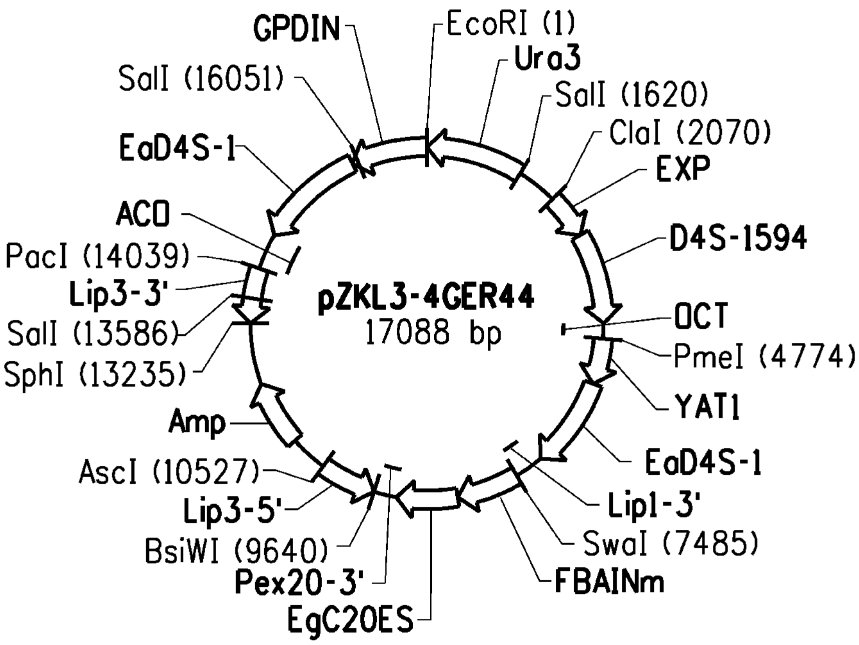
GPDIN
EcoRI (1)
Ura3
SalI (16051)
SalI (1620)
ClaI (2070)
EaD4S-1
EXP
ACO
D4S-1594
PacI (14039)
Lip3-3'
pZKL3-4GER44
17088 bp
OCT
SalI (13586)
PmeI (4774)
SphI (13235)
YAT1
Amp
EaD4S-1
AscI (10527)
Lip1-3'
Lip3-5'
SwaI (7485)
BsiWI (9640)
FBAINm
Pex20-3'
EgC20ES

FIG. 8B

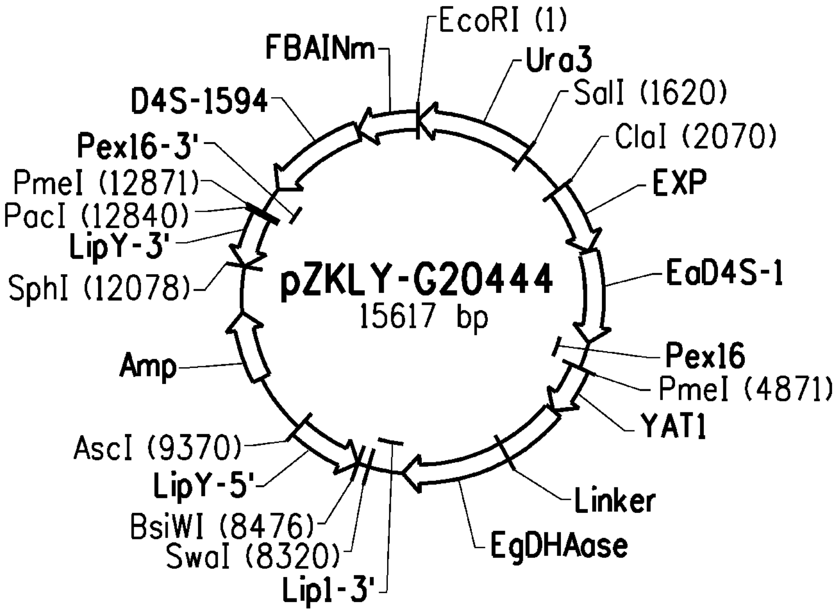
EcoRI (1)
Ura3
SalI (1620)
ClaI (2070)
EXP
EaD4S-1
Pex16
PmeI (4871)
YAT1
Linker
EgDHAase
Lip1-3'
SwaI (8320)
BsiWI (8476)
LipY-5'
AscI (9370)
Amp
SphI (12078)
LipY-3'
PacI (12840)
PmeI (12871)
Pex16-3'
D4S-1594
FBAINm
pZKLY-G20444
15617 bp

FIG. 9

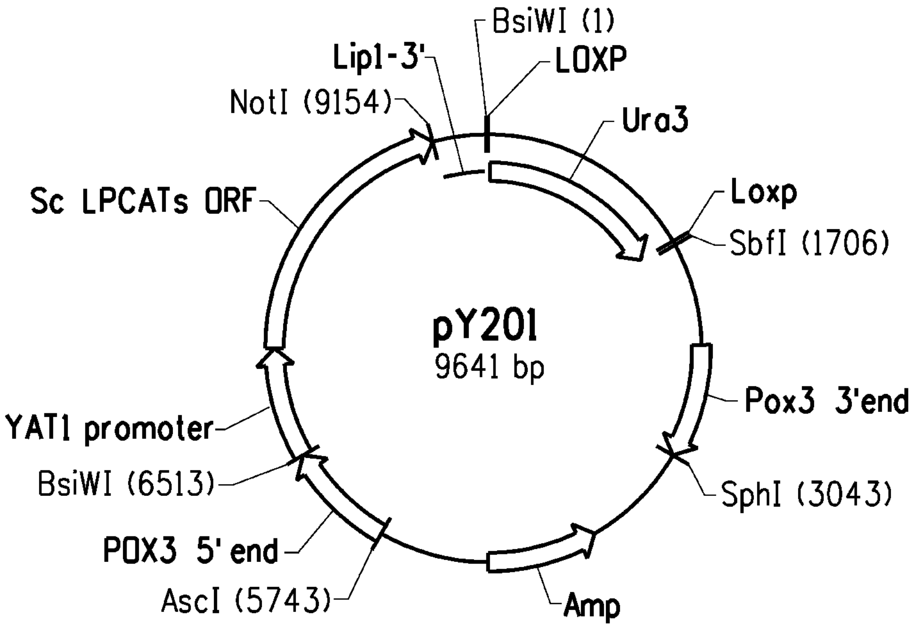


FIG. 10A

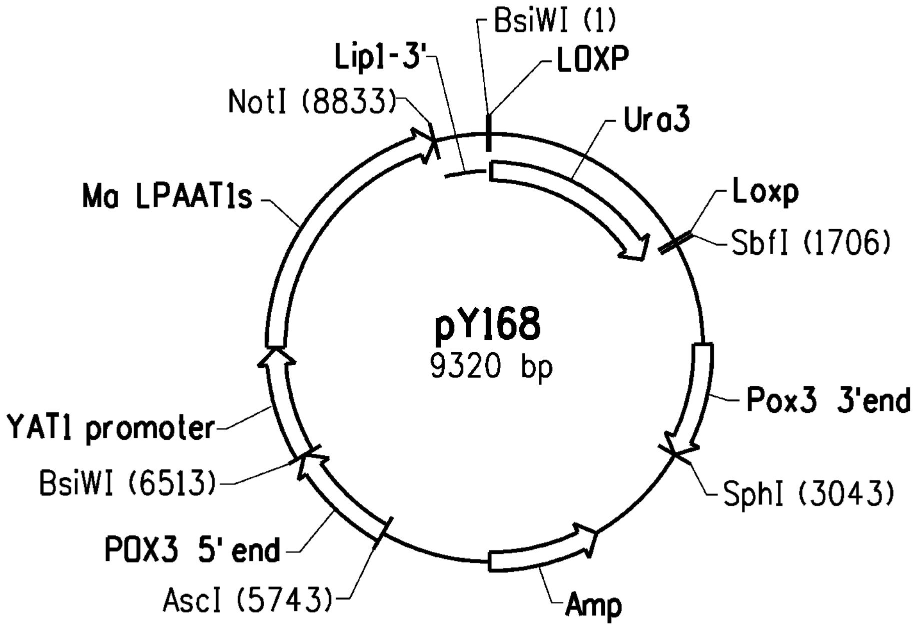


FIG. 10B

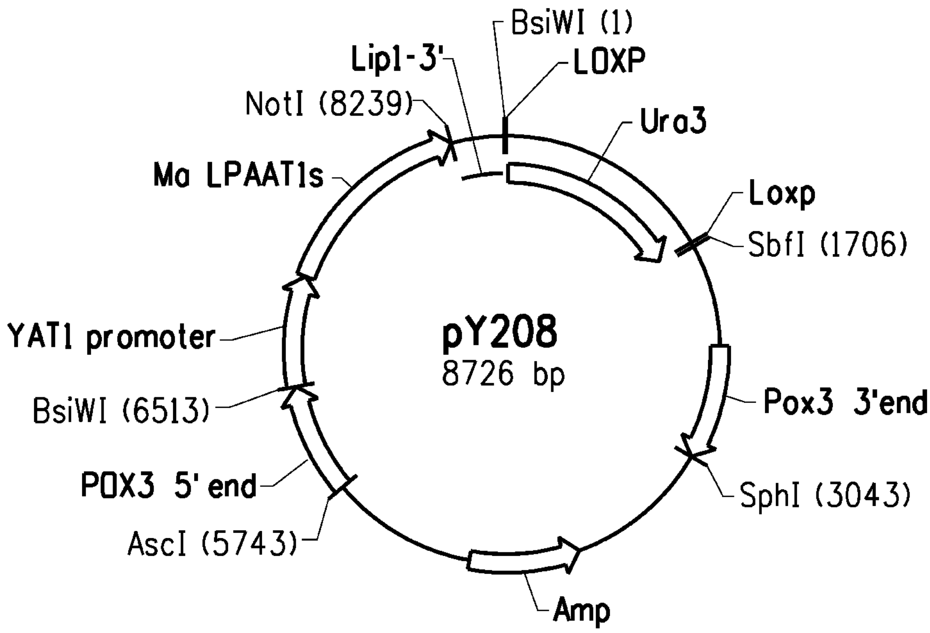


FIG. 11A

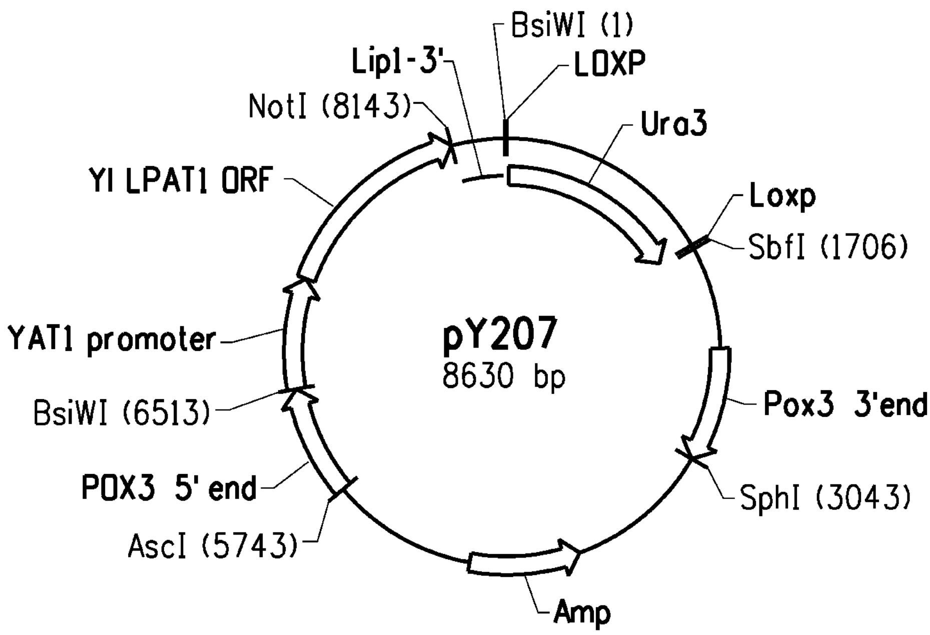


FIG. 11B

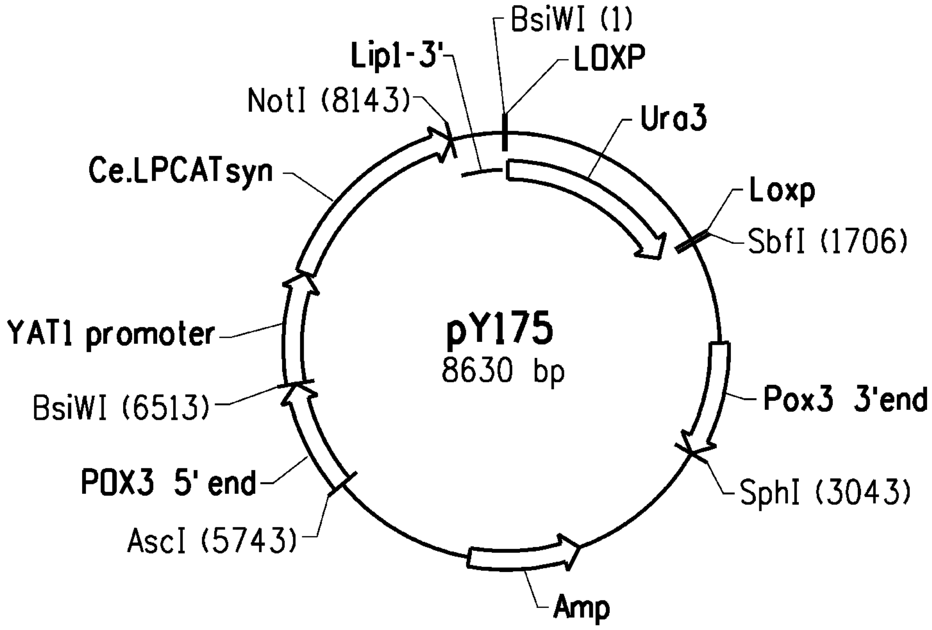


FIG. 12A

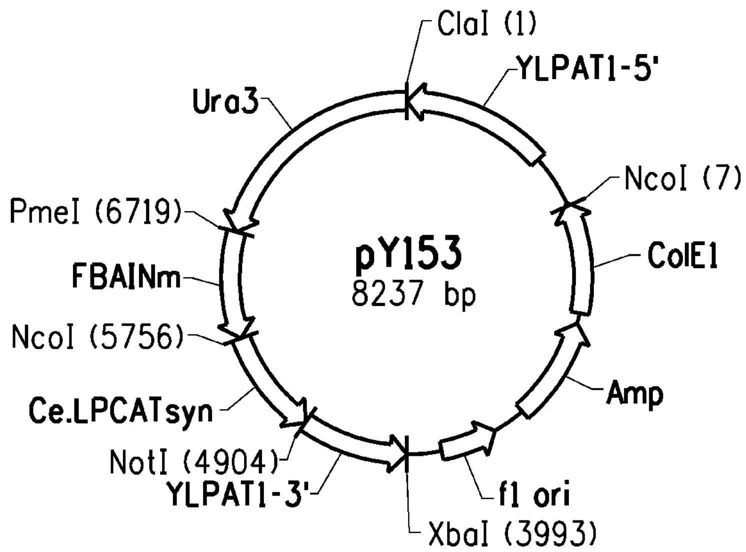


FIG. 12B

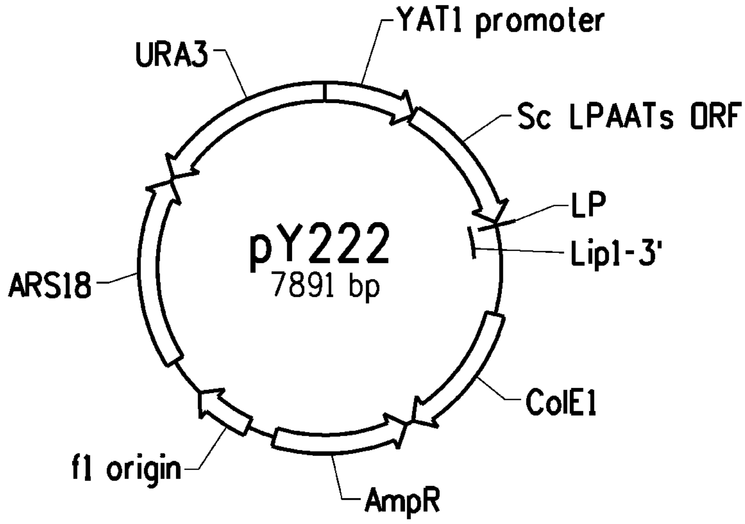


FIG. 13A

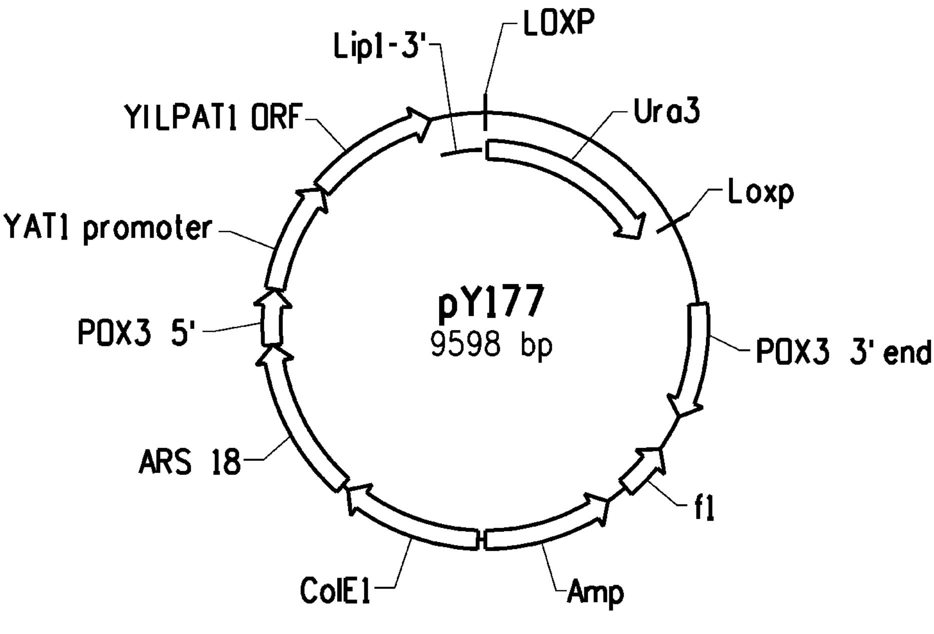


FIG. 13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004076617 A
- WO 2004087902 A
- WO 2006069936 A
- WO 2006052870 A
- WO 2009001315 A
- WO 2009014140 A
- US 20080145867 A1
- US 20100075386 A1
- US 7709239 B
- US 7256033 B
- US 7238482 B
- WO 2005047480 A
- US 7125672 B
- US 4683202 A
- US 20060115881 A1
- WO 2004076617 A2
- US 4910141 A
- US 7259255 B
- US 7001772 B
- US 20070015237 A1
- US 20060094092 A1
- US 20090093543 A1
- US 20060110806 A1
- US 64192909 A
- US 6797303 B
- US 5648564 A
- US 20060094092 A
- US 5366860 A
- EP 272007 A
- US 20080254191 A
- US 7504259 B
- US 7470532 B
- US 7645604 B
- WO 2009046248 A
- WO 2008073367 A
- US 7202356 B
- US 7678560 B
- US 20060094102 A1
- US 20080274521 A1
- WO 200605287 A
- US 7556949 B
- US 7459546 B
- US 20080254191 A1
- US 637877 A
- US 20080254521 A1
- US 7695950 B
- US 20090253188 A1
- US 20080145867 A
- WO 2008076377 A
- US 20090325265 A1
- US 7189559 B
- US 20060168687 A1
- US 61187359 B
- WO 2008073271 A
- US 20080138868 A1
- WO 2007061742 A
- US 20070117190 A1
- WO 2008124048 A
- WO 2005047485 A
- US 20050216975 A1
- WO 2007046817 A
- WO 2008124194 A

### Non-patent literature cited in the description

- **KENNEDY ; WEISS.** *J. Biol. Chem.,* 1956, vol. 222, 193-214
- **LANDS, W.E.** *J. Biol. Chem.,* 1958, vol. 231, 883-888
- **STYMNE S. ; A.K. STOBART.** *Biochem J.,* 1984, vol. 223 (2), 305-314
- *J. Biol. Chem.,* 2009, vol. 284 (1), 1-5
- *J. Lipid Res.,* 2009, vol. 50, S46-S51
- **TAMAKI, H. et al.** *J. Biol. Chem.,* 2007, vol. 282, 34288-34298
- **STAHL, U. et al.** *FEBS Letters,* 2008, vol. 582, 305-309
- **CHEN, Q. et al.** *FEBS Letters,* 2007, vol. 581, 5511-5516
- **BENGHEZAL, M. et al.** *J. Biol. Chem.,* 2007, vol. 282, 30845-30855

- **RIEKHOF et al.** *J. Biol. Chem.,* 2007, vol. 282, 28344-28352
- **HISHIKAWA et al.** *Proc. Natl. Acad. Sci. U.S.A.,* 2008, vol. 105, 2830-2835
- **WEETE.** Fungal Lipid Biochemistry. Plenum, 1980
- **YONGMANITCHAI ; WARD.** *Appl. Environ. Microbiol.,* 1991, vol. 57, 419-25
- **SAMBROOK, J. ; FRITSCH, E. F. ; MANIATIS, T.** Molecular Cloning: A Laboratory Manual. Cold Spring Harbor Laboratory, 1989
- **ALTSCHUL, S. F. et al.** Basic Local Alignment Search Tool. *J. Mol. Biol.,* 1993, vol. 215, 403-410
- Part I, Chapter 2 "Overview of principles of hybridization and the strategy of nucleic acid probe assays. **TIJSSEN.** Laboratory Techniques in Biochemistry and Molecular Biology-Hybridization with Nucleic Acid Probes. Elsevier, 1993
- Current Protocols in Molecular Biology. Greene Publishing and Wiley-Interscience, 1995
- Computational Molecular Biology. Oxford University, 1988
- Biocomputing: Informatics and Genome Projects. Academic, 1993
- Computer Analysis of Sequence Data. Humania, 1994
- Sequence Analysis in Molecular Biology. Academic, 1987
- Sequence Analysis Primer. Stockton, 1991
- **HIGGINS ; SHARP.** *CABIOS,* 1989, vol. 5, 151-153
- **HIGGINS, D.G. et al.** *Comput. Appl. Biosci.,* 1992, vol. 8, 189-191
- **ALTSCHUL et al.** *J. Mol. Biol.,* 1990, vol. 215, 403-410
- Comput. Methods Genome Res. **W. R. PEARSON.** Proc. Int. Symp. Plenum, 1992, 111-20
- **DUJON, B. et al.** *Nature,* 2004, vol. 430 (6995), 35-44
- **ALTSCHUL et al.** *Nucleic Acids Res.,* 1997, vol. 25, 3389-3402
- **SHINDOU et al.** *Biochem. Biophys. Res. Comm.,* 2009, vol. 383, 320-325
- **SIMILARLY ; LEWIN, T.W. et al.** *Biochemistry,* 1999, vol. 38, 5764-5771
- **YAMASHITA et al.** *Biochim, Biophys. Acta,* 2007, vol. 1771, 1202-1215
- **TABOR, S. et al.** *Proc. Natl. Acad. Sci. U.S.A.,* 1985, vol. 82, 1074
- **WALKER et al.** *Proc. Natl. Acad. Sci. U.S.A.,* 1992, vol. 89, 392
- The use of oligonucleotides as specific hybridization probes in the Diagnosis of Genetic Disorders. Human Genetic Diseases: A Practical Approach. 1986, 33-50
- **HERNDON, VA ; RYCHLIK, W.** Methods in Molecular Biology. 1993, vol. 15, 31-39
- **FROHMAN et al.** *Proc. Natl. Acad. Sci. U.S.A.,* 1988, vol. 85, 8998
- **OHARA et al.** *Proc. Natl. Acad. Sci. U.S.A.,* 1989, vol. 86, 5673
- **LOH et al.** *Science,* 1989, vol. 243, 217
- **DOMERGUE, F. et al.** *J. Bio. Chem.,* 2003, vol. 278, 35115
- **ABBADI, A. et al.** *The Plant Cell,* 2004, vol. 16, 2734-2748
- **SILHAVY, T. J. ; BENNAN, M. L. ; ENQUIST, L. W.** Experiments with Gene Fusions. Cold Spring Harbor Laboratory, 1984
- **AUSUBEL, F. M. et al.** Current Protocols in Molecular Biology. Greene Publishing Assoc. and Wiley-Interscience, 1987
- *Methods in Enzymology,* 1991, vol. 194, 186-187
- **JURETZEK et al.** *Yeast,* 2001, vol. 18, 97-113
- **SOUTHERN.** *J. Mol. Biol.,* 1975, vol. 98, 503
- **KROCZEK.** *J. Chromatogr. Biomed. Appl.,* 1993, vol. 618 (1-2), 133-145
- **MACKENZIE et al.** *Appl. Environ. Microbiol.,* 2000, vol. 66, 4655
- **PAPANIKOLAOU S. ; AGGELIS G.** *Bioresour. Technol.,* 2002, vol. 82 (1), 43-9
- **NAKAHARA, T. et al.** Ind. Appl. Single Cell Oils. 1992, 61-97
- **Z. JACOBS.** *Critical Reviews in Biotechnology,* 1992, vol. 12 (5/6), 463-491
- **A. SINGH ; O. WARD.** *Adv. Appl. Microbiol.,* 1997, vol. 45, 271-312
- **T. J. SILHAVY ; M. L. BENNAN ; L. W. ENQUIST.** Experiments with Gene Fusions. Cold Spring Harbor Laboratory, 1984
- Manual of Methods for General Bacteriology. American Society for Microbiology, 1994
- **THOMAS D.** Brock in Biotechnology: A Textbook of Industrial Microbiology. Sinauer Associates: Sunderland, 1989
- **BLIGH, E. G. ; DYER, W. J.** *Can. J. Biochem. Physiol.,* 1959, vol. 37, 911-917
- **ROUGHAN, G. ; NISHIDA I.** *Arch Biochem Biophys.,* 1990, vol. 276 (1), 38-46
- **THOMPSON et al.** *Nucleic Acids Res.,* 1994, vol. 22, 4673-4680
- **ALTSCHUL et al.** *FEBS J.,* 2005, vol. 272, 5101-5109

SEQUENCE LISTING

<110> E.I. duPont de Nemours & Company, Inc.
Yadav, Narendra

<120> IMPROVEMENT OF LONG CHAIN OMEGA-3 AND OMEGA-6 POLYUNSATURATED FATTY ACID BIOSYNTHESIS BY EXPRESSION OF LYSOPHOSPHOLIPID ACYLTRANSFERASES

<130> CL4361

<150> US 61/187,359
<151> 2009-06-16

<160> 101

<170> PatentIn version 3.5

<210> 1
<211> 849
<212> DNA
<213> Caenorhabditis elegans

<220>
<221> CDS
<222> (1)..(849)
<223> GenBank Accession No. CAA98276; "clone T06E8.1"

<300>
<302> METHOD FOR THE PRODUCTION OF POLYUNSATURATED FATTY ACIDS
<310> US 2006-0168687-A1
<311> 2004-01-29
<312> 2006-07-27
<313> (1)..(849)

<300>
<302> METHOD FOR THE PRODUCTION OF POLYUNSATURATED FATTY ACIDS
<310> WO 2004/076617
<311> 2004-01-29
<312> 2004-09-10
<313> (1)..(849)

<400> 1

```
atg gag aac ttc tgg tcg atc gtc gtg ttt ttt cta ctc tca att ctc       48
Met Glu Asn Phe Trp Ser Ile Val Val Phe Phe Leu Leu Ser Ile Leu
1               5                   10                  15

ttc att tta tat aac ata tcg aca gta tgc cac tac tat atg cgg att       96
Phe Ile Leu Tyr Asn Ile Ser Thr Val Cys His Tyr Tyr Met Arg Ile
            20                  25                  30

tcg ttt tat tac ttc aca att tta ttg cat gga atg gaa gtt tgt gtt      144
Ser Phe Tyr Tyr Phe Thr Ile Leu Leu His Gly Met Glu Val Cys Val
        35                  40                  45

aca atg atc cct tct tgg cta aat ggg aag ggt gct gat tac gtg ttt      192
Thr Met Ile Pro Ser Trp Leu Asn Gly Lys Gly Ala Asp Tyr Val Phe
    50                  55                  60

cac tcg ttt ttc tat tgg tgt aaa tgg act ggt gtt cat aca aca gtc      240
His Ser Phe Phe Tyr Trp Cys Lys Trp Thr Gly Val His Thr Thr Val
65                  70                  75                  80

tat gga tat gaa aaa aca caa gtt gaa ggt ccg gct gta gtt att tgt      288
```

```
Tyr Gly Tyr Glu Lys Thr Gln Val Glu Gly Pro Ala Val Val Ile Cys
                85                  90                  95

aat cat cag agt tct ctc gac att cta tcg atg gca tca atc tgg ccg        336
Asn His Gln Ser Ser Leu Asp Ile Leu Ser Met Ala Ser Ile Trp Pro
            100                 105                 110

aag aat tgt gtt gta atg atg aaa cga att ctt gcc tat gtt cca ttc        384
Lys Asn Cys Val Val Met Met Lys Arg Ile Leu Ala Tyr Val Pro Phe
        115                 120                 125

ttc aat ctc gga gcc tac ttt tcc aac aca atc ttc atc gat cga tat        432
Phe Asn Leu Gly Ala Tyr Phe Ser Asn Thr Ile Phe Ile Asp Arg Tyr
    130                 135                 140

aac cgt gaa cgt gcg atg gct tca gtt gat tat tgt gca tct gaa atg        480
Asn Arg Glu Arg Ala Met Ala Ser Val Asp Tyr Cys Ala Ser Glu Met
145                 150                 155                 160

aag aac aga aat ctt aaa ctt tgg gta ttt ccg gaa gga aca aga aat        528
Lys Asn Arg Asn Leu Lys Leu Trp Val Phe Pro Glu Gly Thr Arg Asn
                165                 170                 175

cgt gaa gga ggg ttc att cca ttc aag aaa gga gca ttc aat att gca        576
Arg Glu Gly Gly Phe Ile Pro Phe Lys Lys Gly Ala Phe Asn Ile Ala
            180                 185                 190

gtt cgt gcg cag att ccc att att cca gtt gta ttc tca gac tat cgg        624
Val Arg Ala Gln Ile Pro Ile Ile Pro Val Val Phe Ser Asp Tyr Arg
        195                 200                 205

gat ttc tac tca aag cca ggc cga tat ttc aag aat gat gga gaa gtt        672
Asp Phe Tyr Ser Lys Pro Gly Arg Tyr Phe Lys Asn Asp Gly Glu Val
    210                 215                 220

gtt att cga gtt ctg gat gcg att cca aca aaa ggg ctc act ctt gat        720
Val Ile Arg Val Leu Asp Ala Ile Pro Thr Lys Gly Leu Thr Leu Asp
225                 230                 235                 240

gac gtc agc gag ttg tct gat atg tgt cgg gac gtt atg ttg gca gcc        768
Asp Val Ser Glu Leu Ser Asp Met Cys Arg Asp Val Met Leu Ala Ala
                245                 250                 255

tat aag gaa gtt act cta gaa gct cag caa cga aat gcg aca cgg cgt        816
Tyr Lys Glu Val Thr Leu Glu Ala Gln Gln Arg Asn Ala Thr Arg Arg
            260                 265                 270

gga gaa aca aaa gac ggg aag aaa tct gag taa                            849
Gly Glu Thr Lys Asp Gly Lys Lys Ser Glu
        275                 280
```

<210> 2
<211> 282
<212> PRT
<213> Caenorhabditis elegans

<400> 2

```
Met Glu Asn Phe Trp Ser Ile Val Val Phe Phe Leu Leu Ser Ile Leu
1               5                   10                  15

Phe Ile Leu Tyr Asn Ile Ser Thr Val Cys His Tyr Tyr Met Arg Ile
            20                  25                  30
```

```
Ser Phe Tyr Tyr Phe Thr Ile Leu Leu His Gly Met Glu Val Cys Val
        35                  40                  45

Thr Met Ile Pro Ser Trp Leu Asn Gly Lys Gly Ala Asp Tyr Val Phe
    50                  55                  60

His Ser Phe Phe Tyr Trp Cys Lys Trp Thr Gly Val His Thr Thr Val
65                  70                  75                  80

Tyr Gly Tyr Glu Lys Thr Gln Val Glu Gly Pro Ala Val Val Ile Cys
                85                  90                  95

Asn His Gln Ser Ser Leu Asp Ile Leu Ser Met Ala Ser Ile Trp Pro
            100                 105                 110

Lys Asn Cys Val Val Met Met Lys Arg Ile Leu Ala Tyr Val Pro Phe
        115                 120                 125

Phe Asn Leu Gly Ala Tyr Phe Ser Asn Thr Ile Phe Ile Asp Arg Tyr
    130                 135                 140

Asn Arg Glu Arg Ala Met Ala Ser Val Asp Tyr Cys Ala Ser Glu Met
145                 150                 155                 160

Lys Asn Arg Asn Leu Lys Leu Trp Val Phe Pro Glu Gly Thr Arg Asn
                165                 170                 175

Arg Glu Gly Gly Phe Ile Pro Phe Lys Lys Gly Ala Phe Asn Ile Ala
            180                 185                 190

Val Arg Ala Gln Ile Pro Ile Ile Pro Val Val Phe Ser Asp Tyr Arg
        195                 200                 205

Asp Phe Tyr Ser Lys Pro Gly Arg Tyr Phe Lys Asn Asp Gly Glu Val
    210                 215                 220

Val Ile Arg Val Leu Asp Ala Ile Pro Thr Lys Gly Leu Thr Leu Asp
225                 230                 235                 240

Asp Val Ser Glu Leu Ser Asp Met Cys Arg Asp Val Met Leu Ala Ala
                245                 250                 255

Tyr Lys Glu Val Thr Leu Glu Ala Gln Gln Arg Asn Ala Thr Arg Arg
            260                 265                 270

Gly Glu Thr Lys Asp Gly Lys Lys Ser Glu
        275                 280
```

<210> 3
<211> 7
<212> PRT
<213> Artificial Sequence

<220>
<223> membrane bound O-acyltransferase motif

<220>
<221> MISC_FEATURE
<222> (2)..(2)
<223> Xaa = Val [V] or Ile [I]

<220>
<221> misc_feature
<222> (4)..(5)
<223> Xaa can be any naturally occurring amino acid

<400> 3

```
Met Xaa Leu Xaa Xaa Lys Leu
1               5
```

<210> 4
<211> 8
<212> PRT
<213> Artificial Sequence

<220>
<223> membrane bound O-acyltransferase motif

<220>
<221> misc_feature
<222> (2)..(2)
<223> Xaa can be any naturally occurring amino acid

<220>
<221> misc_feature
<222> (6)..(7)
<223> Xaa can be any naturally occurring amino acid

<400> 4

```
Arg Xaa Lys Tyr Tyr Xaa Xaa Trp
1               5
```

<210> 5
<211> 6
<212> PRT
<213> Artificial Sequence

<220>
<223> membrane bound O-acyltransferase motif

<220>
<221> misc_feature
<222> (3)..(3)
<223> Xaa can be any naturally occurring amino acid

<400> 5

Ser Ala Xaa Trp His Gly
1 5

<210> 6
<211> 859
<212> DNA
<213> Caenorhabditis elegans

<220>
<221> CDS
<222> (3)..(851)
<223> synthetic LPCAT (codon-optimized for Yarrowia lipolytica)

<400> 6

```
cc atg gag aac ttc tgg tcc atc gtc gtg ttc ttt ctg ctc tcc att          47
   Met Glu Asn Phe Trp Ser Ile Val Val Phe Phe Leu Leu Ser Ile
   1               5                   10                  15

ctg ttc atc ctc tac aac att tcg aca gtc tgc cac tac tac atg cga         95
Leu Phe Ile Leu Tyr Asn Ile Ser Thr Val Cys His Tyr Tyr Met Arg
                20                  25                  30

atc tcc ttc tac tac ttt acc atc ctg ctt cac ggc atg gag gtg tgc        143
Ile Ser Phe Tyr Tyr Phe Thr Ile Leu Leu His Gly Met Glu Val Cys
            35                  40                  45

gtt acc atg att ccc tct tgg ctc aac ggc aag ggt gcc gac tac gtg        191
Val Thr Met Ile Pro Ser Trp Leu Asn Gly Lys Gly Ala Asp Tyr Val
        50                  55                  60

ttt cac tcg ttc ttc tac tgg tgc aag tgg act gga gtc cac acc act        239
Phe His Ser Phe Phe Tyr Trp Cys Lys Trp Thr Gly Val His Thr Thr
    65                  70                  75

gtg tat ggc tac gag aag acc cag gtc gaa ggt cct gcc gtg gtc atc        287
Val Tyr Gly Tyr Glu Lys Thr Gln Val Glu Gly Pro Ala Val Val Ile
80                  85                  90                  95

tgc aac cat cag tcc tcg ctc gac att ctg tct atg gct tcc atc tgg        335
Cys Asn His Gln Ser Ser Leu Asp Ile Leu Ser Met Ala Ser Ile Trp
                100                 105                 110

ccc aag aac tgt gtt gtc atg atg aag cgg att ctt gcc tac gtt ccc        383
Pro Lys Asn Cys Val Val Met Met Lys Arg Ile Leu Ala Tyr Val Pro
            115                 120                 125

ttc ttc aac ctg gga gcc tac ttt tcc aac acc atc ttc atc gac cga        431
Phe Phe Asn Leu Gly Ala Tyr Phe Ser Asn Thr Ile Phe Ile Asp Arg
        130                 135                 140

tac aac cga gag cga gct atg gct tct gtc gac tac tgt gcc tcc gag        479
Tyr Asn Arg Glu Arg Ala Met Ala Ser Val Asp Tyr Cys Ala Ser Glu
    145                 150                 155

atg aag aac cga aac ctg aag ctc tgg gtg ttt ccc gaa ggc act cgg        527
Met Lys Asn Arg Asn Leu Lys Leu Trp Val Phe Pro Glu Gly Thr Arg
160                 165                 170                 175

aat cga gag ggt gga ttc att ccc ttc aag aaa ggt gcc ttc aac atc        575
Asn Arg Glu Gly Gly Phe Ile Pro Phe Lys Lys Gly Ala Phe Asn Ile
                180                 185                 190
```

```
gct gtt cga gcc cag att ccc atc att cct gtc gtg ttc tct gac tat        623
Ala Val Arg Ala Gln Ile Pro Ile Ile Pro Val Val Phe Ser Asp Tyr
            195                 200                 205

cga gac ttc tac tcc aag cct ggc cga tac ttc aag aac gat gga gag        671
Arg Asp Phe Tyr Ser Lys Pro Gly Arg Tyr Phe Lys Asn Asp Gly Glu
        210                 215                 220

gtc gtg atc cga gtc ctg gat gcc att ccc acc aag ggt ctg acc ctc        719
Val Val Ile Arg Val Leu Asp Ala Ile Pro Thr Lys Gly Leu Thr Leu
    225                 230                 235

gat gac gtc tct gag ctt tcg gac atg tgt cga gac gtc atg ctg gct        767
Asp Asp Val Ser Glu Leu Ser Asp Met Cys Arg Asp Val Met Leu Ala
240                 245                 250                 255

gcc tac aag gaa gtt acc ctc gag gct cag caa cga aac gcc act cga        815
Ala Tyr Lys Glu Val Thr Leu Glu Ala Gln Gln Arg Asn Ala Thr Arg
                260                 265                 270

aga gga gag acc aag gac ggc aag aaa tcc gag taa gcggccgc               859
Arg Gly Glu Thr Lys Asp Gly Lys Lys Ser Glu
            275                 280
```

<210> 7
<211> 282
<212> PRT
<213> Caenorhabditis elegans

<400> 7

```
Met Glu Asn Phe Trp Ser Ile Val Val Phe Phe Leu Leu Ser Ile Leu
1               5                   10                  15

Phe Ile Leu Tyr Asn Ile Ser Thr Val Cys His Tyr Tyr Met Arg Ile
            20                  25                  30

Ser Phe Tyr Tyr Phe Thr Ile Leu Leu His Gly Met Glu Val Cys Val
        35                  40                  45

Thr Met Ile Pro Ser Trp Leu Asn Gly Lys Gly Ala Asp Tyr Val Phe
    50                  55                  60

His Ser Phe Phe Tyr Trp Cys Lys Trp Thr Gly Val His Thr Thr Val
65                  70                  75                  80

Tyr Gly Tyr Glu Lys Thr Gln Val Glu Gly Pro Ala Val Val Ile Cys
                85                  90                  95

Asn His Gln Ser Ser Leu Asp Ile Leu Ser Met Ala Ser Ile Trp Pro
            100                 105                 110

Lys Asn Cys Val Val Met Met Lys Arg Ile Leu Ala Tyr Val Pro Phe
        115                 120                 125
```

```
Phe Asn Leu Gly Ala Tyr Phe Ser Asn Thr Ile Phe Ile Asp Arg Tyr
    130                 135                 140

Asn Arg Glu Arg Ala Met Ala Ser Val Asp Tyr Cys Ala Ser Glu Met
145                 150                 155                 160

Lys Asn Arg Asn Leu Lys Leu Trp Val Phe Pro Glu Gly Thr Arg Asn
                165                 170                 175

Arg Glu Gly Gly Phe Ile Pro Phe Lys Lys Gly Ala Phe Asn Ile Ala
            180                 185                 190

Val Arg Ala Gln Ile Pro Ile Ile Pro Val Val Phe Ser Asp Tyr Arg
        195                 200                 205

Asp Phe Tyr Ser Lys Pro Gly Arg Tyr Phe Lys Asn Asp Gly Glu Val
    210                 215                 220

Val Ile Arg Val Leu Asp Ala Ile Pro Thr Lys Gly Leu Thr Leu Asp
225                 230                 235                 240

Asp Val Ser Glu Leu Ser Asp Met Cys Arg Asp Val Met Leu Ala Ala
                245                 250                 255

Tyr Lys Glu Val Thr Leu Glu Ala Gln Gln Arg Asn Ala Thr Arg Arg
            260                 265                 270

Gly Glu Thr Lys Asp Gly Lys Lys Ser Glu
        275                 280
```

<210> 8
<211> 1860
<212> DNA
<213> Saccharomyces cerevisiae

<220>
<221> CDS
<222> (1)..(1860)
<223> GenBank Accession No. NP_014818; "YOR175C"

<300>
<302> Genes encoding a novel type of lysophophatidylcholine acyltransferases and their use to increase triacylglycerol production and/or modify fatty acid composition
<310> US-2008-0145867-A1
<311> 2007-06-15
<312> 2008-06-19
<313> (1)..(1860)

<300>
<302> Genes encoding a novel type of lysophophatidylcholine acyltransferases and their use to increase triacylglycerol production and/or modify fatty acid composition

<310> WO 2008/076377
<311> 2007-12-13
<312> 2008-06-26
<313> (1)..(1860)

<300>
<302> USE OF A CLASS OF GENES ENCODING LYSOPHOSPHOLIPID ACYL TRANSFERASES FOR APPLICATION IN AGRICULTURE, BIOTECHNOLOGY AND MEDICINE
<310> WO 2009/001315
<311> 2008-06-25
<312> 2008-12-31
<313> (1)..(1860)

<400> 8

```
atg tac aat cct gtg gac gct gtt tta aca aag ata att acc aac tat        48
Met Tyr Asn Pro Val Asp Ala Val Leu Thr Lys Ile Ile Thr Asn Tyr
1               5                   10                  15

ggg att gat agt ttt aca ctg cga tat gct atc tgc tta ttg gga tcg        96
Gly Ile Asp Ser Phe Thr Leu Arg Tyr Ala Ile Cys Leu Leu Gly Ser
            20                  25                  30

ttc cca ctg aat gct att ttg aag aga att ccc gag aag cgt ata ggt       144
Phe Pro Leu Asn Ala Ile Leu Lys Arg Ile Pro Glu Lys Arg Ile Gly
        35                  40                  45

tta aaa tgt tgt ttt atc att tct atg tcg atg ttt tac tta ttc ggt       192
Leu Lys Cys Cys Phe Ile Ile Ser Met Ser Met Phe Tyr Leu Phe Gly
    50                  55                  60

gtg ctg aat cta gta agt gga ttc agg acc ctg ttt att agt acc atg       240
Val Leu Asn Leu Val Ser Gly Phe Arg Thr Leu Phe Ile Ser Thr Met
65                  70                  75                  80

ttt act tac ttg atc tca aga ttt tac cgt tcc aag ttt atg cca cac       288
Phe Thr Tyr Leu Ile Ser Arg Phe Tyr Arg Ser Lys Phe Met Pro His
                85                  90                  95

ttg aat ttc atg ttt gtt atg ggt cat ttg gca ata aat cat ata cac       336
Leu Asn Phe Met Phe Val Met Gly His Leu Ala Ile Asn His Ile His
            100                 105                 110

gcc caa ttc ctt aac gaa cag act caa act acc gtt gac att aca agt       384
Ala Gln Phe Leu Asn Glu Gln Thr Gln Thr Thr Val Asp Ile Thr Ser
        115                 120                 125

tca caa atg gtt tta gcc atg aaa cta act tct ttt gca tgg tcg tac       432
Ser Gln Met Val Leu Ala Met Lys Leu Thr Ser Phe Ala Trp Ser Tyr
    130                 135                 140

tat gat ggt tca tgc act agc gaa agc gat ttc aaa gat ttg act gag       480
Tyr Asp Gly Ser Cys Thr Ser Glu Ser Asp Phe Lys Asp Leu Thr Glu
145                 150                 155                 160

cat caa aaa tct cgt gct gtc aga ggt cat cca ccc tta tta aag ttc       528
His Gln Lys Ser Arg Ala Val Arg Gly His Pro Pro Leu Leu Lys Phe
                165                 170                 175

ctg gca tat gca ttt ttc tat tca acg ttg cta act ggc cca agt ttc       576
Leu Ala Tyr Ala Phe Phe Tyr Ser Thr Leu Leu Thr Gly Pro Ser Phe
            180                 185                 190

gat tat gcc gat ttt gac agc tgg ttg aat tgt gag atg ttc cgt gac       624
Asp Tyr Ala Asp Phe Asp Ser Trp Leu Asn Cys Glu Met Phe Arg Asp
        195                 200                 205
```

```
ttg cct gaa agc aaa aag cct atg aga aga cac cac cct ggt gaa aga        672
Leu Pro Glu Ser Lys Lys Pro Met Arg Arg His His Pro Gly Glu Arg
    210                 215                 220

aga cag att cca aag aat ggt aaa ctt gca tta tgg aaa gtt gtt caa        720
Arg Gln Ile Pro Lys Asn Gly Lys Leu Ala Leu Trp Lys Val Val Gln
225                 230                 235                 240

ggt ctt gct tgg atg att tta agt aca cta gga atg aag cac ttc ccc        768
Gly Leu Ala Trp Met Ile Leu Ser Thr Leu Gly Met Lys His Phe Pro
                245                 250                 255

gta aaa tac gtt ttg gac aaa gat ggc ttc cca acg aga tct ttt ata        816
Val Lys Tyr Val Leu Asp Lys Asp Gly Phe Pro Thr Arg Ser Phe Ile
            260                 265                 270

ttc aga atc cat tac tta ttc ttg ctt ggt ttc atc cat aga ttc aag        864
Phe Arg Ile His Tyr Leu Phe Leu Leu Gly Phe Ile His Arg Phe Lys
        275                 280                 285

tac tac gct gcc tgg act att tcg gaa gga tct tgt att ttg tgc ggt        912
Tyr Tyr Ala Ala Trp Thr Ile Ser Glu Gly Ser Cys Ile Leu Cys Gly
    290                 295                 300

ttg ggt tat aat ggt tat gat tca aag aca caa aag atc aga tgg gat        960
Leu Gly Tyr Asn Gly Tyr Asp Ser Lys Thr Gln Lys Ile Arg Trp Asp
305                 310                 315                 320

cgt gtc aga aat att gac att tgg acc gta gaa acg gcg cag aat acg       1008
Arg Val Arg Asn Ile Asp Ile Trp Thr Val Glu Thr Ala Gln Asn Thr
                325                 330                 335

cgt gaa atg ttg gaa gca tgg aat atg aat act aac aag tgg cta aaa       1056
Arg Glu Met Leu Glu Ala Trp Asn Met Asn Thr Asn Lys Trp Leu Lys
            340                 345                 350

tac tct gtt tat tta cgt gtc aca aag aag ggc aaa aaa cct ggt ttc       1104
Tyr Ser Val Tyr Leu Arg Val Thr Lys Lys Gly Lys Lys Pro Gly Phe
        355                 360                 365

cgc tca act ttg ttt act ttc cta act tcc gca ttt tgg cat ggt acc       1152
Arg Ser Thr Leu Phe Thr Phe Leu Thr Ser Ala Phe Trp His Gly Thr
    370                 375                 380

aga cct ggg tac tat ctg act ttt gcg aca ggg gct ttg tac caa aca       1200
Arg Pro Gly Tyr Tyr Leu Thr Phe Ala Thr Gly Ala Leu Tyr Gln Thr
385                 390                 395                 400

tgt ggt aaa atc tac aga cgc aat ttt aga cca att ttc ttg cga gaa       1248
Cys Gly Lys Ile Tyr Arg Arg Asn Phe Arg Pro Ile Phe Leu Arg Glu
                405                 410                 415

gat ggt gtc act cct ttg cct tct aaa aaa atc tac gat tta gtt ggc       1296
Asp Gly Val Thr Pro Leu Pro Ser Lys Lys Ile Tyr Asp Leu Val Gly
            420                 425                 430

ata tat gca att aaa cta gca ttt ggt tac atg gtg caa cca ttt att       1344
Ile Tyr Ala Ile Lys Leu Ala Phe Gly Tyr Met Val Gln Pro Phe Ile
        435                 440                 445

atc ctt gat ttg aag cca tct tta atg gta tgg ggc tct gtt tat ttc       1392
Ile Leu Asp Leu Lys Pro Ser Leu Met Val Trp Gly Ser Val Tyr Phe
    450                 455                 460
```

```
tat gtt cat att att gtt gct ttc tca ttt ttc cta ttc aga gga cca        1440
Tyr Val His Ile Ile Val Ala Phe Ser Phe Phe Leu Phe Arg Gly Pro
465                 470                 475                 480

tat gct aaa caa gtt act gaa ttt ttt aaa tcc aaa caa cct aaa gaa        1488
Tyr Ala Lys Gln Val Thr Glu Phe Phe Lys Ser Lys Gln Pro Lys Glu
                485                 490                 495

ata ttc att aga aaa caa aag aag ttg gaa aaa gat att tct gca agc        1536
Ile Phe Ile Arg Lys Gln Lys Lys Leu Glu Lys Asp Ile Ser Ala Ser
            500                 505                 510

tct cca aac ttg ggt ggt ata ttg aag gca aag att gaa cat gaa aag        1584
Ser Pro Asn Leu Gly Gly Ile Leu Lys Ala Lys Ile Glu His Glu Lys
        515                 520                 525

gga aag aca gca gaa gaa gaa gaa atg aac tta ggt att cca cca att        1632
Gly Lys Thr Ala Glu Glu Glu Glu Met Asn Leu Gly Ile Pro Pro Ile
    530                 535                 540

gag tta gaa aag tgg gac aat gct aag gaa gat tgg gaa gat ttc tgc        1680
Glu Leu Glu Lys Trp Asp Asn Ala Lys Glu Asp Trp Glu Asp Phe Cys
545                 550                 555                 560

aaa gat tac aaa gaa tgg aga aat aaa aat ggt ctt gaa ata gaa gag        1728
Lys Asp Tyr Lys Glu Trp Arg Asn Lys Asn Gly Leu Glu Ile Glu Glu
                565                 570                 575

gaa aac ctt tct aaa gct ttt gaa aga ttc aag cag gaa ttt tct aac        1776
Glu Asn Leu Ser Lys Ala Phe Glu Arg Phe Lys Gln Glu Phe Ser Asn
            580                 585                 590

gct gca agt gga tca ggt gaa cgt gtg aga aaa atg agt ttt agt ggt        1824
Ala Ala Ser Gly Ser Gly Glu Arg Val Arg Lys Met Ser Phe Ser Gly
        595                 600                 605

tac tca cca aag cct att tca aaa aag gaa gag tag                        1860
Tyr Ser Pro Lys Pro Ile Ser Lys Lys Glu Glu
    610                 615
```

<210> 9
<211> 619
<212> PRT
<213> Saccharomyces cerevisiae

<400> 9

```
Met Tyr Asn Pro Val Asp Ala Val Leu Thr Lys Ile Ile Thr Asn Tyr
1               5                   10                  15

Gly Ile Asp Ser Phe Thr Leu Arg Tyr Ala Ile Cys Leu Leu Gly Ser
            20                  25                  30

Phe Pro Leu Asn Ala Ile Leu Lys Arg Ile Pro Glu Lys Arg Ile Gly
        35                  40                  45

Leu Lys Cys Cys Phe Ile Ile Ser Met Ser Met Phe Tyr Leu Phe Gly
    50                  55                  60

Val Leu Asn Leu Val Ser Gly Phe Arg Thr Leu Phe Ile Ser Thr Met
```

```
65                  70                  75                  80

Phe Thr Tyr Leu Ile Ser Arg Phe Tyr Arg Ser Lys Phe Met Pro His
                85                  90                  95

Leu Asn Phe Met Phe Val Met Gly His Leu Ala Ile Asn His Ile His
            100                 105                 110

Ala Gln Phe Leu Asn Glu Gln Thr Gln Thr Thr Val Asp Ile Thr Ser
        115                 120                 125

Ser Gln Met Val Leu Ala Met Lys Leu Thr Ser Phe Ala Trp Ser Tyr
    130                 135                 140

Tyr Asp Gly Ser Cys Thr Ser Glu Ser Asp Phe Lys Asp Leu Thr Glu
145                 150                 155                 160

His Gln Lys Ser Arg Ala Val Arg Gly His Pro Pro Leu Leu Lys Phe
                165                 170                 175

Leu Ala Tyr Ala Phe Phe Tyr Ser Thr Leu Leu Thr Gly Pro Ser Phe
            180                 185                 190

Asp Tyr Ala Asp Phe Asp Ser Trp Leu Asn Cys Glu Met Phe Arg Asp
        195                 200                 205

Leu Pro Glu Ser Lys Lys Pro Met Arg Arg His His Pro Gly Glu Arg
    210                 215                 220

Arg Gln Ile Pro Lys Asn Gly Lys Leu Ala Leu Trp Lys Val Val Gln
225                 230                 235                 240

Gly Leu Ala Trp Met Ile Leu Ser Thr Leu Gly Met Lys His Phe Pro
                245                 250                 255

Val Lys Tyr Val Leu Asp Lys Asp Gly Phe Pro Thr Arg Ser Phe Ile
            260                 265                 270

Phe Arg Ile His Tyr Leu Phe Leu Leu Gly Phe Ile His Arg Phe Lys
        275                 280                 285

Tyr Tyr Ala Ala Trp Thr Ile Ser Glu Gly Ser Cys Ile Leu Cys Gly
    290                 295                 300

Leu Gly Tyr Asn Gly Tyr Asp Ser Lys Thr Gln Lys Ile Arg Trp Asp
305                 310                 315                 320

Arg Val Arg Asn Ile Asp Ile Trp Thr Val Glu Thr Ala Gln Asn Thr
                325                 330                 335
```

Arg Glu Met Leu Glu Ala Trp Asn Met Asn Thr Asn Lys Trp Leu Lys
340 345 350

Tyr Ser Val Tyr Leu Arg Val Thr Lys Lys Gly Lys Lys Pro Gly Phe
355 360 365

Arg Ser Thr Leu Phe Thr Phe Leu Thr Ser Ala Phe Trp His Gly Thr
370 375 380

Arg Pro Gly Tyr Tyr Leu Thr Phe Ala Thr Gly Ala Leu Tyr Gln Thr
385 390 395 400

Cys Gly Lys Ile Tyr Arg Arg Asn Phe Arg Pro Ile Phe Leu Arg Glu
405 410 415

Asp Gly Val Thr Pro Leu Pro Ser Lys Lys Ile Tyr Asp Leu Val Gly
420 425 430

Ile Tyr Ala Ile Lys Leu Ala Phe Gly Tyr Met Val Gln Pro Phe Ile
435 440 445

Ile Leu Asp Leu Lys Pro Ser Leu Met Val Trp Gly Ser Val Tyr Phe
450 455 460

Tyr Val His Ile Ile Val Ala Phe Ser Phe Phe Leu Phe Arg Gly Pro
465 470 475 480

Tyr Ala Lys Gln Val Thr Glu Phe Phe Lys Ser Lys Gln Pro Lys Glu
485 490 495

Ile Phe Ile Arg Lys Gln Lys Lys Leu Glu Lys Asp Ile Ser Ala Ser
500 505 510

Ser Pro Asn Leu Gly Gly Ile Leu Lys Ala Lys Ile Glu His Glu Lys
515 520 525

Gly Lys Thr Ala Glu Glu Glu Glu Met Asn Leu Gly Ile Pro Pro Ile
530 535 540

Glu Leu Glu Lys Trp Asp Asn Ala Lys Glu Asp Trp Glu Asp Phe Cys
545 550 555 560

Lys Asp Tyr Lys Glu Trp Arg Asn Lys Asn Gly Leu Glu Ile Glu Glu
565 570 575

Glu Asn Leu Ser Lys Ala Phe Glu Arg Phe Lys Gln Glu Phe Ser Asn
580 585 590

```
Ala Ala Ser Gly Ser Gly Glu Arg Val Arg Lys Met Ser Phe Ser Gly
        595                 600                 605

Tyr Ser Pro Lys Pro Ile Ser Lys Lys Glu Glu
    610                 615
```

<210> 10
<211> 1539
<212> DNA
<213> Yarrowia lipolytica

<220>
<221> CDS
<222> (1)..(1539)
<223> GenBank Accession No. XP_505624; "YALI0F19514p"

<400> 10

```
atg gcc ttt cca tgg gca gat aag tgg gca gcc gat gcg tct gca tct      48
Met Ala Phe Pro Trp Ala Asp Lys Trp Ala Ala Asp Ala Ser Ala Ser
1               5                   10                  15

aca ggg ctg cct ccg gac ctc ctc aag att gca ttc act ctg gtc atg      96
Thr Gly Leu Pro Pro Asp Leu Leu Lys Ile Ala Phe Thr Leu Val Met
            20                  25                  30

tct tat ccg ctg agt tct ctc atg aaa cgg ctg cca gat gac gcc aaa      144
Ser Tyr Pro Leu Ser Ser Leu Met Lys Arg Leu Pro Asp Asp Ala Lys
        35                  40                  45

aac ctc aag atc atc tat atc atc tcc gtg tcc atc ttc tac atg gtg      192
Asn Leu Lys Ile Ile Tyr Ile Ile Ser Val Ser Ile Phe Tyr Met Val
    50                  55                  60

ggt gtc ttc tcc ctc tat ggc gga gct gcc act ctg ctc ttc tcc tca      240
Gly Val Phe Ser Leu Tyr Gly Gly Ala Ala Thr Leu Leu Phe Ser Ser
65                  70                  75                  80

atg ggt acc ttc ttc atc acc caa tgg aag agc cct tac atg ccc tgg      288
Met Gly Thr Phe Phe Ile Thr Gln Trp Lys Ser Pro Tyr Met Pro Trp
                85                  90                  95

gtc aat ttt ggt ttt gtc atg acc cat ctc ttc gtc aat cac ctg cgt      336
Val Asn Phe Gly Phe Val Met Thr His Leu Phe Val Asn His Leu Arg
            100                 105                 110

tcg cag ttt ttc ccc gaa aca tac gac ccc aat gtc att gac atc acc      384
Ser Gln Phe Phe Pro Glu Thr Tyr Asp Pro Asn Val Ile Asp Ile Thr
        115                 120                 125

gga gca cag atg gtt ctg tgt atg aag cta tcg tct ttt gga tgg aac      432
Gly Ala Gln Met Val Leu Cys Met Lys Leu Ser Ser Phe Gly Trp Asn
    130                 135                 140

gtc tac gat gga tgg cag att gag aag ggt gag cag ctc agc gag ttc      480
Val Tyr Asp Gly Trp Gln Ile Glu Lys Gly Glu Gln Leu Ser Glu Phe
145                 150                 155                 160

cag act aaa agg gct gtt ctc aag cac ccc agt ctt atg gac ttc cta      528
Gln Thr Lys Arg Ala Val Leu Lys His Pro Ser Leu Met Asp Phe Leu
                165                 170                 175
```

```
gct ttt gtg ttc tac ttc cct tcc att ctg aca ggt cct tct tac gac        576
Ala Phe Val Phe Tyr Phe Pro Ser Ile Leu Thr Gly Pro Ser Tyr Asp
            180                 185                 190

tat atg gag ttc cat aac tgg ctc gat ctc agc ctg ttc aag gag ctg        624
Tyr Met Glu Phe His Asn Trp Leu Asp Leu Ser Leu Phe Lys Glu Leu
        195                 200                 205

gag aaa gat aag gac ccc aag cga gct gct cga cga aag cga cac aag        672
Glu Lys Asp Lys Asp Pro Lys Arg Ala Ala Arg Arg Lys Arg His Lys
    210                 215                 220

atc ccc cga tct gga atc gct gct tcc aag aaa ctc gcc gct ggt atc        720
Ile Pro Arg Ser Gly Ile Ala Ala Ser Lys Lys Leu Ala Ala Gly Ile
225                 230                 235                 240

ttc tgg atc gtt ctg tgg acc cag gtg gac tct cga atc tcc acc gcc        768
Phe Trp Ile Val Leu Trp Thr Gln Val Asp Ser Arg Ile Ser Thr Ala
                245                 250                 255

tac gct tac tca gac gca ttc acc aag gag cac aac atc ttt gga cga        816
Tyr Ala Tyr Ser Asp Ala Phe Thr Lys Glu His Asn Ile Phe Gly Arg
            260                 265                 270

att gtg tac ctc tac atg ctc ggt ttc atg tac cga ctc aag tac tac        864
Ile Val Tyr Leu Tyr Met Leu Gly Phe Met Tyr Arg Leu Lys Tyr Tyr
        275                 280                 285

gga gcc tgg tcc att tcc gag gga gcc tgc atc ttg tct ggc ctc gga        912
Gly Ala Trp Ser Ile Ser Glu Gly Ala Cys Ile Leu Ser Gly Leu Gly
    290                 295                 300

ttc cat ggc gtg gac ccc aaa act ggc aag tac aag tgg gac cgt gtc        960
Phe His Gly Val Asp Pro Lys Thr Gly Lys Tyr Lys Trp Asp Arg Val
305                 310                 315                 320

cag aac gtg gac ccg tgg gga ttc gaa act ggt caa aac aca aag gct       1008
Gln Asn Val Asp Pro Trp Gly Phe Glu Thr Gly Gln Asn Thr Lys Ala
                325                 330                 335

ctg ctg gag gcc tgg aac cag aac act aac aag tgg cta cga aac tat       1056
Leu Leu Glu Ala Trp Asn Gln Asn Thr Asn Lys Trp Leu Arg Asn Tyr
            340                 345                 350

gtg tac ctc cga gtg gtg ccc aaa ggc caa aag cct gga ttc cga gcc       1104
Val Tyr Leu Arg Val Val Pro Lys Gly Gln Lys Pro Gly Phe Arg Ala
        355                 360                 365

act atc ttc aca ttt gtg gtt tcc gcc ttc tgg cat gga act cga cct       1152
Thr Ile Phe Thr Phe Val Val Ser Ala Phe Trp His Gly Thr Arg Pro
    370                 375                 380

ggc tac tat ctc acc ttt gtg acc gct gcc atg tac cag tct gtt ggt       1200
Gly Tyr Tyr Leu Thr Phe Val Thr Ala Ala Met Tyr Gln Ser Val Gly
385                 390                 395                 400

aag ttc ttc cga cga tac ctg cga ccc ttc ttc atg gag tct gat gga       1248
Lys Phe Phe Arg Arg Tyr Leu Arg Pro Phe Phe Met Glu Ser Asp Gly
                405                 410                 415

aag act gcc ggt ccc tat aag atc tac tac gac att gtg tgt tgg atc       1296
Lys Thr Ala Gly Pro Tyr Lys Ile Tyr Tyr Asp Ile Val Cys Trp Ile
            420                 425                 430

gtt gtc caa acc gca ttt gga tac gct acc cag tcc ttt atg att cta       1344
```

```
Val Val Gln Thr Ala Phe Gly Tyr Ala Thr Gln Ser Phe Met Ile Leu
        435                 440                 445

gac ttc tgg ctg tcg ctc aag tgt tgg aag aac tcc tgg ttc ctg tac       1392
Asp Phe Trp Leu Ser Leu Lys Cys Trp Lys Asn Ser Trp Phe Leu Tyr
    450                 455                 460

cac att gct ctg ggc gcc atc ttt gca att tct agc ccc tac aag gca       1440
His Ile Ala Leu Gly Ala Ile Phe Ala Ile Ser Ser Pro Tyr Lys Ala
465                 470                 475                 480

tgg gcg att ccc aag atc aag aaa aag cag gct gga gcc gtc act gac       1488
Trp Ala Ile Pro Lys Ile Lys Lys Lys Gln Ala Gly Ala Val Thr Asp
                485                 490                 495

aag aag gac gcc aag gag gag gtg aag aag gac acc atc aag acc aag       1536
Lys Lys Asp Ala Lys Glu Glu Val Lys Lys Asp Thr Ile Lys Thr Lys
            500                 505                 510

taa                                                                   1539
```

<210> 11
<211> 512
<212> PRT
<213> Yarrowia lipolytica

<400> 11

```
Met Ala Phe Pro Trp Ala Asp Lys Trp Ala Ala Asp Ala Ser Ala Ser
1               5                   10                  15

Thr Gly Leu Pro Pro Asp Leu Leu Lys Ile Ala Phe Thr Leu Val Met
            20                  25                  30

Ser Tyr Pro Leu Ser Ser Leu Met Lys Arg Leu Pro Asp Asp Ala Lys
        35                  40                  45

Asn Leu Lys Ile Ile Tyr Ile Ile Ser Val Ser Ile Phe Tyr Met Val
    50                  55                  60

Gly Val Phe Ser Leu Tyr Gly Gly Ala Ala Thr Leu Leu Phe Ser Ser
65                  70                  75                  80

Met Gly Thr Phe Phe Ile Thr Gln Trp Lys Ser Pro Tyr Met Pro Trp
                85                  90                  95

Val Asn Phe Gly Phe Val Met Thr His Leu Phe Val Asn His Leu Arg
            100                 105                 110

Ser Gln Phe Phe Pro Glu Thr Tyr Asp Pro Asn Val Ile Asp Ile Thr
        115                 120                 125

Gly Ala Gln Met Val Leu Cys Met Lys Leu Ser Ser Phe Gly Trp Asn
    130                 135                 140
```

```
Val Tyr Asp Gly Trp Gln Ile Glu Lys Gly Glu Gln Leu Ser Glu Phe
145                 150                 155                 160

Gln Thr Lys Arg Ala Val Leu Lys His Pro Ser Leu Met Asp Phe Leu
                165                 170                 175

Ala Phe Val Phe Tyr Phe Pro Ser Ile Leu Thr Gly Pro Ser Tyr Asp
            180                 185                 190

Tyr Met Glu Phe His Asn Trp Leu Asp Leu Ser Leu Phe Lys Glu Leu
        195                 200                 205

Glu Lys Asp Lys Asp Pro Lys Arg Ala Ala Arg Arg Lys Arg His Lys
    210                 215                 220

Ile Pro Arg Ser Gly Ile Ala Ala Ser Lys Lys Leu Ala Ala Gly Ile
225                 230                 235                 240

Phe Trp Ile Val Leu Trp Thr Gln Val Asp Ser Arg Ile Ser Thr Ala
                245                 250                 255

Tyr Ala Tyr Ser Asp Ala Phe Thr Lys Glu His Asn Ile Phe Gly Arg
            260                 265                 270

Ile Val Tyr Leu Tyr Met Leu Gly Phe Met Tyr Arg Leu Lys Tyr Tyr
        275                 280                 285

Gly Ala Trp Ser Ile Ser Glu Gly Ala Cys Ile Leu Ser Gly Leu Gly
    290                 295                 300

Phe His Gly Val Asp Pro Lys Thr Gly Lys Tyr Lys Trp Asp Arg Val
305                 310                 315                 320

Gln Asn Val Asp Pro Trp Gly Phe Glu Thr Gly Gln Asn Thr Lys Ala
                325                 330                 335

Leu Leu Glu Ala Trp Asn Gln Asn Thr Asn Lys Trp Leu Arg Asn Tyr
            340                 345                 350

Val Tyr Leu Arg Val Val Pro Lys Gly Gln Lys Pro Gly Phe Arg Ala
        355                 360                 365

Thr Ile Phe Thr Phe Val Val Ser Ala Phe Trp His Gly Thr Arg Pro
    370                 375                 380

Gly Tyr Tyr Leu Thr Phe Val Thr Ala Ala Met Tyr Gln Ser Val Gly
385                 390                 395                 400

Lys Phe Phe Arg Arg Tyr Leu Arg Pro Phe Phe Met Glu Ser Asp Gly
```

405 410 415

Lys Thr Ala Gly Pro Tyr Lys Ile Tyr Tyr Asp Ile Val Cys Trp Ile
420 425 430

Val Val Gln Thr Ala Phe Gly Tyr Ala Thr Gln Ser Phe Met Ile Leu
435 440 445

Asp Phe Trp Leu Ser Leu Lys Cys Trp Lys Asn Ser Trp Phe Leu Tyr
450 455 460

His Ile Ala Leu Gly Ala Ile Phe Ala Ile Ser Ser Pro Tyr Lys Ala
465 470 475 480

Trp Ala Ile Pro Lys Ile Lys Lys Lys Gln Ala Gly Ala Val Thr Asp
485 490 495

Lys Lys Asp Ala Lys Glu Glu Val Lys Lys Asp Thr Ile Lys Thr Lys
500 505 510

<210> 12
<211> 1870
<212> DNA
<213> Saccharomyces cerevisiae

<220>
<221> CDS
<222> (3)..(1862)
<223> synthetic Alel (codon-optimized for Yarrowia lipolytica)

<400> 12

```
ac atg tac aac ccc gtg gac gca gtg ttg act aag att att aca aac         47
   Met Tyr Asn Pro Val Asp Ala Val Leu Thr Lys Ile Ile Thr Asn
   1               5                   10                  15

tac gga att gat tct ttt acc ctg cga tat gcc att tgt ctg ttg gga        95
Tyr Gly Ile Asp Ser Phe Thr Leu Arg Tyr Ala Ile Cys Leu Leu Gly
                20                  25                  30

tct ttt cct ctt aac gct att ctg aag cgg att cct gaa aag cga atc       143
Ser Phe Pro Leu Asn Ala Ile Leu Lys Arg Ile Pro Glu Lys Arg Ile
            35                  40                  45

ggc ctg aag tgt tgt ttt atc att tct atg tcc atg ttt tat ctc ttc       191
Gly Leu Lys Cys Cys Phe Ile Ile Ser Met Ser Met Phe Tyr Leu Phe
        50                  55                  60

ggc gtt ctg aat ctc gtg agc gga ttt cga acc ctc ttc att tcc aca       239
Gly Val Leu Asn Leu Val Ser Gly Phe Arg Thr Leu Phe Ile Ser Thr
    65                  70                  75

atg ttc aca tac ctt atc tct cgg ttc tac cga tcc aag ttt atg ccc       287
Met Phe Thr Tyr Leu Ile Ser Arg Phe Tyr Arg Ser Lys Phe Met Pro
80                  85                  90                  95

cat ctc aac ttc atg ttc gtc atg ggc cac ttg gct atc aac cac att       335
His Leu Asn Phe Met Phe Val Met Gly His Leu Ala Ile Asn His Ile
```

```
                100                 105                 110

cat gct cag ttc ctg aac gaa caa act caa acg acc gtc gat att aca        383
His Ala Gln Phe Leu Asn Glu Gln Thr Gln Thr Thr Val Asp Ile Thr
            115                 120                 125

tcc tcg cag atg gtc ctg gct atg aag ctg aca agc ttt gcc tgg tct        431
Ser Ser Gln Met Val Leu Ala Met Lys Leu Thr Ser Phe Ala Trp Ser
        130                 135                 140

tac tat gac ggt tcg tgt acg agc gag tcc gac ttc aag gac ctt acc        479
Tyr Tyr Asp Gly Ser Cys Thr Ser Glu Ser Asp Phe Lys Asp Leu Thr
    145                 150                 155

gaa cac cag aag tcc cga gcc gtc cga ggc cat cct ccc ctt ctg aaa        527
Glu His Gln Lys Ser Arg Ala Val Arg Gly His Pro Pro Leu Leu Lys
160                 165                 170                 175

ttt ttg gct tac gcc ttt ttc tac tct acc ctt ctc acc ggt ccc tcc        575
Phe Leu Ala Tyr Ala Phe Phe Tyr Ser Thr Leu Leu Thr Gly Pro Ser
                180                 185                 190

ttc gat tac gct gat ttc gac tct tgg ctg aac tgc gaa atg ttc cgg        623
Phe Asp Tyr Ala Asp Phe Asp Ser Trp Leu Asn Cys Glu Met Phe Arg
            195                 200                 205

gac ctt ccc gag tcc aag aaa ccc atg cga aga cat cat cct ggt gag        671
Asp Leu Pro Glu Ser Lys Lys Pro Met Arg Arg His His Pro Gly Glu
        210                 215                 220

cgg cgt cag att ccc aag aac ggc aag ctc gcc ctg tgg aag gtt gtc        719
Arg Arg Gln Ile Pro Lys Asn Gly Lys Leu Ala Leu Trp Lys Val Val
    225                 230                 235

cag ggc ctc gcc tgg atg att ctg agc acg ttg ggt atg aag cac ttc        767
Gln Gly Leu Ala Trp Met Ile Leu Ser Thr Leu Gly Met Lys His Phe
240                 245                 250                 255

ccc gtg aag tac gtg ctg gac aag gac gga ttt cct acc cgt tcc ttt        815
Pro Val Lys Tyr Val Leu Asp Lys Asp Gly Phe Pro Thr Arg Ser Phe
                260                 265                 270

atc ttc cgt att cat tat ctg ttt ctg ctg gga ttc atc cac cga ttt        863
Ile Phe Arg Ile His Tyr Leu Phe Leu Leu Gly Phe Ile His Arg Phe
            275                 280                 285

aag tat tac gct gcg tgg acg att agc gaa ggt tcg tgc att ctc tgt        911
Lys Tyr Tyr Ala Ala Trp Thr Ile Ser Glu Gly Ser Cys Ile Leu Cys
        290                 295                 300

ggt ctt ggt tat aat gga tac gat tct aag acc cag aag atc cgg tgg        959
Gly Leu Gly Tyr Asn Gly Tyr Asp Ser Lys Thr Gln Lys Ile Arg Trp
    305                 310                 315

gat cga gtg cgg aat att gat att tgg aca gtg gag act gca caa aac       1007
Asp Arg Val Arg Asn Ile Asp Ile Trp Thr Val Glu Thr Ala Gln Asn
320                 325                 330                 335

acc cga gag atg ctg gaa gcg tgg aac atg aat act aac aaa tgg ctg       1055
Thr Arg Glu Met Leu Glu Ala Trp Asn Met Asn Thr Asn Lys Trp Leu
                340                 345                 350

aag tat agc gtg tat ctt aga gtg act aag aag ggt aag aag cca ggt       1103
Lys Tyr Ser Val Tyr Leu Arg Val Thr Lys Lys Gly Lys Lys Pro Gly
            355                 360                 365
```

```
ttt cga tct acc ctg ttt acc ttc ctg acc tcc gcc ttt tgg cac ggt      1151
Phe Arg Ser Thr Leu Phe Thr Phe Leu Thr Ser Ala Phe Trp His Gly
        370                 375                 380

acc cgt cct gga tac tac ctt acc ttc gca act ggt gcc ctg tac caa      1199
Thr Arg Pro Gly Tyr Tyr Leu Thr Phe Ala Thr Gly Ala Leu Tyr Gln
    385                 390                 395

acc tgt gga aag atc tat aga cga aac ttt cgt ccc atc ttt ctg aga      1247
Thr Cys Gly Lys Ile Tyr Arg Arg Asn Phe Arg Pro Ile Phe Leu Arg
400                 405                 410                 415

gaa gat ggc gtg aca cct ctc ccg tcc aag aag att tac gac ctg gtc      1295
Glu Asp Gly Val Thr Pro Leu Pro Ser Lys Lys Ile Tyr Asp Leu Val
                420                 425                 430

ggc att tac gct att aag ctg gcc ttt ggt tac atg gtt caa ccc ttc      1343
Gly Ile Tyr Ala Ile Lys Leu Ala Phe Gly Tyr Met Val Gln Pro Phe
            435                 440                 445

att atc ctt gac ctg aag ccc tct ctt atg gtt tgg gga tcc gtg tat      1391
Ile Ile Leu Asp Leu Lys Pro Ser Leu Met Val Trp Gly Ser Val Tyr
        450                 455                 460

ttc tac gtg cat att att gtg gcc ttc tcg ttc ttt ctg ttc cga gga      1439
Phe Tyr Val His Ile Ile Val Ala Phe Ser Phe Phe Leu Phe Arg Gly
    465                 470                 475

cca tac gct aag cag gtt act gaa ttt ttc aaa agc aag caa ccg aag      1487
Pro Tyr Ala Lys Gln Val Thr Glu Phe Phe Lys Ser Lys Gln Pro Lys
480                 485                 490                 495

gag atc ttc atc cga aag cag aag aag ttg gaa aaa gac atc tct gcc      1535
Glu Ile Phe Ile Arg Lys Gln Lys Lys Leu Glu Lys Asp Ile Ser Ala
                500                 505                 510

tct tcc ccc aac ctc gga ggt att ctt aag gca aaa atc gaa cat gag      1583
Ser Ser Pro Asn Leu Gly Gly Ile Leu Lys Ala Lys Ile Glu His Glu
            515                 520                 525

aag gga aag acg gca gag gag gaa gag atg aac ttg ggc att cca ccc      1631
Lys Gly Lys Thr Ala Glu Glu Glu Glu Met Asn Leu Gly Ile Pro Pro
        530                 535                 540

atc gaa ctg gag aag tgg gac aac gcc aag gag gac tgg gag gat ttc      1679
Ile Glu Leu Glu Lys Trp Asp Asn Ala Lys Glu Asp Trp Glu Asp Phe
    545                 550                 555

tgc aag gac tac aag gag tgg cgg aac aag aac gga ctg gaa att gaa      1727
Cys Lys Asp Tyr Lys Glu Trp Arg Asn Lys Asn Gly Leu Glu Ile Glu
560                 565                 570                 575

gag gag aac ctg tcc aag gcc ttc gag cga ttt aag cag gaa ttt tcc      1775
Glu Glu Asn Leu Ser Lys Ala Phe Glu Arg Phe Lys Gln Glu Phe Ser
                580                 585                 590

aac gct gcg tcg ggc tct ggt gaa cgg gtt cgg aaa atg tcc ttc tcc      1823
Asn Ala Ala Ser Gly Ser Gly Glu Arg Val Arg Lys Met Ser Phe Ser
            595                 600                 605

gga tat tct cct aaa ccc atc tcg aag aaa gaa gaa tag gcggccgc         1870
Gly Tyr Ser Pro Lys Pro Ile Ser Lys Lys Glu Glu
        610                 615
```

<210> 13
<211> 619
<212> PRT
<213> Saccharomyces cerevisiae

<400> 13

Met Tyr Asn Pro Val Asp Ala Val Leu Thr Lys Ile Ile Thr Asn Tyr
1               5                   10                  15

Gly Ile Asp Ser Phe Thr Leu Arg Tyr Ala Ile Cys Leu Leu Gly Ser
            20                  25                  30

Phe Pro Leu Asn Ala Ile Leu Lys Arg Ile Pro Glu Lys Arg Ile Gly
        35                  40                  45

Leu Lys Cys Cys Phe Ile Ile Ser Met Ser Met Phe Tyr Leu Phe Gly
    50                  55                  60

Val Leu Asn Leu Val Ser Gly Phe Arg Thr Leu Phe Ile Ser Thr Met
65                  70                  75                  80

Phe Thr Tyr Leu Ile Ser Arg Phe Tyr Arg Ser Lys Phe Met Pro His
                85                  90                  95

Leu Asn Phe Met Phe Val Met Gly His Leu Ala Ile Asn His Ile His
            100                 105                 110

Ala Gln Phe Leu Asn Glu Gln Thr Gln Thr Thr Val Asp Ile Thr Ser
        115                 120                 125

Ser Gln Met Val Leu Ala Met Lys Leu Thr Ser Phe Ala Trp Ser Tyr
    130                 135                 140

Tyr Asp Gly Ser Cys Thr Ser Glu Ser Asp Phe Lys Asp Leu Thr Glu
145                 150                 155                 160

His Gln Lys Ser Arg Ala Val Arg Gly His Pro Pro Leu Leu Lys Phe
                165                 170                 175

Leu Ala Tyr Ala Phe Phe Tyr Ser Thr Leu Leu Thr Gly Pro Ser Phe
            180                 185                 190

Asp Tyr Ala Asp Phe Asp Ser Trp Leu Asn Cys Glu Met Phe Arg Asp
        195                 200                 205

Leu Pro Glu Ser Lys Lys Pro Met Arg Arg His His Pro Gly Glu Arg
    210                 215                 220

Arg Gln Ile Pro Lys Asn Gly Lys Leu Ala Leu Trp Lys Val Val Gln

```
225                 230                 235                 240

Gly Leu Ala Trp Met Ile Leu Ser Thr Leu Gly Met Lys His Phe Pro
                245                 250                 255

Val Lys Tyr Val Leu Asp Lys Asp Gly Phe Pro Thr Arg Ser Phe Ile
            260                 265                 270

Phe Arg Ile His Tyr Leu Phe Leu Leu Gly Phe Ile His Arg Phe Lys
        275                 280                 285

Tyr Tyr Ala Ala Trp Thr Ile Ser Glu Gly Ser Cys Ile Leu Cys Gly
    290                 295                 300

Leu Gly Tyr Asn Gly Tyr Asp Ser Lys Thr Gln Lys Ile Arg Trp Asp
305                 310                 315                 320

Arg Val Arg Asn Ile Asp Ile Trp Thr Val Glu Thr Ala Gln Asn Thr
                325                 330                 335

Arg Glu Met Leu Glu Ala Trp Asn Met Asn Thr Asn Lys Trp Leu Lys
            340                 345                 350

Tyr Ser Val Tyr Leu Arg Val Thr Lys Lys Gly Lys Lys Pro Gly Phe
        355                 360                 365

Arg Ser Thr Leu Phe Thr Phe Leu Thr Ser Ala Phe Trp His Gly Thr
    370                 375                 380

Arg Pro Gly Tyr Tyr Leu Thr Phe Ala Thr Gly Ala Leu Tyr Gln Thr
385                 390                 395                 400

Cys Gly Lys Ile Tyr Arg Arg Asn Phe Arg Pro Ile Phe Leu Arg Glu
                405                 410                 415

Asp Gly Val Thr Pro Leu Pro Ser Lys Lys Ile Tyr Asp Leu Val Gly
            420                 425                 430

Ile Tyr Ala Ile Lys Leu Ala Phe Gly Tyr Met Val Gln Pro Phe Ile
        435                 440                 445

Ile Leu Asp Leu Lys Pro Ser Leu Met Val Trp Gly Ser Val Tyr Phe
    450                 455                 460

Tyr Val His Ile Ile Val Ala Phe Ser Phe Phe Leu Phe Arg Gly Pro
465                 470                 475                 480

Tyr Ala Lys Gln Val Thr Glu Phe Phe Lys Ser Lys Gln Pro Lys Glu
                485                 490                 495
```

Ile Phe Ile Arg Lys Gln Lys Lys Leu Glu Lys Asp Ile Ser Ala Ser
500 505 510

Ser Pro Asn Leu Gly Gly Ile Leu Lys Ala Lys Ile Glu His Glu Lys
515 520 525

Gly Lys Thr Ala Glu Glu Glu Glu Met Asn Leu Gly Ile Pro Pro Ile
530 535 540

Glu Leu Glu Lys Trp Asp Asn Ala Lys Glu Asp Trp Glu Asp Phe Cys
545 550 555 560

Lys Asp Tyr Lys Glu Trp Arg Asn Lys Asn Gly Leu Glu Ile Glu Glu
565 570 575

Glu Asn Leu Ser Lys Ala Phe Glu Arg Phe Lys Gln Glu Phe Ser Asn
580 585 590

Ala Ala Ser Gly Ser Gly Glu Arg Val Arg Lys Met Ser Phe Ser Gly
595 600 605

Tyr Ser Pro Lys Pro Ile Ser Lys Lys Glu Glu
610 615

<210> 14
<211> 945
<212> DNA
<213> Mortierella alpina

<220>
<221> CDS
<222> (1)..(945)
<223> LPAAT1

<300>
<302> High eicosapentaenoic acid producing strains of Yarrowia lipolytica
<310> US-2006-0115881-A1
<311> 2005-11-02
<312> 2006-06-01
<313> (1)..(945)

<300>
<302> High eicosapentaenoic acid producing strains of Yarrowia lipolytica
<310> WO 2006/052870
<311> 2005-11-03
<312> 2006-05-18
<313> (1)..(945)

<400> 14

```
atg tcc ata ggt tct tcc aat cct gtc ctg ctg gca gcg atc ccc ttc         48
Met Ser Ile Gly Ser Ser Asn Pro Val Leu Leu Ala Ala Ile Pro Phe
1               5                   10                  15
```

```
gtc tac ctc ttc gtc ctc cct cgt gtc ctc gcc ttc ctc cct caa aag        96
Val Tyr Leu Phe Val Leu Pro Arg Val Leu Ala Phe Leu Pro Gln Lys
            20                  25                  30

gcc cag ttc ctc gca aaa tgc atc gtg gtc ttg atc gcc acc ctt atc       144
Ala Gln Phe Leu Ala Lys Cys Ile Val Val Leu Ile Ala Thr Leu Ile
        35                  40                  45

atg tcc gtc gca ggc tgc ttc att tcc atc gtc tgt gcg ctc ctc gat       192
Met Ser Val Ala Gly Cys Phe Ile Ser Ile Val Cys Ala Leu Leu Asp
    50                  55                  60

aaa cgc tat gtg atc aac tac gtc gtc tca aga ctc ttc tca ttc ctc       240
Lys Arg Tyr Val Ile Asn Tyr Val Val Ser Arg Leu Phe Ser Phe Leu
65                  70                  75                  80

gct gca aga ccc tgc ggt gtc acc tac aag atc gtc ggc gag gaa cat       288
Ala Ala Arg Pro Cys Gly Val Thr Tyr Lys Ile Val Gly Glu Glu His
                85                  90                  95

ctg gac aag tac ccc gcc att gtc gtc tgc aac cac cag agc tcc atg       336
Leu Asp Lys Tyr Pro Ala Ile Val Val Cys Asn His Gln Ser Ser Met
            100                 105                 110

gac atg atg gtc ctg gga cgc gtc ttc cca aag cac tgt gtc gtc atg       384
Asp Met Met Val Leu Gly Arg Val Phe Pro Lys His Cys Val Val Met
        115                 120                 125

gca aag aag gaa ctt ctt tac ttt ccg ttc ctg ggc atg ttt atg aag       432
Ala Lys Lys Glu Leu Leu Tyr Phe Pro Phe Leu Gly Met Phe Met Lys
    130                 135                 140

ctg agt aac gcc atc ttc att gac cgc aag aac cac aag aag gcg atc       480
Leu Ser Asn Ala Ile Phe Ile Asp Arg Lys Asn His Lys Lys Ala Ile
145                 150                 155                 160

gag tcc acc acc caa gct gtc gcc gac atg aag aag cac aac tct gga       528
Glu Ser Thr Thr Gln Ala Val Ala Asp Met Lys Lys His Asn Ser Gly
                165                 170                 175

atc tgg att ttc ccc gaa gga aca cgt tcc cgc ttg gac aag gcc gat       576
Ile Trp Ile Phe Pro Glu Gly Thr Arg Ser Arg Leu Asp Lys Ala Asp
            180                 185                 190

ctc ttg ccc ttc aag aag gga gcc ttc cac ctc gcc att caa gcc caa       624
Leu Leu Pro Phe Lys Lys Gly Ala Phe His Leu Ala Ile Gln Ala Gln
        195                 200                 205

ctc ccg atc ctc ccc atc atc tcg caa gga tac tca cac atc tac gat       672
Leu Pro Ile Leu Pro Ile Ile Ser Gln Gly Tyr Ser His Ile Tyr Asp
    210                 215                 220

tcg tca aaa cgc tac ttc ccc ggt gga gag ctc gag atc aga gtc ctg       720
Ser Ser Lys Arg Tyr Phe Pro Gly Gly Glu Leu Glu Ile Arg Val Leu
225                 230                 235                 240

gaa cct atc ccc acc acg gga ttg acc aca gac gat gtg aac gac ctg       768
Glu Pro Ile Pro Thr Thr Gly Leu Thr Thr Asp Asp Val Asn Asp Leu
                245                 250                 255

atg gac aag act cgc aac ctg atg ctg aag cac ctc aag gag atg gat       816
Met Asp Lys Thr Arg Asn Leu Met Leu Lys His Leu Lys Glu Met Asp
            260                 265                 270
```

```
tct caa tac tcc tcc tcc acc gct gaa aac gga tcg acc cat att gac         864
Ser Gln Tyr Ser Ser Ser Thr Ala Glu Asn Gly Ser Thr His Ile Asp
        275                 280                 285

gcc gat atc gca aag tca act gcc aca tcg atc gga aac acg gac gat         912
Ala Asp Ile Ala Lys Ser Thr Ala Thr Ser Ile Gly Asn Thr Asp Asp
    290                 295                 300

gct atc aca aag agg agg aca cca aaa gag tag                             945
Ala Ile Thr Lys Arg Arg Thr Pro Lys Glu
305                 310
```

<210> 15
<211> 314
<212> PRT
<213> Mortierella alpina

<400> 15

```
Met Ser Ile Gly Ser Ser Asn Pro Val Leu Leu Ala Ala Ile Pro Phe
1               5                   10                  15

Val Tyr Leu Phe Val Leu Pro Arg Val Leu Ala Phe Leu Pro Gln Lys
            20                  25                  30

Ala Gln Phe Leu Ala Lys Cys Ile Val Val Leu Ile Ala Thr Leu Ile
        35                  40                  45

Met Ser Val Ala Gly Cys Phe Ile Ser Ile Val Cys Ala Leu Leu Asp
    50                  55                  60

Lys Arg Tyr Val Ile Asn Tyr Val Val Ser Arg Leu Phe Ser Phe Leu
65                  70                  75                  80

Ala Ala Arg Pro Cys Gly Val Thr Tyr Lys Ile Val Gly Glu Glu His
                85                  90                  95

Leu Asp Lys Tyr Pro Ala Ile Val Val Cys Asn His Gln Ser Ser Met
            100                 105                 110

Asp Met Met Val Leu Gly Arg Val Phe Pro Lys His Cys Val Val Met
        115                 120                 125

Ala Lys Lys Glu Leu Leu Tyr Phe Pro Phe Leu Gly Met Phe Met Lys
    130                 135                 140

Leu Ser Asn Ala Ile Phe Ile Asp Arg Lys Asn His Lys Lys Ala Ile
145                 150                 155                 160

Glu Ser Thr Thr Gln Ala Val Ala Asp Met Lys Lys His Asn Ser Gly
                165                 170                 175

Ile Trp Ile Phe Pro Glu Gly Thr Arg Ser Arg Leu Asp Lys Ala Asp
```

```
                180                 185                 190

Leu Leu Pro Phe Lys Lys Gly Ala Phe His Leu Ala Ile Gln Ala Gln
        195                 200                 205

Leu Pro Ile Leu Pro Ile Ile Ser Gln Gly Tyr Ser His Ile Tyr Asp
    210                 215                 220

Ser Ser Lys Arg Tyr Phe Pro Gly Gly Glu Leu Glu Ile Arg Val Leu
225                 230                 235                 240

Glu Pro Ile Pro Thr Thr Gly Leu Thr Thr Asp Asp Val Asn Asp Leu
                245                 250                 255

Met Asp Lys Thr Arg Asn Leu Met Leu Lys His Leu Lys Glu Met Asp
            260                 265                 270

Ser Gln Tyr Ser Ser Ser Thr Ala Glu Asn Gly Ser Thr His Ile Asp
        275                 280                 285

Ala Asp Ile Ala Lys Ser Thr Ala Thr Ser Ile Gly Asn Thr Asp Asp
    290                 295                 300

Ala Ile Thr Lys Arg Arg Thr Pro Lys Glu
305                 310
```

<210> 16
<211> 1549
<212> DNA
<213> Yarrowia lipolytica

<220>
<221> CDS
<222> (501)..(1349)
<223> LPAAT1

<300>
<302> High eicosapentaenoic acid producing strains of Yarrowia lipolytica
<310> US-2006-0115881-A1
<311> 2005-11-02
<312> 2006-06-01
<313> (1)..(1549)

<300>
<302> High eicosapentaenoic acid producing strains of Yarrowia lipolytica
<310> WO 2006/052870
<311> 2005-11-03
<312> 2006-05-18
<313> (1)..(1549)

<400> 16

cacagcataa taccacggca tgaccccgct gactccaacc ttcatttcgg cacatgtagg 60

```
tgcacaaggg acttcaagag gggccaattt catgcggaca catggcgcaa aaaacgcccg      120

actttgatta cacagacacg taataacgac gaagccgaga tgagcacacg tggccaagtc      180

tgccaatggc cccctggacc cccctgacaa agtttcccaa caagcccagc cgtgcatggt      240

gtgtttttgt gcggagacac acgccaatta ggctcatttg agggtatgca gcgaaaaaaa      300

attagtgtgg gtagtttgtt tgcaggaatc aagtgggtgg ttgaaaaaca agaaagagcg      360

acgacaagag agagagaaaa agagagagag actccataaa gcgtgcatca aaattaaggt      420

gtgtgactat ccgaaaacca aacatgaaca gttggatata tgtcgctgtg attgcagttg      480

ctgccgttct cattgcccga atg tcc gtt gca tcc aag ctc gtc ttc tac gtc      533
                     Met Ser Val Ala Ser Lys Leu Val Phe Tyr Val
                     1               5                   10

cgc gcc gcc atc gcc gtg gtc atc ttt gcc gcc tgt gcc acc tac ggc       581
Arg Ala Ala Ile Ala Val Val Ile Phe Ala Ala Cys Ala Thr Tyr Gly
            15                  20                  25

gtg ctg gcg tcc acc att ctc acc gcc atc ggc aag cag ggc ctg gcc       629
Val Leu Ala Ser Thr Ile Leu Thr Ala Ile Gly Lys Gln Gly Leu Ala
        30                  35                  40

caa tgg acc gtt gcc aga gcc ttc tac tac tcg gtg cgc atc ttc ctg       677
Gln Trp Thr Val Ala Arg Ala Phe Tyr Tyr Ser Val Arg Ile Phe Leu
    45                  50                  55

ggt atc agc atc aag ctg cgt agc cgg cag gtg acc gga acc gcc ggt       725
Gly Ile Ser Ile Lys Leu Arg Ser Arg Gln Val Thr Gly Thr Ala Gly
60                  65                  70                  75

ctg gat gcc tcc aag atc cag gtc gcc aac acc acc aag ccc att gac       773
Leu Asp Ala Ser Lys Ile Gln Val Ala Asn Thr Thr Lys Pro Ile Asp
                80                  85                  90

gac atc acc aaa cac ctg ccc cga cca tgc att ctg att tcc aac cac       821
Asp Ile Thr Lys His Leu Pro Arg Pro Cys Ile Leu Ile Ser Asn His
            95                  100                 105

cag aac gaa atg gac att ctg gtg ctc ggt cgc atc ttc ccc cag tac       869
Gln Asn Glu Met Asp Ile Leu Val Leu Gly Arg Ile Phe Pro Gln Tyr
        110                 115                 120

tgc tcc gtc acc gcc aaa aag gcc ctc aag tgg tac cct ctg ctg ggc       917
Cys Ser Val Thr Ala Lys Lys Ala Leu Lys Trp Tyr Pro Leu Leu Gly
    125                 130                 135

cag ttc atg gcg ctg tcc ggc acc atc ttc ctg gac cga aag gac cga       965
Gln Phe Met Ala Leu Ser Gly Thr Ile Phe Leu Asp Arg Lys Asp Arg
140                 145                 150                 155

acc aag tcc gtg cag acc ctc ggc ggc gcc gtc aag acc atc cag agc      1013
Thr Lys Ser Val Gln Thr Leu Gly Gly Ala Val Lys Thr Ile Gln Ser
                160                 165                 170

ggc aac gga ggc aag ggc cag agc gtc ttc atg ttc ccc gag gga acc      1061
Gly Asn Gly Gly Lys Gly Gln Ser Val Phe Met Phe Pro Glu Gly Thr
            175                 180                 185

cga tcc tac tcc aag gac gtc ggc atc atg ccc ttc aag aag ggc tgt      1109
Arg Ser Tyr Ser Lys Asp Val Gly Ile Met Pro Phe Lys Lys Gly Cys
        190                 195                 200
```

```
ttc cac ctg gcg gtc cag tcg ggc gct ccc att gtc ccc gtg gtg gtc       1157
Phe His Leu Ala Val Gln Ser Gly Ala Pro Ile Val Pro Val Val Val
    205                 210                 215

cag aac acc tcc cga atg ttt tct ttc ggc cga ggc aag ctg gac gcc       1205
Gln Asn Thr Ser Arg Met Phe Ser Phe Gly Arg Gly Lys Leu Asp Ala
220                 225                 230                 235

gga gag atc ctt gtc gac gtc ctg agc ccc att gag acc aag ggt ctg       1253
Gly Glu Ile Leu Val Asp Val Leu Ser Pro Ile Glu Thr Lys Gly Leu
                240                 245                 250

gac gcc agc aac gtc gac gct ctc atg gcc acc act tat aag gcc atg       1301
Asp Ala Ser Asn Val Asp Ala Leu Met Ala Thr Thr Tyr Lys Ala Met
            255                 260                 265

tgc gag act gcc gac cag att ggc tac gct ggc cag aag act cag tag       1349
Cys Glu Thr Ala Asp Gln Ile Gly Tyr Ala Gly Gln Lys Thr Gln
        270                 275                 280

agactgcagc acaagaagtg cttgtagcta ctttaggaga gagataggta atatgaaaca     1409

tttttcagat cgacacccac ggcgaaccat tggctgtgga gctatgggtg aatggattaa     1469

tatagcaacg aaatctacct cgattaccaa cgcaaaacga gcccactttc tctgtactgt     1529

gctatatcgt gtataccccca                                                1549
```

<210> 17
<211> 282
<212> PRT
<213> Yarrowia lipolytica

<400> 17

```
Met Ser Val Ala Ser Lys Leu Val Phe Tyr Val Arg Ala Ala Ile Ala
1               5                   10                  15

Val Val Ile Phe Ala Ala Cys Ala Thr Tyr Gly Val Leu Ala Ser Thr
            20                  25                  30

Ile Leu Thr Ala Ile Gly Lys Gln Gly Leu Ala Gln Trp Thr Val Ala
        35                  40                  45

Arg Ala Phe Tyr Tyr Ser Val Arg Ile Phe Leu Gly Ile Ser Ile Lys
    50                  55                  60

Leu Arg Ser Arg Gln Val Thr Gly Thr Ala Gly Leu Asp Ala Ser Lys
65                  70                  75                  80

Ile Gln Val Ala Asn Thr Thr Lys Pro Ile Asp Asp Ile Thr Lys His
                85                  90                  95

Leu Pro Arg Pro Cys Ile Leu Ile Ser Asn His Gln Asn Glu Met Asp
            100                 105                 110
```

```
Ile Leu Val Leu Gly Arg Ile Phe Pro Gln Tyr Cys Ser Val Thr Ala
        115                 120                 125

Lys Lys Ala Leu Lys Trp Tyr Pro Leu Leu Gly Gln Phe Met Ala Leu
    130                 135                 140

Ser Gly Thr Ile Phe Leu Asp Arg Lys Asp Arg Thr Lys Ser Val Gln
145                 150                 155                 160

Thr Leu Gly Gly Ala Val Lys Thr Ile Gln Ser Gly Asn Gly Gly Lys
                165                 170                 175

Gly Gln Ser Val Phe Met Phe Pro Glu Gly Thr Arg Ser Tyr Ser Lys
            180                 185                 190

Asp Val Gly Ile Met Pro Phe Lys Lys Gly Cys Phe His Leu Ala Val
        195                 200                 205

Gln Ser Gly Ala Pro Ile Val Pro Val Val Val Gln Asn Thr Ser Arg
    210                 215                 220

Met Phe Ser Phe Gly Arg Gly Lys Leu Asp Ala Gly Glu Ile Leu Val
225                 230                 235                 240

Asp Val Leu Ser Pro Ile Glu Thr Lys Gly Leu Asp Ala Ser Asn Val
                245                 250                 255

Asp Ala Leu Met Ala Thr Thr Tyr Lys Ala Met Cys Glu Thr Ala Asp
            260                 265                 270

Gln Ile Gly Tyr Ala Gly Gln Lys Thr Gln
        275                 280
```

<210> 18
<211> 303
<212> PRT
<213> Saccharomyces cerevisiae

<220>
<221> MISC_FEATURE
<222> (1)..(303)
<223> Slc1p; GenBank Accession No. NP_010231

<400> 18

```
Met Ser Val Ile Gly Arg Phe Leu Tyr Tyr Leu Arg Ser Val Leu Val
1               5                   10                  15

Val Leu Ala Leu Ala Gly Cys Gly Phe Tyr Gly Val Ile Ala Ser Ile
            20                  25                  30
```

```
Leu Cys Thr Leu Ile Gly Lys Gln His Leu Ala Gln Trp Ile Thr Ala
        35                  40                  45

Arg Cys Phe Tyr His Val Met Lys Leu Met Leu Gly Leu Asp Val Lys
    50                  55                  60

Val Val Gly Glu Glu Asn Leu Ala Lys Lys Pro Tyr Ile Met Ile Ala
65                  70                  75                  80

Asn His Gln Ser Thr Leu Asp Ile Phe Met Leu Gly Arg Ile Phe Pro
                85                  90                  95

Pro Gly Cys Thr Val Thr Ala Lys Lys Ser Leu Lys Tyr Val Pro Phe
            100                 105                 110

Leu Gly Trp Phe Met Ala Leu Ser Gly Thr Tyr Phe Leu Asp Arg Ser
        115                 120                 125

Lys Arg Gln Glu Ala Ile Asp Thr Leu Asn Lys Gly Leu Glu Asn Val
    130                 135                 140

Lys Lys Asn Lys Arg Ala Leu Trp Val Phe Pro Glu Gly Thr Arg Ser
145                 150                 155                 160

Tyr Thr Ser Glu Leu Thr Met Leu Pro Phe Lys Lys Gly Ala Phe His
                165                 170                 175

Leu Ala Gln Gln Gly Lys Ile Pro Ile Val Pro Val Val Val Ser Asn
            180                 185                 190

Thr Ser Thr Leu Val Ser Pro Lys Tyr Gly Val Phe Asn Arg Gly Cys
        195                 200                 205

Met Ile Val Arg Ile Leu Lys Pro Ile Ser Thr Glu Asn Leu Thr Lys
    210                 215                 220

Asp Lys Ile Gly Glu Phe Ala Glu Lys Val Arg Asp Gln Met Val Asp
225                 230                 235                 240

Thr Leu Lys Glu Ile Gly Tyr Ser Pro Ala Ile Asn Asp Thr Thr Leu
                245                 250                 255

Pro Pro Gln Ala Ile Glu Tyr Ala Ala Leu Gln His Asp Lys Lys Val
            260                 265                 270

Asn Lys Lys Ile Lys Asn Glu Pro Val Pro Ser Val Ser Ile Ser Asn
        275                 280                 285

Asp Val Asn Thr His Asn Glu Gly Ser Ser Val Lys Lys Met His
```

290 295 300

<210> 19
<211> 7
<212> PRT
<213> Artificial Sequence

<220>
<223> 1-acyl-sn-glycerol-3-phosphate acyltransferase motif

<220>
<221> misc_feature
<222> (3)..(6)
<223> Xaa can be any naturally occurring amino acid

<400> 19

**Asn His Xaa Xaa Xaa Xaa Asp**
**1 5**

<210> 20
<211> 4
<212> PRT
<213> Artificial Sequence

<220>
<223> 1-acyl-sn-glycerol-3-phosphate acyltransferase motif

<400> 20

**Glu Gly Thr Arg**
**1**

<210> 21
<211> 955
<212> DNA
<213> Mortierella alpina

<220>
<221> CDS
<222> (3)..(947)
<223> synthetic LPAAT1 (codon-optimized for Yarrowia lipolytica)

<400> 21

```
ac atg tct att ggt tcg tcc aac ccc gtg ctc ttg gct gcg att ccc          47
   Met Ser Ile Gly Ser Ser Asn Pro Val Leu Leu Ala Ala Ile Pro
   1               5                   10                  15

ttc gtc tac ctg ttt gtc ctc cca cga gtc ctg gct ttc ctg cct cag          95
Phe Val Tyr Leu Phe Val Leu Pro Arg Val Leu Ala Phe Leu Pro Gln
                20                  25                  30

aag gct cag ttc ctg gcc aaa tgt att gtg gtc ctg att gcc acg ctt         143
Lys Ala Gln Phe Leu Ala Lys Cys Ile Val Val Leu Ile Ala Thr Leu
            35                  40                  45

atc atg tcc gtt gca ggc tgc ttc atc tcg atc gtg tgc gct ctt ctg         191
Ile Met Ser Val Ala Gly Cys Phe Ile Ser Ile Val Cys Ala Leu Leu
        50                  55                  60
```

```
gac aag aga tac gtc atc aat tac gtt gtg tcg cga ttg ttc tcc ttc         239
Asp Lys Arg Tyr Val Ile Asn Tyr Val Val Ser Arg Leu Phe Ser Phe
    65                  70                  75

ctt gcc gct cga ccg tgt ggt gtg acc tat aag att gtt ggt gag gaa         287
Leu Ala Ala Arg Pro Cys Gly Val Thr Tyr Lys Ile Val Gly Glu Glu
80                  85                  90                  95

cac ctc gat aag tac cct gct atc gtg gtc tgt aac cat caa tcc tct         335
His Leu Asp Lys Tyr Pro Ala Ile Val Val Cys Asn His Gln Ser Ser
                100                 105                 110

atg gat atg atg gtt ttg gga cga gtt ttt cca aag cac tgc gtt gtc         383
Met Asp Met Met Val Leu Gly Arg Val Phe Pro Lys His Cys Val Val
            115                 120                 125

atg gcg aag aag gaa ctc ctg tac ttt ccc ttt ttg gga atg ttt atg         431
Met Ala Lys Lys Glu Leu Leu Tyr Phe Pro Phe Leu Gly Met Phe Met
        130                 135                 140

aaa ctg agc aac gct atc ttc atc gac cgg aag aac cac aag aaa gcc         479
Lys Leu Ser Asn Ala Ile Phe Ile Asp Arg Lys Asn His Lys Lys Ala
    145                 150                 155

atc gag tct acc acc caa gcc gtg gcg gac atg aag aag cac aac tct         527
Ile Glu Ser Thr Thr Gln Ala Val Ala Asp Met Lys Lys His Asn Ser
160                 165                 170                 175

gga atc tgg att ttc cca gag ggc acc cgg tct aga ctg gac aag gca         575
Gly Ile Trp Ile Phe Pro Glu Gly Thr Arg Ser Arg Leu Asp Lys Ala
                180                 185                 190

gac ctg ctg ccc ttc aag aaa ggt gcc ttt cat ctt gca att cag gcc         623
Asp Leu Leu Pro Phe Lys Lys Gly Ala Phe His Leu Ala Ile Gln Ala
            195                 200                 205

cag ctc cct att ctc ccc att atc tcg cag ggc tat tcc cat atc tac         671
Gln Leu Pro Ile Leu Pro Ile Ile Ser Gln Gly Tyr Ser His Ile Tyr
        210                 215                 220

gac tct tcg aag cgg tac ttc ccc ggt gga gag ctc gag atc aga gtc         719
Asp Ser Ser Lys Arg Tyr Phe Pro Gly Gly Glu Leu Glu Ile Arg Val
    225                 230                 235

ctg gag ccc att cct aca act ggc ctc act act gat gat gtg aac gac         767
Leu Glu Pro Ile Pro Thr Thr Gly Leu Thr Thr Asp Asp Val Asn Asp
240                 245                 250                 255

ctg atg gac aag aca cga aac ctt atg ctc aag cac ttg aag gag atg         815
Leu Met Asp Lys Thr Arg Asn Leu Met Leu Lys His Leu Lys Glu Met
                260                 265                 270

gat tcc cag tat tcg tcg agc act gct gaa aat gga tcc acg cac atc         863
Asp Ser Gln Tyr Ser Ser Ser Thr Ala Glu Asn Gly Ser Thr His Ile
            275                 280                 285

gac gcc gat att gcc aag tct aca gcc acc agc att ggc aac act gac         911
Asp Ala Asp Ile Ala Lys Ser Thr Ala Thr Ser Ile Gly Asn Thr Asp
        290                 295                 300

gac gca att aca aaa cgt cgt acc cct aag gaa taa gcggccgc                955
Asp Ala Ile Thr Lys Arg Arg Thr Pro Lys Glu
    305                 310
```

<210> 22
<211> 314
<212> PRT
<213> Mortierella alpina

<400> 22

Met Ser Ile Gly Ser Ser Asn Pro Val Leu Leu Ala Ala Ile Pro Phe
1 5 10 15

Val Tyr Leu Phe Val Leu Pro Arg Val Leu Ala Phe Leu Pro Gln Lys
20 25 30

Ala Gln Phe Leu Ala Lys Cys Ile Val Val Leu Ile Ala Thr Leu Ile
35 40 45

Met Ser Val Ala Gly Cys Phe Ile Ser Ile Val Cys Ala Leu Leu Asp
50 55 60

Lys Arg Tyr Val Ile Asn Tyr Val Val Ser Arg Leu Phe Ser Phe Leu
65 70 75 80

Ala Ala Arg Pro Cys Gly Val Thr Tyr Lys Ile Val Gly Glu Glu His
85 90 95

Leu Asp Lys Tyr Pro Ala Ile Val Val Cys Asn His Gln Ser Ser Met
100 105 110

Asp Met Met Val Leu Gly Arg Val Phe Pro Lys His Cys Val Val Met
115 120 125

Ala Lys Lys Glu Leu Leu Tyr Phe Pro Phe Leu Gly Met Phe Met Lys
130 135 140

Leu Ser Asn Ala Ile Phe Ile Asp Arg Lys Asn His Lys Lys Ala Ile
145 150 155 160

Glu Ser Thr Thr Gln Ala Val Ala Asp Met Lys Lys His Asn Ser Gly
165 170 175

Ile Trp Ile Phe Pro Glu Gly Thr Arg Ser Arg Leu Asp Lys Ala Asp
180 185 190

Leu Leu Pro Phe Lys Lys Gly Ala Phe His Leu Ala Ile Gln Ala Gln
195 200 205

Leu Pro Ile Leu Pro Ile Ile Ser Gln Gly Tyr Ser His Ile Tyr Asp
210 215 220

Ser Ser Lys Arg Tyr Phe Pro Gly Gly Glu Leu Glu Ile Arg Val Leu
225 230 235 240

Glu Pro Ile Pro Thr Thr Gly Leu Thr Thr Asp Asp Val Asn Asp Leu
245 250 255

Met Asp Lys Thr Arg Asn Leu Met Leu Lys His Leu Lys Glu Met Asp
260 265 270

Ser Gln Tyr Ser Ser Ser Thr Ala Glu Asn Gly Ser Thr His Ile Asp
275 280 285

Ala Asp Ile Ala Lys Ser Thr Ala Thr Ser Ile Gly Asn Thr Asp Asp
290 295 300

Ala Ile Thr Lys Arg Arg Thr Pro Lys Glu
305 310

<210> 23
<211> 12
<212> PRT
<213> Artificial Sequence

<220>
<223> membrane bound O-acyltransferase motif

<220>
<221> misc_feature
<222> (4)..(6)
<223> Xaa can be any naturally occurring amino acid

<220>
<221> misc_feature
<222> (9)..(11)
<223> Xaa can be any naturally occurring amino acid

<300>
<301> Hideo Shindou, Miki Etoa, Ryo Morimotoa and Takao Shimizua
<302> Identification of membrane O-acyltransferase family motifs
<303> Biochemical and Biophysical Research Communications
<304> 383
<305> 3
<306> 320-325
<307> 2009-04-08
<313> (1)..(12)

<400> 23

Trp His Gly Xaa Xaa Xaa Gly Tyr Xaa Xaa Xaa Phe
1 5 10

<210> 24
<211> 6
<212> PRT
<213> Artificial Sequence

<220>
<223> membrane bound O-acyltransferase motif

<220>
<221> misc_feature
<222> (2)..(5)
<223> Xaa can be any naturally occurring amino acid

<300>
<301> Hideo Shindou, Miki Etoa, Ryo Morimotoa and Takao Shimizua
<302> Identification of membrane O-acyltransferase family motifs
<303> Biochemical and Biophysical Research Communications
<304> 383
<305> 3
<306> 320-325
<307> 2009-04-08
<313> (1)..(6)

<400> 24

```
Tyr Xaa Xaa Xaa Xaa Phe
1               5
```

<210> 25
<211> 9
<212> PRT
<213> Artificial Sequence

<220>
<223> membrane bound O-acyltransferase motif

<220>
<221> misc_feature
<222> (2)..(4)
<223> Xaa can be any naturally occurring amino acid

<220>
<221> misc_feature
<222> (7)..(8)
<223> Xaa can be any naturally occurring amino acid

<300>
<301> Hideo Shindou, Miki Etoa, Ryo Morimotoa and Takao Shimizua
<302> Identification of membrane O-acyltransferase family motifs
<303> Biochemical and Biophysical Research Communications
<304> 383
<305> 3
<306> 320-325
<307> 2009-04-08
<313> (1)..(9)

<400> 25

```
Tyr Xaa Xaa Xaa Tyr Phe Xaa Xaa His
1               5
```

<210> 26
<211> 15
<212> PRT
<213> Artificial Sequence

<220>
<223> motif

<220>
<221> MISC_FEATURE
<222> (2)..(2)
<223> Xaa = Val [V] or Ile [I]

<220>
<221> MISC_FEATURE
<222> (3)..(3)
<223> Xaa = Leu [L] or Ile [I]

<220>
<221> misc_feature
<222> (4)..(5)
<223> Xaa can be any naturally occurring amino acid

<220>
<221> MISC_FEATURE
<222> (7)..(7)
<223> Xaa = Leu [L] or Ile [I] or Val [V]

<220>
<221> misc_feature
<222> (8)..(13)
<223> Xaa can be any naturally occurring amino acid

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION
<310> U.S. Patent Publication No. 2008-0145867-A1
<311> 2007-06-15
<312> 2008-06-19
<313> (1)..(15)

<400> 26

```
Met Xaa Xaa Xaa Xaa Lys Xaa Xaa Xaa Xaa Xaa Xaa Xaa Asp Gly
1               5                   10                  15
```

<210> 27
<211> 24
<212> PRT
<213> Artificial Sequence

<220>
<223> motif

<220>
<221> misc_feature
<222> (2)..(2)
<223> Xaa can be any naturally occurring amino acid

<220>
<221> misc_feature
<222> (6)..(7)
<223> Xaa can be any naturally occurring amino acid

<220>
<221> misc_feature
<222> (9)..(11)
<223> Xaa can be any naturally occurring amino acid

<220>
<221> MISC_FEATURE
<222> (12)..(12)
<223> Xaa = Glu [E] or Asp [D]

<220>
<221> MISC_FEATURE
<222> (13)..(13)
<223> Xaa = Ala [A] or Gly [G]

<220>
<221> misc_feature
<222> (14)..(18)
<223> Xaa can be any naturally occurring amino acid

<220>
<221> misc_feature
<222> (20)..(20)
<223> Xaa can be any naturally occurring amino acid

<220>
<221> MISC_FEATURE
<222> (22)..(22)
<223> Xaa = Phe [F] or Tyr [Y]

<220>
<221> misc_feature
<222> (23)..(23)
<223> Xaa can be any naturally occurring amino acid

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION
<310> U.S. Patent Publication No. 2008-0145867-A1
<311> 2007-06-15
<312> 2008-06-19
<313> (1)..(15)

<400> 27

```
Arg Xaa Lys Tyr Tyr Xaa Xaa Trp Xaa Xaa Xaa Xaa Xaa Xaa Xaa Xaa
1               5                   10                  15

Xaa Xaa Gly Xaa Gly Xaa Xaa Gly
            20
```

<210> 28
<211> 20
<212> PRT
<213> Artificial Sequence

<220>

<223> motif

<220>
<221> misc_feature
<222> (2)..(12)
<223> Xaa can be any naturally occurring amino acid

<220>
<221> misc_feature
<222> (15)..(16)
<223> Xaa can be any naturally occurring amino acid

<220>
<221> MISC_FEATURE
<222> (17)..(17)
<223> Xaa = Thr [T] or Val [V]

<220>
<221> misc_feature
<222> (18)..(19)
<223> Xaa can be any naturally occurring amino acid

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION
<310> U.S. Patent Publication No. 2008-0145867-A1
<311> 2007-06-15
<312> 2008-06-19
<313> (1)..(15)

<400> 28

```
Glu Xaa Xaa Xaa Xaa Xaa Xaa Xaa Xaa Xaa Xaa Xaa Trp Asn Xaa Xaa
1               5                   10                  15

Xaa Xaa Xaa Trp
            20
```

<210> 29
<211> 15
<212> PRT
<213> Artificial Sequence

<220>
<223> motif

<220>
<221> misc_feature
<222> (3)..(3)
<223> Xaa can be any naturally occurring amino acid

<220>
<221> misc_feature
<222> (7)..(8)
<223> Xaa can be any naturally occurring amino acid

<220>

<221> misc_feature
<222> (12)..(13)
<223> Xaa can be any naturally occurring amino acid

<220>
<221> MISC_FEATURE
<222> (14)..(14)
<223> Xaa = Thr [T] or Phe [F]

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION

<310> U.S. Patent Publication No. 2008-0145867-A1
<311> 2007-06-15
<312> 2008-06-19
<313> (1)..(15)

<400> 29

**Ser Ala Xaa Trp His Gly Xaa Xaa Pro Gly Tyr Xaa Xaa Xaa Phe**
**1 5 10 15**

<210> 30
<211> 7
<212> PRT
<213> Artificial Sequence

<220>
<223> 1-acyl-sn-glycerol-3-phosphate acyltransferase motif

<220>
<221> misc_feature
<222> (2)..(3)
<223> Xaa can be any naturally occurring amino acid

<220>
<221> MISC_FEATURE
<222> (6)..(6)
<223> Xaa = Asp [D] or Arg [R]

<300>
<301> Tal M. Lewin, Ping Wang, and Rosalind A. Coleman
<302> Analysis of Amino Acid Motifs Diagnostic for the sn-Glycerol-3-phosphate Acyltransferase Reaction
<303> Biochemistry
<304> 38
<305> 18
<306> 57645771
<307> 1999-04-15
<313> (1)..(7)

<300>
<301> Atsushi Yamashita, Hiroki Nakanishia, Hiroshi Suzukia, Ryo Kamataa, Ken Tanakaa, Keizo Wakua and Takayuki Sugiura
<302> Topology of acyltransferase motifs and substrate specificity and accessibility in 1-acyl-sn-glycero-3-phosphate acyltransferase 1
<303> Biochimica et Biophysica Acta
<304> 1771

<305> 9
<306> 1202-1215
<307> 2007-07-17
<313> (1)..(7)

<400> 30

```
Gly Xaa Xaa Phe Ile Xaa Arg
1               5
```

<210> 31
<211> 6
<212> PRT
<213> Artificial Sequence

<220>
<223> 1-acyl-sn-glycerol-3-phosphate acyltransferase motif

<220>
<221> MISC_FEATURE
<222> (1)..(1)
<223> Xaa = Val [V] or Ile [I]

<220>
<221> MISC_FEATURE
<222> (2)..(2)
<223> Xaa = Pro [P] or Xaa

<220>
<221> MISC_FEATURE
<222> (3)..(3)
<223> Xaa = Ile [I] or Val [V] or Leu [L]

<220>
<221> MISC_FEATURE
<222> (4)..(4)
<223> Xaa = Ile [I] or Val [V]

<220>
<221> MISC_FEATURE
<222> (6)..(6)
<223> Xaa = Val [V] or Ile [I]

<300>
<301> Atsushi Yamashita, Hiroki Nakanishia, Hiroshi Suzukia, Ryo Kamataa, Ken Tanakaa, Keizo Wakua and Takayuki Sugiura
<302> Topology of acyltransferase motifs and substrate specificity and accessibility in 1-acyl-sn-glycero-3-phosphate acyltransferase 1
<303> Biochimica et Biophysica Acta
<304> 1771
<305> 9
<306> 1202-1215
<307> 2007-07-17
<313> (1)..(6)

<400> 31

**Xaa Xaa Xaa Xaa Pro Xaa**
**1 5**

<210> 32
<211> 6
<212> PRT
<213> Artificial Sequence

<220>
<223> 1-acyl-sn-glycerol-3-phosphate acyltransferase motif

<300>
<301> Atsushi Yamashita, Hiroki Nakanishia, Hiroshi Suzukia, Ryo Kamataa, Ken Tanakaa, Keizo Wakua and Takayuki Sugiura
<302> Topology of acyltransferase motifs and substrate specificity and accessibility in 1-acyl-sn-glycero-3-phosphate acyltransferase 1
<303> Biochimica et Biophysica Acta
<304> 1771
<305> 9
<306> 1202-1215
<307> 2007-07-17
<313> (1)..(6)

<400> 32

**Ile Val Pro Ile Val Met**
**1 5**

<210> 33
<211> 8739
<212> DNA
<213> Artificial Sequence

<220>
<223> Plasmid pY116

<400> 33

ggccgccacc gcggcccgag attccggcct cttcggccgc caagcgaccc gggtggacgt 60

ctagaggtac ctagcaatta acagatagtt tgccggtgat aattctctta acctcccaca 120

ctcctttgac ataacgattt atgtaacgaa actgaaattt gaccagatat tgtgtccgcg 180

gtggagctcc agcttttgtt ccctttagtg agggtttaaa cgagcttggc gtaatcatgg 240

tcatagctgt ttcctgtgtg aaattgttat ccgctcacaa ttccacacaa cgtacgagcc 300

ggaagcataa agtgtaaagc ctggggtgcc taatgagtga gctaactcac attaattgcg 360

ttgcgctcac tgcccgcttt ccagtcggga aacctgtcgt gccagctgca ttaatgaatc 420

ggccaacgcg cggggagagg cggtttgcgt attgggcgct cttccgcttc ctcgctcact 480

gactcgctgc gctcggtcgt tcggctgcgg cgagcggtat cagctcactc aaaggcggta 540

atacggttat ccacagaatc aggggataac gcaggaaaga acatgtgagc aaaaggccag 600

caaaaggcca ggaaccgtaa aaaggccgcg ttgctggcgt ttttccatag gctccgcccc 660

cctgacgagc atcacaaaaa tcgacgctca agtcagaggt ggcgaaaccc gacaggacta 720

taaagatacc aggcgtttcc ccctggaagc tccctcgtgc gctctcctgt tccgaccctg 780

ccgcttaccg gatacctgtc cgcctttctc ccttcgggaa gcgtggcgct ttctcatagc 840

tcacgctgta ggtatctcag ttcggtgtag gtcgttcgct ccaagctggg ctgtgtgcac 900

gaaccccccg ttcagcccga ccgctgcgcc ttatccggta actatcgtct tgagtccaac 960

ccggtaagac acgacttatc gccactggca gcagccactg gtaacaggat tagcagagcg 1020

aggtatgtag gcggtgctac agagttcttg aagtggtggc ctaactacgg ctacactaga 1080

aggacagtat ttggtatctg cgctctgctg aagccagtta ccttcggaaa aagagttggt 1140

agctcttgat ccggcaaaca aaccaccgct ggtagcggtg gtttttttgt ttgcaagcag 1200

cagattacgc gcagaaaaaa aggatctcaa gaagatcctt tgatcttttc tacggggtct 1260

gacgctcagt ggaacgaaaa ctcacgttaa gggattttgg tcatgagatt atcaaaaagg 1320

atcttcacct agatcctttt aaattaaaaa tgaagtttta aatcaatcta aagtatatat 1380

gagtaaactt ggtctgacag ttaccaatgc ttaatcagtg aggcacctat ctcagcgatc 1440

tgtctatttc gttcatccat agttgcctga ctccccgtcg tgtagataac tacgatacgg 1500

gagggcttac catctggccc cagtgctgca atgataccgc gagacccacg ctcaccggct 1560

ccagatttat cagcaataaa ccagccagcc ggaagggccg agcgcagaag tggtcctgca 1620

actttatccg cctccatcca gtctattaat tgttgccggg aagctagagt aagtagttcg 1680

ccagttaata gtttgcgcaa cgttgttgcc attgctacag gcatcgtggt gtcacgctcg 1740

tcgtttggta tggcttcatt cagctccggt tcccaacgat caaggcgagt tacatgatcc 1800

cccatgttgt gcaaaaaagc ggttagctcc ttcggtcctc cgatcgttgt cagaagtaag 1860

ttggccgcag tgttatcact catggttatg gcagcactgc ataattctct tactgtcatg 1920

ccatccgtaa gatgcttttc tgtgactggt gagtactcaa ccaagtcatt ctgagaatag 1980

tgtatgcggc gaccgagttg ctcttgcccg gcgtcaatac gggataatac cgcgccacat 2040

agcagaactt taaaagtgct catcattgga aaacgttctt cggggcgaaa actctcaagg 2100

atcttaccgc tgttgagatc cagttcgatg taacccactc gtgcacccaa ctgatcttca 2160

gcatctttta ctttcaccag cgtttctggg tgagcaaaaa caggaaggca aaatgccgca 2220

aaaaagggaa taagggcgac acggaaatgt tgaatactca tactcttcct ttttcaatat 2280

tattgaagca tttatcaggg ttattgtctc atgagcggat acatatttga atgtatttag 2340

aaaaataaac aaataggggt tccgcgcaca tttccccgaa aagtgccacc tgacgcgccc 2400

tgtagcggcg cattaagcgc ggcgggtgtg gtggttacgc gcagcgtgac cgctacactt 2460

gccagcgccc tagcgcccgc tcctttcgct ttcttccctt cctttctcgc cacgttcgcc 2520

ggctttcccc gtcaagctct aaatcggggg ctccctttag ggttccgatt tagtgcttta 2580

cggcacctcg accccaaaaa acttgattag ggtgatggtt cacgtagtgg gccatcgccc 2640

tgatagacgg tttttcgccc tttgacgttg gagtccacgt tctttaatag tggactcttg 2700

ttccaaactg gaacaacact caaccctatc tcggtctatt cttttgattt ataagggatt 2760

ttgccgattt cggcctattg gttaaaaaat gagctgattt aacaaaaatt taacgcgaat 2820

tttaacaaaa tattaacgct tacaatttcc attcgccatt caggctgcgc aactgttggg 2880

aagggcgatc ggtgcgggcc tcttcgctat tacgccagct ggcgaaaggg ggatgtgctg 2940

caaggcgatt aagttgggta acgccagggt tttcccagtc acgacgttgt aaaacgacgg 3000

ccagtgaatt gtaatacgac tcactatagg gcgaattggg taccgggccc cccctcgagg 3060

tcgatggtgt cgataagctt gatatcgaat tcatgtcaca caaaccgatc ttcgcctcaa 3120

ggaaacctaa ttctacatcc gagagactgc cgagatccag tctacactga ttaattttcg 3180

ggccaataat ttaaaaaaat cgtgttatat aatattatat gtattatata tatacatcat 3240

gatgatactg acagtcatgt cccattgcta aatagacaga ctccatctgc cgcctccaac 3300

tgatgttctc aatatttaag gggtcatctc gcattgttta ataataaaca gactccatct 3360

accgcctcca aatgatgttc tcaaaatata ttgtatgaac ttatttttat tacttagtat 3420

tattagacaa cttacttgct ttatgaaaaa cacttcctat ttaggaaaca atttataatg 3480
gcagttcgtt catttaacaa tttatgtaga ataaatgtta taaatgcgta tgggaaatct 3540
taaatatgga tagcataaat gatatctgca ttgcctaatt cgaaatcaac agcaacgaaa 3600
aaaatccctt gtacaacata aatagtcatc gagaaatatc aactatcaaa gaacagctat 3660
tcacacgtta ctattgagat tattattgga cgagaatcac acactcaact gtctttctct 3720
cttctagaaa tacaggtaca agtatgtact attctcattg ttcatacttc tagtcatttc 3780
atcccacata ttccttggat ttctctccaa tgaatgacat tctatcttgc aaattcaaca 3840
attataataa gatataccaa agtagcggta tagtggcaat caaaaagctt ctctggtgtg 3900
cttctcgtat ttatttttat tctaatgatc cattaaaggt atatatttat ttcttgttat 3960
ataatccttt tgtttattac atgggctgga tacataaagg tattttgatt taatttttg 4020
cttaaattca atcccccctc gttcagtgtc aactgtaatg gtaggaaatt accatacttt 4080
tgaagaagca aaaaaaatga aagaaaaaaa aaatcgtatt tccaggttag acgttccgca 4140
gaatctagaa tgcggtatgc ggtacattgt tcttcgaacg taaaagttgc gctccctgag 4200
atattgtaca tttttgcttt tacaagtaca agtacatcgt acaactatgt actactgttg 4260
atgcatccac aacagtttgt tttgtttttt tttgtttttt ttttttctaa tgattcatta 4320
ccgctatgta tacctacttg tacttgtagt aagccgggtt attggcgttc aattaatcat 4380
agacttatga atctgcacgg tgtgcgctgc gagttacttt tagcttatgc atgctacttg 4440
ggtgtaatat tgggatctgt tcggaaatca acggatgctc aaccgatttc gacagtaatt 4500
aattaatttg aatcgaatcg gagcctaaaa tgaacccgag tatatctcat aaaattctcg 4560
gtgagaggtc tgtgactgtc agtacaaggt gccttcatta tgccctcaac cttaccatac 4620
ctcactgaat gtagtgtacc tctaaaaatg aaatacagtg ccaaaagcca aggcactgag 4680
ctcgtctaac ggacttgata tacaaccaat taaaacaaat gaaaagaaat acagttcttt 4740
gtatcatttg taacaattac cctgtacaaa ctaaggtatt gaaatcccac aatattccca 4800
aagtccaccc ctttccaaat tgtcatgcct acaactcata taccaagcac taacctacca 4860
aacaccacta aaaccccaca aaatatatct taccgaatat acagtaacaa gctaccacca 4920
cactcgttgg gtgcagtcgc cagcttaaag atatctatcc acatcagcca caactccctt 4980
cctttaataa accgactaca cccttggcta ttgaggttat gagtgaatat actgtagaca 5040
agacactttc aagaagactg tttccaaaac gtaccactgt cctccactac aaacacaccc 5100
aatctgcttc ttctagtcaa ggttgctaca ccggtaaatt ataaatcatc atttcattag 5160
cagggcaggg ccctttttat agagtcttat acactagcgg accctgccgg tagaccaacc 5220
cgcaggcgcg tcagtttgct ccttccatca atgcgtcgta gaaacgactt actccttctt 5280
gagcagctcc ttgaccttgt tggcaacaag tctccgacct cggaggtgga ggaagagcct 5340
ccgatatcgg cggtagtgat accagcctcg acggactcct tgacggcagc ctcaacagcg 5400

tcaccggcgg gcttcatgtt aagagagaac ttgagcatca tggcggcaga cagaatggtg 5460

gcaatggggt tgaccttctg cttgccgaga tcgggggcag atccgtgaca gggctcgtac 5520

agaccgaacg cctcgttggt gtcgggcaga gaagccagag aggcggaggg cagcagaccc 5580

agagaaccgg ggatgacgga ggcctcgtcg gagatgatat cgccaaacat gttggtggtg 5640

atgatgatac cattcatctt ggagggctgc ttgatgagga tcatggcggc cgagtcgatc 5700

agctggtggt tgagctcgag ctgggggaat tcgtccttga ggactcgagt gacagtcttt 5760

cgccaaagtc gagaggaggc cagcacgttg gccttgtcaa gagaccacac gggaagaggg 5820

gggttgtgct gaagggccag gaaggcggcc attcgggcaa ttcgctcaac ctcaggaacg 5880

gagtaggtct cggtgtcgga agcgacgcca gatccgtcat cctcctttcg ctctccaaag 5940

tagatacctc cgacgagctc tcggacaatg atgaagtcgg tgccctcaac gtttcggatg 6000

ggggagagat cggcgagctt gggcgacagc agctggcagg gtcgcaggtt ggcgtacagg 6060

ttcaggtcct ttcgcagctt gaggagaccc tgctcgggtc gcacgtcggt tcgtccgtcg 6120

ggagtggtcc atacggtgtt ggcagcgcct ccgacagcac cgagcataat agagtcagcc 6180

tttcggcaga tgtcgagagt agcgtcggtg atgggctcgc cctccttctc aatggcagct 6240

cctccaatga gtcggtcctc aaacacaaac tcggtgccgg aggcctcagc aacagacttg 6300

agcaccttga cggcctcggc aatcacctcg gggccacaga agtcgccgcc gagaagaaca 6360

atcttcttgg agtcagtctt ggtcttctta gtttcgggtt ccattgtgga tgtgtgtggt 6420

tgtatgtgtg atgtggtgtg tggagtgaaa atctgtggct ggcaaacgct cttgtatata 6480

tacgcacttt tgcccgtgct atgtggaaga ctaaacctcc gaagattgtg actcaggtag 6540

tgcggtatcg gctagggacc caaaccttgt cgatgccgat agcgctatcg aacgtacccc 6600

agccggccgg gagtatgtcg gaggggacat acgagatcgt caagggtttg tggccaactg 6660

gtatttaaat gtagctaacg gtagcaggcg aactactggt acatacctcc cccggaatat 6720

gtacaggcat aatgcgtatc tgtgggacat gtggtcgttg cgccattatg taagcagcgt 6780

gtactcctct gactgtccat atggtttgct ccatctcacc ctcatcgttt tcattgttca 6840

caggcggcca caaaaaaact gtcttctctc cttctctctt cgccttagtc tactcggacc 6900

agttttagtt tagcttggcg ccactggata aatgagacct caggccttgt gatgaggagg 6960

tcacttatga agcatgttag gaggtgcttg tatggataga gaagcaccca aaataataag 7020

aataataata aaacaggggg cgttgtcatt tcatatcgtg ttttcaccat caatacacct 7080

ccaaacaatg cccttcatgt ggccagcccc aatattgtcc tgtagttcaa ctctatgcag 7140

ctcgtatctt attgagcaag taaaactctg tcagccgata ttgcccgacc cgcgacaagg 7200

gtcaacaagg tggtgtaagg ccttcgcaga agtcaaaact gtgccaaaca aacatctaga 7260

gtctctttgg tgtttctcgc atatatttwa tcggctgtct tacgtatttg cgcctcggta 7320

ccggactaat ttcggatcat ccccaatacg ctttttcttc gcagctgtca acagtgtcca 7380
tgatctatcc acctaaatgg gtcatatgag gcgtataatt tcgtggtgct gataataatt 7440
cccatatatt tgacacaaaa cttccccccc tagacataca tctcacaatc tcacttcttg 7500
tgcttctgtc acacatctcc tccagctgac ttcaactcac acctctgccc cagttggtct 7560
acagcggtat aaggtttctc cgcatagagg tgcaccactc ctcccgatac ttgtttgtgt 7620
gacttgtggg tcacgacata tatatctaca cacattgcgc caccctttgg ttcttccagc 7680
acaacaaaaa cacgacacgc taaccatggc caatttactg accgtacacc aaaatttgcc 7740
tgcattaccg gtcgatgcaa cgagtgatga ggttcgcaag aacctgatgg acatgttcag 7800
ggatcgccag gcgttttctg agcatacctg gaaaatgctt ctgtccgttt gccggtcgtg 7860
ggcggcatgg tgcaagttga ataaccggaa atggtttccc gcagaacctg aagatgttcg 7920
cgattatctt ctatatcttc aggcgcgcgg tctggcagta aaaactatcc agcaacattt 7980
gggccagcta aacatgcttc atcgtcggtc cgggctgcca cgaccaagtg acagcaatgc 8040
tgtttcactg gttatgcggc ggatccgaaa agaaaacgtt gatgccggtg aacgtgcaaa 8100
acaggctcta gcgttcgaac gcactgattt cgaccaggtt cgttcactca tggaaaatag 8160
cgatcgctgc caggatatac gtaatctggc atttctgggg attgcttata acaccctgtt 8220
acgtatagcc gaaattgcca ggatcagggt taaagatatc tcacgtactg acggtgggag 8280
aatgttaatc catattggca gaacgaaaac gctggttagc accgcaggtg tagagaaggc 8340
acttagcctg ggggtaacta aactggtcga gcgatggatt tccgtctctg gtgtagctga 8400
tgatccgaat aactacctgt tttgccgggt cagaaaaaat ggtgttgccg cgccatctgc 8460
caccagccag ctatcaactc gcgccctgga agggattttt gaagcaactc atcgattgat 8520
ttacggcgct aaggatgact ctggtcagag atacctggcc tggtctggac acagtgcccg 8580
tgtcggagcc gcgcgagata tggcccgcgc tggagtttca ataccggaga tcatgcaagc 8640
tggtggctgg accaatgtaa atattgtcat gaactatatc cgtaacctgg atagtgaaac 8700
aggggcaatg gtgcgcctgc tggaagatgg cgattaagc 8739

<210> 34
<211> 13975
<212> DNA
<213> Artificial Sequence

<220>
<223> Plasmid pZKSL-5S5A5

<400> 34

```
cgatggttaa tgctgctgtg tgctgtgtgt gtgtgttgtt tggcgctcat tgttgcgtta      60
tgcagcgtac accacaatat tggaagctta ttagcctttc tatttttttcg tttgcaaggc      120
ttaacaacat tgctgtggag agggatgggg atatggaggc cgctggaggg agtcggagag      180
gcgttttgga gcggcttggc ctggcgccca gctcgcgaaa cgcacctagg accctttggc      240
```

```
acgccgaaat gtgccacttt tcagtctagt aacgccttac ctacgtcatt ccatgcgtgc     300
atgtttgcgc ctttttttccc ttgcccttga tcgccacaca gtacagtgca ctgtacagtg     360
gaggttttgg gggggtctta gatgggagct aaaagcggcc tagcggtaca ctagtgggat     420
tgtatggagt ggcatggagc ctaggtggag cctgacagga cgcacgaccg gctagcccgt     480
gacagacgat gggtggctcc tgttgtccac cgcgtacaaa tgtttgggcc aaagtcttgt     540
cagccttgct tgcgaaccta attcccaatt ttgtcacttc gcacccccat tgatcgagcc     600
ctaacccctg cccatcaggc aatccaatta agctcgcatt gtctgccttg tttagtttgg     660
ctcctgcccg tttcggcgtc cacttgcaca aacacaaaca agcattatat ataaggctcg     720
tctctccctc ccaaccacac tcactttttt gcccgtcttc ccttgctaac acaaaagtca     780
agaacacaaa caaccacccc aaccccctta cacacaagac atatctacag caatggccat     840
ggctctcagt cttaccacag aacagctgtt agaacgccct gatttggttg cgattgatgg     900
catcctctac gaccttgaag ggcttgccaa agttcatcca ggatccgatt tgattctcgc     960
ttctggtgcc tctgatgcct cccctctctt ttattcaatg catccatacg tcaaaccgga    1020
gaactccaaa ttgcttcaac agttcgtccg agggaagcat gaccgcacct cgaaggacat    1080
tgtctacacg tatgattctc ccttcgcaca agacgttaag cggacaatgc gcgaggtgat    1140
gaaagggagg aactggtacg caacccctgg cttctggctg cgcaccgttg ggatcatcgc    1200
cgtgacggcc ttttgcgagt ggcactgggc taccacgggg atggtgctgt ggggcctgtt    1260
gactggattc atgcacatgc agatcggctt atccatccag catgatgcgt cccacggggc    1320
catcagcaag aagccttggg tcaacgccct cttcgcctac ggcattgacg tcatcggatc    1380
gtcccggtgg atttggctgc agtcgcacat catgcggcac cacacctaca ccaaccagca    1440
cggcctcgac ctggatgcgg agtcggcaga gccgttcctg gtgttccaca actacccccgc    1500
cgcaaacacc gcccgaaagt ggttccaccg cttccaggct tggtacatgt accttgtgct    1560
gggggcatac ggggtatcgc tggtgtacaa cccgctctac attttccgga tgcagcacaa    1620
tgacaccatc ccagagtctg tcacggccat gcgggaaaat ggctttctgc ggcgctaccg    1680
cacacttgca ttcgtgatgc gagctttctt catcttccgg accgcattct tgccctggta    1740
cctcactggg acctcattgc tgatcaccat tcctctggtg cccaccgcaa ctggtgcctt    1800
cttgacgttc ttcttcattt tgtcccacaa ttttgatggc tccgaacgga tcccccgacaa    1860
gaactgcaag gttaagcgat ctgagaagga cgttgaggct gaccaaattg actggtatcg    1920
ggcgcaggtg gagacgtcct ccacatacgg tggccccatc gccatgttct tcactggcgg    1980
tctcaatttc cagatcgagc accacctctt tccccggatg tcgtcttggc actacccctt    2040
cgtccagcag gcggtccggg agtgttgcga acgacatgga gtgcgatatg ttttctaccc    2100
taccatcgtc ggcaacatca tctccaccct gaagtacatg cataaggtgg gtgtcgtcca    2160
```

ctgcgtgaag gacgcacagg attcctaagc ggccgcattg atgattggaa acacacacat 2220
gggttatatc taggtgagag ttagttggac agttatatat taaatcagct atgccaacgg 2280
taacttcatt catgtcaacg aggaaccagt gactgcaagt aatatagaat ttgaccacct 2340
tgccattctc ttgcactcct ttactatatc tcatttattt cttatataca aatcacttct 2400
tcttcccagc atcgagctcg gaaacctcat gagcaataac atcgtggatc tcgtcaatag 2460
agggcttttt ggactccttg ctgttggcca ccttgtcctt gctgtctggc tcattctgtt 2520
tcaacgcctt ttaattaact ctcctgcagc ttcctcagcg agacactgct cctgtctgga 2580
cccgagctaa ggctctgttc gacaaacacg ttctgcgaat tggcgagtaa tttcattaat 2640
gcaaatagac gtgtatttga aaaggaggag atgatggtac cagtaattta cggtgtttga 2700
atagacaaat tatatatata aaattaacct gatgaatgag tgtatgaatg gattgcttaa 2760
tataccgagg gagagccggc attatcaata tttattgtcc taagaagcta aaatatgctg 2820
ttccgttgat agcgatgtct gtttgagagt caatggcaga acgcggagga gtattgttag 2880
ttggtgatcg gtttactcga catcgagtag gggcgagac agaagatatc ccgaatccat 2940
tgcattgttt attaggatgt tcacaacaca actccctcta gactctgggg atgtgcgtta 3000
gaagaatgac ctggagcaag agtgttcaga ttgatccgtt caaattttca agattactgt 3060
tggggttgtt tttgaatcca cattagctgg acgactattg catctgagcg ctcggaacgt 3120
ctccctttcc cttcagcttt atacgagtcg attccataag cgcgacctct cgaattgcct 3180
tcggttgtga agccataggc aaaggtgtgg ctatggaatg catgcgacgt cgggcccaat 3240
tcgccctata gtgagtcgta ttacaattca ctggccgtcg ttttacaacg tcgtgactgg 3300
gaaaaccctg gcgttaccca acttaatcgc cttgcagcac atcccccttt cgccagctgg 3360
cgtaatagcg aagaggcccg caccgatcgc ccttcccaac agttgcgcag cctgaatggc 3420
gaatggacgc gccctgtagc ggcgcattaa gcgcggcggg tgtggtggtt acgcgcagcg 3480
tgaccgctac acttgccagc gccctagcgc ccgctccttt cgctttcttc ccttcctttc 3540
tcgccacgtt cgccggcttt ccccgtcaag ctctaaatcg ggggctccct ttagggttcc 3600
gatttagtgc tttacggcac ctcgacccca aaaaacttga ttagggtgat ggttcacgta 3660
gtgggccatc gccctgatag acggtttttc gccctttgac gttggagtcc acgttcttta 3720
atagtggact cttgttccaa actggaacaa cactcaaccc tatctcggtc tattcttttg 3780
atttataagg gattttgccg atttcggcct attggttaaa aaatgagctg atttaacaaa 3840
aatttaacgc gaattttaac aaaatattaa cgcttacaat ttcctgatgc ggtattttct 3900
ccttacgcat ctgtgcggta tttcacaccg catcaggtgg cacttttcgg ggaaatgtgc 3960
gcggaacccc tatttgttta tttttctaaa tacattcaaa tatgtatccg ctcatgagac 4020
aataaccctg ataaatgctt caataatatt gaaaaaggaa gagtatgagt attcaacatt 4080
tccgtgtcgc ccttattccc ttttttgcgg cattttgcct tcctgttttt gctcacccag 4140

aaacgctggt gaaagtaaaa gatgctgaag atcagttggg tgcacgagtg ggttacatcg 4200
aactggatct caacagcggt aagatccttg agagttttcg ccccgaagaa cgttttccaa 4260
tgatgagcac ttttaaagtt ctgctatgtg gcgcggtatt atcccgtatt gacgccgggc 4320
aagagcaact cggtcgccgc atacactatt ctcagaatga cttggttgag tactcaccag 4380
tcacagaaaa gcatcttacg gatggcatga cagtaagaga attatgcagt gctgccataa 4440
ccatgagtga taacactgcg gccaacttac ttctgacaac gatcggagga ccgaaggagc 4500
taaccgcttt tttgcacaac atgggggatc atgtaactcg ccttgatcgt tgggaaccgg 4560
agctgaatga agccatacca aacgacgagc gtgacaccac gatgcctgta gcaatggcaa 4620
caacgttgcg caaactatta actggcgaac tacttactct agcttcccgg caacaattaa 4680
tagactggat ggaggcggat aaagttgcag gaccacttct gcgctcggcc cttccggctg 4740
gctggtttat tgctgataaa tctggagccg gtgagcgtgg gtctcgcggt atcattgcag 4800
cactggggcc agatggtaag ccctcccgta tcgtagttat ctacacgacg gggagtcagg 4860
caactatgga tgaacgaaat agacagatcg ctgagatagg tgcctcactg attaagcatt 4920
ggtaactgtc agaccaagtt tactcatata tactttagat tgatttaaaa cttcattttt 4980
aatttaaaag gatctaggtg aagatccttt ttgataatct catgaccaaa atcccttaac 5040
gtgagttttc gttccactga gcgtcagacc ccgtagaaaa gatcaaagga tcttcttgag 5100
atcctttttt tctgcgcgta atctgctgct tgcaaacaaa aaaaccaccg ctaccagcgg 5160
tggtttgttt gccggatcaa gagctaccaa ctctttttcc gaaggtaact ggcttcagca 5220
gagcgcagat accaaatact gttcttctag tgtagccgta gttaggccac cacttcaaga 5280
actctgtagc accgcctaca tacctcgctc tgctaatcct gttaccagtg gctgctgcca 5340
gtggcgataa gtcgtgtctt accgggttgg actcaagacg atagttaccg gataaggcgc 5400
agcggtcggg ctgaacgggg ggttcgtgca cacagcccag cttggagcga acgacctaca 5460
ccgaactgag atacctacag cgtgagctat gagaaagcgc cacgcttccc gaagggagaa 5520
aggcggacag gtatccggta agcggcaggg tcggaacagg agagcgcacg agggagcttc 5580
caggggggaaa cgcctggtat ctttatagtc ctgtcgggtt tcgccacctc tgacttgagc 5640
gtcgattttt gtgatgctcg tcaggggggc ggagcctatg gaaaaacgcc agcaacgcgg 5700
cctttttacg gttcctggcc ttttgctggc cttttgctca catgttcttt cctgcgttat 5760
cccctgattc tgtggataac cgtattaccg cctttgagtg agctgatacc gctcgccgca 5820
gccgaacgac cgagcgcagc gagtcagtga gcgaggaagc ggaagagcgc ccaatacgca 5880
aaccgcctct ccccgcgcgt tggccgattc attaatgcag ctggcgcgcc gaggtttcca 5940
acgagataac atcgtggcag ctgccaccat gaccgatatg cctgaaataa gtgaattgac 6000
catcagaagt tctgcattca aatagaacta tatcatattc ggctcagttt tttcaataat 6060

agtccaatcc ctaagtctcc tatccaaaat ggttcctgac cagccacatc catgatcatg 6120
actgcgtgac aggaacagtc attccgtgga tgaacgactt tacgctcagg tactgtaaat 6180
atctgtaaag ggcagacaac caaccaattg agtaacctgt gagacttgaa acgtaagatg 6240
acttcacaca caaagtcact tgactcaacc gtggctctca attgcacaaa atcactctgc 6300
actaatctat tgcaggagtc aggctatgaa caactagacg acagctactt atgtgttata 6360
tagaggaata ttaaaaaatc taagaataat cataaagtta caaaataatt atcagatttc 6420
gagccacagg tcacccctaa catgtgttat tgcacaccca caatcctcag cttgatgtca 6480
tttaattctt ccagccacca tctctctctc caaccctaat ggcaaacttt attttggtgg 6540
agcgatgact cttactcaac tgcagcatac ttaagcacaa ttgttcccca gcctgatacg 6600
acacaccatc cattgtcaag cttcaccaca tacaacaaca cagcgtacgc aactaacatg 6660
aatgaatacg atatacatca aagactatga tacgcagtat tgcacactgt acgagtaaga 6720
gcactagcca ctgcactcaa gtgaaaccgt tgcccgggta cgagtatgag tatgtacagt 6780
atgtttagta ttgtacttgg acagtgcttg tatcgtacat tctcaagtgt caaacataaa 6840
tatccgttgc tatatcctcg caccaccacg tagctcgcta tatccctgtg ttgaatccat 6900
ccatcttgga ttgccaattg tgcacacaga accgggcact cacttcccca tccacacttg 6960
cggccgctta ggaatcctga gcgtccttga cacagtgaac cacaccgact ttgtgcatgt 7020
acttgagggt ggaaatgatg ttgcccacaa tggtagggta gaagacgtac cgaactccgt 7080
gtcgttcgca acactctcgg acagcttgct gcacgaaggg atagtgccaa gacgacattc 7140
gaggaaagag gtgatgctcg atctggaagt tgagaccgcc agtaaagaac atggcaatgg 7200
gtccaccgta ggtggaagag gtctccacct gagctctgta ccagtcgatc tgatcggctt 7260
caacgtcctt ctcggagctc ttgaccttgc agttcttgtc ggggattcgc tccgagccat 7320
cgaagttgtg agacaagatg aaaaagaagg tgaggaaggc accggtagca gtgggcacca 7380
gaggaatggt gatgagcagg gaggttccag tgagatacca gggcaagaag gcggttcgaa 7440
agatgaagaa agctcgcata acgaatgcaa gggttcggta ccgtcgcaga aagccgttct 7500
ctcgcatggc tgtgacagac tcgggaatgg tgtcgttgtg ctgcattcgg aagatgtaga 7560
gagggttgta caccagcgaa acgccgtagg ctccaagcac gaggtacatg taccaggcct 7620
ggaatcggtg aaaccacttt cgagcagtgt tggcagcagg gtagttgtgg aacacaagga 7680
atggttctgc ggactcggca tccaggtcga gaccatgctg attggtgtag gtgtgatgtc 7740
gcatgatgtg agactgcagc cagatccatc tggacgatcc aatgacgtcg atgccgtagg 7800
caaagagagc gttgacccag ggctttttgc tgatggcacc atgagaggca tcgtgctgaa 7860
tggacaggcc gatctgcatg tgcatgaatc cagtcaagag accccacagc accattccgg 7920
tagtagccca gtgccactcg caaaaggcgg tgacagcaat gatgccaacg gttcgcagcc 7980
agaatccagg tgtggcatac cagttccgac ctttcatgac ctctcgcata gttcgcttga 8040

cgtcctgtgc aaagggagag tcgtaggtgt agacaatgtc cttggaggtt cggtcgtgct 8100
tgcctcgcac gaactgttga agcagcttcg agttctcggg cttgacgtaa gggtgcatgg 8160
agtagaacag aggagaagca tcggaggcac cagaagcgag gatcaagtcg gatccgggat 8220
ggaccttggc aagaccttcc agatcgtaga gaatgccgtc gatggcaacc aggtcgggtc 8280
gctcgagcag ctgctcggta gtaagggaga gagccatgga gagctgggtt agtttgtgta 8340
gagagtgtgt gttgctagcg actttcggat tgtgtcatta cacaaaacgc gtcgtctcga 8400
cactgatctt gtcgtggata ctcacggctc ggacatcgtc gccgacgatg acaccggact 8460
ttcgcttaag gacgtcagta acaggcattg tgtgatgtgt agtttagatt tcgaatctgt 8520
ggggaaagaa aggaaaaaag agactggcaa ccgattggga gagccactgt ttatatatac 8580
cctagacaag cccccgctt gtaagatgtt ggtcaatgta aaccagtatt aaggttggca 8640
agtgcaggag aagcaaggtg tgggtaccga gcaatggaaa tgtgcggaag gcaaaaaaat 8700
gaggccacgg cctattgtcg ggctatatc caggggcga ttgaagtaca ctaacatgac 8760
atgtgtccac agaccctcaa tctggcctga tgagccaaat ccatacgcgc tttcgcagct 8820
ctaaaggcta taacaagtca caccaccctg ctcgacctca gcgccctcac tttttgttaa 8880
gacaaactgt acacgctgtt ccagcgtttt ctgcctgcac ctggtgggac atttggtgca 8940
acctaaagtg ctcggaacct ctgtggtgtc cagatcagcg cagcagttcc gaggtagttt 9000
tgaggccctt agatgatgca atggtgtcag tcgctggatc acgagtctta atggcagtat 9060
tcgttcttat ttgtgccatt gagccccgtt atcctcgtat cttctacccc ccatcccatc 9120
cctttgttgg tgcaacccta cccatttatt gttgggtgca gcccaaccga cgtggagagc 9180
ttggcttggc catataaaaa ggccccccc tagtggcaat ggcagaaagt cagctgtgag 9240
ttgttgaatt tgtcatctag gcggcctggc cgtcttctcc ggggcaattg gggctgtttt 9300
ttgggacaca aatacgccgc caacccggtc tctcctgaat tctgcagatg ggctgcagga 9360
attccgtcgt cgcctgagtc gactccaact tttcacactg agcgtaaaat gtggagaaga 9420
aatcggcact aaaaagtcag gtagactgga aaatgcgcca tgaaatgaat atctcttgct 9480
acagtaatgc ccagcatcga ggggtattgt gtcaccaaca ctatagtggc agctgaagcg 9540
ctcgtgattg tagtatgagt ctttattggt gatgggaaga gttcactcaa tattctcgtt 9600
actgccaaaa caccacggta atcggccaga caccatggat gtagatcacc aagcctgtga 9660
atgttattcg agctaaaatg cacatggttg gtgaaaggag tagttgctgt cgaattccgt 9720
cgtcgcctga gtcatcattt atttaccagt tggccacaaa cccttgacga tctcgtatgt 9780
cccctccgac atactcccgg ccggctgggg tacgttcgat agcgctatcg gcatcgacaa 9840
ggtttgggtc cctagccgat accgcactac ctgagtcaca atcttcggag gtttagtctt 9900
ccacatagca cgggcaaaag tgcgtatata tacaagagcg tttgccagcc acagattttc 9960

actccacaca ccacatcaca catacaacca cacacatcca caatggaacc cgaaactaag 10020
aagaccaaga ctgactccaa gaagattgtt cttctcggcg gcgacttctg tggccccgag 10080
gtgattgccg aggccgtcaa ggtgctcaag tctgttgctg aggcctccgg caccgagttt 10140
gtgtttgagg accgactcat tggaggagct gccattgaga aggagggcga gcccatcacc 10200
gacgctactc tcgacatctg ccgaaaggct gactctatta tgctcggtgc tgtcggaggc 10260
gctgccaaca ccgtatggac cactcccgac ggacgaaccg acgtgcgacc cgagcagggt 10320
ctcctcaagc tgcgaaagga cctgaacctg tacgccaacc tgcgaccctg ccagctgctg 10380
tcgcccaagc tcgccgatct ctcccccatc cgaaacgttg agggcaccga cttcatcatt 10440
gtccgagagc tcgtcggagg tatctacttt ggagagcgaa aggaggatga cggatctggc 10500
gtcgcttccg acaccgagac ctactccgtt cctgaggttg agcgaattgc ccgaatggcc 10560
gccttcctgg cccttcagca caaccccccct cttcccgtgt ggtctcttga caaggccaac 10620
gtgctggcct cctctcgact ttggcgaaag actgtcactc gagtcctcaa ggacgaattc 10680
ccccagctcg agctcaacca ccagctgatc gactcggccg ccatgatcct catcaagcag 10740
ccctccaaga tgaatggtat catcatcacc accaacatgt ttggcgatat catctccgac 10800
gaggcctccg tcatccccgg ttctctgggt ctgctgccct ccgcctctct ggcttctctg 10860
cccgacacca acgaggcgtt cggtctgtac gagccctgtc acggatctgc cccccgatctc 10920
ggcaagcaga aggtcaaccc cattgccacc attctgtctg ccgccatgat gctcaagttc 10980
tctcttaaca tgaagcccgc cggtgacgct gttgaggctg ccgtcaagga gtccgtcgag 11040
gctggtatca ctaccgccga tatcggaggc tcttcctcca cctccgaggt cggagacttg 11100
ttgccaacaa ggtcaaggag ctgctcaaga aggagtaagt cgtttctacg acgcattgat 11160
ggaaggagca aactgacgcg cctgcgggtt ggtctaccgg cagggtccgc tagtgtataa 11220
gactctataa aaagggccct gccctgctaa tgaaatgatg atttataatt taccggtgta 11280
gcaaccttga ctagaagaag cagattgggt gtgtttgtag tggaggacag tggtacgttt 11340
tggaaacagt cttcttgaaa gtgtcttgtc tacagtatat tcactcataa cctcaatagc 11400
caagggtgta gtcggtttat taaaggaagg gagttgtggc tgatgtggat agatatcttt 11460
aagctggcga ctgcacccaa cgagtgtggt ggtagcttgt ttaaacagag tgtgaaagac 11520
tcactatggt ccgggcttat ctcgaccaat agccaaagtc tggagtttct gagagaaaaa 11580
ggcaagatac gtatgtaaca aagcgacgca tggtacaata ataccggagg catgtatcat 11640
agagagttag tggttcgatg atggcactgg tgcctggtat gactttatac ggctgactac 11700
atatttgtcc tcagacatac aattacagtc aagcacttac ccttggacat ctgtaggtac 11760
cccccggcca agacgatctc agcgtgtcgt atgtcggatt ggcgtagctc cctcgctcgt 11820
caattggctc ccatctactt tcttctgctt ggctacaccc agcatgtctg ctatggctcg 11880
ttttcgtgcc ttatctatcc tcccagtatt accaactcta aatgacatga tgtgattggg 11940

tctacacttt catatcagag ataaggagta gcacagttgc ataaaaagcc caactctaat 12000
cagcttcttc ctttcttgta attagtacaa aggtgattag cgaaatctgg aagcttagtt 12060
ggccctaaaa aaatcaaaaa aagcaaaaaa cgaaaaacga aaaaccacag ttttgagaac 12120
agggaggtaa cgaaggatcg tatatatata tatatatata tatacccacg gatcccgaga 12180
ccggcctttg attcttccct acaaccaacc attctcacca ccctaattca caaccatggc 12240
caccatctcc ctgactaccg agcagctcct ggaacacccc gagctcgttg ccatcgacgg 12300
agtcctgtac gatctcttcg gtctggccaa ggtccatgcc ggaggcaacc tcatcgaagc 12360
tgccggtgca tccgacggaa ccgctctgtt ctactccatg catcctggag tcaagccaga 12420
gaactcgaag cttctgcagc aatttgcccg aggcaagcac gaacgaagct ccaaggatcc 12480
cgtgtacacc ttcgactctc cctttgctca ggacgtcaag cagtccgttc gagaggtcat 12540
gaagggtcga aactggtacg ccactcctgg cttctggctg agaaccgcac tcatcatcgc 12600
ttgtactgcc attggcgagt ggtactggat cacaaccgga gcagtgatgt ggggtatctt 12660
tactggatac ttccactcgc agattggctt ggccattcaa cacgatgctt ctcacggagc 12720
catcagcaaa aagccctggg tcaacgcctt tttcgcttat ggcatcgacg ccattggttc 12780
ctctcgttgg atctggctgc agtcccacat tatgcgacat cacacttaca ccaaccagca 12840
tggcctcgac ctggatgctg cctcggcaga gccgttcatc ttgttccact cctatcctgc 12900
taccaacgcc tctcgaaagt ggtaccaccg atttcaggcg tggtacatgt acatcgttct 12960
gggaatgtat ggtgtctcga tggtgtacaa tcccatgtac ctcttcacaa tgcagcacaa 13020
cgacaccatt cccgaggcca cttctctcag accaggcagc tttttcaatc ggcagcgagc 13080
tttcgccgtt tcccttcgac tgctcttcat cttccgaaac gcctttcttc cctggtacat 13140
tgctggtgcc tctcctctgc tcaccattct tctggtgccc acggtcacag gcatcttcct 13200
cacctttgtg ttcgttctgt cccataactt cgagggagcc gaacggaccc cagagaagaa 13260
ctgcaaggcc aaacgagcta aggaaggcaa ggaggtcaga gacgtggaag aggatcgagt 13320
cgactggtac cgagcacagg ccgagactgc tgccacctac ggtggcagcg tgggaatgat 13380
gcttacaggc ggtctcaacc tgcagatcga gcatcacttg tttccccgaa tgtcctcttg 13440
gcactatccc ttcattcaag acaccgttcg ggagtgttgc aagcgacatg gcgtccgtta 13500
cacatactat cctaccattc tcgagaacat catgtccact cttcgataca tgcagaaggt 13560
gggtgttgct cacaccattc aggatgccca ggagttctaa gcggccgcat gtacatacaa 13620
gattatttat agaaatgaat cgcgatcgaa caaagagtac gagtgtacga gtaggggatg 13680
atgataaaag tggaagaagt tccgcatctt tggatttatc aacgtgtagg acgatacttc 13740
ctgtaaaaat gcaatgtctt taccataggt tctgctgtag atgttattaa ctaccattaa 13800
catgtctact tgtacagttg cagaccagtt ggagtataga atggtacact taccaaaaag 13860

```
tgttgatggt tgtaactacg atatataaaa ctgttgacgg gatccccgct gatatgccta    13920

aggaacaatc aaagaggaag atattaattc agaatgctag tatacagtta gggat         13975
```

<210> 35
<211> 1350
<212> DNA
<213> Euglena gracilis

<220>
<221> CDS
<222> (1)..(1350)
<223> synthetic mutant delta-5 desaturase (codon-optimized for Yarrowia lipolytica)

<400> 35

```
atg gct ctc tcc ctt act acc gag cag ctg ctc gag cga ccc gac ctg        48
Met Ala Leu Ser Leu Thr Thr Glu Gln Leu Leu Glu Arg Pro Asp Leu
1               5                   10                  15

gtt gcc atc gac ggc att ctc tac gat ctg gaa ggt ctt gcc aag gtc        96
Val Ala Ile Asp Gly Ile Leu Tyr Asp Leu Glu Gly Leu Ala Lys Val
            20                  25                  30

cat ccc gga tcc gac ttg atc ctc gct tct ggt gcc tcc gat gct tct       144
His Pro Gly Ser Asp Leu Ile Leu Ala Ser Gly Ala Ser Asp Ala Ser
        35                  40                  45

cct ctg ttc tac tcc atg cac cct tac gtc aag ccc gag aac tcg aag       192
Pro Leu Phe Tyr Ser Met His Pro Tyr Val Lys Pro Glu Asn Ser Lys
    50                  55                  60

ctg ctt caa cag ttc gtg cga ggc aag cac gac cga acc tcc aag gac       240
Leu Leu Gln Gln Phe Val Arg Gly Lys His Asp Arg Thr Ser Lys Asp
65                  70                  75                  80

att gtc tac acc tac gac tct ccc ttt gca cag gac gtc aag cga act       288
Ile Val Tyr Thr Tyr Asp Ser Pro Phe Ala Gln Asp Val Lys Arg Thr
                85                  90                  95

atg cga gag gtc atg aaa ggt cgg aac tgg tat gcc aca cct gga ttc       336
Met Arg Glu Val Met Lys Gly Arg Asn Trp Tyr Ala Thr Pro Gly Phe
            100                 105                 110

tgg ctg cga acc gtt ggc atc att gct gtc acc gcc ttt tgc gag tgg       384
Trp Leu Arg Thr Val Gly Ile Ile Ala Val Thr Ala Phe Cys Glu Trp
        115                 120                 125

cac tgg gct act acc gga atg gtg ctg tgg ggt ctc ttg act gga ttc       432
His Trp Ala Thr Thr Gly Met Val Leu Trp Gly Leu Leu Thr Gly Phe
    130                 135                 140

atg cac atg cag atc ggc ctg tcc att cag cac gat gcc tct cat ggt       480
Met His Met Gln Ile Gly Leu Ser Ile Gln His Asp Ala Ser His Gly
145                 150                 155                 160

gcc atc agc aaa aag ccc tgg gtc aac gct ctc ttt gcc tac ggc atc       528
Ala Ile Ser Lys Lys Pro Trp Val Asn Ala Leu Phe Ala Tyr Gly Ile
                165                 170                 175

gac gtc att gga tcg tcc aga tgg atc tgg ctg cag tct cac atc atg       576
Asp Val Ile Gly Ser Ser Arg Trp Ile Trp Leu Gln Ser His Ile Met
            180                 185                 190
```

```
cga cat cac acc tac acc aat cag cat ggt ctc gac ctg gat gcc gag      624
Arg His His Thr Tyr Thr Asn Gln His Gly Leu Asp Leu Asp Ala Glu
        195                 200                 205

tcc gca gaa cca ttc ctt gtg ttc cac aac tac cct gct gcc aac act      672
Ser Ala Glu Pro Phe Leu Val Phe His Asn Tyr Pro Ala Ala Asn Thr
    210                 215                 220

gct cga aag tgg ttt cac cga ttc cag gcc tgg tac atg tac ctc gtg      720
Ala Arg Lys Trp Phe His Arg Phe Gln Ala Trp Tyr Met Tyr Leu Val
225                 230                 235                 240

ctt gga gcc tac ggc gtt tcg ctg gtg tac aac cct ctc tac atc ttc      768
Leu Gly Ala Tyr Gly Val Ser Leu Val Tyr Asn Pro Leu Tyr Ile Phe
                245                 250                 255

cga atg cag cac aac gac acc att ccc gag tct gtc aca gcc atg cga      816
Arg Met Gln His Asn Asp Thr Ile Pro Glu Ser Val Thr Ala Met Arg
            260                 265                 270

gag aac ggc ttt ctg cga cgg tac cga acc ctt gca ttc gtt atg cga      864
Glu Asn Gly Phe Leu Arg Arg Tyr Arg Thr Leu Ala Phe Val Met Arg
        275                 280                 285

gct ttc ttc atc ttt cga acc gcc ttc ttg ccc tgg tat ctc act gga      912
Ala Phe Phe Ile Phe Arg Thr Ala Phe Leu Pro Trp Tyr Leu Thr Gly
    290                 295                 300

acc tcc ctg ctc atc acc att cct ctg gtg ccc act gct acc ggt gcc      960
Thr Ser Leu Leu Ile Thr Ile Pro Leu Val Pro Thr Ala Thr Gly Ala
305                 310                 315                 320

ttc ctc acc ttc ttt ttc atc ttg tct cac aac ttc gat ggc tcg gag     1008
Phe Leu Thr Phe Phe Phe Ile Leu Ser His Asn Phe Asp Gly Ser Glu
                325                 330                 335

cga atc ccc gac aag aac tgc aag gtc aag agc tcc gag aag gac gtt     1056
Arg Ile Pro Asp Lys Asn Cys Lys Val Lys Ser Ser Glu Lys Asp Val
            340                 345                 350

gaa gcc gat cag atc gac tgg tac aga gct cag gtg gag acc tct tcc     1104
Glu Ala Asp Gln Ile Asp Trp Tyr Arg Ala Gln Val Glu Thr Ser Ser
        355                 360                 365

acc tac ggt gga ccc att gcc atg ttc ttt act ggc ggt ctc aac ttc     1152
Thr Tyr Gly Gly Pro Ile Ala Met Phe Phe Thr Gly Gly Leu Asn Phe
    370                 375                 380

cag atc gag cat cac ctc ttt cct cga atg tcg tct tgg cac tat ccc     1200
Gln Ile Glu His His Leu Phe Pro Arg Met Ser Ser Trp His Tyr Pro
385                 390                 395                 400

ttc gtg cag caa gct gtc cga gag tgt tgc gaa cga cac gga gtt cgg     1248
Phe Val Gln Gln Ala Val Arg Glu Cys Cys Glu Arg His Gly Val Arg
                405                 410                 415

tac gtc ttc tac cct acc att gtg ggc aac atc att tcc acc ctc aag     1296
Tyr Val Phe Tyr Pro Thr Ile Val Gly Asn Ile Ile Ser Thr Leu Lys
            420                 425                 430

tac atg cac aaa gtc ggt gtg gtt cac tgt gtc aag gac gct cag gat     1344
Tyr Met His Lys Val Gly Val Val His Cys Val Lys Asp Ala Gln Asp
        435                 440                 445
```

```
tcc taa                                                             1350
Ser
```

<210> 36
<211> 449
<212> PRT
<213> Euglena gracilis

<400> 36

```
Met Ala Leu Ser Leu Thr Thr Glu Gln Leu Leu Glu Arg Pro Asp Leu
1               5                   10                  15

Val Ala Ile Asp Gly Ile Leu Tyr Asp Leu Glu Gly Leu Ala Lys Val
            20                  25                  30

His Pro Gly Ser Asp Leu Ile Leu Ala Ser Gly Ala Ser Asp Ala Ser
        35                  40                  45

Pro Leu Phe Tyr Ser Met His Pro Tyr Val Lys Pro Glu Asn Ser Lys
    50                  55                  60

Leu Leu Gln Gln Phe Val Arg Gly Lys His Asp Arg Thr Ser Lys Asp
65                  70                  75                  80

Ile Val Tyr Thr Tyr Asp Ser Pro Phe Ala Gln Asp Val Lys Arg Thr
                85                  90                  95

Met Arg Glu Val Met Lys Gly Arg Asn Trp Tyr Ala Thr Pro Gly Phe
            100                 105                 110

Trp Leu Arg Thr Val Gly Ile Ile Ala Val Thr Ala Phe Cys Glu Trp
        115                 120                 125

His Trp Ala Thr Thr Gly Met Val Leu Trp Gly Leu Leu Thr Gly Phe
    130                 135                 140

Met His Met Gln Ile Gly Leu Ser Ile Gln His Asp Ala Ser His Gly
145                 150                 155                 160

Ala Ile Ser Lys Lys Pro Trp Val Asn Ala Leu Phe Ala Tyr Gly Ile
                165                 170                 175

Asp Val Ile Gly Ser Ser Arg Trp Ile Trp Leu Gln Ser His Ile Met
            180                 185                 190

Arg His His Thr Tyr Thr Asn Gln His Gly Leu Asp Leu Asp Ala Glu
        195                 200                 205

Ser Ala Glu Pro Phe Leu Val Phe His Asn Tyr Pro Ala Ala Asn Thr
```

210 215 220

Ala Arg Lys Trp Phe His Arg Phe Gln Ala Trp Tyr Met Tyr Leu Val
225 230 235 240

Leu Gly Ala Tyr Gly Val Ser Leu Val Tyr Asn Pro Leu Tyr Ile Phe
245 250 255

Arg Met Gln His Asn Asp Thr Ile Pro Glu Ser Val Thr Ala Met Arg
260 265 270

Glu Asn Gly Phe Leu Arg Arg Tyr Arg Thr Leu Ala Phe Val Met Arg
275 280 285

Ala Phe Phe Ile Phe Arg Thr Ala Phe Leu Pro Trp Tyr Leu Thr Gly
290 295 300

Thr Ser Leu Leu Ile Thr Ile Pro Leu Val Pro Thr Ala Thr Gly Ala
305 310 315 320

Phe Leu Thr Phe Phe Phe Ile Leu Ser His Asn Phe Asp Gly Ser Glu
325 330 335

Arg Ile Pro Asp Lys Asn Cys Lys Val Lys Ser Ser Glu Lys Asp Val
340 345 350

Glu Ala Asp Gln Ile Asp Trp Tyr Arg Ala Gln Val Glu Thr Ser Ser
355 360 365

Thr Tyr Gly Gly Pro Ile Ala Met Phe Phe Thr Gly Gly Leu Asn Phe
370 375 380

Gln Ile Glu His His Leu Phe Pro Arg Met Ser Ser Trp His Tyr Pro
385 390 395 400

Phe Val Gln Gln Ala Val Arg Glu Cys Cys Glu Arg His Gly Val Arg
405 410 415

Tyr Val Phe Tyr Pro Thr Ile Val Gly Asn Ile Ile Ser Thr Leu Lys
420 425 430

Tyr Met His Lys Val Gly Val Val His Cys Val Lys Asp Ala Gln Asp
435 440 445

Ser

<210> 37
<211> 1365
<212> DNA
<213> Euglena anabaena UTEX 373

<220>
<221> CDS
<222> (1)..(1365)
<223> synthetic mutant delta-5 desaturase (codon-optimized for Yarrowia lipolytica)

<400> 37

```
atg gcc acc atc tcc ctg act acc gag cag ctc ctg gaa cac ccc gag       48
Met Ala Thr Ile Ser Leu Thr Thr Glu Gln Leu Leu Glu His Pro Glu
1               5                   10                  15

ctc gtt gcc atc gac gga gtc ctg tac gat ctc ttc ggt ctg gcc aag       96
Leu Val Ala Ile Asp Gly Val Leu Tyr Asp Leu Phe Gly Leu Ala Lys
            20                  25                  30

gtc cat gcc gga ggc aac ctc atc gaa gct gcc ggt gca tcc gac gga      144
Val His Ala Gly Gly Asn Leu Ile Glu Ala Ala Gly Ala Ser Asp Gly
        35                  40                  45

acc gct ctg ttc tac tcc atg cat cct gga gtc aag cca gag aac tcg      192
Thr Ala Leu Phe Tyr Ser Met His Pro Gly Val Lys Pro Glu Asn Ser
    50                  55                  60

aag ctt ctg cag caa ttt gcc cga ggc aag cac gaa cga agc tcc aag      240
Lys Leu Leu Gln Gln Phe Ala Arg Gly Lys His Glu Arg Ser Ser Lys
65                  70                  75                  80

gat ccc gtg tac acc ttc gac tct ccc ttt gct cag gac gtc aag cag      288
Asp Pro Val Tyr Thr Phe Asp Ser Pro Phe Ala Gln Asp Val Lys Gln
                85                  90                  95

tcc gtt cga gag gtc atg aag ggt cga aac tgg tac gcc act cct ggc      336
Ser Val Arg Glu Val Met Lys Gly Arg Asn Trp Tyr Ala Thr Pro Gly
            100                 105                 110

ttc tgg ctg aga acc gca ctc atc atc gct tgt act gcc att ggc gag      384
Phe Trp Leu Arg Thr Ala Leu Ile Ile Ala Cys Thr Ala Ile Gly Glu
        115                 120                 125

tgg tac tgg atc aca acc gga gca gtg atg tgg ggt atc ttt act gga      432
Trp Tyr Trp Ile Thr Thr Gly Ala Val Met Trp Gly Ile Phe Thr Gly
    130                 135                 140

tac ttc cac tcg cag att ggc ttg gcc att caa cac gat gct tct cac      480
Tyr Phe His Ser Gln Ile Gly Leu Ala Ile Gln His Asp Ala Ser His
145                 150                 155                 160

gga gcc atc agc aaa aag ccc tgg gtc aac gcc ttt ttc gct tat ggc      528
Gly Ala Ile Ser Lys Lys Pro Trp Val Asn Ala Phe Phe Ala Tyr Gly
                165                 170                 175

atc gac gcc att ggt tcc tct cgt tgg atc tgg ctg cag tcc cac att      576
Ile Asp Ala Ile Gly Ser Ser Arg Trp Ile Trp Leu Gln Ser His Ile
            180                 185                 190

atg cga cat cac act tac acc aac cag cat ggc ctc gac ctg gat gct      624
Met Arg His His Thr Tyr Thr Asn Gln His Gly Leu Asp Leu Asp Ala
        195                 200                 205

gcc tcg gca gag ccg ttc atc ttg ttc cac tcc tat cct gct acc aac      672
Ala Ser Ala Glu Pro Phe Ile Leu Phe His Ser Tyr Pro Ala Thr Asn
```

```
        210                 215                 220

gcc tct cga aag tgg tac cac cga ttt cag gcg tgg tac atg tac atc       720
Ala Ser Arg Lys Trp Tyr His Arg Phe Gln Ala Trp Tyr Met Tyr Ile
225                 230                 235                 240

gtt ctg gga atg tat ggt gtc tcg atg gtg tac aat ccc atg tac ctc       768
Val Leu Gly Met Tyr Gly Val Ser Met Val Tyr Asn Pro Met Tyr Leu
                245                 250                 255

ttc aca atg cag cac aac gac acc att ccc gag gcc act tct ctc aga       816
Phe Thr Met Gln His Asn Asp Thr Ile Pro Glu Ala Thr Ser Leu Arg
            260                 265                 270

cca ggc agc ttt ttc aat cgg cag cga gct ttc gcc gtt tcc ctt cga       864
Pro Gly Ser Phe Phe Asn Arg Gln Arg Ala Phe Ala Val Ser Leu Arg
        275                 280                 285

ctg ctc ttc atc ttc cga aac gcc ttt ctt ccc tgg tac att gct ggt       912
Leu Leu Phe Ile Phe Arg Asn Ala Phe Leu Pro Trp Tyr Ile Ala Gly
    290                 295                 300

gcc tct cct ctg ctc acc att ctt ctg gtg ccc acg gtc aca ggc atc       960
Ala Ser Pro Leu Leu Thr Ile Leu Leu Val Pro Thr Val Thr Gly Ile
305                 310                 315                 320

ttc ctc acc ttt gtg ttc gtt ctg tcc cat aac ttc gag gga gcc gaa      1008
Phe Leu Thr Phe Val Phe Val Leu Ser His Asn Phe Glu Gly Ala Glu
                325                 330                 335

cgg acc cca gag aag aac tgc aag gcc aaa cga gct aag gaa ggc aag      1056
Arg Thr Pro Glu Lys Asn Cys Lys Ala Lys Arg Ala Lys Glu Gly Lys
            340                 345                 350

gag gtc aga gac gtg gaa gag gat cga gtc gac tgg tac cga gca cag      1104
Glu Val Arg Asp Val Glu Glu Asp Arg Val Asp Trp Tyr Arg Ala Gln
        355                 360                 365

gcc gag act gct gcc acc tac ggt ggc agc gtg gga atg atg ctt aca      1152
Ala Glu Thr Ala Ala Thr Tyr Gly Gly Ser Val Gly Met Met Leu Thr
    370                 375                 380

ggc ggt ctc aac ctg cag atc gag cat cac ttg ttt ccc cga atg tcc      1200
Gly Gly Leu Asn Leu Gln Ile Glu His His Leu Phe Pro Arg Met Ser
385                 390                 395                 400

tct tgg cac tat ccc ttc att caa gac acc gtt cgg gag tgt tgc aag      1248
Ser Trp His Tyr Pro Phe Ile Gln Asp Thr Val Arg Glu Cys Cys Lys
                405                 410                 415

cga cat ggc gtc cgt tac aca tac tat cct acc att ctc gag aac atc      1296
Arg His Gly Val Arg Tyr Thr Tyr Tyr Pro Thr Ile Leu Glu Asn Ile
            420                 425                 430

atg tcc act ctt cga tac atg cag aag gtg ggt gtt gct cac acc att      1344
Met Ser Thr Leu Arg Tyr Met Gln Lys Val Gly Val Ala His Thr Ile
        435                 440                 445

cag gat gcc cag gag ttc taa                                          1365
Gln Asp Ala Gln Glu Phe
    450
```

<210> 38
<211> 454
<212> PRT
<213> Euglena anabaena UTEX 373

<400> 38

```
Met Ala Thr Ile Ser Leu Thr Thr Glu Gln Leu Leu Glu His Pro Glu
1               5                   10                  15

Leu Val Ala Ile Asp Gly Val Leu Tyr Asp Leu Phe Gly Leu Ala Lys
            20                  25                  30

Val His Ala Gly Gly Asn Leu Ile Glu Ala Ala Gly Ala Ser Asp Gly
        35                  40                  45

Thr Ala Leu Phe Tyr Ser Met His Pro Gly Val Lys Pro Glu Asn Ser
    50                  55                  60

Lys Leu Leu Gln Gln Phe Ala Arg Gly Lys His Glu Arg Ser Ser Lys
65                  70                  75                  80

Asp Pro Val Tyr Thr Phe Asp Ser Pro Phe Ala Gln Asp Val Lys Gln
                85                  90                  95

Ser Val Arg Glu Val Met Lys Gly Arg Asn Trp Tyr Ala Thr Pro Gly
            100                 105                 110

Phe Trp Leu Arg Thr Ala Leu Ile Ile Ala Cys Thr Ala Ile Gly Glu
        115                 120                 125

Trp Tyr Trp Ile Thr Thr Gly Ala Val Met Trp Gly Ile Phe Thr Gly
    130                 135                 140

Tyr Phe His Ser Gln Ile Gly Leu Ala Ile Gln His Asp Ala Ser His
145                 150                 155                 160

Gly Ala Ile Ser Lys Lys Pro Trp Val Asn Ala Phe Phe Ala Tyr Gly
                165                 170                 175

Ile Asp Ala Ile Gly Ser Ser Arg Trp Ile Trp Leu Gln Ser His Ile
            180                 185                 190

Met Arg His His Thr Tyr Thr Asn Gln His Gly Leu Asp Leu Asp Ala
        195                 200                 205

Ala Ser Ala Glu Pro Phe Ile Leu Phe His Ser Tyr Pro Ala Thr Asn
    210                 215                 220

Ala Ser Arg Lys Trp Tyr His Arg Phe Gln Ala Trp Tyr Met Tyr Ile
225                 230                 235                 240
```

```
Val Leu Gly Met Tyr Gly Val Ser Met Val Tyr Asn Pro Met Tyr Leu
                245                 250                 255

Phe Thr Met Gln His Asn Asp Thr Ile Pro Glu Ala Thr Ser Leu Arg
            260                 265                 270

Pro Gly Ser Phe Phe Asn Arg Gln Arg Ala Phe Ala Val Ser Leu Arg
        275                 280                 285

Leu Leu Phe Ile Phe Arg Asn Ala Phe Leu Pro Trp Tyr Ile Ala Gly
    290                 295                 300

Ala Ser Pro Leu Leu Thr Ile Leu Leu Val Pro Thr Val Thr Gly Ile
305                 310                 315                 320

Phe Leu Thr Phe Val Phe Val Leu Ser His Asn Phe Glu Gly Ala Glu
                325                 330                 335

Arg Thr Pro Glu Lys Asn Cys Lys Ala Lys Arg Ala Lys Glu Gly Lys
            340                 345                 350

Glu Val Arg Asp Val Glu Glu Asp Arg Val Asp Trp Tyr Arg Ala Gln
        355                 360                 365

Ala Glu Thr Ala Ala Thr Tyr Gly Gly Ser Val Gly Met Met Leu Thr
    370                 375                 380

Gly Gly Leu Asn Leu Gln Ile Glu His His Leu Phe Pro Arg Met Ser
385                 390                 395                 400

Ser Trp His Tyr Pro Phe Ile Gln Asp Thr Val Arg Glu Cys Cys Lys
                405                 410                 415

Arg His Gly Val Arg Tyr Thr Tyr Tyr Pro Thr Ile Leu Glu Asn Ile
            420                 425                 430

Met Ser Thr Leu Arg Tyr Met Gln Lys Val Gly Val Ala His Thr Ile
        435                 440                 445

Gln Asp Ala Gln Glu Phe
    450
```

<210> 39
<211> 13066
<212> DNA
<213> Artificial Sequence

<220>

<223> Plasmid pZP3-Pa777U

<400> 39

tctcggtcta ttcttttgat ttataaggga ttttgccgat ttcggcctat tggttaaaaa 60
atgagctgat ttaacaaaaa tttaacgcga attttaacaa aatattaacg cttacaattt 120
cctgatgcgg tattttctcc ttacgcatct gtgcggtatt tcacaccgca tcaggtggca 180
cttttcgggg aaatgtgcgc ggaaccccta tttgtttatt tttctaaata cattcaaata 240
tgtatccgct catgagacaa taaccctgat aaatgcttca ataatattga aaaaggaaga 300
gtatgagtat tcaacatttc cgtgtcgccc ttattccctt ttttgcggca ttttgccttc 360
ctgtttttgc tcacccagaa acgctggtga aagtaaaaga tgctgaagat cagttgggtg 420
cacgagtggg ttacatcgaa ctggatctca acagcggtaa gatccttgag agttttcgcc 480
ccgaagaacg ttttccaatg atgagcactt ttaaagttct gctatgtggc gcggtattat 540
cccgtattga cgccgggcaa gagcaactcg gtcgccgcat acactattct cagaatgact 600
tggttgagta ctcaccagtc acagaaaagc atcttacgga tggcatgaca gtaagagaat 660
tatgcagtgc tgccataacc atgagtgata acactgcggc caacttactt ctgacaacga 720
tcggaggacc gaaggagcta accgcttttt tgcacaacat gggggatcat gtaactcgcc 780
ttgatcgttg ggaaccggag ctgaatgaag ccataccaaa cgacgagcgt gacaccacga 840
tgcctgtagc aatggcaaca acgttgcgca aactattaac tggcgaacta cttactctag 900
cttcccggca acaattaata gactggatgg aggcggataa agttgcagga ccacttctgc 960
gctcggccct tccggctggc tggtttattg ctgataaatc tggagccggt gagcgtgggt 1020
ctcgcggtat cattgcagca ctggggccag atggtaagcc ctcccgtatc gtagttatct 1080
acacgacggg gagtcaggca actatggatg aacgaaatag acagatcgct gagataggtg 1140
cctcactgat taagcattgg taactgtcag accaagttta ctcatatata ctttagattg 1200
atttaaaact tcatttttaa tttaaaagga tctaggtgaa gatccttttt gataatctca 1260
tgaccaaaat cccttaacgt gagttttcgt tccactgagc gtcagacccc gtagaaaaga 1320
tcaaaggatc ttcttgagat cctttttttc tgcgcgtaat ctgctgcttg caaacaaaaa 1380
aaccaccgct accagcggtg gtttgtttgc cggatcaaga gctaccaact ctttttccga 1440
aggtaactgg cttcagcaga gcgcagatac caaatactgt tcttctagtg tagccgtagt 1500
taggccacca cttcaagaac tctgtagcac cgcctacata cctcgctctg ctaatcctgt 1560
taccagtggc tgctgccagt ggcgataagt cgtgtcttac cgggttggac tcaagacgat 1620
agttaccgga taaggcgcag cggtcgggct gaacgggggg ttcgtgcaca cagcccagct 1680
tggagcgaac gacctacacc gaactgagat acctacagcg tgagctatga gaaagcgcca 1740
cgcttcccga agggagaaag gcggacaggt atccggtaag cggcagggtc ggaacaggag 1800
agcgcacgag ggagcttcca gggggaaacg cctggtatct ttatagtcct gtcgggtttc 1860
gccacctctg acttgagcgt cgatttttgt gatgctcgtc aggggggcgg agcctatgga 1920
aaaacgccag caacgcggcc tttttacggt tcctggcctt ttgctggcct tttgctcaca 1980

tgttctttcc tgcgttatcc cctgattctg tggataaccg tattaccgcc tttgagtgag 2040
ctgataccgc tcgccgcagc cgaacgaccg agcgcagcga gtcagtgagc gaggaagcgg 2100
aagagcgccc aatacgcaaa ccgcctctcc ccgcgcgttg gccgattcat taatgcagct 2160
ggcgcgccac caatcacaat tctgaaaagc acatcttgat ctcctcattg cggggagtcc 2220
aacggtggtc ttattccccc gaatttcccg ctcaatctcg ttccagaccg acccggacac 2280
agtgcttaac gccgttccga aactctaccg cagatatgct ccaacggact gggctgcata 2340
gatgtgatcc tcggcttgga gaaatggata aaagccggcc aaaaaaaaag cggaaaaaag 2400
cggaaaaaaa gagaaaaaaa atcgcaaaat ttgaaaaata ggggggaaag acgcaaaaac 2460
gcaaggaggg gggagtatat gacactgata agcaagctca caacggttcc tcttattttt 2520
ttcctcatct tctgcctagg ttcccaaaat cccagatgct tctctccagt gccaaaagta 2580
agtaccccac aggttttcgg ccgaaaattc cacgtgcagc aacgtcgtgt ggggtgttaa 2640
aatgtggggg gggggaacca ggacaagagg ctcttgtggg agccgaatga gagcacaaag 2700
cgggcgggtg tgataagggc atttttgccc attttccctt ctcctgtctc tccgacggtg 2760
atggcgttgt gcgtcctcta tttcttttta tttcttttttg ttttatttct ctgactaccg 2820
atttggtttg atttcctcaa ccccacacaa ataagctcgg gccgaggaat atatatatac 2880
acggacacag tcgccctgtg gacaacacgt cactacctct acgatacaca ccgtacgttg 2940
tgtggaagct tgtgagcgga taacaatttc acacaggaaa cagctatgac catgattacg 3000
ccaagctcga aattaaccct cactaaaggg aacaaaagct ggagctccac cgcggacaca 3060
atatctggtc aaatttcagt ttcgttacat ttaaattcct tcacttcaag ttcattcttc 3120
atctgcttct gttttacttt gacaggcaaa tgaagacatg gtacgacttg atggaggcca 3180
agaacgccat ttcaccccga gacaccgaag tgcctgaaat cctggctgcc cccattgata 3240
acatcggaaa ctacggtatt ccggaaagtg tatatagaac ctttccccag cttgtgtctg 3300
tggatatgga tggtgtaatc ccctttgagt actcgtcttg gcttctctcc gagcagtatg 3360
aggctctcta atctagcgca tttaatatct caatgtattt atatatttat cttctcatgc 3420
ggccgcttag ttggctttgg tcttggcagc cttggcctcc ttgagggtaa acatcttggc 3480
atccttgtcg accacgccgt acttggcgta cataagacca attcggatga aggtgggaat 3540
gatgggagaa gccgactttc gcaccagttc gggaaaggcc tgagcgaagg cagcagtggc 3600
ctcgttgagc ttgtagtgag gaatgatggg aaacagatgg tggatctgat gtgtaccaat 3660
gttgtgggac aggttgtcga tgagggctcc gtagcttcgg tccacagagg acaagttgcc 3720
cttgacatag gtccactccg aatcggcgta ccagggagtt tcctcgtcgt tgtgatggag 3780
gaaggtagtg acaaccagca tggtggcgaa tccaaagaga ggtgcgaagt aatacagagc 3840
catggtcttg aggccgtaga cgtaggtaag gtaggcgtac agaccagcaa aggccacgag 3900

agagccgagg gaaatgatga cggcagacat tcttcgcagg tagagaggct cccagggatt 3960
gaagtggttg acctttcggg gaggaaatcc agcaacgagg taggcaaacc aagccgaacc 4020
aagggagatg accatgtgtc gggacagggg atgagagtcg gcttctcgct gagggtagaa 4080
gatctcatcc ttgtcgatgt tgccggtgtt cttgtgatgg tgtcgatggc tgatcttcca 4140
cgactcgtag ggagtcagaa tgatggagtg aatgagtgtg ccaacagaga agttgagcag 4200
gtgggatcgc gagaaggcac catgtccaca gtcgtgaccg atggtaaaga atccccagaa 4260
cacgataccc tggagcagaa tgtagccagt gcaaaggacg gcatcgagca gtgcaaactc 4320
ctgcacgata gcaagggctc gagcatagta cagtccgaga gcaagggaac cggcaatgcc 4380
cagagctcgc acggtatagt agagggacca gggaacagag gcttcgaagc agtgggcagg 4440
cagggatcgc ttgatctcgg tgagagtagg gaactcgtag ggagcggcaa cggtagagga 4500
agccatggtt gtgaattagg gtggtgagaa tggttggttg tagggaagaa tcaaaggccg 4560
gtctcgggat ccgtgggtat atatatatat atatatatat acgatccttc gttacctccc 4620
tgttctcaaa actgtggttt ttcgttttc gttttttgct ttttttgatt tttttagggc 4680
caactaagct tccagatttc gctaatcacc tttgtactaa ttacaagaaa ggaagaagct 4740
gattagagtt gggcttttta tgcaactgtg ctactcctta tctctgatat gaaagtgtag 4800
acccaatcac atcatgtcat ttagagttgg taatactggg aggatagata aggcacgaaa 4860
acgagccata gcagacatgc tgggtgtagc caagcagaag aaagtagatg ggagccaatt 4920
gacgagcgag ggagctacgc caatccgaca tacgacacgc tgagatcgtc ttggccgggg 4980
ggtacctaca gatgtccaag ggtaagtgct tgactgtaat tgtatgtctg aggacaaata 5040
tgtagtcagc cgtataaagt cataccaggc accagtgcca tcatcgaacc actaactctc 5100
tatgatacat gcctccggta ttattgtacc atgcgtcgct ttgttacata cgtatcttgc 5160
ctttttctct cagaaactcc agactttggc tattggtcga gataagcccg gaccatagtg 5220
agtctttcac actctacatt tctcccttgc tccaactatc gattgttgtc tactaactat 5280
cgtacgataa cttcgtatag catacattat acgaagttat cgcgtcgacg agtatctgtc 5340
tgactcgtca ttgccgcctt tggagtacga ctccaactat gagtgtgctt ggatcacttt 5400
gacgatacat tcttcgttgg aggctgtggg tctgacagct gcgttttcgg cgcggttggc 5460
cgacaacaat atcagctgca acgtcattgc tggctttcat catgatcaca tttttgtcgg 5520
caaaggcgac gcccagagag ccattgacgt tctttctaat ttggaccgat agccgtatag 5580
tccagtctat ctataagttc aactaactcg taactattac cataacatat acttcactgc 5640
cccagataag gttccgataa aaagttctgc agactaaatt tatttcagtc tcctcttcac 5700
caccaaaatg ccctcctacg aagctcgagc taacgtccac aagtccgcct ttgccgctcg 5760
agtgctcaag ctcgtggcag ccaagaaaac caacctgtgt gcttctctgg atgttaccac 5820
caccaaggag ctcattgagc ttgccgataa ggtcggacct tatgtgtgca tgatcaaaac 5880

ccatatcgac atcattgacg acttcaccta cgccggcact gtgctccccc tcaaggaact 5940
tgctcttaag cacggtttct tcctgttcga ggacagaaag ttcgcagata ttggcaacac 6000
tgtcaagcac cagtaccggt gtcaccgaat cgccgagtgg tccgatatca ccaacgccca 6060
cggtgtaccc ggaaccggaa tcattgctgg cctgcgagct ggtgccgagg aaactgtctc 6120
tgaacagaag aaggaggacg tctctgacta cgagaactcc cagtacaagg agttcctagt 6180
cccctctccc aacgagaagc tggccagagg tctgctcatg ctggccgagc tgtcttgcaa 6240
gggctctctg gccactggcg agtactccaa gcagaccatt gagcttgccc gatccgaccc 6300
cgagtttgtg gttggcttca ttgcccagaa ccgacctaag ggcgactctg aggactggct 6360
tattctgacc cccggggtgg gtcttgacga caagggagac gctctcggac agcagtaccg 6420
aactgttgag gatgtcatgt ctaccggaac ggatatcata attgtcggcc gaggtctgta 6480
cggccagaac cgagatccta ttgaggaggc caagcgatac cagaaggctg gctgggaggc 6540
ttaccagaag attaactgtt agaggttaga ctatggatat gtaatttaac tgtgtatata 6600
gagagcgtgc aagtatggag cgcttgttca gcttgtatga tggtcagacg acctgtctga 6660
tcgagtatgt atgatactgc acaacctgtg tatccgcatg atctgtccaa tggggcatgt 6720
tgttgtgttt ctcgatacgg agatgctggg tacagtgcta atacgttgaa ctacttatac 6780
ttatatgagg ctcgaagaaa gctgacttgt gtatgactta ttctcaacta catccccagt 6840
cacaatacca ccactgcact accactacac caaaaccatg atcaaaccac ccatggactt 6900
cctggaggca gaagaacttg ttatggaaaa gctcaagaga gagatcataa cttcgtatag 6960
catacattat acgaagttat cctgcaggta aaggaattca tgctgttcat cgtggttaat 7020
gctgctgtgt gctgtgtgtg tgtgttgttt ggcgctcatt gttgcgttat gcagcgtaca 7080
ccacaatatt ggaagcttat tagcctttct atttttcgt ttgcaaggct taacaacatt 7140
gctgtggaga gggatgggga tatggaggcc gctggaggga gtcggagagg cgttttggag 7200
cggcttggcc tggcgcccag ctcgcgaaac gcacctagga ccctttggca cgccgaaatg 7260
tgccactttt cagtctagta acgccttacc tacgtcattc catgcgtgca tgtttgcgcc 7320
ttttttccct tgcccttgat cgccacacag tacagtgcac tgtacagtgg aggttttggg 7380
ggggtcttag atgggagcta aaagcggcct agcggtacac tagtgggatt gtatggagtg 7440
gcatggagcc taggtggagc ctgacaggac gcacgaccgg ctagcccgtg acagacgatg 7500
ggtggctcct gttgtccacc gcgtacaaat gtttgggcca aagtcttgtc agccttgctt 7560
gcgaacctaa ttcccaattt tgtcacttcg cacccccatt gatcgagccc taacccctgc 7620
ccatcaggca atccaattaa gctcgcattg tctgccttgt ttagtttggc tcctgcccgt 7680
ttcggcgtcc acttgcacaa acacaaacaa gcattatata taaggctcgt ctctccctcc 7740
caaccacact cactttttg cccgtcttcc cttgctaaca caaaagtcaa gaacacaaac 7800

aaccacccca acccccttac acacaagaca tatctacagc aatggccatg gcttcttcca 7860
ctgttgctgc gccgtacgag ttcccgacgc tgacggagat caagcgctcg ctgccagcgc 7920
actgctttga ggcctcggtc ccgtggtcgc tctactacac cgtgcgcgcg ctgggcatcg 7980
ccggctcgct cgcgctcggc ctctactacg cgcgcgcgct cgcgatcgtg caggagtttg 8040
ccctgctgga tgcggtgctc tgcacggggt acattctgct gcagggcatc gtattctggg 8100
ggttcttcac catcggccat gactgcggcc acggcgcgtt ctcgcgttcg cacctgctca 8160
acttcagcgt cggcacgctc attcactcga tcatcctcac gccgtacgag tcatggaaga 8220
tctcgcaccg ccaccaccac aagaacacgg gcaacatcga caaggacgag attttctacc 8280
cgcagcgcga ggccgactcg cacccactgt cccgacacat ggtgatctcg ctcggctcgg 8340
cctggttcgc gtacctcgtt gcgggcttcc ctcctcgcaa ggtgaaccac ttcaaccctt 8400
gggaaccgtt gtacctgcgc cgcatgtctg ccgtcatcat ctcactcggc tcgctcgtgg 8460
cgttcgcggg cttgtatgcg tatctcacct acgtctatgg ccttaagacc atggcgctgt 8520
actacttcgc ccctctcttt gggttcgcca cgatgctcgt ggtcactacc tttttgcacc 8580
acaatgacga ggaaacgcca tggtacgccg actcggagtg gacgtacgtc aagggcaacc 8640
tctcgtccgt ggaccgctcg tacggcgcgc tcatcgacaa cctgagccac aacatcggca 8700
cgcaccagat ccaccacctg tttccgatca tcccgcacta caagctgaac gaggcgacgg 8760
cagcgttcgc gcaggcgttc ccggagctcg tgcgcaagag cgcgtcgccg atcatcccga 8820
cgttcatccg catcgggctc atgtacgcca agtacggcgt cgtggacaag gacgccaaga 8880
tgtttacgct caaggaggcc aaggccgcca agaccaaggc caactaggcg gccgcattga 8940
tgattggaaa cacacacatg ggttatatct aggtgagagt tagttggaca gttatatatt 9000
aaatcagcta tgccaacggt aacttcattc atgtcaacga ggaaccagtg actgcaagta 9060
atatagaatt tgaccacctt gccattctct tgcactcctt tactatatct catttatttc 9120
ttatatacaa atcacttctt cttcccagca tcgagctcgg aaacctcatg agcaataaca 9180
tcgtggatct cgtcaataga gggctttttg gactccttgc tgttggccac cttgtccttg 9240
ctgtttaaac agtgtacgca gatctactat agaggaacat ttaaattgcc ccggagaaga 9300
cggccaggcc gcctagatga caaattcaac aactcacagc tgactttctg ccattgccac 9360
tagggggggg cctttttata tggccaagcc aagctctcca cgtcggttgg gctgcaccca 9420
acaataaatg ggtagggttg caccaacaaa gggatgggat gggggtaga agatacgagg 9480
ataacggggc tcaatggcac aaataagaac gaatactgcc attaagactc gtgatccagc 9540
gactgacacc attgcatcat ctaagggcct caaaactacc tcggaactgc tgcgctgatc 9600
tggacaccac agaggttccg agcactttag gttgcaccaa atgtcccacc aggtgcaggc 9660
agaaaacgct ggaacagcgt gtacagtttg tcttaacaaa aagtgagggc gctgaggtcg 9720
agcagggtgg tgtgacttgt tatagccttt agagctgcga aagcgcgtat ggatttggct 9780

catcaggcca gattgagggt ctgtggacac atgtcatgtt agtgtacttc aatcgccccc 9840
tggatatagc cccgacaata ggccgtggcc tcattttttt gccttccgca catttccatt 9900
gctcggtacc cacaccttgc ttctcctgca cttgccaacc ttaatactgg tttacattga 9960
ccaacatctt acaagcgggg ggcttgtcta gggtatatat aaacagtggc tctcccaatc 10020
ggttgccagt ctcttttttc ctttctttcc ccacagattc gaaatctaaa ctacacatca 10080
cagaattccg agccgtgagt atccacgaca agatcagtgt cgagacgacg cgttttgtgt 10140
aatgacacaa tccgaaagtc gctagcaaca cacactctct acacaaacta acccagctct 10200
ggtaccatgg cttcttccac tgttgctgcg ccgtacgagt tcccgacgct gacggagatc 10260
aagcgctcgc tgccagcgca ctgctttgag gcctcggtcc cgtggtcgct ctactacacc 10320
gtgcgcgcgc tgggcatcgc cggctcgctc gcgctcggcc tctactacgc gcgcgcgctc 10380
gcgatcgtgc aggagtttgc cctgctggat gcggtgctct gcacggggta cattctgctg 10440
cagggcatcg tattctgggg gttcttcacc atcggccatg actgcggcca cggcgcgttc 10500
tcgcgttcgc acctgctcaa cttcagcgtc ggcacgctca ttcactcgat catcctcacg 10560
ccgtacgagt catggaagat ctcgcaccgc caccaccaca agaacacggg caacatcgac 10620
aaggacgaga ttttctaccc gcagcgcgag gccgactcgc acccactgtc ccgacacatg 10680
gtgatctcgc tcggctcggc ctggttcgcg tacctcgttg cgggcttccc tcctcgcaag 10740
gtgaaccact tcaacccttg ggaaccgttg tacctgcgcc gcatgtctgc cgtcatcatc 10800
tcactcggct cgctcgtggc gttcgcgggc ttgtatgcgt atctcaccta cgtctatggc 10860
cttaagacca tggcgctgta ctacttcgcc cctctctttg ggttcgccac gatgctcgtg 10920
gtcactacct tttgcacca caatgacgag gaaacgccat ggtacgccga ctcggagtgg 10980
acgtacgtca agggcaacct ctcgtccgtg gaccgctcgt acggcgcgct catcgacaac 11040
ctgagccaca acatcggcac gcaccagatc caccacctgt ttccgatcat cccgcactac 11100
aagctgaacg aggcgacggc agcgttcgcg caggcgttcc cggagctcgt gcgcaagagc 11160
gcgtcgccga tcatcccgac gttcatccgc atcgggctca tgtacgccaa gtacggcgtc 11220
gtggacaagg acgccaagat gtttacgctc aaggaggcca aggccgccaa gaccaaggcc 11280
aactaggcgg ccgcatggag cgtgtgttct gagtcgatgt tttctatgga gttgtgagtg 11340
ttagtagaca tgatgggttt atatatgatg aatgaataga tgtgattttg atttgcacga 11400
tggaattgag aactttgtaa acgtacatgg gaatgtatga atgtgggggt tttgtgactg 11460
gataactgac ggtcagtgga cgccgttgtt caaatatcca agagatgcga gaaactttgg 11520
gtcaagtgaa catgtcctct ctgttcaagt aaaccatcaa ctatgggtag tatatttagt 11580
aaggacaaga gttgagattc tttggagtcc tagaaacgta ttttcgcgtt ccaagatcaa 11640
attagtagag taatacgggc acgggaatcc attcatagtc tcaatcctgc aggtgagtta 11700

attaagatga cgacatttgc gagctggacg aggaatagat ggagcgtgtg ttctgagtcg 11760

atgttttcta tggagttgtg agtgttagta gacatgatgg gtttatatat gatgaatgaa 11820

tagatgtgat tttgatttgc acgatggaat tgagaacttt gtaaacgtac atgggaatgt 11880

atgaatgtgg gggttttgtg actggataac tgacggtcag tggacgccgt tgttcaaata 11940

tccaagagat gcgagaaact ttgggtcaag tgaacatgtc ctctctgttc aagtaaacca 12000

tcaactatgg gtagtatatt tagtaaggac aagagttgag attctttgga gtcctagaaa 12060

cgtattttcg cgttccaaga tcaaattagt agagtaatac gggcacggga atccattcat 12120

agtctcaatt ttcccatagg tgtgctacaa ggtgttgaga tgtggtacag taccaccatg 12180

attcgaggta aagagcccag aagtcattga tgaggtcaag aaatacacag atctacagct 12240

caatacaatg aatatcttct ttcatattct tcaggtgaca ccaagggtgt ctattttccc 12300

cagaaatgcg tgaaaaggcg cgtgtgtagc gtggagtatg ggttcggttg gcgtatcctt 12360

catatatcga cgaaatagta gggcaagaga tgacaaaaag tatctatatg tagacagcgt 12420

agaatatgga tttgattggt ataaattcat ttattgcgtg tctcacaaat actctcgata 12480

agttggggtt aaactggaga tggaacaatg tcgatatctc gacgcatgcg acgtcgggcc 12540

caattcgccc tatagtgagt cgtattacaa ttcactggcc gtcgttttac aacgtcgtga 12600

ctgggaaaac cctggcgtta cccaacttaa tcgccttgca gcacatcccc ctttcgccag 12660

ctggcgtaat agcgaagagg cccgcaccga tcgcccttcc caacagttgc gcagcctgaa 12720

tggcgaatgg acgcgccctg tagcggcgca ttaagcgcgg cgggtgtggt ggttacgcgc 12780

agcgtgaccg ctacacttgc cagcgcccta gcgcccgctc ctttcgcttt cttcccttcc 12840

tttctcgcca cgttcgccgg ctttccccgt caagctctaa atcgggggct ccctttaggg 12900

ttccgattta gtgctttacg gcacctcgac cccaaaaaac ttgattaggg tgatggttca 12960

cgtagtgggc catcgccctg atagacggtt tttcgccctt tgacgttgga gtccacgttc 13020

tttaatagtg gactcttgtt ccaaactgga acaacactca acccta 13066

<210> 40
<211> 4313
<212> DNA
<213> Artificial Sequence

<220>
<223> Plasmid pZKUM

<400> 40

```
taatcgagct tggcgtaatc atggtcatag ctgtttcctg tgtgaaattg ttatccgctc      60
acaattccac acaacatacg agccggaagc ataaagtgta aagcctgggg tgcctaatga     120
gtgagctaac tcacattaat tgcgttgcgc tcactgcccg ctttccagtc gggaaacctg     180
tcgtgccagc tgcattaatg aatcggccaa cgcgcgggga gaggcggttt gcgtattggg     240
cgctcttccg cttcctcgct cactgactcg ctgcgctcgg tcgttcggct gcggcgagcg     300
```

gtatcagctc actcaaaggc ggtaatacgg ttatccacag aatcagggga taacgcagga 360

aagaacatgt gagcaaaagg ccagcaaaag gccaggaacc gtaaaaaggc cgcgttgctg 420

gcgtttttcc ataggctccg cccccctgac gagcatcaca aaaatcgacg ctcaagtcag 480

aggtggcgaa acccgacagg actataaaga taccaggcgt ttccccctgg aagctccctc 540

gtgcgctctc ctgttccgac cctgccgctt accggatacc tgtccgcctt tctcccttcg 600

ggaagcgtgg cgctttctca tagctcacgc tgtaggtatc tcagttcggt gtaggtcgtt 660

cgctccaagc tgggctgtgt gcacgaaccc cccgttcagc ccgaccgctg cgccttatcc 720

ggtaactatc gtcttgagtc caacccggta agacacgact tatcgccact ggcagcagcc 780

actggtaaca ggattagcag agcgaggtat gtaggcggtg ctacagagtt cttgaagtgg 840

tggcctaact acggctacac tagaaggaca gtatttggta tctgcgctct gctgaagcca 900

gttaccttcg gaaaaagagt tggtagctct tgatccggca aacaaaccac cgctggtagc 960

ggtggttttt ttgtttgcaa gcagcagatt acgcgcagaa aaaaaggatc tcaagaagat 1020

cctttgatct tttctacggg gtctgacgct cagtggaacg aaaactcacg ttaagggatt 1080

ttggtcatga gattatcaaa aaggatcttc acctagatcc ttttaaatta aaaatgaagt 1140

tttaaatcaa tctaaagtat atatgagtaa acttggtctg acagttacca atgcttaatc 1200

agtgaggcac ctatctcagc gatctgtcta tttcgttcat ccatagttgc ctgactcccc 1260

gtcgtgtaga taactacgat acgggagggc ttaccatctg gccccagtgc tgcaatgata 1320

ccgcgagacc cacgctcacc ggctccagat ttatcagcaa taaaccagcc agccggaagg 1380

gccgagcgca gaagtggtcc tgcaacttta tccgcctcca tccagtctat taattgttgc 1440

cgggaagcta gagtaagtag ttcgccagtt aatagtttgc gcaacgttgt tgccattgct 1500

acaggcatcg tggtgtcacg ctcgtcgttt ggtatggctt cattcagctc cggttcccaa 1560

cgatcaaggc gagttacatg atcccccatg ttgtgcaaaa aagcggttag ctccttcggt 1620

cctccgatcg ttgtcagaag taagttggcc gcagtgttat cactcatggt tatggcagca 1680

ctgcataatt ctcttactgt catgccatcc gtaagatgct tttctgtgac tggtgagtac 1740

tcaaccaagt cattctgaga atagtgtatg cggcgaccga gttgctcttg cccggcgtca 1800

atacgggata ataccgcgcc acatagcaga actttaaaag tgctcatcat tggaaaacgt 1860

tcttcggggc gaaaactctc aaggatctta ccgctgttga gatccagttc gatgtaaccc 1920

actcgtgcac ccaactgatc ttcagcatct tttactttca ccagcgtttc tgggtgagca 1980

aaaacaggaa ggcaaaatgc cgcaaaaaag ggaataaggg cgacacggaa atgttgaata 2040

ctcatactct tcctttttca atattattga agcatttatc agggttattg tctcatgagc 2100

ggatacatat ttgaatgtat ttagaaaaat aaacaaatag gggttccgcg cacatttccc 2160

cgaaaagtgc cacctgacgc gccctgtagc ggcgcattaa gcgcggcggg tgtggtggtt 2220

acgcgcagcg tgaccgctac acttgccagc gccctagcgc ccgctccttt cgctttcttc 2280
ccttcctttc tcgccacgtt cgccggcttt ccccgtcaag ctctaaatcg ggggctccct 2340
ttagggttcc gatttagtgc tttacggcac ctcgacccca aaaaacttga ttagggtgat 2400
ggttcacgta gtgggccatc gccctgatag acggtttttc gccctttgac gttggagtcc 2460
acgttcttta atagtggact cttgttccaa actggaacaa cactcaaccc tatctcggtc 2520
tattcttttg atttataagg gattttgccg atttcggcct attggttaaa aaatgagctg 2580
atttaacaaa aatttaacgc gaattttaac aaaatattaa cgcttacaat ttccattcgc 2640
cattcaggct gcgcaactgt tgggaagggc gatcggtgcg ggcctcttcg ctattacgcc 2700
agctggcgaa agggggatgt gctgcaaggc gattaagttg ggtaacgcca gggttttccc 2760
agtcacgacg ttgtaaaacg acggccagtg aattgtaata cgactcacta tagggcgaat 2820
tgggtaccgg gccccccctc gaggtcgacg agtatctgtc tgactcgtca ttgccgcctt 2880
tggagtacga ctccaactat gagtgtgctt ggatcacttt gacgatacat tcttcgttgg 2940
aggctgtggg tctgacagct gcgttttcgg cgcggttggc cgacaacaat atcagctgca 3000
acgtcattgc tggctttcat catgatcaca tttttgtcgg caaaggcgac gcccagagag 3060
ccattgacgt tctttctaat ttggaccgat agccgtatag tccagtctat ctataagttc 3120
aactaactcg taactattac cataacatat acttcactgc cccagataag gttccgataa 3180
aaagttctgc agactaaatt tatttcagtc tcctcttcac caccaaaatg ccctcctacg 3240
aagctcgagt gctcaagctc gtggcagcca agaaaaccaa cctgtgtgct tctctggatg 3300
ttaccaccac caaggagctc attgagcttg ccgataaggt cggaccttat gtgtgcatga 3360
tcaaaaccca tatcgacatc attgacgact tcacctacgc cggcactgtg ctcccccctca 3420
aggaacttgc tcttaagcac ggtttcttcc tgttcgagga cagaaagttc gcagatattg 3480
gcaacactgt caagcaccag taccggtgtc accgaatcgc cgagtggtcc gatatcacca 3540
acgcccacgg tgtacccgga accggaatcg attgctggcc tgcgagctgg tgcgtacgag 3600
gaaactgtct ctgaacagaa gaaggaggac gtctctgact acgagaactc ccagtacaag 3660
gagttcctag tcccctctcc caacgagaag ctggccagag gtctgctcat gctggccgag 3720
ctgtcttgca agggctctct ggccactggc gagtactcca agcagaccat tgagcttgcc 3780
cgatccgacc ccgagtttgt ggttggcttc attgcccaga accgacctaa gggcgactct 3840
gaggactggc ttattctgac ccccggggtg ggtcttgacg acaagggaga cgctctcgga 3900
cagcagtacc gaactgttga ggatgtcatg tctaccggaa cggatatcat aattgtcggc 3960
cgaggtctgt acggccagaa ccgagatcct attgaggagg ccaagcgata ccagaaggct 4020
ggctgggagg cttaccagaa gattaactgt tagaggttag actatggata tgtaatttaa 4080
ctgtgtatat agagagcgtg caagtatgga gcgcttgttc agcttgtatg atggtcagac 4140
gacctgtctg atcgagtatg tatgatactg cacaacctgt gtatccgcat gatctgtcca 4200

atggggcatg ttgttgtgtt tctcgatacg gagatgctgg gtacagtgct aatacgttga 4260

actacttata cttatatgag gctcgaagaa agctgacttg tgtatgactt aat 4313

<210> 41
<211> 15991
<212> DNA
<213> Artificial Sequence

<220>
<223> Plasmid pZKL2-5mB89C

<400> 41

```
gtacgttatc atttgaacag tgaaaggcta cagtaacaga agcagttgta aacttcattc       60
cgttgattct gtactacagt accccactac gccgcttccg ctgacactgt tcaacccaaa      120
aactacatct gcgtgcgctg tgtaaggcta tcatcagata catactgtag attctgtaga      180
tgcgaacctg cttgtatcat atacatcccc ctccccctga cctgcacaag caagcaatgt      240
gacattgata ttgctgctta tctagtgccg aggatgtgaa agccgagact caaacatttc      300
ttttactctc ttgttcctga ccagacctgg cggagattac gccagtatga ttcttgcagg      360
tctgagacaa gcctggaaca gccaacattt atttttcgaa gcgagaaaca tgccacaccc      420
cggcacgttc agagatgcat atgatttgtt tttcgagtaa cagtaccccc cccccccccc      480
ccaatgaaac cagtattact cacaccatcc tcattcaaag cgttacactg attacgcgcc      540
catcaacgac agcatgaggg gactgctgat ctgatctaat caaatgacta caaaaatcgc      600
aataatgaag agcaaacgac aaaaaagaaa caggttaacc aatcccgctt caatgtctca      660
ccacaatcca gcactgtttc tcattacctc ctccctctaa tttcagagtt gcatcagggt      720
ccttgatggc gcgccagctg cattaatgaa tcggccaacg cgcggggaga ggcggtttgc      780
gtattgggcg ctcttccgct tcctcgctca ctgactcgct gcgctcggtc gttcggctgc      840
ggcgagcggt atcagctcac tcaaaggcgg taatacggtt atccacagaa tcaggggata      900
acgcaggaaa gaacatgtga gcaaaaggcc agcaaaaggc caggaaccgt aaaaaggccg      960
cgttgctggc gtttttccat aggctccgcc cccctgacga gcatcacaaa aatcgacgct     1020
caagtcagag gtggcgaaac ccgacaggac tataaagata ccaggcgttt ccccctggaa     1080
gctccctcgt gcgctctcct gttccgaccc tgccgcttac cggatacctg tccgcctttc     1140
tcccttcggg aagcgtggcg ctttctcata gctcacgctg taggtatctc agttcggtgt     1200
aggtcgttcg ctccaagctg ggctgtgtgc acgaaccccc cgttcagccc gaccgctgcg     1260
ccttatccgg taactatcgt cttgagtcca acccggtaag acacgactta tcgccactgg     1320
cagcagccac tggtaacagg attagcagag cgaggtatgt aggcggtgct acagagttct     1380
tgaagtggtg gcctaactac ggctacacta gaagaacagt atttggtatc tgcgctctgc     1440
tgaagccagt taccttcgga aaaagagttg gtagctcttg atccggcaaa caaaccaccg     1500
```

ctggtagcgg tggttttttt gtttgcaagc agcagattac gcgcagaaaa aaaggatctc 1560
aagaagatcc tttgatcttt tctacggggt ctgacgctca gtggaacgaa aactcacgtt 1620
aagggatttt ggtcatgaga ttatcaaaaa ggatcttcac ctagatcctt ttaaattaaa 1680
aatgaagttt taaatcaatc taaagtatat atgagtaaac ttggtctgac agttaccaat 1740
gcttaatcag tgaggcacct atctcagcga tctgtctatt tcgttcatcc atagttgcct 1800
gactccccgt cgtgtagata actacgatac gggagggctt accatctggc cccagtgctg 1860
caatgatacc gcgagaccca cgctcaccgg ctccagattt atcagcaata aaccagccag 1920
ccggaagggc cgagcgcaga agtggtcctg caactttatc cgcctccatc cagtctatta 1980
attgttgccg ggaagctaga gtaagtagtt cgccagttaa tagtttgcgc aacgttgttg 2040
ccattgctac aggcatcgtg gtgtcacgct cgtcgtttgg tatggcttca ttcagctccg 2100
gttcccaacg atcaaggcga gttacatgat cccccatgtt gtgcaaaaaa gcggttagct 2160
ccttcggtcc tccgatcgtt gtcagaagta agttggccgc agtgttatca ctcatggtta 2220
tggcagcact gcataattct cttactgtca tgccatccgt aagatgcttt tctgtgactg 2280
gtgagtactc aaccaagtca ttctgagaat agtgtatgcg gcgaccgagt tgctcttgcc 2340
cggcgtcaat acgggataat accgcgccac atagcagaac tttaaaagtg ctcatcattg 2400
gaaaacgttc ttcggggcga aaactctcaa ggatcttacc gctgttgaga tccagttcga 2460
tgtaacccac tcgtgcaccc aactgatctt cagcatcttt tactttcacc agcgtttctg 2520
ggtgagcaaa aacaggaagg caaaatgccg caaaaaaggg aataagggcg acacggaaat 2580
gttgaatact catactcttc ctttttcaat attattgaag catttatcag ggttattgtc 2640
tcatgagcgg atacatattt gaatgtattt agaaaaataa acaaataggg gttccgcgca 2700
catttccccg aaaagtgcca cctgatgcgg tgtgaaatac cgcacagatg cgtaaggaga 2760
aaataccgca tcaggaaatt gtaagcgtta atattttgtt aaaattcgcg ttaaattttt 2820
gttaaatcag ctcatttttt aaccaatagg ccgaaatcgg caaaatccct tataaatcaa 2880
aagaatagac cgagataggg ttgagtgttg ttccagtttg gaacaagagt ccactattaa 2940
agaacgtgga ctccaacgtc aaagggcgaa aaaccgtcta tcagggcgat ggcccactac 3000
gtgaaccatc accctaatca agttttttgg ggtcgaggtg ccgtaaagca ctaaatcgga 3060
accctaaagg gagcccccga tttagagctt gacggggaaa gccggcgaac gtggcgagaa 3120
aggaagggaa gaaagcgaaa ggagcgggcg ctagggcgct ggcaagtgta gcggtcacgc 3180
tgcgcgtaac caccacaccc gccgcgctta atgcgccgct acagggcgcg tccattcgcc 3240
attcaggctg cgcaactgtt gggaagggcg atcggtgcgg gcctcttcgc tattacgcca 3300
gctggcgaaa gggggatgtg ctgcaaggcg attaagttgg gtaacgccag ggttttccca 3360
gtcacgacgt tgtaaaacga cggccagtga attgtaatac gactcactat agggcgaatt 3420
gggcccgacg tcgcatgctg gtttcgattt gtcttagagg aacgcatata cagtaatcat 3480

agagaataaa cgatattcat ttattaaagt agatagttga ggtagaagtt gtaaagagtg 3540
ataaatagct tagataccac agacaccctc ggtgacgaag tactgcagat ggtttccaat 3600
cacattgacc tgctggagca gagtgttacc ggcagagcac tgtttattgc tctggccctg 3660
gcacatgaca acgttggaga gaggagggtg gatcaggggc cagtcaataa agacctcacc 3720
agagcagtgc tggtaaccgt cccagaaggg cacttgaggg acgatatctc ctcggtgggt 3780
gattcggtag agctttcggt ctttggacac cttggagaca tcggggttct cctggccaaa 3840
gaagagttta tcgacccagt tagcaaagcc agcgttaccg acaatgggct gaccaagagt 3900
aacaacgagg ggatcgtggc cgttaacctt gaggttgatt ccgaacagaa gggctgcagc 3960
tcctccgaga gagtgaccgg tgacagcaat ctggtagtcg ggatactgct caatcacaga 4020
gtcgagcttg gggccgatct gattgtaggt gttgttgtag gactggatga agccattgtg 4080
gacaagacag tcatcacaag tagcagtaga agagatgtta gcagcaagat caaagttaat 4140
taactcacct gcaggattga gactatgaat ggattcccgt gcccgtatta ctctactaat 4200
ttgatcttgg aacgcgaaaa tacgtttcta ggactccaaa gaatctcaac tcttgtcctt 4260
actaaatata ctacccatag ttgatggttt acttgaacag agaggacatg ttcacttgac 4320
ccaaagtttc tcgcatctct tggatatttg aacaacggcg tccactgacc gtcagttatc 4380
cagtcacaaa acccccacat tcatacattc ccatgtacgt ttacaaagtt ctcaattcca 4440
tcgtgcaaat caaaatcaca tctattcatt catcatatat aaacccatca tgtctactaa 4500
cactcacaac tccatagaaa acatcgactc agaacacacg ctccatgcgg ccgcttagga 4560
atcctgagcg tccttgacac agtgaaccac accgactttg tgcatgtact tgagggtgga 4620
aatgatgttg cccacaatgg tagggtagaa gacgtaccga actccgtgtc gttcgcaaca 4680
ctctcggaca gcttgctgca cgaagggata gtgccaagac gacattcgag gaaagaggtg 4740
atgctcgatc tggaagttga gaccgccagt aaagaacatg gcaatgggtc caccgtaggt 4800
ggaagaggtc tccacctgag ctctgtacca gtcgatctga tcggcttcaa cgtccttctc 4860
ggagctcttg accttgcagt tcttgtcggg gattcgctcc gagccatcga agttgtgaga 4920
caagatgaaa aagaaggtga ggaaggcacc ggtagcagtg ggcaccagag gaatggtgat 4980
gagcagggag gttccagtga gataccaggg caagaaggcg gttcgaaaga tgaagaaagc 5040
tcgcataacg aatgcaaggg ttcggtaccg tcgcagaaag ccgttctctc gcatggctgt 5100
gacagactcg ggaatggtgt cgttgtgctg cattcggaag atgtagagag ggttgtacac 5160
cagcgaaacg ccgtaggctc caagcacgag gtacatgtac caggcctgga atcggtgaaa 5220
ccactttcga gcagtgttgg cagcagggta gttgtggaac acaaggaatg gttctgcgga 5280
ctcggcatcc aggtcgagac catgctgatt ggtgtaggtg tgatgtcgca tgatgtgaga 5340
ctgcagccag atccatctgg acgatccaat gacgtcgatg ccgtaggcaa agagagcgtt 5400

gacccagggc tttttgctga tggcaccatg agaggcatcg tgctgaatgg acaggccgat 5460
ctgcatgtgc atgaatccag tcaagagacc ccacagcacc attccggtag tagcccagtg 5520
ccactcgcaa aaggcggtga cagcaatgat gccaacggtt cgcagccaga atccaggtgt 5580
ggcataccag ttccgacctt tcatgacctc tcgcatagtt cgcttgacgt cctgtgcaaa 5640
gggagagtcg taggtgtaga caatgtcctt ggaggttcgg tcgtgcttgc ctcgcacgaa 5700
ctgttgaagc agcttcgagt tctcgggctt gacgtaaggg tgcatggagt agaacagagg 5760
agaagcatcg gaggcaccag aagcgaggat caagtcggat ccgggatgga ccttggcaag 5820
accttccaga tcgtagagaa tgccgtcgat ggcaaccagg tcgggtcgct cgagcagctg 5880
ctcggtagta agggagagag ccatgggcag gacctgtgtt agtacattgt cggggagtca 5940
tcaattggtt cgacaggttg tcgactgtta gtatgagctc aattgggctc tggtgggtcg 6000
atgacacttg tcatctgttt ctgttgggtc atgtttccat caccttctat ggtactcaca 6060
attcgtccga ttcgcccgaa tccgttaata ccgactttga tggccatgtt gatgtgtgtt 6120
taattcaaga atgaatatag agaagagaag aagaaaaaag attcaattga gccggcgatg 6180
cagaccctta tataaatgtt gccttggaca gacggagcaa gcccgcccaa acctacgttc 6240
ggtataatat gttaagcttt ttaacacaaa ggtttggctt ggggtaacct gatgtggtgc 6300
aaaagaccgg gcgttggcga gccattgcgc gggcgaatgg ggccgtgact cgtctcaaat 6360
tcgagggcgt gcctcaattc gtgcccccgt ggctttttcc cgccgtttcc gccccgtttg 6420
caccactgca gccgcttctt tggttcggac accttgctgc gagctaggtg ccttgtgcta 6480
cttaaaaagt ggcctcccaa caccaacatg acatgagtgc gtgggccaag acacgttggc 6540
ggggtcgcag tcggctcaat ggcccggaaa aaacgctgct ggagctggtt cggacgcagt 6600
ccgccgcggc gtatggatat ccgcaaggtt ccatagcgcc attgccctcc gtcggcgtct 6660
atcccgcaac ctctaaatag agcgggaata taacccaagc ttcttttttt tcctttaaca 6720
cgcacacccc caactatcat gttgctgctg ctgtttgact ctactctgtg gaggggtgct 6780
cccacccaac ccaacctaca ggtggatccg gcgctgtgat tggctgataa gtctcctatc 6840
cggactaatt ctgaccaatg ggacatgcgc gcaggaccca aatgccgcaa ttacgtaacc 6900
ccaacgaaat gcctacccct ctttggagcc cagcggcccc aaatcccccc aagcagcccg 6960
gttctaccgg cttccatctc caagcacaag cagcccggaa ttctctctct tgagcttttc 7020
cataacaagt tcttctgcct ccaggaagtc catgggtggt ttgatcatgg ttttggtgta 7080
gtggtagtgc agtggtggta ttgtgactgg ggatgtagtt gagaataagt catacacaag 7140
tcagctttct tcgagcctca tataagtata agtagttcaa cgtattagca ctgtacccag 7200
catctccgta tcgagaaaca caacaacatg ccccattgga cagatcatgc ggatacacag 7260
gttgtgcagt atcatacata ctcgatcaga caggtcgtct gaccatcata caagctgaac 7320
aagcgctcca tacttgcacg ctctctatat acacagttaa attacatatc catagtctaa 7380

cctctaacag ttaatcttct ggtaagcctc ccagccagcc ttctggtatc gcttggcctc 7440
ctcaatagga tctcggttct ggccgtacag acctcggccg acaattatga tatccgttcc 7500
ggtagacatg acatcctcaa cagttcggta ctgctgtccg agagcgtctc ccttgtcgtc 7560
aagacccacc ccgggggtca gaataagcca gtcctcagag tcgcccttag gtcggttctg 7620
ggcaatgaag ccaaccacaa actcggggtc ggatcgggca agctcaatgg tctgcttgga 7680
gtactcgcca gtggccagag agcccttgca agacagctcg gccagcatga gcagacctct 7740
ggccagcttc tcgttgggag aggggactag gaactccttg tactgggagt tctcgtagtc 7800
agagacgtcc tccttcttct gttcagagac agtttcctcg gcaccagctc gcaggccagc 7860
aatgattccg gttccgggta caccgtgggc gttggtgata tcggaccact cggcgattcg 7920
gtgacaccgg tactggtgct tgacagtgtt gccaatatct gcgaactttc tgtcctcgaa 7980
caggaagaaa ccgtgcttaa gagcaagttc cttgaggggg agcacagtgc cggcgtaggt 8040
gaagtcgtca atgatgtcga tatgggtttt gatcatgcac acataaggtc cgaccttatc 8100
ggcaagctca atgagctcct tggtggtggt aacatccaga gaagcacaca ggttggtttt 8160
cttggctgcc acgagcttga gcactcgagc ggcaaaggcg gacttgtgga cgttagctcg 8220
agcttcgtag gagggcattt tggtggtgaa gaggagactg aaataaattt agtctgcaga 8280
actttttatc ggaaccttat ctggggcagt gaagtatatg ttatggtaat agttacgagt 8340
tagttgaact tatagataga ctggactata cggctatcgg tccaaattag aaagaacgtc 8400
aatggctctc tgggcgtcgc ctttgccgac aaaaatgtga tcatgatgaa agccagcaat 8460
gacgttgcag ctgatattgt tgtcggccaa ccgcgccgaa aacgcagctg tcagacccac 8520
agcctccaac gaagaatgta tcgtcaaagt gatccaagca cactcatagt tggagtcgta 8580
ctccaaaggc ggcaatgacg agtcagacag atactcgtcg accttttcct tgggaaccac 8640
caccgtcagc ccttctgact cacgtattgt agccaccgac acaggcaaca gtccgtggat 8700
agcagaatat gtcttgtcgg tccatttctc accaacttta ggcgtcaagt gaatgttgca 8760
gaagaagtat gtgccttcat tgagaatcgg tgttgctgat ttcaataaag tcttgagatc 8820
agtttggcca gtcatgttgt ggggggtaat tggattgagt tatcgcctac agtctgtaca 8880
ggtatactcg ctgcccactt tatacttttt gattccgctg cacttgaagc aatgtcgttt 8940
accaaaagtg agaatgctcc acagaacaca ccccagggta tggttgagca aaaaataaac 9000
actccgatac ggggaatcga accccggtct ccacggttct caagaagtat tcttgatgag 9060
agcgtatcga tcgaggaaga ggacaagcgg ctgcttctta agtttgtgac atcagtatcc 9120
aaggcaccat tgcaaggatt caaggctttg aacccgtcat ttgccattcg taacgctggt 9180
agacaggttg atcggttccc tacggcctcc acctgtgtca atcttctcaa gctgcctgac 9240
tatcaggaca ttgatcaact tcggaagaaa cttttgtatg ccattcgatc acatgctggt 9300

ttcgatttgt cttagaggaa cgcatataca gtaatcatag agaataaacg atattcattt 9360
attaaagtag atagttgagg tagaagttgt aaagagtgat aaatagcggc cgctcactga 9420
atctttttgg ctcccttgtg ctttcggacg atgtaggtct gcacgtagaa gttgaggaac 9480
agacacagga cagtaccaac gtagaagtag ttgaaaaacc agccaaacat tctcattcca 9540
tcttgtcggt agcagggaat gttccggtac ttccagacga tgtagaagcc aacgttgaac 9600
tgaatgatct gcatagaagt aatcagggac ttgggcatag ggaacttgag cttgatcagt 9660
cgggtccaat agtagccgta catgatccag tgaatgaagc cgttgagcag cacaaagatc 9720
caaacggctt cgtttcggta gttgtagaac agccacatgt ccataggagc tccgagatgg 9780
tgaaagaact gcaaccaggt cagaggcttg cccatgaggg gcagatagaa ggagtcaatg 9840
tactcgagga acttgctgag gtagaacagc tgagtggtga ttcggaagac attgttgtcg 9900
aaagccttct cgcagttgtc ggacatgaca ccaatggtgt acatggcgta ggccatagag 9960
aggaaggagc ccagcgagta gatggacatg agcaggttgt agttggtgaa cacaaacttc 10020
attcgagact gacccttggg tccgagagga ccaagggtga acttcaggat gacgaaggcg 10080
atggagaggt acagcacctc gcagtgcgag gcatcagacc agagctgagc atagtcgacc 10140
ttgggaagaa cctcctggcc aatggagacg atttcgttca cgacctccat ggttgtgaat 10200
tagggtggtg agaatggttg gttgtaggga agaatcaaag gccggtctcg ggatccgtgg 10260
gtatatatat atatatatat atatacgatc cttcgttacc tccctgttct caaaactgtg 10320
gtttttcgtt tttcgttttt tgcttttttt gattttttta gggccaacta agcttccaga 10380
tttcgctaat cacctttgta ctaattacaa gaaaggaaga agctgattag agttgggctt 10440
tttatgcaac tgtgctactc cttatctctg atatgaaagt gtagacccaa tcacatcatg 10500
tcatttagag ttggtaatac tgggaggata gataaggcac gaaaacgagc catagcagac 10560
atgctgggtg tagccaagca gaagaaagta gatgggagcc aattgacgag cgagggagct 10620
acgccaatcc gacatacgac acgctgagat cgtcttggcc gggggtacc tacagatgtc 10680
caagggtaag tgcttgactg taattgtatg tctgaggaca aatatgtagt cagccgtata 10740
aagtcatacc aggcaccagt gccatcatcg aaccactaac tctctatgat acatgcctcc 10800
ggtattattg taccatgcgt cgctttgtta catacgtatc ttgccttttt ctctcagaaa 10860
ctccagactt tggctattgg tcgagataag cccggaccat agtgagtctt tcacactctg 10920
tttaaacacc actaaaaccc cacaaaatat atcttaccga atatacagat ctactataga 10980
ggaacaattg ccccggagaa gacggccagg ccgcctagat gacaaattca acaactcaca 11040
gctgactttc tgccattgcc actagggggg ggccttttta tatggccaag ccaagctctc 11100
cacgtcggtt gggctgcacc caacaataaa tgggtagggt tgcaccaaca aagggatggg 11160
atgggggta gaagatacga ggataacggg gctcaatggc acaaataaga acgaatactg 11220
ccattaagac tcgtgatcca gcgactgaca ccattgcatc atctaagggc ctcaaaacta 11280

cctcggaact gctgcgctga tctggacacc acagaggttc cgagcacttt aggttgcacc 11340
aaatgtccca ccaggtgcag gcagaaaacg ctggaacagc gtgtacagtt tgtcttaaca 11400
aaaagtgagg gcgctgaggt cgagcagggt ggtgtgactt gttatagcct ttagagctgc 11460
gaaagcgcgt atggatttgg ctcatcaggc cagattgagg gtctgtggac acatgtcatg 11520
ttagtgtact tcaatcgccc cctggatata gccccgacaa taggccgtgg cctcattttt 11580
ttgccttccg cacatttcca ttgctcggta cccacacctt gcttctcctg cacttgccaa 11640
ccttaatact ggtttacatt gaccaacatc ttacaagcgg ggggcttgtc tagggtatat 11700
ataaacagtg gctctcccaa tcggttgcca gtctcttttt tcctttcttt ccccacagat 11760
tcgaaatcta aactacacat cacacaatgc ctgttactga cgtccttaag cgaaagtccg 11820
gtgtcatcgt cggcgacgat gtccgagccg tgagtatcca cgacaagatc agtgtcgaga 11880
cgacgcgttt tgtgtaatga cacaatccga aagtcgctag caacacacac tctctacaca 11940
aactaaccca gctctccatg gtgaaggctt ctcgacaggc tctgcccctc gtcatcgacg 12000
gaaaggtgta cgacgtctcc gcttgggtga acttccaccc tggtggagct gaaatcattg 12060
agaactacca gggacgagat gctactgacg ccttcatggt tatgcactct caggaagcct 12120
tcgacaagct caagcgaatg cccaagatca accaggcttc cgagctgcct ccccaggctg 12180
ccgtcaacga agctcaggag gatttccgaa agctccgaga agagctgatc gccactggca 12240
tgtttgacgc ctctcccctc tggtactcgt acaagatctt gaccaccctg ggtcttggcg 12300
tgcttgcctt cttcatgctg gtccagtacc acctgtactt cattggtgct ctcgtgctcg 12360
gtatgcacta ccagcaaatg ggatggctgt ctcatgacat ctgccaccac cagaccttca 12420
agaaccgaaa ctggaataac gtcctgggtc tggtctttgg caacggactc cagggcttct 12480
ccgtgacctg gtggaaggac agacacaacg cccatcattc tgctaccaac gttcagggtc 12540
acgatcccga cattgataac ctgcctctgc tcgcctggtc cgaggacgat gtcactcgag 12600
cttctcccat ctcccgaaag ctcattcagt tccaacagta ctatttcctg gtcatctgta 12660
ttctcctgcg attcatctgg tgtttccagt ctgtgctgac cgttcgatcc ctcaaggacc 12720
gagacaacca gttctaccga tctcagtaca agaaagaggc cattggactc gctctgcact 12780
ggactctcaa gaccctgttc cacctcttct ttatgccctc catcctgacc tcgatgctgg 12840
tgttctttgt ttccgagctc gtcggtggct tcggaattgc catcgtggtc ttcatgaacc 12900
actaccctct ggagaagatc ggtgattccg tctgggacgg acatggcttc tctgtgggtc 12960
agatccatga gaccatgaac attcgacgag gcatcattac tgactggttc tttggaggcc 13020
tgaactacca gatcgagcac catctctggc ccaccctgcc tcgacacaac ctcactgccg 13080
tttcctacca ggtggaacag ctgtgccaga agcacaacct cccctaccga aaccctctgc 13140
cccatgaagg tctcgtcatc ctgctccgat acctgtccca gttcgctcga atggccgaga 13200

agcagcccgg tgccaaggct cagtaagcgg ccgcatgaga agataaatat ataaatacat 13260
tgagatatta aatgcgctag attagagagc ctcatactgc tcggagagaa gccaagacga 13320
gtactcaaag gggattacac catccatatc cacagacaca agctggggaa aggttctata 13380
tacactttcc ggaataccgt agtttccgat gttatcaatg ggggcagcca ggatttcagg 13440
cacttcggtg tctcggggtg aaatggcgtt cttggcctcc atcaagtcgt accatgtctt 13500
catttgcctg tcaaagtaaa acagaagcag atgaagaatg aacttgaagt gaaggaattt 13560
aaatagttgg agcaagggag aaatgtagag tgtgaaagac tcactatggt ccgggcttat 13620
ctcgaccaat agccaaagtc tggagtttct gagagaaaaa ggcaagatac gtatgtaaca 13680
aagcgacgca tggtacaata ataccggagg catgtatcat agagagttag tggttcgatg 13740
atggcactgg tgcctggtat gactttatac ggctgactac atatttgtcc tcagacatac 13800
aattacagtc aagcacttac ccttggacat ctgtaggtac cccccggcca agacgatctc 13860
agcgtgtcgt atgtcggatt ggcgtagctc cctcgctcgt caattggctc ccatctactt 13920
tcttctgctt ggctacaccc agcatgtctg ctatggctcg ttttcgtgcc ttatctatcc 13980
tcccagtatt accaactcta aatgacatga tgtgattggg tctacacttt catatcagag 14040
ataaggagta gcacagttgc ataaaaagcc caactctaat cagcttcttc ctttcttgta 14100
attagtacaa aggtgattag cgaaatctgg aagcttagtt ggccctaaaa aaatcaaaaa 14160
aagcaaaaaa cgaaaaacga aaaaccacag ttttgagaac agggaggtaa cgaaggatcg 14220
tatatatata tatatatata tatacccacg gatcccgaga ccggcctttg attcttccct 14280
acaaccaacc attctcacca ccctaattca caaccatggg cgtattcatt aaacaggagc 14340
agcttccggc tctcaagaag tacaagtact ccgccgagga tcactcgttc atctccaaca 14400
acattctgcg ccccttctgg cgacagtttg tcaaaatctt ccctctgtgg atggccccca 14460
acatggtgac tctgctgggc ttcttctttg tcattgtgaa cttcatcacc atgctcattg 14520
ttgatcccac ccacgaccgc gagcctccca gatgggtcta cctcacctac gctctgggtc 14580
tgttccttta ccagacattt gatgcctgtg acggatccca tgcccgacga actggccaga 14640
gtggacccct tggagagctg tttgaccact gtgtcgacgc catgaatacc tctctgattc 14700
tcacggtggt ggtgtccacc acccatatgg gatataacat gaagctactg attgtgcaga 14760
ttgccgctct cggaaacttc tacctgtcga cctgggagac ctaccatacc ggaactctgt 14820
acctttctgg cttctctggt cctgttgaag gtatcttgat tctggtggct cttttcgtcc 14880
tcaccttctt cactggtccc aacgtgtacg ctctgaccgt ctacgaggct cttcccgagt 14940
ccatcacttc gctgctgcct gccagcttcc tggacgtcac catcacccag atctacattg 15000
gattcggagt gctgggcatg gtgttcaaca tctacggcgc ctgcggaaac gtgatcaagt 15060
actacaacaa caagggcaag agcgctctcc ccgccattct cggaatcgcc ccctttggca 15120
tcttctacgt cggcgtcttt gcctgggccc atgttgctcc tctgcttctc tccaagtacg 15180

ccatcgtcta tctgtttgcc attggggctg cctttgccat gcaagtcggc cagatgattc 15240

ttgcccatct cgtgcttgct ccctttcccc actggaacgt gctgctcttc ttcccctttg 15300

tgggactggc agtgcactac attgcacccg tgtttggctg ggacgccgat atcgtgtcgg 15360

ttaacactct cttcacctgt tttggcgcca ccctctccat ttacgccttc tttgtgcttg 15420

agatcatcga cgagatcacc aactacctcg atatctggtg tctgcgaatc aagtaccctc 15480

aggagaagaa gaccgaataa gcggccgcat ggagcgtgtg ttctgagtcg atgttttcta 15540

tggagttgtg agtgttagta gacatgatgg gtttatatat gatgaatgaa tagatgtgat 15600

tttgatttgc acgatggaat tgagaacttt gtaaacgtac atgggaatgt atgaatgtgg 15660

gggttttgtg actggataac tgacggtcag tggacgccgt tgttcaaata tccaagagat 15720

gcgagaaact ttgggtcaag tgaacatgtc ctctctgttc aagtaaacca tcaactatgg 15780

gtagtatatt tagtaaggac aagagttgag attctttgga gtcctagaaa cgtattttcg 15840

cgttccaaga tcaaattagt agagtaatac gggcacggga atccattcat agtctcaatc 15900

ctgcaggtga gttaattaat cgagcttggc gtaatcatgg tcatagctgt ttcctgtgtg 15960

aaattgttat ccgctcacaa ttccacacaa c 15991

<210> 42
<211> 1185
<212> DNA
<213> Yarrowia lipolytica

<220>
<221> CDS
<222> (1)..(1185)
<223> diacylglycerol cholinephosphotransferase (Y1CPT1)

<300>
<302> HIGH EICOSAPENTAENOIC ACID PRODUCING STRAINS OF
<310> WO 2006/052870
<311> 2005-11-03
<312> 2006-05-18
<313> (1)..(1185)

<300>
<302> HIGH EICOSAPENTAENOIC ACID PRODUCING STRAINS OF
<310> US 2006-0115881-A1
<311> 2005-11-02
<312> 2006-06-01
<313> (1)..(1185)

<400> 42

```
atg ggc gta ttc att aaa cag gag cag ctt ccg gct ctc aag aag tac        48
Met Gly Val Phe Ile Lys Gln Glu Gln Leu Pro Ala Leu Lys Lys Tyr
1               5                   10                  15

aag tac tcc gcc gag gat cac tcg ttc atc tcc aac aac att ctg cgc        96
Lys Tyr Ser Ala Glu Asp His Ser Phe Ile Ser Asn Asn Ile Leu Arg
            20                  25                  30

ccc ttc tgg cga cag ttt gtc aaa atc ttc cct ctg tgg atg gcc ccc       144
```

```
Pro Phe Trp Arg Gln Phe Val Lys Ile Phe Pro Leu Trp Met Ala Pro
        35                  40                  45

aac atg gtg act ctg ttg ggc ttc ttc ttt gtc att gtg aac ttc atc       192
Asn Met Val Thr Leu Leu Gly Phe Phe Phe Val Ile Val Asn Phe Ile
    50                  55                  60

acc atg ctc att gtt gat ccc acc cac gac cgc gag cct ccc aga tgg       240
Thr Met Leu Ile Val Asp Pro Thr His Asp Arg Glu Pro Pro Arg Trp
65                  70                  75                  80

gtc tac ctc acc tac gct ctg ggt ctg ttc ctt tac cag aca ttt gat       288
Val Tyr Leu Thr Tyr Ala Leu Gly Leu Phe Leu Tyr Gln Thr Phe Asp
                85                  90                  95

gcc tgt gac gga tcc cat gcc cga cga act ggc cag agt gga ccc ctt       336
Ala Cys Asp Gly Ser His Ala Arg Arg Thr Gly Gln Ser Gly Pro Leu
            100                 105                 110

gga gag ctg ttt gac cac tgt gtc gac gcc atg aat acc tct ctg att       384
Gly Glu Leu Phe Asp His Cys Val Asp Ala Met Asn Thr Ser Leu Ile
        115                 120                 125

ctc acg gtg gtg gtg tcc acc acc cat atg gga tat aac atg aag ctg       432
Leu Thr Val Val Val Ser Thr Thr His Met Gly Tyr Asn Met Lys Leu
    130                 135                 140

ctg att gtg cag att gcc gct ctc gga aac ttc tac ctg tcg acc tgg       480
Leu Ile Val Gln Ile Ala Ala Leu Gly Asn Phe Tyr Leu Ser Thr Trp
145                 150                 155                 160

gag acc tac cat acc gga act ctg tac ctt tct ggc ttc tct ggt cct       528
Glu Thr Tyr His Thr Gly Thr Leu Tyr Leu Ser Gly Phe Ser Gly Pro
                165                 170                 175

gtt gaa ggt atc ttg att ctg gtg gct ctt ttc gtc ctc acc ttc ttc       576
Val Glu Gly Ile Leu Ile Leu Val Ala Leu Phe Val Leu Thr Phe Phe
            180                 185                 190

act ggt ccc aac gtg tac gct ctg acc gtc tac gag gct ctt ccc gaa       624
Thr Gly Pro Asn Val Tyr Ala Leu Thr Val Tyr Glu Ala Leu Pro Glu
        195                 200                 205

tcc atc act tcg ctg ctg cct gcc agc ttc ctg gac gtc acc atc acc       672
Ser Ile Thr Ser Leu Leu Pro Ala Ser Phe Leu Asp Val Thr Ile Thr
    210                 215                 220

cag atc tac att gga ttc gga gtg ctg ggc atg gtg ttc aac atc tac       720
Gln Ile Tyr Ile Gly Phe Gly Val Leu Gly Met Val Phe Asn Ile Tyr
225                 230                 235                 240

ggc gcc tgc gga aac gtg atc aag tac tac aac aac aag ggc aag agc       768
Gly Ala Cys Gly Asn Val Ile Lys Tyr Tyr Asn Asn Lys Gly Lys Ser
                245                 250                 255

gct ctc ccc gcc att ctc gga atc gcc ccc ttt ggc atc ttc tac gtc       816
Ala Leu Pro Ala Ile Leu Gly Ile Ala Pro Phe Gly Ile Phe Tyr Val
            260                 265                 270

ggc gtc ttt gcc tgg gcc cat gtt gct cct ctg ctt ctc tcc aag tac       864
Gly Val Phe Ala Trp Ala His Val Ala Pro Leu Leu Leu Ser Lys Tyr
        275                 280                 285

gcc atc gtc tat ctg ttt gcc att ggg gct gcc ttt gcc atg caa gtc       912
Ala Ile Val Tyr Leu Phe Ala Ile Gly Ala Ala Phe Ala Met Gln Val
```

```
    290                 295                 300

ggc cag atg att ctt gcc cat ctc gtg ctt gct ccc ttc ccc cac tgg          960
Gly Gln Met Ile Leu Ala His Leu Val Leu Ala Pro Phe Pro His Trp
305                 310                 315                 320

aac gtg ctg ctc ttc ttc ccc ttt gtg gga ctg gca gtg cac tac att         1008
Asn Val Leu Leu Phe Phe Pro Phe Val Gly Leu Ala Val His Tyr Ile
                325                 330                 335

gca ccc gtg ttt ggc tgg gac gcc gat atc gtg tcg gtt aac act ctc         1056
Ala Pro Val Phe Gly Trp Asp Ala Asp Ile Val Ser Val Asn Thr Leu
            340                 345                 350

ttc acc tgt ttt ggc gcc acc ctc tcc att tac gcc ttc ttt gtg ctt         1104
Phe Thr Cys Phe Gly Ala Thr Leu Ser Ile Tyr Ala Phe Phe Val Leu
        355                 360                 365

gag atc atc gac gag atc acc aac tac ctc gat atc tgg tgt ctg cga         1152
Glu Ile Ile Asp Glu Ile Thr Asn Tyr Leu Asp Ile Trp Cys Leu Arg
    370                 375                 380

atc aag tac cct cag gag aag aag act gag taa                             1185
Ile Lys Tyr Pro Gln Glu Lys Lys Thr Glu
385                 390
```

<210> 43
<211> 394
<212> PRT
<213> Yarrowia lipolytica

<400> 43

```
Met Gly Val Phe Ile Lys Gln Glu Gln Leu Pro Ala Leu Lys Lys Tyr
1               5                   10                  15

Lys Tyr Ser Ala Glu Asp His Ser Phe Ile Ser Asn Asn Ile Leu Arg
            20                  25                  30

Pro Phe Trp Arg Gln Phe Val Lys Ile Phe Pro Leu Trp Met Ala Pro
        35                  40                  45

Asn Met Val Thr Leu Leu Gly Phe Phe Phe Val Ile Val Asn Phe Ile
    50                  55                  60

Thr Met Leu Ile Val Asp Pro Thr His Asp Arg Glu Pro Pro Arg Trp
65                  70                  75                  80

Val Tyr Leu Thr Tyr Ala Leu Gly Leu Phe Leu Tyr Gln Thr Phe Asp
                85                  90                  95

Ala Cys Asp Gly Ser His Ala Arg Arg Thr Gly Gln Ser Gly Pro Leu
            100                 105                 110

Gly Glu Leu Phe Asp His Cys Val Asp Ala Met Asn Thr Ser Leu Ile
        115                 120                 125
```

```
Leu Thr Val Val Val Ser Thr Thr His Met Gly Tyr Asn Met Lys Leu
    130                 135                 140

Leu Ile Val Gln Ile Ala Ala Leu Gly Asn Phe Tyr Leu Ser Thr Trp
145                 150                 155                 160

Glu Thr Tyr His Thr Gly Thr Leu Tyr Leu Ser Gly Phe Ser Gly Pro
                165                 170                 175

Val Glu Gly Ile Leu Ile Leu Val Ala Leu Phe Val Leu Thr Phe Phe
            180                 185                 190

Thr Gly Pro Asn Val Tyr Ala Leu Thr Val Tyr Glu Ala Leu Pro Glu
        195                 200                 205

Ser Ile Thr Ser Leu Leu Pro Ala Ser Phe Leu Asp Val Thr Ile Thr
    210                 215                 220

Gln Ile Tyr Ile Gly Phe Gly Val Leu Gly Met Val Phe Asn Ile Tyr
225                 230                 235                 240

Gly Ala Cys Gly Asn Val Ile Lys Tyr Tyr Asn Asn Lys Gly Lys Ser
                245                 250                 255

Ala Leu Pro Ala Ile Leu Gly Ile Ala Pro Phe Gly Ile Phe Tyr Val
            260                 265                 270

Gly Val Phe Ala Trp Ala His Val Ala Pro Leu Leu Leu Ser Lys Tyr
        275                 280                 285

Ala Ile Val Tyr Leu Phe Ala Ile Gly Ala Ala Phe Ala Met Gln Val
    290                 295                 300

Gly Gln Met Ile Leu Ala His Leu Val Leu Ala Pro Phe Pro His Trp
305                 310                 315                 320

Asn Val Leu Leu Phe Phe Pro Phe Val Gly Leu Ala Val His Tyr Ile
                325                 330                 335

Ala Pro Val Phe Gly Trp Asp Ala Asp Ile Val Ser Val Asn Thr Leu
            340                 345                 350

Phe Thr Cys Phe Gly Ala Thr Leu Ser Ile Tyr Ala Phe Phe Val Leu
        355                 360                 365

Glu Ile Ile Asp Glu Ile Thr Asn Tyr Leu Asp Ile Trp Cys Leu Arg
    370                 375                 380
```

```
Ile Lys Tyr Pro Gln Glu Lys Lys Thr Glu
385                 390
```

<210> 44
<211> 1272
<212> DNA
<213> Artificial Sequence

<220>
<223> mutant EgD8S-23 delta-8 desaturase

<220>
<221> CDS
<222> (2)..(1270)

<300>
<302> MUTANT DELTA-8 DESATURASE GENES ENGINEERED BY TARGETED MUTAGENSIS AND THEIR USE IN MAKING POLYUNSATURATED FATTY ACIDS
<310> WO 2008/073271
<311> 2007-12-05
<312> 2008-06-19
<313> (1)..(1272)

<300>
<302> MUTANT DELTA-8 DESATURASE GENES ENGINEERED BY TARGETED MUTAGENSIS AND THEIR USE IN MAKING POLYUNSATURATED FATTY ACIDS
<310> US 2008-0138868-A1
<311> 2006-12-07
<312> 2008-06-12
<313> (1)..(1272)

<400> 44

```
c atg gtg aag gct tct cga cag gct ctg ccc ctc gtc atc gac gga aag        49
  Met Val Lys Ala Ser Arg Gln Ala Leu Pro Leu Val Ile Asp Gly Lys
  1               5                   10                  15

gtg tac gac gtc tcc gct tgg gtg aac ttc cac cct ggt gga gct gaa          97
Val Tyr Asp Val Ser Ala Trp Val Asn Phe His Pro Gly Gly Ala Glu
            20                  25                  30

atc att gag aac tac cag gga cga gat gct act gac gcc ttc atg gtt         145
Ile Ile Glu Asn Tyr Gln Gly Arg Asp Ala Thr Asp Ala Phe Met Val
        35                  40                  45

atg cac tct cag gaa gcc ttc gac aag ctc aag cga atg ccc aag atc         193
Met His Ser Gln Glu Ala Phe Asp Lys Leu Lys Arg Met Pro Lys Ile
    50                  55                  60

aac cag gct tcc gag ctg cct ccc cag gct gcc gtc aac gaa gct cag         241
Asn Gln Ala Ser Glu Leu Pro Pro Gln Ala Ala Val Asn Glu Ala Gln
65                  70                  75                  80

gag gat ttc cga aag ctc cga gaa gag ctg atc gcc act ggc atg ttt         289
Glu Asp Phe Arg Lys Leu Arg Glu Glu Leu Ile Ala Thr Gly Met Phe
                85                  90                  95

gac gcc tct ccc ctc tgg tac tcg tac aag atc ttg acc acc ctg ggt         337
Asp Ala Ser Pro Leu Trp Tyr Ser Tyr Lys Ile Leu Thr Thr Leu Gly
            100                 105                 110

ctt ggc gtg ctt gcc ttc ttc atg ctg gtc cag tac cac ctg tac ttc         385
Leu Gly Val Leu Ala Phe Phe Met Leu Val Gln Tyr His Leu Tyr Phe
        115                 120                 125
```

```
att ggt gct ctc gtg ctc ggt atg cac tac cag caa atg gga tgg ctg      433
Ile Gly Ala Leu Val Leu Gly Met His Tyr Gln Gln Met Gly Trp Leu
    130                 135                 140

tct cat gac atc tgc cac cac cag acc ttc aag aac cga aac tgg aat      481
Ser His Asp Ile Cys His His Gln Thr Phe Lys Asn Arg Asn Trp Asn
145                 150                 155                 160

aac gtc ctg ggt ctg gtc ttt ggc aac gga ctc cag ggc ttc tcc gtg      529
Asn Val Leu Gly Leu Val Phe Gly Asn Gly Leu Gln Gly Phe Ser Val
                165                 170                 175

acc tgg tgg aag gac aga cac aac gcc cat cat tct gct acc aac gtt      577
Thr Trp Trp Lys Asp Arg His Asn Ala His His Ser Ala Thr Asn Val
            180                 185                 190

cag ggt cac gat ccc gac att gat aac ctg cct ctg ctc gcc tgg tcc      625
Gln Gly His Asp Pro Asp Ile Asp Asn Leu Pro Leu Leu Ala Trp Ser
        195                 200                 205

gag gac gat gtc act cga gct tct ccc atc tcc cga aag ctc att cag      673
Glu Asp Asp Val Thr Arg Ala Ser Pro Ile Ser Arg Lys Leu Ile Gln
    210                 215                 220

ttc caa cag tac tat ttc ctg gtc atc tgt att ctc ctg cga ttc atc      721
Phe Gln Gln Tyr Tyr Phe Leu Val Ile Cys Ile Leu Leu Arg Phe Ile
225                 230                 235                 240

tgg tgt ttc cag tct gtg ctg acc gtt cga tcc ctc aag gac cga gac      769
Trp Cys Phe Gln Ser Val Leu Thr Val Arg Ser Leu Lys Asp Arg Asp
                245                 250                 255

aac cag ttc tac cga tct cag tac aag aaa gag gcc att gga ctc gct      817
Asn Gln Phe Tyr Arg Ser Gln Tyr Lys Lys Glu Ala Ile Gly Leu Ala
            260                 265                 270

ctg cac tgg act ctc aag acc ctg ttc cac ctc ttc ttt atg ccc tcc      865
Leu His Trp Thr Leu Lys Thr Leu Phe His Leu Phe Phe Met Pro Ser
        275                 280                 285

atc ctg acc tcg atg ctg gtg ttc ttt gtt tcc gag ctc gtc ggt ggc      913
Ile Leu Thr Ser Met Leu Val Phe Phe Val Ser Glu Leu Val Gly Gly
    290                 295                 300

ttc gga att gcc atc gtg gtc ttc atg aac cac tac cct ctg gag aag      961
Phe Gly Ile Ala Ile Val Val Phe Met Asn His Tyr Pro Leu Glu Lys
305                 310                 315                 320

atc ggt gat tcc gtc tgg gac gga cat ggc ttc tct gtg ggt cag atc     1009
Ile Gly Asp Ser Val Trp Asp Gly His Gly Phe Ser Val Gly Gln Ile
                325                 330                 335

cat gag acc atg aac att cga cga ggc atc att act gac tgg ttc ttt     1057
His Glu Thr Met Asn Ile Arg Arg Gly Ile Ile Thr Asp Trp Phe Phe
            340                 345                 350

gga ggc ctg aac tac cag atc gag cac cat ctc tgg ccc acc ctg cct     1105
Gly Gly Leu Asn Tyr Gln Ile Glu His His Leu Trp Pro Thr Leu Pro
        355                 360                 365

cga cac aac ctc act gcc gtt tcc tac cag gtg gaa cag ctg tgc cag     1153
Arg His Asn Leu Thr Ala Val Ser Tyr Gln Val Glu Gln Leu Cys Gln
    370                 375                 380
```

```
aag cac aac ctc ccc tac cga aac cct ctg ccc cat gaa ggt ctc gtc       1201
Lys His Asn Leu Pro Tyr Arg Asn Pro Leu Pro His Glu Gly Leu Val
385                 390                 395                 400

atc ctg ctc cga tac ctg tcc cag ttc gct cga atg gcc gag aag cag       1249
Ile Leu Leu Arg Tyr Leu Ser Gln Phe Ala Arg Met Ala Glu Lys Gln
                405                 410                 415

ccc ggt gcc aag gct cag taa gc                                        1272
Pro Gly Ala Lys Ala Gln
            420
```

<210> 45
<211> 422
<212> PRT
<213> Artificial Sequence

<220>
<223> Synthetic Construct

<400> 45

```
Met Val Lys Ala Ser Arg Gln Ala Leu Pro Leu Val Ile Asp Gly Lys
1               5                   10                  15

Val Tyr Asp Val Ser Ala Trp Val Asn Phe His Pro Gly Gly Ala Glu
            20                  25                  30

Ile Ile Glu Asn Tyr Gln Gly Arg Asp Ala Thr Asp Ala Phe Met Val
        35                  40                  45

Met His Ser Gln Glu Ala Phe Asp Lys Leu Lys Arg Met Pro Lys Ile
    50                  55                  60

Asn Gln Ala Ser Glu Leu Pro Pro Gln Ala Ala Val Asn Glu Ala Gln
65                  70                  75                  80

Glu Asp Phe Arg Lys Leu Arg Glu Glu Leu Ile Ala Thr Gly Met Phe
                85                  90                  95

Asp Ala Ser Pro Leu Trp Tyr Ser Tyr Lys Ile Leu Thr Thr Leu Gly
            100                 105                 110

Leu Gly Val Leu Ala Phe Phe Met Leu Val Gln Tyr His Leu Tyr Phe
        115                 120                 125

Ile Gly Ala Leu Val Leu Gly Met His Tyr Gln Gln Met Gly Trp Leu
    130                 135                 140

Ser His Asp Ile Cys His His Gln Thr Phe Lys Asn Arg Asn Trp Asn
145                 150                 155                 160

Asn Val Leu Gly Leu Val Phe Gly Asn Gly Leu Gln Gly Phe Ser Val
                165                 170                 175
```

```
Thr Trp Trp Lys Asp Arg His Asn Ala His His Ser Ala Thr Asn Val
            180                 185                 190

Gln Gly His Asp Pro Asp Ile Asp Asn Leu Pro Leu Leu Ala Trp Ser
        195                 200                 205

Glu Asp Asp Val Thr Arg Ala Ser Pro Ile Ser Arg Lys Leu Ile Gln
    210                 215                 220

Phe Gln Gln Tyr Tyr Phe Leu Val Ile Cys Ile Leu Leu Arg Phe Ile
225                 230                 235                 240

Trp Cys Phe Gln Ser Val Leu Thr Val Arg Ser Leu Lys Asp Arg Asp
                245                 250                 255

Asn Gln Phe Tyr Arg Ser Gln Tyr Lys Lys Glu Ala Ile Gly Leu Ala
            260                 265                 270

Leu His Trp Thr Leu Lys Thr Leu Phe His Leu Phe Phe Met Pro Ser
        275                 280                 285

Ile Leu Thr Ser Met Leu Val Phe Phe Val Ser Glu Leu Val Gly Gly
    290                 295                 300

Phe Gly Ile Ala Ile Val Val Phe Met Asn His Tyr Pro Leu Glu Lys
305                 310                 315                 320

Ile Gly Asp Ser Val Trp Asp Gly His Gly Phe Ser Val Gly Gln Ile
                325                 330                 335

His Glu Thr Met Asn Ile Arg Arg Gly Ile Ile Thr Asp Trp Phe Phe
            340                 345                 350

Gly Gly Leu Asn Tyr Gln Ile Glu His His Leu Trp Pro Thr Leu Pro
        355                 360                 365

Arg His Asn Leu Thr Ala Val Ser Tyr Gln Val Glu Gln Leu Cys Gln
    370                 375                 380

Lys His Asn Leu Pro Tyr Arg Asn Pro Leu Pro His Glu Gly Leu Val
385                 390                 395                 400

Ile Leu Leu Arg Tyr Leu Ser Gln Phe Ala Arg Met Ala Glu Lys Gln
                405                 410                 415

Pro Gly Ala Lys Ala Gln
            420
```

<210> 46
<211> 777
<212> DNA
<213> Euglena gracilis

<220>
<221> CDS
<222> (1)..(777)
<223> synthetic delta-9 elongase (codon-optimized for Yarrowia lipolytica)

<300>
<302> DELTA-9 ELONGASES AND THEIR USE IN MAKING POLYUNSATURATED FATTY ACIDS
<310> WO 2007/061742
<311> 2006-11-16
<312> 2007-05-31
<313> (1)..(777)

<300>
<302> DELTA-9 ELONGASES AND THEIR USE IN MAKING POLYUNSATURATED FATTY ACIDS
<310> US-2007-0117190-A1
<311> 2006-11-16
<312> 2007-05-24
<313> (1)..(777)

<400> 46

```
atg gag gtc gtg aac gaa atc gtc tcc att ggc cag gag gtt ctt ccc        48
Met Glu Val Val Asn Glu Ile Val Ser Ile Gly Gln Glu Val Leu Pro
1               5                   10                  15

aag gtc gac tat gct cag ctc tgg tct gat gcc tcg cac tgc gag gtg        96
Lys Val Asp Tyr Ala Gln Leu Trp Ser Asp Ala Ser His Cys Glu Val
            20                  25                  30

ctg tac ctc tcc atc gcc ttc gtc atc ctg aag ttc acc ctt ggt cct       144
Leu Tyr Leu Ser Ile Ala Phe Val Ile Leu Lys Phe Thr Leu Gly Pro
        35                  40                  45

ctc gga ccc aag ggt cag tct cga atg aag ttt gtg ttc acc aac tac       192
Leu Gly Pro Lys Gly Gln Ser Arg Met Lys Phe Val Phe Thr Asn Tyr
    50                  55                  60

aac ctg ctc atg tcc atc tac tcg ctg ggc tcc ttc ctc tct atg gcc       240
Asn Leu Leu Met Ser Ile Tyr Ser Leu Gly Ser Phe Leu Ser Met Ala
65                  70                  75                  80

tac gcc atg tac acc att ggt gtc atg tcc gac aac tgc gag aag gct       288
Tyr Ala Met Tyr Thr Ile Gly Val Met Ser Asp Asn Cys Glu Lys Ala
                85                  90                  95

ttc gac aac aat gtc ttc cga atc acc act cag ctg ttc tac ctc agc       336
Phe Asp Asn Asn Val Phe Arg Ile Thr Thr Gln Leu Phe Tyr Leu Ser
            100                 105                 110

aag ttc ctc gag tac att gac tcc ttc tat ctg ccc ctc atg ggc aag       384
Lys Phe Leu Glu Tyr Ile Asp Ser Phe Tyr Leu Pro Leu Met Gly Lys
        115                 120                 125

cct ctg acc tgg ttg cag ttc ttt cac cat ctc gga gct cct atg gac       432
Pro Leu Thr Trp Leu Gln Phe Phe His His Leu Gly Ala Pro Met Asp
    130                 135                 140
```

```
atg tgg ctg ttc tac aac tac cga aac gaa gcc gtt tgg atc ttt gtg        480
Met Trp Leu Phe Tyr Asn Tyr Arg Asn Glu Ala Val Trp Ile Phe Val
145                 150                 155                 160

ctg ctc aac ggc ttc att cac tgg atc atg tac ggc tac tat tgg acc        528
Leu Leu Asn Gly Phe Ile His Trp Ile Met Tyr Gly Tyr Tyr Trp Thr
                165                 170                 175

cga ctg atc aag ctc aag ttc cct atg ccc aag tcc ctg att act tct        576
Arg Leu Ile Lys Leu Lys Phe Pro Met Pro Lys Ser Leu Ile Thr Ser
            180                 185                 190

atg cag atc att cag ttc aac gtt ggc ttc tac atc gtc tgg aag tac        624
Met Gln Ile Ile Gln Phe Asn Val Gly Phe Tyr Ile Val Trp Lys Tyr
        195                 200                 205

cgg aac att ccc tgc tac cga caa gat gga atg aga atg ttt ggc tgg        672
Arg Asn Ile Pro Cys Tyr Arg Gln Asp Gly Met Arg Met Phe Gly Trp
    210                 215                 220

ttt ttc aac tac ttc tac gtt ggt act gtc ctg tgt ctg ttc ctc aac        720
Phe Phe Asn Tyr Phe Tyr Val Gly Thr Val Leu Cys Leu Phe Leu Asn
225                 230                 235                 240

ttc tac gtg cag acc tac atc gtc cga aag cac aag gga gcc aaa aag        768
Phe Tyr Val Gln Thr Tyr Ile Val Arg Lys His Lys Gly Ala Lys Lys
                245                 250                 255

att cag tga                                                            777
Ile Gln
```

<210> 47
<211> 258
<212> PRT
<213> Euglena gracilis

<400> 47

```
Met Glu Val Val Asn Glu Ile Val Ser Ile Gly Gln Glu Val Leu Pro
1               5                   10                  15

Lys Val Asp Tyr Ala Gln Leu Trp Ser Asp Ala Ser His Cys Glu Val
            20                  25                  30

Leu Tyr Leu Ser Ile Ala Phe Val Ile Leu Lys Phe Thr Leu Gly Pro
        35                  40                  45

Leu Gly Pro Lys Gly Gln Ser Arg Met Lys Phe Val Phe Thr Asn Tyr
    50                  55                  60

Asn Leu Leu Met Ser Ile Tyr Ser Leu Gly Ser Phe Leu Ser Met Ala
65                  70                  75                  80

Tyr Ala Met Tyr Thr Ile Gly Val Met Ser Asp Asn Cys Glu Lys Ala
                85                  90                  95
```

```
Phe Asp Asn Asn Val Phe Arg Ile Thr Thr Gln Leu Phe Tyr Leu Ser
            100                 105                 110

Lys Phe Leu Glu Tyr Ile Asp Ser Phe Tyr Leu Pro Leu Met Gly Lys
        115                 120                 125

Pro Leu Thr Trp Leu Gln Phe Phe His His Leu Gly Ala Pro Met Asp
    130                 135                 140

Met Trp Leu Phe Tyr Asn Tyr Arg Asn Glu Ala Val Trp Ile Phe Val
145                 150                 155                 160

Leu Leu Asn Gly Phe Ile His Trp Ile Met Tyr Gly Tyr Tyr Trp Thr
                165                 170                 175

Arg Leu Ile Lys Leu Lys Phe Pro Met Pro Lys Ser Leu Ile Thr Ser
            180                 185                 190

Met Gln Ile Ile Gln Phe Asn Val Gly Phe Tyr Ile Val Trp Lys Tyr
        195                 200                 205

Arg Asn Ile Pro Cys Tyr Arg Gln Asp Gly Met Arg Met Phe Gly Trp
    210                 215                 220

Phe Phe Asn Tyr Phe Tyr Val Gly Thr Val Leu Cys Leu Phe Leu Asn
225                 230                 235                 240

Phe Tyr Val Gln Thr Tyr Ile Val Arg Lys His Lys Gly Ala Lys Lys
                245                 250                 255

Ile Gln
```

<210> 48
<211> 14554
<212> DNA
<213> Artificial Sequence

<220>
<223> Plasmid pZKL1-2SR9G85

<400> 48

```
cgatagttgg agcaagggag aaatgtagag tgtgaaagac tcactatggt ccgggcttat      60
ctcgaccaat agccaaagtc tggagtttct gagagaaaaa ggcaagatac gtatgtaaca     120
aagcgacgca tggtacaata ataccggagg catgtatcat agagagttag tggttcgatg     180
atggcactgg tgcctggtat gactttatac ggctgactac atatttgtcc tcagacatac     240
aattacagtc aagcacttac ccttggacat ctgtaggtac cccccggcca agacgatctc     300
agcgtgtcgt atgtcggatt ggcgtagctc cctcgctcgt caattggctc ccatctactt     360
```

tcttctgctt ggctacaccc agcatgtctg ctatggctcg ttttcgtgcc ttatctatcc 420
tcccagtatt accaactcta aatgacatga tgtgattggg tctacacttt catatcagag 480
ataaggagta gcacagttgc ataaaaagcc caactctaat cagcttcttc ctttcttgta 540
attagtacaa aggtgattag cgaaatctgg aagcttagtt ggccctaaaa aaatcaaaaa 600
aagcaaaaaa cgaaaaacga aaaaccacag ttttgagaac agggaggtaa cgaaggatcg 660
tatatatata tatatatata tatacccacg gatcccgaga ccggcctttg attcttccct 720
acaaccaacc attctcacca ccctaattca caaccatggc tgccgtcatc gaggtggcca 780
acgagttcgt cgctatcact gccgagaccc ttcccaaggt ggactatcag cgactctggc 840
gagacatcta ctcctgcgag ctcctgtact tctccattgc tttcgtcatc ctcaagttta 900
cccttggcga gctctcggat tctggcaaaa agattctgcg agtgctgttc aagtggtaca 960
acctcttcat gtccgtcttt tcgctggtgt ccttcctctg tatgggttac gccatctaca 1020
ccgttggact gtactccaac gaatgcgaca gagctttcga caacagcttg ttccgatttg 1080
ccaccaaggt cttctactat tccaagtttc tggagtacat cgactctttc taccttcccc 1140
tcatggccaa gcctctgtcc tttctgcagt tctttcatca cttgggagct cctatggaca 1200
tgtggctctt cgtgcagtac tctggcgaat ccatttggat ctttgtgttc ctgaacggat 1260
tcattcactt tgtcatgtac ggctactatt ggacacggct gatgaagttc aactttccca 1320
tgcccaagca gctcattacc gcaatgcaga tcacccagtt caacgttggc ttctacctcg 1380
tgtggtggta caaggacatt ccctgttacc gaaaggatcc catgcgaatg ctggcctgga 1440
tcttcaacta ctggtacgtc ggtaccgttc ttctgctctt catcaacttc tttgtcaagt 1500
cctacgtgtt tcccaagcct aagactgccg acaaaaaggt ccagggcgcc ggtcccgctc 1560
gacctgccgg acttcctccc gctacctact acgactctct ggccgtcatg ggatccgtga 1620
aggcttctcg acaggctctg cccctcgtca tcgacggaaa ggtgtacgac gtctccgctt 1680
gggtgaactt ccaccctggt ggagctgaaa tcattgagaa ctaccaggga cgagatgcta 1740
ctgacgcctt catggttatg cactctcagg aagccttcga caagctcaag cgaatgccca 1800
agatcaacca ggcttccgag ctgcctcccc aggctgccgt caacgaagct caggaggatt 1860
tccgaaagct ccgagaagag ctgatcgcca ctggcatgtt tgacgcctct cccctctggt 1920
actcgtacaa gatcttgacc accctgggtc ttggcgtgct tgccttcttc atgctggtcc 1980
agtaccacct gtacttcatt ggtgctctcg tgctcggtat gcactaccag caaatgggat 2040
ggctgtctca tgacatctgc caccaccaga ccttcaagaa ccgaaactgg aataacgtcc 2100
tgggtctggt ctttggcaac ggactccagg gcttctccgt gacctggtgg aaggacagac 2160
acaacgccca tcattctgct accaacgttc agggtcacga tcccgacatt gataacctgc 2220
ctctgctcgc ctggtccgag gacgatgtca ctcgagcttc tcccatctcc cgaaagctca 2280
ttcagttcca acagtactat ttcctggtca tctgtattct cctgcgattc atctggtgtt 2340

tccagtctgt gctgaccgtt cgatccctca aggaccgaga caaccagttc taccgatctc 2400
agtacaagaa agaggccatt ggactcgctc tgcactggac tctcaagacc ctgttccacc 2460
tcttctttat gccctccatc ctgacctcga tgctggtgtt ctttgtttcc gagctcgtcg 2520
gtggcttcgg aattgccatc gtggtcttca tgaaccacta ccctctggag aagatcggtg 2580
attccgtctg ggacggacat ggcttctctg tgggtcagat ccatgagacc atgaacattc 2640
gacgaggcat cattactgac tggttctttg gaggcctgaa ctaccagatc gagcaccatc 2700
tctggcccac cctgcctcga cacaacctca ctgccgtttc ctaccaggtg gaacagctgt 2760
gccagaagca caacctcccc taccgaaacc ctctgcccca tgaaggtctc gtcatcctgc 2820
tccgatacct gtcccagttc gctcgaatgg ccgagaagca gcccggtgcc aaggctcagt 2880
aagcggccgc atgagaagat aaatatataa atacattgag atattaaatg cgctagatta 2940
gagagcctca tactgctcgg agagaagcca agacgagtac tcaaagggga ttacaccatc 3000
catatccaca gacacaagct ggggaaaggt tctatataca ctttccggaa taccgtagtt 3060
tccgatgtta tcaatggggg cagccaggat ttcaggcact tcggtgtctc ggggtgaaat 3120
ggcgttcttg gcctccatca agtcgtacca tgtcttcatt tgcctgtcaa agtaaaacag 3180
aagcagatga agaatgaact tgaagtgaag gaatttaaat gtaacgaaac tgaaatttga 3240
ccagatattg tgtccgcggt ggagctccag cttttgttcc ctttagtgag ggttaatttc 3300
gagcttggcg taatcatggt catagctgtt tcctgtgtga aattgttatc cgctcacaag 3360
cttccacaca acgtacgata gttagtagac aacaatcaga acatctccct ccttatataa 3420
tcacacaggc cagaacgcgc taaactaaag cgctttggac actatgttac attggcattg 3480
attgaactga aaccacagtc tccctcgcct gaatcgagca atggatgttg tcggaagtca 3540
acttcactag aagagcggtt ctatgccttg tcaagatcat atcataaact cactctgtat 3600
taccccatct atagaacact tgttatgaat gggcggaaac attccgctat atgcaccttt 3660
ccacactaat gcaaagatgt gcatcttcaa cgggtagtaa gactggttcc gacttccgtt 3720
gcatggagag caatgacctc gataatgcga acatccccca catatacact cttacacagg 3780
ccaatataat ctgtgcattt actaaatatt taagtctatg cacctgcttg atgaaaagcg 3840
gcacggatgg tatcatctag tttccgccaa tccaagaacc aactgtgttg gcagtggtgt 3900
agcccatggc acacagacca aagatgaaaa tacagacatc ggcggttcga gccgtggtgc 3960
ctcgagcaac acccttgtaa tgcaaaagag gagggtaaat gtacaccaga ggcacacatg 4020
caaacgatcc ggtgagagcg acgaaccgat cgagatcgtc ggcacctccc catgcaacaa 4080
aggcggtgac aaacacaagg aagaaccgga aaatgttctt ctgccacttg atggtagagt 4140
tgtacttgcc tgatcgggtg aagagaccat tctcgatgat tcggatggcg cgccagctgc 4200
attaatgaat cggccaacgc gcggggagag gcggtttgcg tattgggcgc tcttccgctt 4260

cctcgctcac tgactcgctg cgctcggtcg ttcggctgcg gcgagcggta tcagctcact 4320
caaaggcggt aatacggtta tccacagaat caggggataa cgcaggaaag aacatgtgag 4380
caaaaggcca gcaaaaggcc aggaaccgta aaaaggccgc gttgctggcg tttttccata 4440
ggctccgccc ccctgacgag catcacaaaa atcgacgctc aagtcagagg tggcgaaacc 4500
cgacaggact ataaagatac caggcgtttc cccctggaag ctccctcgtg cgctctcctg 4560
ttccgaccct gccgcttacc ggatacctgt ccgcctttct cccttcggga agcgtggcgc 4620
tttctcatag ctcacgctgt aggtatctca gttcggtgta ggtcgttcgc tccaagctgg 4680
gctgtgtgca cgaacccccc gttcagcccg accgctgcgc cttatccggt aactatcgtc 4740
ttgagtccaa cccggtaaga cacgacttat cgccactggc agcagccact ggtaacagga 4800
ttagcagagc gaggtatgta ggcggtgcta cagagttctt gaagtggtgg cctaactacg 4860
gctacactag aagaacagta tttggtatct gcgctctgct gaagccagtt accttcggaa 4920
aaagagttgg tagctcttga tccggcaaac aaaccaccgc tggtagcggt ggtttttttg 4980
tttgcaagca gcagattacg cgcagaaaaa aaggatctca agaagatcct ttgatctttt 5040
ctacggggtc tgacgctcag tggaacgaaa actcacgtta agggattttg gtcatgagat 5100
tatcaaaaag gatcttcacc tagatccttt taaattaaaa atgaagtttt aaatcaatct 5160
aaagtatata tgagtaaact tggtctgaca gttaccaatg cttaatcagt gaggcaccta 5220
tctcagcgat ctgtctattt cgttcatcca tagttgcctg actccccgtc gtgtagataa 5280
ctacgatacg ggagggctta ccatctggcc ccagtgctgc aatgataccg cgagacccac 5340
gctcaccggc tccagattta tcagcaataa accagccagc cggaagggcc gagcgcagaa 5400
gtggtcctgc aactttatcc gcctccatcc agtctattaa ttgttgccgg gaagctagag 5460
taagtagttc gccagttaat agtttgcgca acgttgttgc cattgctaca ggcatcgtgg 5520
tgtcacgctc gtcgtttggt atggcttcat tcagctccgg ttcccaacga tcaaggcgag 5580
ttacatgatc ccccatgttg tgcaaaaaag cggttagctc cttcggtcct ccgatcgttg 5640
tcagaagtaa gttggccgca gtgttatcac tcatggttat ggcagcactg cataattctc 5700
ttactgtcat gccatccgta agatgctttt ctgtgactgg tgagtactca accaagtcat 5760
tctgagaata gtgtatgcgg cgaccgagtt gctcttgccc ggcgtcaata cgggataata 5820
ccgcgccaca tagcagaact ttaaaagtgc tcatcattgg aaaacgttct tcggggcgaa 5880
aactctcaag gatcttaccg ctgttgagat ccagttcgat gtaacccact cgtgcaccca 5940
actgatcttc agcatctttt actttcacca gcgtttctgg gtgagcaaaa acaggaaggc 6000
aaaatgccgc aaaaaaggga ataagggcga cacggaaatg ttgaatactc atactcttcc 6060
tttttcaata ttattgaagc atttatcagg gttattgtct catgagcgga tacatatttg 6120
aatgtattta gaaaaataaa caaatagggg ttccgcgcac atttccccga aaagtgccac 6180
ctgatgcggt gtgaaatacc gcacagatgc gtaaggagaa aataccgcat caggaaattg 6240

taagcgttaa tattttgtta aaattcgcgt taaatttttg ttaaatcagc tcatttttta 6300

accaataggc cgaaatcggc aaaatccctt ataaatcaaa agaatagacc gagatagggt 6360

tgagtgttgt tccagtttgg aacaagagtc cactattaaa gaacgtggac tccaacgtca 6420

aagggcgaaa aaccgtctat cagggcgatg gcccactacg tgaaccatca ccctaatcaa 6480

gtttttttggg gtcgaggtgc cgtaaagcac taaatcggaa ccctaaaggg agcccccgat 6540

ttagagcttg acggggaaag ccggcgaacg tggcgagaaa ggaagggaag aaagcgaaag 6600

gagcgggcgc tagggcgctg gcaagtgtag cggtcacgct gcgcgtaacc accacacccg 6660

ccgcgcttaa tgcgccgcta cagggcgcgt ccattcgcca ttcaggctgc gcaactgttg 6720

ggaagggcga tcggtgcggg cctcttcgct attacgccag ctggcgaaag ggggatgtgc 6780

tgcaaggcga ttaagttggg taacgccagg gttttcccag tcacgacgtt gtaaaacgac 6840

ggccagtgaa ttgtaatacg actcactata gggcgaattg ggcccgacgt cgcatgctta 6900

gaagtgagga ttacaagaag cctctggata tcaatgatga acgtactcag cggctggtca 6960

agcatttcga ccgtcgaatc gacgaggtgt tcacctttga caagcgaggg ttcccaattg 7020

atcacgttct cgagttgttc aaatcttctc tcaacatctc tctgcatgaa ctatctctgt 7080

tgacgaacgt gtcacccact gttcctcgaa cgcccttctc cgagtttggt ctgaacatct 7140

tcgatctcaa actgaccccc gcagtgatca atagtgccat gccactgccg atgcggtgcg 7200

aacatccctg gagggattct cggagctcta cacaatgcag attctgtcgt cgagtactct 7260

ctaccttgct cgaatgactt attgtgctac tactgcactc atgcttcgat catgtgccct 7320

actgcacccc aaatttggtg atctgattga gacagagtac cctcttcagc tgattcagaa 7380

gatcatcagc aacatgaatg atgtggttga ccaggcaggc tgttgtagtc acgtccttca 7440

cttcaagttc attcttcatc tgcttctgtt ttactttgac aggcaaatga agacatggta 7500

cgacttgatg gaggccaaga acgccatttc accccgagac accgaagtgc ctgaaatcct 7560

ggctgccccc attgataaca tcggaaacta cggtattccg gaaagtgtat atagaacctt 7620

tccccagctt gtgtctgtgg atatggatgg tgtaatcccc ttaattaact cacctgcagg 7680

attgagacta tgaatggatt cccgtgcccg tattactcta ctaatttgat cttggaacgc 7740

gaaaatacgt ttctaggact ccaaagaatc tcaactcttg tccttactaa atatactacc 7800

catagttgat ggtttacttg aacagagagg acatgttcac ttgacccaaa gtttctcgca 7860

tctcttggat atttgaacaa cggcgtccac tgaccgtcag ttatccagtc acaaaacccc 7920

cacattcata cattcccatg tacgtttaca aagttctcaa ttccatcgtg caaatcaaaa 7980

tcacatctat tcattcatca tatataaacc catcatgtct actaacactc acaactccat 8040

agaaaacatc gactcagaac acacgctcca tgcggccgct taggcaacgg gcttgatgac 8100

agcgggagga gtgcccacat tgtttcggtt tcgaaagaac aggacaccct tgccagctcc 8160

ctcggcacca gcggagggtt caacccactg gcacattcgt gcagatcggt acatggctcg 8220
aatgaatcct cgaggaccgt cctggacatc agctcgatag tgcttgccca tgataggttt 8280
gatggcctcg gtagcttcgt ccgcattgta gaagggaatg gaagagacgt agtgatgcag 8340
gacgtgagtc tcgataatgc cgtggagcag atgacgtcca atgaagccca tctctcggtc 8400
gatggttgca gcggcacctc gcacaaagtt ccactcgtcg ttggtgtagt ggggaagagt 8460
aggatctgtg tgctgcagaa aggtaatggc gacgagccag tggttaaccc acaagtaggg 8520
aacgaagtac cagatggcca tgttgtagaa tccgaacttc tgaacgagaa agtacagagc 8580
ggtggccata agaccaatgc caatgtcgga gagcacgatg agcttggcgt cgctgttctc 8640
gtacagagga gatcggggat cgaaatggtt aactccaccg ccaagaccgt tgtgctttcc 8700
cttgcctcga ccctctcgct gccgctcatg gtagttgtgt ccagtaacgt tggtaatgag 8760
atagttgggc caaccgacca gttgctgaag cacaagcatg agcagggtga aagcaggagt 8820
ttcctcggta agatgggcga gttcgtgggt catcttgccg agtcgagtag cttgctgctc 8880
tcgggttcga ggaacgaaga ccatgtctcg ctccatgttt ccagtggcct tgtgatgctt 8940
ccggtgggag atttgccagc tgaagtaggg aacaagcagg gaagagtgaa gcacccagcc 9000
agtaatgtcg ttgatgattc gggaatcgga gaaagcacca tgtccacact cgtgggcaat 9060
gacccacagt ccagtaccga agagtccctg aagaacggtg tacacagccc acagaccggc 9120
tcgagcagga gtggagggaa tgtactcggg tgtcacaaag ttgtaccaga tgctgaaagt 9180
ggtagtcagg aggacaatgt ctcgaagaat gtagccgtat cccttgagag cagatcgctt 9240
gaagcagtgc ttgggaatag cgttgtagat gtccttgatg gtgaagtcgg gaacttcgaa 9300
ctggttgccg taggtatcca gcatgacacc gtactcggac ttgggcttgg caatgtccac 9360
ctcggacatg gaagacagcg atgtagagga ggccgagtgt ctgggagaat cggagggaga 9420
gacggcagca gactccgagt cggtcacagt ggtggaagtg acggttcgtc ggagggcagg 9480
gttctgcttg ggcagagccg aggtggaggc catggccatt gctgtagata tgtcttgtgt 9540
gtaagggggt tggggtggtt gtttgtgttc ttgacttttg tgttagcaag ggaagacggg 9600
caaaaaagtg agtgtggttg ggagggagag acgagcctta tatataatgc ttgtttgtgt 9660
ttgtgcaagt ggacgccgaa acgggcagga gccaaactaa acaaggcaga caatgcgagc 9720
ttaattggat tgcctgatgg gcaggggtta gggctcgatc aatgggggtg cgaagtgaca 9780
aaattgggaa ttaggttcgc aagcaaggct gacaagactt tggcccaaac atttgtacgc 9840
ggtggacaac aggagccacc catcgtctgt cacgggctag ccggtcgtgc gtcctgtcag 9900
gctccaccta ggctccatgc cactccatac aatcccacta gtgtaccgct aggccgcttt 9960
tagctcccat ctaagacccc cccaaaacct ccactgtaca gtgcactgta ctgtgtggcg 10020
atcaagggca agggaaaaaa ggcgcaaaca tgcacgcatg gaatgacgta ggtaaggcgt 10080
tactagactg aaaagtggca catttcggcg tgccaaaggg tcctaggtgc gtttcgcgag 10140

ctgggcgcca ggccaagccg ctccaaaacg cctctccgac tccctccagc ggcctccata 10200

tccccatccc tctccacagc aatgttgtta agccttgcaa acgaaaaaat agaaaggcta 10260

ataagcttcc aatattgtgg tgtacgctgc ataacgcaac aatgagcgcc aaacaacaca 10320

cacacacagc acacagcagc attaaccacg atgaacagca tgaattctct ctcttgagct 10380

tttccataac aagttcttct gcctccagga agtccatggg tggtttgatc atggttttgg 10440

tgtagtggta gtgcagtggt ggtattgtga ctggggatgt agttgagaat aagtcataca 10500

caagtcagct ttcttcgagc ctcatataag tataagtagt tcaacgtatt agcactgtac 10560

ccagcatctc cgtatcgaga aacacaacaa catgccccat tggacagatc atgcggatac 10620

acaggttgtg cagtatcata catactcgat cagacaggtc gtctgaccat catacaagct 10680

gaacaagcgc tccatacttg cacgctctct atatacacag ttaaattaca tatccatagt 10740

ctaacctcta acagttaatc ttctggtaag cctcccagcc agccttctgg tatcgcttgg 10800

cctcctcaat aggatctcgg ttctggccgt acagacctcg gccgacaatt atgatatccg 10860

ttccggtaga catgacatcc tcaacagttc ggtactgctg tccgagagcg tctcccttgt 10920

cgtcaagacc caccccgggg gtcagaataa gccagtcctc agagtcgccc ttaggtcggt 10980

tctgggcaat gaagccaacc acaaactcgg ggtcggatcg ggcaagctca atggtctgct 11040

tggagtactc gccagtggcc agagagccct tgcaagacag ctcggccagc atgagcagac 11100

ctctggccag cttctcgttg ggagagggga ctaggaactc cttgtactgg gagttctcgt 11160

agtcagagac gtcctccttc ttctgttcag agacagtttc ctcggcacca gctcgcaggc 11220

cagcaatgat tccggttccg ggtacaccgt gggcgttggt gatatcggac cactcggcga 11280

ttcggtgaca ccggtactgg tgcttgacag tgttgccaat atctgcgaac tttctgtcct 11340

cgaacaggaa gaaaccgtgc ttaagagcaa gttccttgag ggggagcaca gtgccggcgt 11400

aggtgaagtc gtcaatgatg tcgatatggg ttttgatcat gcacacataa ggtccgacct 11460

tatcggcaag ctcaatgagc tccttggtgg tggtaacatc cagagaagca cacaggttgg 11520

ttttcttggc tgccacgagc ttgagcactc gagcggcaaa ggcggacttg tggacgttag 11580

ctcgagcttc gtaggagggc attttggtgg tgaagaggag actgaaataa atttagtctg 11640

cagaactttt tatcggaacc ttatctgggg cagtgaagta tatgttatgg taatagttac 11700

gagttagttg aacttataga tagactggac tatacggcta tcggtccaaa ttagaaagaa 11760

cgtcaatggc tctctgggcg tcgcctttgc cgacaaaaat gtgatcatga tgaaagccag 11820

caatgacgtt gcagctgata ttgttgtcgg ccaaccgcgc cgaaaacgca gctgtcagac 11880

ccacagcctc caacgaagaa tgtatcgtca aagtgatcca agcacactca tagttggagt 11940

cgtactccaa aggcggcaat gacgagtcag acagatactc gtcgaccatt aattctcacg 12000

tgacacagat tattaacgtc tcgtaccaac cacagattac gacccattcg cagtcacagt 12060

tcactagggt ttgggttgca tccgttgaga gcggtttgtt tttaaccttc tccatgtgct 12120
cactcaggtt ttgggttcag atcaaatcaa ggcgtgaacc actttgtttg aggacaaatg 12180
tgacacaacc aaccagtgtc aggggcaagt ccgtgacaaa ggggaagata caatgcaatt 12240
actgacagtt acagactgcc tcgatgccct aaccttgccc caaaataaga caactgtcct 12300
cgtttaagcg caaccctatt cagcgtcacg tcataatagc gtttggatag cactagtcta 12360
tgaggagcgt tttatgttgc ggtgagggcg attggtgctc atatgggttc aattgaggtg 12420
gcggaacgag cttagtcttc aattgaggtg cgagcgacac aattgggtgt cacgtggcct 12480
aattgacctc gggtcgtgga gtccccagtt atacagcaac cacgaggtgc atgggtagga 12540
gacgtcacca gacaataggg ttttttttgg actggagagg gttgggcaaa agcgctcaac 12600
gggctgtttg gggagctgtg ggggaggaat tggcgatatt tgtgaggtta acggctccga 12660
tttgcgtgtt ttgtcgctcc tgcatctccc catacccata tcttccctcc ccacctcttt 12720
ccacgataat tttacggatc agcaataagg ttccttctcc tagtttccac gtccatatat 12780
atctatgctg cgtcgtcctt ttcgtgacat caccaaaaca catacaacca tggctctctc 12840
ccttactacc gagcagctgc tcgagcgacc cgacctggtt gccatcgacg gcattctcta 12900
cgatctggaa ggtcttgcca aggtccatcc cggatccgac ttgatcctcg cttctggtgc 12960
ctccgatgct tctcctctgt tctactccat gcacccttac gtcaagcccg agaactcgaa 13020
gctgcttcaa cagttcgtgc gaggcaagca cgaccgaacc tccaaggaca ttgtctacac 13080
ctacgactct ccctttgcac aggacgtcaa gcgaactatg cgagaggtca tgaaaggtcg 13140
gaactggtat gccacacctg gattctggct gcgaaccgtt ggcatcattg ctgtcaccgc 13200
cttttgcgag tggcactggg ctactaccgg aatggtgctg tggggtctct tgactggatt 13260
catgcacatg cagatcggcc tgtccattca gcacgatgcc tctcatggtg ccatcagcaa 13320
aaagccctgg gtcaacgctc tctttgccta cggcatcgac gtcattggat cgtccagatg 13380
gatctggctg cagtctcaca tcatgcgaca tcacacctac accaatcagc atggtctcga 13440
cctggatgcc gagtccgcag aaccattcct tgtgttccac aactaccctg ctgccaacac 13500
tgctcgaaag tggtttcacc gattccaggc ctggtacatg tacctcgtgc ttggagccta 13560
cggcgtttcg ctggtgtaca accctctcta catcttccga atgcagcaca acgacaccat 13620
tcccgagtct gtcacagcca tgcgagagaa cggctttctg cgacggtacc gaacccttgc 13680
attcgttatg cgagctttct tcatctttcg aaccgccttc ttgccctggt atctcactgg 13740
aacctccctg ctcatcacca ttcctctggt gcccactgct accggtgcct tcctcacctt 13800
ctttttcatc ttgtctcaca acttcgatgg ctcggagcga atccccgaca agaactgcaa 13860
ggtcaagagc tccgagaagg acgttgaagc cgatcagatc gactggtaca gagctcaggt 13920
ggagacctct tccacctacg gtggacccat tgccatgttc tttactggcg gtctcaactt 13980
ccagatcgag catcacctct ttcctcgaat gtcgtcttgg cactatccct tcgtgcagca 14040

agctgtccga gagtgttgcg aacgacacgg agttcggtac gtcttctacc ctaccattgt 14100

gggcaacatc atttccaccc tcaagtacat gcacaaagtc ggtgtggttc actgtgtcaa 14160

ggacgctcag gattcctaag cggccgcatg tacatacaag attatttata gaaatgaatc 14220

gcgatcgaac aaagagtacg agtgtacgag taggggatga tgataaaagt ggaagaagtt 14280

ccgcatcttt ggatttatca acgtgtagga cgatacttcc tgtaaaaatg caatgtcttt 14340

accataggtt ctgctgtaga tgttattaac taccattaac atgtctactt gtacagttgc 14400

agaccagttg gagtatagaa tggtacactt accaaaaagt gttgatggtt gtaactacga 14460

tatataaaac tgttgacggg atccccgctg atatgcctaa ggaacaatca aagaggaaga 14520

tattaattca gaatgctagt atacagttag ggat 14554

<210> 49
<211> 2127
<212> DNA
<213> Artificial Sequence

<220>
<223> Multizyme: E389D9eS/EgD8M gene fusion

<220>
<221> CDS
<222> (1)..(2127)
<223> Multizyme: E389D9eS/EgD8M gene fusion

<300>
<302> MULTIZYMES AND THEIR USE IN MAKING POLYUNSATURATED FATTY ACIDS
<310> US-2008-0254191-A1
<311> 2008-04-03
<312> 2008-10-16
<313> (1)..(2127)

<300>
<302> MULTIZYMES AND THEIR USE IN MAKING POLYUNSATURATED FATTY ACIDS
<310> WO 2008/124048
<311> 2008-04-03
<312> 2008-10-16
<313> (1)..(2127)

<400> 49

```
atg gct gcc gtc atc gag gtg gcc aac gag ttc gtc gct atc act gcc          48
Met Ala Ala Val Ile Glu Val Ala Asn Glu Phe Val Ala Ile Thr Ala
1               5                   10                  15

gag acc ctt ccc aag gtg gac tat cag cga ctc tgg cga gac atc tac          96
Glu Thr Leu Pro Lys Val Asp Tyr Gln Arg Leu Trp Arg Asp Ile Tyr
            20                  25                  30

tcc tgc gag ctc ctg tac ttc tcc att gct ttc gtc atc ctc aag ttt         144
Ser Cys Glu Leu Leu Tyr Phe Ser Ile Ala Phe Val Ile Leu Lys Phe
        35                  40                  45

acc ctt ggc gag ctc tcg gat tct ggc aaa aag att ctg cga gtg ctg         192
Thr Leu Gly Glu Leu Ser Asp Ser Gly Lys Lys Ile Leu Arg Val Leu
    50                  55                  60
```

```
ttc aag tgg tac aac ctc ttc atg tcc gtc ttt tcg ctg gtg tcc ttc        240
Phe Lys Trp Tyr Asn Leu Phe Met Ser Val Phe Ser Leu Val Ser Phe
65                  70                  75                  80

ctc tgt atg ggt tac gcc atc tac acc gtt gga ctg tac tcc aac gaa        288
Leu Cys Met Gly Tyr Ala Ile Tyr Thr Val Gly Leu Tyr Ser Asn Glu
                85                  90                  95

tgc gac aga gct ttc gac aac agc ttg ttc cga ttt gcc acc aag gtc        336
Cys Asp Arg Ala Phe Asp Asn Ser Leu Phe Arg Phe Ala Thr Lys Val
            100                 105                 110

ttc tac tat tcc aag ttt ctg gag tac atc gac tct ttc tac ctt ccc        384
Phe Tyr Tyr Ser Lys Phe Leu Glu Tyr Ile Asp Ser Phe Tyr Leu Pro
        115                 120                 125

ctc atg gcc aag cct ctg tcc ttt ctg cag ttc ttt cat cac ttg gga        432
Leu Met Ala Lys Pro Leu Ser Phe Leu Gln Phe Phe His His Leu Gly
    130                 135                 140

gct cct atg gac atg tgg ctc ttc gtg cag tac tct ggc gaa tcc att        480
Ala Pro Met Asp Met Trp Leu Phe Val Gln Tyr Ser Gly Glu Ser Ile
145                 150                 155                 160

tgg atc ttt gtg ttc ctg aac gga ttc att cac ttt gtc atg tac ggc        528
Trp Ile Phe Val Phe Leu Asn Gly Phe Ile His Phe Val Met Tyr Gly
                165                 170                 175

tac tat tgg aca cgg ctg atg aag ttc aac ttt ccc atg ccc aag cag        576
Tyr Tyr Trp Thr Arg Leu Met Lys Phe Asn Phe Pro Met Pro Lys Gln
            180                 185                 190

ctc att acc gca atg cag atc acc cag ttc aac gtt ggc ttc tac ctc        624
Leu Ile Thr Ala Met Gln Ile Thr Gln Phe Asn Val Gly Phe Tyr Leu
        195                 200                 205

gtg tgg tgg tac aag gac att ccc tgt tac cga aag gat ccc atg cga        672
Val Trp Trp Tyr Lys Asp Ile Pro Cys Tyr Arg Lys Asp Pro Met Arg
    210                 215                 220

atg ctg gcc tgg atc ttc aac tac tgg tac gtc ggt acc gtt ctt ctg        720
Met Leu Ala Trp Ile Phe Asn Tyr Trp Tyr Val Gly Thr Val Leu Leu
225                 230                 235                 240

ctc ttc atc aac ttc ttt gtc aag tcc tac gtg ttt ccc aag cct aag        768
Leu Phe Ile Asn Phe Phe Val Lys Ser Tyr Val Phe Pro Lys Pro Lys
                245                 250                 255

act gcc gac aaa aag gtc cag ggc gcc ggt ccc gct cga cct gcc gga        816
Thr Ala Asp Lys Lys Val Gln Gly Ala Gly Pro Ala Arg Pro Ala Gly
            260                 265                 270

ctt cct ccc gct acc tac tac gac tct ctg gcc gtc atg gga tcc gtg        864
Leu Pro Pro Ala Thr Tyr Tyr Asp Ser Leu Ala Val Met Gly Ser Val
        275                 280                 285

aag gct tct cga cag gct ctg ccc ctc gtc atc gac gga aag gtg tac        912
Lys Ala Ser Arg Gln Ala Leu Pro Leu Val Ile Asp Gly Lys Val Tyr
    290                 295                 300

gac gtc tcc gct tgg gtg aac ttc cac cct ggt gga gct gaa atc att        960
Asp Val Ser Ala Trp Val Asn Phe His Pro Gly Gly Ala Glu Ile Ile
305                 310                 315                 320

gag aac tac cag gga cga gat gct act gac gcc ttc atg gtt atg cac       1008
```

```
Glu Asn Tyr Gln Gly Arg Asp Ala Thr Asp Ala Phe Met Val Met His
                325                 330                 335

tct cag gaa gcc ttc gac aag ctc aag cga atg ccc aag atc aac cag        1056
Ser Gln Glu Ala Phe Asp Lys Leu Lys Arg Met Pro Lys Ile Asn Gln
            340                 345                 350

gct tcc gag ctg cct ccc cag gct gcc gtc aac gaa gct cag gag gat        1104
Ala Ser Glu Leu Pro Pro Gln Ala Ala Val Asn Glu Ala Gln Glu Asp
        355                 360                 365

ttc cga aag ctc cga gaa gag ctg atc gcc act ggc atg ttt gac gcc        1152
Phe Arg Lys Leu Arg Glu Glu Leu Ile Ala Thr Gly Met Phe Asp Ala
    370                 375                 380

tct ccc ctc tgg tac tcg tac aag atc ttg acc acc ctg ggt ctt ggc        1200
Ser Pro Leu Trp Tyr Ser Tyr Lys Ile Leu Thr Thr Leu Gly Leu Gly
385                 390                 395                 400

gtg ctt gcc ttc ttc atg ctg gtc cag tac cac ctg tac ttc att ggt        1248
Val Leu Ala Phe Phe Met Leu Val Gln Tyr His Leu Tyr Phe Ile Gly
                405                 410                 415

gct ctc gtg ctc ggt atg cac tac cag caa atg gga tgg ctg tct cat        1296
Ala Leu Val Leu Gly Met His Tyr Gln Gln Met Gly Trp Leu Ser His
            420                 425                 430

gac atc tgc cac cac cag acc ttc aag aac cga aac tgg aat aac gtc        1344
Asp Ile Cys His His Gln Thr Phe Lys Asn Arg Asn Trp Asn Asn Val
        435                 440                 445

ctg ggt ctg gtc ttt ggc aac gga ctc cag ggc ttc tcc gtg acc tgg        1392
Leu Gly Leu Val Phe Gly Asn Gly Leu Gln Gly Phe Ser Val Thr Trp
    450                 455                 460

tgg aag gac aga cac aac gcc cat cat tct gct acc aac gtt cag ggt        1440
Trp Lys Asp Arg His Asn Ala His His Ser Ala Thr Asn Val Gln Gly
465                 470                 475                 480

cac gat ccc gac att gat aac ctg cct ctg ctc gcc tgg tcc gag gac        1488
His Asp Pro Asp Ile Asp Asn Leu Pro Leu Leu Ala Trp Ser Glu Asp
                485                 490                 495

gat gtc act cga gct tct ccc atc tcc cga aag ctc att cag ttc caa        1536
Asp Val Thr Arg Ala Ser Pro Ile Ser Arg Lys Leu Ile Gln Phe Gln
            500                 505                 510

cag tac tat ttc ctg gtc atc tgt att ctc ctg cga ttc atc tgg tgt        1584
Gln Tyr Tyr Phe Leu Val Ile Cys Ile Leu Leu Arg Phe Ile Trp Cys
        515                 520                 525

ttc cag tct gtg ctg acc gtt cga tcc ctc aag gac cga gac aac cag        1632
Phe Gln Ser Val Leu Thr Val Arg Ser Leu Lys Asp Arg Asp Asn Gln
    530                 535                 540

ttc tac cga tct cag tac aag aaa gag gcc att gga ctc gct ctg cac        1680
Phe Tyr Arg Ser Gln Tyr Lys Lys Glu Ala Ile Gly Leu Ala Leu His
545                 550                 555                 560

tgg act ctc aag acc ctg ttc cac ctc ttc ttt atg ccc tcc atc ctg        1728
Trp Thr Leu Lys Thr Leu Phe His Leu Phe Phe Met Pro Ser Ile Leu
                565                 570                 575

acc tcg atg ctg gtg ttc ttt gtt tcc gag ctc gtc ggt ggc ttc gga        1776
Thr Ser Met Leu Val Phe Phe Val Ser Glu Leu Val Gly Gly Phe Gly
```

```
                580                 585                 590

att gcc atc gtg gtc ttc atg aac cac tac cct ctg gag aag atc ggt      1824
Ile Ala Ile Val Val Phe Met Asn His Tyr Pro Leu Glu Lys Ile Gly
        595                 600                 605

gat tcc gtc tgg gac gga cat ggc ttc tct gtg ggt cag atc cat gag      1872
Asp Ser Val Trp Asp Gly His Gly Phe Ser Val Gly Gln Ile His Glu
    610                 615                 620

acc atg aac att cga cga ggc atc att act gac tgg ttc ttt gga ggc      1920
Thr Met Asn Ile Arg Arg Gly Ile Ile Thr Asp Trp Phe Phe Gly Gly
625                 630                 635                 640

ctg aac tac cag atc gag cac cat ctc tgg ccc acc ctg cct cga cac      1968
Leu Asn Tyr Gln Ile Glu His His Leu Trp Pro Thr Leu Pro Arg His
                645                 650                 655

aac ctc act gcc gtt tcc tac cag gtg gaa cag ctg tgc cag aag cac      2016
Asn Leu Thr Ala Val Ser Tyr Gln Val Glu Gln Leu Cys Gln Lys His
                660                 665                 670

aac ctc ccc tac cga aac cct ctg ccc cat gaa ggt ctc gtc atc ctg      2064
Asn Leu Pro Tyr Arg Asn Pro Leu Pro His Glu Gly Leu Val Ile Leu
        675                 680                 685

ctc cga tac ctg tcc cag ttc gct cga atg gcc gag aag cag ccc ggt      2112
Leu Arg Tyr Leu Ser Gln Phe Ala Arg Met Ala Glu Lys Gln Pro Gly
    690                 695                 700

gcc aag gct cag taa                                                  2127
Ala Lys Ala Gln
705
```

<210> 50
<211> 708
<212> PRT
<213> Artificial Sequence

<220>
<223> Synthetic Construct

<400> 50

```
Met Ala Ala Val Ile Glu Val Ala Asn Glu Phe Val Ala Ile Thr Ala
1               5                   10                  15

Glu Thr Leu Pro Lys Val Asp Tyr Gln Arg Leu Trp Arg Asp Ile Tyr
            20                  25                  30

Ser Cys Glu Leu Leu Tyr Phe Ser Ile Ala Phe Val Ile Leu Lys Phe
        35                  40                  45

Thr Leu Gly Glu Leu Ser Asp Ser Gly Lys Lys Ile Leu Arg Val Leu
    50                  55                  60

Phe Lys Trp Tyr Asn Leu Phe Met Ser Val Phe Ser Leu Val Ser Phe
65                  70                  75                  80
```

```
Leu Cys Met Gly Tyr Ala Ile Tyr Thr Val Gly Leu Tyr Ser Asn Glu
                85                  90                  95

Cys Asp Arg Ala Phe Asp Asn Ser Leu Phe Arg Phe Ala Thr Lys Val
            100                 105                 110

Phe Tyr Tyr Ser Lys Phe Leu Glu Tyr Ile Asp Ser Phe Tyr Leu Pro
        115                 120                 125

Leu Met Ala Lys Pro Leu Ser Phe Leu Gln Phe Phe His His Leu Gly
    130                 135                 140

Ala Pro Met Asp Met Trp Leu Phe Val Gln Tyr Ser Gly Glu Ser Ile
145                 150                 155                 160

Trp Ile Phe Val Phe Leu Asn Gly Phe Ile His Phe Val Met Tyr Gly
                165                 170                 175

Tyr Tyr Trp Thr Arg Leu Met Lys Phe Asn Phe Pro Met Pro Lys Gln
            180                 185                 190

Leu Ile Thr Ala Met Gln Ile Thr Gln Phe Asn Val Gly Phe Tyr Leu
        195                 200                 205

Val Trp Trp Tyr Lys Asp Ile Pro Cys Tyr Arg Lys Asp Pro Met Arg
    210                 215                 220

Met Leu Ala Trp Ile Phe Asn Tyr Trp Tyr Val Gly Thr Val Leu Leu
225                 230                 235                 240

Leu Phe Ile Asn Phe Phe Val Lys Ser Tyr Val Phe Pro Lys Pro Lys
                245                 250                 255

Thr Ala Asp Lys Lys Val Gln Gly Ala Gly Pro Ala Arg Pro Ala Gly
            260                 265                 270

Leu Pro Pro Ala Thr Tyr Tyr Asp Ser Leu Ala Val Met Gly Ser Val
        275                 280                 285

Lys Ala Ser Arg Gln Ala Leu Pro Leu Val Ile Asp Gly Lys Val Tyr
    290                 295                 300

Asp Val Ser Ala Trp Val Asn Phe His Pro Gly Gly Ala Glu Ile Ile
305                 310                 315                 320

Glu Asn Tyr Gln Gly Arg Asp Ala Thr Asp Ala Phe Met Val Met His
                325                 330                 335

Ser Gln Glu Ala Phe Asp Lys Leu Lys Arg Met Pro Lys Ile Asn Gln
```

340 345 350

Ala Ser Glu Leu Pro Pro Gln Ala Ala Val Asn Glu Ala Gln Glu Asp
355 360 365

Phe Arg Lys Leu Arg Glu Glu Leu Ile Ala Thr Gly Met Phe Asp Ala
370 375 380

Ser Pro Leu Trp Tyr Ser Tyr Lys Ile Leu Thr Thr Leu Gly Leu Gly
385 390 395 400

Val Leu Ala Phe Phe Met Leu Val Gln Tyr His Leu Tyr Phe Ile Gly
405 410 415

Ala Leu Val Leu Gly Met His Tyr Gln Gln Met Gly Trp Leu Ser His
420 425 430

Asp Ile Cys His His Gln Thr Phe Lys Asn Arg Asn Trp Asn Asn Val
435 440 445

Leu Gly Leu Val Phe Gly Asn Gly Leu Gln Gly Phe Ser Val Thr Trp
450 455 460

Trp Lys Asp Arg His Asn Ala His His Ser Ala Thr Asn Val Gln Gly
465 470 475 480

His Asp Pro Asp Ile Asp Asn Leu Pro Leu Leu Ala Trp Ser Glu Asp
485 490 495

Asp Val Thr Arg Ala Ser Pro Ile Ser Arg Lys Leu Ile Gln Phe Gln
500 505 510

Gln Tyr Tyr Phe Leu Val Ile Cys Ile Leu Leu Arg Phe Ile Trp Cys
515 520 525

Phe Gln Ser Val Leu Thr Val Arg Ser Leu Lys Asp Arg Asp Asn Gln
530 535 540

Phe Tyr Arg Ser Gln Tyr Lys Lys Glu Ala Ile Gly Leu Ala Leu His
545 550 555 560

Trp Thr Leu Lys Thr Leu Phe His Leu Phe Phe Met Pro Ser Ile Leu
565 570 575

Thr Ser Met Leu Val Phe Phe Val Ser Glu Leu Val Gly Gly Phe Gly
580 585 590

Ile Ala Ile Val Val Phe Met Asn His Tyr Pro Leu Glu Lys Ile Gly
595 600 605

Asp Ser Val Trp Asp Gly His Gly Phe Ser Val Gly Gln Ile His Glu
610 615 620

Thr Met Asn Ile Arg Arg Gly Ile Ile Thr Asp Trp Phe Phe Gly Gly
625 630 635 640

Leu Asn Tyr Gln Ile Glu His His Leu Trp Pro Thr Leu Pro Arg His
645 650 655

Asn Leu Thr Ala Val Ser Tyr Gln Val Glu Gln Leu Cys Gln Lys His
660 665 670

Asn Leu Pro Tyr Arg Asn Pro Leu Pro His Glu Gly Leu Val Ile Leu
675 680 685

Leu Arg Tyr Leu Ser Gln Phe Ala Arg Met Ala Glu Lys Gln Pro Gly
690 695 700

Ala Lys Ala Gln
705

<210> 51
<211> 1434
<212> DNA
<213> Fusarium moniliforme

<220>
<221> CDS
<222> (1)..(1434)
<223> synthetic delta-12 desaturase (codon-optimized for Yarrowia lipolytica)

<300>
<302> DELTA-12 DESATURASES SUITABLE FOR ALTERING LEVELS OF POLYUNSATURATED FATTY ACIDS IN OLEAGINOUS YEAST
<310> WO 2005/047485
<311> 2004-11-12
<312> 2005-05-26
<313> (1)..(1434)

<300>
<302> DELTA-12 DESATURASES SUITABLE FOR ALTERING LEVELS OF POLYUNSATURATED FATTY ACIDS IN OLEAGINOUS YEAST
<310> US 2005-0216975-A1
<311> 2004-11-10
<312> 2005-09-29
<313> (1)..(1434)

<400> 51

```
atg gcc tcc acc tcg gct ctg ccc aag cag aac cct gcc ctc cga cga          48
Met Ala Ser Thr Ser Ala Leu Pro Lys Gln Asn Pro Ala Leu Arg Arg
1               5                   10                  15

acc gtc act tcc acc act gtg acc gac tcg gag tct gct gcc gtc tct          96
Thr Val Thr Ser Thr Thr Val Thr Asp Ser Glu Ser Ala Ala Val Ser
```

```
            20                  25                  30

ccc tcc gat tct ccc aga cac tcg gcc tcc tct aca tcg ctg tct tcc          144
Pro Ser Asp Ser Pro Arg His Ser Ala Ser Ser Thr Ser Leu Ser Ser
        35                  40                  45

atg tcc gag gtg gac att gcc aag ccc aag tcc gag tac ggt gtc atg          192
Met Ser Glu Val Asp Ile Ala Lys Pro Lys Ser Glu Tyr Gly Val Met
    50                  55                  60

ctg gat acc tac ggc aac cag ttc gaa gtt ccc gac ttc acc atc aag          240
Leu Asp Thr Tyr Gly Asn Gln Phe Glu Val Pro Asp Phe Thr Ile Lys
65                  70                  75                  80

gac atc tac aac gct att ccc aag cac tgc ttc aag cga tct gct ctc          288
Asp Ile Tyr Asn Ala Ile Pro Lys His Cys Phe Lys Arg Ser Ala Leu
                85                  90                  95

aag gga tac ggc tac att ctt cga gac att gtc ctc ctg act acc act          336
Lys Gly Tyr Gly Tyr Ile Leu Arg Asp Ile Val Leu Leu Thr Thr Thr
            100                 105                 110

ttc agc atc tgg tac aac ttt gtg aca ccc gag tac att ccc tcc act          384
Phe Ser Ile Trp Tyr Asn Phe Val Thr Pro Glu Tyr Ile Pro Ser Thr
        115                 120                 125

cct gct cga gcc ggt ctg tgg gct gtg tac acc gtt ctt cag gga ctc          432
Pro Ala Arg Ala Gly Leu Trp Ala Val Tyr Thr Val Leu Gln Gly Leu
    130                 135                 140

ttc ggt act gga ctg tgg gtc att gcc cac gag tgt gga cat ggt gct          480
Phe Gly Thr Gly Leu Trp Val Ile Ala His Glu Cys Gly His Gly Ala
145                 150                 155                 160

ttc tcc gat tcc cga atc atc aac gac att act ggc tgg gtg ctt cac          528
Phe Ser Asp Ser Arg Ile Ile Asn Asp Ile Thr Gly Trp Val Leu His
                165                 170                 175

tct tcc ctg ctt gtt ccc tac ttc agc tgg caa atc tcc cac cgg aag          576
Ser Ser Leu Leu Val Pro Tyr Phe Ser Trp Gln Ile Ser His Arg Lys
            180                 185                 190

cat cac aag gcc act gga aac atg gag cga gac atg gtc ttc gtt cct          624
His His Lys Ala Thr Gly Asn Met Glu Arg Asp Met Val Phe Val Pro
        195                 200                 205

cga acc cga gag cag caa gct act cga ctc ggc aag atg acc cac gaa          672
Arg Thr Arg Glu Gln Gln Ala Thr Arg Leu Gly Lys Met Thr His Glu
    210                 215                 220

ctc gcc cat ctt acc gag gaa act cct gct ttc acc ctg ctc atg ctt          720
Leu Ala His Leu Thr Glu Glu Thr Pro Ala Phe Thr Leu Leu Met Leu
225                 230                 235                 240

gtg ctt cag caa ctg gtc ggt tgg ccc aac tat ctc att acc aac gtt          768
Val Leu Gln Gln Leu Val Gly Trp Pro Asn Tyr Leu Ile Thr Asn Val
                245                 250                 255

act gga cac aac tac cat gag cgg cag cga gag ggt cga ggc aag gga          816
Thr Gly His Asn Tyr His Glu Arg Gln Arg Glu Gly Arg Gly Lys Gly
            260                 265                 270

aag cac aac ggt ctt ggc ggt gga gtt aac cat ttc gat ccc cga tct          864
Lys His Asn Gly Leu Gly Gly Gly Val Asn His Phe Asp Pro Arg Ser
        275                 280                 285
```

```
cct ctg tac gag aac agc gac gcc aag ctc atc gtg ctc tcc gac att        912
Pro Leu Tyr Glu Asn Ser Asp Ala Lys Leu Ile Val Leu Ser Asp Ile
    290                 295                 300

ggc att ggt ctt atg gcc acc gct ctg tac ttt ctc gtt cag aag ttc        960
Gly Ile Gly Leu Met Ala Thr Ala Leu Tyr Phe Leu Val Gln Lys Phe
305                 310                 315                 320

gga ttc tac aac atg gcc atc tgg tac ttc gtt ccc tac ttg tgg gtt       1008
Gly Phe Tyr Asn Met Ala Ile Trp Tyr Phe Val Pro Tyr Leu Trp Val
                325                 330                 335

aac cac tgg ctc gtc gcc att acc ttt ctg cag cac aca gat cct act       1056
Asn His Trp Leu Val Ala Ile Thr Phe Leu Gln His Thr Asp Pro Thr
            340                 345                 350

ctt ccc cac tac acc aac gac gag tgg aac ttt gtg cga ggt gcc gct       1104
Leu Pro His Tyr Thr Asn Asp Glu Trp Asn Phe Val Arg Gly Ala Ala
        355                 360                 365

gca acc atc gac cga gag atg ggc ttc att gga cgt cat ctg ctc cac       1152
Ala Thr Ile Asp Arg Glu Met Gly Phe Ile Gly Arg His Leu Leu His
    370                 375                 380

ggc att atc gag act cac gtc ctg cat cac tac gtc tct tcc att ccc       1200
Gly Ile Ile Glu Thr His Val Leu His His Tyr Val Ser Ser Ile Pro
385                 390                 395                 400

ttc tac aat gcg gac gaa gct acc gag gcc atc aaa cct atc atg ggc       1248
Phe Tyr Asn Ala Asp Glu Ala Thr Glu Ala Ile Lys Pro Ile Met Gly
                405                 410                 415

aag cac tat cga gct gat gtc cag gac ggt cct cga gga ttc att cga       1296
Lys His Tyr Arg Ala Asp Val Gln Asp Gly Pro Arg Gly Phe Ile Arg
            420                 425                 430

gcc atg tac cga tct gca cga atg tgc cag tgg gtt gaa ccc tcc gct       1344
Ala Met Tyr Arg Ser Ala Arg Met Cys Gln Trp Val Glu Pro Ser Ala
        435                 440                 445

ggt gcc gag gga gct ggc aag ggt gtc ctg ttc ttt cga aac cga aac       1392
Gly Ala Glu Gly Ala Gly Lys Gly Val Leu Phe Phe Arg Asn Arg Asn
    450                 455                 460

aat gtg ggc act cct ccc gct gtc atc aag ccc gtt gcc taa               1434
Asn Val Gly Thr Pro Pro Ala Val Ile Lys Pro Val Ala
465                 470                 475
```

<210> 52
<211> 477
<212> PRT
<213> Fusarium moniliforme

<400> 52

```
Met Ala Ser Thr Ser Ala Leu Pro Lys Gln Asn Pro Ala Leu Arg Arg
1               5                   10                  15

Thr Val Thr Ser Thr Thr Val Thr Asp Ser Glu Ser Ala Ala Val Ser
            20                  25                  30
```

```
Pro Ser Asp Ser Pro Arg His Ser Ala Ser Ser Thr Ser Leu Ser Ser
        35                  40                  45

Met Ser Glu Val Asp Ile Ala Lys Pro Lys Ser Glu Tyr Gly Val Met
    50                  55                  60

Leu Asp Thr Tyr Gly Asn Gln Phe Glu Val Pro Asp Phe Thr Ile Lys
65                  70                  75                  80

Asp Ile Tyr Asn Ala Ile Pro Lys His Cys Phe Lys Arg Ser Ala Leu
                85                  90                  95

Lys Gly Tyr Gly Tyr Ile Leu Arg Asp Ile Val Leu Leu Thr Thr Thr
            100                 105                 110

Phe Ser Ile Trp Tyr Asn Phe Val Thr Pro Glu Tyr Ile Pro Ser Thr
        115                 120                 125

Pro Ala Arg Ala Gly Leu Trp Ala Val Tyr Thr Val Leu Gln Gly Leu
    130                 135                 140

Phe Gly Thr Gly Leu Trp Val Ile Ala His Glu Cys Gly His Gly Ala
145                 150                 155                 160

Phe Ser Asp Ser Arg Ile Ile Asn Asp Ile Thr Gly Trp Val Leu His
                165                 170                 175

Ser Ser Leu Leu Val Pro Tyr Phe Ser Trp Gln Ile Ser His Arg Lys
            180                 185                 190

His His Lys Ala Thr Gly Asn Met Glu Arg Asp Met Val Phe Val Pro
        195                 200                 205

Arg Thr Arg Glu Gln Gln Ala Thr Arg Leu Gly Lys Met Thr His Glu
    210                 215                 220

Leu Ala His Leu Thr Glu Glu Thr Pro Ala Phe Thr Leu Leu Met Leu
225                 230                 235                 240

Val Leu Gln Gln Leu Val Gly Trp Pro Asn Tyr Leu Ile Thr Asn Val
                245                 250                 255

Thr Gly His Asn Tyr His Glu Arg Gln Arg Glu Gly Arg Gly Lys Gly
            260                 265                 270

Lys His Asn Gly Leu Gly Gly Gly Val Asn His Phe Asp Pro Arg Ser
        275                 280                 285

Pro Leu Tyr Glu Asn Ser Asp Ala Lys Leu Ile Val Leu Ser Asp Ile
```

```
    290                 295                 300

Gly Ile Gly Leu Met Ala Thr Ala Leu Tyr Phe Leu Val Gln Lys Phe
305                 310                 315                 320

Gly Phe Tyr Asn Met Ala Ile Trp Tyr Phe Val Pro Tyr Leu Trp Val
                325                 330                 335

Asn His Trp Leu Val Ala Ile Thr Phe Leu Gln His Thr Asp Pro Thr
            340                 345                 350

Leu Pro His Tyr Thr Asn Asp Glu Trp Asn Phe Val Arg Gly Ala Ala
        355                 360                 365

Ala Thr Ile Asp Arg Glu Met Gly Phe Ile Gly Arg His Leu Leu His
    370                 375                 380

Gly Ile Ile Glu Thr His Val Leu His His Tyr Val Ser Ser Ile Pro
385                 390                 395                 400

Phe Tyr Asn Ala Asp Glu Ala Thr Glu Ala Ile Lys Pro Ile Met Gly
                405                 410                 415

Lys His Tyr Arg Ala Asp Val Gln Asp Gly Pro Arg Gly Phe Ile Arg
            420                 425                 430

Ala Met Tyr Arg Ser Ala Arg Met Cys Gln Trp Val Glu Pro Ser Ala
        435                 440                 445

Gly Ala Glu Gly Ala Gly Lys Gly Val Leu Phe Phe Arg Asn Arg Asn
    450                 455                 460

Asn Val Gly Thr Pro Pro Ala Val Ile Lys Pro Val Ala
465                 470                 475
```

<210> 53
<211> 15119
<212> DNA
<213> Artificial Sequence

<220>
<223> Plasmid pZSCP-Ma83

<400> 53

gtacgacccc tctcaggcca agcagaaggc tgagtccatc aagaaggcca acgctatcat 60

tgtcttcaac ctcaagaaca aggctggcaa gaccgagtct tggtaccttg acctcaagaa 120

cgacggtgac gtcggcaagg gcaacaagtc ccccaagggt gatgctgaca tccagctcac 180

tctctctgac gaccacttcc agcagctcgt tgagggtaag gctaacgccc agcgactctt 240

catgaccggc aagctcaagg ttaagggcaa cgtcatgaag gctgccgcca ttgagggtat 300

cctcaagaac gctcagaaca acctctaagc gcatcattta ttgattaatt gatgatttac 360
tatattgatt tcgcaactgt agtgtgattg tatgtgatct ggctcgtagg cttcagtaaa 420
tactagacgg gtatcctacg tagttgtatc atacatcgag cctgtggtta cttgtacaat 480
aattcgtaat gtagagatac cccttgatcc attgcctgtt tctaacatac aatgatctcc 540
acgcaataat cccactcttg actaaaagtt gctactcttg cacggttacc tcggcatagt 600
cacgcctctc ttgtctcgtc tcgaacgcac aaagtcaatt gacaacgcca ctcactcgag 660
tgtgccccaa cagggcacca tatcgactaa tttgaggcca actagggtga ttttggatgg 720
aatttgatcg gaaaaaatag ctgcagaaat tcctggagag aaaaattgac cgcatccaca 780
tggtttgacc aaaaaatcgt ctccatctct gtgctcaact ctcctgacga gatatgcgcg 840
cgcacccccca catgatgtga ttgatctcaa caaacttcac ccagaccctt atctttccgg 900
gaaacttact gtataagtgg tcgtgcgaac agaaagtgtg cgcactttag gtgtctagat 960
ccgattgttc tcgttctgat aatgagccag ccccgcgagg caatgttttt tacaattgaa 1020
aacttcgtta accactcaca ttaccgtttt tgccccatat ttaccctctg gtacactccc 1080
tcttgcatac acacacactg cagtgaaaat gcactccgtt agcaccgttg tgattggttc 1140
agggcacgag tttggtggtt taaggcgcaa ctacatcaat atgaaaacag gagacgctga 1200
aaaggggtaa tatcggactg ctgctatgtt gtatgtactg catgacgaat tggtgttatt 1260
caagaccgtg gcacaggttg ctgcggtacg agacctggta gcttctctaa acggcatgtc 1320
taggtggcgc gccagctgca ttaatgaatc ggccaacgcg cggggagagg cggtttgcgt 1380
attgggcgct cttccgcttc ctcgctcact gactcgctgc gctcggtcgt tcggctgcgg 1440
cgagcggtat cagctcactc aaaggcggta atacggttat ccacagaatc aggggataac 1500
gcaggaaaga acatgtgagc aaaaggccag caaaaggcca ggaaccgtaa aaaggccgcg 1560
ttgctggcgt ttttccatag gctccgcccc cctgacgagc atcacaaaaa tcgacgctca 1620
agtcagaggt ggcgaaaccc gacaggacta taaagatacc aggcgtttcc ccctggaagc 1680
tccctcgtgc gctctcctgt tccgaccctg ccgcttaccg gatacctgtc cgcctttctc 1740
ccttcgggaa gcgtggcgct ttctcatagc tcacgctgta ggtatctcag ttcggtgtag 1800
gtcgttcgct ccaagctggg ctgtgtgcac gaaccccccg ttcagcccga ccgctgcgcc 1860
ttatccggta actatcgtct tgagtccaac ccggtaagac acgacttatc gccactggca 1920
gcagccactg gtaacaggat tagcagagcg aggtatgtag gcggtgctac agagttcttg 1980
aagtggtggc ctaactacgg ctacactaga agaacagtat ttggtatctg cgctctgctg 2040
aagccagtta ccttcggaaa aagagttggt agctcttgat ccggcaaaca aaccaccgct 2100
ggtagcggtg gtttttttgt ttgcaagcag cagattacgc gcagaaaaaa aggatctcaa 2160
gaagatcctt tgatcttttc tacggggtct gacgctcagt ggaacgaaaa ctcacgttaa 2220

gggattttgg tcatgagatt atcaaaaagg atcttcacct agatcctttt aaattaaaaa 2280
tgaagtttta aatcaatcta aagtatatat gagtaaactt ggtctgacag ttaccaatgc 2340
ttaatcagtg aggcacctat ctcagcgatc tgtctatttc gttcatccat agttgcctga 2400
ctccccgtcg tgtagataac tacgatacgg gagggcttac catctggccc cagtgctgca 2460
atgataccgc gagacccacg ctcaccggct ccagatttat cagcaataaa ccagccagcc 2520
ggaagggccg agcgcagaag tggtcctgca actttatccg cctccatcca gtctattaat 2580
tgttgccggg aagctagagt aagtagttcg ccagttaata gtttgcgcaa cgttgttgcc 2640
attgctacag gcatcgtggt gtcacgctcg tcgtttggta tggcttcatt cagctccggt 2700
tcccaacgat caaggcgagt tacatgatcc cccatgttgt gcaaaaaagc ggttagctcc 2760
ttcggtcctc cgatcgttgt cagaagtaag ttggccgcag tgttatcact catggttatg 2820
gcagcactgc ataattctct tactgtcatg ccatccgtaa gatgcttttc tgtgactggt 2880
gagtactcaa ccaagtcatt ctgagaatag tgtatgcggc gaccgagttg ctcttgcccg 2940
gcgtcaatac gggataatac cgcgccacat agcagaactt taaaagtgct catcattgga 3000
aaacgttctt cggggcgaaa actctcaagg atcttaccgc tgttgagatc cagttcgatg 3060
taacccactc gtgcacccaa ctgatcttca gcatctttta ctttcaccag cgtttctggg 3120
tgagcaaaaa caggaaggca aaatgccgca aaaaagggaa taagggcgac acggaaatgt 3180
tgaatactca tactcttcct ttttcaatat tattgaagca tttatcaggg ttattgtctc 3240
atgagcggat acatatttga atgtatttag aaaaataaac aaataggggt tccgcgcaca 3300
tttccccgaa aagtgccacc tgatgcggtg tgaaataccg cacagatgcg taaggagaaa 3360
ataccgcatc aggaaattgt aagcgttaat attttgttaa aattcgcgtt aaatttttgt 3420
taaatcagct cattttttaa ccaataggcc gaaatcggca aaatccctta taaatcaaaa 3480
gaatagaccg agatagggtt gagtgttgtt ccagtttgga acaagagtcc actattaaag 3540
aacgtggact ccaacgtcaa agggcgaaaa accgtctatc agggcgatgg cccactacgt 3600
gaaccatcac cctaatcaag ttttttgggg tcgaggtgcc gtaaagcact aaatcggaac 3660
cctaaaggga gcccccgatt tagagcttga cggggaaagc cggcgaacgt ggcgagaaag 3720
gaagggaaga aagcgaaagg agcgggcgct agggcgctgg caagtgtagc ggtcacgctg 3780
cgcgtaacca ccacacccgc cgcgcttaat gcgccgctac agggcgcgtc cattcgccat 3840
tcaggctgcg caactgttgg gaagggcgat cggtgcgggc ctcttcgcta ttacgccagc 3900
tggcgaaagg gggatgtgct gcaaggcgat taagttgggt aacgccaggg ttttcccagt 3960
cacgacgttg taaaacgacg gccagtgaat tgtaatacga ctcactatag ggcgaattgg 4020
gcccgacgtc gcatgcgtca ctaatcaagg atacctacca tgccactatg atgtttgcag 4080
gaggtgtacc tcggcagtca tcaaaaaatg gaactactgg ctttagatct tgttgtatgg 4140
catcgcgcct aaaaaagaaa ccccccttcca gcgagctact acaagtagtt gtagttgcgg 4200

gcgttggata ccgaaagtca caagcacatg tcgaagctct catctgaaac accgacagtc 4260
gtctgcaccc cgcaagtctc ggttcgtacc agcaccaatg ttaggcagaa ctatacacaa 4320
gagggcggac gatcacttcg gcgttaggca actgaaggct attttcggct ggtactgtag 4380
gggacagagg aaacgcaagt gattagtaaa tcggataata ggcctgttag tttaccgaaa 4440
tggtggggga ggggttccgt ggatatcttg aagttatgga ggctgatcgt tatttgtggg 4500
gatggatatc attgtatgga catactgtag ctactgtata aacaacggat cttacacctg 4560
cctcttgtat gcccattgct tgatcatcta tcgtgttact gtacatatac aatagatata 4620
gggaagaaaa gccggaagta gagaccatag tctggcagaa gtaacggcct cgggtcgaga 4680
gaactataac aaagtccaac ggcgggtctt agaatagccc caaggatcac acagttccgc 4740
aatccagttt cacatgttcc gttgcatgga cttttgcatg tctactgttg ctacgattcc 4800
cccattgcaa ccacagtttg gggttacccc gcattatatt agcatgatta cgaaagagat 4860
aagtatcata tggaacatgt gaagggtagt atgcaggtcc ggcggagaaa gagaatgacg 4920
ttttcattaa gcgattcgct tggcggcttg tgggggatgt gacgatactt acggtaaaga 4980
ccctgtgtga gagctggtac tcgctcgtta cttcgctgat ctgttgggcc gtcaatcgaa 5040
tctcgtggaa cttgcattct tcttaactgt gtctatacaa gacacctaat gaaacataca 5100
agctaccgaa atcattttac tcgtactgac cggtacggta cttgcacaag tagtgaaact 5160
tccgaaaata gccagcctca tgcatcatcg cttcacccct tctgttgacc tcaaaagcat 5220
tccaacggta aaaaattata acgccgccaa ctggatggtt gtgacggcgt tgaccaccaa 5280
tgtgtggggg ctggcggtag gaccgagctt attcgtccca ataagctctt tggatttgat 5340
tctttggggt gtgtggtaaa attcacatgg ggaagaacac ggtggcagtt tgaggcagag 5400
gcccagcgtg tagttcctag ggcatgaata taccgaactc atggcgcaga attgagctga 5460
atgcgcaaaa agctacagga tcaaccgcgt tagaaatgcc gcaaatgtcc actaattccc 5520
cggactgttc caaatgattc tgtggggata aatctcaaac tgggttaggc tttgtcacgt 5580
ttctttgtgt cgtgtcggtt cgtccggggc aatgtgccca cgcttggctg tctccctaca 5640
cctcggtaaa aactatcaca tgctgcccct ctcgagcaag cattaaatgc atatagtcaa 5700
tctaacgaca tatatatagg tagggtgcat cctccggttt agctccccag aatatctctt 5760
attcattaca caaaaacaac aatgtctctc aaggtcgacg gcttcacttc ttaattaagt 5820
tgcgacacat gtcttgatag tatcttgaat tctctctctt gagcttttcc ataacaagtt 5880
cttctgcctc caggaagtcc atgggtggtt tgatcatggt tttggtgtag tggtagtgca 5940
gtggtggtat tgtgactggg gatgtagttg agaataagtc atacacaagt cagctttctt 6000
cgagcctcat ataagtataa gtagttcaac gtattagcac tgtacccagc atctccgtat 6060
cgagaaacac aacaacatgc cccattggac agatcatgcg gatacacagg ttgtgcagta 6120

tcatacatac tcgatcagac aggtcgtctg accatcatac aagctgaaca agcgctccat 6180
acttgcacgc tctctatata cacagttaaa ttacatatcc atagtctaac ctctaacagt 6240
taatcttctg gtaagcctcc cagccagcct tctggtatcg cttggcctcc tcaataggat 6300
ctcggttctg gccgtacaga cctcggccga caattatgat atccgttccg gtagacatga 6360
catcctcaac agttcggtac tgctgtccga gagcgtctcc cttgtcgtca agacccaccc 6420
cgggggtcag aataagccag tcctcagagt cgcccttagg tcggttctgg gcaatgaagc 6480
caaccacaaa ctcggggtcg gatcgggcaa gctcaatggt ctgcttggag tactcgccag 6540
tggccagaga gcccttgcaa gacagctcgg ccagcatgag cagacctctg gccagcttct 6600
cgttgggaga ggggactagg aactccttgt actgggagtt ctcgtagtca gagacgtcct 6660
ccttcttctg ttcagagaca gtttcctcgg caccagctcg caggccagca atgattccgg 6720
ttccgggtac accgtgggcg ttggtgatat cggaccactc ggcgattcgg tgacaccggt 6780
actggtgctt gacagtgttg ccaatatctg cgaactttct gtcctcgaac aggaagaaac 6840
cgtgcttaag agcaagttcc ttgaggggga gcacagtgcc ggcgtaggtg aagtcgtcaa 6900
tgatgtcgat atgggttttg atcatgcaca cataaggtcc gaccttatcg gcaagctcaa 6960
tgagctcctt ggtggtggta acatccagag aagcacacag gttggttttc ttggctgcca 7020
cgagcttgag cactcgagcg gcaaaggcgg acttgtggac gttagctcga gcttcgtagg 7080
agggcatttt ggtggtgaag aggagactga aataaattta gtctgcagaa ctttttatcg 7140
gaaccttatc tggggcagtg aagtatatgt tatggtaata gttacgagtt agttgaactt 7200
atagatagac tggactatac ggctatcggt ccaaattaga aagaacgtca atggctctct 7260
gggcgtcgcc tttgccgaca aaaatgtgat catgatgaaa gccagcaatg acgttgcagc 7320
tgatattgtt gtcggccaac cgcgccgaaa acgcagctgt cagacccaca gcctccaacg 7380
aagaatgtat cgtcaaagtg atccaagcac actcatagtt ggagtcgtac tccaaaggcg 7440
gcaatgacga gtcagacaga tactcgtcga ccttttcctt gggaaccacc accgtcagcc 7500
cttctgactc acgtattgta gccaccgaca caggcaacag tccgtggata gcagaatatg 7560
tcttgtcggt ccatttctca ccaactttag gcgtcaagtg aatgttgcag aagaagtatg 7620
tgccttcatt gagaatcggt gttgctgatt tcaataaagt cttgagatca gtttggccag 7680
tcatgttgtg gggggtaatt ggattgagtt atcgcctaca gtctgtacag gtatactcgc 7740
tgcccacttt atactttttg attccgctgc acttgaagca atgtcgttta ccaaaagtga 7800
gaatgctcca cagaacacac cccagggtat ggttgagcaa aaaataaaca ctccgatacg 7860
gggaatcgaa ccccggtctc cacggttctc aagaagtatt cttgatgaga gcgtatcgat 7920
ggaagccggt agaaccgggc tgcttgtgct tggagatgga agccggtaga accgggctgc 7980
ttggggggat ttggggccgc tgggctccaa agaggggtag gcatttcgtt ggggttacgt 8040
aattgcggca tttgggtcct gcgcgcatgt cccattggtc agaattagtc cggataggag 8100

acttatcagc caatcacagc gccggatcca cctgtaggtt gggttgggtg ggagcacccc 8160
tccacagagt agagtcaaac agcagcagca acatgatagt tgggggtgtg cgtgttaaag 8220
gaaaaaaaag aagcttgggt tatattcccg ctctatttag aggttgcggg atagacgccg 8280
acggagggca atggcgctat ggaaccttgc ggatatccat acgccgcggc ggactgcgtc 8340
cgaaccagct ccagcagcgt tttttccggg ccattgagcc gactgcgacc ccgccaacgt 8400
gtcttggccc acgcactcat gtcatgttgg tgttgggagg ccacttttta agtagcacaa 8460
ggcacctagc tcgcagcaag gtgtccgaac caaagaagcg gctgcagtgg tgcaaacggg 8520
gcggaaacgg cgggaaaaag ccacgggggc acgaattgag gcacgccctc gaatttgaga 8580
cgagtcacgg ccccattcgc ccgcgcaatg gctcgccaac gcccggtctt ttgcaccaca 8640
tcaggttacc ccaagccaaa cctttgtgtt aaaaagctta acatattata ccgaacgtag 8700
gtttgggcgg gcttgctccg tctgtccaag gcaacattta tataagggtc tgcatcgccg 8760
gctcaattga atcttttttc ttcttctctt ctctatattc attcttgaat taaacacaca 8820
tcaaccatgg tcaagcgacc cgctctgcct ctcaccgtgg acggtgtcac ctacgacgtt 8880
tctgcctggc tcaaccacca tcccggaggt gccgacatta tcgagaacta ccgaggtcgg 8940
gatgctaccg acgtcttcat ggttatgcac tccgagaacg ccgtgtccaa actcagacga 9000
atgcccatca tggaaccttc ctctcccctg actccaacac ctcccaagcc aaactccgac 9060
gaacctcagg aggatttccg aaagctgcga gacgagctca ttgctgcagg catgttcgat 9120
gcctctccca tgtggtacgc ttacaagacc ctgtcgactc tcggactggg tgtccttgcc 9180
gtgctgttga tgacccagtg gcactggtac ctggttggtg ctatcgtcct cggcattcac 9240
tttcaacaga tgggatggct ctcgcacgac atttgccatc accagctgtt caaggaccga 9300
tccatcaaca atgccattgg cctgctcttc ggaaacgtgc ttcagggctt ttctgtcact 9360
tggtggaagg accgacacaa cgctcatcac tccgccacca acgtgcaggg tcacgatccc 9420
gacatcgaca acctgcctct cctggcgtgg tccaaggagg acgtcgagcg agctggcccg 9480
ttttctcgac ggatgatcaa gtaccaacag tattacttct ttttcatctg tgcccttctg 9540
cgattcatct ggtgctttca gtccattcat actgccacgg gtctcaagga tcgaagcaat 9600
cagtactatc gaagacagta cgagaaggag tccgtcggtc tggcactcca ctggggtctc 9660
aaggccttgt tctactattt ctacatgccc tcgtttctca ccggactcat ggtgttcttt 9720
gtctccgagc tgcttggtgg cttcggaatt gccatcgttg tcttcatgaa ccactaccct 9780
ctggagaaga ttcaggactc cgtgtgggat ggtcatggct tctgtgctgg acagattcac 9840
gagaccatga acgttcagcg aggcctcgtc acagactggt tttcggtgg cctcaactac 9900
cagatcgaac atcacctgtg gcctactctt cccagacaca acctcaccgc tgcctccatc 9960
aaagtggagc agctgtgcaa gaagcacaac ctgccctacc gatctcctcc catgctcgaa 10020

ggtgtcggca ttcttatctc ctacctgggc accttcgctc gaatggttgc caaggcagac 10080
aaggcctaag cggccgcatt gatgattgga aacacacaca tgggttatat ctaggtgaga 10140
gttagttgga cagttatata ttaaatcagc tatgccaacg gtaacttcat tcatgtcaac 10200
gaggaaccag tgactgcaag taatatagaa tttgaccacc ttgccattct cttgcactcc 10260
tttactatat ctcatttatt tcttatatac aaatcacttc ttcttcccag catcgagctc 10320
ggaaacctca tgagcaataa catcgtggat ctcgtcaata gagggctttt tggactcctt 10380
gctgttggcc accttgtcct tgctgtttaa acagagtgtg aaagactcac tatggtccgg 10440
gcttatctcg accaatagcc aaagtctgga gtttctgaga gaaaaaggca agatacgtat 10500
gtaacaaagc gacgcatggt acaataatac cggaggcatg tatcatagag agttagtggt 10560
tcgatgatgg cactggtgcc tggtatgact ttatacggct gactacatat ttgtcctcag 10620
acatacaatt acagtcaagc acttaccctt ggacatctgt aggtaccccc cggccaagac 10680
gatctcagcg tgtcgtatgt cggattggcg tagctccctc gctcgtcaat tggctcccat 10740
ctactttctt ctgcttggct acacccagca tgtctgctat ggctcgtttt cgtgccttat 10800
ctatcctccc agtattacca actctaaatg acatgatgtg attgggtcta cactttcata 10860
tcagagataa ggagtagcac agttgcataa aaagcccaac tctaatcagc ttcttccttt 10920
cttgtaatta gtacaaaggt gattagcgaa atctggaagc ttagttggcc ctaaaaaaat 10980
caaaaaaagc aaaaaacgaa aaacgaaaaa ccacagtttt gagaacaggg aggtaacgaa 11040
ggatcgtata tatatatata tatatatata cccacggatc ccgagaccgg cctttgattc 11100
ttccctacaa ccaaccattc tcaccaccct aattcacaac catggtctcc aaccacctgt 11160
tcgacgccat gcgagctgcc gctcccggag acgcaccttt cattcgaatc gacaacgctc 11220
ggacctggac ttacgatgac gccattgctc tttccggtcg aatcgctgga gctatggacg 11280
cactcggcat tcgacccgga gacagagttg ccgtgcaggt cgagaagtct gccgaggcgt 11340
tgattctcta cctggcctgt cttcgaaccg gagctgtcta cctgcctctc aacactgcct 11400
acaccctggc cgagctcgac tacttcatcg gcgatgccga accgcgtctg gtggtcgttg 11460
ctcccgcagc tcgaggtggc gtggagacaa ttgccaagcg acacggtgct atcgtcgaaa 11520
ccctcgacgc cgatggacga ggctccttgc tggaccttgc tagagatgag cctgccgact 11580
ttgtcgatgc ttcgcgatct gccgacgatc tggctgctat tctctacact tccggtacaa 11640
ccggacgatc gaagggtgcc atgcttactc atggcaatct gctctccaac gctctcacct 11700
tgcgagacta ttggagagtt accgcagacg atcgactcat ccatgccttg ccaatctttc 11760
acactcatgg tctgttcgtt gctacgaacg tcacactgct tgcaggagcc tcgatgtttc 11820
tgctctccaa gttcgatgcc gacgaggtcg tttctctcat gccacaggcc accatgctta 11880
tgggcgtgcc cacattctac gttcgattgc tgcagagtcc tcgactcgag aagggtgctg 11940
tggccagcat cagactgttc atttctggat cagctccctt gcttgccgaa acccacgccg 12000

agtttcatgc tcgtactggt cacgccattc tcgagcgata cggcatgacg gaaaccaaca 12060
tgaatacttc caacccctac gagggcaagc gtattgccgg aaccgttggt tttcctctgc 12120
ccgacgtcac tgtgcgagtc accgatcccg ccaccggtct cgttcttcca cctgaagaga 12180
ctggcatgat cgagatcaag ggacccaacg tcttcaaggg ctattggcga atgcccgaaa 12240
agaccgctgc cgagtttacc gcagacggtt tctttatctc tggagatctc ggcaagatcg 12300
accgagaagg ttacgttcac attgtgggac gaggcaagga cctggtcatt tccggtggct 12360
acaacatcta tcccaaagag gtcgaaggcg agatcgacca gatcgagggt gtggtcgagt 12420
ctgctgtcat tggtgttcct catcccgatt tcggagaagg tgtcaccgct gttgtcgtgt 12480
gcaaacctgg tgccgttctc gacgaaaaga ccatcgtgtc tgctctgcag gaccgtcttg 12540
cccgatacaa gcaacccaag cggattatct ttgccgacga tctgcctcga aacactatgg 12600
gaaaggttca gaagaacatt cttcgacagc aatacgccga tctctacacc agacgataag 12660
cggccgcatg agaagataaa tatataaata cattgagata ttaaatgcgc tagattagag 12720
agcctcatac tgctcggaga gaagccaaga cgagtactca aaggggatta caccatccat 12780
atccacagac acaagctggg gaaaggttct atatacactt tccggaatac cgtagtttcc 12840
gatgttatca atgggggcag ccaggatttc aggcacttcg gtgtctcggg gtgaaatggc 12900
gttcttggcc tccatcaagt cgtaccatgt cttcatttgc ctgtcaaagt aaaacagaag 12960
cagatgaaga atgaacttga agtgaaggaa tttaaatgat gtcgacgcag taggatgtcc 13020
tgcacgggtc tttttgtggg gtgtggagaa aggggtgctt ggagatggaa gccggtagaa 13080
ccgggctgct tgtgcttgga gatggaagcc ggtagaaccg ggctgcttgg ggggatttgg 13140
ggccgctggg ctccaaagag gggtaggcat ttcgttgggg ttacgtaatt gcggcatttg 13200
ggtcctgcgc gcatgtccca ttggtcagaa ttagtccgga taggagactt atcagccaat 13260
cacagcgccg gatccacctg taggttgggt tgggtgggag caccccctcca cagagtagag 13320
tcaaacagca gcagcaacat gatagttggg ggtgtgcgtg ttaaaggaaa aaaaagaagc 13380
ttgggttata ttcccgctct atttagaggt tgcgggatag acgccgacgg agggcaatgg 13440
cgctatggaa ccttgcggat atccatacgc cgcggcggac tgcgtccgaa ccagctccag 13500
cagcgttttt tccgggccat tgagccgact gcgaccccgc caacgtgtct tggcccacgc 13560
actcatgtca tgttggtgtt gggaggccac tttttaagta gcacaaggca cctagctcgc 13620
agcaaggtgt ccgaaccaaa gaagcggctg cagtggtgca aacggggcgg aaacggcggg 13680
aaaaagccac gggggcacga attgaggcac gccctcgaat ttgagacgag tcacggcccc 13740
attcgcccgc gcaatggctc gccaacgccc ggtcttttgc accacatcag gttaccccaa 13800
gccaaacctt tgtgttaaaa agcttaacat attataccga acgtaggttt gggcgggctt 13860
gctccgtctg tccaaggcaa catttatata agggtctgca tcgccggctc aattgaatct 13920

```
tttttcttct tctcttctct atattcattc ttgaattaaa cacacatcaa ccatggagtc    13980
tggacccatg cctgctggca ttcccttccc tgagtactat gacttcttta tggactggaa    14040
gactcccctg gccatcgctg ccacctacac tgctgccgtc ggtctcttca accccaaggt    14100
tggcaaggtc tcccgagtgg ttgccaagtc ggctaacgca aagcctgccg agcgaaccca    14160
gtccggagct gccatgactg ccttcgtctt tgtgcacaac ctcattctgt gtgtctactc    14220
tggcatcacc ttctactaca tgtttcctgc tatggtcaag aacttccgaa cccacacact    14280
gcacgaagcc tactgcgaca cggatcagtc cctctggaac aacgcacttg gctactgggg    14340
ttacctcttc tacctgtcca agttctacga ggtcattgac accatcatca tcatcctgaa    14400
gggacgacgg tcctcgctgc ttcagaccta ccaccatgct ggagccatga ttaccatgtg    14460
gtctggcatc aactaccaag ccactcccat ttggatcttt gtggtcttca actccttcat    14520
tcacaccatc atgtactgtt actatgcctt cacctctatc ggattccatc ctcctggcaa    14580
aaagtacctg acttcgatgc agattactca gtttctggtc ggtatcacca ttgccgtgtc    14640
ctacctcttc gttcctggct gcatccgaac acccggtgct cagatggctg tctggatcaa    14700
cgtcggctac ctgtttccct tgacctatct gttcgtggac tttgccaagc gaacctactc    14760
caagcgatct gccattgccg ctcagaaaaa ggctcagtaa gcggccgcaa gtgtggatgg    14820
ggaagtgagt gcccggttct gtgtgcacaa ttggcaatcc aagatggatg gattcaacac    14880
agggatatag cgagctacgt ggtggtgcga ggatatagca acggatattt atgtttgaca    14940
cttgagaatg tacgatacaa gcactgtcca agtacaatac taaacatact gtacatactc    15000
atactcgtac ccgggcaacg gtttcacttg agtgcagtgg ctagtgctct tactcgtaca    15060
gtgtgcaata ctgcgtatca tagtctttga tgtatatcgt attcattcat gttagttgc     15119
```

<210> 54
<211> 828
<212> DNA
<213> Mortierella alpina

<220>
<221> CDS
<222> (1)..(828)
<223> synthetic C16/18 elongase (codon-optimized for Yarrowia lipolytica)

<300>
<302> A MORTIERELLA ALPINA C16/18 FATTY ACID ELONGASE
<310> U.S. 7,470,532
<311> 2005-10-19
<312> 2008-12-30
<313> (1)..(828)

<300>
<302> A MORTIERELLA ALPINA C16/18 FATTY ACID ELONGASE
<310> WO 2007/046817

<311> 2005-11-04
<312> 2007-04-26
<313> (1)..(828)

<400> 54

```
atg gag tct gga ccc atg cct gct ggc att ccc ttc cct gag tac tat       48
Met Glu Ser Gly Pro Met Pro Ala Gly Ile Pro Phe Pro Glu Tyr Tyr
1               5                   10                  15

gac ttc ttt atg gac tgg aag act ccc ctg gcc atc gct gcc acc tac       96
Asp Phe Phe Met Asp Trp Lys Thr Pro Leu Ala Ile Ala Ala Thr Tyr
            20                  25                  30

act gct gcc gtc ggt ctc ttc aac ccc aag gtt ggc aag gtc tcc cga      144
Thr Ala Ala Val Gly Leu Phe Asn Pro Lys Val Gly Lys Val Ser Arg
        35                  40                  45

gtg gtt gcc aag tcg gct aac gca aag cct gcc gag cga acc cag tcc      192
Val Val Ala Lys Ser Ala Asn Ala Lys Pro Ala Glu Arg Thr Gln Ser
    50                  55                  60

gga gct gcc atg act gcc ttc gtc ttt gtg cac aac ctc att ctg tgt      240
Gly Ala Ala Met Thr Ala Phe Val Phe Val His Asn Leu Ile Leu Cys
65                  70                  75                  80

gtc tac tct ggc atc acc ttc tac tac atg ttt cct gct atg gtc aag      288
Val Tyr Ser Gly Ile Thr Phe Tyr Tyr Met Phe Pro Ala Met Val Lys
                85                  90                  95

aac ttc cga acc cac aca ctg cac gaa gcc tac tgc gac acg gat cag      336
Asn Phe Arg Thr His Thr Leu His Glu Ala Tyr Cys Asp Thr Asp Gln
            100                 105                 110

tcc ctc tgg aac aac gca ctt ggc tac tgg ggt tac ctc ttc tac ctg      384
Ser Leu Trp Asn Asn Ala Leu Gly Tyr Trp Gly Tyr Leu Phe Tyr Leu
        115                 120                 125

tcc aag ttc tac gag gtc att gac acc atc atc atc atc ctg aag gga      432
Ser Lys Phe Tyr Glu Val Ile Asp Thr Ile Ile Ile Ile Leu Lys Gly
    130                 135                 140

cga cgg tcc tcg ctg ctt cag acc tac cac cat gct gga gcc atg att      480
Arg Arg Ser Ser Leu Leu Gln Thr Tyr His His Ala Gly Ala Met Ile
145                 150                 155                 160

acc atg tgg tct ggc atc aac tac caa gcc act ccc att tgg atc ttt      528
Thr Met Trp Ser Gly Ile Asn Tyr Gln Ala Thr Pro Ile Trp Ile Phe
                165                 170                 175

gtg gtc ttc aac tcc ttc att cac acc atc atg tac tgt tac tat gcc      576
Val Val Phe Asn Ser Phe Ile His Thr Ile Met Tyr Cys Tyr Tyr Ala
            180                 185                 190

ttc acc tct atc gga ttc cat cct cct ggc aaa aag tac ctg act tcg      624
Phe Thr Ser Ile Gly Phe His Pro Pro Gly Lys Lys Tyr Leu Thr Ser
        195                 200                 205

atg cag att act cag ttt ctg gtc ggt atc acc att gcc gtg tcc tac      672
Met Gln Ile Thr Gln Phe Leu Val Gly Ile Thr Ile Ala Val Ser Tyr
    210                 215                 220

ctc ttc gtt cct ggc tgc atc cga aca ccc ggt gct cag atg gct gtc      720
Leu Phe Val Pro Gly Cys Ile Arg Thr Pro Gly Ala Gln Met Ala Val
225                 230                 235                 240

tgg atc aac gtc ggc tac ctg ttt ccc ttg acc tat ctg ttc gtg gac      768
Trp Ile Asn Val Gly Tyr Leu Phe Pro Leu Thr Tyr Leu Phe Val Asp
```

```
            245                 250                 255

ttt gcc aag cga acc tac tcc aag cga tct gcc att gcc gct cag aaa           816
Phe Ala Lys Arg Thr Tyr Ser Lys Arg Ser Ala Ile Ala Ala Gln Lys
                260                 265                 270

aag gct cag taa                                                           828
Lys Ala Gln
        275
```

<210> 55
<211> 275
<212> PRT
<213> Mortierella alpina

<400> 55

```
Met Glu Ser Gly Pro Met Pro Ala Gly Ile Pro Phe Pro Glu Tyr Tyr
1               5                   10                  15

Asp Phe Phe Met Asp Trp Lys Thr Pro Leu Ala Ile Ala Ala Thr Tyr
            20                  25                  30

Thr Ala Ala Val Gly Leu Phe Asn Pro Lys Val Gly Lys Val Ser Arg
        35                  40                  45

Val Val Ala Lys Ser Ala Asn Ala Lys Pro Ala Glu Arg Thr Gln Ser
    50                  55                  60

Gly Ala Ala Met Thr Ala Phe Val Phe Val His Asn Leu Ile Leu Cys
65                  70                  75                  80

Val Tyr Ser Gly Ile Thr Phe Tyr Tyr Met Phe Pro Ala Met Val Lys
                85                  90                  95

Asn Phe Arg Thr His Thr Leu His Glu Ala Tyr Cys Asp Thr Asp Gln
            100                 105                 110

Ser Leu Trp Asn Asn Ala Leu Gly Tyr Trp Gly Tyr Leu Phe Tyr Leu
        115                 120                 125

Ser Lys Phe Tyr Glu Val Ile Asp Thr Ile Ile Ile Ile Leu Lys Gly
    130                 135                 140

Arg Arg Ser Ser Leu Leu Gln Thr Tyr His His Ala Gly Ala Met Ile
145                 150                 155                 160

Thr Met Trp Ser Gly Ile Asn Tyr Gln Ala Thr Pro Ile Trp Ile Phe
                165                 170                 175

Val Val Phe Asn Ser Phe Ile His Thr Ile Met Tyr Cys Tyr Tyr Ala
            180                 185                 190
```

```
Phe Thr Ser Ile Gly Phe His Pro Pro Gly Lys Lys Tyr Leu Thr Ser
        195                 200                 205

Met Gln Ile Thr Gln Phe Leu Val Gly Ile Thr Ile Ala Val Ser Tyr
    210                 215                 220

Leu Phe Val Pro Gly Cys Ile Arg Thr Pro Gly Ala Gln Met Ala Val
225                 230                 235                 240

Trp Ile Asn Val Gly Tyr Leu Phe Pro Leu Thr Tyr Leu Phe Val Asp
                245                 250                 255

Phe Ala Lys Arg Thr Tyr Ser Lys Arg Ser Ala Ile Ala Ala Gln Lys
            260                 265                 270

Lys Ala Gln
        275
```

<210> 56
<211> 1518
<212> DNA
<213> Rhizobium leguminosarum bv. viciae 3841

<220>
<221> CDS
<222> (1)..(1518)
<223> synthetic malonyl-CoA synthetase (codon-optimized for Yarrowia lipolytica)

<400> 56

```
atg gtc tcc aac cac ctg ttc gac gcc atg cga gct gcc gct ccc gga          48
Met Val Ser Asn His Leu Phe Asp Ala Met Arg Ala Ala Ala Pro Gly
1               5                   10                  15

gac gca cct ttc att cga atc gac aac gct cgg acc tgg act tac gat          96
Asp Ala Pro Phe Ile Arg Ile Asp Asn Ala Arg Thr Trp Thr Tyr Asp
            20                  25                  30

gac gcc att gct ctt tcc ggt cga atc gct gga gct atg gac gca ctc         144
Asp Ala Ile Ala Leu Ser Gly Arg Ile Ala Gly Ala Met Asp Ala Leu
        35                  40                  45

ggc att cga ccc gga gac aga gtt gcc gtg cag gtc gag aag tct gcc         192
Gly Ile Arg Pro Gly Asp Arg Val Ala Val Gln Val Glu Lys Ser Ala
    50                  55                  60

gag gcg ttg att ctc tac ctg gcc tgt ctt cga acc gga gct gtc tac         240
Glu Ala Leu Ile Leu Tyr Leu Ala Cys Leu Arg Thr Gly Ala Val Tyr
65                  70                  75                  80

ctg cct ctc aac act gcc tac acc ctg gcc gag ctc gac tac ttc atc         288
Leu Pro Leu Asn Thr Ala Tyr Thr Leu Ala Glu Leu Asp Tyr Phe Ile
                85                  90                  95

ggc gat gcc gaa ccg cgt ctg gtg gtc gtt gct ccc gca gct cga ggt         336
Gly Asp Ala Glu Pro Arg Leu Val Val Val Ala Pro Ala Ala Arg Gly
            100                 105                 110
```

```
ggc gtg gag aca att gcc aag cga cac ggt gct atc gtc gaa acc ctc        384
Gly Val Glu Thr Ile Ala Lys Arg His Gly Ala Ile Val Glu Thr Leu
        115                 120                 125

gac gcc gat gga cga ggc tcc ttg ctg gac ctt gct aga gat gag cct        432
Asp Ala Asp Gly Arg Gly Ser Leu Leu Asp Leu Ala Arg Asp Glu Pro
    130                 135                 140

gcc gac ttt gtc gat gct tcg cga tct gcc gac gat ctg gct gct att        480
Ala Asp Phe Val Asp Ala Ser Arg Ser Ala Asp Asp Leu Ala Ala Ile
145                 150                 155                 160

ctc tac act tcc ggt aca acc gga cga tcg aag ggt gcc atg ctt act        528
Leu Tyr Thr Ser Gly Thr Thr Gly Arg Ser Lys Gly Ala Met Leu Thr
                165                 170                 175

cat ggc aat ctg ctc tcc aac gct ctc acc ttg cga gac tat tgg aga        576
His Gly Asn Leu Leu Ser Asn Ala Leu Thr Leu Arg Asp Tyr Trp Arg
            180                 185                 190

gtt acc gca gac gat cga ctc atc cat gcc ttg cca atc ttt cac act        624
Val Thr Ala Asp Asp Arg Leu Ile His Ala Leu Pro Ile Phe His Thr
        195                 200                 205

cat ggt ctg ttc gtt gct acg aac gtc aca ctg ctt gca gga gcc tcg        672
His Gly Leu Phe Val Ala Thr Asn Val Thr Leu Leu Ala Gly Ala Ser
    210                 215                 220

atg ttt ctg ctc tcc aag ttc gat gcc gac gag gtc gtt tct ctc atg        720
Met Phe Leu Leu Ser Lys Phe Asp Ala Asp Glu Val Val Ser Leu Met
225                 230                 235                 240

cca cag gcc acc atg ctt atg ggc gtg ccc aca ttc tac gtt cga ttg        768
Pro Gln Ala Thr Met Leu Met Gly Val Pro Thr Phe Tyr Val Arg Leu
                245                 250                 255

ctg cag agt cct cga ctc gag aag ggt gct gtg gcc agc atc aga ctg        816
Leu Gln Ser Pro Arg Leu Glu Lys Gly Ala Val Ala Ser Ile Arg Leu
            260                 265                 270

ttc att tct gga tca gct ccc ttg ctt gcc gaa acc cac gcc gag ttt        864
Phe Ile Ser Gly Ser Ala Pro Leu Leu Ala Glu Thr His Ala Glu Phe
        275                 280                 285

cat gct cgt act ggt cac gcc att ctc gag cga tac ggc atg acg gaa        912
His Ala Arg Thr Gly His Ala Ile Leu Glu Arg Tyr Gly Met Thr Glu
    290                 295                 300

acc aac atg aat act tcc aac ccc tac gag ggc aag cgt att gcc gga        960
Thr Asn Met Asn Thr Ser Asn Pro Tyr Glu Gly Lys Arg Ile Ala Gly
305                 310                 315                 320

acc gtt ggt ttt cct ctg ccc gac gtc act gtg cga gtc acc gat ccc       1008
Thr Val Gly Phe Pro Leu Pro Asp Val Thr Val Arg Val Thr Asp Pro
                325                 330                 335

gcc acc ggt ctc gtt ctt cca cct gaa gag act ggc atg atc gag atc       1056
Ala Thr Gly Leu Val Leu Pro Pro Glu Glu Thr Gly Met Ile Glu Ile
            340                 345                 350

aag gga ccc aac gtc ttc aag ggc tat tgg cga atg ccc gaa aag acc       1104
Lys Gly Pro Asn Val Phe Lys Gly Tyr Trp Arg Met Pro Glu Lys Thr
        355                 360                 365
```

```
gct gcc gag ttt acc gca gac ggt ttc ttt atc tct gga gat ctc ggc        1152
Ala Ala Glu Phe Thr Ala Asp Gly Phe Phe Ile Ser Gly Asp Leu Gly
    370                 375                 380

aag atc gac cga gaa ggt tac gtt cac att gtg gga cga ggc aag gac        1200
Lys Ile Asp Arg Glu Gly Tyr Val His Ile Val Gly Arg Gly Lys Asp
385                 390                 395                 400

ctg gtc att tcc ggt ggc tac aac atc tat ccc aaa gag gtc gaa ggc        1248
Leu Val Ile Ser Gly Gly Tyr Asn Ile Tyr Pro Lys Glu Val Glu Gly
                405                 410                 415

gag atc gac cag atc gag ggt gtg gtc gag tct gct gtc att ggt gtt        1296
Glu Ile Asp Gln Ile Glu Gly Val Val Glu Ser Ala Val Ile Gly Val
            420                 425                 430

cct cat ccc gat ttc gga gaa ggt gtc acc gct gtt gtc gtg tgc aaa        1344
Pro His Pro Asp Phe Gly Glu Gly Val Thr Ala Val Val Val Cys Lys
        435                 440                 445

cct ggt gcc gtt ctc gac gaa aag acc atc gtg tct gct ctg cag gac        1392
Pro Gly Ala Val Leu Asp Glu Lys Thr Ile Val Ser Ala Leu Gln Asp
    450                 455                 460

cgt ctt gcc cga tac aag caa ccc aag cgg att atc ttt gcc gac gat        1440
Arg Leu Ala Arg Tyr Lys Gln Pro Lys Arg Ile Ile Phe Ala Asp Asp
465                 470                 475                 480

ctg cct cga aac act atg gga aag gtt cag aag aac att ctt cga cag        1488
Leu Pro Arg Asn Thr Met Gly Lys Val Gln Lys Asn Ile Leu Arg Gln
                485                 490                 495

caa tac gcc gat ctc tac acc aga cga taa                                1518
Gln Tyr Ala Asp Leu Tyr Thr Arg Arg
            500                 505
```

<210> 57
<211> 505
<212> PRT
<213> Rhizobium leguminosarum bv. viciae 3841

<400> 57

```
Met Val Ser Asn His Leu Phe Asp Ala Met Arg Ala Ala Ala Pro Gly
1               5                   10                  15

Asp Ala Pro Phe Ile Arg Ile Asp Asn Ala Arg Thr Trp Thr Tyr Asp
            20                  25                  30

Asp Ala Ile Ala Leu Ser Gly Arg Ile Ala Gly Ala Met Asp Ala Leu
        35                  40                  45

Gly Ile Arg Pro Gly Asp Arg Val Ala Val Gln Val Glu Lys Ser Ala
    50                  55                  60

Glu Ala Leu Ile Leu Tyr Leu Ala Cys Leu Arg Thr Gly Ala Val Tyr
65                  70                  75                  80

Leu Pro Leu Asn Thr Ala Tyr Thr Leu Ala Glu Leu Asp Tyr Phe Ile
```

```
                85                  90                  95

Gly Asp Ala Glu Pro Arg Leu Val Val Val Ala Pro Ala Ala Arg Gly
            100                 105                 110

Gly Val Glu Thr Ile Ala Lys Arg His Gly Ala Ile Val Glu Thr Leu
        115                 120                 125

Asp Ala Asp Gly Arg Gly Ser Leu Leu Asp Leu Ala Arg Asp Glu Pro
    130                 135                 140

Ala Asp Phe Val Asp Ala Ser Arg Ser Ala Asp Asp Leu Ala Ala Ile
145                 150                 155                 160

Leu Tyr Thr Ser Gly Thr Thr Gly Arg Ser Lys Gly Ala Met Leu Thr
                165                 170                 175

His Gly Asn Leu Leu Ser Asn Ala Leu Thr Leu Arg Asp Tyr Trp Arg
            180                 185                 190

Val Thr Ala Asp Asp Arg Leu Ile His Ala Leu Pro Ile Phe His Thr
        195                 200                 205

His Gly Leu Phe Val Ala Thr Asn Val Thr Leu Leu Ala Gly Ala Ser
    210                 215                 220

Met Phe Leu Leu Ser Lys Phe Asp Ala Asp Glu Val Val Ser Leu Met
225                 230                 235                 240

Pro Gln Ala Thr Met Leu Met Gly Val Pro Thr Phe Tyr Val Arg Leu
                245                 250                 255

Leu Gln Ser Pro Arg Leu Glu Lys Gly Ala Val Ala Ser Ile Arg Leu
            260                 265                 270

Phe Ile Ser Gly Ser Ala Pro Leu Leu Ala Glu Thr His Ala Glu Phe
        275                 280                 285

His Ala Arg Thr Gly His Ala Ile Leu Glu Arg Tyr Gly Met Thr Glu
    290                 295                 300

Thr Asn Met Asn Thr Ser Asn Pro Tyr Glu Gly Lys Arg Ile Ala Gly
305                 310                 315                 320

Thr Val Gly Phe Pro Leu Pro Asp Val Thr Val Arg Val Thr Asp Pro
                325                 330                 335

Ala Thr Gly Leu Val Leu Pro Pro Glu Glu Thr Gly Met Ile Glu Ile
            340                 345                 350
```

```
Lys Gly Pro Asn Val Phe Lys Gly Tyr Trp Arg Met Pro Glu Lys Thr
        355                 360                 365

Ala Ala Glu Phe Thr Ala Asp Gly Phe Phe Ile Ser Gly Asp Leu Gly
    370                 375                 380

Lys Ile Asp Arg Glu Gly Tyr Val His Ile Val Gly Arg Gly Lys Asp
385                 390                 395                 400

Leu Val Ile Ser Gly Gly Tyr Asn Ile Tyr Pro Lys Glu Val Glu Gly
                405                 410                 415

Glu Ile Asp Gln Ile Glu Gly Val Val Glu Ser Ala Val Ile Gly Val
            420                 425                 430

Pro His Pro Asp Phe Gly Glu Gly Val Thr Ala Val Val Val Cys Lys
        435                 440                 445

Pro Gly Ala Val Leu Asp Glu Lys Thr Ile Val Ser Ala Leu Gln Asp
    450                 455                 460

Arg Leu Ala Arg Tyr Lys Gln Pro Lys Arg Ile Ile Phe Ala Asp Asp
465                 470                 475                 480

Leu Pro Arg Asn Thr Met Gly Lys Val Gln Lys Asn Ile Leu Arg Gln
                485                 490                 495

Gln Tyr Ala Asp Leu Tyr Thr Arg Arg
            500                 505
```

<210> 58
<211> 1260
<212> DNA
<213> Euglena anabaena UTEX 373

<220>
<221> CDS
<222> (1)..(1260)
<223> synthetic delta-8 desaturase (codon-optimized for Yarrowia lipolytica)

<300>
<302> DELTA-8 DESATURASES AND THEIR USE IN MAKING POLYUNSATURATED FATTY ACIDS
<310> US-2008-0254521-A1
<311> 2008-04-09
<312> 2008-10-16
<313> (1)..(1260)

<300>
<302> DELTA-8 DESATURASES AND THEIR USE IN MAKING POLYUNSATURATED FATTY ACIDS

<310> WO 2008/124194

<311> 2008-04-10
<312> 2008-10-16
<313> (1)..(1260)

<400> 58

```
atg gtc aag cga ccc gct ctg cct ctc acc gtg gac ggt gtc acc tac      48
Met Val Lys Arg Pro Ala Leu Pro Leu Thr Val Asp Gly Val Thr Tyr
1               5                   10                  15

gac gtt tct gcc tgg ctc aac cac cat ccc gga ggt gcc gac att atc      96
Asp Val Ser Ala Trp Leu Asn His His Pro Gly Gly Ala Asp Ile Ile
            20                  25                  30

gag aac tac cga ggt cgg gat gct acc gac gtc ttc atg gtt atg cac     144
Glu Asn Tyr Arg Gly Arg Asp Ala Thr Asp Val Phe Met Val Met His
        35                  40                  45

tcc gag aac gcc gtg tcc aaa ctc aga cga atg ccc atc atg gaa cct     192
Ser Glu Asn Ala Val Ser Lys Leu Arg Arg Met Pro Ile Met Glu Pro
    50                  55                  60

tcc tct ccc ctg act cca aca cct ccc aag cca aac tcc gac gaa cct     240
Ser Ser Pro Leu Thr Pro Thr Pro Pro Lys Pro Asn Ser Asp Glu Pro
65                  70                  75                  80

cag gag gat ttc cga aag ctg cga gac gag ctc att gct gca ggc atg     288
Gln Glu Asp Phe Arg Lys Leu Arg Asp Glu Leu Ile Ala Ala Gly Met
                85                  90                  95

ttc gat gcc tct ccc atg tgg tac gct tac aag acc ctg tcg act ctc     336
Phe Asp Ala Ser Pro Met Trp Tyr Ala Tyr Lys Thr Leu Ser Thr Leu
            100                 105                 110

gga ctg ggt gtc ctt gcc gtg ctg ttg atg acc cag tgg cac tgg tac     384
Gly Leu Gly Val Leu Ala Val Leu Leu Met Thr Gln Trp His Trp Tyr
        115                 120                 125

ctg gtt ggt gct atc gtc ctc ggc att cac ttt caa cag atg gga tgg     432
Leu Val Gly Ala Ile Val Leu Gly Ile His Phe Gln Gln Met Gly Trp
    130                 135                 140

ctc tcg cac gac att tgc cat cac cag ctg ttc aag gac cga tcc atc     480
Leu Ser His Asp Ile Cys His His Gln Leu Phe Lys Asp Arg Ser Ile
145                 150                 155                 160

aac aat gcc att ggc ctg ctc ttc gga aac gtg ctt cag ggc ttt tct     528
Asn Asn Ala Ile Gly Leu Leu Phe Gly Asn Val Leu Gln Gly Phe Ser
                165                 170                 175

gtc act tgg tgg aag gac cga cac aac gct cat cac tcc gcc acc aac     576
Val Thr Trp Trp Lys Asp Arg His Asn Ala His His Ser Ala Thr Asn
            180                 185                 190

gtg cag ggt cac gat ccc gac atc gac aac ctg cct ctc ctg gcg tgg     624
Val Gln Gly His Asp Pro Asp Ile Asp Asn Leu Pro Leu Leu Ala Trp
        195                 200                 205

tcc aag gag gac gtc gag cga gct ggc ccg ttt tct cga cgg atg atc     672
Ser Lys Glu Asp Val Glu Arg Ala Gly Pro Phe Ser Arg Arg Met Ile
    210                 215                 220

aag tac caa cag tat tac ttc ttt ttc atc tgt gcc ctt ctg cga ttc     720
Lys Tyr Gln Gln Tyr Tyr Phe Phe Phe Ile Cys Ala Leu Leu Arg Phe
225                 230                 235                 240
```

```
atc tgg tgc ttt cag tcc att cat act gcc acg ggt ctc aag gat cga         768
Ile Trp Cys Phe Gln Ser Ile His Thr Ala Thr Gly Leu Lys Asp Arg
                245                 250                 255

agc aat cag tac tat cga aga cag tac gag aag gag tcc gtc ggt ctg         816
Ser Asn Gln Tyr Tyr Arg Arg Gln Tyr Glu Lys Glu Ser Val Gly Leu
            260                 265                 270

gca ctc cac tgg ggt ctc aag gcc ttg ttc tac tat ttc tac atg ccc         864
Ala Leu His Trp Gly Leu Lys Ala Leu Phe Tyr Tyr Phe Tyr Met Pro
        275                 280                 285

tcg ttt ctc acc gga ctc atg gtg ttc ttt gtc tcc gag ctg ctt ggt         912
Ser Phe Leu Thr Gly Leu Met Val Phe Phe Val Ser Glu Leu Leu Gly
    290                 295                 300

ggc ttc gga att gcc atc gtt gtc ttc atg aac cac tac cct ctg gag         960
Gly Phe Gly Ile Ala Ile Val Val Phe Met Asn His Tyr Pro Leu Glu
305                 310                 315                 320

aag att cag gac tcc gtg tgg gat ggt cat ggc ttc tgt gct gga cag        1008
Lys Ile Gln Asp Ser Val Trp Asp Gly His Gly Phe Cys Ala Gly Gln
                325                 330                 335

att cac gag acc atg aac gtt cag cga ggc ctc gtc aca gac tgg ttt        1056
Ile His Glu Thr Met Asn Val Gln Arg Gly Leu Val Thr Asp Trp Phe
            340                 345                 350

ttc ggt ggc ctc aac tac cag atc gaa cat cac ctg tgg cct act ctt        1104
Phe Gly Gly Leu Asn Tyr Gln Ile Glu His His Leu Trp Pro Thr Leu
        355                 360                 365

ccc aga cac aac ctc acc gct gcc tcc atc aaa gtg gag cag ctg tgc        1152
Pro Arg His Asn Leu Thr Ala Ala Ser Ile Lys Val Glu Gln Leu Cys
    370                 375                 380

aag aag cac aac ctg ccc tac cga tct cct ccc atg ctc gaa ggt gtc        1200
Lys Lys His Asn Leu Pro Tyr Arg Ser Pro Pro Met Leu Glu Gly Val
385                 390                 395                 400

ggc att ctt atc tcc tac ctg ggc acc ttc gct cga atg gtt gcc aag        1248
Gly Ile Leu Ile Ser Tyr Leu Gly Thr Phe Ala Arg Met Val Ala Lys
                405                 410                 415

gca gac aag gcc                                                        1260
Ala Asp Lys Ala
            420
```

<210> 59
<211> 420
<212> PRT
<213> Euglena anabaena UTEX 373

<400> 59

```
Met Val Lys Arg Pro Ala Leu Pro Leu Thr Val Asp Gly Val Thr Tyr
1               5                   10                  15

Asp Val Ser Ala Trp Leu Asn His His Pro Gly Gly Ala Asp Ile Ile
            20                  25                  30
```

```
Glu Asn Tyr Arg Gly Arg Asp Ala Thr Asp Val Phe Met Val Met His
        35                  40                  45

Ser Glu Asn Ala Val Ser Lys Leu Arg Arg Met Pro Ile Met Glu Pro
    50                  55                  60

Ser Ser Pro Leu Thr Pro Thr Pro Pro Lys Pro Asn Ser Asp Glu Pro
65                  70                  75                  80

Gln Glu Asp Phe Arg Lys Leu Arg Asp Glu Leu Ile Ala Ala Gly Met
                85                  90                  95

Phe Asp Ala Ser Pro Met Trp Tyr Ala Tyr Lys Thr Leu Ser Thr Leu
            100                 105                 110

Gly Leu Gly Val Leu Ala Val Leu Leu Met Thr Gln Trp His Trp Tyr
        115                 120                 125

Leu Val Gly Ala Ile Val Leu Gly Ile His Phe Gln Gln Met Gly Trp
    130                 135                 140

Leu Ser His Asp Ile Cys His His Gln Leu Phe Lys Asp Arg Ser Ile
145                 150                 155                 160

Asn Asn Ala Ile Gly Leu Leu Phe Gly Asn Val Leu Gln Gly Phe Ser
                165                 170                 175

Val Thr Trp Trp Lys Asp Arg His Asn Ala His His Ser Ala Thr Asn
            180                 185                 190

Val Gln Gly His Asp Pro Asp Ile Asp Asn Leu Pro Leu Leu Ala Trp
        195                 200                 205

Ser Lys Glu Asp Val Glu Arg Ala Gly Pro Phe Ser Arg Arg Met Ile
    210                 215                 220

Lys Tyr Gln Gln Tyr Tyr Phe Phe Phe Ile Cys Ala Leu Leu Arg Phe
225                 230                 235                 240

Ile Trp Cys Phe Gln Ser Ile His Thr Ala Thr Gly Leu Lys Asp Arg
                245                 250                 255

Ser Asn Gln Tyr Tyr Arg Arg Gln Tyr Glu Lys Glu Ser Val Gly Leu
            260                 265                 270

Ala Leu His Trp Gly Leu Lys Ala Leu Phe Tyr Tyr Phe Tyr Met Pro
        275                 280                 285

Ser Phe Leu Thr Gly Leu Met Val Phe Phe Val Ser Glu Leu Leu Gly
```

```
    290                 295                 300

Gly Phe Gly Ile Ala Ile Val Val Phe Met Asn His Tyr Pro Leu Glu
305                 310                 315                 320

Lys Ile Gln Asp Ser Val Trp Asp Gly His Gly Phe Cys Ala Gly Gln
                325                 330                 335

Ile His Glu Thr Met Asn Val Gln Arg Gly Leu Val Thr Asp Trp Phe
            340                 345                 350

Phe Gly Gly Leu Asn Tyr Gln Ile Glu His His Leu Trp Pro Thr Leu
        355                 360                 365

Pro Arg His Asn Leu Thr Ala Ala Ser Ile Lys Val Glu Gln Leu Cys
    370                 375                 380

Lys Lys His Asn Leu Pro Tyr Arg Ser Pro Pro Met Leu Glu Gly Val
385                 390                 395                 400

Gly Ile Leu Ile Ser Tyr Leu Gly Thr Phe Ala Arg Met Val Ala Lys
                405                 410                 415

Ala Asp Lys Ala
            420
```

<210> 60
<211> 16424
<212> DNA
<213> Artificial Sequence

<220>
<223> Plasmid pZKL4-220EA41B

<400> 60

```
aattctctct cttgagcttt tccataacaa gttcttctgc ctccaggaag tccatgggtg      60
gtttgatcat ggttttggtg tagtggtagt gcagtggtgg tattgtgact ggggatgtag     120
ttgagaataa gtcatacaca agtcagcttt cttcgagcct catataagta taagtagttc     180
aacgtattag cactgtaccc agcatctccg tatcgagaaa cacaacaaca tgccccattg     240
gacagatcat gcggatacac aggttgtgca gtatcataca tactcgatca gacaggtcgt     300
ctgaccatca tacaagctga acaagcgctc catacttgca cgctctctat atacacagtt     360
aaattacata tccatagtct aacctctaac agttaatctt ctggtaagcc tcccagccag     420
ccttctggta tcgcttggcc tcctcaatag gatctcggtt ctggccgtac agacctcggc     480
cgacaattat gatatccgtt ccggtagaca tgacatcctc aacagttcgg tactgctgtc     540
cgagagcgtc tcccttgtcg tcaagaccca ccccgggggt cagaataagc cagtcctcag     600
agtcgccctt aggtcggttc tgggcaatga agccaaccac aaactcgggg tcggatcggg     660
```

caagctcaat ggtctgcttg gagtactcgc cagtggccag agagcccttg caagacagct 720
cggccagcat gagcagacct ctggccagct tctcgttggg agaggggact aggaactcct 780
tgtactggga gttctcgtag tcagagacgt cctccttctt ctgttcagag acagtttcct 840
cggcaccagc tcgcaggcca gcaatgattc cggttccggg tacaccgtgg gcgttggtga 900
tatcggacca ctcggcgatt cggtgacacc ggtactggtg cttgacagtg ttgccaatat 960
ctgcgaactt tctgtcctcg aacaggaaga aaccgtgctt aagagcaagt tccttgaggg 1020
ggagcacagt gccggcgtag gtgaagtcgt caatgatgtc gatatgggtt ttgatcatgc 1080
acacataagg tccgacctta tcggcaagct caatgagctc cttggtggtg gtaacatcca 1140
gagaagcaca caggttggtt ttcttggctg ccacgagctt gagcactcga gcggcaaagg 1200
cggacttgtg gacgttagct cgagcttcgt aggagggcat tttggtggtg aagaggagac 1260
tgaaataaat ttagtctgca gaacttttta tcggaacctt atctggggca gtgaagtata 1320
tgttatggta atagttacga gttagttgaa cttatagata gactggacta tacggctatc 1380
ggtccaaatt agaaagaacg tcaatggctc tctgggcgtc gcctttgccg acaaaaatgt 1440
gatcatgatg aaagccagca atgacgttgc agctgatatt gttgtcggcc aaccgcgccg 1500
aaaacgcagc tgtcagaccc acagcctcca acgaagaatg tatcgtcaaa gtgatccaag 1560
cacactcata gttggagtcg tactccaaag gcggcaatga cgagtcagac agatactcgt 1620
cgaccttttc cttgggaacc accaccgtca gcccttctga ctcacgtatt gtagccaccg 1680
acacaggcaa cagtccgtgg atagcagaat atgtcttgtc ggtccatttc tcaccaactt 1740
taggcgtcaa gtgaatgttg cagaagaagt atgtgccttc attgagaatc ggtgttgctg 1800
atttcaataa agtcttgaga tcagtttggc cagtcatgtt gtggggggta attggattga 1860
gttatcgcct acagtctgta caggtatact cgctgcccac tttatacttt ttgattccgc 1920
tgcacttgaa gcaatgtcgt ttaccaaaag tgagaatgct ccacagaaca caccccaggg 1980
tatggttgag caaaaaataa acactccgat acggggaatc gaaccccggt ctccacggtt 2040
ctcaagaagt attcttgatg agagcgtatc gatcgaggaa gaggacaagc ggctgcttct 2100
taagtttgtg acatcagtat ccaaggcacc attgcaagga ttcaaggctt tgaacccgtc 2160
atttgccatt cgtaacgctg gtagacaggt tgatcggttc cctacggcct ccacctgtgt 2220
caatcttctc aagctgcctg actatcagga cattgatcaa cttcggaaga aacttttgta 2280
tgccattcga tcacatgctg gtttcgattt gtcttagagg aacgcatata cagtaatcat 2340
agagaataaa cgatattcat ttattaaagt agatagttga ggtagaagtt gtaaagagtg 2400
ataaatagcg gccgcttaag ccagagtggc gacagcagga caaccggcag cagatccgtc 2460
ggtggagccg tcgggttggg cagcaacggc agcagagagc acaggacaaa catcctccag 2520
ggaacctgca gtgggagcaa gcttgtcgtt gtctgcaggt ttggtgccca tagctcgcaa 2580

gtgctcgacc attccgtaca gagcatcgga aaactgagag tagttcttgt agggaagacc 2640

gtattcctcg cacacgccct tgacaacagg agcaatggtg ggatagttgg cgtgagaaat 2700

gctgggaaac aggtgatgct cgatctggtg gttgagtcca ccagaaaggt ggtttgaaaa 2760

gtagccacca gatcgccagt tgacgcaaca cagaacttgg gaggcagccc agtcgttggg 2820

aggtggagtg gggtgcttct gtttcgcctt ggcttcctgc tcggcctgct tgagcatttc 2880

ggtccgtcgg gcagcgataa cggaagaggg attgaggtag tcgcacgact cggaaatgtg 2940

gttgataatg aaacagacgg caaggtactc gccagaaacc agatgagctg taatgaagag 3000

ggccaatccg tgagcgatgc catgcaggta gcagggaagc acaatctgca tgagaaagcc 3060

catgatcttt gcaccccaga acagcatctg accctcgagg ggaacgagtc tggcagagaa 3120

gtcgatggga ccacgtcgct tggcaagaca aaacgtgaaa tcggagataa tgaccttgct 3180

gatggtcatg agagcgaaca acggaaaggc gtacacgtgt tgcagctgat ggtggggttt 3240

ccaaggagtg tccggatgca tccgcatgag gggataggtt ccgaaaacgt ctggatcgtt 3300

ctcgggaaga gcaaactcgg gatcggacac caggttggtg tactggtgat gaccaatgac 3360

gtgttggtac tcccaggcag tcgaggaggc tccgataacg tccatacccc agccagcaag 3420

acggttgagt cgtccagact tggagaaagc accatgattg ccgtcgtgct ggatgctgag 3480

tccaatgtga gagcctgcca gaccccagac ggcagcccaa aagaaggatc cctgtgtcac 3540

catgaggtag aacgaggcag caaaggcagc cataacaagg gctgccttga cagacaggcc 3600

accacgtcga ggttgtccag cctccttgag agtctgaacc actcgctgct tgagctcggc 3660

gtagaaagcg gaggcctgcc agtcgtagaa ggttctgtga tcctgaagtg taccgattcg 3720

gtacttctcc ataaccttgt cgggtcgtcc tgcaggatgg tacgattcga ccagaatagt 3780

ggagtctcgt ccgagaccga gagagataac atctccaccg ggatgcttgc caatgaactc 3840

ggtgatgtcg tacacaccgt cgtgacaggc caaccagcca tcggtgggaa caatgtgtcg 3900

agcgacctct cgcatggtaa actttcggtc ctgtccagta ccggaggtgg ccagagcgtc 3960

gtaataggct gcaggtgggt tgccaacagg tcgaatggga ggtggaagag aagcgatctc 4020

cgagggcttg ccaggtccac cgggtttgac cttggccatg gccattgctg tagatatgtc 4080

ttgtgtgtaa gggggttggg gtggttgttt gtgttcttga cttttgtgtt agcaagggaa 4140

gacgggcaaa aaagtgagtg tggttgggag ggagagacga gccttatata taatgcttgt 4200

ttgtgtttgt gcaagtggac gccgaaacgg gcaggagcca aactaaacaa ggcagacaat 4260

gcgagcttaa ttggattgcc tgatgggcag gggttagggc tcgatcaatg ggggtgcgaa 4320

gtgacaaaat tgggaattag gttcgcaagc aaggctgaca agactttggc ccaaacattt 4380

gtacgcggtg gacaacagga gccacccatc gtctgtcacg ggctagccgg tcgtgcgtcc 4440

tgtcaggctc cacctaggct ccatgccact ccatacaatc ccactagtgt accgctaggc 4500

cgcttttagc tcccatctaa gacccccca aaacctccac tgtacagtgc actgtactgt 4560

gtggcgatca agggcaaggg aaaaaaggcg caaacatgca cgcatggaat gacgtaggta 4620

aggcgttact agactgaaaa gtggcacatt tcggcgtgcc aaagggtcct aggtgcgttt 4680

cgcgagctgg gcgccaggcc aagccgctcc aaaacgcctc tccgactccc tccagcggcc 4740

tccatatccc catccctctc cacagcaatg ttgttaagcc ttgcaaacga aaaaatagaa 4800

aggctaataa gcttccaata ttgtggtgta cgctgcataa cgcaacaatg agcgccaaac 4860

aacacacaca cacagcacac agcagcatta accacgatgt ttaaacagag tgtgaaagac 4920

tcactatggt ccgggcttat ctcgaccaat agccaaagtc tggagtttct gagagaaaaa 4980

ggcaagatac gtatgtaaca aagcgacgca tggtacaata ataccggagg catgtatcat 5040

agagagttag tggttcgatg atggcactgg tgcctggtat gactttatac ggctgactac 5100

atatttgtcc tcagacatac aattacagtc aagcacttac ccttggacat ctgtaggtac 5160

cccccggcca agacgatctc agcgtgtcgt atgtcggatt ggcgtagctc cctcgctcgt 5220

caattggctc ccatctactt tcttctgctt ggctacaccc agcatgtctg ctatggctcg 5280

ttttcgtgcc ttatctatcc tcccagtatt accaactcta aatgacatga tgtgattggg 5340

tctacacttt catatcagag ataaggagta gcacagttgc ataaaaagcc caactctaat 5400

cagcttcttc ctttcttgta attagtacaa aggtgattag cgaaatctgg aagcttagtt 5460

ggccctaaaa aaatcaaaaa aagcaaaaaa cgaaaaacga aaaaccacag ttttgagaac 5520

agggaggtaa cgaaggatcg tatatatata tatatatata tatacccacg gatcccgaga 5580

ccggcctttg attcttccct acaaccaacc attctcacca ccctaattca caaccatggc 5640

tgactctccc gtcatcaacc tctccaccat gtggaagcct ctgtcgctca tggccttgga 5700

tcttgctgtt ctgggacacg tctggaagca ggcacaacag gagggctcca tctcggctta 5760

cgccgactct gtgtggactc ccctcatcat gtccggtctg tacctctcca tgatcttcgt 5820

gggatgtcga tggatgaaga accgagagcc cttcgaaatc aagacctaca tgtttgccta 5880

caacctgtac cagaccctca tgaacctttg cattgtgctg ggcttcctct accaggtcca 5940

cgctaccggt atgcgattct ggggatctgg cgtggaccga tcgcccaagg gtctgggaat 6000

tggctttttc atctatgccc attaccacaa caagtacgtc gagtacttcg acacactctt 6060

catggtgctg cggaaaaaga acaaccagat ttcctttctt cacgtctacc atcacgctct 6120

gctcacctgg gcttggtttg ccgtggtcta cttcgctcct ggaggtgacg gctggtttgg 6180

agcctgctac aattcctcca ttcatgtcct gatgtactct tactatctgc ttgccacctt 6240

cggcatctcc tgtccctgga aaaagatcct cacccagctg caaatggttc agttctgctt 6300

ttgcttcacc cactcgatct acgtgtggat ttgcggttcc gaaatctacc ctcgaccctt 6360

gactgctctc cagtccttcg tgatggtcaa catgctggtt ctctttggca acttctacgt 6420

caagcagtat tctcagaaga atggaaagcc cgagaacggt gccactcctg agaacggtgc 6480

caagcctcag ccctgcgaga acggcaccgt cgagaagcga gagaacgaca ctgccaacgt 6540
tcgataagcg gccgcatgag aagataaata tataaataca ttgagatatt aaatgcgcta 6600
gattagagag cctcatactg ctcggagaga agccaagacg agtactcaaa ggggattaca 6660
ccatccatat ccacagacac aagctgggga aaggttctat atacactttc cggaataccg 6720
tagtttccga tgttatcaat gggggcagcc aggatttcag gcacttcggt gtctcggggt 6780
gaaatggcgt tcttggcctc catcaagtcg taccatgtct tcatttgcct gtcaaagtaa 6840
aacagaagca gatgaagaat gaacttgaag tgaaggaatt taaattgccc cggagaagac 6900
ggccaggccg cctagatgac aaattcaaca actcacagct gactttctgc cattgccact 6960
aggggggggc ctttttatat ggccaagcca agctctccac gtcggttggg ctgcacccaa 7020
caataaatgg gtagggttgc accaacaaag ggatgggatg gggggtagaa gatacgagga 7080
taacggggct caatggcaca aataagaacg aatactgcca ttaagactcg tgatccagcg 7140
actgacacca ttgcatcatc taagggcctc aaaactacct cggaactgct gcgctgatct 7200
ggacaccaca gaggttccga gcactttagg ttgcaccaaa tgtcccacca ggtgcaggca 7260
gaaaacgctg gaacagcgtg tacagtttgt cttaacaaaa agtgagggcg ctgaggtcga 7320
gcagggtggt gtgacttgtt atagccttta gagctgcgaa agcgcgtatg gatttggctc 7380
atcaggccag attgagggtc tgtggacaca tgtcatgtta gtgtacttca atcgcccccct 7440
ggatatagcc ccgacaatag gccgtggcct catttttttg ccttccgcac atttccattg 7500
ctcggtaccc acaccttgct tctcctgcac ttgccaacct taatactggt ttacattgac 7560
caacatctta caagcggggg gcttgtctag ggtatatata aacagtggct ctcccaatcg 7620
gttgccagtc tcttttttcc tttctttccc cacagattcg aaatctaaac tacacatcac 7680
agaactccga gccgtgagta tccacgacaa gatcagtgtc gagacgacgc gttttgtgta 7740
atgacacaat ccgaaagtcg ctagcaacac acactctcta cacaaactaa cccagctctg 7800
gtaccatggc cgagggcaag tccgacggtc ccgtcgttac cctccagtcc atgtggaagc 7860
ccctggctct catggccatc gacgtcggca tcctggtcaa cgtgcgacgg aaggccttca 7920
ccgagttcga cggacactcg aacgtcttcg ccgatcccgt gtacattccc tttgtcatga 7980
acctgttcta cctcaccatg atctttgctg gctgccgatg gatgaagact cgagaaccct 8040
tcgagatcaa gtcctacatg tttgcctaca acgcttacca gacaatgatg aactttctca 8100
ttgtggtcgg cttcatgtat gaggttcact ccaccggtat gcgatactgg ggatccagaa 8160
tcgacacttc taccaagggc ttgggactgg gtttcctcat ctatgcccat taccacaaca 8220
agtacgtgga gtacgtcgac accctgttca tgattctgcg gaagaaaaac aatcagatct 8280
cgttccttca cgtttaccac cattccctgc tcacttgggc atggtgggct gtggtctact 8340
gggctcctgg cggagatgcc tggttcggtg cctgttacaa ctccttcatc cacgttctca 8400
tgtactccta ctatctgttt gccaccttcg gcattcgatg tccctggaaa aagatgctca 8460

cccagttgca aatggtccag ttctgctttt gcttcgctca tgccatgtac gttggatggc 8520
ttggtcacga ggtgtaccct cgatggctca ctgctctgca ggcctttgtg atgctcaaca 8580
tgctggtcct ctttggcaac ttctacatga agtcttactc caaggcgagc aagctcgaac 8640
cagcctctcc cgtgtcgcct gcctctcttg ctcagaagcc cttcgagaac gccaaggtca 8700
agtaagcggc cgcaagtgtg gatggggaag tgagtgcccg gttctgtgtg cacaattggc 8760
aatccaagat ggatggattc aacacaggga tatagcgagc tacgtggtgg tgcgaggata 8820
tagcaacgga tatttatgtt tgacacttga gaatgtacga tacaagcact gtccaagtac 8880
aatactaaac atactgtaca tactcatact cgtacccggg caacggtttc acttgagtgc 8940
agtggctagt gctcttactc gtacagtgtg caatactgcg tatcatagtc tttgatgtat 9000
atcgtattca ttcatgttag ttgcgtacgt agggatcagg tgcttaggaa gctcgaccaa 9060
ccacggagac tgttgaaact ggatgtcggt aacagcatct ggaatgctga atgttcctcg 9120
aataacaaca tatttctcct tgttgaggtg atcataagct atgtatccgg tgattgaagt 9180
ggaatagaag tctcctccga agactgagtc caacgtcatg ttcgggaaat accgacaact 9240
ctctccacat gtaaaatcag ttcgtagagg agtgactggc gcattgacac agtaggcgat 9300
gtttgcaatc cgagaaaact tggccgtaaa gttgtacagc tcctgggagg cttgaactcg 9360
agtttttgaa agtgtcgctg gtggctcgcc gaagagggag gcatagaggt acgcaaccac 9420
ttgcccgagc gtgaggttca tgatgccaat agtgaatgtc atttatcacc gtactgcgca 9480
gtatttatat agggctcatc ggtccatgta tagatctgtc cacttatgac accccccatgt 9540
ctcattaatg tgtaaaggtg gagacgggtg gagtacaggt acagagttgg aggaaatcag 9600
gatagtgggg ttaagacatg ctccgagtcc aaatttcaac tctccattgt cacaagacct 9660
ctggtttcag agttattaca gatctaggcc tgtttcaagg tgaggggacc tcatctggat 9720
cggcacgacg atcgtcacct tacagaggac gtctgtcgca gggaaaggtg atgtggcgcg 9780
ccagctgcat taatgaatcg gccaacgcgc ggggagaggc ggtttgcgta ttgggcgctc 9840
ttccgcttcc tcgctcactg actcgctgcg ctcggtcgtt cggctgcggc gagcggtatc 9900
agctcactca aaggcggtaa tacggttatc cacagaatca ggggataacg caggaaagaa 9960
catgtgagca aaaggccagc aaaaggccag gaaccgtaaa aaggccgcgt tgctggcgtt 10020
tttccatagg ctccgccccc ctgacgagca tcacaaaaat cgacgctcaa gtcagaggtg 10080
gcgaaacccg acaggactat aaagatacca ggcgtttccc cctggaagct ccctcgtgcg 10140
ctctcctgtt ccgaccctgc cgcttaccgg atacctgtcc gcctttctcc cttcgggaag 10200
cgtggcgctt tctcatagct cacgctgtag gtatctcagt tcggtgtagg tcgttcgctc 10260
caagctgggc tgtgtgcacg aaccccccgt tcagcccgac cgctgcgcct tatccggtaa 10320
ctatcgtctt gagtccaacc cggtaagaca cgacttatcg ccactggcag cagccactgg 10380

taacaggatt agcagagcga ggtatgtagg cggtgctaca gagttcttga agtggtggcc 10440
taactacggc tacactagaa gaacagtatt tggtatctgc gctctgctga agccagttac 10500
cttcggaaaa agagttggta gctcttgatc cggcaaacaa accaccgctg gtagcggtgg 10560
tttttttgtt tgcaagcagc agattacgcg cagaaaaaaa ggatctcaag aagatccttt 10620
gatcttttct acggggtctg acgctcagtg gaacgaaaac tcacgttaag ggattttggt 10680
catgagatta tcaaaaagga tcttcaccta gatcctttta aattaaaaat gaagttttaa 10740
atcaatctaa agtatatatg agtaaacttg gtctgacagt taccaatgct taatcagtga 10800
ggcacctatc tcagcgatct gtctatttcg ttcatccata gttgcctgac tccccgtcgt 10860
gtagataact acgatacggg agggcttacc atctggcccc agtgctgcaa tgataccgcg 10920
agacccacgc tcaccggctc cagatttatc agcaataaac cagccagccg gaagggccga 10980
gcgcagaagt ggtcctgcaa ctttatccgc ctccatccag tctattaatt gttgccggga 11040
agctagagta agtagttcgc cagttaatag tttgcgcaac gttgttgcca ttgctacagg 11100
catcgtggtg tcacgctcgt cgtttggtat ggcttcattc agctccggtt cccaacgatc 11160
aaggcgagtt acatgatccc ccatgttgtg caaaaaagcg gttagctcct tcggtcctcc 11220
gatcgttgtc agaagtaagt tggccgcagt gttatcactc atggttatgg cagcactgca 11280
taattctctt actgtcatgc catccgtaag atgcttttct gtgactggtg agtactcaac 11340
caagtcattc tgagaatagt gtatgcggcg accgagttgc tcttgcccgg cgtcaatacg 11400
ggataatacc gcgccacata gcagaacttt aaaagtgctc atcattggaa aacgttcttc 11460
ggggcgaaaa ctctcaagga tcttaccgct gttgagatcc agttcgatgt aacccactcg 11520
tgcacccaac tgatcttcag catcttttac tttcaccagc gtttctgggt gagcaaaaac 11580
aggaaggcaa aatgccgcaa aaaagggaat aagggcgaca cggaaatgtt gaatactcat 11640
actcttcctt tttcaatatt attgaagcat ttatcagggt tattgtctca tgagcggata 11700
catatttgaa tgtatttaga aaaataaaca aataggggtt ccgcgcacat ttccccgaaa 11760
agtgccacct gatgcggtgt gaaataccgc acagatgcgt aaggagaaaa taccgcatca 11820
ggaaattgta agcgttaata ttttgttaaa attcgcgtta aatttttgtt aaatcagctc 11880
attttttaac caataggccg aaatcggcaa aatcccttat aaatcaaaag aatagaccga 11940
gatagggttg agtgttgttc cagtttggaa caagagtcca ctattaaaga acgtggactc 12000
caacgtcaaa gggcgaaaaa ccgtctatca gggcgatggc ccactacgtg aaccatcacc 12060
ctaatcaagt tttttggggt cgaggtgccg taaagcacta aatcggaacc ctaaagggag 12120
cccccgattt agagcttgac ggggaaagcc ggcgaacgtg gcgagaaagg aagggaagaa 12180
agcgaaagga gcgggcgcta gggcgctggc aagtgtagcg gtcacgctgc gcgtaaccac 12240
cacacccgcc gcgcttaatg cgccgctaca gggcgcgtcc attcgccatt caggctgcgc 12300
aactgttggg aagggcgatc ggtgcgggcc tcttcgctat tacgccagct ggcgaaaggg 12360

ggatgtgctg caaggcgatt aagttgggta acgccagggt tttcccagtc acgacgttgt 12420

aaaacgacgg ccagtgaatt gtaatacgac tcactatagg gcgaattggg cccgacgtcg 12480

catgccaagg cgtatattag ttggtgggaa ccagtgtacg accgggtcct gtataaccag 12540

gttcagtggc atacttgtag gagttgttcc cgtggtattt gggcatggct aagacatttc 12600

gccgaccaat gttaagtgca caatagccga tgtagtagat gtaagccaga tggttttgga 12660

gcaggtcgat tcgagaccac agattgaaag tgcctcgatg gcaggcctcg ttttctcctc 12720

cttcacagac agacactttg tcgagtgcag ggtagacgct ttcttggtca atgtacactt 12780

ctccaatggc gtgtgtgtaa ttggaccaat ctggcagtcc aacgaagata tcgttccagt 12840

gggtgagcct gtagtttcgt cgcttgtcgt ttacttcaag gcctgtgtca ttaaaccaca 12900

gctggttgat gtagtttgca aactctgagt ttcctactct cggctggcca aagttgatca 12960

tggtaggatc atgtccaagt aatttgaagt gagttgcgaa aagaagagct tgagcagcgc 13020

ccagcgagtg accagtaaca tacattttgt agtcagtgtg gttggtgagg aacttttcaa 13080

actgaggagc agcattgacc atagtttcgt tgaaggcctt ggcgaaccca tcatggatca 13140

tgcagccttt gcactcacta gttttgatgg gaataagacg gggtcttca accaccagag 13200

cccgctcttt gaggttgtca agacctttgt tctccacttc caagtctggt cggactgccc 13260

atctctgtta attaactcac ctgcaggatt gagactatga atggattccc gtgcccgtat 13320

tactctacta atttgatctt ggaacgcgaa aatacgtttc taggactcca aagaatctca 13380

actcttgtcc ttactaaata tactacccat agttgatggt ttacttgaac agagaggaca 13440

tgttcacttg acccaaagtt tctcgcatct cttggatatt tgaacaacgg cgtccactga 13500

ccgtcagtta tccagtcaca aaaccccac attcatacat tcccatgtac gtttacaaag 13560

ttctcaattc catcgtgcaa atcaaaatca catctattca ttcatcatat ataaacccat 13620

catgtctact aacactcaca actccataga aaacatcgac tcagaacaca cgctccatgc 13680

ggccgcttag gcgagagtag caacggcagg acatccagcg gcagagccgt cggtagaacc 13740

gtcgggttgg gcagccacgg cagcagacag aacagggcag acatcctcca aagatccagc 13800

agtaggagca agtttgtcgt tgtccgcagg cttggtaccc attgctcgca ggtgctccac 13860

catgccgtag agggcatcgg agaactgaga gtagttcttg tagggaagtc cgtactcttc 13920

gcacacaccc ttgacaacgg gagcaatggt gggatagttg gcgtggctga tagaaggaaa 13980

cagatggtgc tcgatctgat gattgagacc tcctgacaag tggttcgaga agtaaccgcc 14040

agatcgccag ttgacacagc aaagaacctg ggaagctgcc cagtcgttgg gtggaggtgt 14100

gggatgcttc tgtttcgcct tggcctcctg ttcggcttgc ttgagcatct ccgttcgtct 14160

ggcagcgatg acagaggagg ggttgaggta gtcacaagac tcggaaatgt ggttgataat 14220

gaagcaaact gccaggtact cgcctgaaac gaggtgggca gtgatgaaca gagccaaacc 14280

atgtgcaatg ccgtgcaggt aacagggaag aacgatctgc atgagaaagc ccatgatctt 14340
tgctccccag aaaagcatct ggccctcgag aggcacaagt cgggcagaaa agtcgatcgg 14400
acctcgacgc ttggcgaggc agaaggtgaa atcgctaatg ataaccttgg agatagtcat 14460
gagggcgaac aggggaaagg catagacgtg ctgcagttgg tgatgaggtt tccacggagt 14520
gtcgggatgc attcgcatga gagggtaggt tccgaacacg tcgggatcgt tctctggaag 14580
ggcaaattcg ggatcggaaa caaggttggt gtattgatgg tggccaatga catgctggta 14640
ctcccaggct gtggacgaag caccaatgac gtccataccc catccggcca gtcgattgag 14700
acggccagac ttcgaaaagg caccgtggtt tccgtcgtgc tgaatggaga ggccgatgtg 14760
acttccagca agaccccaca cagctgccca gaaaaacgag ccctgagtga ccatgagata 14820
gaaggaagcg gcaaacgcag ccataaccag tgcagccttg actgacagtc cgcctcgtct 14880
gggctgacca gcctccttga gggtttgcac gactcgctgc ttgagttccg cgtaaaaggc 14940
agaagcctgc cagtcgtaga aagttcgatg atcctgaaga gtgccgattc tgtacttctc 15000
cataaccttg tcgggtcgac cagcagggtg gtaggactcc acgagaatgg tagagtctcg 15060
acccagtcca agggaaatga catcgccacc gggatgcttt ccgataaact ctgtaatgtc 15120
gtaaacgccg tcgtgacagg ccagccaacc atcggtggga acgatgtgtc gtgcgacttc 15180
tcgcatggtg aactttcggt cctgaccggt tccagaagta gcgagggcgt catagtaggc 15240
agctggaggg tttcccacgg gtcggatggg tggaggcagc gaggcaatct cggagggttt 15300
gccaggacct ccaggcttga ccttggccat gggcaggacc tgtgttagta cattgtcggg 15360
gagtcatcaa ttggttcgac aggttgtcga ctgttagtat gagctcaatt gggctctggt 15420
gggtcgatga cacttgtcat ctgtttctgt tgggtcatgt ttccatcacc ttctatggta 15480
ctcacaattc gtccgattcg cccgaatccg ttaataccga ctttgatggc catgttgatg 15540
tgtgtttaat tcaagaatga atatagagaa gagaagaaga aaaaagattc aattgagccg 15600
gcgatgcaga cccttatata aatgttgcct tggacagacg gagcaagccc gcccaaacct 15660
acgttcggta taatatgtta agctttttaa cacaaaggtt tggcttgggg taacctgatg 15720
tggtgcaaaa gaccgggcgt tggcgagcca ttgcgcgggc gaatggggcc gtgactcgtc 15780
tcaaattcga gggcgtgcct caattcgtgc cccgtggct ttttcccgcc gtttccgccc 15840
cgtttgcacc actgcagccg cttctttggt tcggacacct tgctgcgagc taggtgcctt 15900
gtgctactta aaaagtggcc tcccaacacc aacatgacat gagtgcgtgg gccaagacac 15960
gttggcgggg tcgcagtcgg ctcaatggcc cggaaaaaac gctgctggag ctggttcgga 16020
cgcagtccgc cgcggcgtat ggatatccgc aaggttccat agcgccattg ccctccgtcg 16080
gcgtctatcc cgcaacctct aaatagagcg ggaatataac ccaagcttct ttttttcct 16140
ttaacacgca cacccccaac tatcatgttg ctgctgctgt ttgactctac tctgtggagg 16200
ggtgctccca cccaacccaa cctacaggtg gatccggcgc tgtgattggc tgataagtct 16260

cctatccgga ctaattctga ccaatgggac atgcgcgcag gacccaaatg ccgcaattac 16320

gtaaccccaa cgaaatgcct accctcttt ggagcccagc ggccccaaat ccccccaagc 16380

agcccggttc taccggcttc catctccaag cacaagcagc ccgg 16424

<210> 61
<211> 900
<212> DNA
<213> Euglena anabaena

<220>
<221> CDS
<222> (1)..(900)
<223> synthetic C20 elongase (codon-optimized for Yarrowia lipolytica) ("EaC20ES")

<300>
<302> MULTIZYMES AND THEIR USE IN MAKING POLYUNSATURATED FATTY ACIDS
<310> US-2008-0254191-A1
<311> 2008-04-03
<312> 2008-10-16
<313> (1)..(900)

<400> 61

```
atg gcc gag ggc aag tcc gac ggt ccc gtc gtt acc ctc cag tcc atg        48
Met Ala Glu Gly Lys Ser Asp Gly Pro Val Val Thr Leu Gln Ser Met
1               5                   10                  15

tgg aag ccc ctg gct ctc atg gcc atc gac gtc ggc atc ctg gtc aac        96
Trp Lys Pro Leu Ala Leu Met Ala Ile Asp Val Gly Ile Leu Val Asn
            20                  25                  30

gtg cga cgg aag gcc ttc acc gag ttc gac gga cac tcg aac gtc ttc       144
Val Arg Arg Lys Ala Phe Thr Glu Phe Asp Gly His Ser Asn Val Phe
        35                  40                  45

gcc gat ccc gtg tac att ccc ttt gtc atg aac ctg ttc tac ctc acc       192
Ala Asp Pro Val Tyr Ile Pro Phe Val Met Asn Leu Phe Tyr Leu Thr
    50                  55                  60

atg atc ttt gct ggc tgc cga tgg atg aag act cga gaa ccc ttc gag       240
Met Ile Phe Ala Gly Cys Arg Trp Met Lys Thr Arg Glu Pro Phe Glu
65                  70                  75                  80

atc aag tcc tac atg ttt gcc tac aac gct tac cag aca atg atg aac       288
Ile Lys Ser Tyr Met Phe Ala Tyr Asn Ala Tyr Gln Thr Met Met Asn
                85                  90                  95

ttt ctc att gtg gtc ggc ttc atg tat gag gtt cac tcc acc ggt atg       336
Phe Leu Ile Val Val Gly Phe Met Tyr Glu Val His Ser Thr Gly Met
            100                 105                 110

cga tac tgg gga tcc aga atc gac act tct acc aag ggc ttg gga ctg       384
Arg Tyr Trp Gly Ser Arg Ile Asp Thr Ser Thr Lys Gly Leu Gly Leu
        115                 120                 125

ggt ttc ctc atc tat gcc cat tac cac aac aag tac gtg gag tac gtc       432
Gly Phe Leu Ile Tyr Ala His Tyr His Asn Lys Tyr Val Glu Tyr Val
    130                 135                 140

gac acc ctg ttc atg att ctg cgg aag aaa aac aat cag atc tcg ttc       480
```

```
Asp Thr Leu Phe Met Ile Leu Arg Lys Lys Asn Asn Gln Ile Ser Phe
145                 150                 155                 160

ctt cac gtt tac cac cat tcc ctg ctc act tgg gca tgg tgg gct gtg      528
Leu His Val Tyr His His Ser Leu Leu Thr Trp Ala Trp Trp Ala Val
                165                 170                 175

gtc tac tgg gct cct ggc gga gat gcc tgg ttc ggt gcc tgt tac aac      576
Val Tyr Trp Ala Pro Gly Gly Asp Ala Trp Phe Gly Ala Cys Tyr Asn
            180                 185                 190

tcc ttc atc cac gtt ctc atg tac tcc tac tat ctg ttt gcc acc ttc      624
Ser Phe Ile His Val Leu Met Tyr Ser Tyr Tyr Leu Phe Ala Thr Phe
        195                 200                 205

ggc att cga tgt ccc tgg aaa aag atg ctc acc cag ttg caa atg gtc      672
Gly Ile Arg Cys Pro Trp Lys Lys Met Leu Thr Gln Leu Gln Met Val
    210                 215                 220

cag ttc tgc ttt tgc ttc gct cat gcc atg tac gtt gga tgg ctt ggt      720
Gln Phe Cys Phe Cys Phe Ala His Ala Met Tyr Val Gly Trp Leu Gly
225                 230                 235                 240

cac gag gtg tac cct cga tgg ctc act gct ctg cag gcc ttt gtg atg      768
His Glu Val Tyr Pro Arg Trp Leu Thr Ala Leu Gln Ala Phe Val Met
                245                 250                 255

ctc aac atg ctg gtc ctc ttt ggc aac ttc tac atg aag tct tac tcc      816
Leu Asn Met Leu Val Leu Phe Gly Asn Phe Tyr Met Lys Ser Tyr Ser
            260                 265                 270

aag gcg agc aag ctc gaa cca gcc tct ccc gtg tcg cct gcc tct ctt      864
Lys Ala Ser Lys Leu Glu Pro Ala Ser Pro Val Ser Pro Ala Ser Leu
        275                 280                 285

gct cag aag ccc ttc gag aac gcc aag gtc aag taa                      900
Ala Gln Lys Pro Phe Glu Asn Ala Lys Val Lys
    290                 295
```

<210> 62
<211> 299
<212> PRT
<213> Euglena anabaena

<400> 62

```
Met Ala Glu Gly Lys Ser Asp Gly Pro Val Val Thr Leu Gln Ser Met
1               5                   10                  15

Trp Lys Pro Leu Ala Leu Met Ala Ile Asp Val Gly Ile Leu Val Asn
            20                  25                  30

Val Arg Arg Lys Ala Phe Thr Glu Phe Asp Gly His Ser Asn Val Phe
        35                  40                  45

Ala Asp Pro Val Tyr Ile Pro Phe Val Met Asn Leu Phe Tyr Leu Thr
    50                  55                  60

Met Ile Phe Ala Gly Cys Arg Trp Met Lys Thr Arg Glu Pro Phe Glu
65                  70                  75                  80
```

```
Ile Lys Ser Tyr Met Phe Ala Tyr Asn Ala Tyr Gln Thr Met Met Asn
                85                  90                  95

Phe Leu Ile Val Val Gly Phe Met Tyr Glu Val His Ser Thr Gly Met
            100                 105                 110

Arg Tyr Trp Gly Ser Arg Ile Asp Thr Ser Thr Lys Gly Leu Gly Leu
        115                 120                 125

Gly Phe Leu Ile Tyr Ala His Tyr His Asn Lys Tyr Val Glu Tyr Val
    130                 135                 140

Asp Thr Leu Phe Met Ile Leu Arg Lys Lys Asn Asn Gln Ile Ser Phe
145                 150                 155                 160

Leu His Val Tyr His His Ser Leu Leu Thr Trp Ala Trp Trp Ala Val
                165                 170                 175

Val Tyr Trp Ala Pro Gly Gly Asp Ala Trp Phe Gly Ala Cys Tyr Asn
            180                 185                 190

Ser Phe Ile His Val Leu Met Tyr Ser Tyr Tyr Leu Phe Ala Thr Phe
        195                 200                 205

Gly Ile Arg Cys Pro Trp Lys Lys Met Leu Thr Gln Leu Gln Met Val
    210                 215                 220

Gln Phe Cys Phe Cys Phe Ala His Ala Met Tyr Val Gly Trp Leu Gly
225                 230                 235                 240

His Glu Val Tyr Pro Arg Trp Leu Thr Ala Leu Gln Ala Phe Val Met
                245                 250                 255

Leu Asn Met Leu Val Leu Phe Gly Asn Phe Tyr Met Lys Ser Tyr Ser
            260                 265                 270

Lys Ala Ser Lys Leu Glu Pro Ala Ser Pro Val Ser Pro Ala Ser Leu
        275                 280                 285

Ala Gln Lys Pro Phe Glu Asn Ala Lys Val Lys
    290                 295
```

<210> 63
<211> 912
<212> DNA
<213> Euglena gracilis

<220>

<221> CDS
<222> (1)..(912)
<223> synthetic C20 elongase (codon-optimized for Yarrowia lipolytica) ("EgC20ES")

<300>
<302> MULTIZYMES AND THEIR USE IN MAKING POLYUNSATURATED FATTY ACIDS
<310> US-2008-0254191-A1
<311> 2008-04-03
<312> 2008-10-16
<313> (1)..(912)

<400> 63

```
atg gct gac tct ccc gtc atc aac ctc tcc acc atg tgg aag cct ctg        48
Met Ala Asp Ser Pro Val Ile Asn Leu Ser Thr Met Trp Lys Pro Leu
1               5                   10                  15

tcg ctc atg gcc ttg gat ctt gct gtt ctg gga cac gtc tgg aag cag        96
Ser Leu Met Ala Leu Asp Leu Ala Val Leu Gly His Val Trp Lys Gln
            20                  25                  30

gca caa cag gag ggc tcc atc tcg gct tac gcc gac tct gtg tgg act       144
Ala Gln Gln Glu Gly Ser Ile Ser Ala Tyr Ala Asp Ser Val Trp Thr
        35                  40                  45

ccc ctc atc atg tcc ggt ctg tac ctc tcc atg atc ttc gtg gga tgt       192
Pro Leu Ile Met Ser Gly Leu Tyr Leu Ser Met Ile Phe Val Gly Cys
    50                  55                  60

cga tgg atg aag aac cga gag ccc ttc gaa atc aag acc tac atg ttt       240
Arg Trp Met Lys Asn Arg Glu Pro Phe Glu Ile Lys Thr Tyr Met Phe
65                  70                  75                  80

gcc tac aac ctg tac cag acc ctc atg aac ctt tgc att gtg ctg ggc       288
Ala Tyr Asn Leu Tyr Gln Thr Leu Met Asn Leu Cys Ile Val Leu Gly
                85                  90                  95

ttc ctc tac cag gtc cac gct acc ggt atg cga ttc tgg gga tct ggc       336
Phe Leu Tyr Gln Val His Ala Thr Gly Met Arg Phe Trp Gly Ser Gly
            100                 105                 110

gtg gac cga tcg ccc aag ggt ctg gga att ggc ttt ttc atc tat gcc       384
Val Asp Arg Ser Pro Lys Gly Leu Gly Ile Gly Phe Phe Ile Tyr Ala
        115                 120                 125

cat tac cac aac aag tac gtc gag tac ttc gac aca ctc ttc atg gtg       432
His Tyr His Asn Lys Tyr Val Glu Tyr Phe Asp Thr Leu Phe Met Val
    130                 135                 140

ctg cgg aaa aag aac aac cag att tcc ttt ctt cac gtc tac cat cac       480
Leu Arg Lys Lys Asn Asn Gln Ile Ser Phe Leu His Val Tyr His His
145                 150                 155                 160

gct ctg ctc acc tgg gct tgg ttt gcc gtg gtc tac ttc gct cct gga       528
Ala Leu Leu Thr Trp Ala Trp Phe Ala Val Val Tyr Phe Ala Pro Gly
                165                 170                 175

ggt gac ggc tgg ttt gga gcc tgc tac aat tcc tcc att cat gtc ctg       576
Gly Asp Gly Trp Phe Gly Ala Cys Tyr Asn Ser Ser Ile His Val Leu
            180                 185                 190

atg tac tct tac tat ctg ctt gcc acc ttc ggc atc tcc tgt ccc tgg       624
Met Tyr Ser Tyr Tyr Leu Leu Ala Thr Phe Gly Ile Ser Cys Pro Trp
        195                 200                 205
```

```
aaa aag atc ctc acc cag ctg caa atg gtt cag ttc tgc ttt tgc ttc         672
Lys Lys Ile Leu Thr Gln Leu Gln Met Val Gln Phe Cys Phe Cys Phe
    210                 215                 220

acc cac tcg atc tac gtg tgg att tgc ggt tcc gaa atc tac cct cga         720
Thr His Ser Ile Tyr Val Trp Ile Cys Gly Ser Glu Ile Tyr Pro Arg
225                 230                 235                 240

ccc ttg act gct ctc cag tcc ttc gtg atg gtc aac atg ctg gtt ctc         768
Pro Leu Thr Ala Leu Gln Ser Phe Val Met Val Asn Met Leu Val Leu
                245                 250                 255

ttt ggc aac ttc tac gtc aag cag tat tct cag aag aat gga aag ccc         816
Phe Gly Asn Phe Tyr Val Lys Gln Tyr Ser Gln Lys Asn Gly Lys Pro
            260                 265                 270

gag aac ggt gcc act cct gag aac ggt gcc aag cct cag ccc tgc gag         864
Glu Asn Gly Ala Thr Pro Glu Asn Gly Ala Lys Pro Gln Pro Cys Glu
        275                 280                 285

aac ggc acc gtc gag aag cga gag aac gac act gcc aac gtt cga taa         912
Asn Gly Thr Val Glu Lys Arg Glu Asn Asp Thr Ala Asn Val Arg
    290                 295                 300
```

<210> 64
<211> 303
<212> PRT
<213> Euglena gracilis

<400> 64

```
Met Ala Asp Ser Pro Val Ile Asn Leu Ser Thr Met Trp Lys Pro Leu
1               5                   10                  15

Ser Leu Met Ala Leu Asp Leu Ala Val Leu Gly His Val Trp Lys Gln
            20                  25                  30

Ala Gln Gln Glu Gly Ser Ile Ser Ala Tyr Ala Asp Ser Val Trp Thr
        35                  40                  45

Pro Leu Ile Met Ser Gly Leu Tyr Leu Ser Met Ile Phe Val Gly Cys
    50                  55                  60

Arg Trp Met Lys Asn Arg Glu Pro Phe Glu Ile Lys Thr Tyr Met Phe
65                  70                  75                  80

Ala Tyr Asn Leu Tyr Gln Thr Leu Met Asn Leu Cys Ile Val Leu Gly
                85                  90                  95

Phe Leu Tyr Gln Val His Ala Thr Gly Met Arg Phe Trp Gly Ser Gly
            100                 105                 110

Val Asp Arg Ser Pro Lys Gly Leu Gly Ile Gly Phe Phe Ile Tyr Ala
        115                 120                 125

His Tyr His Asn Lys Tyr Val Glu Tyr Phe Asp Thr Leu Phe Met Val
```

```
        130                 135                 140

Leu Arg Lys Lys Asn Asn Gln Ile Ser Phe Leu His Val Tyr His His
145                 150                 155                 160

Ala Leu Leu Thr Trp Ala Trp Phe Ala Val Val Tyr Phe Ala Pro Gly
                165                 170                 175

Gly Asp Gly Trp Phe Gly Ala Cys Tyr Asn Ser Ser Ile His Val Leu
            180                 185                 190

Met Tyr Ser Tyr Tyr Leu Leu Ala Thr Phe Gly Ile Ser Cys Pro Trp
        195                 200                 205

Lys Lys Ile Leu Thr Gln Leu Gln Met Val Gln Phe Cys Phe Cys Phe
    210                 215                 220

Thr His Ser Ile Tyr Val Trp Ile Cys Gly Ser Glu Ile Tyr Pro Arg
225                 230                 235                 240

Pro Leu Thr Ala Leu Gln Ser Phe Val Met Val Asn Met Leu Val Leu
                245                 250                 255

Phe Gly Asn Phe Tyr Val Lys Gln Tyr Ser Gln Lys Asn Gly Lys Pro
            260                 265                 270

Glu Asn Gly Ala Thr Pro Glu Asn Gly Ala Lys Pro Gln Pro Cys Glu
        275                 280                 285

Asn Gly Thr Val Glu Lys Arg Glu Asn Asp Thr Ala Asn Val Arg
    290                 295                 300
```

<210> 65
<211> 1644
<212> DNA
<213> Euglena anabaena

<220>
<221> CDS
<222> (1)..(1644)
<223> synthetic truncated delta-4 desaturase (codon-optimized for Yarrowia lipolytica)

<400> 65

```
atg gcc aag gtc aaa ccc ggt gga cct ggc aag ccc tcg gag atc gct         48
Met Ala Lys Val Lys Pro Gly Gly Pro Gly Lys Pro Ser Glu Ile Ala
1               5                   10                  15

tct ctt cca cct ccc att cga cct gtt ggc aac cca cct gca gcc tat         96
Ser Leu Pro Pro Pro Ile Arg Pro Val Gly Asn Pro Pro Ala Ala Tyr
            20                  25                  30

tac gac gct ctg gcc acc tcc ggt act gga cag gac cga aag ttt acc        144
```

```
Tyr Asp Ala Leu Ala Thr Ser Gly Thr Gly Gln Asp Arg Lys Phe Thr
        35                  40                  45

atg cga gag gtc gct cga cac att gtt ccc acc gat ggc tgg ttg gcc        192
Met Arg Glu Val Ala Arg His Ile Val Pro Thr Asp Gly Trp Leu Ala
    50                  55                  60

tgt cac gac ggt gtg tac gac atc acc gag ttc att ggc aag cat ccc        240
Cys His Asp Gly Val Tyr Asp Ile Thr Glu Phe Ile Gly Lys His Pro
65                  70                  75                  80

ggt gga gat gtt atc tct ctc ggt ctc gga cga gac tcc act att ctg        288
Gly Gly Asp Val Ile Ser Leu Gly Leu Gly Arg Asp Ser Thr Ile Leu
                85                  90                  95

gtc gaa tcg tac cat cct gca gga cga ccc gac aag gtt atg gag aag        336
Val Glu Ser Tyr His Pro Ala Gly Arg Pro Asp Lys Val Met Glu Lys
            100                 105                 110

tac cga atc ggt aca ctt cag gat cac aga acc ttc tac gac tgg cag        384
Tyr Arg Ile Gly Thr Leu Gln Asp His Arg Thr Phe Tyr Asp Trp Gln
        115                 120                 125

gcc tcc gct ttc tac gcc gag ctc aag cag cga gtg gtt cag act ctc        432
Ala Ser Ala Phe Tyr Ala Glu Leu Lys Gln Arg Val Val Gln Thr Leu
    130                 135                 140

aag gag gct gga caa cct cga cgt ggt ggc ctg tct gtc aag gca gcc        480
Lys Glu Ala Gly Gln Pro Arg Arg Gly Gly Leu Ser Val Lys Ala Ala
145                 150                 155                 160

ctt gtt atg gct gcc ttt gct gcc tcg ttc tac ctc atg gtg aca cag        528
Leu Val Met Ala Ala Phe Ala Ala Ser Phe Tyr Leu Met Val Thr Gln
                165                 170                 175

gga tcc ttc ttt tgg gct gcc gtc tgg ggt ctg gca ggc tct cac att        576
Gly Ser Phe Phe Trp Ala Ala Val Trp Gly Leu Ala Gly Ser His Ile
            180                 185                 190

gga ctc agc atc cag cac gac ggc aat cat ggt gct ttc tcc aag tct        624
Gly Leu Ser Ile Gln His Asp Gly Asn His Gly Ala Phe Ser Lys Ser
        195                 200                 205

gga cga ctc aac cgt ctt gct ggc tgg ggt atg gac gtt atc gga gcc        672
Gly Arg Leu Asn Arg Leu Ala Gly Trp Gly Met Asp Val Ile Gly Ala
    210                 215                 220

tcc tcg act gcc tgg gag tac caa cac gtc att ggt cat cac cag tac        720
Ser Ser Thr Ala Trp Glu Tyr Gln His Val Ile Gly His His Gln Tyr
225                 230                 235                 240

acc aac ctg gtg tcc gat ccc gag ttt gct ctt ccc gag aac gat cca        768
Thr Asn Leu Val Ser Asp Pro Glu Phe Ala Leu Pro Glu Asn Asp Pro
                245                 250                 255

gac gtt ttc gga acc tat ccc ctc atg cgg atg cat ccg gac act cct        816
Asp Val Phe Gly Thr Tyr Pro Leu Met Arg Met His Pro Asp Thr Pro
            260                 265                 270

tgg aaa ccc cac cat cag ctg caa cac gtg tac gcc ttt ccg ttg ttc        864
Trp Lys Pro His His Gln Leu Gln His Val Tyr Ala Phe Pro Leu Phe
        275                 280                 285

gct ctc atg acc atc agc aag gtc att atc tcc gat ttc acg ttt tgt        912
Ala Leu Met Thr Ile Ser Lys Val Ile Ile Ser Asp Phe Thr Phe Cys
```

290 295 300

ctt gcc aag cga cgt ggt ccc atc gac ttc tct gcc aga ctc gtt ccc 960
Leu Ala Lys Arg Arg Gly Pro Ile Asp Phe Ser Ala Arg Leu Val Pro
305 310 315 320

ctc gag ggt cag atg ctg ttc tgg ggt gca aag atc atg ggc ttt ctc 1008
Leu Glu Gly Gln Met Leu Phe Trp Gly Ala Lys Ile Met Gly Phe Leu
325 330 335

atg cag att gtg ctt ccc tgc tac ctg cat ggc atc gct cac gga ttg 1056
Met Gln Ile Val Leu Pro Cys Tyr Leu His Gly Ile Ala His Gly Leu
340 345 350

gcc ctc ttc att aca gct cat ctg gtt tct ggc gag tac ctt gcc gtc 1104
Ala Leu Phe Ile Thr Ala His Leu Val Ser Gly Glu Tyr Leu Ala Val
355 360 365

tgt ttc att atc aac cac att tcc gag tcg tgc gac tac ctc aat ccc 1152
Cys Phe Ile Ile Asn His Ile Ser Glu Ser Cys Asp Tyr Leu Asn Pro
370 375 380

tct tcc gtt atc gct gcc cga cgg acc gaa atg ctc aag cag gcc gag 1200
Ser Ser Val Ile Ala Ala Arg Arg Thr Glu Met Leu Lys Gln Ala Glu
385 390 395 400

cag gaa gcc aag gcg aaa cag aag cac ccc act cca cct ccc aac gac 1248
Gln Glu Ala Lys Ala Lys Gln Lys His Pro Thr Pro Pro Pro Asn Asp
405 410 415

tgg gct gcc tcc caa gtt ctg tgt tgc gtc aac tgg cga tct ggt ggc 1296
Trp Ala Ala Ser Gln Val Leu Cys Cys Val Asn Trp Arg Ser Gly Gly
420 425 430

tac ttt tca aac cac ctt tct ggt gga ctc aac cac cag atc gag cat 1344
Tyr Phe Ser Asn His Leu Ser Gly Gly Leu Asn His Gln Ile Glu His
435 440 445

cac ctg ttt ccc agc att tct cac gcc aac tat ccc acc att gct cct 1392
His Leu Phe Pro Ser Ile Ser His Ala Asn Tyr Pro Thr Ile Ala Pro
450 455 460

gtt gtc aag ggc gtg tgc gag gaa tac ggt ctt ccc tac aag aac tac 1440
Val Val Lys Gly Val Cys Glu Glu Tyr Gly Leu Pro Tyr Lys Asn Tyr
465 470 475 480

tct cag ttt tcc gat gct ctg tac gga atg gtc gag cac ttg cga gct 1488
Ser Gln Phe Ser Asp Ala Leu Tyr Gly Met Val Glu His Leu Arg Ala
485 490 495

atg ggc acc aaa cct gca gac aac gac aag ctt gct ccc act gca ggt 1536
Met Gly Thr Lys Pro Ala Asp Asn Asp Lys Leu Ala Pro Thr Ala Gly
500 505 510

tcc ctg gag gat gtt tgt cct gtg ctc tct gct gcc gtt gct gcc caa 1584
Ser Leu Glu Asp Val Cys Pro Val Leu Ser Ala Ala Val Ala Ala Gln
515 520 525

ccc gac ggc tcc acc gac gga tct gct gcc ggt tgt cct gct gtc gcc 1632
Pro Asp Gly Ser Thr Asp Gly Ser Ala Ala Gly Cys Pro Ala Val Ala
530 535 540

act ctg gct taa 1644
Thr Leu Ala
545

<210> 66
<211> 547
<212> PRT
<213> Euglena anabaena

<400> 66

```
Met Ala Lys Val Lys Pro Gly Gly Pro Gly Lys Pro Ser Glu Ile Ala
1               5                   10                  15

Ser Leu Pro Pro Pro Ile Arg Pro Val Gly Asn Pro Pro Ala Ala Tyr
            20                  25                  30

Tyr Asp Ala Leu Ala Thr Ser Gly Thr Gly Gln Asp Arg Lys Phe Thr
        35                  40                  45

Met Arg Glu Val Ala Arg His Ile Val Pro Thr Asp Gly Trp Leu Ala
    50                  55                  60

Cys His Asp Gly Val Tyr Asp Ile Thr Glu Phe Ile Gly Lys His Pro
65                  70                  75                  80

Gly Gly Asp Val Ile Ser Leu Gly Leu Gly Arg Asp Ser Thr Ile Leu
                85                  90                  95

Val Glu Ser Tyr His Pro Ala Gly Arg Pro Asp Lys Val Met Glu Lys
            100                 105                 110

Tyr Arg Ile Gly Thr Leu Gln Asp His Arg Thr Phe Tyr Asp Trp Gln
        115                 120                 125

Ala Ser Ala Phe Tyr Ala Glu Leu Lys Gln Arg Val Val Gln Thr Leu
    130                 135                 140

Lys Glu Ala Gly Gln Pro Arg Arg Gly Gly Leu Ser Val Lys Ala Ala
145                 150                 155                 160

Leu Val Met Ala Ala Phe Ala Ala Ser Phe Tyr Leu Met Val Thr Gln
                165                 170                 175

Gly Ser Phe Phe Trp Ala Ala Val Trp Gly Leu Ala Gly Ser His Ile
            180                 185                 190

Gly Leu Ser Ile Gln His Asp Gly Asn His Gly Ala Phe Ser Lys Ser
        195                 200                 205

Gly Arg Leu Asn Arg Leu Ala Gly Trp Gly Met Asp Val Ile Gly Ala
    210                 215                 220
```

Ser Ser Thr Ala Trp Glu Tyr Gln His Val Ile Gly His His Gln Tyr
225 230 235 240

Thr Asn Leu Val Ser Asp Pro Glu Phe Ala Leu Pro Glu Asn Asp Pro
245 250 255

Asp Val Phe Gly Thr Tyr Pro Leu Met Arg Met His Pro Asp Thr Pro
260 265 270

Trp Lys Pro His His Gln Leu Gln His Val Tyr Ala Phe Pro Leu Phe
275 280 285

Ala Leu Met Thr Ile Ser Lys Val Ile Ile Ser Asp Phe Thr Phe Cys
290 295 300

Leu Ala Lys Arg Arg Gly Pro Ile Asp Phe Ser Ala Arg Leu Val Pro
305 310 315 320

Leu Glu Gly Gln Met Leu Phe Trp Gly Ala Lys Ile Met Gly Phe Leu
325 330 335

Met Gln Ile Val Leu Pro Cys Tyr Leu His Gly Ile Ala His Gly Leu
340 345 350

Ala Leu Phe Ile Thr Ala His Leu Val Ser Gly Glu Tyr Leu Ala Val
355 360 365

Cys Phe Ile Ile Asn His Ile Ser Glu Ser Cys Asp Tyr Leu Asn Pro
370 375 380

Ser Ser Val Ile Ala Ala Arg Arg Thr Glu Met Leu Lys Gln Ala Glu
385 390 395 400

Gln Glu Ala Lys Ala Lys Gln Lys His Pro Thr Pro Pro Pro Asn Asp
405 410 415

Trp Ala Ala Ser Gln Val Leu Cys Cys Val Asn Trp Arg Ser Gly Gly
420 425 430

Tyr Phe Ser Asn His Leu Ser Gly Gly Leu Asn His Gln Ile Glu His
435 440 445

His Leu Phe Pro Ser Ile Ser His Ala Asn Tyr Pro Thr Ile Ala Pro
450 455 460

Val Val Lys Gly Val Cys Glu Glu Tyr Gly Leu Pro Tyr Lys Asn Tyr
465 470 475 480

Ser Gln Phe Ser Asp Ala Leu Tyr Gly Met Val Glu His Leu Arg Ala

```
                485                 490                 495
Met Gly Thr Lys Pro Ala Asp Asn Asp Lys Leu Ala Pro Thr Ala Gly
            500                 505                 510
Ser Leu Glu Asp Val Cys Pro Val Leu Ser Ala Ala Val Ala Ala Gln
        515                 520                 525
Pro Asp Gly Ser Thr Asp Gly Ser Ala Ala Gly Cys Pro Ala Val Ala
    530                 535                 540
Thr Leu Ala
545
```

<210> 67
<211> 1644
<212> DNA
<213> Euglena anabaena

<220>
<221> CDS
<222> (1)..(1644)
<223> synthetic truncated delta-4 desaturase (codon-optimized for Yarrowia lipolytica)

<400> 67

```
atg gcc aag gtc aag cct gga ggt cct ggc aaa ccc tcc gag att gcc        48
Met Ala Lys Val Lys Pro Gly Gly Pro Gly Lys Pro Ser Glu Ile Ala
1               5                   10                  15

tcg ctg cct cca ccc atc cga ccc gtg gga aac cct cca gct gcc tac        96
Ser Leu Pro Pro Pro Ile Arg Pro Val Gly Asn Pro Pro Ala Ala Tyr
            20                  25                  30

tat gac gcc ctc gct act tct gga acc ggt cag gac cga aag ttc acc       144
Tyr Asp Ala Leu Ala Thr Ser Gly Thr Gly Gln Asp Arg Lys Phe Thr
        35                  40                  45

atg cga gaa gtc gca cga cac atc gtt ccc acc gat ggt tgg ctg gcc       192
Met Arg Glu Val Ala Arg His Ile Val Pro Thr Asp Gly Trp Leu Ala
    50                  55                  60

tgt cac gac ggc gtt tac gac att aca gag ttt atc gga aag cat ccc       240
Cys His Asp Gly Val Tyr Asp Ile Thr Glu Phe Ile Gly Lys His Pro
65                  70                  75                  80

ggt ggc gat gtc att tcc ctt gga ctg ggt cga gac tct acc att ctc       288
Gly Gly Asp Val Ile Ser Leu Gly Leu Gly Arg Asp Ser Thr Ile Leu
                85                  90                  95

gtg gag tcc tac cac cct gct ggt cga ccc gac aag gtt atg gag aag       336
Val Glu Ser Tyr His Pro Ala Gly Arg Pro Asp Lys Val Met Glu Lys
            100                 105                 110

tac aga atc ggc act ctt cag gat cat cga act ttc tac gac tgg cag       384
Tyr Arg Ile Gly Thr Leu Gln Asp His Arg Thr Phe Tyr Asp Trp Gln
        115                 120                 125

gct tct gcc ttt tac gcg gaa ctc aag cag cga gtc gtg caa acc ctc       432
```

```
Ala Ser Ala Phe Tyr Ala Glu Leu Lys Gln Arg Val Val Gln Thr Leu
    130                 135                 140

aag gag gct ggt cag ccc aga cga ggc gga ctg tca gtc aag gct gca      480
Lys Glu Ala Gly Gln Pro Arg Arg Gly Gly Leu Ser Val Lys Ala Ala
145                 150                 155                 160

ctg gtt atg gct gcg ttt gcc gct tcc ttc tat ctc atg gtc act cag      528
Leu Val Met Ala Ala Phe Ala Ala Ser Phe Tyr Leu Met Val Thr Gln
                165                 170                 175

ggc tcg ttt ttc tgg gca gct gtg tgg ggt ctt gct gga agt cac atc      576
Gly Ser Phe Phe Trp Ala Ala Val Trp Gly Leu Ala Gly Ser His Ile
            180                 185                 190

ggc ctc tcc att cag cac gac gga aac cac ggt gcc ttt tcg aag tct      624
Gly Leu Ser Ile Gln His Asp Gly Asn His Gly Ala Phe Ser Lys Ser
        195                 200                 205

ggc cgt ctc aat cga ctg gcc gga tgg ggt atg gac gtc att ggt gct      672
Gly Arg Leu Asn Arg Leu Ala Gly Trp Gly Met Asp Val Ile Gly Ala
    210                 215                 220

tcg tcc aca gcc tgg gag tac cag cat gtc att ggc cac cat caa tac      720
Ser Ser Thr Ala Trp Glu Tyr Gln His Val Ile Gly His His Gln Tyr
225                 230                 235                 240

acc aac ctt gtt tcc gat ccc gaa ttt gcc ctt cca gag aac gat ccc      768
Thr Asn Leu Val Ser Asp Pro Glu Phe Ala Leu Pro Glu Asn Asp Pro
                245                 250                 255

gac gtg ttc gga acc tac cct ctc atg cga atg cat ccc gac act ccg      816
Asp Val Phe Gly Thr Tyr Pro Leu Met Arg Met His Pro Asp Thr Pro
            260                 265                 270

tgg aaa cct cat cac caa ctg cag cac gtc tat gcc ttt ccc ctg ttc      864
Trp Lys Pro His His Gln Leu Gln His Val Tyr Ala Phe Pro Leu Phe
        275                 280                 285

gcc ctc atg act atc tcc aag gtt atc att agc gat ttc acc ttc tgc      912
Ala Leu Met Thr Ile Ser Lys Val Ile Ile Ser Asp Phe Thr Phe Cys
    290                 295                 300

ctc gcc aag cgt cga ggt ccg atc gac ttt tct gcc cga ctt gtg cct      960
Leu Ala Lys Arg Arg Gly Pro Ile Asp Phe Ser Ala Arg Leu Val Pro
305                 310                 315                 320

ctc gag ggc cag atg ctt ttc tgg gga gca aag atc atg ggc ttt ctc     1008
Leu Glu Gly Gln Met Leu Phe Trp Gly Ala Lys Ile Met Gly Phe Leu
                325                 330                 335

atg cag atc gtt ctt ccc tgt tac ctg cac ggc att gca cat ggt ttg     1056
Met Gln Ile Val Leu Pro Cys Tyr Leu His Gly Ile Ala His Gly Leu
            340                 345                 350

gct ctg ttc atc act gcc cac ctc gtt tca ggc gag tac ctg gca gtt     1104
Ala Leu Phe Ile Thr Ala His Leu Val Ser Gly Glu Tyr Leu Ala Val
        355                 360                 365

tgc ttc att atc aac cac att tcc gag tct tgt gac tac ctc aac ccc     1152
Cys Phe Ile Ile Asn His Ile Ser Glu Ser Cys Asp Tyr Leu Asn Pro
    370                 375                 380

tcc tct gtc atc gct gcc aga cga acg gag atg ctc aag caa gcc gaa     1200
Ser Ser Val Ile Ala Ala Arg Arg Thr Glu Met Leu Lys Gln Ala Glu
```

```
385                 390                 395                 400

cag gag gcc aag gcg aaa cag aag cat ccc aca cct cca ccc aac gac      1248
Gln Glu Ala Lys Ala Lys Gln Lys His Pro Thr Pro Pro Pro Asn Asp
                405                 410                 415

tgg gca gct tcc cag gtt ctt tgc tgt gtc aac tgg cga tct ggc ggt      1296
Trp Ala Ala Ser Gln Val Leu Cys Cys Val Asn Trp Arg Ser Gly Gly
            420                 425                 430

tac ttc tcg aac cac ttg tca gga ggt ctc aat cat cag atc gag cac      1344
Tyr Phe Ser Asn His Leu Ser Gly Gly Leu Asn His Gln Ile Glu His
        435                 440                 445

cat ctg ttt cct tct atc agc cac gcc aac tat ccc acc att gct ccc      1392
His Leu Phe Pro Ser Ile Ser His Ala Asn Tyr Pro Thr Ile Ala Pro
    450                 455                 460

gtt gtc aag ggt gtg tgc gaa gag tac gga ctt ccc tac aag aac tac      1440
Val Val Lys Gly Val Cys Glu Glu Tyr Gly Leu Pro Tyr Lys Asn Tyr
465                 470                 475                 480

tct cag ttc tcc gat gcc ctc tac ggc atg gtg gag cac ctg cga gca      1488
Ser Gln Phe Ser Asp Ala Leu Tyr Gly Met Val Glu His Leu Arg Ala
                485                 490                 495

atg ggt acc aag cct gcg gac aac gac aaa ctt gct cct act gct gga      1536
Met Gly Thr Lys Pro Ala Asp Asn Asp Lys Leu Ala Pro Thr Ala Gly
            500                 505                 510

tct ttg gag gat gtc tgc cct gtt ctg tct gct gcc gtg gct gcc caa      1584
Ser Leu Glu Asp Val Cys Pro Val Leu Ser Ala Ala Val Ala Ala Gln
        515                 520                 525

ccc gac ggt tct acc gac ggc tct gcc gct gga tgt cct gcc gtt gct      1632
Pro Asp Gly Ser Thr Asp Gly Ser Ala Ala Gly Cys Pro Ala Val Ala
    530                 535                 540

act ctc gcc taa                                                      1644
Thr Leu Ala
545
```

<210> 68
<211> 547
<212> PRT
<213> Euglena anabaena

<400> 68

```
Met Ala Lys Val Lys Pro Gly Gly Pro Gly Lys Pro Ser Glu Ile Ala
1               5                   10                  15

Ser Leu Pro Pro Pro Ile Arg Pro Val Gly Asn Pro Pro Ala Ala Tyr
            20                  25                  30

Tyr Asp Ala Leu Ala Thr Ser Gly Thr Gly Gln Asp Arg Lys Phe Thr
        35                  40                  45

Met Arg Glu Val Ala Arg His Ile Val Pro Thr Asp Gly Trp Leu Ala
    50                  55                  60
```

Cys His Asp Gly Val Tyr Asp Ile Thr Glu Phe Ile Gly Lys His Pro
65 70 75 80

Gly Gly Asp Val Ile Ser Leu Gly Leu Gly Arg Asp Ser Thr Ile Leu
85 90 95

Val Glu Ser Tyr His Pro Ala Gly Arg Pro Asp Lys Val Met Glu Lys
100 105 110

Tyr Arg Ile Gly Thr Leu Gln Asp His Arg Thr Phe Tyr Asp Trp Gln
115 120 125

Ala Ser Ala Phe Tyr Ala Glu Leu Lys Gln Arg Val Val Gln Thr Leu
130 135 140

Lys Glu Ala Gly Gln Pro Arg Arg Gly Gly Leu Ser Val Lys Ala Ala
145 150 155 160

Leu Val Met Ala Ala Phe Ala Ala Ser Phe Tyr Leu Met Val Thr Gln
165 170 175

Gly Ser Phe Phe Trp Ala Ala Val Trp Gly Leu Ala Gly Ser His Ile
180 185 190

Gly Leu Ser Ile Gln His Asp Gly Asn His Gly Ala Phe Ser Lys Ser
195 200 205

Gly Arg Leu Asn Arg Leu Ala Gly Trp Gly Met Asp Val Ile Gly Ala
210 215 220

Ser Ser Thr Ala Trp Glu Tyr Gln His Val Ile Gly His His Gln Tyr
225 230 235 240

Thr Asn Leu Val Ser Asp Pro Glu Phe Ala Leu Pro Glu Asn Asp Pro
245 250 255

Asp Val Phe Gly Thr Tyr Pro Leu Met Arg Met His Pro Asp Thr Pro
260 265 270

Trp Lys Pro His His Gln Leu Gln His Val Tyr Ala Phe Pro Leu Phe
275 280 285

Ala Leu Met Thr Ile Ser Lys Val Ile Ile Ser Asp Phe Thr Phe Cys
290 295 300

Leu Ala Lys Arg Arg Gly Pro Ile Asp Phe Ser Ala Arg Leu Val Pro
305 310 315 320

```
Leu Glu Gly Gln Met Leu Phe Trp Gly Ala Lys Ile Met Gly Phe Leu
                325                 330                 335

Met Gln Ile Val Leu Pro Cys Tyr Leu His Gly Ile Ala His Gly Leu
            340                 345                 350

Ala Leu Phe Ile Thr Ala His Leu Val Ser Gly Glu Tyr Leu Ala Val
        355                 360                 365

Cys Phe Ile Ile Asn His Ile Ser Glu Ser Cys Asp Tyr Leu Asn Pro
    370                 375                 380

Ser Ser Val Ile Ala Ala Arg Arg Thr Glu Met Leu Lys Gln Ala Glu
385                 390                 395                 400

Gln Glu Ala Lys Ala Lys Gln Lys His Pro Thr Pro Pro Pro Asn Asp
                405                 410                 415

Trp Ala Ala Ser Gln Val Leu Cys Cys Val Asn Trp Arg Ser Gly Gly
            420                 425                 430

Tyr Phe Ser Asn His Leu Ser Gly Gly Leu Asn His Gln Ile Glu His
        435                 440                 445

His Leu Phe Pro Ser Ile Ser His Ala Asn Tyr Pro Thr Ile Ala Pro
    450                 455                 460

Val Val Lys Gly Val Cys Glu Glu Tyr Gly Leu Pro Tyr Lys Asn Tyr
465                 470                 475                 480

Ser Gln Phe Ser Asp Ala Leu Tyr Gly Met Val Glu His Leu Arg Ala
                485                 490                 495

Met Gly Thr Lys Pro Ala Asp Asn Asp Lys Leu Ala Pro Thr Ala Gly
            500                 505                 510

Ser Leu Glu Asp Val Cys Pro Val Leu Ser Ala Ala Val Ala Ala Gln
        515                 520                 525

Pro Asp Gly Ser Thr Asp Gly Ser Ala Ala Gly Cys Pro Ala Val Ala
    530                 535                 540

Thr Leu Ala
545
```

<210> 69
<211> 17088

<212> DNA
<213> Artificial Sequence

<220>
<223> Plasmid pZKL3-4GER44

<400> 69

aattctctct cttgagcttt tccataacaa gttcttctgc ctccaggaag tccatgggtg 60
gtttgatcat ggttttggtg tagtggtagt gcagtggtgg tattgtgact ggggatgtag 120
ttgagaataa gtcatacaca agtcagcttt cttcgagcct catataagta taagtagttc 180
aacgtattag cactgtaccc agcatctccg tatcgagaaa cacaacaaca tgccccattg 240
gacagatcat gcggatacac aggttgtgca gtatcataca tactcgatca gacaggtcgt 300
ctgaccatca tacaagctga acaagcgctc catacttgca cgctctctat atacacagtt 360
aaattacata tccatagtct aacctctaac agttaatctt ctggtaagcc tcccagccag 420
ccttctggta tcgcttggcc tcctcaatag gatctcggtt ctggccgtac agacctcggc 480
cgacaattat gatatccgtt ccggtagaca tgacatcctc aacagttcgg tactgctgtc 540
cgagagcgtc tcccttgtcg tcaagaccca ccccgggggt cagaataagc cagtcctcag 600
agtcgccctt aggtcggttc tgggcaatga agccaaccac aaactcgggg tcggatcggg 660
caagctcaat ggtctgcttg gagtactcgc cagtggccag agagcccttg caagacagct 720
cggccagcat gagcagacct ctggccagct tctcgttggg agaggggact aggaactcct 780
tgtactggga gttctcgtag tcagagacgt cctccttctt ctgttcagag acagtttcct 840
cggcaccagc tcgcaggcca gcaatgattc cggttccggg tacaccgtgg gcgttggtga 900
tatcggacca ctcggcgatt cggtgacacc ggtactggtg cttgacagtg ttgccaatat 960
ctgcgaactt tctgtcctcg aacaggaaga aaccgtgctt aagagcaagt tccttgaggg 1020
ggagcacagt gccggcgtag gtgaagtcgt caatgatgtc gatatgggtt ttgatcatgc 1080
acacataagg tccgacctta tcggcaagct caatgagctc cttggtggtg gtaacatcca 1140
gagaagcaca caggttggtt ttcttggctg ccacgagctt gagcactcga gcggcaaagg 1200
cggacttgtg gacgttagct cgagcttcgt aggagggcat tttggtggtg aagaggagac 1260
tgaaataaat ttagtctgca gaacttttta tcggaacctt atctggggca gtgaagtata 1320
tgttatggta atagttacga gttagttgaa cttatagata gactggacta tacggctatc 1380
ggtccaaatt agaaagaacg tcaatggctc tctgggcgtc gcctttgccg acaaaaatgt 1440
gatcatgatg aaagccagca atgacgttgc agctgatatt gttgtcggcc aaccgcgccg 1500
aaaacgcagc tgtcagaccc acagcctcca acgaagaatg tatcgtcaaa gtgatccaag 1560
cacactcata gttggagtcg tactccaaag gcggcaatga cgagtcagac agatactcgt 1620
cgaccttttc cttgggaacc accaccgtca gcccttctga ctcacgtatt gtagccaccg 1680
acacaggcaa cagtccgtgg atagcagaat atgtcttgtc ggtccatttc tcaccaactt 1740
taggcgtcaa gtgaatgttg cagaagaagt atgtgccttc attgagaatc ggtgttgctg 1800
atttcaataa agtcttgaga tcagtttggc cagtcatgtt gtggggggta attggattga 1860

gttatcgcct acagtctgta caggtatact cgctgcccac tttatacttt ttgattccgc 1920
tgcacttgaa gcaatgtcgt ttaccaaaag tgagaatgct ccacagaaca caccccaggg 1980
tatggttgag caaaaaataa acactccgat acggggaatc gaaccccggt ctccacggtt 2040
ctcaagaagt attcttgatg agagcgtatc gatggttaat gctgctgtgt gctgtgtgtg 2100
tgtgttgttt ggcgctcatt gttgcgttat gcagcgtaca ccacaatatt ggaagcttat 2160
tagcctttct attttttcgt ttgcaaggct taacaacatt gctgtggaga gggatgggga 2220
tatggaggcc gctggaggga gtcggagagg cgttttggag cggcttggcc tggcgcccag 2280
ctcgcgaaac gcacctagga ccctttggca cgccgaaatg tgccactttt cagtctagta 2340
acgccttacc tacgtcattc catgcgtgca tgtttgcgcc tttttccct tgcccttgat 2400
cgccacacag tacagtgcac tgtacagtgg aggttttggg ggggtcttag atgggagcta 2460
aaagcggcct agcggtacac tagtgggatt gtatggagtg gcatggagcc taggtggagc 2520
ctgacaggac gcacgaccgg ctagcccgtg acagacgatg ggtggctcct gttgtccacc 2580
gcgtacaaat gtttgggcca aagtcttgtc agccttgctt gcgaacctaa ttcccaattt 2640
tgtcacttcg cacccccatt gatcgagccc taacccctgc ccatcaggca atccaattaa 2700
gctcgcattg tctgccttgt ttagtttggc tcctgcccgt ttcggcgtcc acttgcacaa 2760
acacaaacaa gcattatata taaggctcgt ctctccctcc caaccacact cactttttg 2820
cccgtcttcc cttgctaaca caaaagtcaa gaacacaaac aaccacccca accccttac 2880
acacaagaca tatctacagc aatggccatg gctcagtcca ccaaggctgc cgacactgct 2940
gccaccgaca agtctctcga caagaaccga ctcatctccc gagacgagct gcggtctcac 3000
aacgttcccc aggatgcctg ggctgccgtc cacggcagag tcatcaacat taccgagttc 3060
gcccgacggc atcctggtgg cgacatcatt ctgcttgccg caggaaagga tgccaccgtg 3120
ctcttcgaga cttaccatcc tcgaggtgtt cccacctcga tcctcgacaa gctgcaggtc 3180
ggcaagatga aggacggaga acttccctcc tcgttctact cgtgggattc cgacttttac 3240
aagaccctgc gagctcgagt ggtcgagcga ttggacaagc tcaacctgcc tcgaagaggt 3300
ggctacgaga tttgggtcaa ggcagtattc ctcctggctg gattctggtt cagcctctac 3360
aagatgtccg tcaacgagac ctactgggct gcctcgctgt ggtccgtgtc tatgggagtc 3420
tttgctgcct tcatcggcac ttgcattcaa cacgatggaa accacggtgc cttctcgacc 3480
agccctgctc tcaacaaggt tgcaggctgg actctggaca tgatcggtgc ttctggcttt 3540
acatgggaga ttcagcatat gctcggacac catccctaca ccaacgtcct ggacgtggac 3600
gaagagaagc gaaaggaagc tggcgacgat tgtcctatgg aggacaagga tcaggagtcc 3660
gacccagatg tcttctcttc gtttcctctc atgcgaatgc acccctacca caaggccgag 3720
tggtaccacc gatatcagca cctgtacgca cccgttctct ttgctttcat gactcttgcc 3780

aaggtgttcc aacaggacat cgaagtcgct accactcagc gactgtacca catcgacgcc 3840

aagtgccgat acaattccat tctcaatgtc cttcggtttt ggtcgatgaa ggtgctctcc 3900

atcggctaca tgctggctgt tccctgctac ttccacggaa tccttggtgg ccttggactg 3960

tttctcatcg gccactttgc ctgtggagag cttctggcaa ccatgttcat tgtcaatcac 4020

gtcatcgagg gtgtgtcctt tggcaaaaag ggagaatctc tcggtctgtc caaggacgtg 4080

gagttcaagc ctacaaccgt ttctggacga actccaatgg agcagacccg tgccgaggcc 4140

aaaaaggctg ccaatggagg caacgtcaag gatgttccct acaacgactg ggctgccgtt 4200

cagtgtcaaa cgagcgtcaa ctggtctcct ggatcgtggt tctggaatca cttctccggt 4260

ggcctctccc accagatcga gcaccatctg tttcccagca tttgtcacac caactacgct 4320

cacatccagg acgttgtcca gaagacttgc gaagagtacg gtgttcctta ccagtccgaa 4380

ccctctttgt tctccgccta tggcaagatg ctgtctcatc tcaagtacct cggaaacgag 4440

aaaaaggtcg cttaagcggc cgcatgtaca tacaagatta tttatagaaa tgaatcgcga 4500

tcgaacaaag agtacgagtg tacgagtagg ggatgatgat aaaagtggaa gaagttccgc 4560

atctttggat ttatcaacgt gtaggacgat acttcctgta aaaatgcaat gtctttacca 4620

taggttctgc tgtagatgtt attaactacc attaacatgt ctacttgtac agttgcagac 4680

cagttggagt atagaatggt acacttacca aaaagtgttg atggttgtaa ctacgatata 4740

taaaactgtt gacgggatct gcgtacactg tttaaacaga gtgtgaaaga ctcactatgg 4800

tccgggctta tctcgaccaa tagccaaagt ctggagtttc tgagagaaaa aggcaagata 4860

cgtatgtaac aaagcgacgc atggtacaat aataccggag gcatgtatca tagagagtta 4920

gtggttcgat gatggcactg gtgcctggta tgactttata cggctgacta catatttgtc 4980

ctcagacata caattacagt caagcactta cccttggaca tctgtaggta ccccccggcc 5040

aagacgatct cagcgtgtcg tatgtcggat tggcgtagct ccctcgctcg tcaattggct 5100

cccatctact ttcttctgct tggctacacc cagcatgtct gctatggctc gttttcgtgc 5160

cttatctatc ctcccagtat taccaactct aaatgacatg atgtgattgg gtctacactt 5220

tcatatcaga gataaggagt agcacagttg cataaaaagc ccaactctaa tcagcttctt 5280

cctttcttgt aattagtaca aaggtgatta gcgaaatctg gaagcttagt tggccctaaa 5340

aaaatcaaaa aaagcaaaaa acgaaaaacg aaaaaccaca gttttgagaa cagggaggta 5400

acgaaggatc gtatatatat atatatatat atatacccac ggatcccgag accggccttt 5460

gattcttccc tacaaccaac cattctcacc accctaattc acaaccatgg ccaaggtcaa 5520

acccggtgga cctggcaagc cctcggagat cgcttctctt ccacctccca ttcgacctgt 5580

tggcaaccca cctgcagcct attacgacgc tctggccacc tccggtactg gacaggaccg 5640

aaagtttacc atgcgagagg tcgctcgaca cattgttccc accgatggct ggttggcctg 5700

tcacgacggt gtgtacgaca tcaccgagtt cattggcaag catcccggtg gagatgttat 5760

ctctctcggt ctcggacgag actccactat tctggtcgaa tcgtaccatc ctgcaggacg 5820
acccgacaag gttatggaga agtaccgaat cggtacactt caggatcaca gaaccttcta 5880
cgactggcag gcctccgctt tctacgccga gctcaagcag cgagtggttc agactctcaa 5940
ggaggctgga caacctcgac gtggtggcct gtctgtcaag gcagcccttg ttatggctgc 6000
ctttgctgcc tcgttctacc tcatggtgac acagggatcc ttcttttggg ctgccgtctg 6060
gggtctggca ggctctcaca ttggactcag catccagcac gacggcaatc atggtgcttt 6120
ctccaagtct ggacgactca accgtcttgc tggctggggt atggacgtta tcggagcctc 6180
ctcgactgcc tgggagtacc aacacgtcat tggtcatcac cagtacacca acctggtgtc 6240
cgatcccgag tttgctcttc ccgagaacga tccagacgtt ttcggaacct atcccctcat 6300
gcggatgcat ccggacactc cttggaaacc ccaccatcag ctgcaacacg tgtacgcctt 6360
tccgttgttc gctctcatga ccatcagcaa ggtcattatc tccgatttca cgttttgtct 6420
tgccaagcga cgtggtccca tcgacttctc tgccagactc gttcccctcg agggtcagat 6480
gctgttctgg ggtgcaaaga tcatgggctt tctcatgcag attgtgcttc cctgctacct 6540
gcatggcatc gctcacggat tggccctctt cattacagct catctggttt ctggcgagta 6600
ccttgccgtc tgtttcatta tcaaccacat ttccgagtcg tgcgactacc tcaatccctc 6660
ttccgttatc gctgccccac ggaccgaaat gctcaagcag gccgagcagg aagccaaggc 6720
gaaacagaag caccccactc cacctcccaa cgactgggct gcctcccaag ttctgtgttg 6780
cgtcaactgg cgatctggtg gctacttttc aaaccacctt tctggtggac tcaaccacca 6840
gatcgagcat cacctgtttc ccagcatttc tcacgccaac tatcccacca ttgctcctgt 6900
tgtcaagggc gtgtgcgagg aatacggtct tccctacaag aactactctc agttttccga 6960
tgctctgtac ggaatggtcg agcacttgcg agctatgggc accaaacctg cagacaacga 7020
caagcttgct cccactgcag gttccctgga ggatgtttgt cctgtgctct ctgctgccgt 7080
tgctgcccaa cccgacggct ccaccgacgg atctgctgcc ggttgtcctg ctgtcgccac 7140
tctggcttaa gcggccgcat gagaagataa atatataaat acattgagat attaaatgcg 7200
ctagattaga gagcctcata ctgctcggag agaagccaag acgagtactc aaaggggatt 7260
acaccatcca tatccacaga cacaagctgg ggaaaggttc tatatacact ttccggaata 7320
ccgtagtttc cgatgttatc aatgggggca gccaggattt caggcacttc ggtgtctcgg 7380
ggtgaaatgg cgttcttggc ctccatcaag tcgtaccatg tcttcatttg cctgtcaaag 7440
taaaacagaa gcagatgaag aatgaacttg aagtgaagga atttaaattg ccccggagaa 7500
gacggccagg ccgcctagat gacaaattca acaactcaca gctgactttc tgccattgcc 7560
actagggggg ggccttttta tatggccaag ccaagctctc cacgtcggtt gggctgcacc 7620
caacaataaa tgggtagggt tgcaccaaca aagggatggg atgggggta gaagatacga 7680

ggataacggg gctcaatggc acaaataaga acgaatactg ccattaagac tcgtgatcca 7740

gcgactgaca ccattgcatc atctaagggc ctcaaaacta cctcggaact gctgcgctga 7800

tctggacacc acagaggttc cgagcacttt aggttgcacc aaatgtccca ccaggtgcag 7860

gcagaaaacg ctggaacagc gtgtacagtt tgtcttaaca aaaagtgagg gcgctgaggt 7920

cgagcagggt ggtgtgactt gttatagcct ttagagctgc gaaagcgcgt atggatttgg 7980

ctcatcaggc cagattgagg gtctgtggac acatgtcatg ttagtgtact tcaatcgccc 8040

cctggatata gccccgacaa taggccgtgg cctcattttt ttgccttccg cacatttcca 8100

ttgctcggta cccacacctt gcttctcctg cacttgccaa ccttaatact ggtttacatt 8160

gaccaacatc ttacaagcgg ggggcttgtc tagggtatat ataaacagtg gctctcccaa 8220

tcggttgcca gtctcttttt tcctttcttt ccccacagat tcgaaatcta aactacacat 8280

cacagaactc cgagccgtga gtatccacga caagatcagt gtcgagacga cgcgttttgt 8340

gtaatgacac aatccgaaag tcgctagcaa cacacactct ctacacaaac taacccagct 8400

ctggtaccat ggctgactct cccgtcatca acctctccac catgtggaag cctctgtcgc 8460

tcatggcctt ggatcttgct gttctgggac acgtctggaa gcaggcacaa caggagggct 8520

ccatctcggc ttacgccgac tctgtgtgga ctcccctcat catgtccggt ctgtacctct 8580

ccatgatctt cgtgggatgt cgatggatga agaaccgaga gcccttcgaa atcaagacct 8640

acatgtttgc ctacaacctg taccagaccc tcatgaacct ttgcattgtg ctgggcttcc 8700

tctaccaggt ccacgctacc ggtatgcgat tctggggatc tggcgtggac cgatcgccca 8760

agggtctggg aattggcttt ttcatctatg cccattacca caacaagtac gtcgagtact 8820

tcgacacact cttcatggtg ctgcggaaaa agaacaacca gatttccttt cttcacgtct 8880

accatcacgc tctgctcacc tgggcttggt ttgccgtggt ctacttcgct cctggaggtg 8940

acggctggtt tggagcctgc tacaattcct ccattcatgt cctgatgtac tcttactatc 9000

tgcttgccac cttcggcatc tcctgtccct ggaaaaagat cctcacccag ctgcaaatgg 9060

ttcagttctg cttttgcttc acccactcga tctacgtgtg gatttgcggt tccgaaatct 9120

accctcgacc cttgactgct ctccagtcct tcgtgatggt caacatgctg gttctctttg 9180

gcaacttcta cgtcaagcag tattctcaga agaatggaaa gcccgagaac ggtgccactc 9240

ctgagaacgg tgccaagcct cagccctgcg agaacggcac cgtcgagaag cgagagaacg 9300

acactgccaa cgttcgataa gcggccgcaa gtgtggatgg ggaagtgagt gcccggttct 9360

gtgtgcacaa ttggcaatcc aagatggatg gattcaacac agggatatag cgagctacgt 9420

ggtggtgcga ggatatagca acggatattt atgtttgaca cttgagaatg tacgatacaa 9480

gcactgtcca agtacaatac taaacatact gtacatactc atactcgtac ccgggcaacg 9540

gtttcacttg agtgcagtgg ctagtgctct tactcgtaca gtgtgcaata ctgcgtatca 9600

tagtctttga tgtatatcgt attcattcat gttagttgcg tacggattgt gtatgtccct 9660

gtacctgcat cttgatggag agagctccgg aaagcggatc aggagctgtc caattttaat 9720
tttataacat ggaaacgagt ccttggagct agaagaccat tttttcaact gccctatcga 9780
ctatatttat ctactccaaa accgactgct tcccaagaat cttcagccaa ggcttccaaa 9840
gtaacccctc gcttcccgac acttaattga aaccttagat gcagtcactg cgagtgaagt 9900
ggactctaac atctccaaca tagcgacgat attgcgaggg tttgaatata actaagatgc 9960
atgatccatt acatttgtag aaatatcata aacaacgaag cacatagaca gaatgctgtt 10020
ggttgttaca tctgaagccg aggtaccgat gtcattttca gctgtcactg cagagacagg 10080
ggtatgtcac atttgaagat catacaaccg acgtttatga aaaccagaga tatagagaat 10140
gtattgacgg ttgtggctat gtcataagtg cagtgaagtg cagtgattat aggtatagta 10200
cacttactgt agctacaagt acatactgct acagtaatac tcatgtatgc aaaccgtatt 10260
ctgtgtctac agaaggcgat acggaagagt caatctctta tgtagagcca tttctataat 10320
cgaaggggcc ttgtaatttc caaacgagta attgagtaat tgaagagcat cgtagacatt 10380
acttatcatg tattgtgaga gggaggagat gcagctgtag ctactgcaca tactgtactc 10440
gcccatgcag ggataatgca tagcgagact tggcagtagg tgacagttgc tagctgctac 10500
ttgtagtcgg gtgggtgata gcatggcgcg ccagctgcat taatgaatcg gccaacgcgc 10560
ggggagaggc ggtttgcgta ttgggcgctc ttccgcttcc tcgctcactg actcgctgcg 10620
ctcggtcgtt cggctgcggc gagcggtatc agctcactca aaggcggtaa tacggttatc 10680
cacagaatca ggggataacg caggaaagaa catgtgagca aaaggccagc aaaaggccag 10740
gaaccgtaaa aaggccgcgt tgctggcgtt tttccatagg ctccgccccc ctgacgagca 10800
tcacaaaaat cgacgctcaa gtcagaggtg gcgaaacccg acaggactat aaagatacca 10860
ggcgtttccc cctggaagct ccctcgtgcg ctctcctgtt ccgaccctgc cgcttaccgg 10920
atacctgtcc gcctttctcc cttcgggaag cgtggcgctt tctcatagct cacgctgtag 10980
gtatctcagt tcggtgtagg tcgttcgctc caagctgggc tgtgtgcacg aaccccccgt 11040
tcagcccgac cgctgcgcct tatccggtaa ctatcgtctt gagtccaacc cggtaagaca 11100
cgacttatcg ccactggcag cagccactgg taacaggatt agcagagcga ggtatgtagg 11160
cggtgctaca gagttcttga agtggtggcc taactacggc tacactagaa gaacagtatt 11220
tggtatctgc gctctgctga agccagttac cttcggaaaa agagttggta gctcttgatc 11280
cggcaaacaa accaccgctg gtagcggtgg tttttttgtt tgcaagcagc agattacgcg 11340
cagaaaaaaa ggatctcaag aagatccttt gatcttttct acggggtctg acgctcagtg 11400
gaacgaaaac tcacgttaag ggattttggt catgagatta tcaaaaagga tcttcaccta 11460
gatcctttta aattaaaaat gaagttttaa atcaatctaa agtatatatg agtaaacttg 11520
gtctgacagt taccaatgct taatcagtga ggcacctatc tcagcgatct gtctatttcg 11580

ttcatccata gttgcctgac tccccgtcgt gtagataact acgatacggg agggcttacc 11640
atctggcccc agtgctgcaa tgataccgcg agacccacgc tcaccggctc cagatttatc 11700
agcaataaac cagccagccg gaagggccga gcgcagaagt ggtcctgcaa ctttatccgc 11760
ctccatccag tctattaatt gttgccggga agctagagta agtagttcgc cagttaatag 11820
tttgcgcaac gttgttgcca ttgctacagg catcgtggtg tcacgctcgt cgtttggtat 11880
ggcttcattc agctccggtt cccaacgatc aaggcgagtt acatgatccc ccatgttgtg 11940
caaaaaagcg gttagctcct tcggtcctcc gatcgttgtc agaagtaagt tggccgcagt 12000
gttatcactc atggttatgg cagcactgca taattctctt actgtcatgc catccgtaag 12060
atgcttttct gtgactggtg agtactcaac caagtcattc tgagaatagt gtatgcggcg 12120
accgagttgc tcttgcccgg cgtcaatacg ggataatacc gcgccacata gcagaacttt 12180
aaaagtgctc atcattggaa aacgttcttc ggggcgaaaa ctctcaagga tcttaccgct 12240
gttgagatcc agttcgatgt aacccactcg tgcacccaac tgatcttcag catcttttac 12300
tttcaccagc gtttctgggt gagcaaaaac aggaaggcaa aatgccgcaa aaaagggaat 12360
aagggcgaca cggaaatgtt gaatactcat actcttcctt tttcaatatt attgaagcat 12420
ttatcagggt tattgtctca tgagcggata catatttgaa tgtatttaga aaaataaaca 12480
aataggggtt ccgcgcacat ttccccgaaa agtgccacct gatgcggtgt gaaataccgc 12540
acagatgcgt aaggagaaaa taccgcatca ggaaattgta agcgttaata ttttgttaaa 12600
attcgcgtta aatttttgtt aaatcagctc attttttaac caataggccg aaatcggcaa 12660
aatcccttat aaatcaaaag aatagaccga gatagggttg agtgttgttc cagtttggaa 12720
caagagtcca ctattaaaga acgtggactc caacgtcaaa gggcgaaaaa ccgtctatca 12780
gggcgatggc ccactacgtg aaccatcacc ctaatcaagt tttttggggt cgaggtgccg 12840
taaagcacta aatcggaacc ctaaagggag cccccgattt agagcttgac ggggaaagcc 12900
ggcgaacgtg gcgagaaagg aagggaagaa agcgaaagga gcgggcgcta gggcgctggc 12960
aagtgtagcg gtcacgctgc gcgtaaccac cacacccgcc gcgcttaatg cgccgctaca 13020
gggcgcgtcc attcgccatt caggctgcgc aactgttggg aagggcgatc ggtgcgggcc 13080
tcttcgctat tacgccagct ggcgaaaggg ggatgtgctg caaggcgatt aagttgggta 13140
acgccagggt tttcccagtc acgacgttgt aaaacgacgg ccagtgaatt gtaatacgac 13200
tcactatagg gcgaattggg cccgacgtcg catgcaggaa tagacatctt caataggagc 13260
attaatacct gtgggatcac tgatgtaaac ttctcccaga gtatgtgaat aaccagcggg 13320
ccatccaaca aagaagtcgt tccagtgagt gactcggtac atccgtcttt cggggttgat 13380
ggtaagtccg tcgtctcctt gcttaaagaa cagagcgtcc acgtagtctg caaaagcctt 13440
gtttccaagt cgaggctgcc catagttgat tagcgttgga tcatatccaa gattcttcag 13500
gttgatgccc atgaatagag cagtgacagc tcctagagag tggccagtta cgatcaattt 13560

gtagtcagtg ttgtttccaa ggaagtcgac cagacgatcc tgtacgttca ccatagtctc 13620
tctgtatgcc ttctgaaagc catcatgaac ttggcagcca ggacaattga tactggcaga 13680
agggtttgtg gagtttatgt cagtagtgtt aagaggaggg atactggtca tgtagggttg 13740
ttggatcgtt tggatgtcag taatagcgtc tgcaatggag aaagtgcctc ggaaaacaat 13800
atacttttcc tttttggtgt gatcgtgggc caaaaatcca gtaactgaag tcgagaagaa 13860
atttcctcca aactggtagt caagagtcac atcgggaaaa tgagcgcaag agtttccaca 13920
ggtaaaatcg ctctgcaggg caaatgggcc aggggctctg acacaatagg ccacgttaga 13980
tagccatccg tacttgagaa caaagtcgta tgtctcctgg gtgataggag ccgttaatta 14040
actcacctgc aggattgaga ctatgaatgg attcccgtgc ccgtattact ctactaattt 14100
gatcttggaa cgcgaaaata cgtttctagg actccaaaga atctcaactc ttgtccttac 14160
taaatatact acccatagtt gatggtttac ttgaacagag aggacatgtt cacttgaccc 14220
aaagtttctc gcatctcttg gatatttgaa caacggcgtc cactgaccgt cagttatcca 14280
gtcacaaaac ccccacattc atacattccc atgtacgttt acaaagttct caattccatc 14340
gtgcaaatca aaatcacatc tattcattca tcatatataa acccatcatg tctactaaca 14400
ctcacaactc catagaaaac atcgactcag aacacacgct ccatgcggcc gcttaggact 14460
ttttgtcgcc gttggtaggc acgggaggag cacccatgag tcgcagatgc tgaaccatgc 14520
cacacacggc atcccagaac gtaacgtagt tcttgtaggg caatccgtat tcctcgcaca 14580
cttccttgac aactcgagca atgatgggat agttcgcgtg agagatgctg ggaaacagat 14640
ggtgctcgat ctgatggttg aggccaccgg agaggtgatt ggccagcacg cctccagatc 14700
gccagttgac acagcactgg acttgtgtca cagcccaatc gttgggtgga ggagtgggct 14760
tgaccttttt cgcctcggct gcctgatgag ctgcctggag catctcggtc cgtcgggcag 14820
cagtttgaaa ggaggtattc ataaattcgc aagactcgga gatgtggtta atgatgaaac 14880
agatggccag gtactctcca gacacaaggt gggcaacaga gaacagagca aggcccatag 14940
cagttccgtg gaggtagcag ggcagcacaa tctgcaagag aaagttcgcc agcttggctc 15000
cccagaacaa cagctggcct tcgagtggaa ccagtctgga cgaacagtcg atagaaccct 15060
ttttcatgga gagacaaaca gcaaagtcgc tggtgagcac cttggaaatg gtcataagag 15120
cgaagagagg aaaggcgaac aggtgttgaa atcggtgatg aggctgccaa gcagtgtcgg 15180
gatgcattcg catgagagga tagctggaaa acacatctgg gtcgttctcg ggaaggctga 15240
acagcgtatc ggaaacgaga ttcgtgtact gatggtgtcc aatgacatgc tggtactccc 15300
acacggtgga cgaggcaccg atcaagtcca tgccccatcc tgcgagtctg ttgaccaggg 15360
tggatcgaga gaaagcaccg tggttgccat cgtgttgaat gctcagtccg acatgcgaac 15420
cggcaaagcc ccagacggca gcccacagga aagacttgtg ggcaacccac atgtaccagg 15480

agacaaagaa gagggtaagc accaggagag ccttgactcc cagtccacct cgacgtgcct 15540
gtccagcctc cttgagtcgt gcaagagccc gtcgtttgag ctcagggtaa aagtccgatt 15600
ctccccaagc gtagaaagtc ttgggatcct ggagtgtgcc gatacggtac ttctccatga 15660
ccttgtctgg tcgtccagcg ggatggtagg actcgaccag aatggtgcag tctcgaccaa 15720
gtccgagagt gatgacgcca cctccaggat gcttggccag gaaatcggtg acgtcgtaca 15780
caccttcgtg acaggtgagc catccatcgg tgggaagaat gtgtcgtctg acctcgtctg 15840
tggtgaacag tcgctccttg ccctgtcccg acacggcgag agagtcatag taagttgcgg 15900
gtggaagacc ggcaggtcta gcgggtcgaa cattggcggt gtcgttttct cgcttctcca 15960
cggtaccgtt ctcgcaaggt tgcggtttgg ccatgggcag gacctgtgtt agtacattgt 16020
cggggagtca tcaattggtt cgacaggttg tcgactgtta gtatgagctc aattgggctc 16080
tggtgggtcg atgacacttg tcatctgttt ctgttgggtc atgtttccat caccttctat 16140
ggtactcaca attcgtccga ttcgcccgaa tccgttaata ccgactttga tggccatgtt 16200
gatgtgtgtt taattcaaga atgaatatag agaagagaag aagaaaaaag attcaattga 16260
gccggcgatg cagaccctta tataaatgtt gccttggaca gacggagcaa gcccgcccaa 16320
acctacgttc ggtataatat gttaagcttt ttaacacaaa ggtttggctt ggggtaacct 16380
gatgtggtgc aaaagaccgg gcgttggcga gccattgcgc gggcgaatgg ggccgtgact 16440
cgtctcaaat tcgagggcgt gcctcaattc gtgccccgt ggctttttcc cgccgtttcc 16500
gccccgtttg caccactgca gccgcttctt tggttcggac accttgctgc gagctaggtg 16560
ccttgtgcta cttaaaaagt ggcctcccaa caccaacatg acatgagtgc gtgggccaag 16620
acacgttggc ggggtcgcag tcggctcaat ggcccggaaa aaacgctgct ggagctggtt 16680
cggacgcagt ccgccgcggc gtatggatat ccgcaaggtt ccatagcgcc attgccctcc 16740
gtcggcgtct atcccgcaac ctctaaatag agcgggaata taacccaagc ttcttttttt 16800
tcctttaaca cgcacacccc caactatcat gttgctgctg ctgtttgact ctactctgtg 16860
gaggggtgct cccacccaac ccaacctaca ggtggatccg gcgctgtgat tggctgataa 16920
gtctcctatc cggactaatt ctgaccaatg ggacatgcgc gcaggaccca aatgccgcaa 16980
ttacgtaacc ccaacgaaat gcctacccct ctttggagcc cagcggcccc aaatcccccc 17040
aagcagcccg gttctaccgg cttccatctc caagcacaag cagcccgg 17088

<210> 70
<211> 1548
<212> DNA
<213> Eutreptiella cf_gymnastica CCMP1594

<220>
<221> CDS

<222> (1)..(1548)
<223> synthetic delta-4 desaturase (codon-optimized for Yarrowia lipolytica) ("E1594D4S")

<400> 70

```
atg gct cag tcc acc aag gct gcc gac act gct gcc acc gac aag tct        48
Met Ala Gln Ser Thr Lys Ala Ala Asp Thr Ala Ala Thr Asp Lys Ser
1               5                   10                  15

ctc gac aag aac cga ctc atc tcc cga gac gag ctg cgg tct cac aac        96
Leu Asp Lys Asn Arg Leu Ile Ser Arg Asp Glu Leu Arg Ser His Asn
            20                  25                  30

gtt ccc cag gat gcc tgg gct gcc gtc cac ggc aga gtc atc aac att       144
Val Pro Gln Asp Ala Trp Ala Ala Val His Gly Arg Val Ile Asn Ile
        35                  40                  45

acc gag ttc gcc cga cgg cat cct ggt ggc gac atc att ctg ctt gcc       192
Thr Glu Phe Ala Arg Arg His Pro Gly Gly Asp Ile Ile Leu Leu Ala
    50                  55                  60

gca gga aag gat gcc acc gtg ctc ttc gag act tac cat cct cga ggt       240
Ala Gly Lys Asp Ala Thr Val Leu Phe Glu Thr Tyr His Pro Arg Gly
65                  70                  75                  80

gtt ccc acc tcg atc ctc gac aag ctg cag gtc ggc aag atg aag gac       288
Val Pro Thr Ser Ile Leu Asp Lys Leu Gln Val Gly Lys Met Lys Asp
                85                  90                  95

gga gaa ctt ccc tcc tcg ttc tac tcg tgg gat tcc gac ttt tac aag       336
Gly Glu Leu Pro Ser Ser Phe Tyr Ser Trp Asp Ser Asp Phe Tyr Lys
            100                 105                 110

acc ctg cga gct cga gtg gtc gag cga ttg gac aag ctc aac ctg cct       384
Thr Leu Arg Ala Arg Val Val Glu Arg Leu Asp Lys Leu Asn Leu Pro
        115                 120                 125

cga aga ggt ggc tac gag att tgg gtc aag gca gta ttc ctc ctg gct       432
Arg Arg Gly Gly Tyr Glu Ile Trp Val Lys Ala Val Phe Leu Leu Ala
    130                 135                 140

gga ttc tgg ttc agc ctc tac aag atg tcc gtc aac gag acc tac tgg       480
Gly Phe Trp Phe Ser Leu Tyr Lys Met Ser Val Asn Glu Thr Tyr Trp
145                 150                 155                 160

gct gcc tcg ctg tgg tcc gtg tct atg gga gtc ttt gct gcc ttc atc       528
Ala Ala Ser Leu Trp Ser Val Ser Met Gly Val Phe Ala Ala Phe Ile
                165                 170                 175

ggc act tgc att caa cac gat gga aac cac ggt gcc ttc tcg acc agc       576
Gly Thr Cys Ile Gln His Asp Gly Asn His Gly Ala Phe Ser Thr Ser
            180                 185                 190

cct gct ctc aac aag gtt gca ggc tgg act ctg gac atg atc ggt gct       624
Pro Ala Leu Asn Lys Val Ala Gly Trp Thr Leu Asp Met Ile Gly Ala
        195                 200                 205

tct ggc ttt aca tgg gag att cag cat atg ctc gga cac cat ccc tac       672
Ser Gly Phe Thr Trp Glu Ile Gln His Met Leu Gly His His Pro Tyr
    210                 215                 220

acc aac gtc ctg gac gtg gac gaa gag aag cga aag gaa gct ggc gac       720
Thr Asn Val Leu Asp Val Asp Glu Glu Lys Arg Lys Glu Ala Gly Asp
225                 230                 235                 240

gat tgt cct atg gag gac aag gat cag gag tcc gac cca gat gtc ttc       768
Asp Cys Pro Met Glu Asp Lys Asp Gln Glu Ser Asp Pro Asp Val Phe
```

245 250 255

tct tcg ttt cct ctc atg cga atg cac ccc tac cac aag gcc gag tgg 816
Ser Ser Phe Pro Leu Met Arg Met His Pro Tyr His Lys Ala Glu Trp
260 265 270

tac cac cga tat cag cac ctg tac gca ccc gtt ctc ttt gct ttc atg 864
Tyr His Arg Tyr Gln His Leu Tyr Ala Pro Val Leu Phe Ala Phe Met
275 280 285

act ctt gcc aag gtg ttc caa cag gac atc gaa gtc gct acc act cag 912
Thr Leu Ala Lys Val Phe Gln Gln Asp Ile Glu Val Ala Thr Thr Gln
290 295 300

cga ctg tac cac atc gac gcc aag tgc cga tac aat tcc att ctc aat 960
Arg Leu Tyr His Ile Asp Ala Lys Cys Arg Tyr Asn Ser Ile Leu Asn
305 310 315 320

gtc ctt cgg ttt tgg tcg atg aag gtg ctc tcc atc ggc tac atg ctg 1008
Val Leu Arg Phe Trp Ser Met Lys Val Leu Ser Ile Gly Tyr Met Leu
325 330 335

gct gtt ccc tgc tac ttc cac gga atc ctt ggt ggc ctt gga ctg ttt 1056
Ala Val Pro Cys Tyr Phe His Gly Ile Leu Gly Gly Leu Gly Leu Phe
340 345 350

ctc atc ggc cac ttt gcc tgt gga gag ctt ctg gca acc atg ttc att 1104
Leu Ile Gly His Phe Ala Cys Gly Glu Leu Leu Ala Thr Met Phe Ile
355 360 365

gtc aat cac gtc atc gag ggt gtg tcc ttt ggc aaa aag gga gaa tct 1152
Val Asn His Val Ile Glu Gly Val Ser Phe Gly Lys Lys Gly Glu Ser
370 375 380

ctc ggt ctg tcc aag gac gtg gag ttc aag cct aca acc gtt tct gga 1200
Leu Gly Leu Ser Lys Asp Val Glu Phe Lys Pro Thr Thr Val Ser Gly
385 390 395 400

cga act cca atg gag cag acc cgt gcc gag gcc aaa aag gct gcc aat 1248
Arg Thr Pro Met Glu Gln Thr Arg Ala Glu Ala Lys Lys Ala Ala Asn
405 410 415

gga ggc aac gtc aag gat gtt ccc tac aac gac tgg gct gcc gtt cag 1296
Gly Gly Asn Val Lys Asp Val Pro Tyr Asn Asp Trp Ala Ala Val Gln
420 425 430

tgt caa acg agc gtc aac tgg tct cct gga tcg tgg ttc tgg aat cac 1344
Cys Gln Thr Ser Val Asn Trp Ser Pro Gly Ser Trp Phe Trp Asn His
435 440 445

ttc tcc ggt ggc ctc tcc cac cag atc gag cac cat ctg ttt ccc agc 1392
Phe Ser Gly Gly Leu Ser His Gln Ile Glu His His Leu Phe Pro Ser
450 455 460

att tgt cac acc aac tac gct cac atc cag gac gtt gtc cag aag act 1440
Ile Cys His Thr Asn Tyr Ala His Ile Gln Asp Val Val Gln Lys Thr
465 470 475 480

tgc gaa gag tac ggt gtt cct tac cag tcc gaa ccc tct ttg ttc tcc 1488
Cys Glu Glu Tyr Gly Val Pro Tyr Gln Ser Glu Pro Ser Leu Phe Ser
485 490 495

gcc tat ggc aag atg ctg tct cat ctc aag tac ctc gga aac gag aaa 1536
Ala Tyr Gly Lys Met Leu Ser His Leu Lys Tyr Leu Gly Asn Glu Lys
500 505 510

```
aag gtc gct taa                                                      1548
Lys Val Ala
    515
```

<210> 71
<211> 515
<212> PRT
<213> Eutreptiella cf_gymnastica CCMP1594

<400> 71

```
Met Ala Gln Ser Thr Lys Ala Ala Asp Thr Ala Ala Thr Asp Lys Ser
1               5                   10                  15

Leu Asp Lys Asn Arg Leu Ile Ser Arg Asp Glu Leu Arg Ser His Asn
            20                  25                  30

Val Pro Gln Asp Ala Trp Ala Ala Val His Gly Arg Val Ile Asn Ile
        35                  40                  45

Thr Glu Phe Ala Arg Arg His Pro Gly Gly Asp Ile Ile Leu Leu Ala
    50                  55                  60

Ala Gly Lys Asp Ala Thr Val Leu Phe Glu Thr Tyr His Pro Arg Gly
65                  70                  75                  80

Val Pro Thr Ser Ile Leu Asp Lys Leu Gln Val Gly Lys Met Lys Asp
                85                  90                  95

Gly Glu Leu Pro Ser Ser Phe Tyr Ser Trp Asp Ser Asp Phe Tyr Lys
            100                 105                 110

Thr Leu Arg Ala Arg Val Val Glu Arg Leu Asp Lys Leu Asn Leu Pro
        115                 120                 125

Arg Arg Gly Gly Tyr Glu Ile Trp Val Lys Ala Val Phe Leu Leu Ala
    130                 135                 140

Gly Phe Trp Phe Ser Leu Tyr Lys Met Ser Val Asn Glu Thr Tyr Trp
145                 150                 155                 160

Ala Ala Ser Leu Trp Ser Val Ser Met Gly Val Phe Ala Ala Phe Ile
                165                 170                 175

Gly Thr Cys Ile Gln His Asp Gly Asn His Gly Ala Phe Ser Thr Ser
            180                 185                 190

Pro Ala Leu Asn Lys Val Ala Gly Trp Thr Leu Asp Met Ile Gly Ala
        195                 200                 205
```

```
Ser Gly Phe Thr Trp Glu Ile Gln His Met Leu Gly His His Pro Tyr
    210                 215                 220

Thr Asn Val Leu Asp Val Asp Glu Glu Lys Arg Lys Glu Ala Gly Asp
225                 230                 235                 240

Asp Cys Pro Met Glu Asp Lys Asp Gln Glu Ser Asp Pro Asp Val Phe
                245                 250                 255

Ser Ser Phe Pro Leu Met Arg Met His Pro Tyr His Lys Ala Glu Trp
            260                 265                 270

Tyr His Arg Tyr Gln His Leu Tyr Ala Pro Val Leu Phe Ala Phe Met
        275                 280                 285

Thr Leu Ala Lys Val Phe Gln Gln Asp Ile Glu Val Ala Thr Thr Gln
    290                 295                 300

Arg Leu Tyr His Ile Asp Ala Lys Cys Arg Tyr Asn Ser Ile Leu Asn
305                 310                 315                 320

Val Leu Arg Phe Trp Ser Met Lys Val Leu Ser Ile Gly Tyr Met Leu
                325                 330                 335

Ala Val Pro Cys Tyr Phe His Gly Ile Leu Gly Gly Leu Gly Leu Phe
            340                 345                 350

Leu Ile Gly His Phe Ala Cys Gly Glu Leu Leu Ala Thr Met Phe Ile
        355                 360                 365

Val Asn His Val Ile Glu Gly Val Ser Phe Gly Lys Lys Gly Glu Ser
    370                 375                 380

Leu Gly Leu Ser Lys Asp Val Glu Phe Lys Pro Thr Thr Val Ser Gly
385                 390                 395                 400

Arg Thr Pro Met Glu Gln Thr Arg Ala Glu Ala Lys Lys Ala Ala Asn
                405                 410                 415

Gly Gly Asn Val Lys Asp Val Pro Tyr Asn Asp Trp Ala Ala Val Gln
            420                 425                 430

Cys Gln Thr Ser Val Asn Trp Ser Pro Gly Ser Trp Phe Trp Asn His
        435                 440                 445

Phe Ser Gly Gly Leu Ser His Gln Ile Glu His His Leu Phe Pro Ser
    450                 455                 460

Ile Cys His Thr Asn Tyr Ala His Ile Gln Asp Val Val Gln Lys Thr
```

```
465                 470                 475                 480

Cys Glu Glu Tyr Gly Val Pro Tyr Gln Ser Glu Pro Ser Leu Phe Ser
                485                 490                 495

Ala Tyr Gly Lys Met Leu Ser His Leu Lys Tyr Leu Gly Asn Glu Lys
            500                 505                 510

Lys Val Ala
        515
```

<210> 72
<211> 1542
<212> DNA
<213> Euglena gracilis

<220>
<221> CDS
<222> (1)..(1542)
<223> synthetic truncated delta-4 desaturase (codon-optimized for Yarrowia lipolytica)

<300>
<302> MULTIZYMES AND THEIR USE IN MAKING POLYUNSATURATED FATTY ACIDS
<310> U.S. Pat. Pub. No. 2008-0254191-A1
<311> 2008-04-03
<312> 2008-10-16
<313> (1)..(1542)

<300>
<302> MULTIZYMES AND THEIR USE IN MAKING POLYUNSATURATED FATTY ACIDS
<310> WO 2008/124048
<311> 2008-04-03
<312> 2008-10-16
<313> (1)..(1542)

<400> 72

```
atg gcc aaa ccg caa cct tgc gag aac ggt acc gtg gag aag cga gaa        48
Met Ala Lys Pro Gln Pro Cys Glu Asn Gly Thr Val Glu Lys Arg Glu
1               5                   10                  15

aac gac acc gcc aat gtt cga ccc gct aga cct gcc ggt ctt cca ccc        96
Asn Asp Thr Ala Asn Val Arg Pro Ala Arg Pro Ala Gly Leu Pro Pro
            20                  25                  30

gca act tac tat gac tct ctc gcc gtg tcg gga cag ggc aag gag cga       144
Ala Thr Tyr Tyr Asp Ser Leu Ala Val Ser Gly Gln Gly Lys Glu Arg
        35                  40                  45

ctg ttc acc aca gac gag gtc aga cga cac att ctt ccc acc gat gga       192
Leu Phe Thr Thr Asp Glu Val Arg Arg His Ile Leu Pro Thr Asp Gly
    50                  55                  60

tgg ctc acc tgt cac gaa ggt gtg tac gac gtc acc gat ttc ctg gcc       240
Trp Leu Thr Cys His Glu Gly Val Tyr Asp Val Thr Asp Phe Leu Ala
65                  70                  75                  80

aag cat cct gga ggt ggc gtc atc act ctc gga ctt ggt cga gac tgc       288
Lys His Pro Gly Gly Gly Val Ile Thr Leu Gly Leu Gly Arg Asp Cys
                85                  90                  95
```

```
acc att ctg gtc gag tcc tac cat ccc gct gga cga cca gac aag gtc        336
Thr Ile Leu Val Glu Ser Tyr His Pro Ala Gly Arg Pro Asp Lys Val
            100                 105                 110

atg gag aag tac cgt atc ggc aca ctc cag gat ccc aag act ttc tac        384
Met Glu Lys Tyr Arg Ile Gly Thr Leu Gln Asp Pro Lys Thr Phe Tyr
        115                 120                 125

gct tgg gga gaa tcg gac ttt tac cct gag ctc aaa cga cgg gct ctt        432
Ala Trp Gly Glu Ser Asp Phe Tyr Pro Glu Leu Lys Arg Arg Ala Leu
    130                 135                 140

gca cga ctc aag gag gct gga cag gca cgt cga ggt gga ctg gga gtc        480
Ala Arg Leu Lys Glu Ala Gly Gln Ala Arg Arg Gly Gly Leu Gly Val
145                 150                 155                 160

aag gct ctc ctg gtg ctt acc ctc ttc ttt gtc tcc tgg tac atg tgg        528
Lys Ala Leu Leu Val Leu Thr Leu Phe Phe Val Ser Trp Tyr Met Trp
                165                 170                 175

gtt gcc cac aag tct ttc ctg tgg gct gcc gtc tgg ggc ttt gcc ggt        576
Val Ala His Lys Ser Phe Leu Trp Ala Ala Val Trp Gly Phe Ala Gly
            180                 185                 190

tcg cat gtc gga ctg agc att caa cac gat ggc aac cac ggt gct ttc        624
Ser His Val Gly Leu Ser Ile Gln His Asp Gly Asn His Gly Ala Phe
        195                 200                 205

tct cga tcc acc ctg gtc aac aga ctc gca gga tgg ggc atg gac ttg        672
Ser Arg Ser Thr Leu Val Asn Arg Leu Ala Gly Trp Gly Met Asp Leu
    210                 215                 220

atc ggt gcc tcg tcc acc gtg tgg gag tac cag cat gtc att gga cac        720
Ile Gly Ala Ser Ser Thr Val Trp Glu Tyr Gln His Val Ile Gly His
225                 230                 235                 240

cat cag tac acg aat ctc gtt tcc gat acg ctg ttc agc ctt ccc gag        768
His Gln Tyr Thr Asn Leu Val Ser Asp Thr Leu Phe Ser Leu Pro Glu
                245                 250                 255

aac gac cca gat gtg ttt tcc agc tat cct ctc atg cga atg cat ccc        816
Asn Asp Pro Asp Val Phe Ser Ser Tyr Pro Leu Met Arg Met His Pro
            260                 265                 270

gac act gct tgg cag cct cat cac cga ttt caa cac ctg ttc gcc ttt        864
Asp Thr Ala Trp Gln Pro His His Arg Phe Gln His Leu Phe Ala Phe
        275                 280                 285

cct ctc ttc gct ctt atg acc att tcc aag gtg ctc acc agc gac ttt        912
Pro Leu Phe Ala Leu Met Thr Ile Ser Lys Val Leu Thr Ser Asp Phe
    290                 295                 300

gct gtt tgt ctc tcc atg aaa aag ggt tct atc gac tgt tcg tcc aga        960
Ala Val Cys Leu Ser Met Lys Lys Gly Ser Ile Asp Cys Ser Ser Arg
305                 310                 315                 320

ctg gtt cca ctc gaa ggc cag ctg ttg ttc tgg gga gcc aag ctg gcg       1008
Leu Val Pro Leu Glu Gly Gln Leu Leu Phe Trp Gly Ala Lys Leu Ala
                325                 330                 335

aac ttt ctc ttg cag att gtg ctg ccc tgc tac ctc cac gga act gct       1056
Asn Phe Leu Leu Gln Ile Val Leu Pro Cys Tyr Leu His Gly Thr Ala
            340                 345                 350
```

```
atg ggc ctt gct ctg ttc tct gtt gcc cac ctt gtg tct gga gag tac      1104
Met Gly Leu Ala Leu Phe Ser Val Ala His Leu Val Ser Gly Glu Tyr
        355                 360                 365

ctg gcc atc tgt ttc atc att aac cac atc tcc gag tct tgc gaa ttt      1152
Leu Ala Ile Cys Phe Ile Ile Asn His Ile Ser Glu Ser Cys Glu Phe
    370                 375                 380

atg aat acc tcc ttt caa act gct gcc cga cgg acc gag atg ctc cag      1200
Met Asn Thr Ser Phe Gln Thr Ala Ala Arg Arg Thr Glu Met Leu Gln
385                 390                 395                 400

gca gct cat cag gca gcc gag gcg aaa aag gtc aag ccc act cct cca      1248
Ala Ala His Gln Ala Ala Glu Ala Lys Lys Val Lys Pro Thr Pro Pro
                405                 410                 415

ccc aac gat tgg gct gtg aca caa gtc cag tgc tgt gtc aac tgg cga      1296
Pro Asn Asp Trp Ala Val Thr Gln Val Gln Cys Cys Val Asn Trp Arg
            420                 425                 430

tct gga ggc gtg ctg gcc aat cac ctc tcc ggt ggc ctc aac cat cag      1344
Ser Gly Gly Val Leu Ala Asn His Leu Ser Gly Gly Leu Asn His Gln
        435                 440                 445

atc gag cac cat ctg ttt ccc agc atc tct cac gcg aac tat ccc atc      1392
Ile Glu His His Leu Phe Pro Ser Ile Ser His Ala Asn Tyr Pro Ile
    450                 455                 460

att gct cga gtt gtc aag gaa gtg tgc gag gaa tac gga ttg ccc tac      1440
Ile Ala Arg Val Val Lys Glu Val Cys Glu Glu Tyr Gly Leu Pro Tyr
465                 470                 475                 480

aag aac tac gtt acg ttc tgg gat gcc gtg tgt ggc atg gtt cag cat      1488
Lys Asn Tyr Val Thr Phe Trp Asp Ala Val Cys Gly Met Val Gln His
                485                 490                 495

ctg cga ctc atg ggt gct cct ccc gtg cct acc aac ggc gac aaa aag      1536
Leu Arg Leu Met Gly Ala Pro Pro Val Pro Thr Asn Gly Asp Lys Lys
            500                 505                 510

tcc taa                                                              1542
Ser
```

<210> 73
<211> 513
<212> PRT
<213> Euglena gracilis

<400> 73

```
Met Ala Lys Pro Gln Pro Cys Glu Asn Gly Thr Val Glu Lys Arg Glu
1               5                   10                  15

Asn Asp Thr Ala Asn Val Arg Pro Ala Arg Pro Ala Gly Leu Pro Pro
            20                  25                  30

Ala Thr Tyr Tyr Asp Ser Leu Ala Val Ser Gly Gln Gly Lys Glu Arg
        35                  40                  45

Leu Phe Thr Thr Asp Glu Val Arg Arg His Ile Leu Pro Thr Asp Gly
```

```
    50                  55                  60

Trp Leu Thr Cys His Glu Gly Val Tyr Asp Val Thr Asp Phe Leu Ala
65                  70                  75                  80

Lys His Pro Gly Gly Gly Val Ile Thr Leu Gly Leu Gly Arg Asp Cys
                85                  90                  95

Thr Ile Leu Val Glu Ser Tyr His Pro Ala Gly Arg Pro Asp Lys Val
            100                 105                 110

Met Glu Lys Tyr Arg Ile Gly Thr Leu Gln Asp Pro Lys Thr Phe Tyr
        115                 120                 125

Ala Trp Gly Glu Ser Asp Phe Tyr Pro Glu Leu Lys Arg Arg Ala Leu
    130                 135                 140

Ala Arg Leu Lys Glu Ala Gly Gln Ala Arg Arg Gly Gly Leu Gly Val
145                 150                 155                 160

Lys Ala Leu Leu Val Leu Thr Leu Phe Phe Val Ser Trp Tyr Met Trp
                165                 170                 175

Val Ala His Lys Ser Phe Leu Trp Ala Ala Val Trp Gly Phe Ala Gly
            180                 185                 190

Ser His Val Gly Leu Ser Ile Gln His Asp Gly Asn His Gly Ala Phe
        195                 200                 205

Ser Arg Ser Thr Leu Val Asn Arg Leu Ala Gly Trp Gly Met Asp Leu
    210                 215                 220

Ile Gly Ala Ser Ser Thr Val Trp Glu Tyr Gln His Val Ile Gly His
225                 230                 235                 240

His Gln Tyr Thr Asn Leu Val Ser Asp Thr Leu Phe Ser Leu Pro Glu
                245                 250                 255

Asn Asp Pro Asp Val Phe Ser Ser Tyr Pro Leu Met Arg Met His Pro
            260                 265                 270

Asp Thr Ala Trp Gln Pro His His Arg Phe Gln His Leu Phe Ala Phe
        275                 280                 285

Pro Leu Phe Ala Leu Met Thr Ile Ser Lys Val Leu Thr Ser Asp Phe
    290                 295                 300

Ala Val Cys Leu Ser Met Lys Lys Gly Ser Ile Asp Cys Ser Ser Arg
305                 310                 315                 320
```

```
Leu Val Pro Leu Glu Gly Gln Leu Leu Phe Trp Gly Ala Lys Leu Ala
                325                 330                 335

Asn Phe Leu Leu Gln Ile Val Leu Pro Cys Tyr Leu His Gly Thr Ala
            340                 345                 350

Met Gly Leu Ala Leu Phe Ser Val Ala His Leu Val Ser Gly Glu Tyr
        355                 360                 365

Leu Ala Ile Cys Phe Ile Ile Asn His Ile Ser Glu Ser Cys Glu Phe
    370                 375                 380

Met Asn Thr Ser Phe Gln Thr Ala Ala Arg Arg Thr Glu Met Leu Gln
385                 390                 395                 400

Ala Ala His Gln Ala Ala Glu Ala Lys Lys Val Lys Pro Thr Pro Pro
                405                 410                 415

Pro Asn Asp Trp Ala Val Thr Gln Val Gln Cys Cys Val Asn Trp Arg
            420                 425                 430

Ser Gly Gly Val Leu Ala Asn His Leu Ser Gly Gly Leu Asn His Gln
        435                 440                 445

Ile Glu His His Leu Phe Pro Ser Ile Ser His Ala Asn Tyr Pro Ile
    450                 455                 460

Ile Ala Arg Val Val Lys Glu Val Cys Glu Glu Tyr Gly Leu Pro Tyr
465                 470                 475                 480

Lys Asn Tyr Val Thr Phe Trp Asp Ala Val Cys Gly Met Val Gln His
                485                 490                 495

Leu Arg Leu Met Gly Ala Pro Pro Val Pro Thr Asn Gly Asp Lys Lys
            500                 505                 510

Ser
```

<210> 74
<211> 15617
<212> DNA
<213> Artificial Sequence

<220>
<223> Plasmid pZKLY-G20444

<400> 74

aattctctct cttgagcttt tccataacaa gttcttctgc ctccaggaag tccatgggtg 60

gtttgatcat ggttttggtg tagtggtagt gcagtggtgg tattgtgact ggggatgtag 120
ttgagaataa gtcatacaca agtcagcttt cttcgagcct catataagta taagtagttc 180
aacgtattag cactgtaccc agcatctccg tatcgagaaa cacaacaaca tgccccattg 240
gacagatcat gcggatacac aggttgtgca gtatcataca tactcgatca gacaggtcgt 300
ctgaccatca tacaagctga acaagcgctc catacttgca cgctctctat atacacagtt 360
aaattacata tccatagtct aacctctaac agttaatctt ctggtaagcc tcccagccag 420
ccttctggta tcgcttggcc tcctcaatag gatctcggtt ctggccgtac agacctcggc 480
cgacaattat gatatccgtt ccggtagaca tgacatcctc aacagttcgg tactgctgtc 540
cgagagcgtc tcccttgtcg tcaagaccca ccccgggggt cagaataagc cagtcctcag 600
agtcgccctt aggtcggttc tgggcaatga agccaaccac aaactcgggg tcggatcggg 660
caagctcaat ggtctgcttg gagtactcgc cagtggccag agagcccttg caagacagct 720
cggccagcat gagcagacct ctggccagct tctcgttggg agaggggact aggaactcct 780
tgtactggga gttctcgtag tcagagacgt cctccttctt ctgttcagag acagtttcct 840
cggcaccagc tcgcaggcca gcaatgattc cggttccggg tacaccgtgg gcgttggtga 900
tatcggacca ctcggcgatt cggtgacacc ggtactggtg cttgacagtg ttgccaatat 960
ctgcgaactt tctgtcctcg aacaggaaga aaccgtgctt aagagcaagt tccttgaggg 1020
ggagcacagt gccggcgtag gtgaagtcgt caatgatgtc gatatgggtt ttgatcatgc 1080
acacataagg tccgacctta tcggcaagct caatgagctc cttggtggtg gtaacatcca 1140
gagaagcaca caggttggtt ttcttggctg ccacgagctt gagcactcga gcggcaaagg 1200
cggacttgtg gacgttagct cgagcttcgt aggagggcat tttggtggtg aagaggagac 1260
tgaaataaat ttagtctgca gaacttttta tcggaacctt atctggggca gtgaagtata 1320
tgttatggta atagttacga gttagttgaa cttatagata gactggacta tacggctatc 1380
ggtccaaatt agaaagaacg tcaatggctc tctgggcgtc gcctttgccg acaaaaatgt 1440
gatcatgatg aaagccagca atgacgttgc agctgatatt gttgtcggcc aaccgcgccg 1500
aaaacgcagc tgtcagaccc acagcctcca acgaagaatg tatcgtcaaa gtgatccaag 1560
cacactcata gttggagtcg tactccaaag gcggcaatga cgagtcagac agatactcgt 1620
cgaccttttc cttgggaacc accaccgtca gcccttctga ctcacgtatt gtagccaccg 1680
acacaggcaa cagtccgtgg atagcagaat atgtcttgtc ggtccatttc tcaccaactt 1740
taggcgtcaa gtgaatgttg cagaagaagt atgtgccttc attgagaatc ggtgttgctg 1800
atttcaataa agtcttgaga tcagtttggc cagtcatgtt gtgggggta attggattga 1860
gttatcgcct acagtctgta caggtatact cgctgcccac tttatacttt ttgattccgc 1920
tgcacttgaa gcaatgtcgt ttaccaaaag tgagaatgct ccacagaaca caccccaggg 1980
tatggttgag caaaaaataa acactccgat acggggaatc gaaccccggt ctccacggtt 2040

ctcaagaagt attcttgatg agagcgtatc gatggttaat gctgctgtgt gctgtgtgtg 2100
tgtgttgttt ggcgctcatt gttgcgttat gcagcgtaca ccacaatatt ggaagcttat 2160
tagcctttct attttttcgt ttgcaaggct taacaacatt gctgtggaga gggatgggga 2220
tatggaggcc gctggaggga gtcggagagg cgttttggag cggcttggcc tggcgcccag 2280
ctcgcgaaac gcacctagga ccctttggca cgccgaaatg tgccactttt cagtctagta 2340
acgccttacc tacgtcattc catgcgtgca tgtttgcgcc ttttttccct tgcccttgat 2400
cgccacacag tacagtgcac tgtacagtgg aggttttggg ggggtcttag atgggagcta 2460
aaagcggcct agcggtacac tagtgggatt gtatggagtg gcatggagcc taggtggagc 2520
ctgacaggac gcacgaccgg ctagcccgtg acagacgatg ggtggctcct gttgtccacc 2580
gcgtacaaat gtttgggcca aagtcttgtc agccttgctt gcgaacctaa ttcccaattt 2640
tgtcacttcg cacccccatt gatcgagccc taacccctgc ccatcaggca atccaattaa 2700
gctcgcattg tctgccttgt ttagtttggc tcctgcccgt ttcggcgtcc acttgcacaa 2760
acacaaacaa gcattatata taaggctcgt ctctccctcc caaccacact cactttttg 2820
cccgtcttcc cttgctaaca caaaagtcaa gaacacaaac aaccacccca accccttac 2880
acacaagaca tatctacagc aatggccatg gccaaggtca aacccggtgg acctggcaag 2940
ccctcggaga tcgcttctct tccacctccc attcgacctg ttggcaaccc acctgcagcc 3000
tattacgacg ctctggccac ctccggtact ggacaggacc gaaagtttac catgcgagag 3060
gtcgctcgac acattgttcc caccgatggc tggttggcct gtcacgacgg tgtgtacgac 3120
atcaccgagt tcattggcaa gcatcccggt ggagatgtta tctctctcgg tctcggacga 3180
gactccacta ttctggtcga atcgtaccat cctgcaggac gacccgacaa ggttatggag 3240
aagtaccgaa tcggtacact tcaggatcac agaaccttct acgactggca ggcctccgct 3300
ttctacgccg agctcaagca gcgagtggtt cagactctca aggaggctgg acaacctcga 3360
cgtggtggcc tgtctgtcaa ggcagccctt gttatggctg cctttgctgc ctcgttctac 3420
ctcatggtga cacagggatc cttcttttgg gctgccgtct ggggtctggc aggctctcac 3480
attggactca gcatccagca cgacggcaat catggtgctt tctccaagtc tggacgactc 3540
aaccgtcttg ctggctgggg tatggacgtt atcggagcct cctcgactgc ctgggagtac 3600
caacacgtca ttggtcatca ccagtacacc aacctggtgt ccgatcccga gtttgctctt 3660
cccgagaacg atccagacgt tttcggaacc tatcccctca tgcggatgca tccggacact 3720
ccttggaaac cccaccatca gctgcaacac gtgtacgcct ttccgttgtt cgctctcatg 3780
accatcagca aggtcattat ctccgatttc acgttttgtc ttgccaagcg acgtggtccc 3840
atcgacttct ctgccagact cgttcccctc gagggtcaga tgctgttctg ggtgcaaag 3900
atcatgggct ttctcatgca gattgtgctt ccctgctacc tgcatggcat cgctcacgga 3960

ttggccctct tcattacagc tcatctggtt tctggcgagt accttgccgt ctgtttcatt 4020
atcaaccaca tttccgagtc gtgcgactac ctcaatccct cttccgttat cgctgcccga 4080
cggaccgaaa tgctcaagca ggccgagcag gaagccaagg cgaaacagaa gcaccccact 4140
ccacctccca acgactgggc tgcctcccaa gttctgtgtt gcgtcaactg gcgatctggt 4200
ggctactttt caaaccacct ttctggtgga ctcaaccacc agatcgagca tcacctgttt 4260
cccagcattt ctcacgccaa ctatcccacc attgctcctg ttgtcaaggg cgtgtgcgag 4320
gaatacggtc ttccctacaa gaactactct cagttttccg atgctctgta cggaatggtc 4380
gagcacttgc gagctatggg caccaaacct gcagacaacg acaagcttgc tcccactgca 4440
ggttccctgg aggatgtttg tcctgtgctc tctgctgccg ttgctgccca acccgacggc 4500
tccaccgacg gatctgctgc cggttgtcct gctgtcgcca ctctggctta agcggccgca 4560
ttgatgattg gaaacacaca catgggttat atctaggtga gagttagttg gacagttata 4620
tattaaatca gctatgccaa cggtaacttc attcatgtca acgaggaacc agtgactgca 4680
agtaatatag aatttgacca ccttgccatt ctcttgcact cctttactat atctcattta 4740
tttcttatat acaaatcact tcttcttccc agcatcgagc tcggaaacct catgagcaat 4800
aacatcgtgg atctcgtcaa tagagggctt tttggactcc ttgctgttgg ccaccttgtc 4860
cttgctgttt aaacagagtg tgaaagactc actatggtcc gggcttatct cgaccaatag 4920
ccaaagtctg gagtttctga gagaaaaagg caagatacgt atgtaacaaa gcgacgcatg 4980
gtacaataat accggaggca tgtatcatag agagttagtg gttcgatgat ggcactggtg 5040
cctggtatga ctttatacgg ctgactacat atttgtcctc agacatacaa ttacagtcaa 5100
gcacttaccc ttggacatct gtaggtaccc cccggccaag acgatctcag cgtgtcgtat 5160
gtcggattgg cgtagctccc tcgctcgtca attggctccc atctactttc ttctgcttgg 5220
ctacacccag catgtctgct atggctcgtt ttcgtgcctt atctatcctc ccagtattac 5280
caactctaaa tgacatgatg tgattgggtc tacactttca tatcagagat aaggagtagc 5340
acagttgcat aaaaagccca actctaatca gcttcttcct ttcttgtaat tagtacaaag 5400
gtgattagcg aaatctggaa gcttagttgg ccctaaaaaa atcaaaaaaa gcaaaaaacg 5460
aaaaacgaaa aaccacagtt ttgagaacag ggaggtaacg aaggatcgta tatatatata 5520
tatatatata tacccacgga tcccgagacc ggcctttgat tcttccctac aaccaaccat 5580
tctcaccacc ctaattcaca accatggctg actctcccgt catcaacctc tccaccatgt 5640
ggaagcctct gtcgctcatg gccttggatc ttgctgttct gggacacgtc tggaagcagg 5700
cacaacagga gggctccatc tcggcttacg ccgactctgt gtggactccc ctcatcatgt 5760
ccggtctgta cctctccatg atcttcgtgg gatgtcgatg gatgaagaac cgagagccct 5820
tcgaaatcaa gacctacatg tttgcctaca acctgtacca gaccctcatg aacctttgca 5880
ttgtgctggg cttcctctac caggtccacg ctaccggtat gcgattctgg ggatctggcg 5940

tggaccgatc gcccaagggt ctgggaattg gctttttcat ctatgcccat taccacaaca 6000

agtacgtcga gtacttcgac acactcttca tggtgctgcg gaaaaagaac aaccagattt 6060

cctttcttca cgtctaccat cacgctctgc tcacctgggc ttggtttgcc gtggtctact 6120

tcgctcctgg aggtgacggc tggtttggag cctgctacaa ttcctccatt catgtcctga 6180

tgtactctta ctatctgctt gccaccttcg gcatctcctg tccctggaaa aagatcctca 6240

cccagctgca aatggttcag ttctgctttt gcttcaccca ctcgatctac gtgtggattt 6300

gcggttccga aatctaccct cgacccttga ctgctctcca gtccttcgtg atggtcaaca 6360

tgctggttct ctttggcaac ttctacgtca agcagtattc tcagaagaat ggaaagcccg 6420

agaacggtgc cactcctgag aacggtgcca agcctcagcc ctgcgagaac ggtaccgtgg 6480

agaagcgaga aaacgacacc gccaatgttc gacccgctag acctgccggt cttccacccg 6540

caacttacta tgactctctc gccgtgtcgg gacagggcaa ggagcgactg ttcaccacag 6600

acgaggtcag acgacacatt cttcccaccg atggatggct cacctgtcac gaaggtgtgt 6660

acgacgtcac cgatttcctg gccaagcatc ctggaggtgg cgtcatcact ctcggacttg 6720

gtcgagactg caccattctg gtcgagtcct accatcccgc tggacgacca gacaaggtca 6780

tggagaagta ccgtatcggc acactccagg atcccaagac tttctacgct tggggagaat 6840

cggactttta ccctgagctc aaacgacggg ctcttgcacg actcaaggag gctggacagg 6900

cacgtcgagg tggactggga gtcaaggctc tcctggtgct taccctcttc tttgtctcct 6960

ggtacatgtg ggttgcccac aagtctttcc tgtgggctgc cgtctggggc tttgccggtt 7020

cgcatgtcgg actgagcatt caacacgatg gcaaccacgg tgctttctct cgatccaccc 7080

tggtcaacag actcgcagga tggggcatgg acttgatcgg tgcctcgtcc accgtgtggg 7140

agtaccagca tgtcattgga caccatcagt acacgaatct cgtttccgat acgctgttca 7200

gccttcccga gaacgaccca gatgtgtttt ccagctatcc tctcatgcga atgcatcccg 7260

acactgcttg gcagcctcat caccgatttc aacacctgtt cgcctttcct ctcttcgctc 7320

ttatgaccat ttccaaggtg ctcaccagcg actttgctgt ttgtctctcc atgaaaaagg 7380

gttctatcga ctgttcgtcc agactggttc cactcgaagg ccagctgttg ttctggggag 7440

ccaagctggc gaactttctc ttgcagattg tgctgccctg ctacctccac ggaactgcta 7500

tgggccttgc tctgttctct gttgcccacc ttgtgtctgg agagtacctg gccatctgtt 7560

tcatcattaa ccacatctcc gagtcttgcg aatttatgaa tacctccttt caaactgctg 7620

cccgacggac cgagatgctc caggcagctc atcaggcagc cgaggcgaaa aaggtcaagc 7680

ccactcctcc acccaacgat tgggctgtga cacaagtcca gtgctgtgtc aactggcgat 7740

ctggaggcgt gctggccaat cacctctccg gtggcctcaa ccatcagatc gagcaccatc 7800

tgtttcccag catctctcac gcgaactatc ccatcattgc tcgagttgtc aaggaagtgt 7860

gcgaggaata cggattgccc tacaagaact acgttacgtt ctgggatgcc gtgtgtggca 7920
tggttcagca tctgcgactc atgggtgctc ctcccgtgcc taccaacggc gacaaaaagt 7980
cctaagcggc cgcatgagaa gataaatata taaatacatt gagatattaa atgcgctaga 8040
ttagagagcc tcatactgct cggagagaag ccaagacgag tactcaaagg ggattacacc 8100
atccatatcc acagacacaa gctggggaaa ggttctatat acactttccg gaataccgta 8160
gtttccgatg ttatcaatgg gggcagccag gatttcaggc acttcggtgt ctcggggtga 8220
aatggcgttc ttggcctcca tcaagtcgta ccatgtcttc atttgcctgt caaagtaaaa 8280
cagaagcaga tgaagaatga acttgaagtg aaggaattta aatgtaacga aactgaaatt 8340
tgaccagata ttgtgtccgc ggtggagctc cagcttttgt tccctttagt gagggttaat 8400
ttcgagcttg gcgtaatcat ggtcatagct gtttcctgtg tgaaattgtt atccgctcac 8460
aagcttccac acaacgtacg ttgattgagg tggagccaga tgggctattg tttcatatat 8520
agactggcag ccacctcttt ggcccagcat gtttgtatac ctggaaggga aaactaaaga 8580
agctggctag tttagtttga ttattatagt agatgtccta atcactagag attagaatgt 8640
cttggcgatg attagtcgtc gtcccctgta tcatgtctag accaactgtg tcatgaagtt 8700
ggtgctggtg ttttacctgt gtactacaag taggtgtcct agatctagtg tacagagccg 8760
tttagaccca tgtggacttc accattaacg atggaaaatg ttcattatat gacagtatat 8820
tacaatggac ttgctccatt tcttccttgc atcacatgtt ctccacctcc atagttgatc 8880
aacacatcat agtagctaag gctgctgctc tcccactaca gtccaccaca agttaagtag 8940
caccgtcagt acagctaaaa gtacacgtct agtacgtttc ataactagtc aagtagcccc 9000
tattacagat atcagcacta tcacgcacga gtttttctct gtgctatcta atcaacttgc 9060
caagtattcg gagaagatac actttcttgg catcaggtat acgagggagc ctatcagatg 9120
aaaaagggta tattggatcc attcatatcc acctacacgt tgtcataatc tcctcattca 9180
cgtgattcat ttcgtgacac tagtttctca ctttcccccc cgcacctata gtcaacttgg 9240
cggacacgct acttgtagct gacgttgatt tatagaccca atcaaagcgg gttatcggtc 9300
aggtagcact tatcattcat cgttcatact acgatgagca atctcgggca tgtccggaaa 9360
agtgtcgggc gcgccagctg cattaatgaa tcggccaacg cgcggggaga ggcggtttgc 9420
gtattgggcg ctcttccgct tcctcgctca ctgactcgct gcgctcggtc gttcggctgc 9480
ggcgagcggt atcagctcac tcaaaggcgg taatacggtt atccacagaa tcaggggata 9540
acgcaggaaa gaacatgtga gcaaaaggcc agcaaaaggc caggaaccgt aaaaaggccg 9600
cgttgctggc gtttttccat aggctccgcc cccctgacga gcatcacaaa aatcgacgct 9660
caagtcagag gtggcgaaac ccgacaggac tataaagata ccaggcgttt ccccctggaa 9720
gctccctcgt gcgctctcct gttccgaccc tgccgcttac cggatacctg tccgcctttc 9780
tcccttcggg aagcgtggcg ctttctcata gctcacgctg taggtatctc agttcggtgt 9840

aggtcgttcg ctccaagctg ggctgtgtgc acgaaccccc cgttcagccc gaccgctgcg 9900
ccttatccgg taactatcgt cttgagtcca acccggtaag acacgactta tcgccactgg 9960
cagcagccac tggtaacagg attagcagag cgaggtatgt aggcggtgct acagagttct 10020
tgaagtggtg gcctaactac ggctacacta gaagaacagt atttggtatc tgcgctctgc 10080
tgaagccagt taccttcgga aaaagagttg gtagctcttg atccggcaaa caaaccaccg 10140
ctggtagcgg tggttttttt gtttgcaagc agcagattac gcgcagaaaa aaaggatctc 10200
aagaagatcc tttgatcttt tctacggggt ctgacgctca gtggaacgaa aactcacgtt 10260
aagggatttt ggtcatgaga ttatcaaaaa ggatcttcac ctagatcctt ttaaattaaa 10320
aatgaagttt taaatcaatc taaagtatat atgagtaaac ttggtctgac agttaccaat 10380
gcttaatcag tgaggcacct atctcagcga tctgtctatt tcgttcatcc atagttgcct 10440
gactccccgt cgtgtagata actacgatac gggagggctt accatctggc cccagtgctg 10500
caatgatacc gcgagaccca cgctcaccgg ctccagattt atcagcaata aaccagccag 10560
ccggaagggc cgagcgcaga agtggtcctg caactttatc cgcctccatc cagtctatta 10620
attgttgccg ggaagctaga gtaagtagtt cgccagttaa tagtttgcgc aacgttgttg 10680
ccattgctac aggcatcgtg gtgtcacgct cgtcgtttgg tatggcttca ttcagctccg 10740
gttcccaacg atcaaggcga gttacatgat cccccatgtt gtgcaaaaaa gcggttagct 10800
ccttcggtcc tccgatcgtt gtcagaagta agttggccgc agtgttatca ctcatggtta 10860
tggcagcact gcataattct cttactgtca tgccatccgt aagatgcttt tctgtgactg 10920
gtgagtactc aaccaagtca ttctgagaat agtgtatgcg gcgaccgagt tgctcttgcc 10980
cggcgtcaat acgggataat accgcgccac atagcagaac tttaaaagtg ctcatcattg 11040
gaaaacgttc ttcggggcga aaactctcaa ggatcttacc gctgttgaga tccagttcga 11100
tgtaacccac tcgtgcaccc aactgatctt cagcatcttt tactttcacc agcgtttctg 11160
ggtgagcaaa aacaggaagg caaaatgccg caaaaaaggg aataagggcg acacggaaat 11220
gttgaatact catactcttc ctttttcaat attattgaag catttatcag ggttattgtc 11280
tcatgagcgg atacatattt gaatgtattt agaaaaataa acaaataggg gttccgcgca 11340
catttccccg aaaagtgcca cctgatgcgg tgtgaaatac cgcacagatg cgtaaggaga 11400
aaataccgca tcaggaaatt gtaagcgtta atattttgtt aaaattcgcg ttaaattttt 11460
gttaaatcag ctcatttttt aaccaatagg ccgaaatcgg caaaatccct tataaatcaa 11520
aagaatagac cgagataggg ttgagtgttg ttccagtttg gaacaagagt ccactattaa 11580
agaacgtgga ctccaacgtc aaagggcgaa aaaccgtcta tcagggcgat ggcccactac 11640
gtgaaccatc accctaatca agttttttgg ggtcgaggtg ccgtaaagca ctaaatcgga 11700
accctaaagg gagcccccga tttagagctt gacggggaaa gccggcgaac gtggcgagaa 11760

aggaagggaa gaaagcgaaa ggagcgggcg ctagggcgct ggcaagtgta gcggtcacgc 11820
tgcgcgtaac caccacaccc gccgcgctta atgcgccgct acagggcgcg tccattcgcc 11880
attcaggctg cgcaactgtt gggaagggcg atcggtgcgg gcctcttcgc tattacgcca 11940
gctggcgaaa gggggatgtg ctgcaaggcg attaagttgg gtaacgccag ggttttccca 12000
gtcacgacgt tgtaaaacga cggccagtga attgtaatac gactcactat agggcgaatt 12060
gggcccgacg tcgcatgcat tccgacagca gcgactgggc accatgatca agcgaaacac 12120
cttcccccag ctgccctggc aaaccatcaa gaaccctact ttcatcaagt gcaagaacgg 12180
ttctactctt ctcacctccg gtgtctacgg ctggtgccga aagcctaact acaccgctga 12240
tttcatcatg tgcctcacct gggctctcat gtgcggtgtt gcttctcccc tgccttactt 12300
ctacccggtc ttcttcttcc tggtgctcat ccaccgagct taccgagact ttgagcgact 12360
ggagcgaaag tacggtgagg actaccagga gttcaagcga caggtccctt ggatcttcat 12420
ccccttatgtt ttctaaacga taagcttagt gagcgaatgg tgaggttact taattgagtg 12480
gccagcctat gggattgtat aacagacagt caatatatta ctgaaaagac tgaacagcca 12540
gacggagtga ggttgtgagt gaatcgtaga gggcggctat tacagcaagt ctactctaca 12600
gtgtactaac acagcagaga acaaatacag gtgtgcattc ggctatctga gaattagttg 12660
gagagctcga gaccctcggc gataaactgc tcctcggttt tgtgtccata cttgtacgga 12720
ccattgtaat ggggcaagtc gttgagttct cgtcgtccga cgttcagagc acagaaacca 12780
atgtaatcaa tgtagcagag atggttctgc aaaagattga tttgtgcgag caggttaatt 12840
aaaaggcgtt gaaacagaat gagccagttt aaacagcaag gacaaggtgg ccaacagcaa 12900
ggagtccaaa aagccctcta ttgacgagat ccacgatgtt attgctcatg aggtttccga 12960
gctcgatgct gggaagaaga agtgatttgt atataagaaa taaatgagat atagtaaagg 13020
agtgcaagag aatggcaagg tggtcaaatt ctatattact tgcagtcact ggttcctcgt 13080
tgacatgaat gaagttaccg ttggcatagc tgatttaata tataactgtc caactaactc 13140
tcacctagat ataacccatg tgtgtgtttc caatcatcaa tgcggccgct taagcgacct 13200
ttttctcgtt tccgaggtac ttgagatgag acagcatctt gccataggcg gagaacaaag 13260
agggttcgga ctggtaagga acaccgtact cttcgcaagt cttctggaca acgtcctgga 13320
tgtgagcgta gttggtgtga caaatgctgg gaaacagatg gtgctcgatc tggtgggaga 13380
ggccaccgga gaagtgattc cagaaccacg atccaggaga ccagttgacg ctcgtttgac 13440
actgaacggc agcccagtcg ttgtagggaa catccttgac gttgcctcca ttggcagcct 13500
ttttggcctc ggcacgggtc tgctccattg gagttcgtcc agaaacggtt gtaggcttga 13560
actccacgtc cttggacaga ccgagagatt ctcccttttt gccaaaggac acaccctcga 13620
tgacgtgatt gacaatgaac atggttgcca gaagctctcc acaggcaaag tggccgatga 13680
gaaacagtcc aaggccacca aggattccgt ggaagtagca gggaacagcc agcatgtagc 13740

cgatggagag caccttcatc gaccaaaacc gaaggacatt gagaatggaa ttgtatcggc 13800

acttggcgtc gatgtggtac agtcgctgag tggtagcgac ttcgatgtcc tgttggaaca 13860

ccttggcaag agtcatgaaa gcaaagagaa cgggtgcgta caggtgctga tatcggtggt 13920

accactcggc cttgtggtag gggtgcattc gcatgagagg aaacgaagag aagacatctg 13980

ggtcggactc ctgatccttg tcctccatag gacaatcgtc gccagcttcc tttcgcttct 14040

cttcgtccac gtccaggacg ttggtgtagg gatggtgtcc gagcatatgc tgaatctccc 14100

atgtaaagcc agaagcaccg atcatgtcca gagtccagcc tgcaaccttg ttgagagcag 14160

ggctggtcga gaaggcaccg tggtttccat cgtgttgaat gcaagtgccg atgaaggcag 14220

caaagactcc catagacacg gaccacagcg aggcagccca gtaggtctcg ttgacggaca 14280

tcttgtagag gctgaaccag aatccagcca ggaggaatac tgccttgacc caaatctcgt 14340

agccacctct tcgaggcagg ttgagcttgt ccaatcgctc gaccactcga gctcgcaggg 14400

tcttgtaaaa gtcggaatcc cacgagtaga acgaggaggg aagttctccg tccttcatct 14460

tgccgacctg cagcttgtcg aggatcgagg tgggaacacc tcgaggatgg taagtctcga 14520

agagcacggt ggcatccttt cctgcggcaa gcagaatgat gtcgccacca ggatgccgtc 14580

gggcgaactc ggtaatgttg atgactctgc cgtggacggc agcccaggca tcctggggaa 14640

cgttgtgaga ccgcagctcg tctcgggaga tgagtcggtt cttgtcgaga gacttgtcgg 14700

tggcagcagt gtcggcagcc ttggtggact gagccatggt accagagctg ggttagtttg 14760

tgtagagagt gtgtgttgct agcgactttc ggattgtgtc attacacaaa acgcgtcgtc 14820

tcgacactga tcttgtcgtg gatactcacg gctcggaact ctgtgatgtg tagtttagat 14880

ttcgaatctg tggggaaaga aaggaaaaaa gagactggca accgattggg agagccactg 14940

tttatatata ccctagacaa gcccccccgct tgtaagatgt tggtcaatgt aaaccagtat 15000

taaggttggc aagtgcagga gaagcaaggt gtgggtaccg agcaatggaa atgtgcggaa 15060

ggcaaaaaaa tgaggccacg gcctattgtc ggggctatat ccagggggcg attgaagtac 15120

actaacatga catgtgtcca cagaccctca atctggcctg atgagccaaa tccatacgcg 15180

ctttcgcagc tctaaaggct ataacaagtc acaccaccct gctcgacctc agcgccctca 15240

ctttttgtta agacaaactg tacacgctgt tccagcgttt tctgcctgca cctggtggga 15300

catttggtgc aacctaaagt gctcggaacc tctgtggtgt ccagatcagc gcagcagttc 15360

cgaggtagtt ttgaggccct tagatgatgc aatggtgtca gtcgctggat cacgagtctt 15420

aatggcagta ttcgttctta tttgtgccat tgagccccgt tatcctcgta tcttctaccc 15480

cccatcccat ccctttgttg gtgcaaccct acccatttat tgttgggtgc agcccaaccg 15540

acgtggagag cttggcttgg ccatataaaa aggccccccc ctagtggcaa tggcagaaag 15600

tcagctgtga gttgttg 15617

<210> 75
<211> 2382
<212> DNA
<213> Euglena gracilis

<220>
<221> CDS
<222> (1)..(2382)
<223> synthetic DHA synthase (codon-optimized for Yarrowia lipolytica)

<300>
<302> MULTIZYMES AND THEIR USE IN MAKING POLYUNSATURATED FATTY ACIDS
<310> U.S. Pat. Pub. No. 2008-0254191-A1
<311> 2008-04-03
<312> 2008-10-16
<313> (1)..(2382)

<300>
<302> MULTIZYMES AND THEIR USE IN MAKING POLYUNSATURATED FATTY ACIDS
<310> WO 2008/124048
<311> 2008-04-03
<312> 2008-10-16
<313> (1)..(2382)

<400> 75

```
atg gct gac tct ccc gtc atc aac ctc tcc acc atg tgg aag cct ctg        48
Met Ala Asp Ser Pro Val Ile Asn Leu Ser Thr Met Trp Lys Pro Leu
1               5                   10                  15

tcg ctc atg gcc ttg gat ctt gct gtt ctg gga cac gtc tgg aag cag        96
Ser Leu Met Ala Leu Asp Leu Ala Val Leu Gly His Val Trp Lys Gln
            20                  25                  30

gca caa cag gag ggc tcc atc tcg gct tac gcc gac tct gtg tgg act       144
Ala Gln Gln Glu Gly Ser Ile Ser Ala Tyr Ala Asp Ser Val Trp Thr
        35                  40                  45

ccc ctc atc atg tcc ggt ctg tac ctc tcc atg atc ttc gtg gga tgt       192
Pro Leu Ile Met Ser Gly Leu Tyr Leu Ser Met Ile Phe Val Gly Cys
    50                  55                  60

cga tgg atg aag aac cga gag ccc ttc gaa atc aag acc tac atg ttt       240
Arg Trp Met Lys Asn Arg Glu Pro Phe Glu Ile Lys Thr Tyr Met Phe
65                  70                  75                  80

gcc tac aac ctg tac cag acc ctc atg aac ctt tgc att gtg ctg ggc       288
Ala Tyr Asn Leu Tyr Gln Thr Leu Met Asn Leu Cys Ile Val Leu Gly
                85                  90                  95

ttc ctc tac cag gtc cac gct acc ggt atg cga ttc tgg gga tct ggc       336
Phe Leu Tyr Gln Val His Ala Thr Gly Met Arg Phe Trp Gly Ser Gly
            100                 105                 110

gtg gac cga tcg ccc aag ggt ctg gga att ggc ttt ttc atc tat gcc       384
Val Asp Arg Ser Pro Lys Gly Leu Gly Ile Gly Phe Phe Ile Tyr Ala
        115                 120                 125

cat tac cac aac aag tac gtc gag tac ttc gac aca ctc ttc atg gtg       432
His Tyr His Asn Lys Tyr Val Glu Tyr Phe Asp Thr Leu Phe Met Val
    130                 135                 140

ctg cgg aaa aag aac aac cag att tcc ttt ctt cac gtc tac cat cac       480
Leu Arg Lys Lys Asn Asn Gln Ile Ser Phe Leu His Val Tyr His His
```

```
145                 150                 155                 160

gct ctg ctc acc tgg gct tgg ttt gcc gtg gtc tac ttc gct cct gga          528
Ala Leu Leu Thr Trp Ala Trp Phe Ala Val Val Tyr Phe Ala Pro Gly
                165                 170                 175

ggt gac ggc tgg ttt gga gcc tgc tac aat tcc tcc att cat gtc ctg          576
Gly Asp Gly Trp Phe Gly Ala Cys Tyr Asn Ser Ser Ile His Val Leu
            180                 185                 190

atg tac tct tac tat ctg ctt gcc acc ttc ggc atc tcc tgt ccc tgg          624
Met Tyr Ser Tyr Tyr Leu Leu Ala Thr Phe Gly Ile Ser Cys Pro Trp
        195                 200                 205

aaa aag atc ctc acc cag ctg caa atg gtt cag ttc tgc ttt tgc ttc          672
Lys Lys Ile Leu Thr Gln Leu Gln Met Val Gln Phe Cys Phe Cys Phe
    210                 215                 220

acc cac tcg atc tac gtg tgg att tgc ggt tcc gaa atc tac cct cga          720
Thr His Ser Ile Tyr Val Trp Ile Cys Gly Ser Glu Ile Tyr Pro Arg
225                 230                 235                 240

ccc ttg act gct ctc cag tcc ttc gtg atg gtc aac atg ctg gtt ctc          768
Pro Leu Thr Ala Leu Gln Ser Phe Val Met Val Asn Met Leu Val Leu
                245                 250                 255

ttt ggc aac ttc tac gtc aag cag tat tct cag aag aat gga aag ccc          816
Phe Gly Asn Phe Tyr Val Lys Gln Tyr Ser Gln Lys Asn Gly Lys Pro
            260                 265                 270

gag aac ggt gcc act cct gag aac ggt gcc aag cct cag ccc tgc gag          864
Glu Asn Gly Ala Thr Pro Glu Asn Gly Ala Lys Pro Gln Pro Cys Glu
        275                 280                 285

aac ggt acc gtg gag aag cga gaa aac gac acc gcc aat gtt cga ccc          912
Asn Gly Thr Val Glu Lys Arg Glu Asn Asp Thr Ala Asn Val Arg Pro
    290                 295                 300

gct aga cct gcc ggt ctt cca ccc gca act tac tat gac tct ctc gcc          960
Ala Arg Pro Ala Gly Leu Pro Pro Ala Thr Tyr Tyr Asp Ser Leu Ala
305                 310                 315                 320

gtg tcg gga cag ggc aag gag cga ctg ttc acc aca gac gag gtc aga         1008
Val Ser Gly Gln Gly Lys Glu Arg Leu Phe Thr Thr Asp Glu Val Arg
                325                 330                 335

cga cac att ctt ccc acc gat gga tgg ctc acc tgt cac gaa ggt gtg         1056
Arg His Ile Leu Pro Thr Asp Gly Trp Leu Thr Cys His Glu Gly Val
            340                 345                 350

tac gac gtc acc gat ttc ctg gcc aag cat cct gga ggt ggc gtc atc         1104
Tyr Asp Val Thr Asp Phe Leu Ala Lys His Pro Gly Gly Gly Val Ile
        355                 360                 365

act ctc gga ctt ggt cga gac tgc acc att ctg gtc gag tcc tac cat         1152
Thr Leu Gly Leu Gly Arg Asp Cys Thr Ile Leu Val Glu Ser Tyr His
    370                 375                 380

ccc gct gga cga cca gac aag gtc atg gag aag tac cgt atc ggc aca         1200
Pro Ala Gly Arg Pro Asp Lys Val Met Glu Lys Tyr Arg Ile Gly Thr
385                 390                 395                 400

ctc cag gat ccc aag act ttc tac gct tgg gga gaa tcg gac ttt tac         1248
Leu Gln Asp Pro Lys Thr Phe Tyr Ala Trp Gly Glu Ser Asp Phe Tyr
                405                 410                 415
```

```
cct gag ctc aaa cga cgg gct ctt gca cga ctc aag gag gct gga cag      1296
Pro Glu Leu Lys Arg Arg Ala Leu Ala Arg Leu Lys Glu Ala Gly Gln
            420                 425                 430

gca cgt cga ggt gga ctg gga gtc aag gct ctc ctg gtg ctt acc ctc      1344
Ala Arg Arg Gly Gly Leu Gly Val Lys Ala Leu Leu Val Leu Thr Leu
        435                 440                 445

ttc ttt gtc tcc tgg tac atg tgg gtt gcc cac aag tct ttc ctg tgg      1392
Phe Phe Val Ser Trp Tyr Met Trp Val Ala His Lys Ser Phe Leu Trp
    450                 455                 460

gct gcc gtc tgg ggc ttt gcc ggt tcg cat gtc gga ctg agc att caa      1440
Ala Ala Val Trp Gly Phe Ala Gly Ser His Val Gly Leu Ser Ile Gln
465                 470                 475                 480

cac gat ggc aac cac ggt gct ttc tct cga tcc acc ctg gtc aac aga      1488
His Asp Gly Asn His Gly Ala Phe Ser Arg Ser Thr Leu Val Asn Arg
                485                 490                 495

ctc gca gga tgg ggc atg gac ttg atc ggt gcc tcg tcc acc gtg tgg      1536
Leu Ala Gly Trp Gly Met Asp Leu Ile Gly Ala Ser Ser Thr Val Trp
            500                 505                 510

gag tac cag cat gtc att gga cac cat cag tac acg aat ctc gtt tcc      1584
Glu Tyr Gln His Val Ile Gly His His Gln Tyr Thr Asn Leu Val Ser
        515                 520                 525

gat acg ctg ttc agc ctt ccc gag aac gac cca gat gtg ttt tcc agc      1632
Asp Thr Leu Phe Ser Leu Pro Glu Asn Asp Pro Asp Val Phe Ser Ser
    530                 535                 540

tat cct ctc atg cga atg cat ccc gac act gct tgg cag cct cat cac      1680
Tyr Pro Leu Met Arg Met His Pro Asp Thr Ala Trp Gln Pro His His
545                 550                 555                 560

cga ttt caa cac ctg ttc gcc ttt cct ctc ttc gct ctt atg acc att      1728
Arg Phe Gln His Leu Phe Ala Phe Pro Leu Phe Ala Leu Met Thr Ile
                565                 570                 575

tcc aag gtg ctc acc agc gac ttt gct gtt tgt ctc tcc atg aaa aag      1776
Ser Lys Val Leu Thr Ser Asp Phe Ala Val Cys Leu Ser Met Lys Lys
            580                 585                 590

ggt tct atc gac tgt tcg tcc aga ctg gtt cca ctc gaa ggc cag ctg      1824
Gly Ser Ile Asp Cys Ser Ser Arg Leu Val Pro Leu Glu Gly Gln Leu
        595                 600                 605

ttg ttc tgg gga gcc aag ctg gcg aac ttt ctc ttg cag att gtg ctg      1872
Leu Phe Trp Gly Ala Lys Leu Ala Asn Phe Leu Leu Gln Ile Val Leu
    610                 615                 620

ccc tgc tac ctc cac gga act gct atg ggc ctt gct ctg ttc tct gtt      1920
Pro Cys Tyr Leu His Gly Thr Ala Met Gly Leu Ala Leu Phe Ser Val
625                 630                 635                 640

gcc cac ctt gtg tct gga gag tac ctg gcc atc tgt ttc atc att aac      1968
Ala His Leu Val Ser Gly Glu Tyr Leu Ala Ile Cys Phe Ile Ile Asn
                645                 650                 655

cac atc tcc gag tct tgc gaa ttt atg aat acc tcc ttt caa act gct      2016
His Ile Ser Glu Ser Cys Glu Phe Met Asn Thr Ser Phe Gln Thr Ala
            660                 665                 670
```

```
gcc cga cgg acc gag atg ctc cag gca gct cat cag gca gcc gag gcg        2064
Ala Arg Arg Thr Glu Met Leu Gln Ala Ala His Gln Ala Ala Glu Ala
        675                 680                 685

aaa aag gtc aag ccc act cct cca ccc aac gat tgg gct gtg aca caa        2112
Lys Lys Val Lys Pro Thr Pro Pro Pro Asn Asp Trp Ala Val Thr Gln
    690                 695                 700

gtc cag tgc tgt gtc aac tgg cga tct gga ggc gtg ctg gcc aat cac        2160
Val Gln Cys Cys Val Asn Trp Arg Ser Gly Gly Val Leu Ala Asn His
705                 710                 715                 720

ctc tcc ggt ggc ctc aac cat cag atc gag cac cat ctg ttt ccc agc        2208
Leu Ser Gly Gly Leu Asn His Gln Ile Glu His His Leu Phe Pro Ser
                725                 730                 735

atc tct cac gcg aac tat ccc atc att gct cga gtt gtc aag gaa gtg        2256
Ile Ser His Ala Asn Tyr Pro Ile Ile Ala Arg Val Val Lys Glu Val
            740                 745                 750

tgc gag gaa tac gga ttg ccc tac aag aac tac gtt acg ttc tgg gat        2304
Cys Glu Glu Tyr Gly Leu Pro Tyr Lys Asn Tyr Val Thr Phe Trp Asp
        755                 760                 765

gcc gtg tgt ggc atg gtt cag cat ctg cga ctc atg ggt gct cct ccc        2352
Ala Val Cys Gly Met Val Gln His Leu Arg Leu Met Gly Ala Pro Pro
    770                 775                 780

gtg cct acc aac ggc gac aaa aag tcc taa                                2382
Val Pro Thr Asn Gly Asp Lys Lys Ser
785                 790
```

<210> 76
<211> 793
<212> PRT
<213> Euglena gracilis

<400> 76

```
Met Ala Asp Ser Pro Val Ile Asn Leu Ser Thr Met Trp Lys Pro Leu
1               5                   10                  15

Ser Leu Met Ala Leu Asp Leu Ala Val Leu Gly His Val Trp Lys Gln
            20                  25                  30

Ala Gln Gln Glu Gly Ser Ile Ser Ala Tyr Ala Asp Ser Val Trp Thr
        35                  40                  45

Pro Leu Ile Met Ser Gly Leu Tyr Leu Ser Met Ile Phe Val Gly Cys
    50                  55                  60

Arg Trp Met Lys Asn Arg Glu Pro Phe Glu Ile Lys Thr Tyr Met Phe
65                  70                  75                  80

Ala Tyr Asn Leu Tyr Gln Thr Leu Met Asn Leu Cys Ile Val Leu Gly
                85                  90                  95

Phe Leu Tyr Gln Val His Ala Thr Gly Met Arg Phe Trp Gly Ser Gly
```

```
            100                 105                 110

Val Asp Arg Ser Pro Lys Gly Leu Gly Ile Gly Phe Phe Ile Tyr Ala
        115                 120                 125

His Tyr His Asn Lys Tyr Val Glu Tyr Phe Asp Thr Leu Phe Met Val
    130                 135                 140

Leu Arg Lys Lys Asn Asn Gln Ile Ser Phe Leu His Val Tyr His His
145                 150                 155                 160

Ala Leu Leu Thr Trp Ala Trp Phe Ala Val Val Tyr Phe Ala Pro Gly
                165                 170                 175

Gly Asp Gly Trp Phe Gly Ala Cys Tyr Asn Ser Ser Ile His Val Leu
            180                 185                 190

Met Tyr Ser Tyr Tyr Leu Leu Ala Thr Phe Gly Ile Ser Cys Pro Trp
        195                 200                 205

Lys Lys Ile Leu Thr Gln Leu Gln Met Val Gln Phe Cys Phe Cys Phe
    210                 215                 220

Thr His Ser Ile Tyr Val Trp Ile Cys Gly Ser Glu Ile Tyr Pro Arg
225                 230                 235                 240

Pro Leu Thr Ala Leu Gln Ser Phe Val Met Val Asn Met Leu Val Leu
                245                 250                 255

Phe Gly Asn Phe Tyr Val Lys Gln Tyr Ser Gln Lys Asn Gly Lys Pro
            260                 265                 270

Glu Asn Gly Ala Thr Pro Glu Asn Gly Ala Lys Pro Gln Pro Cys Glu
        275                 280                 285

Asn Gly Thr Val Glu Lys Arg Glu Asn Asp Thr Ala Asn Val Arg Pro
    290                 295                 300

Ala Arg Pro Ala Gly Leu Pro Pro Ala Thr Tyr Tyr Asp Ser Leu Ala
305                 310                 315                 320

Val Ser Gly Gln Gly Lys Glu Arg Leu Phe Thr Thr Asp Glu Val Arg
                325                 330                 335

Arg His Ile Leu Pro Thr Asp Gly Trp Leu Thr Cys His Glu Gly Val
            340                 345                 350

Tyr Asp Val Thr Asp Phe Leu Ala Lys His Pro Gly Gly Gly Val Ile
        355                 360                 365
```

Thr Leu Gly Leu Gly Arg Asp Cys Thr Ile Leu Val Glu Ser Tyr His
370 375 380

Pro Ala Gly Arg Pro Asp Lys Val Met Glu Lys Tyr Arg Ile Gly Thr
385 390 395 400

Leu Gln Asp Pro Lys Thr Phe Tyr Ala Trp Gly Glu Ser Asp Phe Tyr
405 410 415

Pro Glu Leu Lys Arg Arg Ala Leu Ala Arg Leu Lys Glu Ala Gly Gln
420 425 430

Ala Arg Arg Gly Gly Leu Gly Val Lys Ala Leu Leu Val Leu Thr Leu
435 440 445

Phe Phe Val Ser Trp Tyr Met Trp Val Ala His Lys Ser Phe Leu Trp
450 455 460

Ala Ala Val Trp Gly Phe Ala Gly Ser His Val Gly Leu Ser Ile Gln
465 470 475 480

His Asp Gly Asn His Gly Ala Phe Ser Arg Ser Thr Leu Val Asn Arg
485 490 495

Leu Ala Gly Trp Gly Met Asp Leu Ile Gly Ala Ser Ser Thr Val Trp
500 505 510

Glu Tyr Gln His Val Ile Gly His His Gln Tyr Thr Asn Leu Val Ser
515 520 525

Asp Thr Leu Phe Ser Leu Pro Glu Asn Asp Pro Asp Val Phe Ser Ser
530 535 540

Tyr Pro Leu Met Arg Met His Pro Asp Thr Ala Trp Gln Pro His His
545 550 555 560

Arg Phe Gln His Leu Phe Ala Phe Pro Leu Phe Ala Leu Met Thr Ile
565 570 575

Ser Lys Val Leu Thr Ser Asp Phe Ala Val Cys Leu Ser Met Lys Lys
580 585 590

Gly Ser Ile Asp Cys Ser Ser Arg Leu Val Pro Leu Glu Gly Gln Leu
595 600 605

Leu Phe Trp Gly Ala Lys Leu Ala Asn Phe Leu Leu Gln Ile Val Leu
610 615 620

```
Pro Cys Tyr Leu His Gly Thr Ala Met Gly Leu Ala Leu Phe Ser Val
625                 630                 635                 640

Ala His Leu Val Ser Gly Glu Tyr Leu Ala Ile Cys Phe Ile Ile Asn
                645                 650                 655

His Ile Ser Glu Ser Cys Glu Phe Met Asn Thr Ser Phe Gln Thr Ala
            660                 665                 670

Ala Arg Arg Thr Glu Met Leu Gln Ala Ala His Gln Ala Ala Glu Ala
        675                 680                 685

Lys Lys Val Lys Pro Thr Pro Pro Pro Asn Asp Trp Ala Val Thr Gln
    690                 695                 700

Val Gln Cys Cys Val Asn Trp Arg Ser Gly Gly Val Leu Ala Asn His
705                 710                 715                 720

Leu Ser Gly Gly Leu Asn His Gln Ile Glu His His Leu Phe Pro Ser
                725                 730                 735

Ile Ser His Ala Asn Tyr Pro Ile Ile Ala Arg Val Val Lys Glu Val
            740                 745                 750

Cys Glu Glu Tyr Gly Leu Pro Tyr Lys Asn Tyr Val Thr Phe Trp Asp
        755                 760                 765

Ala Val Cys Gly Met Val Gln His Leu Arg Leu Met Gly Ala Pro Pro
    770                 775                 780

Val Pro Thr Asn Gly Asp Lys Lys Ser
785                 790
```

<210> 77
<211> 9641
<212> DNA
<213> Artificial Sequence

<220>
<223> Plasmid pY201

<400> 77

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
```

ccagataagg ttccgataaa aagttctgca gactaaattt atttcagtct cctcttcacc 420
accaaaatgc cctcctacga agctcgagct aacgtccaca agtccgcctt tgccgctcga 480
gtgctcaagc tcgtggcagc caagaaaacc aacctgtgtg cttctctgga tgttaccacc 540
accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag ccccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
atttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280

tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580
ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260

atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180

ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300
atttttgccc attttccctt ctcctgtctc tccgacggtg atggcgttgt gcgtcctcta 6360
tttcttttta tttcttttttg ttttatttct ctgactaccg atttggtttg atttcctcaa 6420
ccccacacaa ataagctcgg gccgaggaat atatatatac acggacacag tcgccctgtg 6480
gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catgtacaac cccgtggacg cagtgttgac 7320
taagattatt acaaactacg gaattgattc ttttaccctg cgatatgcca tttgtctgtt 7380
gggatctttt cctcttaacg ctattctgaa gcggattcct gaaaagcgaa tcggcctgaa 7440
gtgttgtttt atcatttcta tgtccatgtt ttatctcttc ggcgttctga atctcgtgag 7500
cggatttcga accctcttca tttccacaat gttcacatac cttatctctc ggttctaccg 7560
atccaagttt atgccccatc tcaacttcat gttcgtcatg ggccacttgg ctatcaacca 7620
cattcatgct cagttcctga acgaacaaac tcaaacgacc gtcgatatta catcctcgca 7680
gatggtcctg gctatgaagc tgacaagctt tgcctggtct tactatgacg gttcgtgtac 7740
gagcgagtcc gacttcaagg accttaccga acaccagaag tcccgagccg tccgaggcca 7800
tcctcccctt ctgaaatttt tggcttacgc ctttttctac tctacccttc tcaccggtcc 7860
ctccttcgat tacgctgatt tcgactcttg gctgaactgc gaaatgttcc gggaccttcc 7920
cgagtccaag aaacccatgc gaagacatca tcctggtgag cggcgtcaga ttcccaagaa 7980
cggcaagctc gccctgtgga aggttgtcca gggcctcgcc tggatgattc tgagcacgtt 8040
gggtatgaag cacttccccg tgaagtacgt gctggacaag gacggatttc ctacccgttc 8100
ctttatcttc cgtattcatt atctgtttct gctgggattc atccaccgat ttaagtatta 8160

cgctgcgtgg acgattagcg aaggttcgtg cattctctgt ggtcttggtt ataatggata 8220
cgattctaag acccagaaga tccggtggga tcgagtgcgg aatattgata tttggacagt 8280
ggagactgca caaaacaccc gagagatgct ggaagcgtgg aacatgaata ctaacaaatg 8340
gctgaagtat agcgtgtatc ttagagtgac taagaagggt aagaagccag gttttcgatc 8400
taccctgttt accttcctga cctccgcctt ttggcacggt acccgtcctg gatactacct 8460
taccttcgca actggtgccc tgtaccaaac ctgtggaaag atctatagac gaaactttcg 8520
tcccatcttt ctgagagaag atggcgtgac acctctcccg tccaagaaga tttacgacct 8580
ggtcggcatt tacgctatta agctggcctt tggttacatg gttcaaccct tcattatcct 8640
tgacctgaag ccctctctta tggtttgggg atccgtgtat ttctacgtgc atattattgt 8700
ggccttctcg ttctttctgt tccgaggacc atacgctaag caggttactg aatttttcaa 8760
aagcaagcaa ccgaaggaga tcttcatccg aaagcagaag aagttggaaa aagacatctc 8820
tgcctcttcc cccaacctcg gaggtattct taaggcaaaa atcgaacatg agaagggaaa 8880
gacggcagag gaggaagaga tgaacttggg cattccaccc atcgaactgg agaagtggga 8940
caacgccaag gaggactggg aggatttctg caaggactac aaggagtggc ggaacaagaa 9000
cggactggaa attgaagagg agaacctgtc caaggccttc gagcgattta agcaggaatt 9060
ttccaacgct gcgtcgggct ctggtgaacg ggttcggaaa atgtccttct ccggatattc 9120
tcctaaaccc atctcgaaga aagaagaata ggcggccgca tgagaagata aatatataaa 9180
tacattgaga tattaaatgc gctagattag agagcctcat actgctcgga gagaagccaa 9240
gacgagtact caaaggggat tacaccatcc atatccacag acacaagctg gggaaaggtt 9300
ctatatacac tttccggaat accgtagttt ccgatgttat caatgggggc agccaggatt 9360
tcaggcactt cggtgtctcg ggtgaaatgg gcgttcttgg cctccatcaa gtcgtaccat 9420
gtcttcattt gcctgtcaaa gtaaaacaga agcagatgaa gaatgaactt gaagtgaagg 9480
aatttaaatg taacgaaact gaaatttgac cagatattgt gtccgcggtg gagctccagc 9540
ttttgttccc tttagtgagg gttaatttcg agcttggcgt aatcatggtc atagctgttt 9600
cctgtgtgaa attgttatcc gctcacaagc ttccacacaa c 9641

<210> 78
<211> 34
<212> DNA
<213> Escherichia coli

<400> 78
ataacttcgt ataatgtatg ctatacgaag ttat 34

<210> 79
<211> 40
<212> DNA

<213> Artificial Sequence

<220>
<223> Primer 798

<400> 79
ctaattcaca accatggcct ttccatgggc agataagtgg 40

<210> 80
<211> 36
<212> DNA
<213> Artificial Sequence

<220>
<223> Primer 799

<400> 80
ctcatgcggc cgcttacttg gtcttgatgg tgtcct 36

<210> 81
<211> 40
<212> DNA
<213> Artificial Sequence

<220>
<223> Primer 800

<400> 81
cgatagttag tagacaacaa tcgatagttg gagcaaggga 40

<210> 82
<211> 36
<212> DNA
<213> Artificial Sequence

<220>
<223> Primer 801

<400> 82
gaaaggccat ggttgtgaat tagggtggtg agaatg 36

<210> 83
<211> 9320
<212> DNA
<213> Artificial Sequence

<220>
<223> Plasmid pY168

<400> 83

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
```

ccagataagg ttccgataaa aagttctgca gactaaattt atttcagtct cctcttcacc 420
accaaaatgc cctcctacga agctcgagct aacgtccaca agtccgcctt tgccgctcga 480
gtgctcaagc tcgtggcagc caagaaaacc aacctgtgtg cttctctgga tgttaccacc 540
accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag ccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
attttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340

tgattttgat ttgcacgatg gaattgagaa ctttgtaaac gtacatggga atgtatgaat 2400
gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580
ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260

atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240

ggacaagagg ctcttgtggg agccgaatga gagcacaaag cgggcgggtg tgataagggc 6300
atttttgccc attttccctt ctcctgtctc tccgacggtg atggcgttgt gcgtcctcta 6360
tttcttttta tttctttttg ttttatttct ctgactaccg atttggtttg atttcctcaa 6420
ccccacacaa ataagctcgg gccgaggaat atatatatac acggacacag tcgccctgtg 6480
gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catggccttt ccctgggcag ataagtgggc 7320
agccgatgcg tctgcatcta cagggctgcc tccggacctc ctcaagattg cattcactct 7380
ggtcatgtct tatccgctga gttctctcat gaaacggctg ccagatgacg ccaaaaacct 7440
caagatcatc tatatcatct ccgtgtccat cttctacatg gtgggtgtct tctccctcta 7500
tggcggagct gccactctgc tcttctcctc aatgggtacc ttcttcatca cccaatggaa 7560
gagcccttac atgccctggg tcaattttgg ttttgtcatg acccatctct tcgtcaatca 7620
cctgcgttcg cagtttttcc ccgaaacata cgaccccaat gtcattgaca tcaccggagc 7680
acagatggtt ctgtgtatga agctatcgtc ttttggatgg aacgtctacg atggatggca 7740
gattgagaag ggtgagcagc tcagcgagtt ccagactaaa agggctgttc tcaagcaccc 7800
cagtcttatg gacttcctag cttttgtgtt ctacttccct tccattctga caggtccttc 7860
ttacgactat atggagttcc ataactggct cgatctcagc ctgttcaagg agctggagaa 7920
agataaggac cccaagcgag ctgctcgacg aaagcgacac aagatccccc gatctggaat 7980
cgctgcttcc aagaaactcg ccgctggtat cttctggatc gttctgtgga cccaggtgga 8040
ctctcgaatc tccaccgcct acgcttactc agacgcattc accaaggagc acaacatctt 8100
tggacgaatt gtgtacctct acatgctcgg tttcatgtac cgactcaagt actacggagc 8160

ctggtccatt tccgagggag cctgcatctt gtctggcctc ggattccatg gcgtggaccc 8220

caaaactggc aagtacaagt gggaccgtgt ccagaacgtg gacccgtggg gattcgaaac 8280

tggtcaaaac acaaaggctc tgctggaggc ctggaaccag aacactaaca agtggctacg 8340

aaactatgtg tacctccgag tggtgcccaa aggccaaaag cctggattcc gagccactat 8400

cttcacattt gtggtttccg ccttctggca tggaactcga cctggctact atctcacctt 8460

tgtgaccgct gccatgtacc agtctgttgg taagttcttc cgacgatacc tgcgaccctt 8520

cttcatggag tctgatggaa agactgccgg tccctataag atctactacg acattgtgtg 8580

ttggatcgtt gtccaaaccg catttggata cgctacccag tcctttatga ttctagactt 8640

ctggctgtcg ctcaagtgtt ggaagaactc ctggttcctg taccacattg ctctgggcgc 8700

catctttgca atttctagcc cctacaaggc atgggcgatt cccaagatca agaaaaagca 8760

ggctggagcc gtcactgaca agaaggacgc caaggaggag gtgaagaagg acaccatcaa 8820

gaccaagtaa gcggccgcat gagaagataa atatataaat acattgagat attaaatgcg 8880

ctagattaga gagcctcata ctgctcggag agaagccaag acgagtactc aaaggggatt 8940

acaccatcca tatccacaga cacaagctgg ggaaaggttc tatatacact ttccggaata 9000

ccgtagtttc cgatgttatc aatgggggca gccaggattt caggcacttc ggtgtctcgg 9060

ggtgaaatgg cgttcttggc ctccatcaag tcgtaccatg tcttcatttg cctgtcaaag 9120

taaaacagaa gcagatgaag aatgaacttg aagtgaagga atttaaatgt aacgaaactg 9180

aaatttgacc agatattgtg tccgcggtgg agctccagct tttgttccct ttagtgaggg 9240

ttaatttcga gcttggcgta atcatggtca tagctgtttc ctgtgtgaaa ttgttatccg 9300

ctcacaagct tccacacaac 9320

<210> 84
<211> 8726
<212> DNA
<213> Artificial Sequence

<220>
<223> Plasmid pY208

<400> 84

gtacgataac ttcgtatagc atacattata cgaagttatc gcgtcgacga gtatctgtct 60

gactcgtcat tgccgccttt ggagtacgac tccaactatg agtgtgcttg gatcactttg 120

acgatacatt cttcgttgga ggctgtgggt ctgacagctg cgttttcggc gcggttggcc 180

gacaacaata tcagctgcaa cgtcattgct ggctttcatc atgatcacat ttttgtcggc 240

aaaggcgacg cccagagagc cattgacgtt ctttctaatt tggaccgata gccgtatagt 300

ccagtctatc tataagttca actaactcgt aactattacc ataacatata cttcactgcc 360

ccagataagg ttccgataaa aagttctgca gactaaattt atttcagtct cctcttcacc 420

accaaaatgc cctcctacga agctcgagct aacgtccaca agtccgcctt tgccgctcga 480

gtgctcaagc tcgtggcagc caagaaaacc aacctgtgtg cttctctgga tgttaccacc 540
accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag cccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
atttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400

gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580
ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320
accgcttttt tgcacaacat gggggatcat gtaactcgcc ttgatcgttg ggaaccggag 4380

ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300

atttttgccc attttccctt ctcctgtctc tccgacggtg atggcgttgt gcgtcctcta 6360
tttcttttta tttcttttg ttttatttct ctgactaccg atttggtttg atttcctcaa 6420
ccccacacaa ataagctcgg gccgaggaat atatatatac acggacacag tcgccctgtg 6480
gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catgtctatt ggttcgtcca accccgtgct 7320
cttggctgcg attcccttcg tctacctgtt tgtcctccca cgagtcctgg ctttcctgcc 7380
tcagaaggct cagttcctgg ccaaatgtat tgtggtcctg attgccacgc ttatcatgtc 7440
cgttgcaggc tgcttcatct cgatcgtgtg cgctcttctg gacaagagat acgtcatcaa 7500
ttacgttgtg tcgcgattgt tctccttcct tgccgctcga ccgtgtggtg tgacctataa 7560
gattgttggt gaggaacacc tcgataagta ccctgctatc gtggtctgta accatcaatc 7620
ctctatggat atgatggttt tgggacgagt tttccaaag cactgcgttg tcatggcgaa 7680
gaaggaactc ctgtactttc ccttttggg aatgtttatg aaactgagca acgctatctt 7740
catcgaccgg aagaaccaca agaaagccat cgagtctacc acccaagccg tggcggacat 7800
gaagaagcac aactctggaa tctggatttt cccagagggc acccggtcta gactggacaa 7860
ggcagacctg ctgcccttca agaaaggtgc ctttcatctt gcaattcagg cccagctccc 7920
tattctcccc attatctcgc agggctattc ccatatctac gactcttcga agcggtactt 7980
ccccggtgga gagctcgaga tcagagtcct ggagcccatt cctacaactg gcctcactac 8040
tgatgatgtg aacgacctga tggacaagac acgaaacctt atgctcaagc acttgaagga 8100
gatggattcc cagtattcgt cgagcactgc tgaaaatgga tccacgcaca tcgacgccga 8160
tattgccaag tctacagcca ccagcattgg caacactgac gacgcaatta caaaacgtcg 8220
taccccctaag gaataagcgg ccgcatgaga agataaatat ataaatacat tgagatatta 8280

**aatgcgctag attagagagc ctcatactgc tcggagagaa gccaagacga gtactcaaag 8340**

**gggattacac catccatatc cacagacaca agctggggaa aggttctata tacactttcc 8400**

**ggaataccgt agtttccgat gttatcaatg ggggcagcca ggatttcagg cacttcggtg 8460**

**tctcggggtg aaatggcgtt cttggcctcc atcaagtcgt accatgtctt catttgcctg 8520**

**tcaaagtaaa acagaagcag atgaagaatg aacttgaagt gaaggaattt aaatgtaacg 8580**

**aaactgaaat ttgaccagat attgtgtccg cggtggagct ccagcttttg ttccctttag 8640**

**tgagggttaa tttcgagctt ggcgtaatca tggtcatagc tgtttcctgt gtgaaattgt 8700**

**tatccgctca caagcttcca cacaac 8726**

<210> 85
<211> 31
<212> DNA
<213> Artificial Sequence

<220>
<223> Primer 856

<400> 85
tcacaacaca tgtccgttgc atccaagctc g 31

<210> 86
<211> 28
<212> DNA
<213> Artificial Sequence

<220>
<223> Primer 857

<400> 86
tttagcggcc gcctactgag tcttctgg 28

<210> 87
<211> 8630
<212> DNA
<213> Artificial Sequence

<220>
<223> Plasmid pY207

<400> 87

gtacgataac ttcgtatagc atacattata cgaagttatc gcgtcgacga gtatctgtct 60

gactcgtcat tgccgccttt ggagtacgac tccaactatg agtgtgcttg gatcactttg 120

acgatacatt cttcgttgga ggctgtgggt ctgacagctg cgttttcggc gcggttggcc 180

gacaacaata tcagctgcaa cgtcattgct ggctttcatc atgatcacat ttttgtcggc 240

aaaggcgacg cccagagagc cattgacgtt ctttctaatt tggaccgata gccgtatagt 300

ccagtctatc tataagttca actaactcgt aactattacc ataacatata cttcactgcc 360

ccagataagg ttccgataaa aagttctgca gactaaattt atttcagtct cctcttcacc 420

accaaaatgc cctcctacga agctcgagct aacgtccaca agtccgcctt tgccgctcga 480
gtgctcaagc tcgtggcagc caagaaaacc aacctgtgtg cttctctgga tgttaccacc 540
accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag cccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
atttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400

gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580
ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320

accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300

atttttgccc attttccctt ctcctgtctc tccgacggtg atggcgttgt gcgtcctcta 6360
tttcttttta tttctttttg ttttatttct ctgactaccg atttggtttg atttcctcaa 6420
ccccacacaa ataagctcgg gccgaggaat atatatatac acggacacag tcgccctgtg 6480
gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catgtccgtt gcatccaagc tcgtcttcta 7320
cgtccgcgcc gccatcgccg tggtcatctt tgccgcctgt gccacctacg gcgtgctggc 7380
gtccaccatt ctcaccgcca tcggcaagca gggcctggcc caatggaccg ttgccagagc 7440
cttctactac tcggtgcgca tcttcctggg tatcagcatc aagctgcgta gccggcaggt 7500
gaccggaacc gccggtctgg atgcctccaa gatccaggtc gccaacacca ccaagcccat 7560
tgacgacatc accaaacacc tgccccgacc atgcattctg atttccaacc accagaacga 7620
aatggacatt ctggtgctcg gtcgcatctt cccccagtac tgctccgtca ccgccaaaaa 7680
ggccctcaag tggtaccctc tgctgggcca gttcatggcg ctgtccggca ccatcttcct 7740
ggaccgaaag gaccgaacca agtccgtgca gaccctcggc ggcgccgtca agaccatcca 7800
gagcggcaac ggaggcaagg gccagagcgt cttcatgttc cccgagggaa cccgatccta 7860
ctccaaggac gtcggcatca tgcccttcaa gaagggctgt ttccacctgg cggtccagtc 7920
gggcgctccc attgtccccg tggtggtcca gaacacctcc cgaatgtttt ctttcggccg 7980
aggcaagctg gacgccggag agatccttgt cgacgtcctg agccccattg agaccaaggg 8040
tctggacgcc agcaacgtcg acgctctcat ggccaccact tataaggcca tgtgcgagac 8100
tgccgaccag attggctacg ctggccagaa gactcagtag gcggccgcat gagaagataa 8160
atatataaat acattgagat attaaatgcg ctagattaga gagcctcata ctgctcggag 8220

```
agaagccaag acgagtactc aaaggggatt acaccatcca tatccacaga cacaagctgg    8280
ggaaaggttc tatatacact ttccggaata ccgtagtttc cgatgttatc aatgggggca    8340
gccaggattt caggcacttc ggtgtctcgg ggtgaaatgg cgttcttggc ctccatcaag    8400
tcgtaccatg tcttcatttg cctgtcaaag taaaacagaa gcagatgaag aatgaacttg    8460
aagtgaagga atttaaatgt aacgaaactg aaatttgacc agatattgtg tccgcggtgg    8520
agctccagct tttgttccct ttagtgaggg ttaatttcga gcttggcgta atcatggtca    8580
tagctgtttc ctgtgtgaaa ttgttatccg ctcacaagct tccacacaac               8630
```

<210> 88
<211> 8630
<212> DNA
<213> Artificial Sequence

<220>
<223> Plasmid pY175

<400> 88

gtacgataac ttcgtatagc atacattata cgaagttatc gcgtcgacga gtatctgtct 60
gactcgtcat tgccgccttt ggagtacgac tccaactatg agtgtgcttg gatcactttg 120
acgatacatt cttcgttgga ggctgtgggt ctgacagctg cgttttcggc gcggttggcc 180
gacaacaata tcagctgcaa cgtcattgct ggctttcatc atgatcacat ttttgtcggc 240
aaaggcgacg cccagagagc cattgacgtt ctttctaatt tggaccgata gccgtatagt 300
ccagtctatc tataagttca actaactcgt aactattacc ataacatata cttcactgcc 360
ccagataagg ttccgataaa aagttctgca gactaaattt atttcagtct cctcttcacc 420
accaaaatgc cctcctacga agctcgagct aacgtccaca agtccgcctt tgccgctcga 480
gtgctcaagc tcgtggcagc caagaaaacc aacctgtgtg cttctctgga tgttaccacc 540
accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260

taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag cccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
atttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580
ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180

taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160

ggcgataagt cgtgtcttac cgggttggac tcaagacgat agttaccgga taaggcgcag 5220

cggtcgggct gaacgggggg ttcgtgcaca cagcccagct tggagcgaac gacctacacc 5280

gaactgagat acctacagcg tgagctatga gaaagcgcca cgcttcccga agggagaaag 5340

gcggacaggt atccggtaag cggcagggtc ggaacaggag agcgcacgag ggagcttcca 5400

gggggaaacg cctggtatct ttatagtcct gtcgggtttc gccacctctg acttgagcgt 5460

cgatttttgt gatgctcgtc aggggggcgg agcctatgga aaaacgccag caacgcggcc 5520

tttttacggt tcctggcctt ttgctggcct tttgctcaca tgttctttcc tgcgttatcc 5580

cctgattctg tggataaccg tattaccgcc tttgagtgag ctgataccgc tcgccgcagc 5640

cgaacgaccg agcgcagcga gtcagtgagc gaggaagcgg aagagcgccc aatacgcaaa 5700

ccgcctctcc ccgcgcgttg gccgattcat taatgcagct ggcgcgccac caatcacaat 5760

tctgaaaagc acatcttgat ctcctcattg cggggagtcc aacggtggtc ttattccccc 5820

gaatttcccg ctcaatctcg ttccagaccg acccggacac agtgcttaac gccgttccga 5880

aactctaccg cagatatgct ccaacggact gggctgcata gatgtgatcc tcggcttgga 5940

gaaatggata aaagccggcc aaaaaaaaag cggaaaaaag cggaaaaaaa gagaaaaaaa 6000

atcgcaaaat ttgaaaaata gggggaaaag acgcaaaaac gcaaggaggg gggagtatat 6060

gacactgata agcaagctca caacggttcc tcttattttt ttcctcatct tctgcctagg 6120

ttcccaaaat cccagatgct tctctccagt gccaaaagta agtaccccac aggttttcgg 6180

ccgaaaattc cacgtgcagc aacgtcgtgt ggggtgttaa aatgtggggg gggggaacca 6240

ggacaagagg ctcttgtggg agccgaatga gagcacaaag cgggcgggtg tgataagggc 6300

atttttgccc attttccctt ctcctgtctc tccgacggtg atggcgttgt gcgtcctcta 6360

tttcttttta tttctttttg ttttatttct ctgactaccg atttggtttg atttcctcaa 6420

ccccacacaa ataagctcgg gccgaggaat atatatatac acggacacag tcgccctgtg 6480

gacaacacgt cactacctct acgatacaca ccgtacgata gttagtagac aacaatcgat 6540

agttggagca agggagaaat gtagagtgtg aaagactcac tatggtccgg gcttatctcg 6600

accaatagcc aaagtctgga gtttctgaga gaaaaaggca agatacgtat gtaacaaagc 6660

gacgcatggt acaataatac cggaggcatg tatcatagag agttagtggt tcgatgatgg 6720

cactggtgcc tggtatgact ttatacggct gactacatat ttgtcctcag acatacaatt 6780

acagtcaagc acttaccctt ggacatctgt aggtaccccc cggccaagac gatctcagcg 6840

tgtcgtatgt cggattggcg tagctccctc gctcgtcaat tggctcccat ctactttctt 6900

ctgcttggct acacccagca tgtctgctat ggctcgtttt cgtgccttat ctatcctccc 6960

agtattacca actctaaatg acatgatgtg attgggtcta cactttcata tcagagataa 7020

ggagtagcac agttgcataa aaagcccaac tctaatcagc ttcttccttt cttgtaatta 7080

```
gtacaaaggt gattagcgaa atctggaagc ttagttggcc ctaaaaaaat caaaaaaagc      7140
aaaaaacgaa aaacgaaaaa ccacagtttt gagaacaggg aggtaacgaa ggatcgtata      7200
tatatatata tatatatata cccacggatc ccgagaccgg cctttgattc ttccctacaa      7260
ccaaccattc tcaccaccct aattcacaac catggagaac ttctggtcca tcgtcgtgtt      7320
ctttctgctc tccattctgt tcatcctcta caacatttcg acagtctgcc actactacat      7380
gcgaatctcc ttctactact ttaccatcct gcttcacggc atggaggtgt gcgttaccat      7440
gattccctct tggctcaacg gcaagggtgc cgactacgtg tttcactcgt tcttctactg      7500
gtgcaagtgg actggagtcc acaccactgt gtatggctac gagaagaccc aggtcgaagg      7560
tcctgccgtg gtcatctgca accatcagtc ctcgctcgac attctgtcta tggcttccat      7620
ctggcccaag aactgtgttg tcatgatgaa gcggattctt gcctacgttc ccttcttcaa      7680
cctgggagcc tacttttcca acaccatctt catcgaccga tacaaccgag agcgagctat      7740
ggcttctgtc gactactgtg cctccgagat gaagaaccga aacctgaagc tctgggtgtt      7800
tcccgaaggc actcggaatc gagagggtgg attcattccc ttcaagaaag gtgccttcaa      7860
catcgctgtt cgagcccaga ttcccatcat tcctgtcgtg ttctctgact atcgagactt      7920
ctactccaag cctggccgat acttcaagaa cgatggagag gtcgtgatcc gagtcctgga      7980
tgccattccc accaagggtc tgaccctcga tgacgtctct gagctttcgg acatgtgtcg      8040
agacgtcatg ctggctgcct acaaggaagt taccctcgag gctcagcaac gaaacgccac      8100
tcgaagagga gagaccaagg acggcaagaa atccgagtaa gcggccgcat gagaagataa      8160
atatataaat acattgagat attaaatgcg ctagattaga gagcctcata ctgctcggag      8220
agaagccaag acgagtactc aaaggggatt acaccatcca tatccacaga cacaagctgg      8280
ggaaaggttc tatatacact ttccggaata ccgtagtttc cgatgttatc aatgggggca      8340
gccaggattt caggcacttc ggtgtctcgg ggtgaaatgg cgttcttggc ctccatcaag      8400
tcgtaccatg tcttcatttg cctgtcaaag taaaacagaa gcagatgaag aatgaacttg      8460
aagtgaagga atttaaatgt aacgaaactg aaatttgacc agatattgtg tccgcggtgg      8520
agctccagct tttgttccct ttagtgaggg ttaatttcga gcttggcgta atcatggtca      8580
tagctgtttc ctgtgtgaaa ttgttatccg ctcacaagct tccacacaac                 8630
```

<210> 89
<211> 8237
<212> DNA
<213> Artificial Sequence

<220>
<223> Plasmid pY153

<400> 89

```
cgataccatg gtcgggcaat gagaacggca gcaactgcaa tcacagcgac atatatccaa      60
ctgttcatgt ttggttttcg gatagtcaca caccttaatt ttgatgcacg ctttatggag     120
```

tctctctctc tttttctctc tctcttgtcg tcgctctttc ttgtttttca accacccact 180
tgattcctgc aaacaaacta cccacactaa tttttttcg ctgcataccc tcaaatgagc 240
ctaattggcg tgtgtctccg cacaaaaaca caccatgcac ggctgggctt gttgggaaac 300
tttgtcaggg gggtccaggg ggccattggc agacttggcc acgtgtgctc atctcggctt 360
cgtcgttatt acgtgtctgt gtaatcaaag tcgggcgttt tttgcgccat gtgtccgcat 420
gaaattggcc cctcttgaag tcccttgtgc acctacacgt gccgaaatga aggttggagt 480
cagcggggtc atgccgtggt attatgctgt ggcatgtggc attaagctgt ggcatcaagc 540
cgtggaatca agccgtggtt cacgcccttg attgcgcagg cacatggcgc cattcttgcc 600
tctgctgtaa gcccggcttt gtgtgattca gagacgctgc taccgcacaa ctgcccatac 660
tccttctcct actgtataca tccacccctc atgctgataa cattatcatc tcatctcaac 720
tcaacatttc caccaacttg ggatcaaaaa cacgttctaa tactgtacac tgtctacgat 780
ataattaccg tacagtgtgg ttataatgaa catctattag agagaattgt ttgttgctcg 840
tatcagtcat tgggaagcgg gataccatgt cattttcacc tatatcaacc atgaaactac 900
agtatgtaca gtagaagtat atactgtact gttttgtaac tatatgtaca gtagaagtat 960
atactatact gttattaact atacttgtga ctagtgttcc aaactacaag tatatactgt 1020
acttgtacac gactatccga ccagtatcca gtatacaata accaactact ctacgtacgt 1080
actaaactaa acaaatagat caatgctcaa tgtcgagctc cagcttttgt tccctttagt 1140
gagggttaat ttcgagcttg gcgtaatcat ggtcatagct gtttcctgtg tgaaattgtt 1200
atccgctcac aattccacac aacatacgag ccggaagcat aaagtgtaaa gcctggggtg 1260
cctaatgagt gagctaactc acattaattg cgttgcgctc actgcccgct ttccagtcgg 1320
gaaacctgtc gtgccagctg cattaatgaa tcggccaacg cgcggggaga ggcggtttgc 1380
gtattgggcg ctcttccgct tcctcgctca ctgactcgct gcgctcggtc gttcggctgc 1440
ggcgagcggt atcagctcac tcaaaggcgg taatacggtt atccacagaa tcaggggata 1500
acgcaggaaa gaacatgtga gcaaaaggcc agcaaaaggc caggaaccgt aaaaaggccg 1560
cgttgctggc gtttttccat aggctccgcc cccctgacga gcatcacaaa aatcgacgct 1620
caagtcagag gtggcgaaac ccgacaggac tataaagata ccaggcgttt ccccctggaa 1680
gctccctcgt gcgctctcct gttccgaccc tgccgcttac cggatacctg tccgcctttc 1740
tcccttcggg aagcgtggcg ctttctcata gctcacgctg taggtatctc agttcggtgt 1800
aggtcgttcg ctccaagctg ggctgtgtgc acgaaccccc cgttcagccc gaccgctgcg 1860
ccttatccgg taactatcgt cttgagtcca acccggtaag acacgactta tcgccactgg 1920
cagcagccac tggtaacagg attagcagag cgaggtatgt aggcggtgct acagagttct 1980
tgaagtggtg gcctaactac ggctacacta gaaggacagt atttggtatc tgcgctctgc 2040

tgaagccagt taccttcgga aaaagagttg gtagctcttg atccggcaaa caaaccaccg 2100
ctggtagcgg tggttttttt gtttgcaagc agcagattac gcgcagaaaa aaaggatctc 2160
aagaagatcc tttgatcttt tctacggggt ctgacgctca gtggaacgaa aactcacgtt 2220
aagggatttt ggtcatgaga ttatcaaaaa ggatcttcac ctagatcctt ttaaattaaa 2280
aatgaagttt taaatcaatc taaagtatat atgagtaaac ttggtctgac agttaccaat 2340
gcttaatcag tgaggcacct atctcagcga tctgtctatt tcgttcatcc atagttgcct 2400
gactccccgt cgtgtagata actacgatac gggagggctt accatctggc cccagtgctg 2460
caatgatacc gcgagaccca cgctcaccgg ctccagattt atcagcaata aaccagccag 2520
ccggaagggc cgagcgcaga agtggtcctg caactttatc cgcctccatc cagtctatta 2580
attgttgccg ggaagctaga gtaagtagtt cgccagttaa tagtttgcgc aacgttgttg 2640
ccattgctac aggcatcgtg gtgtcacgct cgtcgtttgg tatggcttca ttcagctccg 2700
gttcccaacg atcaaggcga gttacatgat cccccatgtt gtgcaaaaaa gcggttagct 2760
ccttcggtcc tccgatcgtt gtcagaagta agttggccgc agtgttatca ctcatggtta 2820
tggcagcact gcataattct cttactgtca tgccatccgt aagatgcttt tctgtgactg 2880
gtgagtactc aaccaagtca ttctgagaat agtgtatgcg gcgaccgagt tgctcttgcc 2940
cggcgtcaat acgggataat accgcgccac atagcagaac tttaaaagtg ctcatcattg 3000
gaaaacgttc ttcggggcga aaactctcaa ggatcttacc gctgttgaga tccagttcga 3060
tgtaacccac tcgtgcaccc aactgatctt cagcatcttt tactttcacc agcgtttctg 3120
ggtgagcaaa aacaggaagg caaaatgccg caaaaaaggg aataagggcg acacggaaat 3180
gttgaatact catactcttc ctttttcaat attattgaag catttatcag ggttattgtc 3240
tcatgagcgg atacatattt gaatgtattt agaaaaataa acaaataggg gttccgcgca 3300
catttccccg aaaagtgcca cctgacgcgc cctgtagcgg cgcattaagc gcggcgggtg 3360
tggtggttac gcgcagcgtg accgctacac ttgccagcgc cctagcgccc gctcctttcg 3420
ctttcttccc ttcctttctc gccacgttcg ccggctttcc ccgtcaagct ctaaatcggg 3480
ggctcccttt agggttccga tttagtgctt tacggcacct cgaccccaaa aaacttgatt 3540
agggtgatgg ttcacgtagt gggccatcgc cctgatagac ggtttttcgc cctttgacgt 3600
tggagtccac gttctttaat agtggactct tgttccaaac tggaacaaca ctcaacccta 3660
tctcggtcta ttcttttgat ttataaggga ttttgccgat ttcggcctat tggttaaaaa 3720
atgagctgat ttaacaaaaa tttaacgcga attttaacaa aatattaacg cttacaattt 3780
ccattcgcca ttcaggctgc gcaactgttg ggaagggcga tcggtgcggg cctcttcgct 3840
attacgccag ctggcgaaag ggggatgtgc tgcaaggcga ttaagttggg taacgccagg 3900
gttttcccag tcacgacgtt gtaaaacgac ggccagtgaa ttgtaatacg actcactata 3960
gggcgaattg ggtaccgggc cccccctcga gtctagagca gggtgttgga ggggatggag 4020

aggttgagta aagcggaagt tgcggtttgc tggtcgcgtt ttaatcttct tttgtaattt 4080
aatctcggta tgccgacgtg tttcggcgag ttaattttag ctgtcaaaaa aatggatcac 4140
caggatagac aaagaagaga gtgggacaag gatctttcca gccgctaata tcggccagtt 4200
taagagcaat tcacggtccc tggagtccat ataacaacgc caatctcgca cttgtctgct 4260
ccctcgtcac ccgtccaact gtccaatcca atctatgcct ctcatgatca tcttcacact 4320
acagtagagt aggtaaaggt agacttatgg gctcctcgaa taattggact ctgtctaaac 4380
cttgtgaagt tacctcggtc ggtagagttc ggctggaagg ccatgattac taacaacatc 4440
gtacgagaga aaatcaagat gagtaataca atctcgatga gtaatacaat ctcgatgagt 4500
aatacaatct cgatgagtaa gactaggtgg tgtcacgact tttagaggaa tgagcagctt 4560
tcagggttat tatagagaca cgtccgcgga cgaagtagct ggtacatcga gcataagcat 4620
ggtacaagta ggagtagact aaaaaccaac agtttgaata gtccatgaca gtacgggcgg 4680
gtacgactga tctaagagaa ctggggtata cacgatatag cacagtacag agaaagtggg 4740
ctcgttttgc gttggtaatc gaggtagatt tcgttgctat attaatccat tcacccatag 4800
ctccacagcc aatggttcgc cgtgggtgtc gatctgaaaa atgtttcata ttacctatct 4860
ctctcctaaa gtagctacaa gcacttcttg tgctgcagtc tgcggccgct tactcggatt 4920
tcttgccgtc cttggtctct cctcttcgag tggcgtttcg ttgctgagcc tcgagggtaa 4980
cttccttgta ggcagccagc atgacgtctc gacacatgtc cgaaagctca gagacgtcat 5040
cgagggtcag acccttggtg ggaatggcat ccaggactcg gatcacgacc tctccatcgt 5100
tcttgaagta tcggccaggc ttggagtaga agtctcgata gtcagagaac acgacaggaa 5160
tgatgggaat ctgggctcga acagcgatgt tgaaggcacc tttcttgaag ggaatgaatc 5220
caccctctcg attccgagtg ccttcgggaa acacccagag cttcaggttt cggttcttca 5280
tctcggaggc acagtagtcg acagaagcca tagctcgctc tcggttgtat cggtcgatga 5340
agatggtgtt ggaaaagtag gctcccaggt tgaagaaggg aacgtaggca agaatccgct 5400
tcatcatgac aacacagttc ttgggccaga tggaagccat agacagaatg tcgagcgagg 5460
actgatggtt gcagatgacc acggcaggac cttcgacctg ggtcttctcg tagccataca 5520
cagtggtgtg gactccagtc cacttgcacc agtagaagaa cgagtgaaac acgtagtcgg 5580
cacccttgcc gttgagccaa gagggaatca tggtaacgca cacctccatg ccgtgaagca 5640
ggatggtaaa gtagtagaag gagattcgca tgtagtagtg gcagactgtc gaaatgttgt 5700
agaggatgaa cagaatggag agcagaaaga acacgacgat ggaccagaag ttctccatgg 5760
taccagagct gggttagttt gtgtagagag tgtgtgttgc tagcgacttt cggattgtgt 5820
cattacacaa aacgcgtcgt ctcgacactg atcttgtcgt ggatactcac ggctcggaat 5880
tctgtgatgt gtagtttaga tttcgaatct gtggggaaag aaaggaaaaa agagactggc 5940

aaccgattgg gagagccact gtttatatat accctagaca agccccccgc ttgtaagatg 6000
ttggtcaatg taaaccagta ttaaggttgg caagtgcagg agaagcaagg tgtgggtatc 6060
gagcaatgga aatgtgcgga aggcaaaaaa atgaggccac ggcctattgt cggggctata 6120
tccagggggc gattgaagta cactaacatg acatgtgtcc acagaccctc aatctggcct 6180
gatgagccaa atccatacgc gctttcgcag ctctaaaggc tataacaagt cacaccaccc 6240
tgctcgacct cagcgccctc actttttgtt aagacaaact gtacacgctg ttccagcgtt 6300
ttctgcctgc acctggtggg acatttggtg caacctaaag tgctcggaac ctctgtggtg 6360
tccagatcag cgcagcagtt ccgaggtagt tttgaggccc ttagatgatg caatggtgtc 6420
agtcgctgga tcacgagtct taatggcagt attcgttctt atttgtgcca ttgagccccg 6480
ttatcctcgt atcttctacc ccccatccca tccctttgtt ggtgcaaccc tacccattta 6540
ttgttgggtg cagcccaacc gacgtggaga gcttggcttg gccatataaa aaggcccccc 6600
cctagtggca atggcagaaa gtcagctgtg agttgttgaa tttgtcatct aggcggcctg 6660
gccgtcttct ccggggcaat ttaaatgttc ctctatagta gatctgcgta cactgtttaa 6720
acgtcgacga gtatctgtct gactcgtcat tgccgccttt ggagtacgac tccaactatg 6780
agtgtgcttg gatcactttg acgatacatt cttcgttgga ggctgtgggt ctgacagctg 6840
cgttttcggc gcggttggcc gacaacaata tcagctgcaa cgtcattgct ggctttcatc 6900
atgatcacat ttttgtcggc aaaggcgacg cccagagagc cattgacgtt ctttctaatt 6960
tggaccgata gccgtatagt ccagtctatc tataagttca actaactcgt aactattacc 7020
ataacatata cttcactgcc ccagataagg ttccgataaa aagttctgca gactaaattt 7080
atttcagtct cctcttcacc accaaaatgc cctcctacga agctcgagct aacgtccaca 7140
agtccgcctt tgccgctcga gtgctcaagc tcgtggcagc caagaaaacc aacctgtgtg 7200
cttctctgga tgttaccacc accaaggagc tcattgagct tgccgataag gtcggacctt 7260
atgtgtgcat gatcaaaacc catatcgaca tcattgacga cttcacctac gccggcactg 7320
tgctcccccct caaggaactt gctcttaagc acggtttctt cctgttcgag gacagaaagt 7380
tcgcagatat tggcaacact gtcaagcacc agtaccggtg tcaccgaatc gccgagtggt 7440
ccgatatcac caacgcccac ggtgtacccg gaaccggaat cattgctggc ctgcgagctg 7500
gtgccgagga aactgtctct gaacagaaga aggaggacgt ctctgactac gagaactccc 7560
agtacaagga gttcctagtc ccctctccca acgagaagct ggccagaggt ctgctcatgc 7620
tggccgagct gtcttgcaag ggctctctgg ccactggcga gtactccaag cagaccattg 7680
agcttgcccg atccgacccc gagtttgtgg ttggcttcat tgcccagaac cgacctaagg 7740
gcgactctga ggactggctt attctgaccc ccggggtggg tcttgacgac aagggagacg 7800
ctctcggaca gcagtaccga actgttgagg atgtcatgtc taccggaacg gatatcataa 7860
ttgtcggccg aggtctgtac ggccagaacc gagatcctat tgaggaggcc aagcgatacc 7920

```
agaaggctgg ctgggaggct taccagaaga ttaactgtta gaggttagac tatggatatg     7980
taatttaact gtgtatatag agagcgtgca agtatggagc gcttgttcag cttgtatgat     8040
ggtcagacga cctgtctgat cgagtatgta tgatactgca caacctgtgt atccgcatga     8100
tctgtccaat ggggcatgtt gttgtgtttc tcgatacgga gatgctgggt acagtgctaa     8160
tacgttgaac tacttatact tatatgaggc tcgaagaaag ctgacttgtg tatgacttaa     8220
ttaattactg tcgaaat                                                    8237
```

<210> 90
<211> 1350
<212> DNA
<213> Euglena gracilis

<220>
<221> CDS
<222> (1)..(1350)
<223> mutant delta-5 desaturase

<400> 90

```
atg gct ctc agt ctt acc aca gaa cag ctg tta gaa cgc cct gat ttg        48
Met Ala Leu Ser Leu Thr Thr Glu Gln Leu Leu Glu Arg Pro Asp Leu
1               5                   10                  15

gtt gcg att gat ggc atc ctc tac gac ctt gaa ggg ctt gcc aaa gtt        96
Val Ala Ile Asp Gly Ile Leu Tyr Asp Leu Glu Gly Leu Ala Lys Val
            20                  25                  30

cat cca gga tcc gat ttg att ctc gct tct ggt gcc tct gat gcc tcc       144
His Pro Gly Ser Asp Leu Ile Leu Ala Ser Gly Ala Ser Asp Ala Ser
        35                  40                  45

cct ctc ttt tat tca atg cat cca tac gtc aaa ccg gag aac tcc aaa       192
Pro Leu Phe Tyr Ser Met His Pro Tyr Val Lys Pro Glu Asn Ser Lys
    50                  55                  60

ttg ctt caa cag ttc gtc cga ggg aag cat gac cgc acc tcg aag gac       240
Leu Leu Gln Gln Phe Val Arg Gly Lys His Asp Arg Thr Ser Lys Asp
65                  70                  75                  80

att gtc tac acg tat gat tct ccc ttc gca caa gac gtt aag cgg aca       288
Ile Val Tyr Thr Tyr Asp Ser Pro Phe Ala Gln Asp Val Lys Arg Thr
                85                  90                  95

atg cgc gag gtg atg aaa ggg agg aac tgg tac gca acc cct ggc ttc       336
Met Arg Glu Val Met Lys Gly Arg Asn Trp Tyr Ala Thr Pro Gly Phe
            100                 105                 110

tgg ctg cgc acc gtt ggg atc atc gcc gtg acg gcc ttt tgc gag tgg       384
Trp Leu Arg Thr Val Gly Ile Ile Ala Val Thr Ala Phe Cys Glu Trp
        115                 120                 125

cac tgg gct acc acg ggg atg gtg ctg tgg ggc ctg ttg act gga ttc       432
His Trp Ala Thr Thr Gly Met Val Leu Trp Gly Leu Leu Thr Gly Phe
    130                 135                 140

atg cac atg cag atc ggc tta tcc atc cag cat gat gcg tcc cac ggg       480
Met His Met Gln Ile Gly Leu Ser Ile Gln His Asp Ala Ser His Gly
145                 150                 155                 160
```

```
gcc atc agc aag aag cct tgg gtc aac gcc ctc ttc gcc tac ggc att        528
Ala Ile Ser Lys Lys Pro Trp Val Asn Ala Leu Phe Ala Tyr Gly Ile
                165                 170                 175

gac gtc atc gga tcg tcc cgg tgg att tgg ctg cag tcg cac atc atg        576
Asp Val Ile Gly Ser Ser Arg Trp Ile Trp Leu Gln Ser His Ile Met
            180                 185                 190

cgg cac cac acc tac acc aac cag cac ggc ctc gac ctg gat gcg gag        624
Arg His His Thr Tyr Thr Asn Gln His Gly Leu Asp Leu Asp Ala Glu
        195                 200                 205

tcg gca gag ccg ttc ctg gtg ttc cac aac tac ccc gcc gca aac acc        672
Ser Ala Glu Pro Phe Leu Val Phe His Asn Tyr Pro Ala Ala Asn Thr
    210                 215                 220

gcc cga aag tgg ttc cac cgc ttc cag gct tgg tac atg tac ctt gtg        720
Ala Arg Lys Trp Phe His Arg Phe Gln Ala Trp Tyr Met Tyr Leu Val
225                 230                 235                 240

ctg ggg gca tac ggg gta tcg ctg gtg tac aac ccg ctc tac att ttc        768
Leu Gly Ala Tyr Gly Val Ser Leu Val Tyr Asn Pro Leu Tyr Ile Phe
                245                 250                 255

cgg atg cag cac aat gac acc atc cca gag tct gtc acg gcc atg cgg        816
Arg Met Gln His Asn Asp Thr Ile Pro Glu Ser Val Thr Ala Met Arg
            260                 265                 270

gaa aat ggc ttt ctg cgg cgc tac cgc aca ctt gca ttc gtg atg cga        864
Glu Asn Gly Phe Leu Arg Arg Tyr Arg Thr Leu Ala Phe Val Met Arg
        275                 280                 285

gct ttc ttc atc ttc cgg acc gca ttc ttg ccc tgg tac ctc act ggg        912
Ala Phe Phe Ile Phe Arg Thr Ala Phe Leu Pro Trp Tyr Leu Thr Gly
    290                 295                 300

acc tca ttg ctg atc acc att cct ctg gtg ccc acc gca act ggt gcc        960
Thr Ser Leu Leu Ile Thr Ile Pro Leu Val Pro Thr Ala Thr Gly Ala
305                 310                 315                 320

ttc ttg acg ttc ttc ttc att ttg tcc cac aat ttt gat ggc tcc gaa       1008
Phe Leu Thr Phe Phe Phe Ile Leu Ser His Asn Phe Asp Gly Ser Glu
                325                 330                 335

cgg atc ccc gac aag aac tgc aag gtt aag cga tct gag aag gac gtt       1056
Arg Ile Pro Asp Lys Asn Cys Lys Val Lys Arg Ser Glu Lys Asp Val
            340                 345                 350

gag gct gac caa att gac tgg tat cgg gcg cag gtg gag acg tcc tcc       1104
Glu Ala Asp Gln Ile Asp Trp Tyr Arg Ala Gln Val Glu Thr Ser Ser
        355                 360                 365

aca tac ggt ggc ccc atc gcc atg ttc ttc act ggc ggt ctc aat ttc       1152
Thr Tyr Gly Gly Pro Ile Ala Met Phe Phe Thr Gly Gly Leu Asn Phe
    370                 375                 380

cag atc gag cac cac ctc ttt ccc cgg atg tcg tct tgg cac tac ccc       1200
Gln Ile Glu His His Leu Phe Pro Arg Met Ser Ser Trp His Tyr Pro
385                 390                 395                 400

ttc gtc cag cag gcg gtc cgg gag tgt tgc gaa cga cat gga gtg cga       1248
Phe Val Gln Gln Ala Val Arg Glu Cys Cys Glu Arg His Gly Val Arg
                405                 410                 415
```

```
tat gtt ttc tac cct acc atc gtc ggc aac atc atc tcc acc ctg aag       1296
Tyr Val Phe Tyr Pro Thr Ile Val Gly Asn Ile Ile Ser Thr Leu Lys
            420                 425                 430

tac atg cat aag gtg ggt gtc gtc cac tgc gtg aag gac gca cag gat       1344
Tyr Met His Lys Val Gly Val Val His Cys Val Lys Asp Ala Gln Asp
        435                 440                 445

tcc taa                                                               1350
Ser
```

<210> 91
<211> 449
<212> PRT
<213> Euglena gracilis

<400> 91

Met Ala Leu Ser Leu Thr Thr Glu Gln Leu Leu Glu Arg Pro Asp Leu
1               5                   10                  15

Val Ala Ile Asp Gly Ile Leu Tyr Asp Leu Glu Gly Leu Ala Lys Val
            20                  25                  30

His Pro Gly Ser Asp Leu Ile Leu Ala Ser Gly Ala Ser Asp Ala Ser
        35                  40                  45

Pro Leu Phe Tyr Ser Met His Pro Tyr Val Lys Pro Glu Asn Ser Lys
    50                  55                  60

Leu Leu Gln Gln Phe Val Arg Gly Lys His Asp Arg Thr Ser Lys Asp
65                  70                  75                  80

Ile Val Tyr Thr Tyr Asp Ser Pro Phe Ala Gln Asp Val Lys Arg Thr
                85                  90                  95

Met Arg Glu Val Met Lys Gly Arg Asn Trp Tyr Ala Thr Pro Gly Phe
            100                 105                 110

Trp Leu Arg Thr Val Gly Ile Ile Ala Val Thr Ala Phe Cys Glu Trp
        115                 120                 125

His Trp Ala Thr Thr Gly Met Val Leu Trp Gly Leu Leu Thr Gly Phe
    130                 135                 140

Met His Met Gln Ile Gly Leu Ser Ile Gln His Asp Ala Ser His Gly
145                 150                 155                 160

Ala Ile Ser Lys Lys Pro Trp Val Asn Ala Leu Phe Ala Tyr Gly Ile
                165                 170                 175

Asp Val Ile Gly Ser Ser Arg Trp Ile Trp Leu Gln Ser His Ile Met

180 185 190

Arg His His Thr Tyr Thr Asn Gln His Gly Leu Asp Leu Asp Ala Glu
195 200 205

Ser Ala Glu Pro Phe Leu Val Phe His Asn Tyr Pro Ala Ala Asn Thr
210 215 220

Ala Arg Lys Trp Phe His Arg Phe Gln Ala Trp Tyr Met Tyr Leu Val
225 230 235 240

Leu Gly Ala Tyr Gly Val Ser Leu Val Tyr Asn Pro Leu Tyr Ile Phe
245 250 255

Arg Met Gln His Asn Asp Thr Ile Pro Glu Ser Val Thr Ala Met Arg
260 265 270

Glu Asn Gly Phe Leu Arg Arg Tyr Arg Thr Leu Ala Phe Val Met Arg
275 280 285

Ala Phe Phe Ile Phe Arg Thr Ala Phe Leu Pro Trp Tyr Leu Thr Gly
290 295 300

Thr Ser Leu Leu Ile Thr Ile Pro Leu Val Pro Thr Ala Thr Gly Ala
305 310 315 320

Phe Leu Thr Phe Phe Phe Ile Leu Ser His Asn Phe Asp Gly Ser Glu
325 330 335

Arg Ile Pro Asp Lys Asn Cys Lys Val Lys Arg Ser Glu Lys Asp Val
340 345 350

Glu Ala Asp Gln Ile Asp Trp Tyr Arg Ala Gln Val Glu Thr Ser Ser
355 360 365

Thr Tyr Gly Gly Pro Ile Ala Met Phe Phe Thr Gly Gly Leu Asn Phe
370 375 380

Gln Ile Glu His His Leu Phe Pro Arg Met Ser Ser Trp His Tyr Pro
385 390 395 400

Phe Val Gln Gln Ala Val Arg Glu Cys Cys Glu Arg His Gly Val Arg
405 410 415

Tyr Val Phe Tyr Pro Thr Ile Val Gly Asn Ile Ile Ser Thr Leu Lys
420 425 430

Tyr Met His Lys Val Gly Val Val His Cys Val Lys Asp Ala Gln Asp
435 440 445

Ser

<210> 92
<211> 1254
<212> DNA
<213> Mortierella alpina

<220>
<221> CDS
<222> (1)..(1254)
<223> GenBank Accession No. CQ891250

<300>
<302> Novel plant acyltransferases specific for long-chained, multiply unsaturated fatty acids
<310> WO 2004/087902
<311> 2004-03-26
<312> 2004-10-14
<313> (1)..(1254)

<400> 92

```
atg gat gaa tcc acc acg acc acc acg cac cac tca gag acc agc agc        48
Met Asp Glu Ser Thr Thr Thr Thr Thr His His Ser Glu Thr Ser Ser
1               5                   10                  15

aag acg tcc tcg cac ccc cgc cgg ctc ggt ccc gag atg aac cct atc        96
Lys Thr Ser Ser His Pro Arg Arg Leu Gly Pro Glu Met Asn Pro Ile
            20                  25                  30

tac aag ggt ctg cga gcc att gtc tgg gcc ttt tac ttc aac ctg gga       144
Tyr Lys Gly Leu Arg Ala Ile Val Trp Ala Phe Tyr Phe Asn Leu Gly
        35                  40                  45

gcg tcg ctt ata tcg atc acg cag gtg ctg tcg ctg cct ctg gcg ttg       192
Ala Ser Leu Ile Ser Ile Thr Gln Val Leu Ser Leu Pro Leu Ala Leu
    50                  55                  60

att gct cca ggg gtc tac cag tgg cac atc agc aaa aca cag ggt cac       240
Ile Ala Pro Gly Val Tyr Gln Trp His Ile Ser Lys Thr Gln Gly His
65                  70                  75                  80

ttt gga gct ttc ctg ctc cgg atg aac cag ctc ttt gcg ccg tca gat       288
Phe Gly Ala Phe Leu Leu Arg Met Asn Gln Leu Phe Ala Pro Ser Asp
                85                  90                  95

att gtc ttg aca ggg gac gag agt gtc agg gga atc gtc aag gtc tac       336
Ile Val Leu Thr Gly Asp Glu Ser Val Arg Gly Ile Val Lys Val Tyr
            100                 105                 110

aaa gga cgg aac ctg aag gag gcc ggt gag cca ggc agc ggt cag gga       384
Lys Gly Arg Asn Leu Lys Glu Ala Gly Glu Pro Gly Ser Gly Gln Gly
        115                 120                 125

gag gac att ctt ctg gat atg ccc gag agg atg gtt ttc att gcg aac       432
Glu Asp Ile Leu Leu Asp Met Pro Glu Arg Met Val Phe Ile Ala Asn
    130                 135                 140

cac cag atc tac tct gac tgg atg tac ctc tgg tgc ttc tcc tat ttt       480
His Gln Ile Tyr Ser Asp Trp Met Tyr Leu Trp Cys Phe Ser Tyr Phe
145                 150                 155                 160
```

```
gca gag agg cac agg gca ctg aag att att ctt cgg ggc gac ctg acc         528
Ala Glu Arg His Arg Ala Leu Lys Ile Ile Leu Arg Gly Asp Leu Thr
                165                 170                 175

tgg atc cct gtc ttt ggc tgg ggt atg cgg ttc ttt gac ttt atc ttt         576
Trp Ile Pro Val Phe Gly Trp Gly Met Arg Phe Phe Asp Phe Ile Phe
            180                 185                 190

ttg aaa cgt aat gac tgg gca cac gat cgc cgt gcc att gag gaa aac         624
Leu Lys Arg Asn Asp Trp Ala His Asp Arg Arg Ala Ile Glu Glu Asn
        195                 200                 205

ttg gga cgt gtc aag gaa aag gat ccc ctc tgg ctc gtg gtc ttc ccc         672
Leu Gly Arg Val Lys Glu Lys Asp Pro Leu Trp Leu Val Val Phe Pro
    210                 215                 220

gag gga aca gtc gtc tcc aag gaa acg cgt ctc cga tcc gtt gcc ttt         720
Glu Gly Thr Val Val Ser Lys Glu Thr Arg Leu Arg Ser Val Ala Phe
225                 230                 235                 240

tca aag aag gct agt ctg tcg gat cac cgc cat gtg ctg ctt cca agg         768
Ser Lys Lys Ala Ser Leu Ser Asp His Arg His Val Leu Leu Pro Arg
                245                 250                 255

acc agc ggt ctg ttt gtg tgc atc aac aag ttg cgt gga tct gtc gac         816
Thr Ser Gly Leu Phe Val Cys Ile Asn Lys Leu Arg Gly Ser Val Asp
            260                 265                 270

tac ttg tac gat gca acc gtt ggc tac tcg aat gtc gag tat ggc gag         864
Tyr Leu Tyr Asp Ala Thr Val Gly Tyr Ser Asn Val Glu Tyr Gly Glu
        275                 280                 285

att ccg cag gag ctt tac ccg tta cca gga ctg tat atc aac aaa gca         912
Ile Pro Gln Glu Leu Tyr Pro Leu Pro Gly Leu Tyr Ile Asn Lys Ala
    290                 295                 300

cag ccc aag gag atc aac atg cac ctg cgt cga ttt gcg atc aag gat         960
Gln Pro Lys Glu Ile Asn Met His Leu Arg Arg Phe Ala Ile Lys Asp
305                 310                 315                 320

atc ccc acg tca gaa ccc gaa ttt gtg gaa tgg gtc cga gct cgg tgg        1008
Ile Pro Thr Ser Glu Pro Glu Phe Val Glu Trp Val Arg Ala Arg Trp
                325                 330                 335

gtg gag aag gat gag ttg atg gaa gag ttt tat acc aag ggc cga ttt        1056
Val Glu Lys Asp Glu Leu Met Glu Glu Phe Tyr Thr Lys Gly Arg Phe
            340                 345                 350

cca tca caa ctg acg gcc gcc gac att ggt gag aag gag gtc aag acg        1104
Pro Ser Gln Leu Thr Ala Ala Asp Ile Gly Glu Lys Glu Val Lys Thr
        355                 360                 365

gca gga ggt cca acg gag gga cag agt gtc agg atc ccg ctc aag gcg        1152
Ala Gly Gly Pro Thr Glu Gly Gln Ser Val Arg Ile Pro Leu Lys Ala
    370                 375                 380

cga ggc atg atg gac tac ctc atg ccc tcg gtc atg aat ctg atc gcc        1200
Arg Gly Met Met Asp Tyr Leu Met Pro Ser Val Met Asn Leu Ile Ala
385                 390                 395                 400

ctt cct gtg ctg gcg ttt gcg atg aga tat gca gtg cag caa gca tcg        1248
Leu Pro Val Leu Ala Phe Ala Met Arg Tyr Ala Val Gln Gln Ala Ser
                405                 410                 415
```

```
ggc tga                                                            1254
Gly
```

<210> 93
<211> 417
<212> PRT
<213> Mortierella alpina

<400> 93

```
Met Asp Glu Ser Thr Thr Thr Thr Thr His His Ser Glu Thr Ser Ser
1               5                   10                  15

Lys Thr Ser Ser His Pro Arg Arg Leu Gly Pro Glu Met Asn Pro Ile
            20                  25                  30

Tyr Lys Gly Leu Arg Ala Ile Val Trp Ala Phe Tyr Phe Asn Leu Gly
        35                  40                  45

Ala Ser Leu Ile Ser Ile Thr Gln Val Leu Ser Leu Pro Leu Ala Leu
    50                  55                  60

Ile Ala Pro Gly Val Tyr Gln Trp His Ile Ser Lys Thr Gln Gly His
65                  70                  75                  80

Phe Gly Ala Phe Leu Leu Arg Met Asn Gln Leu Phe Ala Pro Ser Asp
                85                  90                  95

Ile Val Leu Thr Gly Asp Glu Ser Val Arg Gly Ile Val Lys Val Tyr
            100                 105                 110

Lys Gly Arg Asn Leu Lys Glu Ala Gly Glu Pro Gly Ser Gly Gln Gly
        115                 120                 125

Glu Asp Ile Leu Leu Asp Met Pro Glu Arg Met Val Phe Ile Ala Asn
    130                 135                 140

His Gln Ile Tyr Ser Asp Trp Met Tyr Leu Trp Cys Phe Ser Tyr Phe
145                 150                 155                 160

Ala Glu Arg His Arg Ala Leu Lys Ile Ile Leu Arg Gly Asp Leu Thr
                165                 170                 175

Trp Ile Pro Val Phe Gly Trp Gly Met Arg Phe Phe Asp Phe Ile Phe
            180                 185                 190

Leu Lys Arg Asn Asp Trp Ala His Asp Arg Arg Ala Ile Glu Glu Asn
        195                 200                 205

Leu Gly Arg Val Lys Glu Lys Asp Pro Leu Trp Leu Val Val Phe Pro
```

```
    210                 215                 220

Glu Gly Thr Val Val Ser Lys Glu Thr Arg Leu Arg Ser Val Ala Phe
225                 230                 235                 240

Ser Lys Lys Ala Ser Leu Ser Asp His Arg His Val Leu Leu Pro Arg
                245                 250                 255

Thr Ser Gly Leu Phe Val Cys Ile Asn Lys Leu Arg Gly Ser Val Asp
            260                 265                 270

Tyr Leu Tyr Asp Ala Thr Val Gly Tyr Ser Asn Val Glu Tyr Gly Glu
        275                 280                 285

Ile Pro Gln Glu Leu Tyr Pro Leu Pro Gly Leu Tyr Ile Asn Lys Ala
    290                 295                 300

Gln Pro Lys Glu Ile Asn Met His Leu Arg Arg Phe Ala Ile Lys Asp
305                 310                 315                 320

Ile Pro Thr Ser Glu Pro Glu Phe Val Glu Trp Val Arg Ala Arg Trp
                325                 330                 335

Val Glu Lys Asp Glu Leu Met Glu Glu Phe Tyr Thr Lys Gly Arg Phe
            340                 345                 350

Pro Ser Gln Leu Thr Ala Ala Asp Ile Gly Glu Lys Glu Val Lys Thr
        355                 360                 365

Ala Gly Gly Pro Thr Glu Gly Gln Ser Val Arg Ile Pro Leu Lys Ala
    370                 375                 380

Arg Gly Met Met Asp Tyr Leu Met Pro Ser Val Met Asn Leu Ile Ala
385                 390                 395                 400

Leu Pro Val Leu Ala Phe Ala Met Arg Tyr Ala Val Gln Gln Ala Ser
                405                 410                 415

Gly
```

<210> 94
<211> 1170
<212> DNA
<213> Mortierella alpina

<220>
<221> CDS
<222> (1)..(1170)

<223> Gen Bank Accession No. CQ891252

<300>
<302> Novel plant acyltransferases specific for long-chained, multiply unsaturated fatty acids
<310> WO 2004/087902
<311> 2004-03-26
<312> 2004-10-14
<313> (1)..(1170)

<400> 94

```
atg aac cct atc tac aag ggt ctg cga gcc att gtc tgg gcc ttt tac      48
Met Asn Pro Ile Tyr Lys Gly Leu Arg Ala Ile Val Trp Ala Phe Tyr
1               5                   10                  15

ttc aac ctg gga gcg tcg ctt ata tcg atc acg cag gtg ctg tcg ctg      96
Phe Asn Leu Gly Ala Ser Leu Ile Ser Ile Thr Gln Val Leu Ser Leu
            20                  25                  30

cct ctg gcg ttg att gct cca ggg gtc tac cag tgg cac atc agc aaa      144
Pro Leu Ala Leu Ile Ala Pro Gly Val Tyr Gln Trp His Ile Ser Lys
        35                  40                  45

aca cag ggt cac ttt gga gct ttc ctg ctc cgg atg aac cag ctc ttt      192
Thr Gln Gly His Phe Gly Ala Phe Leu Leu Arg Met Asn Gln Leu Phe
    50                  55                  60

gcg ccg tca gat att gtc ttg aca ggg gac gag agt gtc agg gga atc      240
Ala Pro Ser Asp Ile Val Leu Thr Gly Asp Glu Ser Val Arg Gly Ile
65                  70                  75                  80

gtc aag gtc tac aaa gga cgg aac ctg aag gag gcc ggt gag cca ggc      288
Val Lys Val Tyr Lys Gly Arg Asn Leu Lys Glu Ala Gly Glu Pro Gly
                85                  90                  95

agc ggt cag gga gag gac att ctt ctg gat atg ccc gag agg atg gtt      336
Ser Gly Gln Gly Glu Asp Ile Leu Leu Asp Met Pro Glu Arg Met Val
            100                 105                 110

ttc att gcg aac cac cag atc tac tct gac tgg atg tac ctc tgg tgc      384
Phe Ile Ala Asn His Gln Ile Tyr Ser Asp Trp Met Tyr Leu Trp Cys
        115                 120                 125

ttc tcc tat ttt gca gag agg cac agg gca ctg aag att att ctt cgg      432
Phe Ser Tyr Phe Ala Glu Arg His Arg Ala Leu Lys Ile Ile Leu Arg
    130                 135                 140

ggc gac ctg acc tgg atc cct gtc ttt ggc tgg ggt atg cgg ttc ttt      480
Gly Asp Leu Thr Trp Ile Pro Val Phe Gly Trp Gly Met Arg Phe Phe
145                 150                 155                 160

gac ttt atc ttt ttg aaa cgt aat gac tgg gca cac gat cgc cgt gcc      528
Asp Phe Ile Phe Leu Lys Arg Asn Asp Trp Ala His Asp Arg Arg Ala
                165                 170                 175

att gag gaa aac ttg gga cgt gtc aag gaa aag gat ccc ctc tgg ctc      576
Ile Glu Glu Asn Leu Gly Arg Val Lys Glu Lys Asp Pro Leu Trp Leu
            180                 185                 190

gtg gtc ttc ccc gag gga aca gtc gtc tcc aag gaa acg cgt ctc cga      624
Val Val Phe Pro Glu Gly Thr Val Val Ser Lys Glu Thr Arg Leu Arg
        195                 200                 205

tcc gtt gcc ttt tca aag aag gct agt ctg tcg gat cac cgc cat gtg      672
Ser Val Ala Phe Ser Lys Lys Ala Ser Leu Ser Asp His Arg His Val
    210                 215                 220
```

```
ctg ctt cca agg acc agc ggt ctg ttt gtg tgc atc aac aag ttg cgt        720
Leu Leu Pro Arg Thr Ser Gly Leu Phe Val Cys Ile Asn Lys Leu Arg
225                 230                 235                 240

gga tct gtc gac tac ttg tac gat gca acc gtt ggc tac tcg aat gtc        768
Gly Ser Val Asp Tyr Leu Tyr Asp Ala Thr Val Gly Tyr Ser Asn Val
                245                 250                 255

gag tat ggc gag att ccg cag gag ctt tac ccg tta cca gga ctg tat        816
Glu Tyr Gly Glu Ile Pro Gln Glu Leu Tyr Pro Leu Pro Gly Leu Tyr
            260                 265                 270

atc aac aaa gca cag ccc aag gag atc aac atg cac ctg cgt cga ttt        864
Ile Asn Lys Ala Gln Pro Lys Glu Ile Asn Met His Leu Arg Arg Phe
        275                 280                 285

gcg atc aag gat atc ccc acg tca gaa ccc gaa ttt gtg gaa tgg gtc        912
Ala Ile Lys Asp Ile Pro Thr Ser Glu Pro Glu Phe Val Glu Trp Val
    290                 295                 300

cga gct cgg tgg gtg gag aag gat gag ttg atg gaa gag ttt tat acc        960
Arg Ala Arg Trp Val Glu Lys Asp Glu Leu Met Glu Glu Phe Tyr Thr
305                 310                 315                 320

aag ggc cga ttt cca tca caa ctg acg gcc gcc gac att ggt gag aag       1008
Lys Gly Arg Phe Pro Ser Gln Leu Thr Ala Ala Asp Ile Gly Glu Lys
                325                 330                 335

gag gtc aag acg gca gga ggt cca acg gag gga cag agt gtc agg atc       1056
Glu Val Lys Thr Ala Gly Gly Pro Thr Glu Gly Gln Ser Val Arg Ile
            340                 345                 350

ccg ctc aag gcg cga ggc atg atg gac tac ctc atg ccc tcg gtc atg       1104
Pro Leu Lys Ala Arg Gly Met Met Asp Tyr Leu Met Pro Ser Val Met
        355                 360                 365

aat ctg atc gcc ctt cct gtg ctg gcg ttt gcg atg aga tat gca gtg       1152
Asn Leu Ile Ala Leu Pro Val Leu Ala Phe Ala Met Arg Tyr Ala Val
    370                 375                 380

cag caa gca tcg ggc tga                                               1170
Gln Gln Ala Ser Gly
385
```

<210> 95
<211> 389
<212> PRT
<213> Mortierella alpina

<400> 95

```
Met Asn Pro Ile Tyr Lys Gly Leu Arg Ala Ile Val Trp Ala Phe Tyr
1               5                   10                  15

Phe Asn Leu Gly Ala Ser Leu Ile Ser Ile Thr Gln Val Leu Ser Leu
            20                  25                  30

Pro Leu Ala Leu Ile Ala Pro Gly Val Tyr Gln Trp His Ile Ser Lys
        35                  40                  45
```

Thr Gln Gly His Phe Gly Ala Phe Leu Leu Arg Met Asn Gln Leu Phe
50 55 60

Ala Pro Ser Asp Ile Val Leu Thr Gly Asp Glu Ser Val Arg Gly Ile
65 70 75 80

Val Lys Val Tyr Lys Gly Arg Asn Leu Lys Glu Ala Gly Glu Pro Gly
85 90 95

Ser Gly Gln Gly Glu Asp Ile Leu Leu Asp Met Pro Glu Arg Met Val
100 105 110

Phe Ile Ala Asn His Gln Ile Tyr Ser Asp Trp Met Tyr Leu Trp Cys
115 120 125

Phe Ser Tyr Phe Ala Glu Arg His Arg Ala Leu Lys Ile Ile Leu Arg
130 135 140

Gly Asp Leu Thr Trp Ile Pro Val Phe Gly Trp Gly Met Arg Phe Phe
145 150 155 160

Asp Phe Ile Phe Leu Lys Arg Asn Asp Trp Ala His Asp Arg Arg Ala
165 170 175

Ile Glu Glu Asn Leu Gly Arg Val Lys Glu Lys Asp Pro Leu Trp Leu
180 185 190

Val Val Phe Pro Glu Gly Thr Val Val Ser Lys Glu Thr Arg Leu Arg
195 200 205

Ser Val Ala Phe Ser Lys Lys Ala Ser Leu Ser Asp His Arg His Val
210 215 220

Leu Leu Pro Arg Thr Ser Gly Leu Phe Val Cys Ile Asn Lys Leu Arg
225 230 235 240

Gly Ser Val Asp Tyr Leu Tyr Asp Ala Thr Val Gly Tyr Ser Asn Val
245 250 255

Glu Tyr Gly Glu Ile Pro Gln Glu Leu Tyr Pro Leu Pro Gly Leu Tyr
260 265 270

Ile Asn Lys Ala Gln Pro Lys Glu Ile Asn Met His Leu Arg Arg Phe
275 280 285

Ala Ile Lys Asp Ile Pro Thr Ser Glu Pro Glu Phe Val Glu Trp Val
290 295 300

Arg Ala Arg Trp Val Glu Lys Asp Glu Leu Met Glu Glu Phe Tyr Thr

```
305                 310                 315                 320

Lys Gly Arg Phe Pro Ser Gln Leu Thr Ala Ala Asp Ile Gly Glu Lys
                325                 330                 335

Glu Val Lys Thr Ala Gly Gly Pro Thr Glu Gly Gln Ser Val Arg Ile
            340                 345                 350

Pro Leu Lys Ala Arg Gly Met Met Asp Tyr Leu Met Pro Ser Val Met
        355                 360                 365

Asn Leu Ile Ala Leu Pro Val Leu Ala Phe Ala Met Arg Tyr Ala Val
    370                 375                 380

Gln Gln Ala Ser Gly
385
```

<210> 96
<211> 926
<212> DNA
<213> Saccharomyces cerevisiae

<220>
<221> CDS
<222> (4)..(915)

<400> 96

```
aac atg tct gtt att gga cga ttt ctt tac tac ctg aga tcg gtg ctc          48
    Met Ser Val Ile Gly Arg Phe Leu Tyr Tyr Leu Arg Ser Val Leu
    1               5                   10                  15

gtc gtt ttg gcc ctc gct gga tgt ggc ttc tat ggc gtg att gcc tct          96
Val Val Leu Ala Leu Ala Gly Cys Gly Phe Tyr Gly Val Ile Ala Ser
                20                  25                  30

atc ctg tgt act ctc atc ggc aag cag cat ctc gcg caa tgg att acc         144
Ile Leu Cys Thr Leu Ile Gly Lys Gln His Leu Ala Gln Trp Ile Thr
            35                  40                  45

gcc cga tgc ttt tac cac gtg atg aaa ctg atg ctg gga ttg gac gtc         192
Ala Arg Cys Phe Tyr His Val Met Lys Leu Met Leu Gly Leu Asp Val
        50                  55                  60

aaa gtc gtg ggt gaa gag aac ctg gct aag aag ccc tat atc atg atc         240
Lys Val Val Gly Glu Glu Asn Leu Ala Lys Lys Pro Tyr Ile Met Ile
    65                  70                  75

gct aac cac cag tcc acc ctc gat atc ttt atg ctc ggc aga atc ttc         288
Ala Asn His Gln Ser Thr Leu Asp Ile Phe Met Leu Gly Arg Ile Phe
80                  85                  90                  95

cct ccc gga tgc acc gtt acc gca aag aag agc ctt aag tac gtc ccc         336
Pro Pro Gly Cys Thr Val Thr Ala Lys Lys Ser Leu Lys Tyr Val Pro
                100                 105                 110

ttc ctg ggc tgg ttt atg gcg ctt tcc ggt aca tac ttc ctc gac cgt         384
Phe Leu Gly Trp Phe Met Ala Leu Ser Gly Thr Tyr Phe Leu Asp Arg
            115                 120                 125
```

```
tcc aag cgg caa gag gct att gat acc ctg aac aaa gga ctg gag aac        432
Ser Lys Arg Gln Glu Ala Ile Asp Thr Leu Asn Lys Gly Leu Glu Asn
        130                 135                 140

gtc aaa aag aat aag cga gcc ctc tgg gtt ttt ccc gaa ggt acc cgg        480
Val Lys Lys Asn Lys Arg Ala Leu Trp Val Phe Pro Glu Gly Thr Arg
    145                 150                 155

tct tac acg tcg gag ctt acc atg ctg ccg ttc aag aag ggc gcc ttt        528
Ser Tyr Thr Ser Glu Leu Thr Met Leu Pro Phe Lys Lys Gly Ala Phe
160                 165                 170                 175

cat ttg gcc cag cag gga aag atc cct atc gtc cca gtg gtt gtg tct        576
His Leu Ala Gln Gln Gly Lys Ile Pro Ile Val Pro Val Val Val Ser
                180                 185                 190

aac act agc acg ctc gtc agc cct aag tac ggt gtg ttc aac cga ggc        624
Asn Thr Ser Thr Leu Val Ser Pro Lys Tyr Gly Val Phe Asn Arg Gly
            195                 200                 205

tgt atg att gtc cga att ctg aag ccc atc tcg act gag aac ctg aca        672
Cys Met Ile Val Arg Ile Leu Lys Pro Ile Ser Thr Glu Asn Leu Thr
        210                 215                 220

aaa gat aag att ggc gag ttc gct gag aaa gtg cga gat cag atg gtt        720
Lys Asp Lys Ile Gly Glu Phe Ala Glu Lys Val Arg Asp Gln Met Val
    225                 230                 235

gac aca ctg aag gaa atc ggt tat tcc ccc gca atc aac gac acc acc        768
Asp Thr Leu Lys Glu Ile Gly Tyr Ser Pro Ala Ile Asn Asp Thr Thr
240                 245                 250                 255

ctt cct ccg cag gca att gag tac gcc gca ttg cag cat gac aag aag        816
Leu Pro Pro Gln Ala Ile Glu Tyr Ala Ala Leu Gln His Asp Lys Lys
                260                 265                 270

gtg aac aaa aag att aag aac gaa cct gtt cca tcg gtg tcc att tct        864
Val Asn Lys Lys Ile Lys Asn Glu Pro Val Pro Ser Val Ser Ile Ser
            275                 280                 285

aat gat gtg aat act cac aac gaa gga tcg tct gtc aag aag atg cac        912
Asn Asp Val Asn Thr His Asn Glu Gly Ser Ser Val Lys Lys Met His
        290                 295                 300

tag gcggccgcat g                                                       926
```

<210> 97
<211> 303
<212> PRT
<213> Saccharomyces cerevisiae

<400> 97

```
Met Ser Val Ile Gly Arg Phe Leu Tyr Tyr Leu Arg Ser Val Leu Val
1               5                   10                  15

Val Leu Ala Leu Ala Gly Cys Gly Phe Tyr Gly Val Ile Ala Ser Ile
            20                  25                  30

Leu Cys Thr Leu Ile Gly Lys Gln His Leu Ala Gln Trp Ile Thr Ala
        35                  40                  45
```

```
Arg Cys Phe Tyr His Val Met Lys Leu Met Leu Gly Leu Asp Val Lys
    50                  55                  60

Val Val Gly Glu Glu Asn Leu Ala Lys Lys Pro Tyr Ile Met Ile Ala
65                  70                  75                  80

Asn His Gln Ser Thr Leu Asp Ile Phe Met Leu Gly Arg Ile Phe Pro
                85                  90                  95

Pro Gly Cys Thr Val Thr Ala Lys Lys Ser Leu Lys Tyr Val Pro Phe
            100                 105                 110

Leu Gly Trp Phe Met Ala Leu Ser Gly Thr Tyr Phe Leu Asp Arg Ser
        115                 120                 125

Lys Arg Gln Glu Ala Ile Asp Thr Leu Asn Lys Gly Leu Glu Asn Val
    130                 135                 140

Lys Lys Asn Lys Arg Ala Leu Trp Val Phe Pro Glu Gly Thr Arg Ser
145                 150                 155                 160

Tyr Thr Ser Glu Leu Thr Met Leu Pro Phe Lys Lys Gly Ala Phe His
                165                 170                 175

Leu Ala Gln Gln Gly Lys Ile Pro Ile Val Pro Val Val Val Ser Asn
            180                 185                 190

Thr Ser Thr Leu Val Ser Pro Lys Tyr Gly Val Phe Asn Arg Gly Cys
        195                 200                 205

Met Ile Val Arg Ile Leu Lys Pro Ile Ser Thr Glu Asn Leu Thr Lys
    210                 215                 220

Asp Lys Ile Gly Glu Phe Ala Glu Lys Val Arg Asp Gln Met Val Asp
225                 230                 235                 240

Thr Leu Lys Glu Ile Gly Tyr Ser Pro Ala Ile Asn Asp Thr Thr Leu
                245                 250                 255

Pro Pro Gln Ala Ile Glu Tyr Ala Ala Leu Gln His Asp Lys Lys Val
            260                 265                 270

Asn Lys Lys Ile Lys Asn Glu Pro Val Pro Ser Val Ser Ile Ser Asn
        275                 280                 285

Asp Val Asn Thr His Asn Glu Gly Ser Ser Val Lys Lys Met His
    290                 295                 300
```

<210> 98
<211> 32
<212> DNA
<213> Artificial Sequence

<220>
<223> Primer 869

<400> 98
actggcgcg ccactgccga gatccagtct ac 32

<210> 99
<211> 28
<212> DNA
<213> Artificial Sequence

<220>
<223> Primer 870

<400> 99
tcagcctagg agcatccgtt gatttccg 28

<210> 100
<211> 7891
<212> DNA
<213> Artificial Sequence

<220>
<223> Plasmid pY222

<400> 100

```
tcgaccgtac gatagttagt agacaacaat cgatagttgg agcaagggag aaatgtagag      60
tgtgaaagac tcactatggt ccgggcttat ctcgaccaat agccaaagtc tggagtttct     120
gagagaaaaa ggcaagatac gtatgtaaca aagcgacgca tggtacaata ataccggagg     180
catgtatcat agagagttag tggttcgatg atggcactgg tgcctggtat gactttatac     240
ggctgactac atatttgtcc tcagacatac aattacagtc aagcacttac ccttggacat     300
ctgtaggtac cccccggcca agacgatctc agcgtgtcgt atgtcggatt ggcgtagctc     360
cctcgctcgt caattggctc ccatctactt tcttctgctt ggctacaccc agcatgtctg     420
ctatggctcg ttttcgtgcc ttatctatcc tcccagtatt accaactcta aatgacatga     480
tgtgattggg tctacacttt catatcagag ataaggagta gcacagttgc ataaaaagcc     540
caactctaat cagcttcttc ctttcttgta attagtacaa aggtgattag cgaaatctgg     600
aagcttagtt ggccctaaaa aaatcaaaaa aagcaaaaaa cgaaaaacga aaaaccacag     660
ttttgagaac agggaggtaa cgaaggatcg tatatatata tatatatata tatacccacg     720
gatcccgaga ccggcctttg attcttccct acaaccaacc attctcacca ccctaattca     780
caaccatgtc tgttattgga cgatttcttt actacctgag atcggtgctc gtcgttttgg     840
ccctcgctgg atgtggcttc tatggcgtga ttgcctctat cctgtgtact ctcatcggca     900
agcagcatct cgcgcaatgg attaccgccc gatgctttta ccacgtgatg aaactgatgc     960
```

tgggattgga cgtcaaagtc gtgggtgaag agaacctggc taagaagccc tatatcatga 1020
tcgctaacca ccagtccacc ctcgatatct ttatgctcgg cagaatcttc cctcccggat 1080
gcaccgttac cgcaaagaag agccttaagt acgtcccctt cctgggctgg tttatggcgc 1140
tttccggtac atacttcctc gaccgttcca agcggcaaga ggctattgat accctgaaca 1200
aaggactgga gaacgtcaaa aagaataagc gagccctctg ggtttttccc gaaggtaccc 1260
ggtcttacac gtcggagctt accatgctgc cgttcaagaa gggcgccttt catttggccc 1320
agcagggaaa gatccctatc gtcccagtgg ttgtgtctaa cactagcacg ctcgtcagcc 1380
ctaagtacgg tgtgttcaac cgaggctgta tgattgtccg aattctgaag cccatctcga 1440
ctgagaacct gacaaaagat aagattggcg agttcgctga gaaagtgcga gatcagatgg 1500
ttgacacact gaaggaaatc ggttattccc ccgcaatcaa cgacaccacc cttcctccgc 1560
aggcaattga gtacgccgca ttgcagcatg acaagaaggt gaacaaaaag attaagaacg 1620
aacctgttcc atcggtgtcc atttctaatg atgtgaatac tcacaacgaa ggatcgtctg 1680
tcaagaagat gcactaggcg gccgcatgag aagataaata tataaataca ttgagatatt 1740
aaatgcgcta gattagagag cctcatactg ctcggagaga agccaagacg agtactcaaa 1800
ggggattaca ccatccatat ccacagacac aagctgggga aaggttctat atacactttc 1860
cggaataccg tagtttccga tgttatcaat gggggcagcc aggatttcag gcacttcggt 1920
gtctcggggt gaaatggcgt tcttggcctc catcaagtcg taccatgtct tcatttgcct 1980
gtcaaagtaa aacagaagca gatgaagaat gaacttgaag tgaaggaatt taaatgtaac 2040
gaaactgaaa tttgaccaga tattgtgtcc gcggtggagc tccagctttt gttcccttta 2100
gtgagggtta atttcgagct tggcgtaatc atggtcatag ctgtttcctg tgtgaaattg 2160
ttatccgctc acaagcttcc acacaacgta cgagccggaa gcataaagtg taaagcctgg 2220
ggtgcctaat gagtgagcta actcacatta attgcgttgc gctcactgcc cgctttccag 2280
tcgggaaacc tgtcgtgcca gctgcattaa tgaatcggcc aacgcgcggg gagaggcggt 2340
ttgcgtattg ggcgctcttc cgcttcctcg ctcactgact cgctgcgctc ggtcgttcgg 2400
ctgcggcgag cggtatcagc tcactcaaag gcggtaatac ggttatccac agaatcaggg 2460
gataacgcag gaaagaacat gtgagcaaaa ggccagcaaa aggccaggaa ccgtaaaaag 2520
gccgcgttgc tggcgttttt ccataggctc cgcccccctg acgagcatca caaaaatcga 2580
cgctcaagtc agaggtggcg aaacccgaca ggactataaa gataccaggc gtttccccct 2640
ggaagctccc tcgtgcgctc tcctgttccg accctgccgc ttaccggata cctgtccgcc 2700
tttctccctt cgggaagcgt ggcgctttct catagctcac gctgtaggta tctcagttcg 2760
gtgtaggtcg ttcgctccaa gctgggctgt gtgcacgaac cccccgttca gcccgaccgc 2820
tgcgccttat ccggtaacta tcgtcttgag tccaacccgg taagacacga cttatcgcca 2880

ctggcagcag ccactggtaa caggattagc agagcgaggt atgtaggcgg tgctacagag 2940
ttcttgaagt ggtggcctaa ctacggctac actagaagga cagtatttgg tatctgcgct 3000
ctgctgaagc cagttacctt cggaaaaaga gttggtagct cttgatccgg caaacaaacc 3060
accgctggta gcggtggttt ttttgtttgc aagcagcaga ttacgcgcag aaaaaaagga 3120
tctcaagaag atcctttgat cttttctacg gggtctgacg ctcagtggaa cgaaaactca 3180
cgttaaggga ttttggtcat gagattatca aaaaggatct tcacctagat ccttttaaat 3240
taaaaatgaa gttttaaatc aatctaaagt atatatgagt aaacttggtc tgacagttac 3300
caatgcttaa tcagtgaggc acctatctca gcgatctgtc tatttcgttc atccatagtt 3360
gcctgactcc ccgtcgtgta gataactacg atacgggagg gcttaccatc tggccccagt 3420
gctgcaatga taccgcgaga cccacgctca ccggctccag atttatcagc aataaaccag 3480
ccagccggaa gggccgagcg cagaagtggt cctgcaactt tatccgcctc catccagtct 3540
attaattgtt gccgggaagc tagagtaagt agttcgccag ttaatagttt gcgcaacgtt 3600
gttgccattg ctacaggcat cgtggtgtca cgctcgtcgt ttggtatggc ttcattcagc 3660
tccggttccc aacgatcaag gcgagttaca tgatccccca tgttgtgcaa aaaagcggtt 3720
agctccttcg gtcctccgat cgttgtcaga agtaagttgg ccgcagtgtt atcactcatg 3780
gttatggcag cactgcataa ttctcttact gtcatgccat ccgtaagatg cttttctgtg 3840
actggtgagt actcaaccaa gtcattctga gaatagtgta tgcggcgacc gagttgctct 3900
tgcccggcgt caatacggga taataccgcg ccacatagca gaactttaaa agtgctcatc 3960
attggaaaac gttcttcggg gcgaaaactc tcaaggatct taccgctgtt gagatccagt 4020
tcgatgtaac ccactcgtgc acccaactga tcttcagcat cttttacttt caccagcgtt 4080
tctgggtgag caaaaacagg aaggcaaaat gccgcaaaaa agggaataag ggcgacacgg 4140
aaatgttgaa tactcatact cttccttttt caatattatt gaagcattta tcagggttat 4200
tgtctcatga gcggatacat atttgaatgt atttagaaaa ataaacaaat aggggttccg 4260
cgcacatttc cccgaaaagt gccacctgac gcgccctgta gcggcgcatt aagcgcggcg 4320
ggtgtggtgg ttacgcgcag cgtgaccgct acacttgcca gcgccctagc gcccgctcct 4380
ttcgctttct tcccttcctt tctcgccacg ttcgccggct ttccccgtca agctctaaat 4440
cgggggctcc ctttagggtt ccgatttagt gctttacggc acctcgaccc caaaaaactt 4500
gattagggtg atggttcacg tagtgggcca tcgccctgat agacggtttt tcgccctttg 4560
acgttggagt ccacgttctt taatagtgga ctcttgttcc aaactggaac aacactcaac 4620
cctatctcgg tctattcttt tgatttataa gggattttgc cgatttcggc ctattggtta 4680
aaaaatgagc tgatttaaca aaaatttaac gcgaatttta acaaaatatt aacgcttaca 4740
atttccattc gccattcagg ctgcgcaact gttgggaagg gcgatcggtg cgggcctctt 4800
cgctattacg ccagctggcg aaagggggat gtgctgcaag gcgattaagt tgggtaacgc 4860

cagggttttc ccagtcacga cgttgtaaaa cgacggccag tgaattgtaa tacgactcac 4920

tatagggcga attgggtacc gggccccccc tcgaggtcga tggtgtcgat aagcttgata 4980

tcgaattcat gtcacacaaa ccgatcttcg cctcaaggaa acctaattct acatccgaga 5040

gactgccgag atccagtcta cactgattaa ttttcgggcc aataatttaa aaaaatcgtg 5100

ttatataata ttatatgtat tatatatata catcatgatg atactgacag tcatgtccca 5160

ttgctaaata gacagactcc atctgccgcc tccaactgat gttctcaata tttaaggggt 5220

catctcgcat tgtttaataa taaacagact ccatctaccg cctccaaatg atgttctcaa 5280

aatatattgt atgaacttat ttttattact tagtattatt agacaactta cttgctttat 5340

gaaaaacact tcctatttag gaaacaattt ataatggcag ttcgttcatt taacaattta 5400

tgtagaataa atgttataaa tgcgtatggg aaatcttaaa tatggatagc ataaatgata 5460

tctgcattgc ctaattcgaa atcaacagca acgaaaaaaa tcccttgtac aacataaata 5520

gtcatcgaga aatatcaact atcaaagaac agctattcac acgttactat tgagattatt 5580

attggacgag aatcacacac tcaactgtct ttctctcttc tagaaataca ggtacaagta 5640

tgtactattc tcattgttca tacttctagt catttcatcc cacatattcc ttggatttct 5700

ctccaatgaa tgacattcta tcttgcaaat tcaacaatta taataagata taccaaagta 5760

gcggtatagt ggcaatcaaa aagcttctct ggtgtgcttc tcgtatttat ttttattcta 5820

atgatccatt aaaggtatat atttatttct tgttatataa tccttttgtt tattacatgg 5880

gctggataca taaaggtatt ttgatttaat tttttgctta aattcaatcc cccctcgttc 5940

agtgtcaact gtaatggtag gaaattacca tacttttgaa gaagcaaaaa aaatgaaaga 6000

aaaaaaaaat cgtatttcca ggttagacgt tccgcagaat ctagaatgcg gtatgcggta 6060

cattgttctt cgaacgtaaa agttgcgctc cctgagatat tgtacatttt tgcttttaca 6120

agtacaagta catcgtacaa ctatgtacta ctgttgatgc atccacaaca gtttgttttg 6180

tttttttttg tttttttttt ttctaatgat tcattaccgc tatgtatacc tacttgtact 6240

tgtagtaagc cgggttattg gcgttcaatt aatcatagac ttatgaatct gcacggtgtg 6300

cgctgcgagt tacttttagc ttatgcatgc tacttgggtg taatattggg atctgttcgg 6360

aaatcaacgg atgctcaatc gatttcgaca gtaattaatt aagtcataca caagtcagct 6420

ttcttcgagc ctcatataag tataagtagt tcaacgtatt agcactgtac ccagcatctc 6480

cgtatcgaga aacacaacaa catgccccat tggacagatc atgcggatac acaggttgtg 6540

cagtatcata catactcgat cagacaggtc gtctgaccat catacaagct gaacaagcgc 6600

tccatacttg cacgctctct atatacacag ttaaattaca tatccatagt ctaacctcta 6660

acagttaatc ttctggtaag cctcccagcc agccttctgg tatcgcttgg cctcctcaat 6720

aggatctcgg ttctggccgt acagacctcg gccgacaatt atgatatccg ttccggtaga 6780

catgacatcc tcaacagttc ggtactgctg tccgagagcg tctcccttgt cgtcaagacc 6840
caccccgggg gtcagaataa gccagtcctc agagtcgccc ttaggtcggt tctgggcaat 6900
gaagccaacc acaaactcgg ggtcggatcg ggcaagctca atggtctgct tggagtactc 6960
gccagtggcc agagagccct tgcaagacag ctcggccagc atgagcagac ctctggccag 7020
cttctcgttg ggagagggga ctaggaactc cttgtactgg gagttctcgt agtcagagac 7080
gtcctccttc ttctgttcag agacagtttc ctcggcacca gctcgcaggc cagcaatgat 7140
tccggttccg ggtacaccgt gggcgttggt gatatcggac cactcggcga ttcggtgaca 7200
ccggtactgg tgcttgacag tgttgccaat atctgcgaac tttctgtcct cgaacaggaa 7260
gaaaccgtgc ttaagagcaa gttccttgag ggggagcaca gtgccggcgt aggtgaagtc 7320
gtcaatgatg tcgatatggg ttttgatcat gcacacataa ggtccgacct tatcggcaag 7380
ctcaatgagc tccttggtgg tggtaacatc cagagaagca cacaggttgg ttttcttggc 7440
tgccacgagc ttgagcactc gagcggcaaa ggcggacttg tggacgttag ctcgagcttc 7500
gtaggagggc attttggtgg tgaagaggag actgaaataa atttagtctg cagaactttt 7560
tatcggaacc ttatctgggg cagtgaagta tatgttatgg taatagttac gagttagttg 7620
aacttataga tagactggac tatacggcta tcggtccaaa ttagaaagaa cgtcaatggc 7680
tctctgggcg tcgcctttgc cgacaaaaat gtgatcatga tgaaagccag caatgacgtt 7740
gcagctgata ttgttgtcgg ccaaccgcgc cgaaaacgca gctgtcagac ccacagcctc 7800
caacgaagaa tgtatcgtca aagtgatcca agcacactca tagttggagt cgtactccaa 7860
aggcggcaat gacgagtcag acagatactc g 7891

<210> 101
<211> 9598
<212> DNA
<213> Artificial Sequence

<220>
<223> Plasmid pY177

<400> 101

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
ccagataagg ttccgataaa aagttctgca gactaaattt atttcagtct cctcttcacc     420
accaaaatgc cctcctacga agctcgagct aacgtccaca agtccgcctt tgccgctcga     480
gtgctcaagc tcgtggcagc caagaaaacc aacctgtgtg cttctctgga tgttaccacc     540
```

accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag ccccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
atttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460

agatgcgaga aactttgggt caagtgaaca tgtcctctct gttcaagtaa accatcaact 2520

atgggtagta tatttagtaa ggacaagagt tgagattctt tggagtccta gaaacgtatt 2580

ttcgcgttcc aagatcaaat tagtagagta atacgggcac gggaatccat tcatagtctc 2640

aattttccca taggtgtgct acaaggtgtt gagatgtggt acagtaccac catgattcga 2700

ggtaaagagc ccagaagtca ttgatgaggt caagaaatac acagatctac agctcaatac 2760

aatgaatatc ttctttcata ttcttcaggt gacaccaagg gtgtctattt tccccagaaa 2820

tgcgtgaaaa ggcgcgtgtg tagcgtggag tatgggttcg gttggcgtat ccttcatata 2880

tcgacgaaat agtagggcaa gagatgacaa aaagtatcta tatgtagaca gcgtagaata 2940

tggatttgat tggtataaat tcatttattg cgtgtctcac aaatactctc gataagttgg 3000

ggttaaactg gagatggaac aatgtcgata tctcgacgca tgcgacgtcg ggcccaattc 3060

gccctatagt gagtcgtatt acaattcact ggccgtcgtt ttacaacgtc gtgactggga 3120

aaaccctggc gttacccaac ttaatcgcct tgcagcacat ccccctttcg ccagctggcg 3180

taatagcgaa gaggcccgca ccgatcgccc ttcccaacag ttgcgcagcc tgaatggcga 3240

atggacgcgc cctgtagcgg cgcattaagc gcggcgggtg tggtggttac gcgcagcgtg 3300

accgctacac ttgccagcgc cctagcgccc gctcctttcg ctttcttccc ttcctttctc 3360

gccacgttcg ccggctttcc ccgtcaagct ctaaatcggg ggctcccttt agggttccga 3420

tttagtgctt tacggcacct cgaccccaaa aaacttgatt agggtgatgg ttcacgtagt 3480

gggccatcgc cctgatagac ggtttttcgc cctttgacgt tggagtccac gttctttaat 3540

agtggactct tgttccaaac tggaacaaca ctcaacccta tctcggtcta ttcttttgat 3600

ttataaggga ttttgccgat ttcggcctat tggttaaaaa atgagctgat ttaacaaaaa 3660

tttaacgcga attttaacaa aatattaacg cttacaattt cctgatgcgg tattttctcc 3720

ttacgcatct gtgcggtatt tcacaccgca tcaggtggca cttttcgggg aaatgtgcgc 3780

ggaaccccta tttgtttatt tttctaaata cattcaaata tgtatccgct catgagacaa 3840

taaccctgat aaatgcttca ataatattga aaaaggaaga gtatgagtat tcaacatttc 3900

cgtgtcgccc ttattccctt ttttgcggca ttttgccttc ctgtttttgc tcacccagaa 3960

acgctggtga aagtaaaaga tgctgaagat cagttgggtg cacgagtggg ttacatcgaa 4020

ctggatctca acagcggtaa gatccttgag agttttcgcc ccgaagaacg ttttccaatg 4080

atgagcactt ttaaagttct gctatgtggc gcggtattat cccgtattga cgccgggcaa 4140

gagcaactcg gtcgccgcat acactattct cagaatgact tggttgagta ctcaccagtc 4200

acagaaaagc atcttacgga tggcatgaca gtaagagaat tatgcagtgc tgccataacc 4260

atgagtgata acactgcggc caacttactt ctgacaacga tcggaggacc gaaggagcta 4320

accgcttttt tgcacaacat gggggatcat gtaactcgcc ttgatcgttg ggaaccggag 4380

ctgaatgaag ccataccaaa cgacgagcgt gacaccacga tgcctgtagc aatggcaaca 4440

acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac tgccgagatc 5760
cagtctacac tgattaattt tcgggccaat aatttaaaaa aatcgtgtta tataatatta 5820
tatgtattat atatatacat catgatgata ctgacagtca tgtcccattg ctaaatagac 5880
agactccatc tgccgcctcc aactgatgtt ctcaatattt aaggggtcat ctcgcattgt 5940
ttaataataa acagactcca tctaccgcct ccaaatgatg ttctcaaaat atattgtatg 6000
aacttatttt tattacttag tattattaga caacttactt gctttatgaa aaacacttcc 6060
tatttaggaa acaatttata atggcagttc gttcatttaa caatttatgt agaataaatg 6120
ttataaatgc gtatgggaaa tcttaaatat ggatagcata aatgatatct gcattgccta 6180
attcgaaatc aacagcaacg aaaaaaatcc cttgtacaac ataaatagtc atcgagaaat 6240
atcaactatc aaagaacagc tattcacacg ttactattga gattattatt ggacgagaat 6300
cacacactca actgtctttc tctcttctag aaatacaggt acaagtatgt actattctca 6360

ttgttcatac ttctagtcat ttcatcccac atattccttg gatttctctc caatgaatga 6420
cattctatct tgcaaattca acaattataa taagatatac caaagtagcg gtatagtggc 6480
aatcaaaaag cttctctggt gtgcttctcg tatttatttt tattctaatg atccattaaa 6540
ggtatatatt tatttcttgt tatataatcc ttttgtttat tacatgggct ggatacataa 6600
aggtattttg atttaatttt ttgcttaaat tcaatccccc ctcgttcagt gtcaactgta 6660
atggtaggaa attaccatac ttttgaagaa gcaaaaaaaa tgaaagaaaa aaaaaatcgt 6720
atttccaggt tagacgttcc gcagaatcta gaatgcggta tgcggtacat tgttcttcga 6780
acgtaaaagt tgcgctccct gagatattgt acatttttgc ttttacaagt acaagtacat 6840
cgtacaacta tgtactactg ttgatgcatc cacaacagtt tgttttgttt ttttttgttt 6900
tttttttttc taatgattca ttaccgctat gtatacctac ttgtacttgt agtaagccgg 6960
gttattggcg ttcaattaat catagactta tgaatctgca cggtgtgcgc tgcgagttac 7020
ttttagctta tgcatgctac ttgggtgtaa tattgggatc tgttcggaaa tcaacggatg 7080
ctcctaggtt cccaaaatcc cagatgcttc tctccagtgc caaaagtaag taccccacag 7140
gttttcggcc gaaaattcca cgtgcagcaa cgtcgtgtgg ggtgttaaaa tgtggggggg 7200
gggaaccagg acaagaggct cttgtgggag ccgaatgaga gcacaaagcg ggcgggtgtg 7260
ataagggcat ttttgcccat tttcccttct cctgtctctc cgacggtgat ggcgttgtgc 7320
gtcctctatt tctttttatt tctttttgtt ttatttctct gactaccgat ttggtttgat 7380
ttcctcaacc ccacacaaat aagctcgggc cgaggaatat atatatacac ggacacagtc 7440
gccctgtgga caacacgtca ctacctctac gatacacacc gtacgatagt tagtagacaa 7500
caatcgatag ttggagcaag ggagaaatgt agagtgtgaa agactcacta tggtccgggc 7560
ttatctcgac caatagccaa agtctggagt ttctgagaga aaaaggcaag atacgtatgt 7620
aacaaagcga cgcatggtac aataataccg gaggcatgta tcatagagag ttagtggttc 7680
gatgatggca ctggtgcctg gtatgacttt atacggctga ctacatattt gtcctcagac 7740
atacaattac agtcaagcac ttacccttgg acatctgtag gtaccccccg gccaagacga 7800
tctcagcgtg tcgtatgtcg gattggcgta gctccctcgc tcgtcaattg gctcccatct 7860
actttcttct gcttggctac acccagcatg tctgctatgg ctcgttttcg tgccttatct 7920
atcctcccag tattaccaac tctaaatgac atgatgtgat tgggtctaca ctttcatatc 7980
agagataagg agtagcacag ttgcataaaa agcccaactc taatcagctt cttcctttct 8040
tgtaattagt acaaaggtga ttagcgaaat ctggaagctt agttggccct aaaaaaatca 8100
aaaaaagcaa aaaacgaaaa acgaaaaacc acagttttga gaacagggag gtaacgaagg 8160
atcgtatata tatatatata tatatatacc cacggatccc gagaccggcc tttgattctt 8220
ccctacaacc aaccattctc accaccctaa ttcacaacca tgtccgttgc atccaagctc 8280
gtcttctacg tccgcgccgc catcgccgtg gtcatctttg ccgcctgtgc cacctacggc 8340

```
gtgctggcgt ccaccattct caccgccatc ggcaagcagg gcctggccca atggaccgtt     8400
gccagagcct tctactactc ggtgcgcatc ttcctgggta tcagcatcaa gctgcgtagc     8460
cggcaggtga ccggaaccgc cggtctggat gcctccaaga tccaggtcgc caacaccacc     8520
aagcccattg acgacatcac caaacacctg ccccgaccat gcattctgat ttccaaccac     8580
cagaacgaaa tggacattct ggtgctcggt cgcatcttcc cccagtactg ctccgtcacc     8640
gccaaaaagg ccctcaagtg gtaccctctg ctgggccagt tcatggcgct gtccggcacc     8700
atcttcctgg accgaaagga ccgaaccaag tccgtgcaga ccctcggcgg cgccgtcaag     8760
accatccaga gcggcaacgg aggcaagggc cagagcgtct tcatgttccc cgagggaacc     8820
cgatcctact ccaaggacgt cggcatcatg cccttcaaga agggctgttt ccacctggcg     8880
gtccagtcgg gcgctcccat tgtccccgtg gtggtccaga acacctcccg aatgttttct     8940
ttcggccgag gcaagctgga cgccggagag atccttgtcg acgtcctgag ccccattgag     9000
accaagggtc tggacgccag caacgtcgac gctctcatgg ccaccactta taaggccatg     9060
tgcgagactg ccgaccagat tggctacgct ggccagaaga ctcagtaggc ggccgcatga     9120
gaagataaat atataaatac attgagatat taaatgcgct agattagaga gcctcatact     9180
gctcggagag aagccaagac gagtactcaa aggggattac accatccata tccacagaca     9240
caagctgggg aaaggttcta tatacacttt ccggaatacc gtagtttccg atgttatcaa     9300
tgggggcagc caggatttca ggcacttcgg tgtctcgggg tgaaatggcg ttcttggcct     9360
ccatcaagtc gtaccatgtc ttcatttgcc tgtcaaagta aaacagaagc agatgaagaa     9420
tgaacttgaa gtgaaggaat ttaaatgtaa cgaaactgaa atttgaccag atattgtgtc     9480
cgcggtggag ctccagcttt tgttcccttt agtgagggtt aatttcgagc ttggcgtaat     9540
catggtcata gctgtttcct gtgtgaaatt gttatccgct cacaagcttc cacacaac       9598
```